(19) Europäisches Patentamt
European Patent Office
Office européen des brevets

(11) EP 1 914 229 B1

(12) EUROPEAN PATENT SPECIFICATION

(45) Date of publication and mention of the grant of the patent:
**16.06.2010 Bulletin 2010/24**

(21) Application number: **06796306.6**

(22) Date of filing: **08.08.2006**

(51) Int Cl.:
*C07D 307/91* (2006.01) *A61K 31/343* (2006.01)
*A61K 31/357* (2006.01) *A61K 31/36* (2006.01)
*A61K 31/443* (2006.01) *A61P 3/10* (2006.01)
*C07D 405/12* (2006.01) *C07D 407/12* (2006.01)

(86) International application number:
**PCT/JP2006/315621**

(87) International publication number:
**WO 2007/018193 (15.02.2007 Gazette 2007/07)**

# (54) NOVEL CERCOSPORAMIDE DERIVATIVE

NEUARTIGE CERCOSPORAMIDDERIVATE

NOUVEAU DÉRIVÉ DE CERCOSPORAMIDE

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.08.2005 JP 2005230558**
**28.12.2005 JP 2005379060**

(43) Date of publication of application:
**23.04.2008 Bulletin 2008/17**

(73) Proprietor: **Daiichi Sankyo Company, Limited**
**Chuo-ku**
**Tokyo 103-8426 (JP)**

(72) Inventors:
- **FURUKAWA, Akihiro**
  **1-2-58, Hiromachi, Shinagawa-ku, Tokyo 140-8710 (JP)**
- **OHSUMI, Jun**
  **1-2-58, Hiromachi, Shinagawa-ku, Tokyo 140-8710 (JP)**
- **ARITA, Tsuyoshi**
  **1-2-58, Hiromachi, Shinagawa-ku, Tokyo 140-8710 (JP)**
- **FUKUZAKI, Takehiro**
  **1-2-58, Hiromachi, Shinagawa-ku, Tokyo 140-8710 (JP)**
- **ARAKI, Kazushi**
  **1-2-58, Hiromachi, Shinagawa-ku, Tokyo 140-8710 (JP)**
- **MORI, Makoto**
  **1-2-58, Hiromachi, Shinagawa-ku, Tokyo 140-8710 (JP)**
- **MOMOSE, Takayuki**
  **1-2-58, Hiromachi, Shinagawa-ku, Tokyo 140-8710 (JP)**
- **HONDA, Takeshi**
  **1-2-58, Hiromachi, Shinagawa-ku, Tokyo 140-8710 (JP)**
- **KUROHA, Masanori**
  **1-2-58, Hiromachi, Shinagawa-ku, Tokyo 140-8710 (JP)**
- **SATOH, Susumu**
  **1-2-58, Hiromachi, Shinagawa-ku, Tokyo 140-8710 (JP)**

(74) Representative: **Gibson, Christian John Robert**
**Marks & Clerk LLP**
**90 Long Acre**
**London**
**WC2E 9RA (GB)**

(56) References cited:
**WO-A-03/059905 US-A- 4 983 587**

- **CONOVER M.A. ET AL.: 'Usnic acid amide, a phytotoxin and antifungal agent from Cercosporidum henningsii' PHYTOCHEMISTRY vol. 31, no. 9, 1992, pages 2999 - 3001, XP003008712**
- **COOPER A.B. ET AL.: 'Synthesis of (+)-8-methylcercosporamide: stereochemical correlation of natural (-)-cercosporamide with (+)-usnic acid' TETRAHEDRON vol. 48, no. 23, 1992, pages 4757 - 4766, XP003008713**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SUGAWARA F. ET AL.: 'The structure and biological activity of cercosporamide from Cercosporidium henningsii' JOURNAL OF ORGANIC CHEMISTRY vol. 56, no. 3, 1991, pages 909 - 910, XP003008714
- SUSSMAN A. ET AL.: 'Discovery of cercosporamide, a known antifungal natural product, as a selective pkc1 kinase inhibitor through high-throughput screening' EUKARYOTIC CELL vol. 3, no. 4, 2004, pages 932 - 943, XP003008715
- HONG Z. ET AL.: 'Cloning and characterization of KNR4, a yeast gene involved in (1,3)-beta-glucan synthesis' MOLECULAR AND CELLULAR BIOLOGY vol. 14, no. 2, 1994, pages 1017 - 1025, XP003008716

**Description**

Technical Field

The present invention relates to a medicine, in particular, a novel cercosporamide derivative, a pharmacologically acceptable salt thereof or an ester thereof having a hypoglycemic effect.
The present invention also relates to a therapeutic and/or prophylactic agent for diabetes (in particular, type II diabetes), comprising a novel cercosporamide derivative, a pharmacologically acceptable salt thereof or an ester thereof as an active ingredient.

Background Art

Cercosporamide is a natural substance isolated from cultures of the fungus *Cercosporidium henningsii* (Patent Document 1, Non-Patent Document 1, Non-Patent Document 2, Non-Patent Document 3, Non-Patent Document 4, Non-Patent Document 5). A known cercosporamide derivative is obtained by chemically modifying a hydroxyl group of cercosporamide to an ether bond, and cercosporamide and such a cercosporamide derivative have been reported to be useful as agricultural chemicals or antifungal agents (Patent Document 1). However, it is not known that cercosporamide and its ether derivative have a hypoglycemic effect. Further, already known cercosporamide derivatives do not have a substituent on the nitrogen atom of the carbamoyl group at the 4-position thereof, and cercosporamide derivatives not having a simple carbamoyl group ($-CONH_2$) at the 4-position have not been reported.

[Patent Document 1]
U.S. Patent No. 4,983,587
[Non-Patent Document 1]
J. Org. Chem., 56, 909-910 (1991).
[Non-Patent Document 2]
Tetrahedron, 48, 4757-4766 (1992).
[Non-Patent Document 3]
Molecular and Cellular Biology, 14, 1017-1025 (1994).
[Non-Patent Document 4]
Phytochemistry, 31, 2999-3001 (1992).
[Non-Patent Document 5]
Eukaryotic Cell, 3, 932-943 (2004).

WO 03/059905 A discloses dibenzofurane derivatives of pyrrolidone carboxamides, for the treatment of various conditions including diabetes and obesity.

Disclosure of the Invention

The present inventors have made extensive studies to develop a therapeutic and/or prophylactic agent for diabetes or the like and have found that a novel cercosporamide derivative having a substituent on the nitrogen atom of the carbamoyl group at the 4-position of cercosporamide has an excellent hypoglycemic effect. This finding has led to the completion of the present invention.
Specifically, the present invention provides a novel cercosporamide derivative, a pharmacologically acceptable salt thereof or an ester thereof useful as a therapeutic or prophylactic agent for diabetes (in particular, type II diabetes).
The present invention relates to a cercosporamide derivative having the general formula (I):

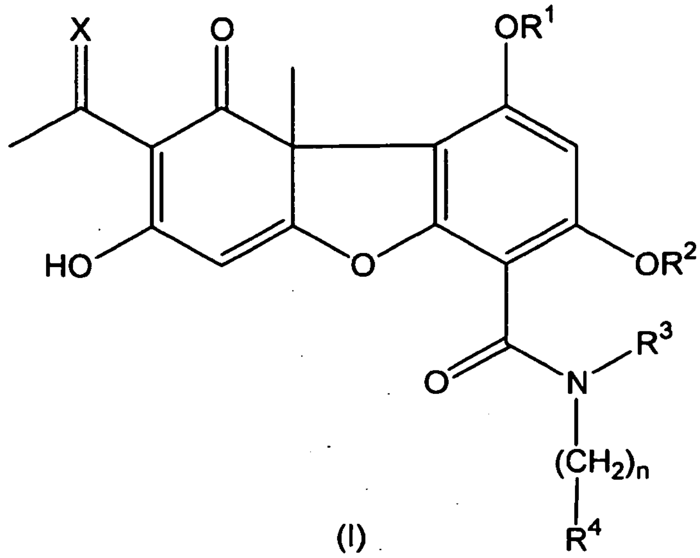

(I)

[wherein X represents an oxygen atom, a group represented by the formula =N-O-$R^5$ or a group represented by the formula =N-$R^5$,
$R^1$ represents a hydrogen atom or a $C_1$-$C_6$ alkyl group,
$R^2$ represents a hydrogen atom, a $C_1$-$C_6$ alkyl group or a $C_1$-$C_6$ halogenated alkyl group,
$R^3$ represents a hydrogen atom or a $C_1$-$C_6$ alkyl group,
$R^4$ represents a $C_6$-$C_{10}$ aryl group which may be substituted with one to five group(s) independently selected from Substituent Group a, a heterocyclic group which may be substituted with one to five group(s) independently selected from Substituent Group b or a phenyl group fused with $C_3$-$C_6$ cycloalkane which may be substituted with one to three group(s) independently selected from Substituent Group b,
$R^5$ represents a $C_1$-$C_6$ alkyl group, a $C_1$-$C_6$ halogenated alkyl group, a $C_2$-$C_6$ alkenyl group, a $C_2$-$C_6$ alkynyl group or a $C_1$-$C_6$ alkyl group substituted with one group selected from Substituent Group c,
n represents 1, 2 or 3,
Substituent Group a represents the group consisting of a halogen atom, a $C_1$-$C_6$ alkyl group, a $C_1$-$C_6$ halogenated alkyl group, a $C_2$-$C_6$ alkenyl group, a $C_2$-$C_6$ alkynyl group, a $C_1$-$C_6$ alkoxy group, a $C_1$-$C_6$ halogenated alkoxy group, a $C_2$-$C_6$ alkenyloxy group, a $C_2$-$C_6$ alkynyloxy group, a ($C_1$-$C_6$ alkoxy)-($C_1$-$C_6$ alkyl) group, a ($C_2$-$C_6$ alkenyloxy)-($C_1$-$C_6$ alkyl) group, a ($C_2$-$C_6$ alkynyloxy)-($C_1$-$C_6$ alkyl) group, a $C_1$-$C_6$ alkylthio group, a hydroxy group, a carboxyl group, a $C_2$-$C_7$ alkylcarbonyl group, a $C_4$-$C_7$ cycloalkylcarbonyl group, a $C_2$-$C_7$ alkylcarbonyloxy group, a $C_2$-$C_7$ alkoxycarbonyl group, an amino group, a mono-$C_1$-$C_6$ alkylamino group, a di-($C_1$-$C_6$ alkyl)amino group, a mono-($C_1$-$C_6$ alkyl)aminocarbonyl group, a di-($C_1$-$C_6$ alkyl)aminocarbonyl group, a mono-($C_1$-$C_6$ alkyl)aminocarbonyloxy group, a di-($C_1$-$C_6$ alkyl)aminocarbonyloxy group, a mono-$C_2$-$C_7$ alkylcarbonylamino group, a mono-$C_1$-$C_6$ alkylsulfonylamino group, a cyano group, a nitro group, a carbamoyl group, a phenyloxy group which may be substituted with one to five group(s) independently selected from Substituent Group d, a phenylthio group which may be substituted with one to five group (s) independently selected from Substituent Group d, a phenyloxymethyl group which may be substituted with one to five group(s) independently selected from Substituent Group d, a phenylmethyloxy group which may be substituted with one to five group(s) independently selected from Substituent Group d, a phenylthiomethyl group which may be substituted with one to five group(s) independently selected from Substituent Group d, a phenyloxycarbonyl group which may be substituted with one to five group(s) independently selected from Substituent Group d, a phenylcarbonylamino group which may be substituted with one to five group(s) independently selected from Substituent Group d, a phenylsulfonylamino group which may be substituted with one to five group(s) independently selected from Substituent Group d, a phenylaminocarbonyloxy group which may be substituted with one to five group(s) independently selected from Substituent Group d, a phenyloxycarbonylamino group which may be substituted with one to five group (s) independently selected from Substituent Group d, a phenylaminocarbonylamino group which may be substituted with one to five group(s) independently selected from Substituent Group d, a $C_1$-$C_6$ alkyl group substituted with one carboxyl group, a $C_1$-$C_6$ alkyl group substituted with one $C_2$-$C_7$ alkoxycarbonyl group, a $C_1$-$C_6$ alkoxy group substituted with one $C_2$-$C_7$ alkoxycarbonyl group and a $C_1$-$C_6$ alkoxy group substituted with one di-($C_1$-$C_6$ alkyl)aminocarbonyl group,

Substituent Group b represents the group consisting of a halogen atom, a $C_1$-$C_6$ alkyl group, a $C_1$-$C_6$ halogenated alkyl group, a $C_1$-$C_6$ alkoxy group, a $C_2$-$C_6$ alkynyloxy group, a hydroxy group and a carboxyl group,
Substituent Group c represents the group consisting of a $C_3$-$C_6$ cycloalkyl group, a phenyl group, a hydroxy group, a carboxyl group, a $C_2$-$C_7$ alkylcarbonyl group and a $C_2$-$C_7$ alkoxycarbonyl group, and
Substituent Group d represents the group consisting of a halogen atom, a $C_1$-$C_6$ alkyl group, a $C_1$-$C_6$ halogenated alkyl group and a $C_1$-$C_6$ alkoxy group],
a pharmacologically acceptable salt thereof or an ester thereof.

Preferred embodiments of the present invention include:

(2) The cercosporamide derivative, a pharmacologically acceptable salt thereof or an ester thereof according to (1), wherein the general formula (I) is a general formula (Ia):

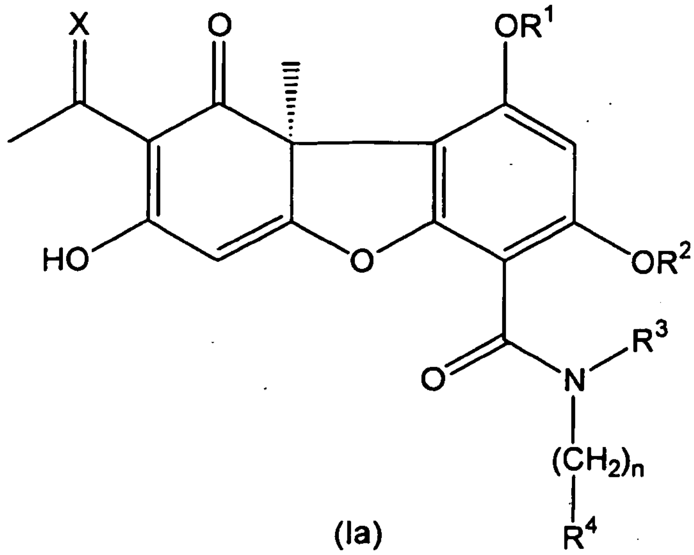

(Ia) ;

(3) The cercosporamide derivative, a pharmacologically acceptable salt thereof or an ester thereof according to (1) or (2), wherein X is an oxygen atom or a group represented by the formula =N-O-$R^5$;
(4) The cercosporamide derivative, a pharmacologically acceptable salt thereof or an ester thereof according to any one of (1) to (3), wherein $R^5$ is a methyl group, an ethyl group, a propyl group, a 2-propenyl group or a cyclopropylmethyl group;
(5) The cercosporamide derivative, a pharmacologically acceptable salt thereof or an ester thereof according to (1) or (2), wherein X is an oxygen atom;
(6) The cercosporamide derivative, a pharmacologically acceptable salt thereof or an ester thereof according to any one of (1) to (5), wherein $R^1$ is a hydrogen atom;
(7) The cercosporamide derivative, a pharmacologically acceptable salt thereof or an ester thereof according to any one of (1) to (6), wherein $R^2$ is a methyl group;
(8) The cercosporamide derivative, a pharmacologically acceptable salt thereof or an ester thereof according to any one of (1) to (7), wherein $R^3$ is a hydrogen atom;
(9) The cercosporamide derivative, a pharmacologically acceptable salt thereof or an ester thereof according to any one of (1) to (8), wherein $R^4$ is a phenyl group substituted with one to five group(s) independently selected from Substituent Group a or a 1-naphthyl group which may be substituted with one to three group(s) independently selected from Substituent Group a;
(10) The cercosporamide derivative, a pharmacologically acceptable salt thereof or an ester thereof according to any one of (1) to (8), wherein $R^4$ is a phenyl group substituted with one to five group(s) independently selected from Substituent Group e or a 1-naphthyl group which may be substituted with one to three group(s) independently selected from Substituent Group e, and
Substituent Group e represents the group consisting of a halogen atom; a $C_1$-$C_6$ alkyl group; a $C_1$-$C_6$ halogenated alkyl group; a $C_2$-$C_6$ alkenyl group; a $C_1$-$C_6$ alkoxy group; a $C_2$-$C_6$ alkynyloxy group; a ($C_1$-$C_6$ alkoxy)-($C_1$-$C_6$ alkyl) group; a ($C_2$-$C_6$ alkynyloxy)-($C_1$-$C_6$ alkyl) group; a hydroxy group; a carboxyl group; a $C_4$-$C_7$ cycloalkylcarbonyl

group; a $C_2$-$C_7$ alkoxycarbonyl group; a di-($C_1$-$C_6$ alkyl)aminocarbonyl group; a di-($C_1$-$C_6$ alkyl)aminocarbonyloxy group; a mono-$C_1$-$C_6$ alkylsulfonylamino group; a cyano group; a nitro group; a phenyloxy group, a phenylthio group, a phenyloxymethyl group, a phenylmethyloxy group, a phenyloxycarbonyl group, a phenylcarbonylamino group or a phenylsulfonylamino group which may be substituted with one to three group(s) independently selected from a fluorine atom, a chlorine atom, a methyl group and a trifluoromethyl group; a $C_1$-$C_6$ alkyl group substituted with one carboxyl group; and a $C_1$-$C_6$ alkyl group substituted with one $C_2$-$C_7$ alkoxycarbonyl group;

(11) The cercosporamide derivative, a pharmacologically acceptable salt thereof or an ester thereof according to any one of (1) to (8), wherein $R^4$ is a 1-naphthyl group substituted with one to five group(s) independently selected from a fluorine atom, a chlorine atom, a bromine atom; a methyl group and an ethyl group;

(12) The cercosporamide derivative, a pharmacologically acceptable salt thereof or an ester thereof according to any one of (1) to (8), wherein $R^4$ is a 1-naphthyl group substituted at the 2- and/or 3-position(s) with one or two group(s) independently selected from a methyl group and an ethyl group and substituted at the 4-, 5-, 6-, 7- and/or 8-position(s) with one to three group(s) independently selected from a fluorine atom and a chlorine atom;

(13) The cercosporamide derivative, a pharmacologically acceptable salt thereof or an ester thereof according to any one of (1) to (8), wherein $R^4$ is a 1-naphthyl group substituted at the 2-position with a methyl group or an ethyl group and substituted at the 4-, 5-, 6-, 7- and/or 8-position(s) with one to three group(s) independently selected from a fluorine atom and a chlorine atom;

(14) The cercosporamide derivative, a pharmacologically acceptable salt thereof or an ester thereof according to any one of (1) to (13), wherein n is 1;

(15) The cercosporamide derivative, a pharmacologically acceptable salt thereof or an ester thereof according to (1), wherein the compound having the general formula (I) is
(9aS)-8-acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide,
(9aS)-8-acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-9-oxo-N-(2,3,5,6-tetramethylbenzyl)-9,9a-dihydrodibenzo[b,d]furan-4-carboxamide,
(9aS)-8-acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide,
(9aS)-8-acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide,
(9aS)-8-acetyl-1,7-dihydroxy-3-methoxy-9a-methyl-N-(1-naphthylmethyl)-9-oxo-9,9a-dihydrodibenzo[b,d]furan-4-carboxamide,
(9aS)-8-acetyl-N-[(4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2-methyl-1-naphthyl)methyl]-9-oxo-9,9a-dihydrodibenzo[b,d]furan-4-carboxamide,
(9aS)-8-acetyl-N-[(2-ethyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-chlorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamide,
(9aS)-8-acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide,
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide,
(9aS)-8-acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide,
(9aS)-1,7-dihydroxy-3-methoxy-8-[(E)-N-methoxyethaneimidoyl]-9a-methyl-N-[(2-methyl-1-naphthyl)methyl]-9-oxo-9,9a-dihydrodibenzo[b,d]furan-4-carboxamide or
(9aS)-8-[(1E)-N-(allyloxy)ethaneimidoyl]-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide;
(16) The cercosporamide derivative, a pharmacologically acceptable salt thereof or an ester thereof according to (1), wherein the compound having the general formula (I) is
(9aS)-8-acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-chloro-2-ethyl-1-naphthyl)methyl]-1,7-dihydroxy-3-methoxy-9a-methyl-9-oxo-9,9a-dihydrodibenzo[b,d]fiuan-4-carboxamide,
(9aS)-8-acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-6,7-difluoro-1-naphthyl)methyl]- 1,7-dihydroxy-3-methoxy-9a-methyl-9-oxo-9,9a-dihydrodibenzo[b,d]furan-4-carboxamide,
(9aS)-8-acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide or (9aS)-8-acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide;
(17) The cercosporamide derivative or a pharmacologically acceptable salt thereof according to (1), wherein the compound having the general formula (I) is
(9aS)-8-acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide,
(9aS)-8-acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-9-oxo-N-(2,3,5,6-tetramethylbenzyl)-9,9a-dihydrodibenzo[b,d]furan-4-carboxamide,
(9aS)-8-acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide,
(9aS)-8-acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide,

(9aS)-8-acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide,
(9aS)-8-acetyl-1,7-dihydroxy-3-methoxy-9a-methyl-N-(1-naphthylmethyl)-9-oxo-9,9a-dihydrodibenzo[b,d]furan-4-carboxamide,
(9aS)-8-acetyl-N-[(4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2-methyl-1-naphthyl)methyl]-9-oxo-9,9a-dihydrodibenzo[b,d]furan-4-carboxamide,
(9aS)-8-acetyl-N-[(2-ethyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-chlorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(2,4-difluorobenyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamide,
(9aS)-8-acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide,
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide,
(9aS)-8-acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide,
(9aS)-1,7-dihydroxy-3-methoxy-8-[(E)-N-methoxyethaneimidoyl]-9a-methyl-N-[(2-methyl-1-naphthyl)methyl]-9-oxo-9,9a-dihydrodibenzo[b,d]furan-4-carboxamide or
(9aS)-8-[(1E)-N-(allyloxy)ethaneimidoyl]-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide;
(18) The cercosporamide derivative or a pharmacologically acceptable salt thereof according to (1), wherein the compound having the general formula (I) is
(9aS)-8-acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide,

(9aS)-8-acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamideor
(9aS)-8-acetyl-1,7-dihydroxy-3-methoxy-9a-methyl-9-oxo-N-[(5,6,7-trifluoro-2-methyl-l-naphthyl)methyl]-9,9a-dihydrodibenzo[b,d]furan-4-carboxamide;
(19) A pharmaceutical composition comprising the cercosporamide derivative, a pharmacologically acceptable salt thereof or an ester thereof according to any one of (1) to (18) as an active ingredient;
(20) The pharmaceutical composition according to (19) for lowering blood glucose, comprising the cercosporamide derivative, a pharmacologically acceptable salt thereof or an ester thereof according to any one of (1) to (18) as an active ingredient;
(21) The pharmaceutical composition according to (19) for treatment and/or prevention of diabetes, comprising the cercosporamide derivative, a pharmacologically acceptable salt thereof or an ester thereof according to any one of (1) to (18) as an active ingredient;
(22) Use of the cercosporamide derivative, a pharmacologically acceptable salt thereof or an ester thereof according to any one of (1) to (18) for the manufacture of a pharmaceutical composition;
(23) The use according to (22), wherein the pharmaceutical composition is a composition for lowering blood glucose;
(24) The use according to (22), wherein the pharmaceutical composition is a composition for treatment and/or prevention of diabetes.

The "$C_1$-$C_6$ alkyl group" in the present invention is a linear or branched alkyl group having one to six carbon atom(s). Examples of such a group include a methyl group, ethyl group, propyl group, isopropyl group, butyl group, isobutyl group, s-butyl group, t-butyl group, pentyl group, isopentyl group, 2-methylbutyl group, neopentyl group, 1-ethylpropyl group, hexyl group, isohexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, 1,3-dimethylbutyl group, 2,3-dimethylbutyl group, 1-ethylbutyl group and 2-ethylbutyl group. The group is preferably a linear or branched alkyl group having one to four carbon atom(s) ($C_1$-$C_4$ alkyl group), more preferably a methyl group, ethyl group or propyl group, still more preferably a methyl group or ethyl group ($C_1$-$C_2$ alkyl group), and particularly preferably a methyl group for $R^2$, $R^3$, $R^5$ and Substituent Group d.
The "halogen atom" in the present invention is a fluorine atom, chlorine atom, bromine atom or iodine atom. The halogen atom is preferably a fluorine atom or chlorine atom, and more preferably a fluorine atom for Substituent Group a.
The "$C_1$-$C_6$ halogenated alkyl group" in the present invention is a group in which the same or different one to five aforementioned "halogen atom" are bonded to the aforementioned "$C_1$-$C_6$ alkyl group". Examples of such a group include a trifluoromethyl group, trichloromethyl group, difluoromethyl group, dibromomethyl group, fluoromethyl group, 2,2,2-trifluoroethyl group, 2,2,2-trichloroethyl group, 2-chloroethyl group, 2-fluoroethyl group, pentafluoroethyl group, 3-chloropropyl group, 4-fluorobutyl group and 6-iodohexyl group. The group is preferably a group in which the same or different one to five aforementioned "halogen atom" are bonded to the aforementioned "$C_1$-$C_4$ alkyl group" ($C_1$-$C_4$ halogenated alkyl group), more preferably a group in which the same or different one to five aforementioned "halogen atom" are bonded to the aforementioned "$C_1$-$C_2$ alkyl group" ($C_1$-$C_2$ halogenated alkyl group), still more preferably a difluoromethyl group for $R^2$ and Substituent Group a, and still more preferably a trifluoromethyl group for Substituent Group d.
The "$C_6$-$C_{10}$ aryl group" in the present invention is an aromatic hydrocarbon group having six to ten carbon atoms. The group is, for example, a phenyl group, indenyl group or a naphthyl group, preferably a phenyl group or naphthyl group, and more preferably a naphthyl group.
The "heterocyclic group" in the present invention is a four- to seven-membered heterocyclic group which contains one to three sulfur atom(s), oxygen atom(s) or/and nitrogen atom(s) and may further contain one or two nitrogen atom(s) and in which two oxygen atoms may be bonded to the sulfur atom. Examples of such a group include "aromatic heterocyclic groups" such as a furyl group, thienyl group, pyrrolyl group, azepinyl group, pyrazolyl group, imidazolyl group, oxazolyl group, isoxazolyl group, thiazolyl group, isothiazolyl group, 1,2,3-oxadiazolyl group, triazolyl group, tetrazolyl group, thiadiazolyl group, pyranyl group, pyridyl group, pyridazinyl group, pyrimidinyl group and pyrazinyl group; and "partially or completely reduced saturated heterocyclic groups" such as a tetrahydropyranyl group, tetrahydrothienyl group, morpholinyl group, thiomorpholinyl group, pyrrolidinyl group, pyrrolinyl group, imidazolidinyl group, pyrazolidinyl group, piperidinyl group, piperazinyl group, oxazolidinyl group, isoxazolidinyl group, thiazolidinyl group, pyrazolidinyl group, dioxolanyl group and dioxanyl group. The aforementioned heterocyclic group may be fused with another cyclic group such as a benzene ring ("fused bicyclic heteroaryl group"). Examples of such a group include a

benzothienyl group, benzothiazolyl group, benzoxazolyl group, isobenzofuranyl group, 1,3-dihydroisobenzofuranyl group, quinolyl group, 1,3-benzodioxolanyl group, 1,4-benzodioxanyl group, indolyl group, isoindolyl group and indolinyl group. The group is preferably a five- to seven-membered heterocyclic group containing one to three sulfur atom(s), oxygen atom(s) or/and nitrogen atom(s), more preferably a pyridyl group, 1,3-benzodioxolanyl group or 1,4-benzodioxanyl group, and still more preferably a 3-pyridyl group or 1,4-benzodioxanyl group.

The "$C_3$-$C_6$ cycloalkane" in the present invention is cyclopropane, cyclobutane, cyclopentane or cyclohexane.

The "phenyl group fused with $C_3$-$C_6$ cycloalkane" in the present invention is a group in which a phenyl group is fused with the aforementioned "$C_3$-$C_6$ cycloalkane". Such a group is preferably a 5,6,7,8-tetrahydro-1-naphthyl group or 2,3-dihydro-1H-indan-4-yl group.

The "$C_2$-$C_6$ alkenyl group" in the present invention is the aforementioned "$C_1$-$C_6$ alkyl group" having one double bond and two to six carbon atoms. Examples of such a group include an ethenyl group, 1-propenyl group, 2-propenyl group, 1-methyl-2-propenyl group, 1-methyl-1-propenyl group, 2-methyl-1-propenyl group, 2-methyl-2-propenyl group, 2-ethyl-2-propenyl group, 1-butenyl group, 2-butenyl group, 1-methyl-2-butenyl group, 1-ethyl-2-butenyl group, 3-butenyl group, 1-methyl-3-butenyl group, 1-pentenyl group, 4-pentenyl group, 1-methyl-4-pentenyl group, 2-methyl-4-pentenyl group and 5-hexenyl group. The group is preferably an alkenyl group having two to four carbon atoms ($C_2$-$C_4$ alkenyl group), and more preferably a 2-propenyl group.

The "$C_2$-$C_6$ alkynyl group" in the present invention is the aforementioned "$C_1$-$C_6$ alkyl group" having one triple bond and two to six carbon atoms. Examples of such a group include an ethynyl group, 1-propynyl group, 2-propynyl group, 1-methyl-2-propynyl group, 2-methyl-2-propynyl group, 2-ethyl-2-propynyl group, 1-butynyl group, 2-butynyl group, 1-methyl-2-butynyl group, 2-methyl-2-butynyl group, 1-ethyl-2-butynyl group, 3-butynyl group, 1-methyl-3-butynyl group, 2-methyl-3-butynyl group, 2-pentynyl group, 2-methyl-4-pentynyl group and 5-hexynyl group. The group is preferably an alkynyl group having two to four carbon atoms ($C_2$-$C_4$ alkynyl group), and more preferably a 2-propynyl group or 2-butynyl group.

The "$C_1$-$C_6$ alkoxy group" in the present invention is a group in which the aforementioned aforementioned "$C_1$-$C_6$ alkyl group" is bonded to an oxygen atom, and is a linear or branched alkoxy group having one to six carbon atom(s). Examples of such a group include a methoxy group, ethoxy group, propoxy group, isopropoxy group, butoxy group, isobutoxy group, s-butoxy group, t-butoxy group, pentoxy group, 2-methylbutoxy group, 2-ethylpropoxy group, neopentoxy group, 4-methylpentoxy group and 2,3-dimethylbutoxy group. The group is preferably a linear or branched alkoxy group having one to four carbon atom(s) ($C_1$-$C_4$ alkoxy group), and more preferably a propoxy group or butoxy group.

The "$C_1$-$C_6$ halogenated alkoxy group" in the present invention is a group in which the same or different one to five aforementioned "halogen atom" are bonded to the aforementioned "$C_1$-$C_6$ alkoxy group". Examples of such a group include a trifluoromethoxy group, trichloromethoxy group, difluoromethoxy group, dibromomethoxy group, fluoromethoxy group, 2,2,2-trifluoroethoxy group, 2,2,2-trichloroethoxy group, 2-bromoethoxy group, 2-chloroethoxy group, 2-fluoroethoxy group, 2-iodoethoxy group, pentafluoroethoxy group, 4-fluorobutoxy group and 6-iodoheptyloxy group. The group is preferably a group in which the same or different one to five aforementioned "halogen atom" are bonded to the aforementioned "$C_1$-$C_4$ alkoxy group" ($C_1$-$C_4$ halogenated alkoxy group), more preferably a group in which the same or different one to five aforementioned "halogen atom" are bonded to the aforementioned "$C_1$-$C_2$ alkoxy group" ($C_1$-$C_2$ halogenated alkoxy group), and still more preferably a trifluoromethoxy group.

The "$C_2$-$C_6$ alkenyloxy group" in the present invention is a group in which the aforementioned "$C_2$-$C_6$ alkenyl group" is bonded to an oxygen atom, and is an alkenyloxy group having two to six carbon atoms. Examples of such a group include an ethenyloxy group, 2-propenyloxy group, 1-propenyloxy group, 3-butenyloxy group, 2-butenyloxy group, 1-butenyloxy group and 5-hexenyloxy group. The group is preferably a linear or branched alkenyloxy group having two to four carbon atoms ($C_2$-$C_4$ alkenyloxy group), and more preferably a 2-propenyloxy group.

The "$C_2$-$C_6$ alkynyloxy group" in the present invention is a group in which the aforementioned "$C_2$-$C_6$ alkynyl group" is bonded to an oxygen atom, and is an alkynyloxy group having two to six carbon atoms. Examples of such a group include an ethynyloxy group, 2-propynyloxy group, 1-propynyloxy group, 3-butynyloxy group, 2-butynyloxy group, 1-butynyloxy group, 2-pentynyloxy group and 5-hexynyloxy group. The group is preferably a linear or branched alkynyloxy group having four to six carbon atoms ($C_4$-$C_6$ alkynyloxy group), and more preferably a 2-butynyloxy group or 2-pentynyloxy group.

The "($C_1$-$C_6$ alkoxy)-($C_1$-$C_6$ alkyl) group" in the present invention is a group in which one aforementioned "$C_1$-$C_6$ alkoxy group" is bonded to the aforementioned "$C_1$-$C_6$ alkyl group". Examples of such a group include a methoxymethyl group, ethoxymethyl group, propoxymethyl group, isopropoxymethyl group, butoxymethyl group, isobutoxymethyl group, s-butoxymethyl group, t-butoxymethyl group, 2-methoxyethyl group, 2-ethoxyethyl group, 2-butoxyethyl group, 1-butoxyethyl group, 1-isobutoxyethyl group and 3-isopropoxypropyl group. The group is preferably a group in which one aforementioned "$C_1$-$C_4$ alkoxy group" is bonded to the aforementioned "$C_1$-$C_4$ alkyl group" (($C_1$-$C_4$ alkoxy)-($C_1$-$C_4$ alkyl) group), more preferably a group in which one aforementioned "$C_1$-$C_2$ alkoxy group" is bonded to the aforementioned "$C_1$-$C_2$ alkyl group" (($C_1$-$C_2$ alkoxy)-($C_1$-$C_2$ alkyl) group), and still more preferably a methoxymethyl group.

The "($C_2$-$C_6$ alkenyloxy)-($C_1$-$C_6$ alkyl) group" in the present invention is a group in which one aforementioned "$C_2$-$C_6$ alkenyloxy group" is bonded to the aforementioned "$C_1$-$C_6$ alkyl group". Examples of such a group include an ethenyloxymethyl group, 2-propenyloxymethyl group, 1-propenyloxymethyl group, 3-butenyloxymethyl group, 5-hexenyloxymethyl group, ethenyloxyethyl group, 2-propenyloxyethyl group and 2-propenyloxypropyl group. The group is preferably a group in which one aforementioned "$C_2$-$C_4$ alkenyloxy group" is bonded to the aforementioned "$C_1$-$C_4$ alkyl group" (($C_2$-$C_4$ alkenyloxy)-($C_1$-$C_4$ alkyl) group), and more preferably a 2-propenyloxymethyl group.

The "($C_2$-$C_6$ alkynyloxy)-($C_1$-$C_6$ alkyl) group" in the present invention is a group in which one aforementioned "$C_2$-$C_6$ alkynyloxy group" is bonded to the aforementioned "$C_1$-$C_6$ alkyl group". Examples of such a group include an ethynyloxymethyl group, 2-propynyloxymethyl group, 1-propynyloxymethyl group, 3-butynyloxymethyl group, 2-butynyloxymethyl group, 1-butynyloxymethyl group, 5-hexynyloxymethyl group, 2-pentynyloxymethyl group, ethynyloxypropyl group and 2-propynyloxypropyl group. The group is preferably a group in which one aforementioned "$C_4$-$C_6$ alkynyloxy group" is bonded to the aforementioned "$C_1$-$C_4$ alkyl group" (($C_4$-$C_6$ alkynyloxy)-($C_1$-$C_4$ alkyl) group), more preferably a 2-butynyloxymethyl group or 2-pentynyloxymethyl group, and still more preferably a 2-butynyloxymethyl group.

The "$C_1$-$C_6$ alkylthio group" in the present invention is a group in which the aforementioned "$C_1$-$C_6$ alkyl group" is bonded to a sulfur atom, and is a linear or branched alkylthio group having one to six carbon atom(s). Examples of such a group include a methylthio group, ethylthio group, propylthio group, isopropylthio group, butylthio group, isobutylthio group, s-butylthio group, t-butylthio group, pentylthio group, isopentylthio group, 2-methylbutylthio group, neopentylthio group, 1-ethylpropylthio group, hexylthio group, isohexylthio group and 2-ethylbutylthio group. The group is preferably a linear or branched alkylthio group having one to four carbon atom(s) ($C_1$-$C_4$ alkylthio group), and more preferably a propylthio group or butylthio group.

The "$C_2$-$C_7$ alkylcarbonyl group" in the present invention is a group in which the aforementioned "$C_1$-$C_6$ alkyl group" is bonded to a carbonyl group. Examples of such a group include an acetyl group, propionyl group, butyryl group, isobutyryl group, pentanoyl group, pivaloyl group, valeryl group and isovaleryl group. The group is preferably a group in which the aforementioned "$C_1$-$C_4$ alkyl group" is bonded to a carbonyl group ($C_2$-$C_5$ alkylcarbonyl group), more preferably an acetyl group or propionyl group ($C_2$-$C_3$ alkylcarbonyl group), and still more preferably an acetyl group.

The "$C_4$-$C_7$ cycloalkylcarbonyl group" in the present invention is a cyclopropylcarbonyl group, cyclobutylcarbonyl group, cyclopentylcarbonyl group or cyclohexylcarbonyl group. The group is preferably a cyclopropylcarbonyl group.

The "$C_2$-$C_7$ alkylcarbonyloxy group" in the present invention is a group in which the aforementioned "$C_2$-$C_7$ alkylcarbonyl group" is bonded to an oxygen atom. Examples of such a group include an acetyloxy group, propionyloxy group, butyryloxy group, isobutyryloxy group, pentanoyloxy group, pivaloyloxy group, valeryloxy group and isovaleryloxy group. The group is preferably a group in which the aforementioned "$C_2$-$C_5$ alkylcarbonyl group" is bonded to an oxygen atom ($C_2$-$C_5$ alkylcarbonyloxy group), more preferably an acetyloxy group or propionyloxy group ($C_2$-$C_3$ alkylcarbonyloxy group), and still more preferably an acetyloxy group.

The "$C_2$-$C_7$ alkoxycarbonyl group" in the present invention is a group in which the aforementioned "$C_1$-$C_6$ alkoxy group" is bonded to a carbonyl group. Examples of such a group include a methoxycarbonyl group, ethoxycarbonyl group, propoxycarbonyl group, isopropoxycarbonyl group, butoxycarbonyl group, isobutoxycarbonyl group, s-butoxycarbonyl group, t-butoxycarbonyl group, pentoxycarbonyl group, 2-methylbutoxycarbonyl group, neopentoxycarbonyl group, hexyloxycarbonyl group, 4-methylpentoxycarbonyl group, 1,1-dimethylbutoxycarbonyl group, 1,2-dimethylbutoxycarbonyl group and 2,3-dimethylbutoxycarbonyl group. The group is preferably a group in which the aforementioned "$C_1$-$C_4$ alkoxy group" is bonded to a carbonyl group ($C_2$-$C_5$ alkoxycarbonyl group), more preferably a methoxycarbonyl group or ethoxycarbonyl group ($C_2$-$C_3$ alkoxycarbonyl group), and still more preferably a methoxycarbonyl group.

The "mono-$C_1$-$C_6$ alkylamino group" in the present invention is a group in which one aforementioned "$C_1$-$C_6$ alkyl group" is bonded to an amino group. Examples of such a group include a methylamino group, ethylamino group, propylamino group, isopropylamino group, butylamino group, isobutylamino group, s-butylamino group, t-butylamino group, pentylamino group, isopentylamino group, 2-methylbutylamino group, neopentylamino group and 2-ethylbutylamino group. The group is preferably a group in which one aforementioned "$C_1$-$C_4$ alkyl group" is bonded to an amino group (mono-$C_1$-$C_4$ alkylamino group), more preferably a methylamino group or ethylamino group (mono-$C_1$-$C_2$ alkylamino group), and still more preferably a methylamino group.

The "dl-($C_1$-$C_6$ alkyl)amino group" in the present invention is a group in which the same or different two aforementioned "$C_1$-$C_6$ alkyl group" are bonded to an amino group. Examples of such a group include a dimethylamino group, diethylamino group, dipropylamino group, diisopropylamino group, dibutylamino group, dipentylamino group, diisopentylamino group, dineopentylamino group, dihexylamino group, N-ethyl-N-methylamino group, N-methyl-N-propylamino group, N-isopropyl-N-methylamino group, N-butyl-N-methylamino group, N-isobutyl-N-methylamino group, N-methyl-N-pentylamino group and N-ethyl-N-isopentylamino group. The group is preferably a group in which the same or different two aforementioned "$C_1$-$C_4$ alkyl group" are bonded to an amino group (di-($C_1$-$C_4$ alkyl)amino group), more preferably a dimethylamino group, diethylamino group or N-ethyl-N-methylamino group (di-($C_1$-$C_2$ alkyl)amino group), and still more preferably a dimethylamino group.

The "mono-($C_1$-$C_6$ alkyl)aminocarbonyl group" in the present invention is a group in which an amino group

with one aforementioned "$C_1$-$C_6$ alkyl group" bonded thereto is bonded to a carbonyl group. Examples of such a group include a methylaminocarbonyl group, ethylaminocarbonyl group, propylaminocarbonyl group, isopropylaminocarbonyl group, butylaminocarbonyl group, isobutylaminocarbonyl group, s-butylaminocarbonyl group, t-butylaminocarbonyl group, pentylaminocarbonyl group, isopentylaminocarbonyl group, 2-methylbutylaminocarbonyl group, neopentylaminocarbonyl group, 1-ethylpropylaminocarbonyl group and 2-ethylbutylaminocarbonyl group. The group is preferably a group in which an amino group with one aforementioned "$C_1$-$C_4$ alkyl group" bonded thereto is bonded to a carbonyl group (mono-($C_1$-$C_4$ alkyl)aminocarbonyl group), more preferably a methylaminocarbonyl group or ethylaminocarbonyl group (mono-($C_1$-$C_2$ alkyl)aminocarbonyl group), and still more preferably a methylaminocarbonyl group.

The "di-($C_1$-$C_6$ alkyl)aminocarbonyl group" in the present invention is a group in which an amino group with the same or different two aforementioned "$C_1$-$C_6$ alkyl group" bonded thereto is bonded to a carbonyl group. Examples of such a group include a dimethylaminocarbonyl group, diethylaminocarbonyl group, dipropylaminocarbonyl group, diisopropylaminocarbonyl group, dibutylaminocarbonyl group, N-ethyl-N-methylaminocarbonyl group, N-methyl-N-propylaminocarbonyl group, N-isopropyl-N-methylaminocarbonyl group, N-butyl-N-methylaminocarbonyl group and N-ethyl-N-isopentylaminocarbonyl group. The group is preferably a group in which an amino group with the same or different two aforementioned "$C_1$-$C_4$ alkyl group" bonded thereto is bonded to a carbonyl group (di-($C_1$-$C_4$ alkyl)aminocarbonyl group), more preferably a dimethylaminocarbonyl group, diethylaminocarbonyl group or N-ethyl-N-methylaminocarbonyl group (di-($C_1$-$C_2$ alkyl)aminocarbonyl group), and still more preferably a dimethylaminocarbonyl group.

The "mono-($C_1$-$C_6$ alkyl)aminocarbonyloxy group" in the present invention is a group in which a carbonyl group with one aforementioned "mono-($C_1$-$C_6$ alkyl)amino group" bonded thereto is bonded to an oxygen atom. Examples of such a group include a methylaminocarbonyloxy group, ethylaminocarbonyloxy group, propylaminocarbonyloxy group, isopropylaminocarbonyloxy group, butylaminocarbonyloxy group, isobutylaminocarbonyloxy group, s-butylaminocarbonyloxy group, t-butylaminocarbonyloxy group, pentylaminocarbonyloxy group and 2-ethylbutylaminocarbonyloxy group. The group is preferably a group in which a carbonyl group with one aforementioned "$C_1$-$C_4$ alkylamino group" bonded thereto is bonded to an oxygen atom (mono-($C_1$-$C_4$ alkyl)aminocarbonyloxy group), more preferably a methylaminocarbonyloxy group or ethylaminocarbonyloxy group (mono-($C_1$-$C_2$ alkyl)aminocarbonyloxy group), and still more preferably a methylaminocarbonyloxy group.

The "di-($C_1$-$C_6$ allcyl)aminocarbonyloxy group" in the present invention is a group in which a carbonyl group with one aforementioned "di-($C_1$-$C_6$ alkyl)amino group" bonded thereto is bonded to an oxygen atom. Examples of such a group include a dimethylaminocarbonyloxy group, diethylaminocarbonyloxy group, dipropylaminocarbonyloxy group, diisopropylaminocarbonyloxy group, dibutylaminocarbonyloxy group, N-ethyl-N-methylaminocarbonyloxy group, N-methyl-N-propylaminocarbonyloxy group, N-isopropyl-N-methylaminocarbonyloxy group, N-butyl-N-methylaminocarbonyloxy group, N-isobutyl-N-methylaminocarbonyloxy group, N-methyl-N-pentylaminocarbonyloxy group and N-ethyl-N-isopentylaminocarbonyloxy group. The group is preferably a group in which a carbonyl group with one aforementioned "di-($C_1$-$C_4$ alkyl)amino group" bonded thereto is bonded to an oxygen atom (di-($C_1$-$C_4$ alkyl)aminocarbonyloxy group), more preferably a dimethylaminocarbonyloxy group, diethylaminocarbonyloxy group or N-ethyl-N-methylaminocarbonyloxy group (di-($C_1$-$C_2$ alkyl)aminocarbonyloxy group), and still more preferably a dimethylaminocarbonyloxy group.

The "mono-$C_2$-$C_7$ alkylcarbonylamino group" in the present invention is a group in which a carbonyl group with one aforementioned "$C_1$-$C_6$ alkyl group" bonded thereto is bonded to an amino group. Examples of such a group include an acetamide group, ethylcarbonylamino group, propylcarbonylamino group, isopropylcarbonylamino group, butylcarbonylamino group, isobutylcarbonylamino group, s-butylcarbonylamino group, t-butylcarbonylamino group, pentylcarbonylamino group and 2-ethylbutylcarbonylamino group. The group is preferably a group in which a carbonyl group with one aforementioned "$C_1$-$C_4$ alkyl group" bonded thereto is bonded to an amino group (mono-$C_2$-$C_5$ alkylcarbonylamino group), more preferably an acetamide group or ethylcarbonylamino group (mono-$C_2$-$C_3$ alkylcarbonylamino group), and still more preferably an acetamide group.

The "mono-$C_1$-$C_6$ alkylsulfonylamino group" in the present invention is a group in which a sulfonyl group with one aforementioned "$C_1$-$C_6$ alkyl group" bonded thereto is bonded to an amino group. Examples of such a group include a methylsulfonylamino group, ethylsulfonylamino group, propylsulfonylamino group, isopropylsulfonylamino group, butylsulfonylamino group, t-butylsulfonylamino group and 2-ethylbutylsulfonylamino group. The group is preferably a group in which a sulfonyl group with one aforementioned "$C_1$-$C_4$ alkyl group" bonded thereto is bonded to an amino group (mono-$C_1$-$C_4$ alkylsulfonylamino group), more preferably a methylsulfonylamino group or ethylsulfonylamino group (mono-$C_1$-$C_2$ alkylsulfonylamino group), and still more preferably a methylsulfonylamino group.

The "$C_3$-$C_6$ cycloalkyl group" in the present invention is a cyclopropyl group, cyclobutyl group, cyclopentyl group or cyclohexyl group, and preferably a cyclopropyl group.

The "$C_6$-$C_{10}$ aryl group which may be substituted with one to five group(s) independently selected from Substituent Group a" in the present invention is the aforementioned "$C_6$-$C_{10}$ aryl group" which may be substituted with one to five group(s) independently selected from Substituent Group a. The group preferably represents a phenyl group substituted with one to five group(s) independently selected from Substituent Group a or a 1-naphthyl group which may be substituted with one to three group(s) independently selected from Substituent Group a, more preferably represents

a phenyl group substituted with one to five group(s) independently selected from Substituent Group e or a 1-naphthyl group which may be substituted with one to three group(s) independently selected from Substituent Group e, still more preferably represents a phenyl group substituted at the 2- and 6-positions with $C_1$-$C_3$ alkyl groups and further substituted at the 3-, 4- and/or 5-position(s) with one to three group(s) independently selected from a $C_1$-$C_3$ alkyl group, a $C_2$-$C_6$ alkynyloxy group, a $C_2$-$C_6$ alkynyloxymethyl group, a phenylmethyloxy group substituted with one to three group(s) independently selected from a chlorine atom and a methyl group and a phenylsulfonylamino group substituted with one to three group(s) independently selected from a chlorine atom and a methyl group; a 1-naphthyl group; a 1-naphthyl group substituted at the 2-, 3- or 4-position with one group selected from a halogen atom, a $C_1$-$C_6$ alkyl group, a $C_1$-$C_6$ halogenated alkyl group, a $C_1$-$C_6$ alkoxy group, a $C_2$-$C_6$ alkynyloxy group, a $C_2$-$C_7$ alkoxycarbonyl group, a phenyloxy group which may be substituted with one or two group(s) independently selected from a fluorine atom, a chlorine atom and a trifluoromethyl group, a phenylmethyloxy group which may be substituted with one or two group(s) independently selected from a fluorine atom, a chlorine atom and a trifluoromethyl group and a phenylsulfonylamino group which may be substituted with one or two group(s) independently selected from a fluorine atom, a chlorine atom and a trifluoromethyl group; a 1-naphthyl group substituted at the 2- and 3-positions or at the 2- and 4-positions with two groups independently selected from a $C_1$-$C_6$ alkyl group, a $C_1$-$C_6$ alkoxy group, a $C_2$-$C_6$ alkynyloxy group and a phenylmethyloxy group substituted with one group selected from a fluorine atom and a chlorine atom; or a 1-naphthyl group substituted at the 2-, 3- and 4-positions with three groups independently selected from a $C_1$-$C_6$ alkyl group, a $C_1$-$C_6$ alkoxy group and a $C_2$-$C_6$ alkynyloxy group, particularly preferably represents a phenyl group substituted at the 5- and 6-positions with methyl groups and further substituted at the 2- and 3-positions with $C_1$-$C_3$ alkyl groups; a phenyl group which is substituted at the 2- and 6-positions with methyl groups, is further substituted at the 4-position with one group selected from a 2-butynyloxy group, a 2-pentynyloxy group, a (2,4-dichlorophenyl)sulfonylamino group and a (2,4-dichloro-5-methylphenyl) sulfonylamino group, and may be still further substituted at the 3-position with a methyl group; a 1-naphthyl group; a 1-naphthyl group substituted at the 2- or 3-position with one group selected from a methyl group and an ethyl group; a 1-naphthyl group substituted at the 4-position with one group selected from a fluorine atom, a 4-chlorophenyloxy group, a 3-chlorophenyloxy group, a 2,4-dichlorophenyloxy group, a phenylmethyloxy group, a 4-fluorophenylmethyloxy group, a 3-fluorophenylmethyloxy group, a 3-chlorophenylmethyloxy group and a 2,4-difluorophenylmethyloxy group; a 1-naphthyl group substituted at the 2- and 4-positions with two groups independently selected from a methyl group and an ethyl group; or a 1-naphthyl group which is substituted at the 2- and 3-positions with two groups independently selected from a methyl group and an ethyl group and may be further substituted at the 4-position with a methyl group, and most preferably represents a 1-naphthyl group substituted at the 2- and/or 3-position(s) with one or two group(s) independently selected from a methyl group and an ethyl group.

The "$C_6$-$C_{10}$ aryl group which may be substituted with one to five group(s) independently selected from Substituent Group a" in the present invention also preferably represents a 1-naphthyl group substituted with one to five group(s) independently selected from a fluorine atom, a chlorine atom, a bromine atom, a methyl group and an ethyl group, more preferably represents a 1-naphthyl group substituted at the 2- and/or 3-position(s) with one or two group (s) independently selected from a methyl group and an ethyl group and further substituted at the 4-, 5-, 6-, 7- and/or 8-position(s) with one to three group(s) independently selected from a fluorine atom and a chlorine atom, and still more preferably represents a 1-naphthyl group substituted at the 2-position with a methyl group or an ethyl group and further substituted at the 4-, 5-, 6-, 7- and/or 8-position(s) with one to three group(s) independently selected from a fluorine atom and a chlorine atom.

The "heterocyclic group which may be substituted with one to five group(s) independently selected from Substituent Group b" in the present invention is the aforementioned "heterocyclic group" which may be substituted with one to five group(s) independently selected from Substituent Group b. The group preferably represents a pyridyl group, 1,3-benzodioxolanyl group or 1,4-benzodioxanyl group which may be substituted with one to three group(s) independently selected from Substituent Group b.

The "phenyl group fused with $C_3$-$C_6$ cycloalkane which may be substituted with one to three group(s) independently selected from Substituent Group b" in the present invention is the aforementioned "phenyl group fused with $C_3$-$C_6$ cycloalkane" which may be substituted with one to three group(s) independently selected from Substituent Group b. The group preferably represents a 5,6,7,8-tetrahydro-1-naphthyl group or 2,3-dihydro-1H-indan-4-yl group which may be substituted with one to three group(s) independently selected from Substituent Group b.

The "$C_1$-$C_6$ alkyl group substituted with one group selected from Substituent Group c" in the present invention is the aforementioned "$C_1$-$C_6$ alkyl group" substituted with one group selected from Substituent Group c. The group is preferably a cyclopropylmethyl group.

The "phenyloxy group which may be substituted with one to five group(s) independently selected from Substituent Group d" in the present invention is a phenyloxy group which may be substituted with one to five group(s) independently selected from Substituent Group d. The group is preferably a phenyloxy group which may be substituted with one to three group(s) independently selected from a fluorine atom, a chlorine atom, a methyl group and a trifluoromethyl group, more preferably a phenyloxy group which may be substituted with one or two group(s) independently

selected from a fluorine atom, a chlorine atom and a trifluoromethyl group, and still more preferably a 4-chlorophenyloxy group, 3-chlorophenyloxy group or 2,4-dichlorophenyloxy group.

The "phenylthio group which may be substituted with one to five group(s) independently selected from Substituent Group d" in the present invention is a phenylthio group which may be substituted with one to five group(s) independently selected from Substituent Group d. The group is preferably a phenylthio group which may be substituted with one to three group(s) independently selected from a fluorine atom, a chlorine atom, a methyl group and a trifluoromethyl group.

The "phenyloxymethyl group which may be substituted with one to five group(s) independently selected from Substituent Group d" in the present invention is a phenyloxymethyl group which may be substituted with one to five group(s) independently selected from Substituent Group d. The group is preferably a phenyloxymethyl group which may be substituted with one to three group(s) independently selected from a fluorine atom, a chlorine atom, a methyl group and a trifluoromethyl group.

The "phenylmethyloxy group which may be substituted with one to five group(s) independently selected from Substituent Group d" in the present invention is a phenylmethyloxy group which may be substituted with one to five group(s) independently selected from Substituent Group d. The group is preferably a phenylmethyloxy group which may be substituted with one to three group(s) independently selected from a fluorine atom, a chlorine atom, a methyl group and a trifluoromethyl group, more preferably a phenylmethyloxy group substituted with one to three group(s) independently selected from a chlorine atom and a methyl group, a phenylmethyloxy group which may be substituted with one or two group(s) independently selected from a fluorine atom, a chlorine atom and a trifluoromethyl group or a phenylmethyloxy group substituted with one group selected from a fluorine atom and a chlorine atom, and still more preferably a phenylmethyloxy group, 4-fluorophenylmethyloxy group, 3-fluorophenylmethyloxy group, 3-chlorophenylmethyloxy group or 2,4-difluorophenylmethyloxy group.

The "phenylthiomethyl group which may be substituted with one to five group(s) independently selected from Substituent Group d" in the present invention is a phenylthiomethyl group which may be substituted with one to five group(s) independently selected from Substituent Group d. The group is preferably a phenylthiomethyl group which may be substituted with one to three group(s) independently selected from a fluorine atom, a chlorine atom, a methyl group and a trifluoromethyl group.

The "phenyloxycarbonyl group which may be substituted with one to five group(s) independently selected from Substituent Group d" in the present invention is a phenyloxycarbonyl group which may be substituted with one to five group(s) independently selected from Substituent Group d. The group is preferably a phenyloxycarbonyl group which may be substituted with one to three group(s) independently selected from a fluorine atom, a chlorine atom, a methyl group and a trifluoromethyl group.

The "phenylcarbonylamino group which may be substituted with one to five group(s) independently selected from Substituent Group d" in the present invention is a phenylcarbonylamino group which may be substituted with one to five group(s) independently selected from Substituent Group d. The group is preferably a phenylcarbonylamino group which may be substituted with one to three group(s) independently selected from a fluorine atom, a chlorine atom, a methyl group and a trifluoromethyl group.

The "phenylsulfonylamino group which may be substituted with one to five group(s) independently selected from Substituent Group d" in the present invention is a phenylsulfonylamino group which may be substituted with one to five group(s) independently selected from Substituent Group d. The group is preferably a phenylsulfonylamino group which may be substituted with one to three group(s) independently selected from a fluorine atom, a chlorine atom, a methyl group and a trifluoromethyl group, more preferably a phenylsulfonylamino group substituted with one to three group(s) independently selected from a chlorine atom and a methyl group or a phenylsulfonylamino group which may be substituted with one or two group(s) independently selected from a fluorine atom, a chlorine atom and a trifluoromethyl group, and still more preferably a (2,4-dichlorophenyl)sulfonylamino group or (2,4-dichloro-5-methylphenyl)sulfonylamino group.

The "phenylaminocarbonyloxy group which may be substituted with one to five group(s) independently selected from Substituent Group d" in the present invention is a phenylaminocarbonyloxy group which may be substituted with one to five group(s) independently selected from Substituent Group d. The group is preferably a phenylaminocarbonyloxy group which may be substituted with one to three group(s) independently selected from a fluorine atom, a chlorine atom, a methyl group and a trifluoromethyl group.

The "phenyloxycarbonylamino group which may be substituted with one to five group(s) independently selected from Substituent Group d" in the present invention is a phenyloxycarbonylamino group which may be substituted with one to five group(s) independently selected from Substituent Group d. The group is preferably a phenyloxycarbonylamino group which may be substituted with one to three group(s) independently selected from a fluorine atom, a chlorine atom, a methyl group and a trifluoromethyl group.

The "phenylaminocarbonylamino group which may be substituted with one to five group(s) independently selected from Substituent Group d" in the present invention is a phenylaminocarbonylamino group which may be substituted with one to five group(s) independently selected from Substituent Group d. The group is preferably a phenylaminocarbonylamino group which may be substituted with one to three group(s) independently selected from a fluorine atom, a chlorine atom, a methyl group and a trifluoromethyl group.

The "$C_1$-$C_6$ alkyl group substituted with one carboxyl group" in the present invention is the aforementioned "$C_1$-$C_6$ alkyl group" substituted with one carboxyl group. The group is preferably a 2-carboxyethyl group.

The "$C_1$-$C_6$ alkyl group substituted with one $C_2$-$C_7$ alkoxycarboxyl group" in the present invention is the aforementioned "$C_1$-$C_6$ alkyl group" substituted with one aforementioned "$C_2$-$C_7$ alkoxycarboxyl group". The group is preferably a 2-(methoxycarbonyl)ethyl group.

The "$C_1$-$C_6$ alkoxy group substituted with one $C_2$-$C_7$ alkoxycarboxyl group" in the present invention is the aforementioned "$C_1$-$C_6$ alkoxy group" substituted with one aforementioned "$C_2$-$C_7$ alkoxycarboxyl group". The group is preferably a 2-(methoxycarbonyl)ethoxy group.

The "$C_1$-$C_6$ alkoxy group substituted with one di-($C_1$-$C_6$ alkyl)aminocarbonyl group" in the present invention is the aforementioned "$C_1$-$C_6$ alkoxy group" substituted with one aforementioned "di-($C_1$-$C_6$ alkyl)aminocarbonyl group". The group is preferably a 2-(dimethylaminocarbonyl)ethoxy group.

In the present invention, the general formula (I) is preferably the general formula (Ia).

In the present invention, X is preferably an oxygen atom or a group represented by the formula =N-O-$R^5$ (wherein $R^5$ is preferably a methyl group, an ethyl group, a propyl group, a 2-propenyl group or a cyclopropylmethyl group), and X is more preferably an oxygen atom.

In the present invention, $R^1$ is preferably a hydrogen atom.

In the present invention, $R^2$ is preferably a hydrogen atom, a methyl group, an ethyl group or a difluoromethyl group, and $R^2$ is more preferably a methyl group.

In the present invention, $R^3$ is preferably a hydrogen atom.

In the present invention, $R^4$ is preferably a phenyl group substituted with one to five group(s) independently selected from Substituent Group a or a 1-naphthyl group which may be substituted with one to three group(s) independently selected from Substituent Group a, $R^4$ is more preferably a phenyl group substituted with one to five group(s) independently selected from Substituent Group e or a 1-naphthyl group which may be substituted with one to three group(s) independently selected from Substituent Group e, $R^4$ is still more preferably a phenyl group substituted at the 2- and 6-positions with $C_1$-$C_3$ alkyl groups and further substituted at the 3-, 4- and/or 5-position(s) with one to three group(s) independently selected from a $C_1$-$C_3$ alkyl group, a $C_2$-$C_6$ alkynyloxy group, a $C_2$-$C_6$ alkynyloxymethyl group, a phenylmethyloxy group substituted with one to three group(s) independently selected from a chlorine atom and a methyl group and a phenylsulfonylamino group substituted with one to three group(s) independently selected from a chlorine atom and a methyl group; a 1-naphthyl group; a 1-naphthyl group substituted at the 2-, 3- or 4-position with one group selected from a halogen atom, a $C_1$-$C_6$ alkyl group, a $C_1$-$C_6$ halogenated alkyl group, a $C_1$-$C_6$ alkoxy group, a $C_2$-$C_6$ alkynyloxy group, a $C_2$-$C_7$ alkoxycarbonyl group, a phenyloxy group which may be substituted with one or two group(s) independently selected from a fluorine atom, a chlorine atom and a trifluoromethyl group, a phenylmethyloxy group which may be substituted with one or two group(s) independently selected from a fluorine atom, a chlorine atom and a trifluoromethyl group and a phenylsulfonylamino group which may be substituted with one or two group(s) independently selected from a fluorine atom, a chlorine atom and a trifluoromethyl group; a 1-naphthyl group substituted at the 2- and 3-positions or at the 2- and 4-positions with two groups independently selected from a $C_1$-$C_6$ alkyl group, a $C_1$-$C_6$ alkoxy group, a $C_2$-$C_6$ alkynyloxy group and a phenylmethyloxy group substituted with one group selected from a fluorine atom and a chlorine atom; or a 1-naphthyl group substituted at the 2-, 3- and 4-positions with three groups independently selected from a $C_1$-$C_6$ alkyl group, a $C_1$-$C_6$ alkoxy group and a $C_2$-$C_6$ alkynyloxy group, $R^4$ is particularly preferably a phenyl group substituted at the 5- and 6-positions with methyl groups and further substituted at the 2- and 3-positions with $C_1$-$C_3$ alkyl groups; a phenyl group which is substituted at the 2- and 6-positions with methyl groups, is further substituted at the 4-position with one group selected from a 2-butynyloxy group, a 2-pentynyloxy group, a (2,4-dichlorophenyl)sulfonylamino group and a (2,4-dichloro-5-methylphenyl)sulfonylamino group, and may be still further substituted at the 3-position with a methyl group; a 1-naphthyl group; a 1-naphthyl group substituted at the 2- or 3-position with one group selected from a methyl group and an ethyl group; a 1-naphthyl group substituted at the 4-position with one group selected from a fluorine atom, a 4-chlorophenyloxy group, a 3-chlorophenyloxy group, a 2,4-dichlorophenyloxy group, a phenylmethyloxy group, a 4-fluorophenylmethyloxy group, a 3-fluorophenylmethyloxy group, a 3-chlorophenylmethyloxy group and a 2,4-difluorophenylmethyloxy group; a 1-naphthyl group substituted at the 2- and 4-positions with two groups independently selected from a methyl group and an ethyl group; or a 1-naphthyl group which is substituted at the 2- and 3-positions with two groups independently selected from a methyl group and an ethyl group and may be further substituted at the 4-position with a methyl group, and $R^4$ is most preferably a 1-naphthyl group substituted at the 2- and/or 3-position(s) with one or two group(s) independently selected from a methyl group and an ethyl group.

In the present invention, $R^4$ is also preferably a 1-naphthyl group substituted with one to five group(s) independently selected from a fluorine atom, a chlorine atom, a bromine atom, a methyl group and an ethyl group, $R^4$ is

more preferably a 1-naphthyl group substituted at the 2- and/or 3-position(s) with one or two group(s) independently selected from a methyl group and an ethyl group and substituted at the 4-, 5-, 6-, 7- and/or 8-position(s) with one to three group(s) independently selected from a fluorine atom and a chlorine atom, and $R^4$ is still more preferably a 1-naphthyl group substituted at the 2-position with a methyl group or an ethyl group and substituted at the 4-, 5-, 6-, 7- and/or 8-position(s) with one to three group(s) independently selected from a fluorine atom and a chlorine atom.

In the present invention, n is preferably 1.

The cercosporamide derivative having the general formula (I) according to the present invention, a pharmacologically acceptable salt thereof or an ester thereof includes all isomers (such as a keto-enol isomer, a diastereomer, an optical isomer and a rotamer).

The cercosporamide derivative having the general formula (I) according to the present invention, a pharmacologically acceptable salt thereof or an ester thereof has an asymmetric carbon atom in its molecule and therefore has various isomers. These isomers and isomer mixtures of the compound of the present invention are all represented by a single formula, specifically, the general formula (I). Accordingly, the present invention includes all of these isomers and mixtures of these isomers at any ratio.

The cercosporamide derivative having the general formula (I) according to the present invention, a pharmacologically acceptable salt thereof or an ester thereof has a double bond in its molecule and therefore has various geometric isomers. These isomers and isomer mixtures of the compound of the present invention are all represented by a single formula, specifically, the general formula (I). Accordingly, the present invention includes all of these isomers and mixtures of these isomers at any ratio.

The aforementioned stereoisomer can be obtained by synthesizing the compound of the present invention using a stereospecific raw material compound or using an asymmetric synthesis or asymmetric induction technique or by isolating the synthesized compound of the present invention by a common optical resolution or separation method if desired.

A cercosporamide derivative having the general formula (Ia), a pharmacologically acceptable salt thereof or an ester thereof is more preferable than a cercosporamide derivative having the general formula (Ib), a pharmacologically acceptable salt thereof or an ester thereof.

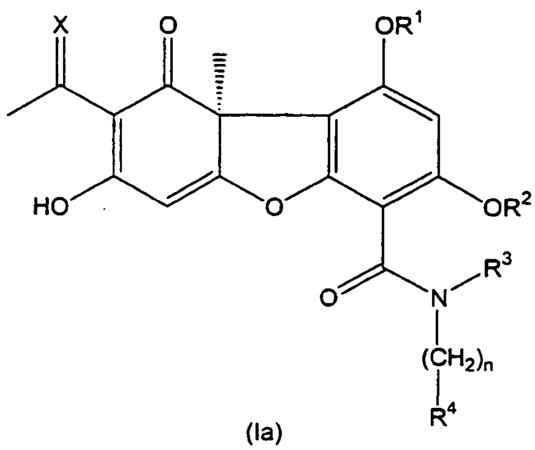

(Ia)

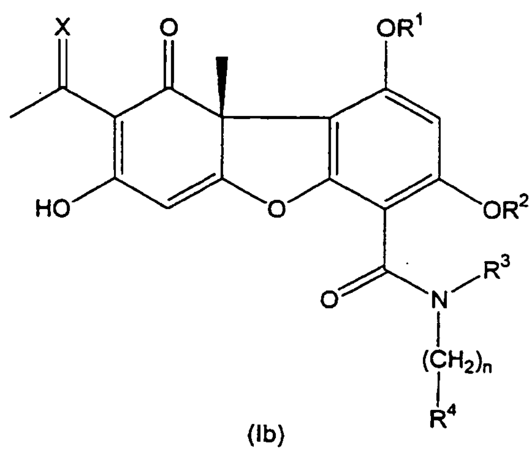

(Ib)

The "pharmacologically acceptable salt thereof" represents a salt that can be obtained by reacting a cercosporamide derivative having the general formula (I) having a basic group such as an amino group with an acid or reacting a derivative having an acidic group such as a carboxyl group with a base.

Preferable examples of the salt based on a basic group include hydrohalides such as hydrofluorides, hydrochlorides, hydrobromides and hydroiodides; inorganic acid salts such as nitrates, perchlorates, sulfates and phosphates; alkyl sulfonates such as methanesulfonates and ethanesulfonates; halogenated alkyl sulfonates such as trifluoromethanesulfonates; aryl sulfonates such as benzenesulfonates and p-toluenesulfonates; and organic acid salts such as acetates, malates, fumarates, succinates, citrates, ascorbates, tartrates, oxalates and maleates. Hydrohalides, inorganic acid salts or organic acid salts are most preferable.

On the other hand, preferable examples of the salt based on an acidic group include alkaline metal salts such as sodium salts, potassium salts and lithium salts; alkaline earth metal salts such as calcium salts and magnesium salts; and metal salts such as aluminum salts and iron salts. Alkaline metal salts are most preferable.

The cercosporamide derivative having the general formula (I) according to the present invention, a pharmacologically acceptable salt thereof or an ester thereof may absorb moisture or adsorb water to form a hydrate when left

to stand in the air or in a purification or preparation step, and such a hydrate is also included in the salt of the present invention.

The cercosporamide derivative having the general formula (I) according to the present invention, a pharmacologically acceptable salt thereof or an ester thereof may absorb some other solvent to form a solvate, and such a solvate is also included in the salt of the present invention.

The aforementioned "ester" refers to an ester of the compound (I) of the present invention having a hydroxy group which can be esterified. Such an ester may be an "ester of a hydroxy group" which refers to an ester in which an ester residue is a "general protecting group" or a "protecting group that can be cleaved in vivo by a biological method such as hydrolysis". The ester preferably refers to an ester in which an ester residue is a "protecting group that can be cleaved in vivo by a biological method such as hydrolysis".

The "general protecting group" refers to a protecting group that can be cleaved by a chemical method such as hydrogenolysis, hydrolysis, electrolysis or photolysis.

Preferable examples of the "general protecting group" for the "ester of a hydroxy group" include "alkylcarbonyl groups" such as formyl group, alkanoyl groups such as acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, icosanoyl and henicosanoyl, halogenated alkylcarbonyl groups such as chloroacetyl, dichloroacetyl, trichloroacetyl and trifluoroacetyl, alkoxyalkylcarbonyl groups such as methoxyacetyl, and unsaturated alkylcarbonyl groups such as acryloyl, propioloyl, methacryloyl, crotonoyl, isocrotonoyl and (E)-2-methyl-2-butenoyl (preferably the aforementioned "$C_2$-$C_7$ alkylcarbonyl groups"); "arylcarbonyl groups" such as arylcarbonyl groups such as benzoyl, $\alpha$-naphthoyl and $\beta$-naphthoyl, halogenated arylcarbonyl groups such as 2-bromobenzoyl and 4-chlorobenzoyl, $C_1$-$C_6$ alkylated arylcarbonyl groups such as 2,4,6-trimethylbenzoyl and 4-toluoyl, $C_1$-$C_6$ alkoxylated arylcarbonyl groups such as 4-anisoyl, nitrated arylcarbonyl groups such as 4-nitrobenzoyl and 2-nitrobenzoyl, $C_2$-$C_7$ alkoxycarbonylated arylcarbonyl groups such as 2-(methoxycarbonyl)benzoyl and arylated arylcarbonyl groups such as 4-phenylbenzoyl; "alkoxycarbonyl groups" such as the aforementioned "$C_2$-$C_7$ alkoxycarbonyl groups" and $C_2$-$C_7$ alkoxycarbonyl groups substituted with halogen or a tri-($C_1$-$C_6$ alkyl)silyl group such as 2,2,2-trichloroethoxycarbonyl and 2-trimethylsilylethoxycarbonyl; "tetrahydropyranyl or tetrahydrothiopyranyl groups" such as tetrahydropyran-2-yl, 3-bromotetrahydropyran-2-yl, 4-methoxytetrahydropyran-4-yl, tetrahydrothioppan-2-yl and 4-methoxytetrahydrothiopyran-4-yl; "tetrahydrofuranyl or tetrahydrothiofuranyl groups" such as tetrahydrofuran-2-yl and tetrahydrothiofuran-2-yl; "silyl groups" such as tri-($C_1$-$C_6$ alkyl)silyl groups such as trimethylsilyl, triethylsilyl, isopropyldimethylsilyl, t-butyldimethylsilyl, methyldiisopropylsilyl, methyl-di-t-butylsilyl and triisopropylsilyl and ($C_1$-$C_6$ alkyl)diarylsilyl or di-($C_1$-$C_6$ alkyl)arylsilyl groups such as diphenylmethylsilyl, diphenylbutylsilyl, diphenylisopropylsilyl and phenyldiisopropylsilyl; "alkoxymethyl groups" such as $C_1$-$C_6$ alkoxymethyl groups such as methoxymethyl, 1,1-dimethyl-1-methoxymethyl, ethoxymethyl, propoxymethyl, isopropoxymethyl, butoxymethyl and t-butoxymethyl, $C_1$-$C_6$ alkoxy $C_1$-$C_6$ alkoxymethyl groups such as 2-methoxyethoxymethyl and $C_1$-$C_6$ halogenated alkoxymethyl groups such as 2,2,2-trichloroethoxymethyl and bis(2-chloroethoxy)methyl; "substituted ethyl groups" such as $C_1$-$C_6$ alkoxyethyl groups such as 1-ethoxyethyl and 1-(isopropoxy)ethyl and halogenated ethyl groups such as 2,2,2-trichloroethyl; "aralkyl groups" such as $C_1$-$C_6$ alkyl groups substituted with one to three aryl group(s) such as benzyl, $\alpha$-naphthylmethyl, $\beta$-naphthylmethyl, diphenylmethyl, triphenylmethyl, $\alpha$-naphthyldiphenylmethyl and 9-anthrylmethyl and $C_1$-$C_6$ alkyl groups substituted with one to three aryl group(s) having an aryl ring substituted with $C_1$-$C_6$ alkyl, $C_1$-$C_6$ alkoxy, nitro, halogen or cyano such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; "alkenyloxycarbonyl groups" such as vinyloxycarbonyl and allyloxycarbonyl; and "aralkyloxycarbonyl groups" having an aryl ring which may be substituted with 1 or 2 $C_1$-$C_6$ alkoxy or nitro group(s) such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl. Alkylcarbonyl groups, silyl groups or aralkyl groups are preferable.

The "protecting group that can be cleaved in vivo by a biological method such as hydrolysis" refers to a protecting group cleaved in the human body by a biological method such as hydrolysis to produce a free acid or a salt thereof. Whether or not the compound is such a derivative can be determined by administering the compound to an experimental animal such as a rat or mouse by intravenous injection and then examining the body fluid of the animal to detect the original compound or a pharmacologically acceptable salt thereof.

Preferable examples of the "protecting group that can be cleaved in vivo by a biological method such as hydrolysis" for the "ester of a hydroxy group" include 1-(acyloxy) "$C_1$-$C_6$ alkyl groups" such as 1-formyloxy "$C_1$-$C_6$ alkyl groups" or 1-("alkylcarbonyl"oxy) "$C_1$-$C_6$ alkyl groups" such as formyloxymethyl, acetoxymethyl, propionyloxymethyl, butyryloxymethyl, pivaloyloxymethyl, valeryloxy methyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl, 1-("cycloalkyl"carbonyloxy) "$C_1$-$C_6$ alkyl groups" such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl and 1-cyclohexylcarbonyloxybutyl and 1-("arylcarbonyl"oxy) "$C_1$-$C_6$ alkyl groups" such as benzoyloxymethyl; "carbonyloxy $C_1$-$C_6$ alkyl groups" such as ($C_2$-$C_7$ alkoxycarbonyloxy)alkyl groups such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 1-(methoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl and oxodioxolenylmethyl groups such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl; "phthalidyl groups" such as phthalidyl, dimethylphthalidyl, and dimethoxyphthalidyl; "alkylcarbonyl groups"; "arylcarbonyl groups" such as arylcarbonyl groups such as benzoyl, α-naphthoyl and β-naphthoyl, halogenated arylcarbonyl groups such as 2-bromobenzoyl and 4-chlorobenzoyl, $C_1$-$C_6$ alkylated arylcarbonyl groups such as 2,4,6-trimethylbenzoyl and 4-toluoyl, $C_1$-$C_6$ alkoxylated arylcarbonyl groups such as 4-anisoyl, nitrated arylcarbonyl groups such as 4-nitrobenzoyl and 2-nitrobenzoyl, $C_2$-$C_7$ alkoxycarbonylated arylcarbonyl groups such as 2-(methoxycarbonyl)benzoyl and arylated arylcarbonyl groups such as 4-phenylbenzoyl; "succinic acid half ester salt residues"; "phosphoric acid ester salt residues"; "ester-forming residues of amino acids"; a "carbamoyl group"; a "carbamoyl group substituted with 1 or 2 $C_1$-$C_6$ alkyl groups"; and "1-(acyloxy)alkyloxycarbonyl groups" such as pivaloyloxymethyloxycarbonyl.

Methods for selecting and producing the "ester of a hydroxy group" compound having a "protecting group that can be cleaved in vivo by a biological method such as hydrolysis" are described in Design of Prodrugs, Elsevier, Amsterdam 1985 and Iyakuhin No Kaihatsu (Development of Drugs), Vol. 7, Bunshi Sekkei (Molecular Design), Hirokawa Publishing Company 1990, for example. Examples of the present compound include acetates, pivaloates and benzoates.

The cercosporamide derivative having the general formula (I) according to the present invention, a pharmacologically acceptable salt thereof or an ester thereof is preferably the cercosporamide derivative having the general formula (I) according to the present invention or a pharmacologically acceptable salt thereof.

Positions in the compound having the general formula (I) according to the present invention are numbered as follows.

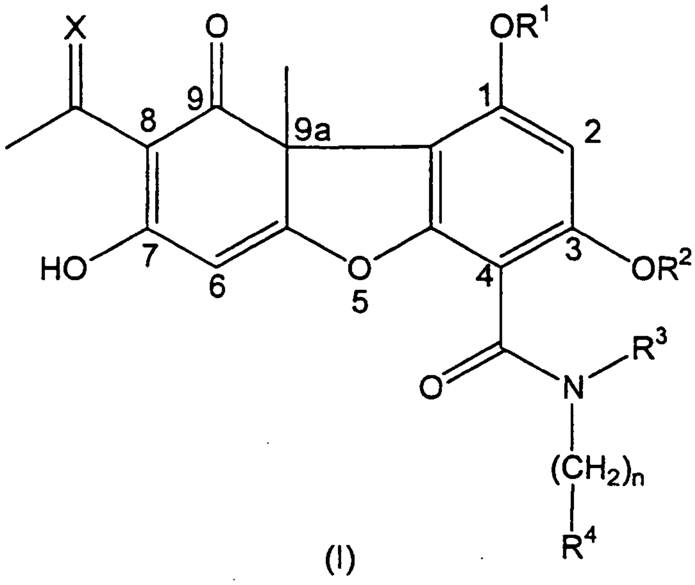

(I)

Specific examples of the compound having the general formula (I) according to the present invention include the compounds shown in the following Tables 1 to 3; however, the present invention is not limited to these groups.

The abbreviations in the following Tables 1 to 3 are as follows. Specifically, Me represents a methyl group,
Et represents an ethyl group,
nPr represents an n-propyl group,
iPr represents an isopropyl group,
cPr represents a cyclopropyl group,
nBu represents an n-butyl group,
Ph represents a phenyl group,
1-Naph represents a 1-naphthyl group,
4-F-$C_6H_4$ represents a 4-fluorophenyl group,
2,3-di-F-$C_6H_3$ represents a 2,3-difluorophenyl group,
2,4-di-Me-1-Naph represents a 2,4-dimethyl-1-naphthyl group,
2-Et-4-Me-1-Naph represents a 2-ethyl-4-methyl-1-naphthyl group,
3-F-2-Me-1-Naph represents a 3-fluoro-2-methyl-1-naphthyl group,
4-$NHSO_2$(2,4-di-Cl-$C_6H_3$)-$C_6H_4$ represents a 4-(2,4-dichlorophenyl)sulfonylaminophenyl group,
2,3,4-tri-F-$C_6H_2$ represents a 2,3,4-trifluorophenyl group,
3-Cl-2,6-di-F-$C_6H_2$ represents a 3-chloro-2,6-difluorophenyl group,
2,3,4,6-tetra-F-$C_6H$ represents a 2,3,4,6-tetrafluorophenyl group,
4-$OCH_2$(2,4-di-Cl-$C_6H_3$)-2,3,6-tri-Me-$C_6H$ represents a 4-(2,4-dichlorophenyl)methyloxy-2,3,6-trimethylphenyl group,
2,3,4,5,6-pent-F-$C_6$ represents a 2,3,4,5,6-pentafluorophenyl group,
2-(CH=$CH_2$)-1-Naph represents a 2-vinyl-1-naphthyl group,
CC represents a carbon-carbon triple bond,
4-Py represents a 4-pyridyl group,
Ph(A) represents a 5,6,7,8-tetrahydro-1-naphthyl group,
Ph(B) represents a 2,3-dihydro-1H-indan-4-yl group,
Ph(C) represents a 2,3-dihydro-1,4-benzodioxin-5-yl group, and
Ph(D) represents a 1,3-benzodioxolan-4-yl group.

(Table 1)

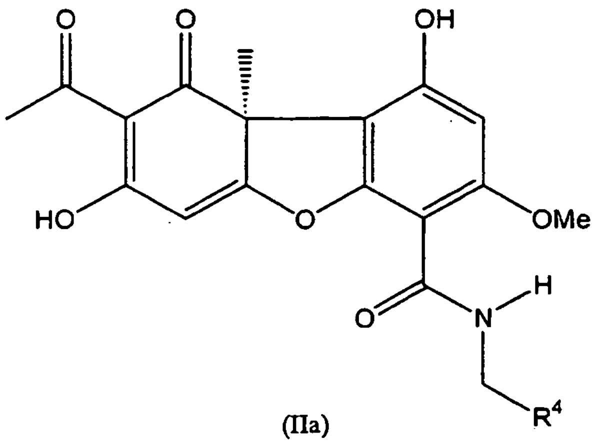

(IIa)

| Compound No. | $R^4$ |
|---|---|
| 1-1 | $C_6H_5$ |
| 1-2 | 4-F-$C_6H_4$ |
| 1-3 | 3-F-$C_6H_4$ |
| 1-4 | 2-F-$C_6H_4$ |
| 1-5 | 4-Cl-$C_6H_4$ |
| 1-6 | 3-Cl-$C_6H_4$ |
| 1-7 | 2-Cl-$C_6H_4$ |
| 1-8 | 4-Br-$C_6H_4$ |
| 1-9 | 3-Br-$C_6H_4$ |
| 1-10 | 2-Br-$C_6H_4$ |
| 1-11 | 4-Me-$C_6H_4$ |
| 1-12 | 3-Me-$C_6H_4$ |
| 1-13 | 2-Me-$C_6H_4$ |
| 1-14 | 4-Et-$C_6H_4$ |
| 1-15 | 3-Et-$C_6H_4$ |
| 1-16 | 2-Et-$C_6H_4$ |
| 1-17 | 4-OMe-$C_6H_4$ |
| 1-18 | 3-OMe-$C_6H_4$ |
| 1-19 | 2-OMe-$C_6H_4$ |
| 1-20 | 4-OEt-$C_6H_4$ |
| 1-21 | 3-OEt-$C_6H_4$ |
| 1-22 | 2-OEt-$C_6H_4$ |
| 1-23 | 4-OPh-$C_6H_4$ |
| 1-24 | 3-OPh-$C_6H_4$ |
| 1-25 | 2-OPh-$C_6H_4$ |
| 1-26 | 4-$NHSO_2$(2,4-di-Cl-$C_6H_3$)-$C_6H_4$ |
| 1-27 | 2,3-di-F-$C_6H_3$ |
| 1-28 | 2,4-di-F-$C_6H_3$ |
| 1-29 | 2,5-di-F-$C_6H_3$ |
| 1-30 | 2,6-di-F-$C_6H_3$ |
| 1-31 | 3,4-di-F-$C_6H_3$ |
| 1-32 | 3,5-di-F-$C_6H_3$ |
| 1-33 | 2,3-di-Cl-$C_6H_3$ |
| 1-34 | 2,4-di-Cl-$C_6H_3$ |

(continued)

| Compound No. | $R^4$ |
|---|---|
| 1-35 | 2,5-di-Cl-$C_6H_3$ |
| 1-36 | 2,6-di-Cl-$C_6H_3$ |
| 1-37 | 3,4-di-Cl-$C_6H_3$ |
| 1-38 | 3,5-di-Cl-$C_6H_3$ |
| 1-39 | 2,3-di-Me-$C_6H_3$ |
| 1-40 | 2,4-di-Me-$C_6H_3$ |
| 1-41 | 2,5-di-Me-$C_6H_3$ |
| 1-42 | 2,6-di-Me-$C_6H_3$ |
| 1-43 | 3,4-di-Me-$C_6H_3$ |
| 1-44 | 3,5-di-Me-$C_6H_3$ |
| 1-45 | 2-Cl-3-F-$C_6H_3$ |
| 1-46 | 3-Cl-2-F-$C_6H_3$ |
| 1-47 | 2-Br-3-F-$C_6H_3$ |
| 1-48 | 3-Br-2-F-$C_6H_3$ |
| 1-49 | 2-F-3-Me-$C_6H_3$ |
| 1-50 | 3-F-2-Me-$C_6H_3$ |
| 1-51 | 2-Cl-3-Me-$C_6H_3$ |
| 1-52 | 3-Cl-2-Me-$C_6H_3$ |
| 1-53 | 2-I-3-Me-$C_6H_3$ |
| 1-54 | 3-I-2-Me-$C_6H_3$ |
| 1-55 | 2-F-5-$NO_2$-$C_6H_3$ |
| 1-56 | 5-F-2-$NO_2$-$C_6H_3$ |
| 1-57 | 2,3,4-tri-F-$C_6H_2$ |
| 1-58 | 2,3,5-tri-F-$C_6H_2$ |
| 1-59 | 2,3,6-tri-F-$C_6H_2$ |
| 1-60 | 2,4,5-tri-F-$C_6H_2$ |
| 1-61 | 2,4,6-tri-F-$C_6H_2$ |
| 1-62 | 2,3,4-tri-Me-$C_6H_2$ |
| 1-63 | 2,3,5-tri-Me-$C_6H_2$ |
| 1-64 | 2,3,6-tri-Me-$C_6H_2$ |
| 1-65 | 2,4,5-tri-Me-$C_6H_2$ |
| 1-66 | 2,4,6-tri-Me-$C_6H_2$ |
| 1-67 | 2,3,6-tri-Et-$C_6H_2$ |
| 1-68 | 2,4,6-tri-Et-$C_6H_2$ |
| 1-69 | 3-Cl-2,6-di-F-$C_6H_2$ |
| 1-70 | 2,3-di-F-4-Me-$C_6H_2$ |
| 1-71 | 2,6-di-Me-4-F-$C_6H_2$ |
| 1-72 | 6-Cl-2-F-3-Me-$C_6H_2$ |
| 1-73 | 2,3-di-Me-4-OMe-$C_6H_2$ |
| 1-74 | 2,5-di-Me-4-OMe-$C_6H_2$ |
| 1-75 | 2,6-di-Me-4-OnPr-$C_6H_2$ |
| 1-76 | 2,6-di-Me-4-($OCH_2$-CC-Me)-$C_6H_2$ |
| 1-77 | 2,6-di-Me-4-($OCH_2$-CC-Et)-$C_6H_2$ |
| 1-78 | 2,6-di-Me-4-$NHSO_2$Me-$C_6H_2$ |
| 1-79 | 2,6-di-Me-4-$NHSO_2$Et-$C_6H_2$ |
| 1-80 | 2,6-di-Me-4-$NHSO_2$nPr-$C_6H_2$ |
| 1-81 | 4-$OCH_2$(2,4-di-Cl-$C_6H_3$)-2,6-di-Me-$C_6H_2$ |
| 1-82 | 4-$OCH_2$(2,4-di-F-$C_6H_3$)-2,6-di-Me-$C_6H_2$ |
| 1-83 | 2,6-di-Me-4-NHCOPh-$C_6H_2$ |
| 1-84 | 4-NHCO(4-Cl-$C_6H_4$)-2,6-di-Me-$C_6H_2$ |

(continued)

| Compound No. | $R^4$ |
|---|---|
| 1-85 | 4-NHCO(2,4-di-F-$C_6H_3$)-2,6-di-Me-$C_6H_2$ |
| 1-86 | 4-NHCO(2,4-di-Cl-$C_6H_3$)-2,6-di-Me-$C_6H_2$ |
| 1-87 | 2,6-di-Me-4-$NHSO_2$Ph-$C_6H_2$ |
| 1-88 | 4-$NHSO_2$(4-Cl-$C_6H_4$)-2,6-di-Me-$C_6H_2$ |
| 1-89 | 4-$NHSO_2$(3-Cl-$C_6H_4$)-2,6-di-Me-$C_6H_2$ |
| 1-90 | 4-$NHSO_2$(2-Cl-$C_6H_4$)-2,6-di-Me-$C_6H_2$ |
| 1-91 | 4-$NHSO_2$(2,4-di-F-$C_6H_3$)-2,6-di-Me-$C_6H_2$ |
| 1-92 | 4-$NHSO_2$(2,4-di-Cl-$C_6H_3$)-2,6-di-Me-$C_6H_2$ |
| 1-93 | 4-$NHSO_2$(2,4-di-Cl-5-Me-$C_6H_2$)-2,6-di-Me-$C_6H_2$ |
| 1-94 | 4-$CH_2O$(2,4-di-F-$C_6H_3$)-2,6-di-Me-$C_6H_2$ |
| 1-95 | 4-$CH_2O$(2,4-di-Cl-$C_6H_3$)-2,6-di-Me-$C_6H_2$ |
| 1-96 | 4-$CH_2O$(2,4-di-Cl-5-Me-$C_6H_2$)-2,6-di-Me-$C_6H_2$ |
| 1-97 | 4-O(2,4-di-F-$C_6H_3$)-2,6-di-Me-$C_6H_2$ |
| 1-98 | 4-O(2,4-di-Cl-$C_6H_3$)-2,6-di-Me-$C_6H_2$ |
| 1-99 | 4-S(2,4-di-F-$C_6H_3$)-2,6-di-Me-$C_6H_2$ |
| 1-100 | 4-S(2,4-di-Cl-$C_6H_3$)-2,6-di-Me-$C_6H_2$ |
| 1-101 | 4-$CH_2S$(2,4-di-F-$C_6H_3$)-2,6-di-Me-$C_6H_2$ |
| 1-102 | 4-$CH_2S$(2,4-di-Cl-$C_6H_3$)-2,6-di-Me-$C_6H_2$ |
| 1-103 | 4-$CO_2$(2,4-di-F-$C_6H_3$)-2,6-di-Me-$C_6H_2$ |
| 1-104 | 4-$CO_2$(2,4-di-Cl-$C_6H_3$)-2,6-di-Me-$C_6H_2$ |
| 1-105 | 4-O(C=O)NH(2,4-di-F-$C_6H_3$)-2,6-di-Me-$C_6H_2$ |
| 1-106 | 4-O(C=O)NH(2,4-di-Cl-$C_6H_3$)-2,6-di-Me-$C_6H_2$ |
| 1-107 | 4-$NHCO_2$(2,4-di-F-$C_6H_3$)-2,6-di-Me-$C_6H_2$ |
| 1-108 | 4-$NHCO_2$(2,4-di-Cl-$C_6H_3$)-2,6-di-Me-$C_6H_2$ |
| 1-109 | 4-NHCONH(2,4-di-F-$C_6H_3$)-2,6-di-Me-$C_6H_2$ |
| 1-110 | 4-NHCONH(2,4-di-Cl-$C_6H_3$)-2,6-di-Me-$C_6H_2$ |
| 1-111 | 2,3,4,6-tetra-F-$C_6H$ |
| 1-112 | 2,3,5,6-tetra-F-$C_6H$ |
| 1-113 | 2,3,4,6-tetra-Cl-$C_6H$ |
| 1-114 | 2,3,5,6-tetra-Cl-$C_6H$ |
| 1-115 | 2,3,4,6-tetra-Me-$C_6H$ |
| 1-116 | 2,3,5,6-tetra-Me-$C_6H$ |
| 1-117 | 2,3,4,6-tetra-Et-$C_6H$ |
| 1-118 | 2,3,5,6-tetra-Et-$C_6H$ |
| 1-119 | 2-Et-3,5,6-tri-Me-$C_6H$ |
| 1-120 | 3-Et-2,5,6-tri-Me-$C_6H$ |
| 1-121 | 2-nPr-3,5,6-tri-Me-$C_6H$ |
| 1-122 | 3-nPr-2,5,6-tri-Me-$C_6H$ |
| 1-123 | 3-nBu-2,5,6-tri-Me-$C_6H$ |
| 1-124 | 4-nBu-2,3,6-tri-Me-$C_6H$ |
| 1-125 | 2,3-di-Et-5,6-di-Me-$C_6H$ |
| 1-126 | 2,5-di-Et-3,6-di-Me-$C_6H$ |
| 1-127 | 2-Br-3,5,6-tri-Me-$C_6H$ |
| 1-128 | 3-Br-2,5,6-tri-Me-$C_6H$ |
| 1-129 | 3-$CO_2$Me-2,5,6-tri-Me-$C_6H$ |
| 1-130 | 3-$CO_2$H-2,5,6-tri-Me-$C_6H$ |
| 1-131 | 4-OH-2,3,6-tri-Me-$C_6H$ |
| 1-132 | 4-OnPr-2,3,6-tri-Me-$C_6H$ |
| 1-133 | 4-OPh-2,3,6-tri-Me-$C_6H$ |
| 1-134 | 4-($OCH_2$-CC-Me)-2,3,6-tri-Me-$C_6H$ |

(continued)

| Compound No. | $R^4$ |
|---|---|
| 1-135 | 4-(OCH$_2$-CC-Et)-2,3,6-tri-Me-$C_6H$ |
| 1-136 | 4-$CH_2$OH-2,3,6-tri-Me-$C_6H$ |
| 1-137 | 4-$CH_2$OnPr-2,3,6-tri-Me-$C_6H$ |
| 1-138 | 4-$CH_2$OPh-2,3,6-tri-Me-$C_6H$ |
| 1-139 | 4-($CH_2$O$CH_2$-CC-Me)-2,3,6-tri-Me-$C_6H$ |
| 1-140 | 4-($CH_2$O$CH_2$-CC-Et)-2,3,6-tri-Me-$C_6H$ |
| 1-141 | 4-$CO_2$Me-2,3,6-tri-Me-$C_6H$ |
| 1-142 | 4-O-(2,4-di-F-$C_6H_3$)-2,3,6-tri-Me-$C_6H$ |
| 1-143 | 4-O(2,4-di-Cl-$C_6H_3$)-2,3,6-tri-Me-$C_6H$ |
| 1-144 | 4-S(2,4-di-F-$C_6H_3$)-2,3,6-tri-Me-$C_6H$ |
| 1-145 | 4-S(2,4-di-Cl-$C_6H_3$)-2,3,6-tri-Me-$C_6H$ |
| 1-146 | 4-$CH_2$O(2,4-di-F-$C_6H_3$)-2,3,6-tri-Me-$C_6H$ |
| 1-147 | 4-$CH_2$O(2,4-di-Cl-$C_6H_3$)-2,3,6-tri-Me-$C_6H$ |
| 1-148 | 4-O$CH_2$(2,4-di-F-$C_6H_3$)-2,3,6-tri-Me-$C_6H$ |
| 1-149 | 4-O$CH_2$(2,4-di-Cl-$C_6H_3$)-2,3,6-tri-Me-$C_6H$ |
| 1-150 | 4-$CH_2$S(2,4-di-F-$C_6H_3$)-2,3,6-tri-Me-$C_6H$ |
| 1-151 | 4-$CH_2$S(2,4-di-Cl-$C_6H_3$)-2,3,6-tri-Me-$C_6H$ |
| 1-152 | 4-$CO_2$(2,4-di-F-$C_6H_3$)-2,3,6-tri-Me-$C_6H$ |
| 1-153 | 4-$CO_2$(2,4-di-Cl-$C_6H_3$)-2,3,6-tri-Me-$C_6H$ |
| 1-154 | 4-NHCO(2,4-di-F-$C_6H_3$)-2,3,6-tri-Me-$C_6H$ |
| 1-155 | 4-NHCO(2,4-di-Cl-$C_6H_3$)-2,3,6-tri-Me-$C_6H$ |
| 1-156 | 4-NH$SO_2$(2,4-di-F-$C_6H_3$)-2,3,6-tri-Me-$C_6H$ |
| 1-157 | 4-NH$SO_2$(2,4-di-Cl-$C_6H_3$)-2,3,6-tri-Me-$C_6H$ |
| 1-158 | 4-O(C=O)NH(2,4-di-F-$C_6H_3$)-2,3,6-tri-Me-$C_6H$ |
| 1-159 | 4-O(C=O)NH(2,4-di-Cl-$C_6H_3$)-2,3,6-tri-Me-$C_6H$ |
| 1-160 | 4-NH$CO_2$(2,4-di-F-$C_6H_3$)-2,3,6-tri-Me-$C_6H$ |
| 1-161 | 4-NH$CO_2$(2,4-di-Cl-$C_6H_3$)-2,3,6-tri-Me-$C_6H$ |
| 1-162 | 4-NHCONH(2,4-di-F-$C_6H_3$)-2,3,6-tri-Me-$C_6H$ |
| 1-163 | 4-NHCONH(2,4-di-Cl-$C_6H_3$)-2,3,6-tri-Me-$C_6H$ |
| 1-164 | 2,3,4,5,6-pent-F-$C_6$ |
| 1-165 | 2,3,4,5,6-pent-Cl-$C_6$ |
| 1-166 | 2,3,4,5,6-pent-Me-$C_6$ |
| 1-167 | 2,3,4,5,6-pent-Et-$C_6$ |
| 1-168 | 4-OH-2,3,5,6-tetra-Me-$C_6$ |
| 1-169 | 4-OnPr-2,3,5,6-tetra-Me-$C_6$ |
| 1-170 | 4-OPh-2,3,5,6-tetra-Me-$C_6$ |
| 1-171 | 4-O$CH_2$Ph-2,3,5,6-tetra-Me-$C_6$ |
| 1-172 | 4-(O$CH_2$-CC-Me)-2,3,5,6-tetra-Me-$C_6$ |
| 1-173 | 4-(O$CH_2$-CC-Et)-2,3,5,6-tetra-Me-$C_6$ |
| 1-174 | 4-O$CH_2$(4-F-$C_6H_4$)-2,3,5,6-tetra-Me-$C_6$ |
| 1-175 | 4-O$CH_2$(4-Cl-$C_6H_4$)-2,3,5,6-tetra-Me-$C_6$ |
| 1-176 | 4-O(2,4-di-F-$C_6H_3$)-2,3,5,6-tetra-Me-$C_6$ |
| 1-177 | 4-O(2,4-di-Cl-$C_6H_3$)-2,3,5,6-tetra-Me-$C_6$ |
| 1-178 | 4-S(2,4-di-F-$C_6H_3$)-2,3,5,6-tetra-Me-$C_6$ |
| 1-179 | 4-S(2,4-di-Cl-$C_6H_3$)-2,3,5,6-tetra-Me-$C_6$ |
| 1-180 | 4-$CH_2$O(2,4-di-F-$C_6H_3$)-2,3,5,6-tetra-Me-$C_6$ |
| 1-181 | 4-$CH_2$O(2,4-di-Cl-$C_6H_3$)-2,3,5,6-tetra-Me-$C_6$ |
| 1-182 | 4-O$CH_2$(2,4-di-F-$C_6H_3$)-2,3,5,6-tetra-Me-$C_6$ |
| 1-183 | 4-O$CH_2$(2,4-di-Cl-$C_6H_3$)-2,3,5,6-tetra-Me-$C_6$ |
| 1-184 | 4-$CH_2$S(2,4-di-F-$C_6H_3$)-2,3,5,6-tetra-Me-$C_6$ |

(continued)

| Compound No. | $R^4$ |
|---|---|
| 1-185 | 4-$CH_2S$(2,4-di-Cl-$C_6H_3$)-2,3,5,6-tetra-Me-$C_6$ |
| 1-186 | 4-$CO_2$(2,4-di-F-$C_6H_3$)-2,3,5,6-tetra-Me-$C_6$ |
| 1-187 | 4-$CO_2$(2,4-di-Cl-$C_6H_3$)-2,3,5,6-tetra-Me-$C_6$ |
| 1-188 | 4-NHCO(2,4-di-F-$C_6H_3$)-2,3,5,6-tetra-Me-$C_6$ |
| 1-189 | 4-NHCO(2,4-di-Cl-$C_6H_3$)-2,3,5,6-tetra-Me-$C_6$ |
| 1-190 | 4-$NHSO_2$(2,4-di-F-$C_6H_3$)-2,3,5,6-tetra-Me-$C_6$ |
| 1-191 | 4-$NHSO_2$(2,4-di-Cl-$C_6H_3$)-2,3,5,6-tetra-Me-$C_6$ |
| 1-192 | 4-O(C=O)NH(2,4-di-F-$C_6H_3$)-2,3,5,6-tetra-Me-$C_6$ |
| 1-193 | 4-O(C=O)NH(2,4-di-Cl-$C_6H_3$)-2,3,5,6-tetra-Me-$C_6$ |
| 1-194 | 4-$NHCO_2$(2,4-di-F-$C_6H_3$)-2,3,5,6-tetra-Me-$C_6$ |
| 1-195 | 4-$NHCO_2$(2,4-di-Cl-$C_6H_3$)-2,3,5,6-tetra-Me-$C_6$ |
| 1-196 | 4-NHCONH(2,4-di-F-$C_6H_3$)-2,3,5,6-tetra-Me-$C_6$ |
| 1-197 | 4-NHCONH(2,4-di-Cl-$C_6H_3$)-2,3,5,6-tetra-Me-$C_6$ |
| 1-198 | 4-$CO_2$Me-2,3,6-tri-Me-$C_6H$ |
| 1-199 | 1-Naph |
| 1-200 | 2-Naph |
| 1-201 | 2-F-1-Naph |
| 1-202 | 3-F-1-Naph |
| 1-203 | 4-F-1-Naph |
| 1-204 | 2-Cl-1-Naph |
| 1-205 | 3-Cl-1-Naph |
| 1-206 | 4-Cl-1-Naph |
| 1-207 | 2-Br-1-Naph |
| 1-208 | 3-Br-1-Naph |
| 1-209 | 4-Br-1-Naph |
| 1-210 | 2-Me-1-Naph |
| 1-211 | 3-Me-1-Naph |
| 1-212 | 4-Me-1-Naph |
| 1-213 | 2-Et-1-Naph |
| 1-214 | 3-Et-1-Naph |
| 1-215 | 4-Et-1-Naph |
| 1-216 | 2-nPr-1-Naph |
| 1-217 | 3-nPr-1-Naph |
| 1-218 | 4-nPr-1-Naph |
| 1-219 | 2-iPr-1-Naph |
| 1-220 | 3-iPr-1-Naph |
| 1-221 | 4-iPr-1-Naph |
| 1-222 | 2-nBu-1-Naph |
| 1-223 | 3-nBu-1-Naph |
| 1-224 | 4-nBu-1-Naph |
| 1-225 | 2-$CH_2F$-1-Naph |
| 1-226 | 3-$CH_2F$-1-Naph |
| 1-227 | 4-$CH_2F$-1-Naph |
| 1-228 | 2-$CHF_2$-1-Naph |
| 1-229 | 3-$CHF_2$-1-Naph |
| 1-230 | 4-$CHF_2$-1-Naph |
| 1-231 | 2-$CF_3$-1-Naph |
| 1-232 | 3-$CF_3$-1-Naph |
| 1-233 | 4-$CF_3$-1-Naph |
| 1-234 | 2-$CH_2CH_2F$-1-Naph |

(continued)

| Compound No. | $R^4$ |
|---|---|
| 1-235 | 3-$CH_2CH_2F$-1-Naph |
| 1-236 | 4-$CH_2CH_2F$-1-Naph |
| 1-237 | 2-$CH_2CHF_2$-1-Naph |
| 1-238 | 3-$CH_2CHF_2$-1-Naph |
| 1-239 | 4-$CH_2CHF_2$-1-Naph |
| 1-240 | 2-$CH_2CF_3$-1-Naph |
| 1-241 | 3-$CH_2CF_3$-1-Naph |
| 1-242 | 4-$CH_2CF_3$-1-Naph |
| 1-243 | 2-$CH_2Cl$-1-Naph |
| 1-244 | 3-$CH_2Cl$-1-Naph |
| 1-245 | 4-$CH_2Cl$-1-Naph |
| 1-246 | 2-$CHCl_2$-1-Naph |
| 1-247 | 3-$CHCl_2$-1-Naph |
| 1-248 | 4-$CHCl_2$-1-Naph |
| 1-249 | 2-$CCl_3$-1-Naph |
| 1-250 | 3-$CCl_3$-1-Naph |
| 1-251 | 4-$CCl_3$-1-Naph |
| 1-252 | 2-$CH_2CH_2Cl$-1-Naph |
| 1-253 | 3-$CH_2CH_2Cl$-1-Naph |
| 1-254 | 4-$CH_2CH_2Cl$-1-Naph |
| 1-255 | 2-$CH_2CHCl_2$-1-Naph |
| 1-256 | 3-$CH_2CHCl_2$-1-Naph |
| 1-257 | 4-$CH_2CHCl_2$-1-Naph |
| 1-258 | 2-$CH_2CCl_3$-1-Naph |
| 1-259 | 3-$CH_2CCl_3$-1-Naph |
| 1-260 | 4-$CH_2CCl_3$-1-Naph |
| 1-261 | 2-($CH=CH_2$)-1-Naph |
| 1-262 | 3-($CH=CH_2$)-1-Naph |
| 1-263 | 4-($CH=CH_2$)-1-Naph |
| 1-264 | 2-[$CH=C(CH_3)_2$]-1-Naph |
| 1-265 | 3-[$CH=C(CH_3)_2$]-1-Naph |
| 1-266 | 4-[$CH=C(CH_3)_2$]-1-Naph |
| 1-267 | 2-OMe-1-Naph |
| 1-268 | 3-OMe-1-Naph |
| 1-269 | 4-OMe-1-Naph |
| 1-270 | 2-OEt-1-Naph |
| 1-271 | 3-OEt-1-Naph |
| 1-272 | 4-OEt-1-Naph |
| 1-273 | 2-OnPr-1-Naph |
| 1-274 | 3-OnPr-1-Naph |
| 1-275 | 4-OnPr-1-Naph |
| 1-276 | 2-OiPr-1-Naph |
| 1-277 | 3-OiPr-1-Naph |
| 1-278 | 4-OiPr-1-Naph |
| 1-279 | 2-OnBu-1-Naph |
| 1-280 | 3-OnBu-1-Naph |
| 1-281 | 4-OnBu-1-Naph |
| 1-282 | 2-($OCH_2$-CC-Me)-1-Naph |
| 1-283 | 3-($OCH_2$-CC-Me)-1-Naph |
| 1-284 | 4-($OCH_2$-CC-Me)-1-Naph |

(continued)

| Compound No. | $R^4$ |
|---|---|
| 1-285 | 2-($OCH_2$-CC-Et)-1-Naph |
| 1-286 | 3-($OCH_2$-CC-Et)-1-Naph |
| 1-287 | 4-($OCH_2$-CC-Et)-1-Naph |
| 1-288 | 2-$CH_2$OMe-1-Naph |
| 1-289 | 3-$CH_2$OMe-1-Naph |
| 1-290 | 4-$CH_2$OMe-1-Naph |
| 1-291 | 2-OH-1-Naph |
| 1-292 | 3-OH-1-Naph |
| 1-293 | 4-OH-1-Naph |
| 1-294 | 2-$CO_2$H-1-Naph |
| 1-295 | 3-$CO_2$H-1-Naph |
| 1-296 | 4-$CO_2$H-1-Naph |
| 1-297 | 2-(C=O)Me-1-Naph |
| 1-298 | 3-(C=O)Me-1-Naph |
| 1-299 | 4-(C=O)Me-1-Naph |
| 1-300 | 2-(C=O)Et-1-Naph |
| 1-301 | 3-(C=O)Et-1-Naph |
| 1-302 | 4-(C=O)Et-1-Naph |
| 1-303 | 2-(C=O)nPr-1-Naph |
| 1-304 | 3-(C=O)nPr-1-Naph |
| 1-305 | 4-(C=O)nPr-1-Naph |
| 1-306 | 2-(C=O)cPr-1-Naph |
| 1-307 | 3-(C=O)cPr-1-Naph |
| 1-308 | 4-(C=O)cPr-1-Naph |
| 1-309 | 2-O(C=O)Me-1-Naph |
| 1-310 | 3-O(C=O)Me-1-Naph |
| 1-311 | 4-O(C=O)Me-1-Naph |
| 1-312 | 2-O(C=O)Et-1-Naph |
| 1-313 | 3-O(C=O)Et-1-Naph |
| 1-314 | 4-O(C=O)Et-1-Naph |
| 1-315 | 2-O(C=O)nPr-1-Naph |
| 1-316 | 3-O(C=O)nPr-1-Naph |
| 1-317 | 4-O(C=O)nPr-1-Naph |
| 1-318 | 2-$CO_2$Me-1-Naph |
| 1-319 | 3-$CO_2$Me-1-Naph |
| 1-320 | 4-$CO_2$Me-1-Naph |
| 1-321 | 2-$CO_2$Et-1-Naph |
| 1-322 | 3-$CO_2$Et-1-Naph |
| 1-323 | 4-$CO_2$Et-1-Naph |
| 1-324 | 2-$CO_2$nPr-1-Naph |
| 1-325 | 3-$CO_2$nPr-1-Naph |
| 1-326 | 4-$CO_2$nPr-1-Naph |
| 1-327 | 2-$NH_2$-1-Naph |
| 1-328 | 3-$NH_2$-1-Naph |
| 1-329 | 4-$NH_2$-1-Naph |
| 1-330 | 2-CONHMe-1-Naph |
| 1-331 | 3-CONHMe-1-Naph |
| 1-332 | 4-CONHMe-1-Naph |
| 1-333 | 2-CONHEt-1-Naph |
| 1-334 | 3-CONHEt-1-Naph |

(continued)

| Compound No. | $R^4$ |
|---|---|
| 1-335 | 4-CONHEt-1-Naph |
| 1-336 | 2-$CONMe_2$-1-Naph |
| 1-337 | 3-$CONMe_2$-1-Naph |
| 1-338 | 4-$CONMe_2$-1-Naph |
| 1-339 | 2-$CONEt_2$-1-Naph |
| 1-340 | 3-$CONEt_2$-1-Naph |
| 1-341 | 4-$CONEt_2$-1-Naph |
| 1-342 | 2-O(C=O)NHMe-1-Naph |
| 1-343 | 3-O(C=O)NHMe-1-Naph |
| 1-344 | 4-O(C=O)NHMe-1-Naph |
| 1-345 | 2-O(C=O)NHEt-1-Naph |
| 1-346 | 3-O(C=O)NHEt-1-Naph |
| 1-347 | 4-O(C=O)NHEt-1-Naph |
| 1-348 | 2-O(C=O)$NMe_2$-1-Naph |
| 1-349 | 3-O(C=O)$NMe_2$-1-Naph |
| 1-350 | 4-O(C=O)$NMe_2$-1-Naph |
| 1-351 | 2-O(C=O)$NEt_2$-1-Naph |
| 1-352 | 3-O(C=O)$NEt_2$-1-Naph |
| 1-353 | 4-O(C=O)$NEt_2$-1-Naph |
| 1-354 | 2-NHCOMe-1-Naph |
| 1-355 | 3-NHCOMe-1-Naph |
| 1-356 | 4-NHCOMe-1-Naph |
| 1-357 | 2-NHCOEt-1-Naph |
| 1-358 | 3-NHCOEt-1-Naph |
| 1-359 | 4-NHCOEt-1-Naph |
| 1-360 | 2-$NHSO_2Me$-1-Naph |
| 1-361 | 3-$NHSO_2Me$-1-Naph |
| 1-362 | 4-$NHSO_2Me$-1-Naph |
| 1-363 | 2-$NHSO_2Et$-1-Naph |
| 1-364 | 3-$NHSO_2Et$-1-Naph |
| 1-365 | 4-$NHSO_2Et$-1-Naph |
| 1-366 | 2-CN-1-Naph |
| 1-367 | 3-CN-1-Naph |
| 1-368 | 4-CN-1-Naph |
| 1-369 | 2-$NO_2$-1-Naph |
| 1-370 | 3-$NO_2$-1-Naph |
| 1-371 | 4-$NO_2$-1-Naph |
| 1-372 | 2-$CONH_2$-1-Naph |
| 1-373 | 3-$CONH_2$-1-Naph |
| 1-374 | 4-$CONH_2$-1-Naph |
| 1-375 | 2-$CH_2CH_2CO_2H$-1-Naph |
| 1-376 | 3-$CH_2CH_2CO_2H$-1-Naph |
| 1-377 | 2-$CH_2CH_2CH_2CO_2H$-1-Naph |
| 1-378 | 3-$CH_2CH_2CH_2CO_2H$-1-Naph |
| 1-379 | 2-$CH_2CH_2CO_2Me$-1-Naph |
| 1-380 | 3-$CH_2CH_2CO_2Me$-1-Naph |
| 1-381 | 2-$CH_2CH_2CH_2CO_2Me$-1-Naph |
| 1-382 | 3-$CH_2CH_2CH_2CO_2Me$-1-Naph |
| 1-383 | 2-$CH_2CH_2CO_2Et$-1-Naph |
| 1-384 | 3-$CH_2CH_2CO_2Et$-1-Naph |

(continued)

| Compound No. | $R^4$ |
|---|---|
| 1-385 | 2-$CH_2CH_2CH_2CO_2$Et-1-Naph |
| 1-386 | 3-$CH_2CH_2CH_2CO_2$Et-1-Naph |
| 1-387 | 2-$OCH_2CO_2$Me-1-Naph |
| 1-388 | 3-$OCH_2CO_2$Me-1-Naph |
| 1-389 | 2-$OCH_2CH_2CO_2$Me-1-Naph |
| 1-390 | 3-$OCH_2CH_2CO_2$Me-1-Naph |
| 1-391 | 2-$OCH_2CO_2$Et-1-Naph |
| 1-392 | 3-$OCH_2CO_2$Et-1-Naph |
| 1-393 | 2-$OCH_2CH_2CO_2$Et-1-Naph |
| 1-394 | 3-$OCH_2CH_2CO_2$Et-1-Naph |
| 1-395 | 2-$OCH_2CONMe_2$-1-Naph |
| 1-396 | 4-$OCH_2COMMe_2$-1-Naph |
| 1-397 | 2-$OCH_2CH_2CONMe_2$-1-Naph |
| 1-398 | 4-$OCH_2CH_2CONMe_2$-1-Naph |
| 1-399 | 2-$OCH_2CONEt_2$-1-Naph |
| 1-400 | 4-$OCH_2CONEt_2$-1-Naph |
| 1-401 | 2-$OCH_2CH_2CONEt_2$-1-Naph |
| 1-402 | 4-$OCH_2CH_2CONEt_2$-1-Naph |
| 1-403 | 4-OPh-1-Naph |
| 1-404 | 4-O(4-F-$C_6H_4$)-1-Naph |
| 1-405 | 4-O(3-F-$C_6H_4$)-1-Naph |
| 1-406 | 4-O(2-F-$C_6H_4$)-1-Naph |
| 1-407 | 4-O(4-Cl-$C_6H_4$)-1-Naph |
| 1-408 | 4-O(3-Cl-$C_6H_4$)-1-Naph |
| 1-409 | 4-O(2-Cl-$C_6H_4$)-1-Naph |
| 1-410 | 4-O(4-$CF_3$-$C_6H_4$)-1-Naph |
| 1-411 | 4-O(3-$CF_3$-$C_6H_4$)-1-Naph |
| 1-412 | 4-O(2-$CF_3$-$C_6H_4$)-1-Naph |
| 1-413 | 4-O(2,4-di-F-$C_6H_3$)-1-Naph |
| 1-414 | 4-O(2,4-di-Cl-$C_6H_3$)-1-Naph |
| 1-415 | 4-O(3-Cl-4-F-$C_6H_3$)-1-Naph |
| 1-416 | 4-O(3,4-di-Cl-$C_6H_3$)-1-Naph |
| 1-417 | 4-SPh-1-Naph |
| 1-418 | 4-S(2,4-di-Cl-$C_6H_3$)-1-Naph |
| 1-419 | 4-$OCH_2$Ph-1-Naph |
| 1-420 | 4-$OCH_2$(4-F-$C_6H_4$)-1-Naph |
| 1-421 | 4-$OCH_2$(3-F-$C_6H_4$)-1-Naph |
| 1-422 | 4-$OCH_2$(2-F-$C_6H_4$)-1-Naph |
| 1-423 | 4-$OCH_2$(4-Cl-$C_6H_4$)-1-Naph |
| 1-424 | 4-$OCH_2$(3-Cl-$C_6H_4$)-1-Naph |
| 1-425 | 4-$OCH_2$(2-Cl-$C_6H_4$)-1-Naph |
| 1-426 | 4-$OCH_2$(4-$CF_3$-$C_6H_4$)-1-Naph |
| 1-427 | 4-$OCH_2$(3-$CF_3$-$C_6H_4$)-1-Naph |
| 1-428 | 4-$OCH_2$(2-$CF_3$-$C_6H_4$)-1-Naph |
| 1-429 | 4-$OCH_2$(2,4-di-F-$C_6H_3$)-1-Naph |
| 1-430 | 4-$OCH_2$(2,4-di-Cl-$C_6H_3$)-1-Naph |
| 1-431 | 4-$OCH_2$(3-Cl-4-F-$C_6H_3$)-1-Naph |
| 1-432 | 4-$OCH_2$(3,4-di-Cl-$C_6H_3$)-1-Naph |
| 1-433 | 4-$NHSO_2$Ph-1-Naph |
| 1-434 | 4-$NHSO_2$(2,4-di-Cl-$C_6H_3$)-1-Naph |

(continued)

| Compound No. | $R^4$ |
|---|---|
| 1-435 | 2,3-di-Me-1-Naph |
| 1-436 | 2,3-di-Et-1-Naph |
| 1-437 | 2-Et-3-Me-1-Naph |
| 1-438 | 3-Et-2-Me-1-Naph |
| 1-439 | 4-OMe-2-Me-1-Naph |
| 1-440 | 4-OEt-2-Me-1-Naph |
| 1-441 | 2-Me-4-OnPr-1-Naph |
| 1-442 | 2-Me-4-($OCH_2$-CC-Me)-1-Naph |
| 1-443 | 4-($OCH_2$-CC-Et)-2-Me-1-Naph |
| 1-444 | 4-$OCH_2$(4-F-$C_6H_4$)-2-Me-1-Naph |
| 1-445 | 4-$OCH_2$(3-F-$C_6H_4$)-2-Me-1-Naph |
| 1-446 | 4-$OCH_2$(2-F-$C_6H_4$)-2-Me-1-Naph |
| 1-447 | 4-$OCH_2$(4-Cl-$C_6H_4$)-2-Me-1-Naph |
| 1-448 | 4-$OCH_2$(3-Cl-$C_6H_4$)-2-Me-1-Naph |
| 1-449 | 4-$OCH_2$(2-Cl-$C_6H_4$)-2-Me-1-Naph |
| 1-450 | 2,3,4-tri-Me-1-Naph |
| 1-451 | 2,3-di-Me-4-OMe-1-Naph |
| 1-452 | 2,3-di-Me-4-OEt-1-Naph |
| 1-453 | 2,3-di-Me-4-OnPr-1-Naph |
| 1-454 | 4-($OCH_2$-CC-Me)-2,3-di-Me-1-Naph |
| 1-455 | 2,3-di-Me-4-($OCH_2$-CC-Et)-1-Naph |
| 1-456 | 2,3-di-Me-4-$OCH_2$(4-F-$C_6H_4$)-1-Naph |
| 1-457 | 2,3-di-Me-4-$OCH_2$(3-F-$C_6H_4$)-1-Naph |
| 1-458 | 2,3-di-Me-4-$OCH_2$(2-F-$C_6H_4$)-1-Naph |
| 1-459 | 4-$OCH_2$(4-Cl-$C_6H_4$)-2,3-di-Me-1-Naph |
| 1-460 | 4-$OCH_2$(3-Cl-$C_6H_4$)-2,3-di-Me-1-Naph |
| 1-461 | 4-$OCH_2$(2-Cl-$C_6H_4$)-2,3-di-Me-1-Naph |
| 1-462 | 4-Py |
| 1-463 | 3-Py |
| 1-464 | 2-Py |
| 1-465 | Ph (A) |
| 1-466 | Ph (B) |
| 1-467 | Ph (C) |
| 1-468 | Ph (D) |
| 1-469 | 5-F-1-Naph |
| 1-470 | 6-F-1-Naph |
| 1-471 | 7-F-1-Naph |
| 1-472 | 8-F-1-Naph |
| 1-473 | 5-Cl-1-Naph |
| 1-474 | 6-Cl-1-Naph |
| 1-475 | 7-Cl-1-Naph |
| 1-476 | 8-Cl-1-Naph |
| 1-477 | 5-Br-1-Naph |
| 1-478 | 6-Br-1-Naph |
| 1-479 | 7-Br-1-Naph |
| 1-480 | 8-Br-1-Naph |
| 1-481 | 5-Me-1-Naph |
| 1-482 | 6-Me-1-Naph |
| 1-483 | 7-Me-1-Naph |
| 1-484 | 8-Me-1-Naph |

(continued)

| Compound No. | $R^4$ |
|---|---|
| 1-485 | 5-Et-1-Naph |
| 1-486 | 6-Et-1-Naph |
| 1-487 | 7-Et-1-Naph |
| 1-488 | 8-Et-1-Naph |
| 1-489 | 4-O(4-Me-$C_6H_4$)-1-Naph |
| 1-490 | 4-O(3-Me-$C_6H_4$)-1-Naph |
| 1-491 | 4-O(2-Me-$C_6H_4$)-1-Naph |
| 1-492 | 2,4-di-Me-1-Naph |
| 1-493 | 2,4-di-Et-1-Naph |
| 1-494 | 2-Et-4-Me-1-Naph |
| 1-495 | 4-Et-2-Me-1-Naph |
| 1-496 | 2,3-di-F-1-Naph |
| 1-497 | 2,4-di-F-1-Naph |
| 1-498 | 2,5-di-F-1-Naph |
| 1-499 | 2,6-di-F-1-Naph |
| 1-500 | 2,7-di-F-1-Naph |
| 1-501 | 2,8-di-F-1-Naph |
| 1-502 | 3,4-di-F-1-Naph |
| 1-503 | 3,5-di-F-1-Naph |
| 1-504 | 3,6-di-F-1-Naph |
| 1-505 | 3,7-di-F-1-Naph |
| 1-506 | 3,8-di-F-1-Naph |
| 1-507 | 4,5-di-F-1-Naph |
| 1-508 | 4,6-di-F-1-Naph |
| 1-509 | 4,7-di-F-1-Naph |
| 1-510 | 4,8-di-F-1-Naph |
| 1-511 | 5,7-di-F-1-Naph |
| 1-512 | 2,3-di-Cl-1-Naph |
| 1-513 | 2,4-di-Cl-1-Naph |
| 1-514 | 2,5-di-Cl-1-Naph |
| 1-515 | 2,6-di-Cl-1-Naph |
| 1-516 | 2,7-di-Cl-1-Naph |
| 1-517 | 2,8-di-Cl-1-Naph |
| 1-518 | 3,4-di-Cl-1-Naph |
| 1-519 | 3,5-di-Cl-1-Naph |
| 1-520 | 3,6-di-Cl-1-Naph |
| 1-521 | 3,7-di-Cl-1-Naph |
| 1-522 | 3,8-di-Cl-1-Naph |
| 1-523 | 4,5-di-Cl-1-Naph |
| 1-524 | 4,6-di-Cl-1-Naph |
| 1-525 | 4,7-di-Cl-1-Naph |
| 1-526 | 4,8-di-Cl-1-Naph |
| 1-527 | 5,7-di-Cl-1-Naph |
| 1-528 | 2,3-di-Br-1-Naph |
| 1-529 | 2,4-di-Br-1-Naph |
| 1-530 | 2,5-di-Br-1-Naph |
| 1-531 | 2,6-di-Br-1-Naph |
| 1-532 | 2,7-di-Br-1-Naph |
| 1-533 | 2,8-di-Br-1-Naph |
| 1-534 | 3,4-di-Br-1-Naph |

(continued)

| Compound No. | $R^4$ |
|---|---|
| 1-535 | 3,5-di-Br-1-Naph |
| 1-536 | 3,6-di-Br-1-fVaph |
| 1-537 | 3,7-di-Br-1-Naph |
| 1-538 | 3,8-di-Br-1-Naph |
| 1-539 | 4,5-di-Br-1-Naph |
| 1-540 | 4,6-di-Br-1-Naph |
| 1-541 | 4,7-di-Br-1-Naph |
| 1-542 | 4,8-di-Br-1-Naph |
| 1-543 | 5,7-di-Br-1-Naph |
| 1-544 | 3-F-2-Me-1-Naph |
| 1-545 | 4-F-2-Me-1-Naph |
| 1-546 | 5-F-2-Me-1-Naph |
| 1-547 | 6-F-2-Me-1-Naph |
| 1-548 | 7-F-2-Me-1-Naph |
| 1-549 | 8-F-2-Me-1-Naph |
| 1-550 | 3-Cl-2-Me-1-Naph |
| 1-551 | 4-Cl-2-Me-1-Naph |
| 1-552 | 5-Cl-2-Me-1-Naph |
| 1-553 | 6-Cl-2-Me-1-Naph |
| 1-554 | 7-Cl-Me-1-Naph |
| 1-555 | 8-Cl-2-Me-1-Naph |
| 1-556 | 3-Br-2-Me-1-Naph |
| 1-557 | 4-Br-2-Me-1-Naph |
| 1-558 | 5-Br-2-Me-1-Naph |
| 1-559 | 6-Br-2-Me-1-Naph |
| 1-560 | 7-Br-2-Me-1-Naph |
| 1-561 | 8-Br-2-Me-1-Naph |
| 1-562 | 2-Et-3-F-1-Naph |
| 1-563 | 2-Et-4-F-1-Naph |
| 1-564 | 2-Et-5-F-1-Naph |
| 1-565 | 2-Et-6-F-1-Naph |
| 1-566 | 2-Et-7-F-1-Naph |
| 1-567 | 2-Et-8-F-1-Naph |
| 1-568 | 3-Cl-2-Et-1-Naph |
| 1-569 | 4-Cl-2-Et-1-Naph |
| 1-570 | 5-Cl-2-Et-1-Naph |
| 1-571 | 6-Cl-2-Et-1-Naph |
| 1-572 | 7-Cl-2-Et-1-Naph |
| 1-573 | 8-Cl-2-Et-1-Naph |
| 1-574 | 3-Br-2-Et-1-Naph |
| 1-575 | 4-Br-2-Et-1-Naph |
| 1-576 | 5-Br-2-Et-1-Naph |
| 1-577 | 6-Br-2-Et-1-Naph |
| 1-578 | 7-Br-2-Et-1-Naph |
| 1-579 | 8-Br-2-Et-1-Naph |
| 1-580 | 2-Et-4-F-3-Me-1-Naph |
| 1-581 | 2-Et-5-F-3-Me-1-Naph |
| 1-582 | 2-Et-6-F-3-Me-1-Naph |
| 1-583 | 2-Et-7-F-3-Me-1-Naph |
| 1-584 | 2-Et-8-F-3-Me-1-Naph |

(continued)

| Compound No. | $R^4$ |
|---|---|
| 1-585 | 4-Cl-2-Et-3-Me-1-Naph |
| 1-586 | 5-Cl-2-Et-3-Me-1-Naph |
| 1-587 | 6-Cl-2-Et-3-Me-1-Naph |
| 1-588 | 7-Cl-2-Et-3-Me-1-Naph |
| 1-589 | 8-Cl-2-Et-3-Me-1-Naph |
| 1-590 | 4-Br-2-Et-3-Me-1-Naph |
| 1-591 | 5-Br-2-Et-3-Me-1-Naph |
| 1-592 | 6-Br-2-Et-3-Me-1-Naph |
| 1-593 | 7-Br-2-Et-3-Me-1-Naph |
| 1-594 | 8-Br-2-Et-3-Me-1-Naph |
| 1-595 | 2,5-di-Me-1-Naph |
| 1-596 | 2,6-di-Me-1-Naph |
| 1-597 | 2,7-di-Me-1-Naph |
| 1-598 | 2,8-di-Me-1-Naph |
| 1-599 | 2-Et-5-Me-1-Naph |
| 1-600 | 2-Et-6-Me-1-Naph |
| 1-601 | 2-Et-7-Me-1-Naph |
| 1-602 | 2-Et-8-Me-1-Naph |
| 1-603 | 3,4-di-F-2-Me-1-Naph |
| 1-604 | 3,5-di-F-2-Me-1-Naph |
| 1-605 | 3,6-di-F-2-Me-1-Naph |
| 1-606 | 3,7-di-F-2-Me-1-Naph |
| 1-607 | 3,8-di-F-2-Me-1-Naph |
| 1-608 | 4,5-di-F-2-Me-1-Naph |
| 1-609 | 4,6-di-F-2-Me-1-Naph |
| 1-610 | 4,7-di-F-2-Me-1-Naph |
| 1-611 | 4,8-di-F-2-Me-1-Naph |
| 1-612 | 5,6-di-F-2-Me-1-Naph |
| 1-613 | 5,7-di-F-2-Me-1-Naph |
| 1-614 | 5,8-di-F-2-Me-1-Naph |
| 1-615 | 6,7-di-F-2-Me-1-Naph |
| 1-616 | 6.8-di-F-2-Me-1-Naph |
| 1-617 | 7,8-di-F-2-Me-1-Naph |
| 1-618 | 3,4-di-F-2-Et-1-Naph |
| 1-619 | 3,5-di-F-2-Et-1-Naph |
| 1-620 | 3,6-di-F-2-Et-1-Naph |
| 1-621 | 3,7-di-F-2-Et-1-Naph |
| 1-622 | 3,8-di-F-2-Et-1-Naph |
| 1-623 | 4,5-di-F-2-Et-1-Naph |
| 1-624 | 4,6-di-F-2-Et-1-Naph |
| 1-625 | 4,7-di-F-2-Et-1-Naph |
| 1-626 | 4,8-di-F-2-Et-1-Naph |
| 1-627 | 5,6-di-F-2-Et-1-Naph |
| 1-628 | 5,7-di-F-2-Et-1-Naph |
| 1-629 | 5,8-di-F-2-Et-1-Naph |
| 1-630 | 6,7-di-F-2-Et-1-Naph |
| 1-631 | 6,8-di-F-2-Et-1-Naph |
| 1-632 | 7,8-di-F-2-Et-1-Naph |
| 1-633 | 2-Et-4-F-6-Me-1-Naph |
| 1-634 | 2-Et-5-F-6-Me-1-Naph |

(continued)

| Compound No. | $R^4$ |
|---|---|
| 1-635 | 2-Et-7-F-6-Me-1-Naph |
| 1-636 | 2-Et-8-F-6-Me-1-Naph |
| 1-637 | 2-Et-4-F-7-Me-1-Naph |
| 1-638 | 2-Et-5-F-7-Me-1-Naph |
| 1-639 | 2-Et-6-F-7-Me-1-Naph |
| 1-640 | 2-Et-8-F-7-Me-1-Naph |
| 1-641 | 2-Me-5,6,7-tri-F-1-Naph |
| 1-642 | 2-Me-5,6,7-tri-Cl-1-Naph |
| 1-643 | 2-Et-5,6,7-tri-F-1-Naph |
| 1-644 | 2-Et-5,6,7-tri-Cl-1-Naph |

(Table 2)

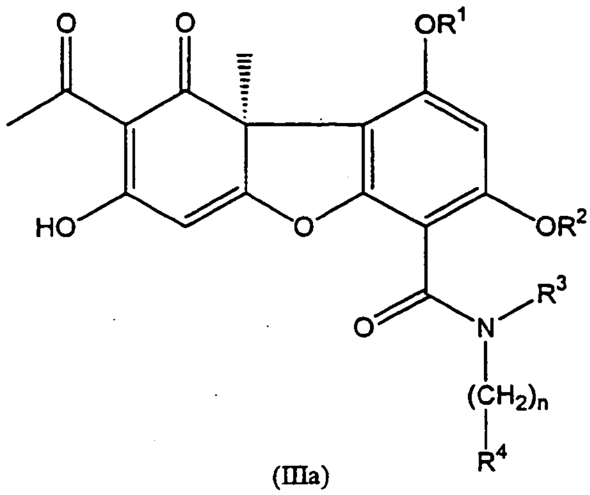

(IIIa)

| Compound No. | $R^1$ | $R^2$ | $R^3$ | n | $R^4$ |
|---|---|---|---|---|---|
| 2-1 | H | H | H | 1 | $C_6H_5$ |
| 2-2 | H | H | H | 1 | 1-Naph |
| 2-3 | H | H | H | 1 | 2-Me-1-Naph |
| 2-4 | H | H | H | 1 | 2-Et-1-Naph |
| 2-5 | H | H | H | 1 | 2-Et-3-Me-1-Naph |
| 2-6 | H | Me | Me | 1 | $C_6H_5$ |
| 2-7 | H | Me | H | 2 | $C_6H_5$ |
| 2-8 | H | Me | Me | 2 | $C_6H_5$ |
| 2-9 | H | Me | Me | 1 | 4-O(2,4-di-Cl-$C_6H_3$)-2,6-di-Me-$C_6H_2$ |
| 2-10 | H | Me | H | 2 | 4-O(2,4-di-Cl-$C_6H_3$-2,6-di-Me-$C_6H_2$ |
| 2-11 | H | Me | Me | 2 | 4-O(2,4-di-Cl-$C_6H_3$)-2,6-di-Me-$C_6H_2$ |
| 2-12 | H | Me | Me | 1 | 4-S(2,4-di-Cl-$C_6H_3$)-2,6-di-Me-$C_6H_2$ |
| 2-13 | H | Me | H | 2 | 4-S(2,4-di-Cl-$C_6H_3$)-2,6-di-Me-$C_6H_2$ |
| 2-14 | H | Me | Me | 2 | 4-S(2,4-di-Cl-$C_6H_3$)-2,6-di-Me-$C_6H_2$ |
| 2-15 | H | Me | Me | 1 | 4-$CH_2$O(2,4-di-Cl-$C_6H_3$)-2,6-di-Me-$C_6H_2$ |
| 2-16 | H | Me | H | 2 | 4-$CH_2$O(2,4-di-Cl-$C_6H_3$)-2,6-di-Me-$C_6H_2$ |
| 2-17 | H | Me | Me | 2 | 4-$CH_2$O (2,4-di-Cl-$C_6H_3$)-2,6-di-Me-$C_6H_2$ |

(continued)

| Compound No. | $R^1$ | $R^2$ | $R^3$ | n | $R^4$ |
|---|---|---|---|---|---|
| 2-18 | H | Me | Me | 1 | 4-OCH$_2$(2,4-di-Cl-C$_6$H$_3$)-2,6-di-Me.C$_6$H$_2$ |
| 2-19 | H | Me | H | 2 | 4-OCH$_2$(2,4-di-Cl-C$_6$H$_3$)-2,6-di-Me-C$_6$H$_2$ |
| 2-20 | H | Me | Me | 2 | 4-OCH$_2$(2,4-di-Cl-C$_6$H$_3$)-2,6-di-Me-C$_6$H$_2$ |
| 2-21 | H | Me | Me | 1 | 4-CH$_2$S(2,4-di-Cl-C$_6$H$_3$)-2,6-di-Me-C$_6$H$_2$ |
| 2-22 | H | Me | H | 2 | 4-CH$_2$S(2,4-di-Cl-C$_6$H$_3$)-2,6-di-Me-C$_6$H$_2$ |
| 2-23 | H | Me | Me | 2 | 4-CH$_2$S(2,4-di-Cl-C$_6$H$_3$)-2,6-di-Me-C$_6$H$_2$ |
| 2-24 | H | Me | Me | 1 | 4-CO$_2$(2,4-di-C1-C$_6$H$_3$)-2,6-di-Me-C$_6$H$_2$ |
| 2-25 | H | Me | H | 2 | 4-CO$_2$(2,4-di-Cl-C$_6$H$_3$)-2,6-di-Me-C$_6$H$_2$ |
| 2-26 | H | Me | Me | 2 | 4-CO$_2$(2,4-di-Cl-C$_6$H$_3$)-2,6-di-Me-C$_6$H$_2$ |
| 2-27 | H | Me | Me | 1 | 4-NHCO(2,4-di-Cl-C$_6$H$_3$)-2,6-di-Me-C$_6$H$_2$ |
| 2-28 | H | Me | H | 2 | 4-NHCO(2,4-di-Cl-C$_6$H$_3$)-2,6-di-Me-C$_6$H$_2$ |
| 2-29 | H | Me | Me | 2 | 4-NHCO(2,4-di-Cl-C$_6$H$_3$)-2,6-di-Me-C$_6$H$_2$ |
| 2-30 | H | Me | Me | 1 | 4-NHSO$_2$(2,4-di-Cl-C$_6$H$_3$)-2,6-di-Me-C$_6$H$_2$ |
| 2-31 | H | Me | H | 2 | 4-NHSO$_2$(2,4-di-Cl-C$_6$H$_3$)-2,6-di-Me-C$_6$H$_2$ |
| 2-32 | H | Me | Me | 2 | 4-NHSO$_2$(2,4-di-Cl-C$_6$H$_3$)-2,6-di-Me-C$_6$H$_2$ |
| 2-33 | H | Me | Me | 1 | 4-O(C=O)NH(2,4-di-Cl-C$_6$H$_3$)-2,6-di-Me-C$_6$H$_2$ |
| 2-34 | H | Me | H | 2 | 4-O(C=O)NH(2,4-di-Cl-C$_6$H$_3$)-2,6-di-Me-C$_6$H$_2$ |
| 2-35 | H | Me | Me | 2 | 4-O(C=O)NH(2,4-di-Cl-C$_6$H$_3$)-2,6-di-Me-C$_6$H$_2$ |
| 2-36 | H | Me | Me | 1 | 4-NHCO$_2$(2,4-di-Cl-C$_6$H$_3$)-2,6-di-Me-C$_6$H$_2$ |
| 2-37 | H | Me | H | 2 | 4-NHCO$_2$(2,4-di-Cl-C$_6$H$_3$)-2,6-di-Me-C$_6$H$_2$ |
| 2-38 | H | Me | Me | 2 | 4-NHCO$_2$(2,4-di-Cl-C$_6$H$_3$)-2,6-di-Me-C$_6$H$_2$ |
| 2-39 | H | Me | Me | 1 | 4-NHCONH(2,4-di-Cl-C$_6$H$_3$)-2,6-di-Me-C$_6$H$_2$ |
| 2-40 | H | Me | H | 2 | 4-NHCONH(2,4-di-Cl-C$_6$H$_3$)-2,6-di-Me-C$_6$H$_2$ |
| 2-41 | H | Me | Me | 2 | 4-NHCONH(2,4-di-Cl-C$_6$H$_3$)-2,6-di-Me-C$_6$H$_2$ |
| 2-42 | H | Me | Me | 1 | 1-Naph |
| 2-43 | H | Me | H | 2 | 1-Naph |
| 2-44 | H | Me | Me | 2 | 1-Naph |
| 2-45 | H | Me | Me | 1 | 2-F-1-Naph |
| 2-46 | H | Me | H | 2 | 2-F-1-Naph |
| 2-47 | H | Me | Me | 2 | 2-F-1-Naph |
| 2-48 | H | Me | Me | 1 | 2-Cl-1-Naph |
| 2-49 | H | Me | H | 2 | 2-Cl-1-Naph |
| 2-50 | H | Me | Me | 2 | 2-Cl-1-Naph |
| 2-51 | H | Me | Me | 1 | 2-Me-1-Naph |
| 2-52 | H | Me | H | 2 | 2-Me-1-Naph |
| 2-53 | H | Me | Me | 2 | 2-Me-1-Naph |
| 2-54 | H | Me | Me | 1 | 2-Et-1-Naph |
| 2-55 | H | Me | H | 2 | 2-Et-1-Naph |
| 2-56 | H | Me | Me | 2 | 2-Et-1-Naph |
| 2-57 | H | Me | Me | 1 | 3-Me-1-Naph |
| 2-58 | H | Me | H | 2 | 3-Me-1-Naph |
| 2-59 | H | Me | Me | 2 | 3-Me-1-Naph |
| 2-60 | H | Me | Me | 1 | 4-Me-1-Naph |
| 2-61 | H | Me | H | 2 | 4-Me-1-Naph |
| 2-62 | H | Me | Me | 2 | 4-Me-1-Naph |
| 2-63 | H | Me | Me | 1 | 2-Et-3-Me-1-Naph |
| 2-64 | H | Me | H | 2 | 2-Et-3-Me-1-Naph |

(continued)

| Compound No. | $R^1$ | $R^2$ | $R^3$ | n | $R^4$ |
|---|---|---|---|---|---|
| 2-65 | H | Me | Me | 2 | 2-Et-3-Me-1-Naph |
| 2-66 | H | Et | H | 1 | $C_6H_5$ |
| 2-67 | H | Et | H | 1 | 1-Naph |
| 2-68 | H | Et | H | 1 | 2-Me-1-Naph |
| 2-69 | H | Et | H | 1 | 2-Et-1-Naph |
| 2-70 | H | Et | H | 1 | 2-Et-3-Me-1-Naph |
| 2-71 | H | $CHF_2$ | H | 1 | $C_6H_5$ |
| 2-72 | H | $CHF_2$ | H | 1 | 1-Naph |
| 2-73 | H | $CHF_2$ | H | 1 | 2-Me-1-Naph |
| 2-74 | H | $CHF_2$ | H | 1 | 2-Et-1-Naph |
| 2-75 | H | $CHF_2$ | H | 1 | 2-Et-3-Me-1-Naph |
| 2-76 | Me | H | H | 1 | $C_6H_5$ |
| 2-77 | Me | H | H | 1 | 1-Naph |
| 2-78 | Me | H | H | 1 | 2-Me-1-Naph |
| 2-79 | Me | H | H | 1 | 2-Et-1-Naph |
| 2-80 | Me | H | H | 1 | 2-Et-3-Me-1-Naph |
| 2-81 | Me | Me | H | 1 | $C_6H_5$ |
| 2-82 | Me | Me | H | 1 | 1-Naph |
| 2-83 | Me | Me | H | 1 | 2-Me-1-Naph |
| 2-84 | Me | Me | H | 1 | 2-Et-1-Naph |
| 2-85 | Me | Me | H | 1 | 2-Et-3-Me-1-Naph |
| 2-86 | Et | Me | H | 1 | $C_6H_5$ |
| 2-87 | Et | Me | H | 1 | 1-Naph |
| 2-88 | Et | Me | H | 1 | 2-Me-1-Naph |
| 2-89 | Et | Me | H | 1 | 2-Et-1-Naph |
| 2-90 | Et | Me | H | 1 | 2-Et-3-Me-1-Naph |
| 2-91 | Et | Et | H | 1 | $C_6H_5$ |
| 2-92 | Et | Et | H | 1 | 1-Naph |
| 2-93 | Et | Et | H | 1 | 2-Me-1-Naph |
| 2-94 | Et | Et | H | 1 | 2-Et-1-Naph |
| 2-95 | Et | Et | H | 1 | 2-Et-3-Me-1-Naph |
| 2-96 | Pr | Me | H | 1 | $C_6H_5$ |
| 2-97 | Pr | Me | H | 1 | 1-Naph |
| 2-98 | Pr | Me | H | 1 | 2-Me-1-Naph |
| 2-99 | Pr | Me | H | 1 | 2-Et-1-Naph |
| 2-100 | Pr | Me | H | 1 | 2-Et-3-Me-1-Naph |

(Table 3)

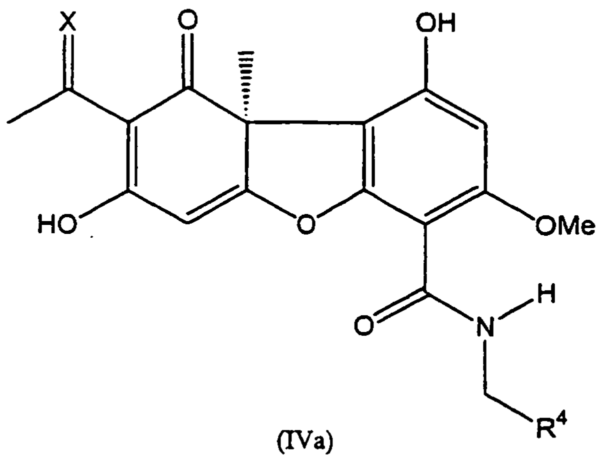

(IVa)

| Compound No. | X | $R^4$ |
|---|---|---|
| 3-1 | MeO-N= | $C_6H_5$ |
| 3-2 | MeO-N= | 4-O(2,4-di-Cl-$C_6H_3$)-2,6-di-Me-$C_6H_2$ |
| 3-3 | MeO-N= | 4-S(2,4-di-Cl-$C_6H_3$)-2,6-di-Me-$C_6H_2$ |
| 3-4 | MeO-N= | 4-$CH_2$O (2,4-di-Cl-$C_6H_3$)-2,6-di-Me-$C_6H_2$ |
| 3-5 | MeO-N= | 4-O$CH_2$(2,4-di-Cl-$C_6H_3$)-2,6-di-Me-$C_6H_2$ |
| 3-6 | MeO-N= | 4-$CH_2$S(2,4-di-Cl-$C_6H_3$)-2,6-di-Me-$C_6H_2$ |
| 3-7 | MeO-N= | 4-$CO_2$(2,4-di-Cl-$C_6H_3$)-2,6-di-Me-$C_6H_2$ |
| 3-8 | MeO-N= | 4-NHCO(2,4-di-Cl-$C_6H_3$)-2,6-di-Me-$C_6H_2$ |
| 3-9 | MeO-N= | 4-NH$SO_2$(2,4-di-Cl-$C_6H_3$)-2,6-di-Me-$C_6H_2$ |
| 3-10 | MeO-N= | 4-O(C=O)NH(2,4-di-Cl-$C_6H_3$)-2,6-di-Me-$C_6H_2$ |
| 3-11 | MeO-N= | 4-NH$CO_2$(2,4-di-Cl-Cl)-2,6-di-Me-$C_5H_2$ |
| 3-12 | MeO-N= | 4-NH$CO_2$(2,4-di-Cl-$C_6H_3$)-2,6-di-Me-$C_6H_2$ |
| 3-13 | MeO-N= | 4-OH-2,3,6-tri-Me-$C_6H$ |
| 3-14 | MeO-N= | 4-(O$CH_2$-CC- Me)- 2,3,6- tri- Me- $C_6H$ |
| 3-15 | MeO-N= | 1- Naph |
| 3-16 | MeO-N= | 2- F- 1- Naph |
| 3-17 | MeO-N= | 2- Cl- 1- Naph |
| 3-18 | MeO-N= | 2- Me- 1- Naph |
| 3-19 | MeO-N= | 2- Et- 1- Naph |
| 3-20 | MeO-N= | 3- Me- 1- Naph |
| 3-21 | MeO-N= | 4- Me- 1- Naph |
| 3-22 | MeO-N= | 2- Et- 3- Me- 1- Naph |
| 3-23 | EtO-N= | 1- Naph |
| 3-24 | EtO-N= | 2- Me- 1- Naph |
| 3-25 | EtO-N= | 2- Et- 1- Naph |
| 3-26 | EtO-N= | 2- Et- 3- Me- 1- Naph |
| 3-27 | PrO-N= | 1- Naph |
| 3-28 | PrO-N= | 2- Me- 1- Naph |
| 3-29 | PrO-N= | 2- Et- 1- Naph |
| 3-30 | PrO-N= | 2- Et- 3- Me- 1- Naph |
| 3-31 | $H_2C$=CH-$CH_2$O-N= | $C_6H_5$ |
| 3-32 | $H_2C$=CH-$CH_2$O-N= | 4-O(2,4-di-Cl-$C_6H_3$)-2,6-di-Me-$C_6H_2$ |
| 3-33 | $H_2C$=CH-$CH_2$O-N= | 4-S(2,4-di-Cl-$C_6H_3$)-2,6-di-Me-$C_6H_2$ |

(continued)

| Compound No. | X | $R^4$ |
|---|---|---|
| 3-34 | $H_2C=CH-CH_2O-N=$ | 4-$CH_2O$(2,4-di-Cl-$C_6H_3$)-2,6-di-Me-$C_6H_2$ |
| 3-35 | $H_2C=CH-CH_2O-N=$ | 4-$OCH_2$(2,4-di-Cl-$C_6H_3$)-2,6-di-Me-$C_6H_2$ |
| 3-36 | $H_2C=CH-CH_2O-N=$ | 4-$CH_2S$(2,4-di-Cl-$C_6H_3$)-2,6-di-Me-$C_6H_2$ |
| 3-37 | $H_2C=CH-CH_2O-N=$ | 4-$CO_2$(2,4-di-Cl-$C_6H_3$)-2,6-di-Me-$C_6H_2$ |
| 3-38 | $H_2C=CH-CH_2O-N=$ | 4-NHCO(2,4-di-Cl-$C_6H_3$)-2,6-di-Me-$C_6H_2$ |
| 3-39 | $H_2C=CH-CH_2O-N=$ | 4-$NHSO_2$(2,4-di-Cl-$C_6H_3$)-2,6-di-Me-$C_6N_2$ |
| 3-40 | $H_2C=CH-CH_2O-N=$ | 4-O(C=O)NH(2,4-di-Cl-$C_6H_3$)-2,6-di-Me-$C_6H_2$ |
| 3-41 | $H_2C=CH-CH_2O-N=$ | 4-$NHCO_2$(2,4-di-Cl-$C_6H_3$)-2,6-di-Me-$C_6H_2$ |
| 3-42 | $H_2C=CH-CH_2O-N=$ | 4-$NHCO_2$(2,4-di-Cl-$C_6H_3$)-2,6-di-Me-$C_6H_2$ |
| 3-43 | $H_2C=CH-CH_2O-N=$ | 4-OH-2,3,6-tri-Me-$C_6H$ |
| 3-44 | $H_2C=CH-CH_2O-N=$ | 4-($OCH_2$-CC- Me)- 2,3,6- tri- Me- $C_6H$ |
| 3-45 | $H_2C=CH-CH_2O-N=$ | 1- Naph |
| 3-46 | $H_2C=CH-CH_2O-N=$ | 2- F- 1- Naph |
| 3-47 | $H_2C=CH-CH_2O-N=$ | 2- Cl- 1- Naph |
| 3-48 | $H_2C=CH-CH_2O-N=$ | 2- Me- 1- Naph |
| 3-49 | $H_2C=CH-CH_2O-N=$ | 2- Et- 1- Naph |
| 3-50 | $H_2C=CH-CH_2O-N=$ | 3- Me- 1- Naph |
| 3-51 | $H_2C=CH-CH_2O-N=$ | 4- Me- 1- Naph |
| 3-52 | $H_2C=CH-CH_2O-N=$ | 2- Et- 3- Me- 1- Naph |
| 3-53 | H-CC-$CH_2O$-N= | $C_6H_5$ |
| 3-54 | H-CC-$CH_2O$-N= | 4-O(2,4-di-Cl-$C_6H_3$)-2,6-di-Me-$C_6H_2$ |
| 3-55 | H-CC-$CH_2O$-N= | 4-S(2,4-di-Cl-$C_6H_3$)-2,6-di-Me-$C_6H_2$ |
| 3-56 | H-CC-$CH_2O$-N= | 4-$CH_2O$(2,4-di-Cl-$C_6H_3$)-2,6-di-Me-$C_6H_2$ |
| 3-57 | H-CC-$CH_2O$-N= | 4-$OCH_2$(2,4-di-Cl-$C_6H_3$)-2,6-di-Me-$C_6H_2$ |
| 3-58 | H-CC-$CH_2O$-N= | 4-$CH_2S$(2,4-di-Cl-$C_6H_3$)-2,6-di-Me-$C_6H_2$ |
| 3-59 | H-CC-$CH_2O$-N= | 4-$CO_2$(2,4-di-Cl-$C_6H_3$)-2,6-di-Me-$C_6H_2$ |
| 3-60 | H-CC-$CH_2O$-N= | 4-NHCO (2,4-di-Cl-$C_6H_3$)-2,6-di-Me-$C_6H_2$ |
| 3-61 | H-CC-$CH_2O$-N= | 4-$NHSO_2$(2,4-di-Cl-$C_6H_3$)-2,6-di-Me-$C_6H_2$ |
| 3-62 | H-CC-$CH_2O$-N= | 4-O(C=O)NH(2,4-di-Cl-$C_6H_3$)-2,6-di-Me-$C_6H_2$ |
| 3-63 | H-CC-$CH_2O$-N= | 4-$NHCO_2$(2,4-di-Cl-$C_6H_3$)-2,6-di-Me-$C_6H_2$ |
| 3-64 | H-CC-$CH_2O$-N= | 4-$NHCO_2$(2,4-di-Cl-$C_6H_3$)-2,6-di-Me-$C_6H_2$ |
| 3-65 | H-CC-$CH_2O$-N= | 4-OH-2,3,6-tri-Me-$C_6H$ |
| 3-66 | H-CC-$CH_2O$-N= | 4-($OCH_2$-CC- Me)- 2,3,6- tri- Me- $C_6H$ |
| 3-67 | H-CC-$CH_2O$-N= | 1- Naph |
| 3-68 | H-CC-$CH_2O$-N= | 2- F- 1- Naph |
| 3-69 | H-CC-$CH_2O$-N= | 2- Cl- 1- Naph |
| 3-70 | H-CC-$CH_2O$-N= | 2- Me- 1- Naph |
| 3-71 | H-CC-$CH_2O$-N= | 2- Et- 1- Naph |
| 3-72 | H-CC-$CH_2O$-N= | 3- Me- 1- Naph |
| 3-73 | H-CC-$CH_2O$-N= | 4- Me- 1- Naph |
| 3-74 | H-CC-$CH_2O$-N= | 2- Et- 3- Me- 1- Naph |
| 3-75 | Me-CC-$CH_2O$-N= | 1- Naph |
| 3-76 | Me-CC-$CH_2O$-N= | 2- Me- 1- Naph |
| 3-77 | Me-CC-$CH_2O$-N= | 2- Et- 1- Naph |
| 3-78 | Me-CC-$CH_2O$-N= | 2- Et- 3- Me- 1- Naph |
| 3-79 | cPr-$CH_2O$-N= | 1- Naph |
| 3-80 | cPr-$CH_2O$-N= | 2- Me- 1- Naph |
| 3-81 | cPr-$CH_2O$-N= | 2- Et- 1- Naph |
| 3-82 | cPr-$CH_2O$-N= | 2- Et- 3- Me- 1- Naph |
| 3-83 | Ph-$CH_2O$-N= | 1- Naph |

(continued)

| Compound No. | X | $R^4$ |
|---|---|---|
| 3-84 | Ph-$CH_2$O-N= | 2- Me- 1- Naph |
| 3-85 | Ph-$CH_2$O-N= | 2- Et- 1- Naph |
| 3-86 | Ph-$CH_2$O-N= | 2- Et- 3- Me- 1- Naph |
| 3-87 | HO$CH_2CH_2$O-N= | 1- Naph |
| 3-88 | HO$CH_2CH_2$O-N= | 2- Me- 1- Naph |
| 3-89 | HO$CH_2CH_2$O-N= | 2- Et- 1- Naph |
| 3-90 | HO$CH_2CH_2$O-N= | 2- Et- 3- Me- 1- Naph |
| 3-91 | HO(C=O)$CH_2$O-N= | 1- Naph |
| 3-92 | HO(C=O)$CH_2$O-N= | 2- Me- 1- Naph |
| 3-93 | HO(C=O)$CH_2$O-N= | 2- Et- 1- Naph |
| 3-94 | HO(C=O)$CH_2$O-N= | 2- Et- 3- Me- 1- Naph |
| 3-95 | Me(C=O)$CH_2$O-N= | 1- Naph |
| 3-96 | Me(C=O)$CH_2$O-N= | 2- Me- 1- Naph |
| 3-97 | Me(C=O)$CH_2$O-N= | 2- Et- 1- Naph |
| 3-98 | Me(C=O)$CH_2$O-N= | 2- Et- 3- Me- 1- Naph |
| 3-99 | MeO(C=O)$CH_2$O-N= | 1- Naph |
| 3-100 | MeO(C=O)$CH_2$O-N= | 2- Me- 1- Naph |
| 3-101 | MeO(C=O)$CH_2$O-N= | 2- Et- 1- Naph |
| 3-102 | MeO(C=O)$CH_2$O-N= | 2- Et- 3- Me- 1- Naph |
| 3-103 | Me-N= | 1- Naph |
| 3-104 | Me-N= | 2- Me- I- Naph |
| 3-105 | Me-N= | 2- Et- 1- Naph |
| 3-106 | Me-N= | 2- Et- 3- Me- 1- Naph |
| 3-107 | Ph$CH_2$-N= | 1- Naph |
| 3-108 | Ph$CH_2$-N= | 2- Me- 1- Naph |
| 3-109 | Ph$CH_2$-N= | 2- Et- 1- Naph |
| 3-110 | Ph$CH_2$-N= | 2- Et- 3- Me- 1- Naph |

In Tables 1 to 3, a preferable compound is a compound No. 1-1, 1-5, 1-12, 1-23, 1-26, 1-30, 1-36, 1-40, 1-61, 1-64, 1-66, 1-68, 1-71, 1-73, 1-75, 1-76, 1-77, 1-80, 1-81, 1-87, 1-88, 1-90, 1-91, 1-92, 1-93, 1-97, 1-98, 1-105, 1-106, 1-109, 1-111, 1-112, 1-115, 1-116, 1-118, 1-119, 1-121, 1-123, 1-131, 1-132, 1-134, 1-135, 1-138, 1-139, 1-142, 1-143, 1-147, 1-149, 1-156, 1-157, 1-159, 1-162, 1-163, 1-164, 1-166, 1-169, 1-172, 1-173, 1-174, 1-176, 1-177, 1-183, 1-190, 1-191, 1-192, 1-196, 1-197, 1-199, 1-201, 1-202, 1-203, 1-204, 1-205, 1-206, 1-207, 1-209, 1-210, 1-211, 1-212, 1-213, 1-214, 1-222, 1-223, 1-225, 1-228, 1-231, 1-240, 1-261, 1-268, 1-269, 1-270, 1-272, 1-275, 1-276, 1-281, 1-284, 1-287, 1-319, 1-335, 1-339, 1-347, 1-349, 1-353, 1-361, 1-365, 1-401, 1-403, 1-404, 1-406, 1-407, 1-408, 1-409, 1-410, 1-412, 1-413, 1-414, 1-419, 1-420, 1-421, 1-422, 1-423, 1-424, 1-425, 1-426, 1-428, 1-429, 1-430, 1-431, 1-432, 1-433, 1-434, 1-435, 1-436, 1-437, 1-438, 1-439, 1-440, 1-441, 1-442, 1-443, 1-444, 1-445, 1-446, 1-447, 1-448, 1-449, 1-450, 1-453, 1-454, 1-455, 1-456, 1-457, 1-458, 1-459, 1-460, 1-461, 1-465, 1-469, 1-470, 1-471, 1-472, 1-474, 1-475, 1-476, 1-481, 1-482, 1-483, 1-484, 1-492, 1-493, 1-494, 1-496, 1-497, 1-498, 1-499, 1-500, 1-501, 1-544, 1-546, 1-548, 1-550, 1-551, 1-552, 1-553, 1-554, 1-555, 1-562, 1-563, 1-564, 1-565, 1-566, 1-567, 1-568, 1-569, 1-570, 1-571, 1-572, 1-573, 1-580, 1-581, 1-582, 1-583, 1-584, 1-595, 1-596,1-597, 1-598, 1-599, 1-600, 1-601, 1-602, 2-68, 2-73, 2-89, 2-94, 2-98, 2-99, 3-2, 3-5, 3-14, 3-18, 3-24, 3-28, 3-39, 3-44, 3-48, 3-70, 3-76, 3-80, 3-84, 3-96, 3-100, 3-104 or 3-108,
a more preferable compound is
(9aS)-8-acetyl-1,7-dihydroxy-3-methoxy-9a-methyl-9-oxo-N-(2,3,6-trimethylbenzyl)-9,9a-dihydrodibenzo[b,d]furan-4-carboxamide (compound No. 1-64),
(9aS)-8-acetyl-1,7-dihydroxy-N-(mesitylmethyl)-3-methoxy-9a-methyl-9-oxo-9,9a-dihydrodibenzo[b,d]furan-4-carboxamide (compound No. 1-66),
(9aS)-8-acetyl-1,7-dihydroxy-3-methoxy-9a-methyl-9-oxo-N-(2,4,6-triethylbenzyl)-9,9a-dihydrodibenzo[b,d]furan-4-carboxamide (compound No. 1-68),
(9aS)-8-acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide (compound No. 1-76),
(9aS)-8-acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide (compound No. 1-77),
(9aS)-8-acetyl-N-{4-[(2,4-dichlorobenzyl)oxy]-2,6-dimethylbenzyl}-1,7-dihydroxy-3-methoxy-9a-methyl-9-oxo-9,9a-dihydrodibenzo[b,d]furan-4-carboxamide (compound No. 1-81),
(9aS)-8-acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (compound No. 1-92),
(9aS)-8-acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (compound No. 1-93),
(9aS)-8-acetyl-1,7-dihydroxy-3-methoxy-9a-methyl-9-oxo-N-(2,3,5,6-tetramethylbenzyl)-9,9a-dihydrodibenzo[b,d]furan-4-carboxamide (compound No. 1-116),
(9aS)-8-acetyl-N-(2-ethyl-3,5,6-trimethylbenzyl)-1,7-dihydroxy-3-methoxy-9a-methyl-9-oxo-9,9a-dihydrodibenzo[b,d]furan-4-carboxamide (compound No. 1-119),
(9aS)-8-acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide (compound No. 1-121),
(9aS)-8-acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide (compound No. 1-134),
(9aS)-8-acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide (compound No. 1-135),
(9aS)-8-acetyl-N-{4-[(2-butynyloxy)methyl]-2,3,6-trimethylbenzyl}-1,7-dihydroxy-3-methoxy-9a-methyl-9-oxo-9,9a-dihydrodibenzo[b,d]furan-4-carboxamide (compound No. 1-139),
(9aS)-8-acetyl-N-{4-[(2,4-dichlorobenzyl)oxy]-2,3,6-trimethylbenzyl}-1,7-dihydroxy-3-methoxy-9a-methyl-9-oxo-9,9a-dihydrodibenzo[b,d]furan-4-carboxamide (compound No. 1-149),
(9aS)-8-acetyl-N-[4-(2-butynyloxy)-2,3,5,6-tetramethylbenzyl]-1,7-dihydroxy-3-methoxy-9a-methyl-9-oxo-9,9a-dihydrodibenzo[b,d]furan-4-carboxamide (compound No. 1-172),
(9aS)-8-acetyl-1,7-dihydroxy-3-methoxy-9a-methyl-9-oxo-N-[2,3,5,6-tetramethyl-4-(2-pentynyloxy)benzyl]-9,9a-dihydrodibenzo[b,d]furan-4-carboxamide (compound No. 1-173),
(9aS)-8-acetyl-1,7-dihydroxy-3-methoxy-9a-methyl-N-(1-naphthylmethyl)-9-oxo-9,9a-dihydrodibenzo[b,d]furan-4-carboxamide (compound No. 1-199),
(9aS)-8-acetyl-N-[(4-fluoro-1-naphthyl)methyl]-1,7-dihydroxy-3-methoxy-9a-methyl-9-oxo-9,9a-dihydrodibenzo[b,d]furan-4-carboxamide (compound No. 1-203),
(9aS)-8-acetyl-N-[(2-chloro-1-naphthyl)methyl]-1,7-dihydroxy-3-methoxy-9a-methyl-9-oxo-9,9a-dihydrodibenzo[b,d]furan-4-carboxamide (compound No. 1-204),
(9aS)-8-acetyl-1,7-dihydroxy-3-methoxy-9a-methyl-N-[(2-methyl-1-naphthyl)methyl]-9-oxo-9,9a-dihydrodibenzo[b,d]furan-4-carboxamide (compound No. 1-210),
(9aS)-8-acetyl-N-[(2-ethyl-1-naphthyl)methyl]-1,7-dihydroxy-3-methoxy-9a-methyl-9-oxo-9,9a-dihydrodibenzo[b,d]furan-4-carboxamide (compound No. 1-213),
(9aS)-8-acetyl-N-[(2-butyl-1-naphthyl)methyl]-1,7-dihydroxy-3-methoxy-9a-methyl-9-oxo-9,9a-dihydrodibenzo[b,d]furan-4-carboxamide (compound No. 1-222);
(9aS)-8-acetyl-1,7-dihydroxy-3-methoxy-9a-methyl-N-[(3-butyl-1-naphthyl)methyl]-9-oxo-9,9a-dihydrodibenzo[b,d]furan-4-carboxamide (compound No. 1-223),
(9aS)-8-acetyl-N-{[2-(difluoromethyl)-naphthyl]methyl}-1,7-dihydroxy-3-methoxy-9a-methyl-9-oxo-9,9a-dihydrodibenzo[b,d]furan-4-carboxamide (compound No. 1-228),
(9aS)-8-acetyl-N-[(4-butoxy-1-naphthyl)methyl]-1,7-dihydroxy-3-methoxy-9a-methyl-9-oxo-9,9a-dihydrodibenzo[b,d]furan-4-carboxamide (compound No. I-281),
(9aS)-8-acetyl-N-{[4-(but-2-yn-1-yloxy)-1-naphthyl]methyl}-1,7-dihydroxy-3-methoxy-9a-methyl-9-oxo-9,9a-dihydrodibenzo[b,d]furan-4-carboxamide (compound No. 1-284),
(9aS)-8-acetyl-1,7-dihydroxy-3-methoxy-9a-methyl-9-oxo-N-{[4-(pent-2-yn-1-yloxy)-1-naphthyl]methyl}-9,9a-dihydrodibenzo[b,d]furan-4-carboxamide (compound No. 1-287),
methyl 4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthoate (compound No. 1-319),
(9aS)-8-acetyl-1,7-dihydroxy-3-methoxy-9a-methyl-9-oxo-N-[(4-phenoxy-1-naphthyl)methyl]-9,9a-dihydrodibenzo[b,d]furan-4-carboxamide (compound No. 1-403),
(9aS)-8-acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-407),
(9aS)-8-acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-408),
(9aS)-8-acetyl-N-{[4-(2-chlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-409),
(9aS)-8-acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-414),
(9aS)-8-acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide (compound No. 1-419),
(9aS)-8-acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-420),
(9aS)-8-acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-421),
(9aS)-8-acetyl-N-({4-[(4-chlorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-423),
(9aS)-8-acetyl-N-({4-[(3-chlorobenzyl)oxy]-1-naphthyl}methyl)-1,7-dbydroxy-3-methoxy-9a-methyl-9-oxo-9,9a-dihydrodibenzo[b,d]furan-4-carboxamide (compound No. 1-424),
(9aS)-8-acetyl-1,7-dihydroxy-3-methoxy-9a-methyl-9-oxo-N-[(4-{[4-(trifluoromethyl)benzyl]oxy}-1-naphthyl)methyl]-9,9a-dihydrodibenzo[b,d]furan-4-carboxamide (compound No. 1-426),
(9aS)-8-acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-429),
(9aS)-8-acetyl-N-({4-[(3-chloro-4-fluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-431),
(9aS)-8-acetyl-1,7-dihydroxy-3-methoxy-9a-methyl-9-oxo-N-({4-[(phenylsulfonyl)amino]-1-naphthyl}methyl)-9,9a-dihydrodibenzo[b,d]furan-4-carboxamide (compound No. 1-433),
(9aS)-8-acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamide (compound No.1-435),
(9aS)-8-acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide (compound No. 1-437),
(9aS)-8-acetyl-N-{[4-(2-butynyloxy)-2-methyl-1-naphthyl]methyl}-1,7-dihydroxy-3-methoxy-9a-methyl-9-oxo-9,9a-dihydrodibenzo[b,d]furan-4-carboxamide (compound No. 1-442),
(9aS)-8-acetyl-N-{[2-methyl-4-(2-pentynyloxy)-1-naphthyl]methyl}--1,7-dihydroxy-3-methoxy-9a-methyl-9-oxo-9,9a-dihydrodibenzo[b,d]furan-4-carboxamide (compound No. 1-443),
(9aS)-8-acetyl-N-({4-[(4-fluorobenzyl)oxy]-2-methyl-1-naphthyl}methyl)-1,7-dihydroxy-3-methoxy-9a-methyl-9-oxo-9,9a-dihydrodibenzo[b,d]furan-4-carboxamide (compound No. 1-444),
(9aS)-8-acetyl-N-({4-[(2-fluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-446),
(9aS)-8-aceTyl-N-({4-[(3-chlorobenzyl)oxy]-2-methyl-1-naphthyl}methyl)-1,7-dihydroxy-3-methoxy-9a-methyl-9-oxo-9,9a-dihydrodibenzo[b,d]furan-4-carboxamide (compound No. 1-448),
(9aS)-8-acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide (compound No. 1-450),
(9aS)-8-acetyl-N-[(2,3-dimethyl-4-propoxy-1-naphthyl)methyl]-1,7-dihydroxy-3-methoxy-9a-methyl-9-oxo-9,9a-dihydrodibenzo[b,d]furan-4-carboxamide (compound No. 1-453),
(9aS)-8-acetyl-N-{[4-(2-butynyloxy)-2,3-dimethyl-1-naphthyl]methyl}-1,7-dihydroxy-3-methoxy-9a-methyl-9-oxo-9,9a-dihydrodibenzo[b,d]furan-4-carboxamide (compound No. 1-454),
(9aS)-8-acetyl-N-{[2,3-dimethyl-4-(2-pentynyloxy)-1-naphthyl]methyl}-1,7-dihydroxy-3-methoxy-9a-methyl-9-oxo-9,9a-dihydrodibenzo[b,d]furan-4-carboxamide (compound No. 1-455),
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide (compound No. 1-492),
(9aS)-8-acetyl-N-[(2-ethyl-4-methyl-1-naphthyl)methyl]-1,7-dihydroxy-3-methoxy-9a-methyl-9-oxo-9,9a-dihydrodibenzo[b,d]furan-4-carboxamide (compound No. 1-494),
(9aS)-8-acetyl-3-ethoxy-1,7-dihydroxy-9a-methyl-N-[(2-methyl-1-naphthyl)methyl]-9-oxo-9,9a-dihydrodibenzo[b,d]furan-4-carboxamide (compound No. 2-68),
(9aS)-8-acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide (compound No. 2-73),
(9aS)-N-[4-(but-2-ynyloxy)-2,3,6-trimethylbenzyl]-1,7-dihydroxy-3-methoxy-8-[(1E)-N-methoxyethaneimidoyl]-9a-methyl-9-oxo-9,9a-dihydrodibenzo[b,d]furan-4-carboxamide (compound No. 3-14),
(9aS)-1,7-dihydroxy-3-methoxy-8-[(1E)-N-methoxyethaneimidoyl]-9a-methyl-N-[(2-methyl-1-naphthyl)methyl]-9-oxo-9,9a-dihydrodibenzo[b,d]furan-4-carboxamide (compound No. 3-18),
(9aS)-8-[(1E)-N-ethoxyethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (compound No. 3-24),
(9aS)-1,7-dihydroxy-3-methoxy-9a-methyl-N-[(2-methyl-1-naphthyl)methyl]-9-oxo-8-[(1E)-N-propoxyethaneimidoyl]-9,9a-dihydrodibenzo[b,d]furan-4-carboxamide (compound No. 3-28),
(9aS)-8-[(1E)-N-(allyloxy) ethaneimidoyl]-N-(4-{[(2,4-dichlorophenyl) sulfonyl] amino}-2,6-dimethylbenzyl)-1,7-dihydroxy-3-methoxy-9a-methyl-9-oxo-9,9a-dihydrodibenzo[b,d]furan-4-carboxamide (compound No. 3-39),
(9aS)-8-[(1E)-N-(allyloxy)ethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (compound No. 3-48),
(9aS)-8-[(1E)-N-(cyclopropylmethoxy)ethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (compound No. 3-80) or
(9aS)-1,7-dihydroxy-3-methoxy-9a-methyl-8-[(1E)-N-methylethaneimidoyl]-N-[(2-methyl-1-naphthyl)methyl]-9-oxo-9,9a-dihydrodibenzo[b,d]furan-4-carboxamide (compound No. 3-104), and
a still more preferable compound is
(9aS)-8-acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide (compound No. 1-76),
(9aS)-8-acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide (compound No. 1-77),
(9aS)-8-acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (compound No. 1-92),
(9aS)-8-acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (compound No. 1-93),
(9aS)-8-acetyl-1,7-dihydroxy-3-methoxy-9a-methyl-9-oxo-N-(2,3,5,6-tetramethylbenzyl)-9,9a-dihydrodibenzo[b,d]furan-4-carboxamide (compound No. 1-116),
(9aS)-8-acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide (compound No. 1-121),
(9aS)-8-acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide (compound No. 1-134),
(9aS)-8-acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide (compound No. 1-135),
(9aS)-8-acetyl-1,7-dihydroxy-3-methoxy-9a-methyl-N-(1-naphthylmethyl)-9-oxo-9,9a-dihydrodibenzo[b,d]furan-4-carboxamide (compound No. 1-199),
(9aS)-8-acetyl-N-[(4-fluoro-1-naphthyl)methyl]-1,7-dihydroxy-3-methoxy-9a-methyl-9-oxo-9,9a-dihydrodibenzo[b,d]furan-4-carboxamide (compound No. 1-203),
(9aS)-8-acetyl-1,7-dihydroxy-3-methoxy-9a-methyl-N-[(2-methyl-1-naphthyl)methyl]-9-oxo-9,9a-dihydrodibenzo[b,d]furan-4-carboxamide (compound No. 1-210),
(9aS)-8-acetyl-N-[(2-ethyl-1-naphthyl)methyl]-1,7-dihydroxy-3-methoxy-9a-methyl-9-oxo-9,9a-dihydrodibenzo[b,d]furan-4-carboxamide (compound No. 1-213),
(9aS)-8-acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-407),
(9aS)-8-acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-408),
(9aS)-8-acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide (compound No. 1-414),
(9aS)-8-acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide (compound No. 1-419),
(9aS)-8-acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-420),
(9aS)-8-acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-421),
(9aS)-8-acetyl-N-({4-[(3-chlorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-424),
(9aS)-8-acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide (compound No. 1-429),
(9aS)-8-acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamide (compound No. 1-435),
(9aS)-8-acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide (compound No. 1-437),
(9aS)-8-acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide (compound No. 1-450),
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide (compound No. 1-492),
(9aS)-8-acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide (compound No. 2-73),

(9aS)-1,7-dihydroxy-3-methoxy-8-[(1E)-N-methoxyethaneimidoyl]-9a-methyl-N-[(2-methyl-1-naphthyl)methyl]-9-oxo-9,9a-dihydrodibenzo[b,d]furan-4-carboxamide (compound No. 3-18) or
(9aS)-8-[(1E)-N-(allyloxy) ethaneimidoyl]-N-(4-{[(2,4-dichlorophenyl) sulfonyl] amino}-2,6-dimethylbenzyl)-1,7-dihydroxy-3-methoxy-9a-methyl-9-oxo-9,9a-dihydrodibenzo[b,d]furan-4-carboxamide (compound No. 3-39).

In Table 1, a preferable compound is a compound No. 1-492, 1-494, 1-545, 1-546, 1-547, 1-548, 1-549, 1-551, 1-553, 1-554, 1-557, 1-563, 1-566, 1-567, 1-572, 1-597, 1-613, 1-615, 1-628, 1-630, 1-631, 1-632, 1-637, 1-641 or 1-643, a more preferable compound is
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide (compound No. 1-492),
(9aS)-8-acetyl-N-[(2-ethyl-4-methyl-1-naphthyl)methyl]-1,7-dihydroxy-3-methoxy-9a-methyl-9-oxo-9,9a-dihydrodibenzo[b,d]furan-4-carboxamide (compound No. 1-494),
(9aS)-8-acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide (compound No. 1-545),
(9aS)-8-acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide (compound No. 1-548),
(9aS)-8-acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide (compound No. 1-563),
(9aS)-8-acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide (compound No. 1-566),
(9aS)-8-acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide (compound No. 1-567),
(9aS)-8-acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide (compound No. 1-572),
(9aS)-8-acetyl-N-[(2,7-dimethyl-1-naphthyl)methyl]-1,7-dihydroxy-3-methoxy-9a-methyl-9-oxo-9,9a-dihydrodibenzo[b,d]furan-4-carboxamide (compound No. 1-597),
(9aS)-8-acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide (compound No. 1-613),
(9aS)-8-acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide (compound No. 1-628),
(9aS)-8-acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide (compound No. 1-630),
(9aS)-8-acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide (compound No. 1-631),
(9aS)-8-acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide (compound No. 1-632),
(9aS)-8-acetyl-N-[(2-ethyl-4-fluoro-7-methyl-1-naphthyl)methyl]-1,7-dihydroxy-3-methoxy-9a-methyl-9-oxo-9,9a-dihydrodibenzo[b,d]furan-4-carboxamide (compound No. 1-637),
(9aS)-8-acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide (compound No. 1-641) or
(9aS)-8-acetyl-N-[(2-ethyl-5,6,7-trifluoro-1-naphthyl)methyl]-1,7-dihydroxy-3-methoxy-9a-methyl-9-oxo-9,9a-dihydrodibenzo[b,d]furan-4-carboxamide (compound No. 1-643), and
a still more preferable compound is
(9aS)-8-acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide (compound No. 1-545),
(9aS)-8-acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide (compound No. 1-548),
(9aS)-8-acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide (compound No. 1-563),
(9aS)-8-acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide (compound No. 1-566),
(9aS)-8-acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide (compound No. 1-567),
(9aS)-8-acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide (compound No. 1-572),
(9aS)-8-acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide (compound No. 1-613),
(9aS)-8-acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide (compound No. 1-628),

(9aS)-8-acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide (compound No. 1-630),
(9aS)-8-acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide (compound No. 1-631),
(9aS)-8-acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide (compound No. 1-632) or
(9aS)-8-acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide (compound No. 1-641).

Effect of the Invention

The cercosporamide derivative having the general formula (I) according to the present invention, a pharmacologically acceptable salt thereof or an ester thereof has an excellent hypoglycemic effect and can be used as a medicine for treatment and/or prevention of diabetes (in particular, type II diabetes).

BEST MODE FOR CARRYING OUT THE INVENTION

The compound having the general formula (I) according to the present invention can be produced according to the method described below.
Method A is a method for producing a compound having the general formula (I-A).

Method A

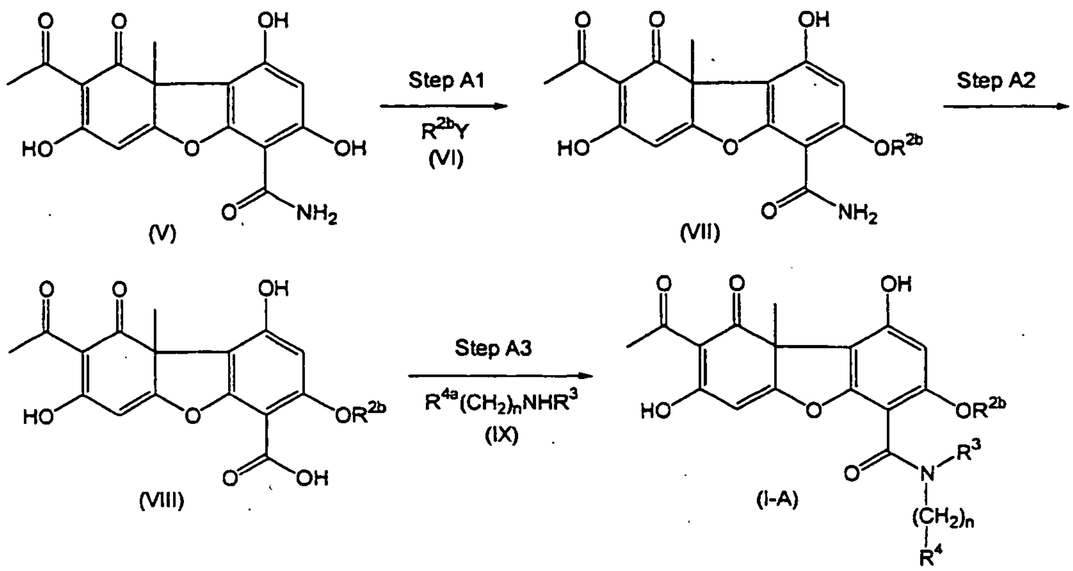

(V) (VII) (VIII) (I-A)

In the present invention, $R^3$, $R^4$ and n are as defined above, $R^{2b}$ represents a $C_1$-$C_6$ alkyl group or a $C_1$-$C_6$ halogenated alkyl group, $R^{4a}$ represents the same group as $R^4$ except that the amino group, the hydroxyl group and/or the carboxyl group contained in $R^4$ as a substituent is an amino, hydroxyl and/or carboxyl group which may be protected, and Y represents a halogen atom, a $C_1$-$C_6$ alkylsulfonyloxy group, a $C_1$-$C_6$ alkoxysulfonyloxy group or a $C_6$-$C_{10}$ arylsulfonyloxy group (preferably a halogen atom, more preferably a chlorine atom, a bromine atom or an iodine atom, and still more preferably a bromine atom or an iodine atom).
The protecting group for the "amino group which may be protected", "hydroxy group which may be protected" and "carboxyl group which may be protected" as defined above for $R^{4a}$ refers to a protecting group that can be cleaved by a chemical method such as hydrogenolysis, hydrolysis, electrolysis or photolysis and represents a protecting group generally used in the organic synthesis chemistry (see T. W. Greene et al., Protective Groups in Organic Synthesis, 3rd Edition, John Wiley & Sons, Inc. 1999, for example).
The "protecting group" for the "hydroxy group which may be protected" as defined above for $R^{4a}$ is not particularly limited so long as it is a protecting group for a hydroxy group used in the field of organic synthesis chemistry; the protecting

group is the same as the "general protecting group for an ester of a hydroxy group", for example.

The "protecting group" for the "carboxyl group which may be protected" as defined above for $R^{4a}$ is not particularly limited so long as it is a protecting group for a carboxyl group used in the field of organic synthesis chemistry; the protecting group is a "general protecting group for an ester of a carboxyl group", for example, and is preferably the aforementioned "$C_1$-$C_6$ alkyl group"; the aforementioned "$C_2$-$C_6$ alkenyl group"; the aforementioned "$C_2$-$C_6$ alkynyl group"; the aforementioned "$C_1$-$C_6$ halogenated alkyl group"; a hydroxy "$C_1$-$C_6$ alkyl group" such as 2-hydroxyethyl, 2,3-dihydroxypropyl, 3-hydroxypropyl, 3,4-dihydroxybutyl or 4-hydroxybutyl; a "$C_2$-$C_7$ alkylcarbonyl"-"$C_1$-$C_6$ alkyl group" such as acetylmethyl; the aforementioned "aralkyl group"; or the aforementioned "silyl group", and more preferably a $C_1$-$C_6$ alkyl group or an aralkyl group.

The "protecting group" for the "amino group which may be protected" as defined above for $R^{4a}$ is not particularly limited so long as it is a protecting group for an amino group used in the field of organic synthesis chemistry; the protecting group is the same "alkylcarbonyl group"; "arylcarbonyl group"; "alkoxycarbonyl group"; "alkenyloxycarbonyl group"; "aralkyloxycarbonyl group"; "silyl group"; or "aralkyl group" as in the aforementioned "general protecting group for an ester of a hydroxy group" or a "substituted methylene group forming a Schiff base" such as N,N-dimethylaminomethylene, benzylidene, 4-methoxybenzylidene, 4-nitrobenzylidene, salicylidene, 5-chlorosalicylidene, diphenylmethylene or (5-chloro-2-hydroxyphenyl)phenylmethylene, for example, and is preferably an alkylcarbonyl group, an arylcarbonyl group or an alkoxycarbonyl group, and most preferably an alkoxycarbonyl group.

Step A1

This step is a step of producing a compound having the general formula (VII). (i) The case where a compound having the general formula (VI) is a solid or liquid

This step is carried out by reacting a known mycotoxin, cercosporamide (V) [Journal of Organic Chemistry, 1991, Vol. 56, p.909-910, for example] isolated from the natural environment with a compound having the general formula (VI) which is a known compound or is easily obtained from a known compound as a starting material by a method similar to a known method, in an inert solvent in the presence of a base in an argon or nitrogen atmosphere.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene; toluene and xylene; and mixed solvents thereof. The inert solvent is preferably an amide, sulfoxide or nitrile, and more preferably N,N-dimethylformamide or N,N-dimethylacetamide.

Examples of the base used in this step include alkali metal carbonates such as sodium carbonate, potassium carbonate and lithium carbonate; alkali metal bicarbonates such as sodium bicarbonate, potassium bicarbonate and lithium bicarbonate; alkali metal hydrides such as lithium hydride, sodium hydride and potassium hydride; alkali metal hydroxides such as sodium hydroxide, potassium hydroxide, barium hydroxide and lithium hydroxide; alkali metal alkoxides such as sodium methoxide, sodium ethoxide, sodium t-butoxide, potassium methoxide, potassium ethoxide, potassium t-butoxide and lithium methoxide; and organometallic bases such as butyllithium, lithium diisopropylamide and lithium bis(trimethylsilyl)amide. The base is preferably an alkali metal carbonate, alkali metal bicarbonate, alkali metal hydride, alkali metal hydroxide or organometallic base, and more preferably sodium carbonate, potassium carbonate or cesium carbonate.

The reaction temperature in this step varies depending on the raw material compound, the inert solvent used and the like, but is usually 0˚C to 100˚C, and preferably 20˚C to 80˚C.

The reaction time in this step varies depending on the raw material compound, the inert solvent used, the reaction temperature and the like, but is usually 1 hour to 96 hours, and preferably 2 hours to 72 hours.

(ii) The case where a compound having the general formula (VI) is a gas

This step is carried out by reacting cercosporamide (V) with a compound having the general formula (VI) which is a known compound or is easily obtained from a known compound as a starting material by a method similar to a known method, in an inert solvent in the presence of a base in an argon or nitrogen atmosphere.

The inert solvent used in this step is not particularly limited so long as it does not inhibit the reaction and allows the starting material to be dissolved therein to a certain extent. Examples of the inert solvent include hydrocarbons such

as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably an ether, nitrile, ketone or halogenated hydrocarbon, and more preferably dichloromethane or 1,2-dichloroethane. A mixed solvent with water (whose mixing ratio is 1:100 to 100:1, and preferably 1:10 to 10:1) may be used as necessary.

Examples of the base used in this step include alkali metal carbonates such as sodium carbonate, potassium carbonate and lithium carbonate; alkali metal bicarbonates such as sodium bicarbonate, potassium bicarbonate and lithium bicarbonate; alkali metal hydrides such as lithium hydride, sodium hydride and potassium hydride; and alkali metal hydroxides such as sodium hydroxide, potassium hydroxide, barium hydroxide and lithium hydroxide. The base is preferably an alkali metal carbonate, alkali metal bicarbonate or alkali metal hydroxide, and more preferably sodium hydroxide or potassium hydroxide.

The reaction temperature in this step varies depending on the raw material compound, the inert solvent used and the like, but is usually 0˚C to 80˚C, and preferably 10˚C to 50˚C.

The reaction time in this step varies depending on the raw material compound, the inert solvent used, the reaction temperature and the like, but is usually 1 hour to 96 hours, and preferably 2 hours to 72 hours.

Step A2

This step is a step of producing a compound having the general formula (VIII). This step is a step of reacting the compound having the general formula (VII) with sodium nitrite in an inert solvent in the presence of sulfuric acid and is carried out in accordance with the method described in Journal of Organic Chemistry, 1995, Vol. 60, p.7739-7746, for example.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; nitriles such as acetonitrile and isobutyronitrile; aromatic hydrocarbons such as benzene, toluene and xylene; and mixed solvents thereof. The inert solvent is preferably a hydrocarbon, ether or nitrile, and more preferably dioxane or acetonitrile. A mixed solvent with water (whose mixing ratio is 1:100 to 100:1, and preferably 1:2 to 2:1) maybe used as necessary.

The reaction temperature in this step varies depending on the raw material compound, the inert solvent used and the like, but is usually -5˚C to 80˚C, and preferably 0˚C to 40˚C.

The reaction time in this step varies depending on the raw material compound, the inert solvent used, the reaction temperature and the like, but is usually 1 hour to 24 hours, and preferably 2 hours to 10 hours.

Step A3

This step is a step of producing a compound having the general formula (I-A) and consists of (i) to (ii).

(i) This step is carried out by reacting the compound having the general formula (VIII) with a compound having the general formula (IX) which is a known compound or is easily obtained from a known compound as a starting material by a method similar to a known method, in an inert solvent in the presence of a condensing agent and in the presence or absence of a base.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably an amide, ether or halogenated hydrocarbon, and more preferably N,N-dimethylformamide, tetrahydrofuran or dichloromethane.

Examples of the condensing agent used in this step include 1-propanephosphonic acid cyclic anhydride (T3P), dicyclohexylcarbodiimide (DCCD), 1-ethyl-3-(3-dimethylaminopropyl)carbodiimide hydrochloride (EDCI), isobutyl chloroformate (IBCF), 1,1'-carbonylbis-1H-imidazole (CDI), diethylphosphoryl cyanide (DEPC) and diphenylphosphoryl azide (DPPA). The condensing agent may be present together with 1-hydroxybenzotriazole as necessary and is preferably EDCI.

Examples of the base used in this step include N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU). The base is preferably triethylamine, diisopropylethylamine or 4-(N,N-dimethylamino)pyridine.

The reaction temperature in this step varies depending on the raw material compound, the inert solvent used and the like, but is usually -10°C to 100°C, and preferably 0°C to 50°C.

The reaction time in this step varies depending on the raw material compound, the inert solvent used, the reaction temperature and the like, but is usually 1 hour to 48 hours, and preferably 2 hours to 24 hours.

(ii) This step is carried out by reacting the compound obtained in (i) above with a base in an inert solvent.

The inert solvent used in this step is not particularly limited so long as it does not inhibit the reaction and allows the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; water; and mixed solvents thereof. The inert solvent is preferably tetrahydrofuran, dioxane, water or a mixed solvent thereof. A mixed solvent with water has a mixing ratio of 1:100 to 100:1, and preferably 1:10 to 10:1.

Examples of the base used in this step include alkali metal carbonates such as sodium carbonate, potassium carbonate and lithium carbonate; alkali metal bicarbonates such as sodium bicarbonate, potassium bicarbonate and lithium bicarbonate; alkali metal hydroxides such as sodium hydroxide, potassium hydroxide, barium hydroxide and lithium hydroxide; and alkali metal alkoxides such as sodium methoxide, sodium ethoxide, sodium t-butoxide, potassium methoxide, potassium ethoxide, potassium t-butoxide and lithium methoxide. The base is preferably an alkali metal carbonate, alkali metal bicarbonate or alkali metal hydroxide, and more preferably sodium hydroxide or potassium hydroxide.

The reaction temperature in this step varies depending on the raw material compound, the inert solvent used and the like, but is usually -10°C to 100°C, and preferably 0°C to 40°C.

The reaction time in this step varies depending on the raw material compound, the inert solvent used, the reaction temperature and the like, but is usually 0.5 hour to 72 hours, and preferably 1 hour to 24 hours.

Deprotection reaction of the protecting group for the amino group, hydroxy group and/or carboxyl group in $R^{4a}$ may be further carried out in order to obtain a desired compound after completion of the above reaction. The deprotection reaction is carried out in accordance with a known method (such as the method described in Theodora W. Greene and Peter G. M. Wuts, "Protective Groups in Organic Synthesis", 1999, A Wiley-Interscience Publication).

Method B is a method for producing a compound having the general formula (I-B).

Method B

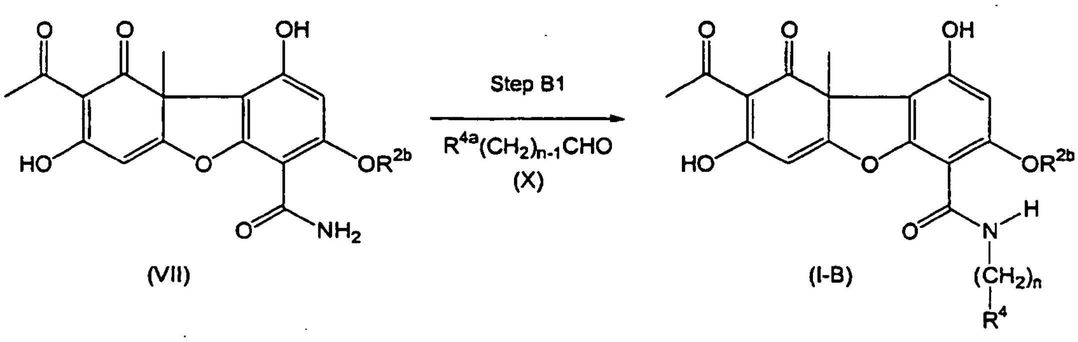


(VII) (I-B)

In the present invention, $R^4$, $R^{2b}$, $R^{4a}$ and n are as defined above.

Step B1

This step is a step of producing a compound having the general formula (I-B).

This step is a step of reacting the compound having the general formula (VII) obtained in the above Step A1 of Method A with a compound having the general formula (X) which is a known compound or is easily obtained from a known compound as a starting material by a method similar to a known method and a hydrosilane in an inert solvent in the presence of an acid, and is carried out according to the method described in Tetrahedron Lett. 1999, Vol. 40, p. 2295-2298, for example.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; nitriles such as acetonitrile and isobutyronitrile; esters such as ethyl formate, ethyl acetate, propyl acetate, butyl acetate and diethyl carbonate; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably a nitrile, halogenated hydrocarbon or aromatic hydrocarbon, and more preferably acetonitrile, dichloromethane or toluene.

Examples of the acid used in this step include organic sulfonic acids such as methanesulfonic acid, p-toluenesulfonic acid, camphorsulfonic acid and trifluoromethanesulfonic acid; carboxylic acids such as acetic acid, formic acid and trifluoroacetic acid; Lewis acids such as zinc chloride, tin tetrachloride, boron trifluoride and boron tribromide; and acidic ion exchange resins. The acid is preferably a carboxylic acid, and more preferably trifluoroacetic acid.

The hydrosilane used in this step is trichlorosilane, trimethylsilane, triethylsilane, tributylsilane, ethyldimethylsilane, diethylmethylsilane or t-butyldimethylsilane, and preferably triethylsilane.

The reaction temperature in this step varies depending on the raw material compound, the inert solvent used and the like, but is usually 0˚C to 120˚C, and preferably 10˚C to 110˚C.

The reaction time in this step varies depending on the raw material compound, the inert solvent used, the reaction temperature and the like, but is usually 1 hour to 72 hours, and preferably 4 hours to 48 hours.

Deprotection reaction of the protecting group for the amino group, hydroxy group and/or carboxyl group in $R^{4a}$ may be further carried out in order to obtain a desired compound after completion of the above reaction.

Method C is a method for producing a compound having the general formula (I-C).

Method C

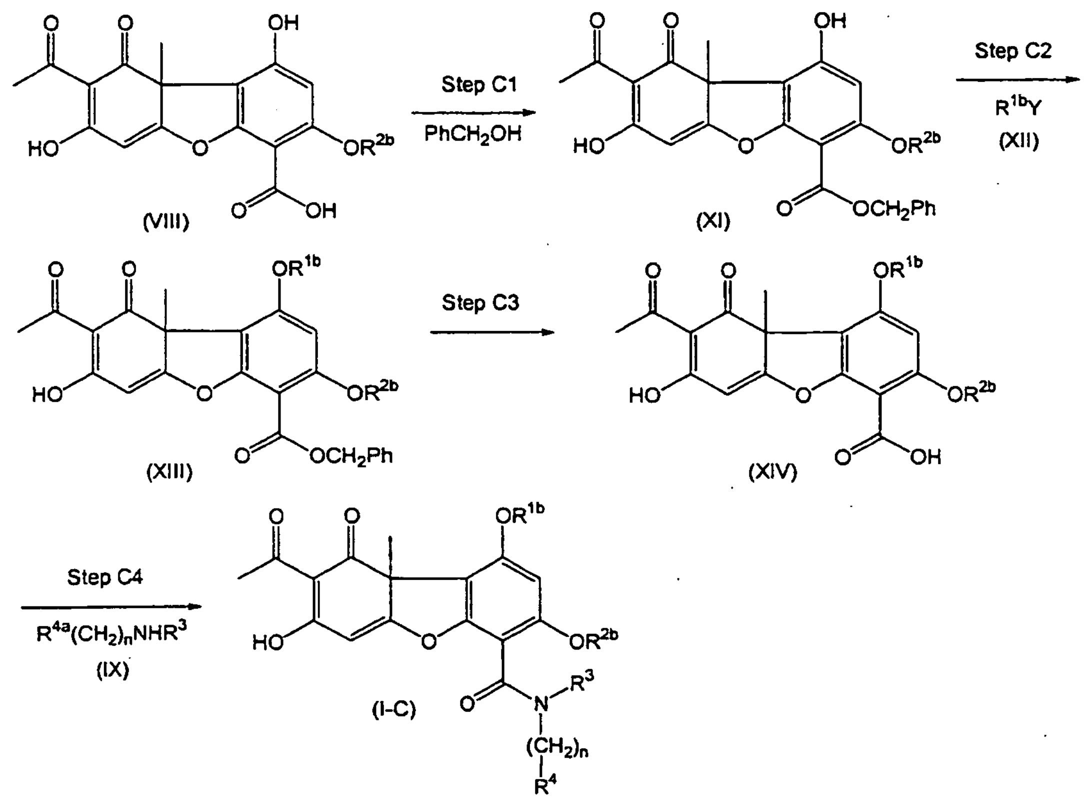


(VIII) (XI) (XIII) (XIV) (I-C)

In the present invention, $R^3$, $R^4$, $R^{2b}$, $R^{4a}$, Y and n are as defined above, and $R^{1b}$ represents a $C_1$-$C_6$ alkyl group.

Step C1

This step is a step of producing a compound having the general formula (XI).

This step is carried out by reacting the compound having the general formula (VIII) obtained in the above Step A2 of Method A with benzyl alcohol in an inert solvent in the presence of a base and a condensing agent.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof The inert solvent is preferably an amide or ether, and more preferably N,N-dimethylformamide or tetrahydrofuran.

Examples of the base used in this step include N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU). The base is preferably triethylamine, diisopropylethylamine or 4-(N,N-dimethylamino)pyridine.

Examples of the condensing agent used in this step include 1-propanephosphonic acid cyclic anhydride (T3P), dicyclohexylcarbodiimide (DCCD), 1-ethyl-3-(3-dimethylaminopropyl)carbodiimide hydrochloride (EDCI), isobutyl chloroformate (IBCF), 1,1'-carbonylbis-1H-imidazole (CDI), diethylphosphoryl cyanide (DEPC) and diphenylphosphoryl azide (DPPA). The condensing agent is preferably DCCD or EDCI, and more preferably EDCI.

The reaction temperature in this step varies depending on the raw-material compound, the inert solvent used and the like, but is usually -20°C to 100°C, and preferably-10°C to 40°C.

The reaction time in this step varies depending on the raw material compound, the inert solvent used, the

reaction temperature and the like, but is usually 0.5 hour to 48 hours, and preferably 1 hour to 24 hours.

Step C2

This step is a step of producing a compound having the general formula (XIII).

This step is carried out by reacting the compound having the general formula (XI) with a compound having the general formula (XII) which is a known compound or is easily obtained from a known compound as a starting material by a method similar to a known method, in an inert solvent in the presence of a base in an argon or nitrogen atmosphere.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; halogenated hydrocarbons such as 1,2-dichloroethane, dichlorobenzene, chloroform and carbon tetrachloride; aromatic hydrocarbons such as benzene, toluene and xylene; and mixed solvents thereof. The inert solvent is preferably an amide or ether, and more preferably N,N-dimethylformamide or tetrahydrofuran.

Examples of the base used in this step include alkali metal carbonates such as sodium carbonate, potassium carbonate and lithium carbonate; alkali metal bicarbonates such as sodium bicarbonate, potassium bicarbonate and lithium bicarbonate; alkali metal hydrides such as lithium hydride, sodium hydride and potassium hydride; alkali metal hydroxides such as sodium hydroxide, potassium hydroxide, barium hydroxide and lithium hydroxide; and organometallic bases such as butyllithium, lithium diisopropylamide and lithium bis(trimethylsilyl)amide. The base is preferably an alkali metal hydride, and more preferably sodium hydride or potassium hydride.

The reaction temperature in this step varies depending on the raw material compound, the inert solvent used and the like, but is usually -20˚C to 100˚C, and preferably-10˚C to 50˚C.

The reaction time in this step varies depending on the raw material compound, the inert solvent used, the reaction temperature and the like, but is usually 0.5 hour to 48 hours, and preferably 1 hour to 24 hours.

Step C3

This step is a step of producing a compound having the general formula (XIV).

This step is carried out by reducing the compound having the general formula (XIII) in an inert solvent in the presence of a palladium catalyst in a hydrogen atmosphere.

The inert solvent used in this step is not particularly limited so long as it does not inhibit the reaction and allows the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; esters such as ethyl formate, ethyl acetate, propyl acetate, butyl acetate and diethyl carbonate; and mixed solvents thereof. The inert solvent is preferably an ether, alcohol or ester, and more preferably tetrahydrofuran or ethyl acetate. A mixed solvent with water (whose mixing ratio is 1:100 to 100:1, and preferably 1:10 to 10:1) may be used as necessary.

The palladium catalyst used in this step is a divalent palladium catalyst or a zerovalent palladium catalyst, for example, and is preferably palladium-active carbon, palladium (II) acetate, palladium (II) trifluoroacetate, palladium black, palladium (II) bromide, palladium (II) chloride, palladium (II) iodide, palladium (II) cyanide, palladium (II) nitrate, palladium (II) oxide, palladium (II) sulfate, dichlorobis(acetonitrile)palladium (II), dichlorobis(benzonitrile)palladium (II), dichloro(1,5-cyclooctadiene)palladium (II), acetylacetone palladium (II), palladium (II) sulfide, dichloro[1,1'-bis(diphenylphosphino)ferrocene]palladium (II), tris(dibenzylideneacetone)dipalladium (0), tetrakis(acetonitrile)palladium (II) tetrafluoroborate or an aryl chloride-palladium dimer, and more preferably palladium-active carbon.

The reaction temperature in this step varies depending on the raw material compound, the inert solvent used and the like, but is usually 0˚C to 80˚C, and preferably 10˚C to 60˚C.

The reaction time in this step varies depending on the raw material compound, the inert solvent used, the reaction temperature and the like, but is usually 0.1 hour to 48 hours, and preferably 0.5 hour to 24 hours.

Step C4

This step is a step of producing a compound having the general formula (I-C) and consists of (i) to (ii).

(i) This step is carried out in the same manner as in (i) of the above Step A3 of Method A by reacting the compound having the general formula (XIV) with a compound having the general formula (IX) which is a known compound or is easily obtained from a known compound as a starting material by a method similar to a known method, in an inert solvent in the presence of a condensing agent.

(ii) This step is carried out in the same manner as in (ii) of the above Step A3 of Method A by reacting the compound obtained in (i) above with a base in an inert solvent.

Deprotection reaction of the protecting group for the amino group, hydroxy group and/or carboxyl group in $R^{4a}$ may be further carried out in order to obtain a desired compound after completion of the above reaction.

Method D is a method for producing a compound having the general formula (I-D).

Method D

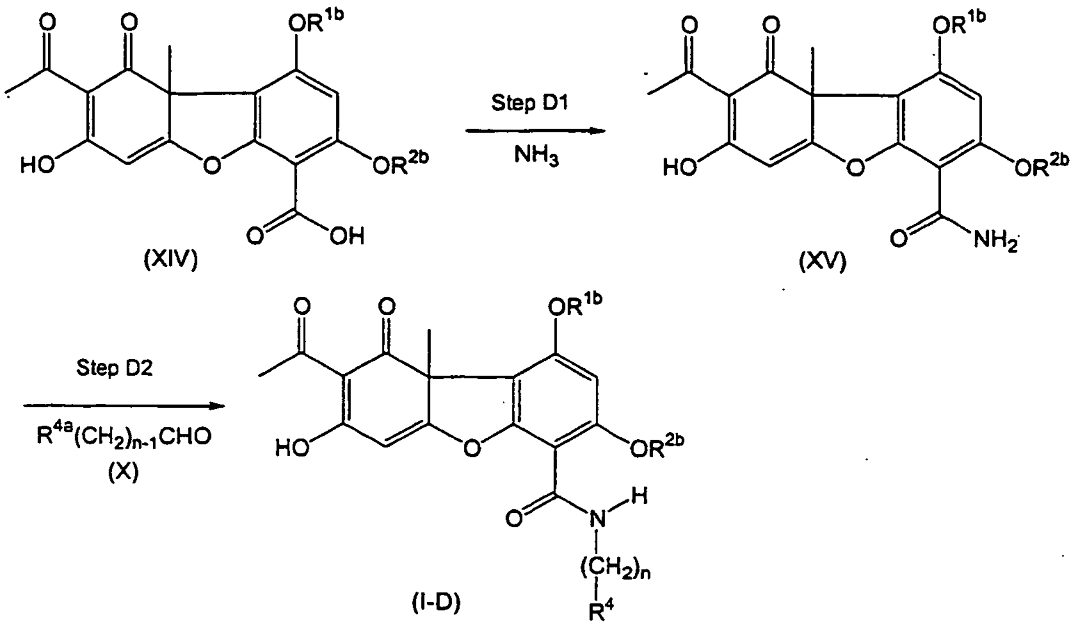


(XIV) (XV) (I-D)

In the present invention, $R^4$, $R^{1b}$, $R^{2b}$, $R^{4a}$ and n are as defined above.

Step D1

This step is a step of producing a compound having the general formula (XV).

This step is carried out by reacting the compound having the general formula (XIV) obtained in the above Step C3 of Method C with ammonia in an inert solvent in the presence of a condensing agent and in the presence or absence of a base.

The inert solvent used in this step is not particularly limited so long as it does not inhibit the reaction and allows the starting material to be dissolved therein to a certain extent. Examples of the inert solvent include amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; nitriles such as acetonitrile and isobutyronitrile; esters such as ethyl formate, ethyl acetate, propyl acetate, butyl acetate and diethyl carbonate; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably an amide, ether or alcohol, and more preferably N,N-dimethylformamide, tetrahydrofuran or i-propanol.

Examples of the condensing agent used in this step include 1-propanephosphonic acid cyclic anhydride (T3P), dicyclohexylcarbodiimide (DCCD), 1-ethyl-3-(3-dimethylaminopropyl)carbodiimide hydrochloride (EDCI), isobutyl chloroformate (IBCF), 1,1'-carbonylbis-1H-imidazole (CDI), diethylphosphoryl cyanide (DEPC) and diphenylphosphoryl azide

(DPPA). The condensing agent may be present together with 1-hydroxybenzotriazole as necessary and is preferably DCCD or EDCI, and more preferably EDCI.

Examples of the base used in this step include N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU). The base is preferably triethylamine, diisopropylethylamine or 4-(N,N-dimethylamino)pyridine.

The ammonia used in this step is aqueous ammonia, ammonia gas or a solution of ammonia in isopropanol, for example, and preferably a solution of ammonia in isopropanol.

The reaction temperature in this step varies depending on the raw material compound, the inert solvent used and the like, but is usually -20˚C to 80˚C, and preferably 0˚C to 60˚C.

The reaction time in this step varies depending on the raw material compound, the inert solvent used, the reaction temperature and the like, but is usually 0.2 hour to 12 hours, and preferably 0.5 hour to 6 hours.

Step D2

This step is a step of producing a compound having the general formula (I-D).

This step is carried out in the same manner as in the above Step B1 of Method B by reacting the compound having the general formula (XV) with a compound having the general formula (X) which is a known compound or is easily obtained from a known compound as a starting material by a method similar to a known method and a hydrosilane in an inert solvent in the presence of an acid.

Deprotection reaction of the protecting group for the amino group, hydroxy group and/or carboxyl group in $R^{4a}$ may be further carried out in order to obtain a desired compound after completion of the above reaction.

Method E is a method for producing a compound having the general formula (I-E).

Method E

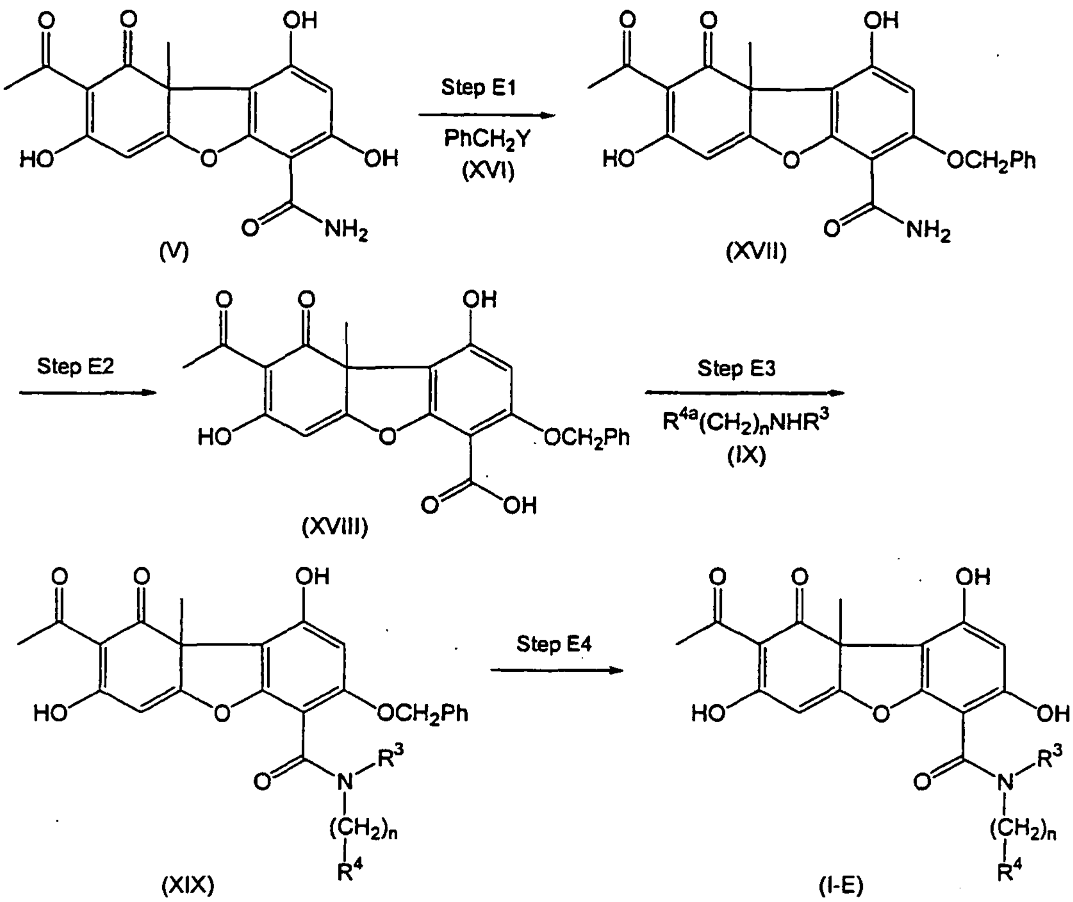


(V) (XVII) (XVIII) (XIX) (I-E)

In the present invention, $R^3$, $R^4$, $R^{4a}$, Y and n are as defined above.

Step E1

This step is a step of producing a compound (XVII).

This step is carried out by reacting a known mycotoxin, cercosporamide (V) [Journal of Organic Chemistry, 1991, Vol. 56, p.909-910, for example] isolated from the natural environment with a compound having the general formula (XVI) which is a known compound or is easily obtained from a known compound as a starting material by a method similar to a known method, in an inert solvent in the presence of a base in an argon or nitrogen atmosphere.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably an amide, sulfoxide or nitrile, and more preferably N,N-dimethylformamide.

Examples of the base used in this step include alkali metal carbonates such as sodium carbonate, potassium carbonate and lithium carbonate; alkali metal bicarbonates such as sodium bicarbonate, potassium bicarbonate and lithium bicarbonate; alkali metal hydrides such as lithium hydride, sodium hydride and potassium hydride; alkali metal hydroxides such as sodium hydroxide, potassium hydroxide, barium hydroxide and lithium hydroxide; alkali metal alkoxides such as sodium methoxide, sodium ethoxide, sodium t-butoxide, potassium methoxide, potassium ethoxide, potassium t-butoxide and lithium methoxide; and organometallic bases such as butyllithium, lithium diisopropylamide and lithium bis(trimethylsilyl)amide. The base is preferably an alkali metal carbonate, alkali metal bicarbonate, alkali metal

hydride, alkali metal hydroxide or organometallic base, and more preferably sodium carbonate, potassium carbonate or cesium carbonate.

The reaction temperature in this step varies depending on the raw material compound, the inert solvent used and the like, but is usually 0˚C to 100˚C, and preferably 20˚C to 80˚C.

The reaction time in this step varies depending on the raw material compound, the inert solvent used, the reaction temperature and the like, but is usually 1 hour to 96 hours, and preferably 2 hours to 72 hours.

Step E2

This step is a step of producing a compound (XVIII).

This step is carried out in the same manner as in the above Step A2 of Method A by reacting the compound (XVII) with sodium nitrite in an inert solvent in the presence of sulfuric acid.

Step E3

This step is a step of producing a compound having the general formula (XIX) and consists of (i) to (ii).

(i) This step is carried out in the same manner as in (i) of the above Step A3 of Method A by reacting the compound (XVIII) with a compound having the general formula (IX) which is a known compound or is easily obtained from a known compound as a starting material by a method similar to a known method, in an inert solvent in the presence of a condensing agent and in the presence or absence of a base.

(ii) This step is carried out in the same manner as in (ii) of the above Step A3 of Method A by reacting the compound obtained in (i) above with a base in an inert solvent.

Deprotection reaction of the protecting group for the amino group, hydroxy group and/or carboxyl group in $R^{4a}$ may be further carried out in order to obtain a desired compound after completion of the above reaction.

Step E4

This step is a step of producing a compound having the general formula (I-E).

This step is carried out by reducing the compound having the general formula (XIX) in an inert solvent in the presence of a palladium catalyst in a hydrogen atmosphere.

The inert solvent used in this step is not particularly limited so long as it does not inhibit the reaction and allows the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; esters such as ethyl formate, ethyl acetate, propyl acetate, butyl acetate and diethyl carbonate; and mixed solvents thereof. The inert solvent is preferably an ether, alcohol or ester, and more preferably ethanol or ethyl acetate. A mixed solvent with water (whose mixing ratio is 1:100 to 100:1, and preferably 1: 10 to 10: 1) may be used as necessary.

The palladium catalyst used in this step is a divalent palladium catalyst or a zerovalent palladium catalyst, for example, and is preferably palladium-active carbon, palladium (II) acetate, palladium (II) trifluoroacetate, palladium black, palladium (II) bromide, palladium (II) chloride, palladium (II) iodide, palladium (II) cyanide, palladium (II) nitrate, palladium (II) oxide, palladium (II) sulfate, dichlorobis(acetonitrile)palladium (II), dichlorobis(benzonitrile)palladium (II), dichloro(1,5-cyclooctadiene)palladium (II), acetylacetone palladium (II), palladium (II) sulfide, dichloro[1,1'-bis(diphenylphosphino)ferrocene]palladium (II), tris(dibenzylideneacetone)dipalladium (0), tetrakis(acetonitrile)palladium (II) tetrafluoroborate or an aryl chloride-palladium dimer, and more preferably palladium-active carbon.

The reaction temperature in this step varies depending on the raw material compound, the inert solvent used and the like, but is usually 10˚C to 80˚C, and preferably 20˚C to 70˚C.

The reaction time in this step varies depending on the raw material compound, the inert solvent used, the reaction temperature and the like, but is usually 0.5 hour to 48 hours, and preferably 1 hour to 36 hours.

Method F is a method for producing a compound having the general formula (I-F).

Method F

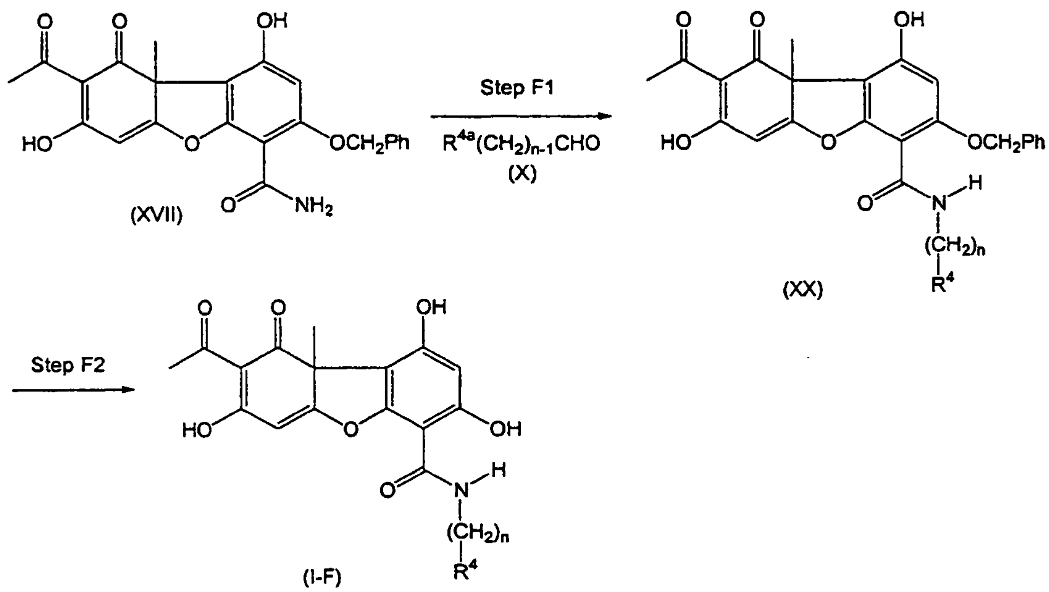

(XVII)
(XX)
(I-F)

In the present invention, $R^4$, $R^{4a}$ and n are as defined above.

Step F1

This step is a step of producing a compound having the general formula (XX).

This step is carried out in the same manner as in the above Step B1 of Method B by reacting the compound (XVII) obtained in the above Step E1 of Method E with a compound having the general formula (X) which is a known compound or is easily obtained from a known compound as a starting material by a method similar to a known method and a hydrosilane in an inert solvent in the presence of an acid.

Deprotection reaction of the protecting group for the amino group, hydroxy group and/or carboxyl group in $R^{4a}$ may be further carried out in order to obtain a desired compound after completion of the above reaction.

Step F2

This step is a step of producing a compound having the general formula (I-F).

This step is carried out in the same manner as in the above Step E4 of Method E by reducing the compound having the general formula (XX) in an inert solvent in the presence of a palladium catalyst in a hydrogen atmosphere.

Method G is a method for producing a compound having the general formula (I-G).

Method G

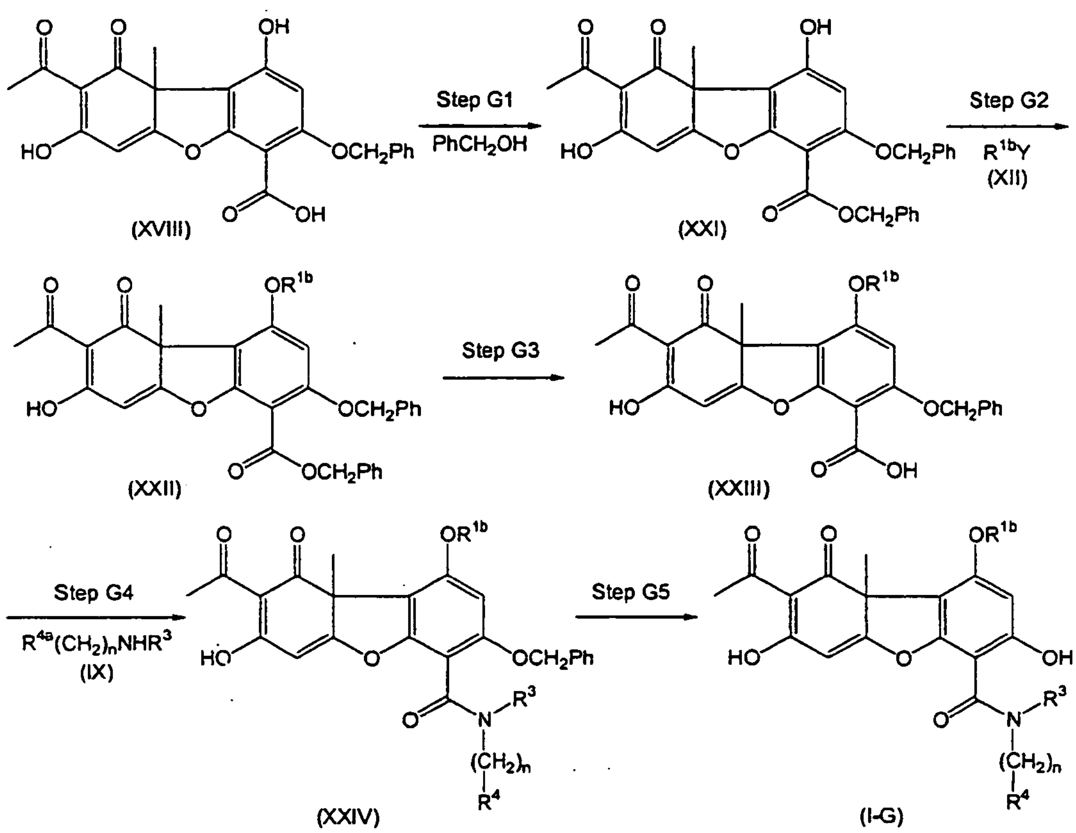


(XVIII) (XXI) (XXII) (XXIII) (XXIV) (I-G)

In the present invention, $R^3$, $R^4$, $R^{1b}$, $R^{4a}$, Y and n are as defined above.

Step G1

This step is a step of producing a compound (XXI).

This step is carried out in the same manner as in the above Step C1 of Method C by reacting the compound (XVIII) obtained in the above Step E2 of Method E with benzyl alcohol in an inert solvent in the presence of a base and a condensing agent.

Step G2

This step is a step of producing a compound having the general formula (XXII).

This step is carried out in the same manner as in the above Step C2 of Method C by reacting the compound (XXI) with a compound having the general formula (XII) which is a known compound or is easily obtained from a known compound as a starting material by a method similar to a known method, in an inert solvent in the presence of a base in an argon or nitrogen atmosphere.

Step G3

This step is a step of producing a compound having the general formula (XXIII).

This step is carried out by reacting the compound having the general formula (XXII) with a base in an inert solvent.

The inert solvent used in this step is not particularly limited so long as it does not inhibit the reaction and allows the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; sulfoxides such as dimethyl sulfoxide and sulfolane; nitriles such as acetonitrile; aromatic hydrocarbons such as benzene, toluene and xylene; and water. The inert solvent is preferably an alcohol, ether or water, and more preferably ethanol, tetrahydrofuran or water. A mixed solvent of ethanol with water or a mixed solvent of tetrahydrofuran with water (whose mixing ratio is 1:100 to 100:1) may be used as necessary.

Examples of the base used in this step include alkali metal carbonates such as sodium carbonate, potassium

carbonate and lithium carbonate; alkali metal bicarbonates such as sodium bicarbonate, potassium bicarbonate and lithium bicarbonate; and alkali metal hydroxides such as sodium hydroxide, potassium hydroxide, barium hydroxide and lithium hydroxide. The base is preferably an alkali metal hydroxide, and more preferably sodium hydroxide or potassium hydroxide. The base may be used as an aqueous solution (1% to 50%, for example, and preferably 3% to 20%) as necessary.

The reaction temperature in this step varies depending on the raw material compound, the inert solvent used and the like, but is usually 0˚C to 130˚C, and preferably 20˚C to 100˚C.

The reaction time in this step varies depending on the raw material compound, the inert solvent used and the like, but is usually 0.5 hour to 12 hours, and preferably 1 hour to 5 hours.

Step G4

This step is a step of producing a compound having the general formula (XXIV) and consists of (i) to (ii).

(i) This step is carried out in the same manner as in (i) of the above Step A3 of Method A by reacting the compound having the general formula (XXIII) with a compound having the general formula (IX) which is a known compound or is easily obtained from a known compound as a starting material by a method similar to a known method, in an inert solvent in the presence of a condensing agent and in the presence or absence of a base.

(ii) This step is carried out in the same manner as in (ii) of the above Step A3 of Method A by reacting the compound obtained in (i) above with a base in an inert solvent.

Deprotection reaction of the protecting group for the amino group, hydroxy group and/or carboxyl group in $R^{4a}$ may be further carried out in order to obtain a desired compound after completion of the above reaction.

Step G5

This step is a step of producing a compound having the general formula (I-G).

This step is carried out in the same manner as in the above Step E4 of Method E by reducing the compound having the general formula (XXTV) in an inert solvent in the presence of a palladium catalyst in a hydrogen atmosphere.

Method H is a method for producing a compound having the general formula (I-H).

Method H

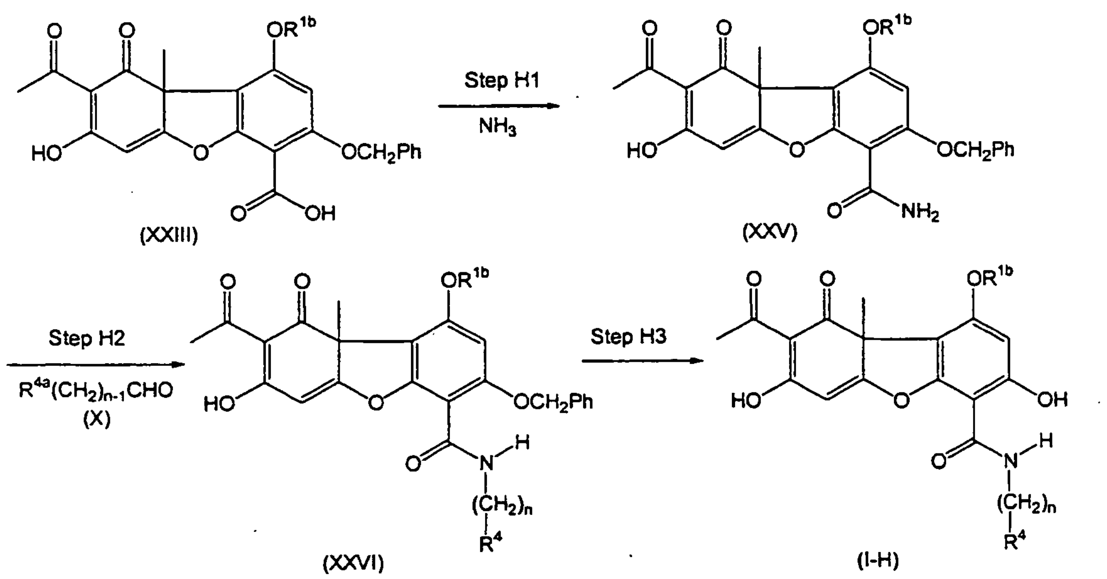


In the present invention, $R^4$, $R^{1b}$, $R^{4a}$ and n are as defined above.

Step H1

This step is a step of producing a compound having the general formula (XXV).

This step is carried out in the same manner as in the above Step D1 of Method D by reacting the compound having the general formula (XXIII) obtained in the above Step G3 of Method G with ammonia in an inert solvent in the presence of a condensing agent and in the presence or absence of a base.

Step H2

This step is a step of producing a compound having the general formula (XXVI).

This step is carried out in the same manner as in the above Step B1 of Method B by reacting the compound having the general formula (XXV) with a compound having the general formula (X) which is a known compound or is easily obtained from a known compound as a starting material by a method similar to a known method and a hydrosilane in an inert solvent in the presence of an acid.

Deprotection reaction of the protecting group for the amino group, hydroxy group and/or carboxyl group in $R^{4a}$ may be further carried out in order to obtain a desired compound after completion of the above reaction.

Step H3

This step is a step of producing a compound having the general formula (I-H).

This step is carried out in the same manner as in the above Step E4 of Method E by reducing the compound having the general formula (XXVI) in an inert solvent in the presence of a palladium catalyst in a hydrogen atmosphere.

Method I is a method for producing a compound having the general formula (I-I).

Method I

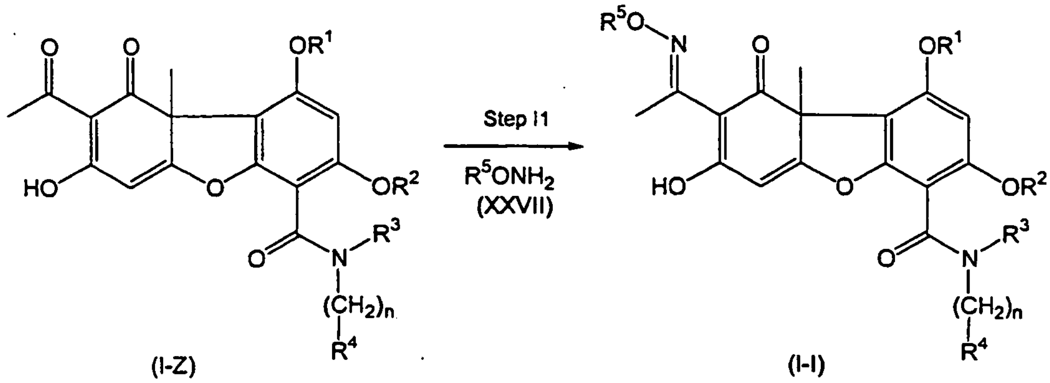


(I-Z) (I-I)

In the present invention, $R^1$, $R^2$, $R^3$, $R^4$, $R^5$ and n are as defined above.

Step I1

This step is a step of producing a compound having the general formula (I-I).

This step is carried out by reacting the compound having the general formula (I-Z) obtained in the above Methods A to H with a compound having the general formula (XXVII) which is a known compound or is easily obtained from a known compound as a starting material by a method similar to a known method, in an inert solvent in the presence or absence of a base.

The inert solvent used in this step is not particularly limited so long as it does not inhibit the reaction and allows the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; halogenated hydrocarbons such as dichloromethane, 1,2-dichloroethane, dichlorobenzene, chloroform and carbon tetrachloride; and mixed solvents thereof. The inert solvent is preferably an ether or alcohol, and more preferably tetrahydrofuran, methanol or ethanol.

When the compound having the general formula (XXVII) used for the reaction in this step is a salt such as a hydrochloride, the salt may be present together with a base in order to appropriately neutralize the salt. Examples of the base include alkali metal carbonates such as sodium carbonate, potassium carbonate and lithium carbonate; alkali metal bicarbonates such as sodium bicarbonate, potassium bicarbonate and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU). The base is preferably an alkali metal bicarbonate, and more preferably sodium bicarbonate.

The reaction temperature in this step varies depending on the raw material compound, the inert solvent used and the like, but is usually 0˚C to 80˚C, and preferably 20˚C to 50˚C.

The reaction time in this step varies depending on the raw material compound, the inert solvent used, the reaction temperature and the like, but is usually 0.5 hour to 36 hours, and preferably 1 hour to 24 hours.

Method J is a method for producing a compound having the general formula (I-J).

Method J

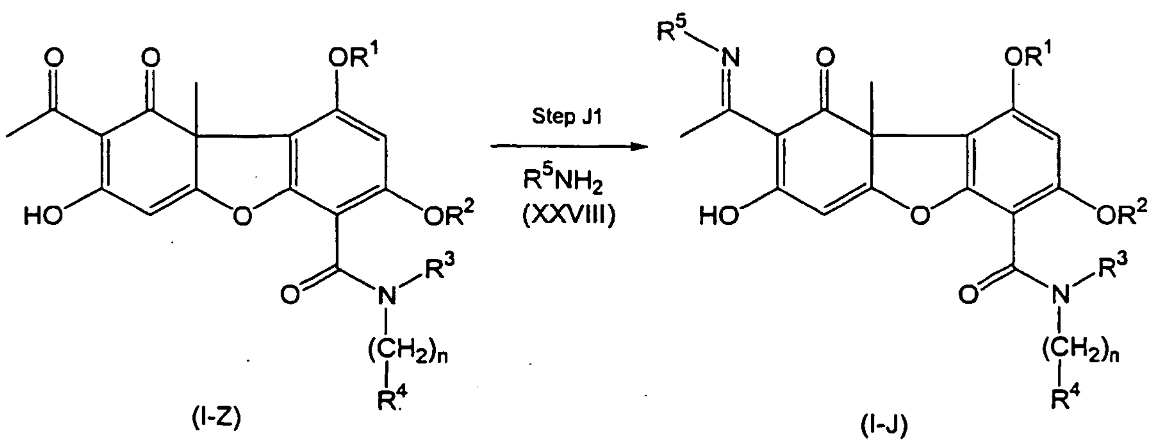


(I-Z) (I-J)

In the present invention, $R^1$, $R^1$, $R^3$, $R^4$, $R^5$ and n are as defined above.

Step J1

This step is a step of producing a compound having the general formula (I-J).

This step is carried out by reacting the compound having the general formula (I-Z) obtained in the above Methods A to H with a compound having the general formula (XXVIII) which is a known compound or is easily obtained from a known compound as a starting material by a method similar to a known method, in an inert solvent in the presence or absence of a base.

The inert solvent used in this step is not particularly limited so long as it does not inhibit the reaction and allows the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; nitriles such as acetonitrile and isobutyronitrile; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably an ether, alcohol or halogenated hydrocarbon, and more preferably tetrahydrofuran, methanol, ethanol or dichloromethane.

When the compound having the general formula (XXVIII) used for the reaction in this step is a salt such as a hydrochloride, the salt may be present together with a base in order to appropriately neutralize the salt. Examples of the base include alkali metal carbonates such as sodium carbonate, potassium carbonate and lithium carbonate; alkali metal bicarbonates such as sodium bicarbonate, potassium bicarbonate and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-rene (DBU). The base is preferably an alkali metal bicarbonate, and more preferably sodium bicarbonate.

The reaction temperature in this step varies depending on the raw material compound, the inert solvent used and the like, but is usually 0°C to 80°C, and preferably 10°C to 50°C.

The reaction time in this step varies depending on the raw material compound, the inert solvent used, the reaction temperature and the like, but is usually 0.5 hour to 48 hours, and preferably 1 hour to 24 hours.

Method K is a method for producing a compound having the general formula (XXXV) and a compound having the general formula (XXXVII) among the compounds having the general formula (X) used in the above Step B1 of Method B, the above Step D2 of Method D, the above Step F1 of Method F and the above Step H2 of Method H.

Method K

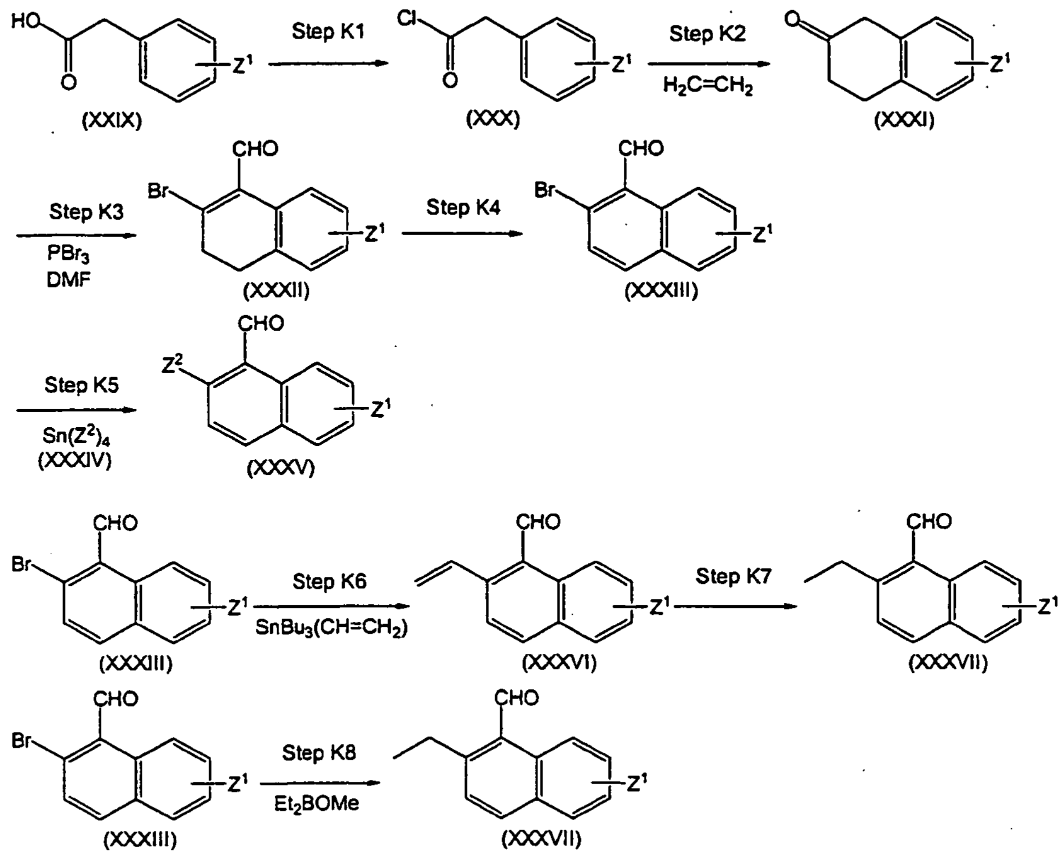


In the present invention, $Z^1$ represents a halogen atom or a $C_1$-$C_6$ alkyl group, and $Z^2$ represents a methyl group or an ethyl group.

$Z^1$ is bonded to the 5-, 6-, 7- and/or 8-position(s) of the compound having the general formula (XXXV) or the compound having the general formula (XXXVII), and the number of $Z^1$ is one to four.

Step K1

This step is a step of producing a compound having the general formula (XXX).

This step is carried out by reacting a compound having the general formula (XXIX) which is a known compound or is easily obtained from a known compound as a starting material by a method similar to a known method with a chlorinating agent in an inert solvent in the presence or absence of a catalytic amount of N,N-dimethylformamide.

The inert solvent used in this step is not particularly limited so long as it does not inhibit the reaction and allows

the starting material to be dissolved therein to a certain extent. Examples of the inert solvent include ethers such as diethyl ether, diisopropyl ether, tetrahydrofuran, dioxane, dimethoxyethane and diethylene glycol dimethyl ether; esters such as ethyl formate, ethyl acetate, propyl acetate, butyl acetate and diethyl carbonate; halogenated hydrocarbons such as dichloromethane, 1,2-dichloroethane and dichlorobenzene; and aromatic hydrocarbons such as benzene, toluene and xylene. The inert solvent is preferably a halogenated hydrocarbon, and more preferably dichloromethane.

Examples of the chlorinating agent used in this step include inorganic acids such as hydrochloric acid; halogen molecules such as chlorine; phosphorus reagents such as phosphorus trichloride, phosphorus pentachloride and phosphorus oxychloride; chlorides such as oxalyl chloride; sulfinic acid reagents such as thionyl chloride; and sulfonic acid reagents such as sulfonyl chloride and toluenesulfonyl chloride. The chlorinating agent is preferably a chloride or sulfinic acid reagent, and more preferably oxalyl chloride or thionyl chloride.

The reaction temperature in this step varies depending on the raw material compound, the inert solvent used and the like, but is usually 0˚C to 130˚C, and preferably 10˚C to 80˚C.

The reaction time in this step varies depending on the raw material compound, the inert solvent used, the reaction temperature and the like, but is usually 0.1 hour to 24 hours, and preferably 0.5 hour to 5 hours.

Step K2

This step is a step of producing a compound having the general formula (XXXI).

This step is a step of reacting the compound having the general formula (XXX) with ethylene in an inert solvent in the presence of a Lewis acid and is carried out in accordance with the method described in Journal of Organic Chemistry, 1968, Vol. 33, p.4288-4290, for example.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether, halogenated hydrocarbons such as dichloromethane, 1,2-dichloroethane, chloroform and carbon tetrachloride; and mixed solvents thereof. The inert solvent is preferably a halogenated hydrocarbon, and more preferably dichloromethane.

Examples of the Lewis acid used in this step include aluminum chloride, zinc chloride, tin tetrachloride, titanium trichloride, boron trifluoride and boron tribromide. The Lewis acid is preferably aluminum chloride.

The reaction temperature in this step varies depending on the raw material compound, the inert solvent used and the like, but is usually -75˚C to 40˚C, and preferably -20˚C to 30˚C.

The reaction time in this step varies depending on the raw material compound, the inert solvent used, the reaction temperature and the like, but is usually 0.5 hour to 24 hours, and preferably 1 hour to 10 hours.

Step K3

This step is a step of producing a compound having the general formula (XXXII).

This step is a step of reacting the compound having the general formula (XXXI) with phosphorus tribromide and N,N-dimethylformamide in an inert solvent and is carried out in accordance with the method described in Journal of Chemical Society Perkin Transaction 1, 1988, p.2595-2601, for example.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably a halogenated hydrocarbon, and more preferably chloroform.

The reaction temperature in this step varies depending on the raw material compound, the inert solvent used and the like, but is usually -10˚C to 70˚C, and preferably 0˚C to 60˚C.

The reaction time in this step varies depending on the raw material compound, the inert solvent used, the reaction temperature and the like, but is usually 1 hour to 8 hours, and preferably 2 hours to 6 hours.

Step K4

This step is a step of producing a compound having the general formula (XXXIII).

This step is carried out by reacting the compound having the general formula (XXXII) with dichlorodicyano-p-benzoquinone (DDQ) or a palladium catalyst in an inert solvent.

The inert solvent used in this step is not particularly limited so long as it does not inhibit the reaction and allows

the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably an aromatic hydrocarbon, and more preferably toluene when reacting with DDQ, and is preferably an aromatic hydrocarbon, and more preferably toluene when reacting with a palladium catalyst.

The palladium catalyst used in this step is a divalent palladium catalyst or a zerovalent palladium catalyst, for example, and is preferably palladium-active carbon, palladium (II) acetate, palladium (II) trifluoroacetate, palladium black, palladium (II) bromide, palladium (II) chloride or palladium (II) iodide, and more preferably palladium-active carbon.

The reaction temperature in this step varies depending on the raw material compound, the inert solvent used and the like, but is usually 10˚C to 160˚C, and preferably 60˚C to 120˚C when reacting with DDQ and preferably 30˚C to 120˚C when reacting with a palladium catalyst.

The reaction time in this step varies depending on the raw material compound, the inert solvent used, the reaction temperature and the like, but is usually 0.5 hour to 168 hours, and preferably 6 hours to 96 hours when reacting with DDQ and preferably 1 hour to 120 hours when reacting with a palladium catalyst.

Step K5

This step is a step of producing a compound having the general formula (XXXV).

This step is carried out by reacting the compound having the general formula (XXXIII) with a compound having the general formula (XXXIV) in an inert solvent in the presence of a palladium catalyst.

The inert solvent used in this step is not particularly limited so long as it does not inhibit the reaction and allows the starting material to be dissolved therein to a certain extent. Examples of the inert solvent include amides such as formamide, N,N-dimethylformamide, N,N-dimethylacetamide, N-methyl-2-pyrrolidone, N-methyl-2-pyrrolidinone and hexamethylphosphoric triamide; esters such as ethyl formate, ethyl acetate, propyl acetate, butyl acetate and diethyl carbonate; aromatic hydrocarbons such as benzene, toluene and xylene; and mixed solvents thereof. The inert solvent is preferably an amide, and more preferably N,N-dimethylformamide.

The palladium catalyst used in this step is a divalent palladium catalyst or a zerovalent palladium catalyst, for example, and is preferably tetrakis(triphenylphosphine)palladium, palladium-active carbon, palladium (II) acetate, palladium (II) trifluoroacetate, palladium black, palladium (II) bromide, palladium (II) chloride or dichloro[1,1'-bis(diphenylphosphino)ferrocene]palladium (II), and more preferably tetrakis(triphenylphosphine)palladium.

The reaction temperature in this step varies depending on the raw material compound, the inert solvent used and the like, but is usually 20˚C to 160˚C, and preferably 40˚C to 120˚C.

The reaction time in this step varies depending on the raw material compound, the inert solvent used, the reaction temperature and the like, but is usually 0.5 hour to 12 hours, and preferably 1 hour to 8 hours.

Step K6

This step is a step of producing a compound having the general formula (XXXVI).

This step is carried out by reacting the compound having the general formula (XXXIII) with tri-n-butylvinyltin in an inert solvent in the presence of a palladium catalyst.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methyl-2-pyrrolidinone and hexamethylphosphoric triamide; aromatic hydrocarbons such as benzene, toluene and xylene; and mixed solvents thereof. The inert solvent is preferably an amide, and more preferably N,N-dimethylformamide.

The palladium catalyst used in this step is a divalent palladium catalyst or a zerovalent palladium catalyst, for example, and is preferably tetrakis(triphenylphosphine)palladium, palladium-active carbon, palladium (II) acetate, palladium (II) trifluoroacetate, palladium black, palladium (II) bromide, palladium (II) chloride, palladium (II) iodide or dichloro [1,1'-bis(diphenylphosphino)ferrocene]palladium (II), and more preferably tetrakis(triphenylphosphine)palladium.

The reaction temperature in this step varies depending on the raw material compound, the inert solvent used and the like, but is usually 20˚C to 160˚C, and preferably 40˚C to 120˚C.

The reaction time in this step varies depending on the raw material compound, the inert solvent used, the reaction temperature and the like, but is usually 0.5 hour to 24 hours, and preferably 1 hour to 12 hours.

Step K7

This step is a step of producing a compound having the general formula (XXXVII).

This step is carried out by reacting the compound having the general formula (XXXVI) in an inert solvent in the presence of a palladium catalyst in a hydrogen atmosphere.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, t-butanol, isoamyl alcohol, diethylene glycol, glycerol, octanol, cyclohexanol and methyl cellosolve; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; and mixed solvents thereof. The inert solvent is preferably an alcohol or ester, and more preferably ethanol or ethyl acetate.

The palladium catalyst used in this step is palladium-active carbon or palladium black, and more preferably palladium-active carbon.

The reaction temperature in this step varies depending on the raw material compound, the inert solvent used and the like, but is usually 0˚C to 100˚C, and preferably 20˚C to 60˚C.

The reaction time in this step varies depending on the raw material compound, the inert solvent used, the reaction temperature and the like, but is usually 0.5 hour to 48 hours, and preferably 1 hour to 24 hours.

Step K8

This step is a step of producing a compound having the general formula (XXXVII).

This step is carried out by reacting the compound having the general formula (XXXIII) with diethylmethoxyborane in an inert solvent in the presence of a palladium catalyst.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methyl-2-pyrrolidinone and hexamethylphosphoric triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; aromatic hydrocarbons such as benzene, toluene and xylene; and mixed solvents thereof. The inert solvent is preferably an amide, and more preferably N,N-dimethylformamide.

The palladium catalyst used in this step is a divalent palladium catalyst or a zerovalent palladium catalyst, for example, and is preferably tetrakis(triphenylphosphine)palladium, palladium-active carbon, palladium (II) acetate, palladium (II) trifluoroacetate, palladium black, palladium (II) bromide, palladium (II) chloride, palladium (II) iodide or dichloro [1,1'-bis(diphenylphosphino)ferrocene]palladium (II), and more preferably dichloro[1,1'-bis(diphenylphosphino)ferrocene]palladium (II).

The reaction temperature in this step varies depending on the raw material compound, the inert solvent used and the like, but is usually 20˚C to 160˚C, and preferably 40˚C to 120˚C.

The reaction time in this step varies depending on the raw material compound, the inert solvent used, the reaction temperature and the like, but is usually 0.5 hour to 48 hours, and preferably 1 hour to 24 hours.

Method L is a method for producing a compound having the general formula (ILVI) and a compound having the general formula (L) among the compounds having the general formula (X) used in the above Step B1 of Method B, the above Step D2 of Method D, the above Step F1 of Method F and the above Step H2 of Method H.

Method L

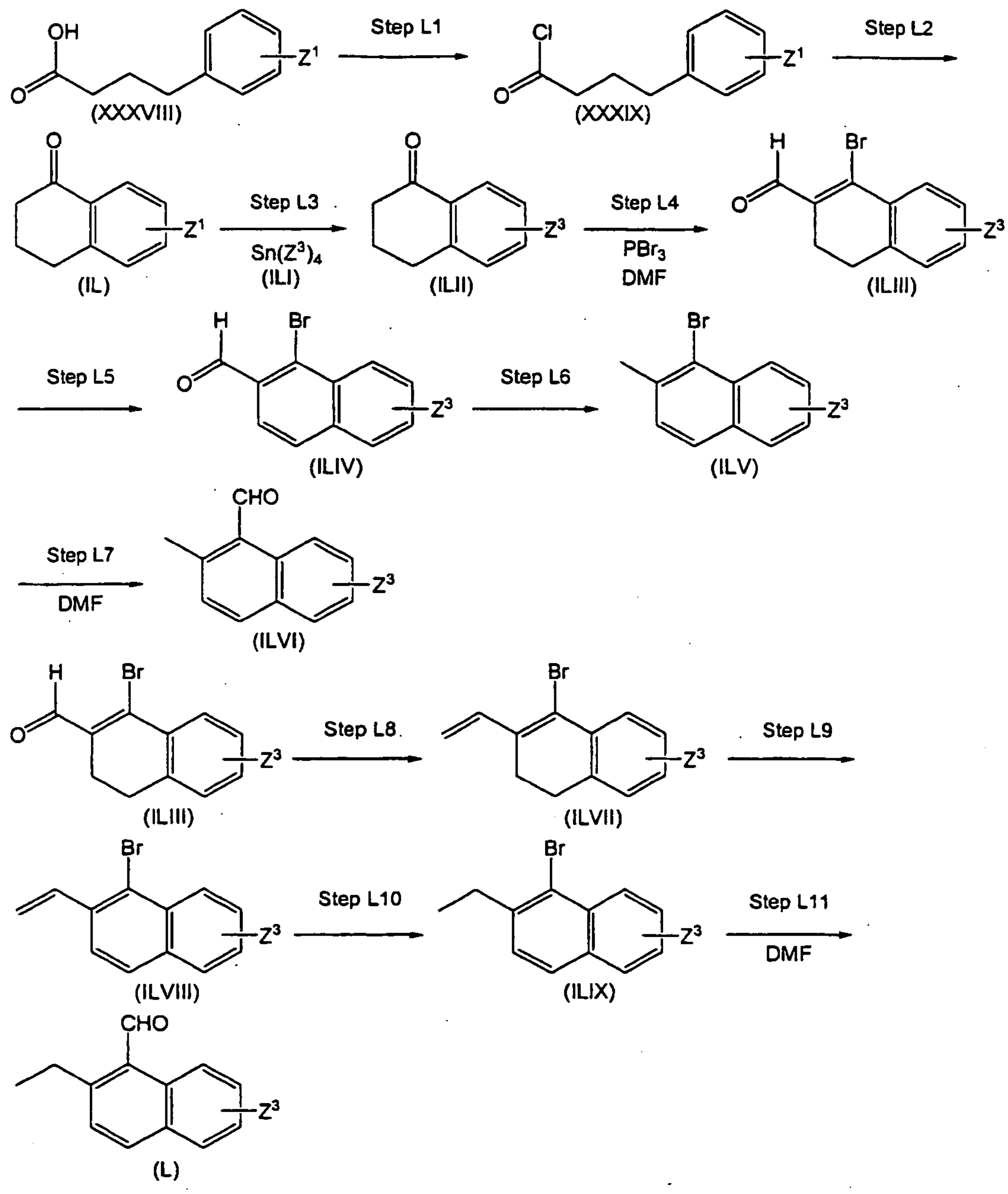


(XXXVIII) (XXXIX) (IL) (ILII) (ILIII) (ILIV) (ILV) (ILVI) (ILIII) (ILVII) (ILVIII) (ILIX) (L)

In the present invention, $Z^1$ is as defined above, and $Z^3$ represents a $C_1$-$C_6$ alkyl group when Method L comprises Step L3, and represents $Z^1$ when Method L does not comprise Step L3 (when a compound having the general formula (IL) is the same as a compound having the general formula (ILII)).

$Z^3$ is bonded to the 5-, 6-, 7- and/or 8-position(s) of the compound having the general formula (ILVI) or the compound having the general formula (L), and the number of $Z^1$ and $Z^3$ is one to four.

Step L1

This step is a step of producing a compound having the general formula (XXXIX).

This step is carried out in the same manner as in the above Step K1 of Method K by reacting a compound having the general formula (XXXVIII) which is a known compound or is easily obtained from a known compound as a starting material by a method similar to a known method with a chlorinating agent in an inert solvent in the presence or absence of a catalytic amount of N,N-dimethylformamide.

Step L2

This step is a step of producing a compound having the general formula (IL).

This step is carried out by reacting the compound having the general formula (XXXIX) in an inert solvent in the presence of a Lewis acid.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; halogenated hydrocarbons such as dichloromethane, 1,2-dichloroethane, dichlorobenzene, chloroform and carbon tetrachloride; and mixed solvents thereof. The inert solvent is preferably a halogenated hydrocarbon, and more preferably dichloromethane.

Examples of the Lewis acid used in this step include aluminum chloride, zinc chloride, tin tetrachloride, titanium trichloride, boron trifluoride and boron tribromide. The Lewis acid is preferably aluminum chloride.

The reaction temperature in this step varies depending on the raw material compound, the inert solvent used and the like, but is usually 0˚C to 60˚C, and preferably 10˚C to 50˚C.

The reaction time in this step varies depending on the raw material compound, the inert solvent used, the reaction temperature and the like, but is usually 0.5 hour to 48 hours, and preferably 1 hour to 24 hours.

Step L3

This step is a step of producing a compound having the general formula (ILII). This step is carried out by reacting the compound having the general formula (IL) with a compound having the general formula (ILI) in an inert solvent in the presence of a palladium catalyst.

The inert solvent used in this step is not particularly limited so long as it does not inhibit the reaction and allows the starting material to be dissolved therein to a certain extent. Examples of the inert solvent include amides such as formamide, N,N-dimethylformamide, N,N-dimethylacetamide, N-methyl-2-pyrrolidone, N-methyl-2-pyrrolidinone and hexamethylphosphoric triamide; esters such as ethyl formate, ethyl acetate, propyl acetate, butyl acetate and diethyl carbonate; aromatic hydrocarbons such as benzene, toluene and xylene; and mixed solvents thereof. The inert solvent is preferably an amide, and more preferably N,N-dimethylformamide.

The palladium catalyst used in this step is a divalent palladium catalyst or a zerovalent palladium catalyst, for example, and is preferably tetrakis(triphenylphosphine)palladium, palladium-active carbon, palladium (II) acetate, palladium (II) trifluoroacetate, palladium black, palladium (II) bromide, palladium (II) chloride or dichloro[1,1'-bis(diphenylphosphino)ferrocene]palladium (II), and more preferably tetrakis(triphenylphosphine)palladium.

The reaction temperature in this step varies depending on the raw material compound, the inert solvent used and the like, but is usually 20˚C to 160˚C, and preferably 40˚C to 120˚C.

The reaction time in this step varies depending on the raw material compound, the inert solvent used, the reaction temperature and the like, but is usually 0.5 hour to 48 hours, and preferably 1 hour to 24 hours.

Step L4

This step is a step of producing a compound having the general formula (ILIII).

This step is carried out in the same manner as in the above Step K3 of Method K by reacting the compound having the general formula (ILII) with phosphorus tribromide and N,N-dimethylformamide in an inert solvent.

Step L5

This step is a step of producing a compound having the general formula (ILIV).

This step is carried out in the same manner as in the above Step K4 of Method K by reacting the compound having the general formula (ILIII) with dichlorodicyano-p-benzoquinone (DDQ) or a palladium catalyst in an inert solvent.

Step L6

This step is a step of producing a compound having the general formula (ILV).

This step is carried out by reacting the compound having the general formula (ILIV) with triethylsilane in an inert solvent in the presence of tri(pentafluorophenyl)borane.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran,

dioxane, dimethoxyethane and diethylene glycol dimethyl ether; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is preferably a halogenated hydrocarbon, and more preferably dichloromethane.

The reaction temperature in this step varies depending on the raw material compound, the inert solvent used and the like, but is usually 0˚C to 100˚C, and preferably 20˚C to 60˚C.

The reaction time in this step varies depending on the raw material compound, the inert solvent used, the reaction temperature and the like, but is usually 2 hours to 96 hours, and preferably 6 hours to 72 hours.

Step L7

This step is a step of producing a compound having the general formula (ILVI).

This step is carried out by reacting the compound having the general formula (ILV) with N,N-dimethylformamide in an inert solvent in the presence of an organometallic reagent.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; ethers such as diethyl ether, diisopropyl ether, tetrahydrofuran, dioxane, dimethoxyethane and diethylene glycol dimethyl ether; aromatic hydrocarbons such as benzene, toluene and xylene; and mixed solvents thereof. The inert solvent is preferably an ether, and more preferably tetrahydrofuran.

The organometallic reagent used in this step is butyllithium, sodium hexamethyldisilazide, potassium hexamethyldisilazide, lithium hexamethyldisilazide or lithium diisopropylamide, for example, and is preferably butyllithium.

The reaction temperature in this step varies depending on the raw material compound, the inert solvent used and the like, but is usually -100˚C to 80˚C, and preferably -78˚C to 30˚C.

The reaction time in this step varies depending on the raw material compound, the inert solvent used, the reaction temperature and the like, but is usually 0.1 hour to 24 hours, and preferably 0.5 hour to 8 hours.

Step L8

This step is a step of producing a compound having the general formula (ILVII).

This step is carried out by reacting the compound having the general formula (ILIII) with (methyl)triphenylphosphonium bromide in an inert solvent in the presence of a base.

The inert solvent used in this step is not particularly limited so long as it does not inhibit the reaction and allows the starting material to be dissolved therein to a certain extent. Examples of the inert solvent include hydrocarbons such as pentane, hexane, octane, petroleum ether and ligroin; and ethers such as diethyl ether, diisopropyl ether, tetrahydrofuran, dioxane, dimethoxyethane and diethylene glycol dimethyl ether. The inert solvent is preferably an ether, and more preferably tetrahydrofuran.

Examples of the base used in this step include alkali metal hydrides such as lithium hydride, sodium hydride and potassium hydride; alkali metal alkoxides such as sodium methoxide, sodium ethoxide, sodium t-butoxide, potassium methoxide, potassium ethoxide, potassium t-butoxide and lithium methoxide; alkali metal trialkylsiloxides such as sodium trimethylsiloxide, potassium trimethylsiloxide and lithium trimethylsiloxide; and organometallic bases such as butyl lithium, lithium diisopropylamide and lithium bis(trimethylsilyl)amide. The base is preferably an alkali metal hydride, and more preferably sodium hydride.

The reaction temperature in this step varies depending on the raw material compound, the inert solvent used and the like, but is usually -78˚C to 80˚C, and preferably -20˚C to 60˚C.

The reaction time in this step varies depending on the raw material compound, the inert solvent used, the reaction temperature and the like, but is usually 0.5 hour to 12 hours, and preferably 1 hour to 8 hours.

Step L9

This step is a step of producing a compound having the general formula (IILVIII).

This step is carried out in the same manner as in the above Step K4 of Method K by reacting the compound having the general formula (ILVTI) with dichlorodicyano-p-benzoquinone (DDQ) or a palladium catalyst in an inert solvent.

Step L10

This step is a step of producing a compound having the general formula (ILIX).

This step is carried out in the same manner as in the above Step K7 of Method K by reacting the compound having the general formula (ILVIII) in an inert solvent in the presence of a palladium catalyst in a hydrogen atmosphere.

Step L11

This step is a step of producing a compound having the general formula (L).

This step is carried out in the same manner as in Step L7 by reacting the compound having the general formula (ILIX) with N,N-dimethylformamide in an inert solvent in the presence of an organometallic reagent.

In each of the above steps, after completion of the reaction, each target compound may be isolated from the reaction mixture and purified according to a conventional method and used for the next step, or the crude product after the reaction may be concentrated under reduced pressure and used for the next step. The target compound is usually obtained as follows. The reaction mixture is appropriately neutralized and the insoluble matter is removed by filtration if present. Then, water and an organic solvent immiscible with water (such as dichloromethane, diethyl ether or ethyl acetate) are added, and the organic layer containing the target compound is separated. The organic layer is washed with water or the like and then dried over anhydrous magnesium sulfate, anhydrous sodium sulfate, anhydrous sodium bicarbonate or the like and filtered. Then, the solvent is evaporated. The resulting target compound may be separated and purified if necessary by appropriately combining conventional methods, for example, methods conventionally used for separation and purification of organic compounds such as recrystallization and reprecipitation and eluting with an appropriate eluent by application of chromatography. The target compound insoluble in a solvent may be purified by washing the resulting solid crude product with a solvent.

The raw material compound having the general formula (VI), (IX), (X), (XII), (XVI), (XXVII), (XXVIII), (XXIX), (XXXIV), (XXXVIII) or (ILI) is a known compound or is easily produced from a known compound as a starting material by a known method or a method similar to a known method.

The known substance cercosporamide (V) is produced according to the method described below.

1. Cercosporamide-Producing Fungi

Lachnum fuscescens SANK 19096 strain was isolated from fruiting bodies formed on fallen leaf collected in Nagano Prefecture, Japan.

Cercosporamide was found in a culture of the separated Lachnum fuscescens SANK 19096 strain (hereinafter also simply referred to as "SANK 19096 strain").

The cercosporamide-producing fungus is not particularly limited so long as it is a fungus of Lachnum producing cercosporamide, but is preferably Lachnum fuscescens SANK 19096 strain.

The SANK 19096 strain is described as follows.

In describing mycological characteristics, indication of colony coloring was in accordance with "Kornerup, A. and Wanscher, J.H. (1978), Methuen Handbook of Colour, 3rd ed., Eyre Methuen, London, UK, 1-252".

The following media were used in order to observe mycological characters of the SANK 19096 strain under culture.

Potato dextrose agar medium (hereinafter referred to as "PDA medium"): 39 g of Nissui Potato Dextrose Agar Medium (Nissui Pharmaceutical Co., Ltd.) is dissolved in 1000 ml of distilled water followed by sterilizing for 15 minutes at 121˚C and preparing plates.

Modified Weitzman and Silva-Hutner medium (hereinafter referred to as "WSH medium"): 10 g of Nisshoku Oatmeal, 1 g of magnesium sulfate heptahydrate, 1 g of potassium dihydrogenphosphate, 1 g of sodium nitrate and 20 g of agar are dissolved in 1000 ml of distilled water followed by sterilizing for 15 minutes at 121˚C and preparing plates.

Malt extract agar medium (hereinafter referred to as "MEA medium"): 20 g of malt extract, 1 g ofpolypepton (Nihon Pharmaceutical Co., Ltd.), 20 g of glucose and 20 g of agar are dissolved in 1000 ml of distilled water followed by sterilizing for 15 minutes at 121˚C and preparing plates.

Corn meal agar medium (hereinafter referred to as "CMA medium"): 17 g of Nissui Corn Meal Agar (Nissui Pharmaceutical Co., Ltd.) is dissolved in 1000 ml of distilled water followed by sterilizing for 15 minutes at 121˚C and preparing plates.

The mycological properties of a dried fruiting body specimen used to isolate strain SANK 19096 are described as follows. Apothecium is short stipe; the disc is slightly concave, 557 μm in diameter and pale brown when rehydrated with distilled water. The disc is deep cupulate with incurving margin, 499 μm in diameter and pale brown when dry. The ectal excipulum is "textura prismatica", hyaline to subhyaline, composed of thick-walled cells and with short hair-like projections or hairs from the outermost layer. The medullary excipulum is "textura intricata" and hyaline. The hairs are cylindrical, straight or slightly curved, obtuse at the apex, multi-septate, granulate all over, brown, 4.5 to 6.0 μm in width and 71 to 76 μm in length. Amorphous resinous matters are rarely attached to the ends of the hairs. The stipe is cylindrical, pale brown and 115 μm in height. The asci are inoperculate, cylindrical-clavate, 8-spored, 30 to 43 μm in length and 3.5 to 5 μm in width. The tip of the ascus is stained blue by Melzer's reagent. The paraphyses are lanceolate, aseptate or occasionally 1- or 2-septate, hyaline, 50.9 to 68.7 μm in length, 2.7 to 4.1 μm at the widest point and exceed the asci by 9.1 to 16.5 μm. The ascospores are fusiform to oblong-ellipsoid, aseptate, hyaline, 5.5 to 8.5 in length and 1.3 to 2.2

μm in width.

Strain SANK 19096 demonstrates the mycological properties indicated below on the various media described above.

Colonies on PDA medium reach a diameter of 19 to 21 mm following culturing for 21 days at 23˚C. The colonies are velvety to cottony, composed of a mycelia layer protruding toward the center and reddish gray (11B2) to white. Exudates, sclerotia and conidia are not observed. The back of the colonies is grayish orange (5B4) in the center and white along the margins, have radiating wrinkles. Soluble pigment is not observed. Mycelia are septate, branched, hyaline and 1.5 to 3.5 μm in diameter.

Colonies on WSH medium reach a diameter of 29 to 31 mm following culturing for 21 days at 23˚C. The colonies are floccose to velvety, composed of a thin mycelia layer and white. Exudates, sclerotia and conidia are not observed. The back of the colonies is white. Soluble pigments are not observed.

Colonies on CMA medium reach a diameter of 33 to 34 mm following culturing for 21 days at 23˚C. The colonies consist only of an extremely thin mycelia layer and are colorless. Exudates, sclerotia and conidia are not observed. The back of the colonies is colorless. Soluble pigments are not observed.

Colonies on MEA medium reach a diameter of 21 to 22 mm following culturing for 21 days at 23˚C. The colonies are cottony in the center, mycelia concentrate toward the margins and become velvety and grayish yellow (4C4) to yellowish white (4A1), while the concentrated portion of airborne mycelia are white. The margins are composed only of basal mycelia and brownish orange (5C3) to orange gray (5C2). Exudates, sclerotia and conidia are not observed. The back of the colonies is yellowish brown (5D6) to grayish orange (5B3) and white along the margins. Soluble pigments are not observed.

The morphological characteristics of the present fungus agreed well with the description of Lachnum species in the document by Spooner (Spooner, BM (1987) Bibliotheca Mycologica 116: 1-711). Thus, this fungus was identified as Lachnum sp. and named Lachnum sp. strain SANK 19096.

Strain SANK 19096 agreed, with only few exceptions, with the descriptions of Dasyscyphus fuscescens (Pers.) Gray of the document by Dennis, RWG (Dennis, RWG (1949) Mycological Papers 32: 1-97) and Lachnum fuscescens (Pers.) P. Karst. in the document by Tanaka and Hosoya (Tanaka, I and Hosoya, T (2001) Mycoscience 42: 597-609). Dasyscyphus fuscescens is currently treated as a synonym of Lachnum fuscescens. In addition, in a document by Baral and Krieglsteiner (Baral, HO and Krieglsteiner, GJ (1985) Beihefte zur Zeitschrift für Mykologie 6: 1-160), a new genus Brunnipila is established for Lachnum species having brown hairs in the manner of Lachnum fuscescens, and is treated as a different genus. In the document by Baral and Krieglsteiner, Lachnum fuscescens is treated as a synonym of Brunnipila fuscescens (Pers.) Baral. However, this approach is not widely accepted, and in the publication by Krik, et al. (Krik, PM et al. (2001) Ainsworth & Bisby's Dictionary of Fungi 9th Edition, CABI International, Wallingford, UK, 1-655), the genus Brunnipila is treated as a synonym of the genus Lachnum. Thus, in the present patent, strain SANK 19096 was identified as Lachnum fuscescens in accordance with the document by Tanaka and Hosoya, and was named Lachnum fuscescens SANK 19096.

Strain SANK 19096 was internationally deposited as Lachnum sp. SANK 19096 on May 24, 2005 at the International Patent Organism Depository of the National Institute of Advanced Industrial Science and Technology (location: Chuo #6, 1-1-1 Higashi, Tsukuba City, Ibaraki Prefecture, Japan), and was assigned the deposit number FERM BP-10338.

As is commonly known, fungi are susceptible to mutation either naturally or by artificial manipulation (such as by irradiating with ultraviolet light, irradiating with radiation or treating with chemicals or drugs), and this applies similarly to the strains SANK 19096 of the present invention. All mutant strains of these strains SANK 19096 are included in the present invention.

In addition, strains obtained by genetic techniques such as recombination, transduction or transformation are also included in these mutant strains. Namely, cercosporamide-producing strain SANK 19096, mutant strains thereof and mycotic strains not clearly distinguished therefrom are all included in strains SANK 19096.

2. Fermentation

Cercosporamide producers can be cultured using media containing a carbon source, a nitrogen source and an inorganic salt capable of being assimilated by microorganisms in the manner of that ordinarily used for fermentative production by microorganisms.

Examples of carbon sources include glucose, fructose, maltose, sucrose, mannitol, glycerin, dextrin, oats, rye, pressed barley, cornstarch, potato, cornmeal, soybean meal, cottonseed oil, molasses, citric acid and tartaric acid, and these can be used alone or two or more can be used in combination. The content of the carbon source normally varies between 1 and 10% by weight of the amount of medium.

Examples of nitrogen sources that can be used include substances containing protein or hydrolysates thereof and inorganic salts. Specific examples of such nitrogen sources include soybean meal, wheat bran, peanut meal,

cottonseed oil, casein hydrolysates, farmamine, fish meal, corn steep liquor, peptone, meat extract, yeast, yeast extract, malt extract, sodium nitrate, ammonium nitrate and ammonium sulfate, and these can be used alone or two or more can be used in combination. The content of the nitrogen source normally varies between 0.1 and 10% by weight of the amount of medium.

Examples of nutritive inorganic salts that can be used include ordinary salts and metal salts capable of providing ions. Specific examples of salts include sodium, ammonium, calcium, phosphate, sulfate, chloride and carbonate salts. Specific examples of metal salts include potassium, calcium, cobalt, manganese, iron and magnesium salts.

In addition, vitamins such as vitamin B 1 or biotin, mycelium growth promoters such as thiamine and the like may also be added it necessary.

For liquid culturing of cercosporamide producers, silicone oil, vegetable oil or surfactant and the like can be used as an antifoaming agent. Although the pH of the medium when culturing strain SANK 19096 for the purpose of production of cercosporamide varies according to the medium components, growth temperature and the like, a pH ordinarily used for the production of fermentation products may be adopted.

Although varying according to medium components, pH and the like, the temperature for the fermentation of strain SANK 19096 is normally 10 to 30˚C, and a temperature range of 20 to 26˚C is favorable for growth. A fermentation temperature of 23 to 26˚C is preferable for the production of cercosporamide.

There are no particular limitations on the fermentation method for producing cercosporamide, and any method commonly used to culture microorganisms may be used. Examples of methods that can be used include solid culture, agitation culture, shaking culture and aeration-agitation culture.

Although the following indicates a fermentation method of culturing strain SANK 19096 as an example of a method for producing cercosporamide, the fermentation method is not limited to this method provided it is used to produce cercosporamide.

Fermentation of strain SANK 19096 normally starts with a seed culture comprised of one or two or more stages followed by shaking on a rotary shaker for 5 to 10 days (and preferably 7 days) at 20 to 26˚C (and preferably 26˚C) or until the seed culture grows sufficiently.

In the case of solid culturing of strain SANK 19096, the aforementioned seed culture broth can be inoculated into a final fermentation medium in a solid medium culture vessel such as an Erlenmeyer flask. Fermentation is carried out in the form of a standing culture by allowing the solid culture vessel to stand for 10 to 20 days (and preferably 14 days) at 20 to 26˚C (and preferably 26˚C). The desired cercosporamide can be obtained from final fermentation medium (cultured material).

In the case of liquid culturing of strain SANK 19096 on a comparatively small scale, the seed culture broth is inoculated into an Erlenmeyer flask containing the final fermentation medium followed by shaking on a rotary shaker at 20 to 26˚C (and preferably 26˚C) for several days. The desired cercosporamide can be obtained from the resulting fermented broth (culture).

On the other hand, in the case of liquid culturing of strain SANK 19096 on a comparatively large scale, it is preferable to use a suitable fermentation tank equipped with an agitator, an aeration apparatus, temperature controller and the like. The use of such an apparatus makes it possible to prepare the medium in advance in said tank. For example, the final production medium is heated at 121 ˚C for sterilization and then chilled. Next, the final production medium is inoculated with the seed culture followed by final fermentation while aerating and agitating at 26˚C. The desired cercosporamide can be obtained from the resulting fermented broth (cultured material). This type of fermentation method allows the desired cercosporamide to be produced in large quantities.

In the case of producing cercosporamide by fermentation of a cercosporamide producer of the present invention, the amount of cercosporamide produced in said culture can be monitored at time intervals by high-performance liquid chromatography (HPLC) and the like to be described later. In the case of monitoring by HPLC analysis, the product is previously extracted from the culture with a hydrophilic solvent such as acetone followed by evaporation of said hydrophilic solvent and extracting with a solvent that is not miscible with water such as ethyl acetate. Next, after evaporation of the water-immiscible solvent, the product dissolved in a suitable solvent is preferably subjected to analysis or testing.

3. Extraction and Purification of Cercosporamide

Cercosporamide can be extracted from a fermented solid medium (cultured material) or a fermented liquid medium (cultured material) of a cercosporamide producer according to a method indicated in the previous section "2. Fermentation". There are no particular limitations on the methods used to extract and purify cercosporamide providing the cercosporamide can be extracted and purified.

For example, after the completion of liquid fermentation of a cercosporamide producer, an organic solvent miscible with water such as acetone is added to the fermented broth (cultured material), the extract is separated into a soluble fraction (culture supernatant) and insoluble fraction (mycelium) by a filtration procedure using diatomaceous earth as an aid, or by a centrifugation procedure, and cercosporamide present in the resulting culture supernatant can

be further extracted and purified by utilizing the physicochemical properties of the cercosporamide. In addition, as another method, after adding a filtration aid such as Celite to a fermented broth (cultured material) following completion of liquid fermentation of a cercosporamide producer, the fermented broth is filtered, the insoluble fraction containing the resulting mycelium is immersed in an aqueous solution of a solvent miscible with water such as acetone, and the cercosporamide can then be yielded by further extraction and purification from the mycelium extract by utilizing the physicochemical properties thereof.

Examples of methods for the purification of cercosporamide from culture supernatant include, but are not limited to, an extraction method by adding an acidic substance such as hydrochloric acid to the culture supernatant to adjust the pH to 3.0 followed by the addition of ethyl acetate.

The resulting extract can be purified by adsorption column chromatography using a carrier such as silica gel or magnesium-silica gel-based Florisil, thin layer chromatography, partition column chromatography using TSK Gel ToyoPearl HW-40F (registered trademark, Tosoh Corp.) or Sephadex LH-20 (registered trademark, Amersham Biosciences K.K.) and the like, reverse phase column chromatography using Cosmosil 140C18 (registered trademark, Nacalai Tesque Inc.), or HPLC using a normal phase or reversed phase column to isolate the desired cercosporamide.

The behavior of the compound in each purification step during purification of the cercosporamide of the present invention can be monitored, for example, by HPLC under the conditions indicated below.

(HPLC Conditions for Detecting Cercosporamide)

Separation column: YMC J'sphere ODS-H80 S-4, 4.6 x 150 mm (YMC Co., Ltd.)
Mobile phase: acetonitrile: 0.4% triethylamine-phosphate buffer (pH 3.2) (45:55)
Flow rate: 1.0 ml/minute
Detection: Ultraviolet absorption at, 225 nm
Retention time: 9.3 minute

When the cercosporamide derivative having the general formula (I) according to the present invention, a pharmacologically acceptable salt thereof or an ester thereof is used as a medicine, the medicine is administered as is or after mixing with an appropriate pharmacologically acceptable excipient or diluent orally in the form of, for example, tablets, capsules, granules, powder or syrup, or parenterally by injection or in the form of suppository.

These formulations are prepared by a known method using additives such as an excipient (e.g., organic excipients including sugar derivatives such as lactose, sucrose, glucose, mannitol and sorbitol; starch derivatives such as corn starch, potato starch, $\alpha$-starch and dextrin; cellulose derivatives such as crystalline cellulose; gum arabic; dextran; and pullulan; inorganic excipients including silicate derivatives such as light anhydrous silicic acid, synthetic aluminum silicate, calcium silicate and magnesium aluminometasilicate; phosphates such as calcium hydrogen phosphate; carbonates such as calcium carbonate; and sulfates such as calcium sulfate), a lubricant (e.g., stearic acid and metal stearates such as calcium stearate and magnesium stearate; talc; colloidal silica; waxes such as beegum and spermaceti; boric acid; adipic acid; sulfates such as sodium sulfate; glycol; fumaric acid; sodium benzoate; DL leucine; fatty acid sodium salt; lauryl sulfates such as sodium lauryl sulfate and magnesium lauryl sulfate; silicic acids such as silicic acid anhydride and silicic acid hydrate; and the above starch derivatives), a binder (e.g., hydroxypropyl cellulose, hydroxypropyl methylcellulose, polyvinylpyrrolidone, macrogol and compounds listed above as excipients), a disintegrant (e.g., cellulose derivatives such as low substitution degree hydroxypropyl cellulose, carboxymethylcellulose, calcium carboxymethylcellulose and internally cross-linked sodium carboxymethylcellulose; and chemically modified starch/cellulose such as carboxymethyl starch, sodium carboxymethyl starch and cross-linked polyvinylpyrrolidone), a stabilizer (parahydroxybenzoate such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), a corrigent (e.g., sweeteners, acidulants and flavourings generally used), or a diluent.

The dose of the preparation varies according to the symptoms, the age and the like of the patient (a warm-blooded animal, in particular, a human). However, the preparation is preferably orally administered at 0.0015 mg/kg body weight (preferably 0.008 mg/kg body weight) per dose per day at the lower limit to 70 mg/kg body weight (preferably 7 mg/kg body weight) per dose per day at the upper limit or intravenously administered at 0.00015 mg/kg body weight (preferably 0.0008 mg/kg body weight) per dose per day at the lower limit to 8.5 mg/kg body weight (preferably 5 mg/kg body weight) per dose per day at the upper limit to an adult once to six times per day according to symptoms.

Examples

The present invention will be described in more detail below with reference to Examples, Reference Examples,

Test Example and Preparation Examples; however, the scope of the present invention is not limited thereto.

(Example 1) (9aS)-8-Acetyl-N-benzyl-1,7-dihydroxy-3-methoxy-9a-methyl-9-oxo-9,9a-dihydrodibenzo[b,d]furan-4-carboxamide (exemplary compound No. 1-1)

(1a) (9aS)-8-Acetyl-1,7-dihydroxy-3-methoxy-9a-methyl-9-oxo-9,9a-dihydrodibenzo[b,d]furan-4-carboxamide

Cercosporamide obtained in Reference Example 3 (10.49 g, 31.7 mmol) was dissolved in dimethylformamide (50 mL). Methyl iodide (5.0 mL, 80 mmol) and potassium carbonate (9.03 g, 65.3 mmol) were added, and the mixture was stirred in a nitrogen atmosphere at room temperature for 24 hours. Potassium carbonate was separated by filtration from the reaction solution and 0.1 N hydrochloric acid (200 mL) was added, followed by extraction with ethyl acetate (500 mL). The organic layer was washed with brine and then dried over anhydrous sodium sulfate. The solvent was evaporated under reduced pressure and the resulting residue was recrystallized from ethyl acetate to give the target compound (7.19 g, yield: 66%) as yellow crystals.
$^1$H-NMR ($CDCl_3$, 400MHz):$\delta$ ppm: 1.76 (3H, s), 2.65 (3H, s), 3.94 (3H, s), 5.73 (1H, brs), 6.04 (1H, s), 6.36 (1H,s), 7.10 (1H, brs), 10.79 (1H, s), 18.83 (1H, s).
MS (ESI) m/z: 346.0933 $(M+H)^+$

(1b) (9aS)-8-Acetyl-N-benzyl-1,7-dihydroxy-3-methoxy-9a-methyl-9-oxo-9,9a-dihydrodibenzo[b,d]furan-4-carboxamide

(9aS)-8-Acetyl-1,7-dihydroxy-3-methoxy-9a-methyl-9-oxo-9,9a-dihydrodibenzo[b,d]furan-4-carboxamide produced in Example (1a) (1.11 g, 3.21 mmol) was suspended in toluene (50 mL). Benzaldehyde (1.30 mL, 12.7 mmol), triethylsilane (2.00 mL, 12.6 mmol) and trifluoroacetic acid (1.00 mL, 13.0 mmol) were added, and the mixture was stirred with heating to reflux in a nitrogen atmosphere for eight hours. The reaction solution was cooled to room temperature and then the solvent was evaporated under reduced pressure. The resulting residue was purified by silica gel column chromatography (elution solvent: methylene chloride/methanol = 80/1 to 60/1) to give the target compound (1.04 g, yield: 74%) as a yellow solid.
$^1$H-NMR ($CDCl_3$, 500MHz):$\delta$ ppm: 1.76 (3H, s), 2.65 (3H, s), 3.87 (3H, s), 4.63 (1H, dd, J=5.4, 15.1Hz), 4.69 (1H, dd, J=5.4, 15.1Hz), 6.02 (1H, s), 6.33 (1H,s), 7.28-7.38 (6H, m), 10.70 (1H, s), 18.82 (1H, s).
MS (ESI) m/z: 436.1428 $(M+H)^+$.

(Example 2) (9aS)-8-Acetyl-N-benzyl-1,3,7-trihydroxy-9a-methyl-9-oxo-9,9a-dihydrodibenzo[b,d]furan-4-carboxamide (exemplary compound No. 2-1)

(2a) (9aS)-8-Acetyl-3-(benzyloxy)-1,7-dihydroxy-9a-methyl-9-oxo-9,9a-dihydrodibenzo[b,d]furan-4-carboxamide

Cercosporamide obtained in Reference Example 3 (2.07 g, 6.25 mmol) was dissolved in dimethylformamide (30 mL). Benzyl bromide (1.90 mL, 16.0 mmol) and potassium carbonate (2.40 g, 17.4 mmol) were added, and the mixture was stirred in a nitrogen atmosphere at 70°C for five hours. Potassium carbonate was separated by filtration from the reaction solution and 0.1 N hydrochloric acid (100 mL) was added, followed by extraction with ethyl acetate (300 mL). The organic layer was washed with brine and then dried over anhydrous sodium sulfate. The solvent was evaporated under reduced pressure, and the resulting residue was purified by silica gel column chromatography (elution solvent: methylene chloride/methanol = 50/1 to 20/1) to give the target compound (805 mg, yield: 31%) as a yellow solid.
$^1$H-NMR ($CDCl_3$, 400MHz):$\delta$ ppm: 1.77 (3H, s), 2.65 (3H, s), 5.14 (2H, d, J=1.9Hz), 5.61 (1H, brs), 6.04 (1H, s), 6.45 (1H,s), 7.10 (1H, brs), 7.40-7.46 (5H, m), 10.81 (1H, s), 18.83 (1H, S).
MS (ESI) m/z: 444.1049 $(M+H)^+$

(2b) (9aS)-8-Acetyl-N-benzyl-3-(benzyloxy)-1,7-dihydroxy-9a-methyl-9-oxo-9,9a-dihydrodibenzo[b,d]furan-4-carboxamide

Reaction and post-treatment were carried out in accordance with Example (1b) using (9aS)-8-acetyl-3-(benzyloxy)-l,7-dihydroxy-9a-methyl-9-oxo-9,9a-dihydrodibenzo[b,d]furan-4-carboxamide produced in Example (2a) (1.46 g, 3.46 mmol), benzaldehyde (1.40 mL, 13.7 mmol), triethylsilane (2.20 mL, 13.8 mmol), trifluoroacetic acid (1.00 mL, 12.6 mmol) and toluene (50 mL) to give the target compound (1.05 g, yield: 59%) as a yellow solid.
$^1$H-NMR($CDCl_3$, 500MHz):$\delta$ ppm: 1.77 (3H, s), 2.65 (3H, s), 4.46-4.58 (2H, m), 5.06 (2H, d, J=3.4Hz), 6.04 (1H, s), 6.43 (1H,s), 7.13-7.38 (10H, m), 7.45 (1H, brs), 10.73 (1H, s), 18.82 (1H, s).
MS (ESI) m/z: 534.1540 $(M+Na)^+$

(2c) (9aS)-8-Acetyl-N-benzyl-1,3,7-trihydroxy-9a-methyl-9-oxo-9,9a-dihydrodibenzo[b,d]furan-4-carboxamide

(9aS)-8-Acetyl-N-benzyl-3-(benzyloxy)-1,7-dihydroxy-9a-methyl-9-oxo-9,9a-dihydrodibenzo[b,d]furan-4-carboxamide produced in Example (2b) (922 mg, 1.80 mmol) was dissolved in a mixed solvent of ethyl acetate (10 mL) and ethanol (15 mL). A 10% palladium-carbon catalyst (157 mg) was added, and the mixture was stirred in a hydrogen atmosphere at 1 atm at room temperature for six hours. The palladium-carbon catalyst was separated by filtration from the reaction solution, and then the solvent was evaporated under reduced pressure. The resulting residue was purified by silica gel column chromatography (elution solvent: methylene chloride/methanol = 100/1) to give the target compound (499 mg, yield: 66%) as a yellow solid.
$^1$H-NMR ($CDCl_3$, 500MHz):$\delta$ ppm: 1.75 (3H, s), 2.66 (3H, s), 4.63-4.72 (2H, m), 5.92 (1H, s), 6.34 (1H,s), 7.31-7.41 (6H, m), 10.49 (1H, s), ,13.20 (1H, s), 18.82 (1H, s).
MS (FAB) m/z: 422.1216 $(M+H)^+$

(Example 3) (9aS)-8-Acetyl-1,7-dihydroxy-3-methoxy-N-(2-methoxybenzyl)-9a-methyl-9-oxo-9,9a-dihydrodibenzo[b,d]furan-4-carboxamide (exemplary compound No. 1-19)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.900 g, 2.61 mmol), 2-anisaldehyde (1.26 mL, 10.4 mmol), triethylsilane (1.66 mL, 10.4 mmol), trifluoroacetic acid (0.800 mL, 10.4 mmol) and toluene (27 mL) to give the target compound (0.752 g, yield: 62%) as a yellow solid.
$^1$H-NMR ($CDCl_3$, 400MHz):$\delta$ ppm: 1.74 (3H, s), 2.64 (3H, s), 3.87 (3H, s), 3.89 (3H, s), 4.59-4.64 (2H, m), 6.00 (1H, s), 6.31 (1H, s), 6.90-6.93 (2H, m), 7.25-7.29 (1H, m), 7.37 (1H, d, J=7.5Hz), 7.58 (1H, brs), 10.66 (1H, s), 18.80 (1H, s).
MS (FAB) m/z: 466.1490 $(M+H)^+$

(Example 4) (9aS)-8-Acetyl-1,7-dihydroxy-3-methoxy-N-(3-methoxybenzyl)-9a-methyl-9-oxo-9,9a-dihydrodibenzo[b,d]furan-4-carboxamide (exemplary compound No. 1-18)

Reaction and post-treatment were carried out in accordance with Example (1b) using (9aS)-8-acetyl-1,7-di.hydroxy-3-methoxy-9a-methyl-9-oxo-9,9a-dihydrodibenzo[b,d]furan-4-carboxamide produced in Example (1a) (0.900 g, 2.61 mmol), 3-anisaldehyde (1.27 mL, 10.4 mmol), triethylsilane (1.66 mL, 10.4 mmol), trifluoroacetic acid (0.800 mL, 10.4 mmol) and toluene (27 mL) to give the target compound (0.868 g, yield: 72%) as a yellow solid.
$^1$H-NMR ($CDCl_3$, 400MHz):$\delta$ ppm: 1.76 (3H, s), 2.65 (3H, s), 3.81 (3H, s), 3.88 (3H, s), 4.61-4.65 (2H, m), 6.02 (1H, s), 6.33 (1H, s), 6.82 (1H, d, J=8.3Hz), 6.92-6.96 (2H, m), 7.24-7.30 (2H, m), 10.69 (1H, s), 18.81 (1H, s).
MS (FAB) m/z: 466.1490 $(M+H)^+$

(Example 5) (9aS)-8-Acetyl-1,7-dihydroxy-3-methoxy-N-(4-methoxybenzyl)-9a-methyl-9-oxo-9,9a-dihydrodibenzo[b,d]furan-4-carboxamide (exemplary compound No. 1-17)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.900 g, 2.61 mmol), 4-anisaldehyde (0.705 mL, 5.79 mmol), triethylsilane (0.922 mL, 5.79 mmol), trifluoroacetic acid (0.446 mL, 5.79 mmol) and toluene (15 mL) to give the target compound (0.375 g, yield: 56%) as a yellow solid.
$^1$H-NMR ($CDCl_3$, 500MHz):$\delta$ ppm: 1.75 (3H, s), 2.65 (3H, s), 3.80 (3H, s), 3.86 (3H, s), 4.56-4.60 (2H, m), 6.00 (1H, s), 6.32 (1H, s), 6.88 (2H, d, J=8.3Hz), 7.29 (2H, d, J=8.3Hz), 10.68 (1H, s), 18.81 (1H, s).
MS (FAB) m/z: 466.1507 $(M+H)^+$.

(Example 6) (9aS)-8-Acetyl-1,7-dihydroxy-3-methoxy-N-(2-fluorobenzyl)-9a-methyl-9-oxo-9,9a-dihydrodibenzo[b,d]furan-4-carboxamide (exemplary compound No. 1-4)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.800 g, 2.32 mmol), 2-fluorobenzaldehyde (0.976 g, 9.27 mmol), triethylsilane (1.48 mL, 9.27 mmol), trifluoroacetic acid (0.710 mL, 9.27 mmol) and toluene (24 mL) to give the target compound (0.786 g, yield: 75%) as a yellow solid.
$^1$H-NMR ($CDCl_3$, 500MHz):$\delta$ ppm: 1.74 (3H, s), 2.65 (3H, s), 3.90 (3H, s), 4.63-4.72 (2H, m), 6.02 (1H, s), 6.33 (1H, s), 7.04-7.13 (2H, m), 7.26 (1H, m), 7.45 (1H, t, J=7.9Hz), 7.58 (1H, brs), 10.72 (1H, s), 18.82 (1H, s).
MS (ESI) m/z: 454.1304 $(M+H)^+$

(Example 7) (9aS)-8-Acetyl-1,7-dihydroxy-3-methoxy-N-(3-fluorobenzyl)-9a-methyl-9-oxo-9,9a-dihydrodibenzo[b,d]furan-4-carboxamide (exemplary compound No. 1-3)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.550 g, 1.59 mmol), 3-fluorobenzaldehyde (0.676 g, 6.37 mmol), triethylsilane (1.01 mL, 6.37 mmol), trifluoroacetic acid (0.491 mL, 6.37 mmol) and toluene (16.5 mL) to give the target compound (0.332 g, yield: 46%) as a yellow solid.
$^1$H-NMR ($CDCl_3$, 500MHz):δ ppm: 1.76 (3H, s), 2.65 (3H, s), 3.90 (3H, s), 4.60-4.71 (2H, m), 6.02 (1H, s), 6.35 (1H, s), 6.97 (1H, m), 7.09 (1H, d, J=9.8Hz), 7.14 (1H, d, J=7.3Hz), 7.31 (1H, m), 7.40 (1H, brs), 10.73 (1H, s), 18.83 (1H, s).
MS (ESI) m/z: 454.1311 $(M+H)^+$

(Example 8) (9aS)-8-Acetyl-1,7-dihydroxy-3-methoxy-N-(4-fluorobenzyl)-9a-methyl-9-oxo-9,9a-dihydrodibenzo[b,d]furan-4-carboxamide (exemplary compound No. 1-2)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.550 g, 1.59 mmol), 4-fluorobenzaldehyde (0.683 g, 6.37 mmol), triethylsilane (1.01 mL, 6.37 mmol), trifluoroacetic acid (0.491 mL, 6.37 mmol) and toluene (16.5 mL) to give the target compound (0.333 g, yield: 46%) as a yellow solid.
$^1$H-NMR ($CDCl_3$,400MHz):δ ppm: 1.75 (3H, s), 2.65 (3H, s), 3.88 (3H, s), 4.55-4.67 (2H, m), 6.02 (1H, s), 6.33 (1H, s), 7.03 (2H, t, J=8.7Hz), 7.32-7.35 (3H, m), 10.72 (1H, s), 18.82 (1H, s).
MS (ESI) m/z: 454.1307 $(M+H)^+$

(Example 9) (9aS)-8-Acetyl-1,7-dihydroxy-3-methoxy-9a-methyl-9-oxo-N-(pyridin-3-ylmethyl)-9,9a-dihydrodibenzo[b,d]furan-4-carboxamide (exemplary compound No. 1-463)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.900 g, 2.61 mmol), 3-pyridinecarboxaldehyde (0.980 mL, 10.4 mmol), triethylsilane (1.66 mL, 10.4 mmol), trifluoroacetic acid (0.800 mL, 10.4 mmol) and toluene (27 mL) to give the target compound (0.221 g, yield: 19%) as a yellow solid.
$^1$H-NMR ($CDCl_3$, 500MHz):δ ppm: 1.76 (3H, s), 2.65 (3H, s), 3.92 (3H, s), 4.63-4.75 (2H, m), 6.03 (1H, s), 6.35 (1H, s), 7.39 (1H, t, J=6.0Hz), 7.60 (1H, brs), 7.88 (1H, d, J=8.3Hz), 8.57 (1H, d, J=4.4Hz), 8.68 (1H, s), 10.78 (1H, s), 18.83 (1H, s). MS (ESI) m/z: 437.1369 $(M+H)^+$

(Example 10) (9aS)-8-Acetyl-1,7-dihydroxy-3-methoxy-9a-methyl-N-(1-naphthylmethyl)-9-oxo-9,9a-dihydrodibenzo[b,d]furan-4-carboxamide (exemplary compound No. 1-199)

(9aS)-8-Acetyl-1,7-dihydroxy-3-methoxy-9a-methyl-9-oxo-9,9a-dihydrodibenzo[b,d]furan-4-carboxamide produced in Example (1a) (5.04 g, 14.6 mmol) was suspended in acetonitrile (200 mL). 1-Naphthaldehyde (4.00 mL, 29.5 mmol), triethylsilane (4.70 mL, 29.5 mmol), and trifluoroacetic acid (2.30 mL, 29.9 mmol) were added, and the mixture was stirred in a nitrogen atmosphere at room temperature for three days. The solvent was evaporated under reduced pressure, and the resulting residue was purified by silica gel column chromatography (elution solvent: methylene chloride/methanol = 80/1 to 60/1) to give the target compound (5.46 g, yield: 77%) as a yellow solid.
$^1$H-NMR ($CDCl_3$, 400MHz):δ ppm: 1.75 (3H, s), 2.64 (3H, s), 3.72 (3H, s), 5.03 (1H, dd, J=4.9, 14.7Hz), 5.11 (1H, dd, J=5.4, 14.7Hz), 6.00 (1H, s), 6.27 (1H,s), 7.29 (1H, brs), 7.43-7.59 (3H, m), 7.83 (1H, d, J=8.6Hz), 7.90 (1H, d, J=8.6Hz), 8.12 (1H, d, J=8.6Hz), 10.66 (1H, s), 18.82 (1H, s).
MS (FAB) m/z: 486.1551 $(M+H)^+$.

(Example 11) (9aS)-8-Acetyl-N-(2,6-difluorobenzyl)-1,7-dihydroxy-3-methoxy-9a-methyl-9-oxo-9,9a-dihydrodibenzo[b,d]furan-4-carboxamide (exemplary compound No. 1-30)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (702 mg, 2.03 mmol), 2,6-difluorobenzaldehyde (870 μL, 8.08 mmol), triethylsilane (1.30 mL, 8.16 mmol), trifluoroacetic acid (630 μL, 8.18 mmol) and toluene (20 mL) to give the target compound (559 mg, yield: 58%) as a yellow solid.
$^1$H-NMR ($CDCl_3$, 500MHz):δ ppm: 1.73 (3H, s), 2.64 (3H, s), 3.89 (3H, s), 4.65 (1H, dd, J=5.9, 14.6Hz), 4.78 (1H, dd, J=5.9, 14.6Hz), 6.02 (1H, s), 6.32 (1H,s), 6.91 (2H, t, J=7.8Hz), 7.23 (1H, m), 7.66 (1H, brs), 10.72 (1H, s), 18.82 (1H, s).
MS (ESI) m/z: 472.1198 $(M+H)^+$

(Example 12) (9aS)-8-Acetyl-N-(2,3-difluorobenzyl)-1,7-dihydroxy-3-methoxy-9a-methyl-9-oxo-9,9a-dihydrodibenzo[b,d]furan-4-carboxamide (exemplary compound No. 1-27)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (602 mg, 1.74 mmol), 2,3-difluorobenzaldehyde (760 μL, 6.95 mmol), triethylsilane (1.10 mL, 6.91 mmol), trifluoroacetic acid (540 μL, 7.01 mmol) and toluene (20 mL) to give the target compound (420 mg, yield: 51%) as a yellow solid.
$^1$H-NMR ($CDCl_3$, 500MHz):δ ppm: 1.74 (3H, s), 2.65 (3H, s), 3.92 (3H, s), 4.64 (1H, dd, J=5.9, 15.1Hz), 4.72 (1H, dd, J=5.9, 15.1Hz), 6.02 (1H, s), 6.34 (1H,s), 7.02-7.11 (2H, m), 7.22(1H, t, J=7.8Hz), 7.66 (1H, brs), 10.72 (1H, s), 18.83 (1H, s).
MS (ESI) m/z: 472.1205 $(M+H)^+$

(Example 13) (9aS)-8-Acetyl-N-(2,4-difluorobenzyl)-1,7-dihydroxy-3-methoxy-9a-methyl-9-oxo-9,9a-dihydrodibenzo[b,d]furan-4-carboxamide (exemplary compound No. 1-28)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (607 mg, 1.76 mmol), 2,4-difluorobenzaldehyde (770 μL, 7.04 mmol), triethylsilane (1.12 mL, 7.03 mmol), trifluoroacetic acid (540 μL, 7.01 mmol) and toluene (20 mL) to give the target compound (356 mg, yield: 43%) as a yellow solid.
$^1$H-NMR ($CDCl_3$, 500MHz):δ ppm: 1.74 (3H, s), 2.65 (3H, s), 3.90 (3H, s), 4.58 (1H, dd, J=5.9, 15.1Hz), 4.65 (1H, dd, J=5.9, 15.1Hz), 6.02 (1H, s), 6.33 (1H,s), 6.80-6.87 (2H, m), 7.45 (1H, m), 7.59 (1H, brs), 10.74 (1H, s), 18.83 (1H, s).
MS (ESI) m/z: 472.1214 $(M+H)^+$

(Example 14) (9aS)-8-Acetyl-N-(2,5-difluorobenzyl)-1,7-dihydroxy-3-methoxy-9a-methyl-9-oxo-9,9a-dihydrodibenzo[b,d]furan-4-carboxamide (exemplary compound No. 1-29)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (606 mg, 1.76 mmol), 2,5-difluorobenzaldehyde (760 μL, 7.01 mmol), triethylsilane (1.12 mL, 7.03 mmol), trifluoroacetic acid (540 μL, 7.01 mmol) and toluene (20 mL) to give the target compound (487 mg, yield: 59%) as a yellow solid.
$^1$H-NMR ($CDCl_3$, 500MHz):δ ppm: 1.75 (3H, s), 2.65 (3H, s), 3.92 (3H, s), 4.62 (1H, dd, J=5.9, 15.6Hz), 4.69 (1H, dd, J=5.9, 15.6Hz), 6.03 (1H, s), 6.35 (1H,s), 6.90-7.03 (2H, m), 7.18 (1H, m), 7.67 (1H, brs), 10.75 (1H, s), 18.83 (1H, s).
MS (ESI) m/z: 472.1207 $(M+H)^+$

(Example 15) (9aS)-8-Acetyl-N-[(4-fluoro-1-naphthyl)methyl]-1,7-dihydroxy-3-methoxy-9a-methyl-9-oxo-9,9a-dihydrodibenzo[b,d]furan-4-carboxamide (exemplary compound No. 1-203)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.350 g, 0.101 mmol), 4-fluoro-1-naphthalenecarboxaldehyde (0.706 mL, 4.05 mmol); triethylsilane (0.645 mL, 4.05 mmol), trifluoroacetic acid (0.312 mL, 4.05 mmol) and toluene (10.5 mL) to give the target compound (0.304 g, yield: 60%) as a yellow solid. $^1$H-NMR ($CDCl_3$, 500MHz):δ ppm: 1.76 (3H, s), 2.65 (3H, s), 3.72 (3H, s), 3.88 (3H, s), 5.00-5.10 (2H, m), 6.02 (1H, s), 6.28 (1H, s), 7.12 (1H, m), 7.48 (1H, m), 7.59-7.65 (2H, m), 8.11-8.19 (2H, m), 10.69 (1H, s), 18.84 (1H, s).
MS (FAB) m/z: 504.1447 $(M+H)^+$.

(Example 16) (9aS)-8-Acetyl-N-(2-chlorobenzyl)-1,7-dihydroxy-3-methoxy-9a-methyl-9-oxo-9,9a-dihydrodibenzo[b,d]furan-4-carboxamide (exemplary compound No. 1-7)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (607 mg, 1.76 mmol), 2-chlorobenzaldehyde (790 μL, 7.02 mmol), triethylsilane (1.12 mL, 7.03 mmol), trifluoroacetic acid (540 μL, 7.01 mmol) and toluene (20 mL) to give the target compound (440 mg, yield: 53%) as a yellow solid.
$^1$H-NMR ($CDCl_3$, 500MHz):δ ppm: 1.74 (3H, s), 2.65 (3H, s), 3.91 (3H, s), 4.64-4.76 (2H, m), 6.02 (1H, s), 6.33 (1H,s), 7.21-7.25 (2H, m), 7.38 (1H, dd, J=6.5, 2.4Hz), 7.52 (1H, dd, J=6.5, 2.7Hz), 7.79 (1H, brs), 10.73 (1H, s), 18.82 (1H, s).
MS (ESI) m/z: 470.1008 $(M+H)^+$

(Example 17) (9aS)-8-Acetyl-N-(2-bromobenzyl)-1,7-dihydroxy-3-methoxy-9a-methyl-9-oxo-9,9a-dihydrodibenzo[b,d]furan-4-carboxamide (exemplary compound No. 1-10)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (606 mg, 1.75 mmol), 2-bromobenzaldehyde (810 μL, 7.00 mmol), triethylsilane (1.12 mL, 7.03 mmol), trifluoroacetic acid (540 μL, 7.01 mmol) and toluene (20 mL) to give the target compound (332 mg, yield: 37%) as a yellow solid.
$^1$H-NMR ($CDCl_3$, 500MHz):δ ppm: 1.74 (3H, s), 2.65 (3H, s), 3.92 (3H, s), 4.62-4.75 (2H, m), 6.03 (1H, s), 6.33 (1H,s), 7.15 (1H, t, J=7.8Hz), 7.29 (1H, t, J=7.8Hz), 7.54 (1H, d, J=7.8Hz), 7.57 (1H, d, J=7.8Hz), 7.83 (1H, brs), 10.73 (1H, s), 18.83 (1H, s).
MS (ESI) m/z: 514.0505 $(M+H)^+$

(Example 18) (9aS)-8-Acetyl-N-(2-fluoro-5-nitrobenzyl)-1,7-dihydroxy-3-methoxy-9a-methyl-9-oxo-9,9a-dihydrodibenzo[b,d]furan-4-carboxamide (exemplary compound No. 1-55)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (610 mg, 1.77 mmol), 2-fluoro-5-nitrobenzaldehyde (1.19 g, 7.03 mmol), triethylsilane (1.12 mL, 7.03 mmol), trifluoroacetic acid (540 μL, 7.01 mmol) and toluene (20 mL) to give the target compound (170 mg, yield: 19%) as a yellow solid.
$^1$H-NMR ($CDCl_3$, 500MHz):δ ppm: 1.76 (3H, s), 2.65 (3H, s), 3.98 (3H, s), 4.68-4.82 (2H, m), 6.03 (1H, s), 6.38 (1H,s), 7.21 (1H, t, J=9.0Hz), 7.59 (1H, t, brs), 8.18 (1H, m), 8.39 (1H, dd, J=6.4, 2.9Hz), 10.78 (1H, s), 18.83 (1H, s).
MS (ESI) m/z: 499.1148 $(M+H)^+$

(Example 19) (9aS)-8-Acetyl-1,7-dihydroxy-3-methoxy-9a-methyl-9-oxo-N-(pentafluorobenzyl)-9,9a-dihydrodibenzo[b,d]furan-4-carboxamide (exemplary compound No. 1-164)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (608 mg, 1.76 mmol), pentafluorobenzaldehyde (870 μL, 7.06 mmol), triethylsilane (1.12 mL, 7.03 mmol), trifluoroacetic acid (540 μL, 7.01 mmol) and toluene (20 mL) to give the target compound (261 mg, yield: 28%) as a yellow solid.
$^1$H-NMR ($CDCl_3$, 500MHz):δ ppm: 1.73 (3H, s), 2.65 (3H, s), 3.93 (3H, s), 4.62 (1H, dd, J=6.4, 14.7Hz), 4.76 (1H, dd, J=6.4, 14.7Hz), 6.03 (1H, s), 6.34 (1H,s), 7.83 (1H, brs), 10.80 (1H, s), 18.83 (1H, s).
MS (ESI) m/z: 526.0915 $(M+H)^+$

(Example 20) (9aS)-8-Acetyl-1,7-dihydroxy-3-methoxy-9a-methyl-9-oxo-N-(4-phenoxybenzyl)-9,9a-dihydrodibenzo[b,d]furan-4-carboxamide (exemplary compound No. 1-23)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.600 g, 1.74 mmol), 4-phenoxybenzaldehyde (1.22 mL, 6.95 mmol), triethylsilane (1.11 mL, 6.95 mmol), trifluoroacetic acid (0.535 mL, 6.95 mmol) and toluene (18.0 mL) to give the target compound (0.363 g, yield: 40%) as a yellow solid.
$^1$H-NMR ($CDCl_3$, 500MHz):δ ppm: 1.75 (3H, s), 2.65 (3H, s), 3.88 (3H, s), 4.58-4.64 (2H, m), 6.01 (1H, s), 6.33 (1H, s), 6.80-7.10 (4H, m), 7.26-7.40 (5H, m), 10.70 (1H, s), 18.82 (1H, s).
MS (ESI) m/z: 528.1653 $(M+H)^+$.

(Example 21) (9aS)-8-Acetyl-1,7-dihydroxy-3-methoxy-9a-methyl-N-(2-methylbenzyl)-9-oxo-9,9a-dihydrodibenzo[b,d]furan-4-carboxamide (exemplary compound No. 1-13)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 5.79 mmol), 2-methylbenzaldehyde (0.268 mL, 2.32 mmol), triethylsilane (0.369 mL, 2.32 mmol), trifluoroacetic acid (0.179 mL, 2.32 mmol) and toluene (6.0 mL) to give the target compound (0.212 g, yield: 82%) as a yellow solid.
$^1$H-NMR ($CDCl_3$, 500MHz):δ ppm: 1.75 (3H, s), 2.39 (3H, s), 2.65 (3H, s), 3.86 (3H, s), 4.58-4.68 (2H, m), 6.01 (1H, s), 6.32 (1H, s), 7.11-7.22 (4H, m), 7.34 (1H, m), 10.67 (1H, s), 18.81 (1H, s).
MS (ESI) m/z: 450.1570 $(M+H)^+$

(Example 22) (9aS)-8-Acetyl-1,7-dihydroxy-3-methoxy-9a-methyl-N-(3-methylbenzyl)-9-oxo-9,9a-dihydrodibenzo[b,d]furan-4-carboxamide (exemplary compound No. 1-12)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 5.79 mmol), 3-methylbenzaldehyde (0.273 mL, 2.32 mmol), triethylsilane (0.369 mL, 2.32 mmol), trifluoroacetic acid (0.179 mL, 2.32 mmol) and toluene (6.0 mL) to give the target compound (0.217 g, yield: 83%) as a yellow solid.
$^1$H-NMR ($CDCl_3$, 500MHz):$\delta$ ppm: 1.76 (3H, s), 2.36 (3H, s), 2.65 (3H, s), 3.87 (3H, s), 4.57-4.67 (2H, m), 6.02 (1H, s), 6.33 (1H, s), 7.09-7.29 (5H, m), 10.68 (1H, s), 18.81 (1H, s).
MS (ESI) m/z: 450.1560 $(M+H)^+$

(Example 23) (9aS)-8-Acetyl-1,7-dihydroxy-3-methoxy-9a-methyl-N-(4-methylbenzyl)-9-oxo-9,9a-dihydrodibenzo[b,d]furan-4-carboxamide (exemplary compound No. 1-11)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 5.79 mmol), 4-methylbenzaldehyde (0.273 mL, 2.32 mmol), triethylsilane (0.369 mL, 2.32 mmol), trifluoroacetic acid (0.179 mL, 2.32 mmol) and toluene (6.0 mL) to give the target compound (0.193 g, yield: 74%) as a yellow solid.
$^1$H-NMR ($CDCl_3$, 500MHz):$\delta$ ppm: 1.75 (3H, s), 2.34 (3H, s), 2.65 (3H, s), 3.86 (3H, s), 4.55-4.65 (2H, m), 6.01 (1H, s), 6.32 (1H, s), 7.14-7.18 (2H, m), 7.25-7.28 (3H, m), 10.67 (1H, s), 18.81 (1H, s).
MS (ESI) m/z: 450.1559 $(M+H)^+$

(Example 24) (9aS)-8-Acetyl-N-(4-chlorobenzyl)-1,7-dihydroxy-3-methoxy-9a-methyl-9-oxo-9,9a-dihydrodibenzo[b,d]furan-4-carboxamide (exemplary compound No. 1-5)

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (253 mg, 0.733 mmol), 4-chlorobenzaldehyde (412 mg, 2.93 mmol), triethylsilane (470 $\mu$L, 2.95 mmol), trifluoroacetic acid (230 $\mu$L, 2.99 mmol) and toluene (10 mL) to give the target compound (151 mg, yield: 44%) as a yellow solid.
$^1$H-NMR ($CDCl_3$, 500MHz):$\delta$ ppm: 1.76 (3H, s), 2.66 (3H, s), 3.89 (3H, s), 4.59 (1H, dd, J=5.9, 15.6Hz), 4.65 (1H, dd, J=5.9, 15.6Hz), 6.03 (1H, s), 6.35 (1H,s), 7.32 (4H, s), 7.40 (1H, brs), 10.73 (1H, s), 18.83 (1H, s).
MS (FAB) m/z: 470.0996 $(M+H)^+$

(Example 25) (9aS)-8-Acetyl-1,7-dihydroxy-3-methoxy-9a-methyl-N-[(4-methyl-1-naphthyl)methyl]-9-oxo-9,9a-dihydrodibenzo[b,d]furan-4-carboxamide (exemplary compound No. 1-212)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (301 mg, 0.872 mmol), 4-methyl-1-naphthaldehyde (469 mg, 2.76 mmol), triethylsilane (420 $\mu$L, 2.64 mmol), trifluoroacetic acid (210 $\mu$L, 2.73 mmol) and acetonitrile (10 mL) to give the target compound (330 mg, yield: 66%) as a yellow solid.
$^1$H-NMR ($CDCl_3$, 400MHz):$\delta$ ppm: 1.75 (3H, s), 2.64 (3H, s), 2.70 (3H, s), 3.70 (3H, s), 5.02 (1H, dd, J=5.2, 14.7Hz), 5.08 (1H, dd, J=5.2, 14.7Hz), 6.00 (1H, s), 6.26 (1H,s), 7.21-7.60 (5H, s), 8.05-8.14 (2H, m), 10.65 (1H, s), 18.82 (1H, s).
MS (FAB) m/z: 500.1703 $(M+H)^+$.

(Example 26) (9aS)-8-Acetyl-1,7-dihydroxy-3-methoxy-N-[(4-methoxy-1-naphthyl)methyl]-9a-methyl-9-oxo-9,9a-dihydrodibenzo[b,d]furan-4-carboxamide (exemplary compound No. 1-269)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (303 mg, 0.877 mmol), 4-methoxy-1-naphthaldehyde (503 mg, 2.70 mmol), triethylsilane (420 $\mu$L, 2.64 mmol), trifluoroacetic acid (210 $\mu$L, 2.73 mmol) and acetonitrile (10 mL) to give the target compound (247 mg, yield: 55%) as a yellow solid.
$^1$H-NMR ($CDCl_3$, 400MHz):$\delta$ ppm: 1.74 (3H, s), 2.64 (3H, s), 3.68 (3H, s), 4.01 (3H, s), 4.96 (1H, dd, J=5.2, 14.3Hz), 5.02 (1H, dd, J=5.2, 14.3Hz), 6.00 (1H, s), 6.25 (1H,s), 6.77 (1H, d, J=7.9Hz), 7.17 (1H, brs), 7.47 (1H, d, J=7.9Hz), 7.50-7.60 (2H, m), 8.05 (1H, d, J=8.3Hz), 8.33 (1H, d, J=8.3Hz), 10.64 (1H, s), 18.82 (1H, s).
MS (FAB) m/z: 516.1651 $(M+H)^+$

(Example 27) (9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide (exemplary compound No. 1-210)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (304 mg, 0.880 mmol), 2-methyl-1-naphthaldehyde (448 mg, 2.63 mmol), triethylsilane (420 μL, 2.64 mmol), trifluoroacetic acid (210 μL, 2.73 mmol) and acetonitrile (10 mL) to give the target compound (379 mg, yield: 86%) as a yellow solid.
$^1$H-NMR ($CDCl_3$, 500MHz):δ ppm: 1.74 (3H, s), 2.63 (3H, s), 2.64 (3H, s), 3.62 (3H, s), 5.05 (1H, dd, J=4.9, 14.2Hz), 5.12 (1H, dd, J=4.9, 14.2Hz), 6.00 (1H, s), 6.23 (1H,s), 7.02 (1H, brs), 7.35 (1H, d, J=8.3Hz), 7.45-7.66 (2H, m), 7.75 (1H, d, J=8.8Hz), 7.84 (1H, d, J=8.3Hz), 8:12 (1H, d, J=8.8Hz), 10.63 (1H, s), 18.82 (1H, s). MS (FAB) m/z: 516.1651 $(M+H)^+$

(Example 28) (9aS)-8-Acetyl-N-(2,6-dichlorobenzyl)-1,7-dihydroxy-3-methoxy-9a-methyl-9-oxo-9,9a-dihydrodibenzo[b,d]furan-4-carboxamide (exemplary compound No. 1-36)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (300 mg, 0.869 mmol), 4-chlorobenzaldehyde (468 mg, 2.67 mmol), triethylsilane (420 μL, 2.64 mmol), trifluoroacetic acid (210 μL, 2.73 mmol) and acetonitrile (10 mL) to give the target compound (292 mg, yield: 67%) as a yellow solid.
$^1$H-NMR ($CDCl_3$, 500MHz):δ ppm: 1.73 (3H, s), 2.64 (3H, s), 3.87 (3H, s), 4.86 (1H, dd, J=5.9, 13.9Hz), 5.01 (1H, dd, J=5.9, 13.9Hz), 6.03 (1H, s), 6.31 (1H,s), 7.19 (1H, t, J=8.1Hz), 7.34 (2H, d, J=8.1Hz), 7.72 (1H, brs), 10.73 (1H, s), 18.82 (1H, s).
MS (FAB) m/z: 504.0616 $(M^+)$

(Example 29) (9aS)-8-Acetyl-N-[(4-bromo-1-naphthyl)methyl]-1,7-dihydroxy-3-methoxy-9a-methyl-9-oxo-9,9a-dihydrodibenzo[b,d]furan-4-carboxamide (exemplary compound No. 1-209)

(29a) 4-Bromo-1-naphthaldehyde

1-Bromo-4-methylnaphthalene (1.06 g, 4.79 mmol) was dissolved in a 50% acetic acid aqueous solution (100 ml). Diammonium cerium nitrate (7.61 g, 13.9 mmol) was added, and the mixture was stirred at 80°C for four hours. The reaction solution was cooled to room temperature and then water (300 mL) was added, followed by extraction with ethyl acetate (300 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 10/1) to give the target compound (173 mg, yield: 15%) as a light yellow solid.
$^1$H-NMR ($CDCl_3$, 400MHz):δ ppm: 7.69-7.78 (2H, m), 7.82 (1H, d, J=7.8Hz), 7.99 (1H, d, J=7.8Hz), 8.38 (1H, d, J=8.4Hz), 9.29 (1H, d, J=8.4Hz), 10.38 (1H, s). MS (EI) m/z: 233.9679 $(M^+)$

(29b) (9aS)-8-Acetyl-N-[(4-bromo-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (249 mg, 0.721 mmol), 4-bromo-1-naphthaldehyde produced in Example (29a) (171 mg, 0.726 mmol), triethylsilane (350 μL, 2.20 mmol), trifluoroacetic acid (170 μL, 2.21 mmol) and acetonitrile (10 mL) to give the target compound (199 mg, yield: 49%) as a yellow solid.
$^1$H-NMR ($CDCl_3$, 400MHz):δ ppm: 1.75 (3H, s), 2.65 (3H, s), 3.73 (3H, s), 5.02 (1H, dd, J=5.6, 14.7Hz), 5.09 (1H, dd, J=5.6, 14.7Hz), 6.01 (1H, s), 6.28 (1H,s), 7.33 (1H, brs), 7.39 (1H, d, J=7.8Hz), 7.60-7.67 (2H, m), 7.76 (1H, d, J=7.8Hz), 8.12 (1H, m), 8.32 (1H, m), 10.70 (1H, s), 18.83 (1H, s).
MS (FAB) m/z: 564.0662 $(M+H)^+$

(Example 30) (9aS)-8-Acetyl-N-(2,3-dimethylbenzyl)-1,7-dihydroxy-3-methoxy-9a-methyl-9-oxo-9,9a-dihydrodibenzo[b,d]furan-4-carboxamide (exemplary compound No. 1-39)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 0.579 mmol), 2,3-dimethylbenzaldehyde (0.303 mL, 2.32 mmol), triethylsilane (0.369 mL, 2.32 mmol), trifluoroacetic acid (0.179 mL, 2.32 mmol) and acetonitrile (10.0 mL) to give the target compound (0.245 g, yield: 91%) as a yellow solid.

$^1$H-NMR ($CDCl_3$, 500MHz):δ ppm: 1.75 (3H, s), 2.28 (3H, s), 2.31 (3H, s), 2.65 (3H, s), 3.84 (3H, s), 4.63-4.71 (2H, m), 6.00 (1H, s), 6.31 (1H, s), 7.10-7.26 (4H, m), 10.66 (1H, s), 18.82 (1H, s).
MS (FAB) m/z: 464.1710 $(M+H)^+$.

(Example 31) (9aS)-8-Acetyl-N-(2,6-dimethylbenzyl)-1,7-dihydroxy-3-methoxy-9a-methyl-9-oxo-9,9a-dihydrodibenzo [b,d]furan-4-carboxamide (exemplary compound No. 1-42)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 0.579 mmol), 2,6-dimethylbenzaldehyde (0.311 g, 2.32 mmol), triethylsilane (0.369 mL, 2.32 mmol), trifluoroacetic acid (0.179 mL, 2.32 mmol) and acetonitrile (10.0 mL) to give the target compound (0.238 g, yield: 89%) as a yellow solid.
$^1$H-NMR ($CDCl_3$, 500MHz):δ ppm: 1.75 (3H, s), 2.43 (6H, s), 2.64 (3H, s), 3.79 (3H, s), 4.59-4.69 (2H, m), 6.01 (1H, s), 6.28 (1H, s), 6.75 (1H, brs), 7.05-7.14 (3H, m), 10.64 (1H, s), 18.82 (1H, s).
MS (FAB) m/z: 464.1724 $(M+H)^+$

(Example 32) (9aS)-8-Acetyl-1,7-dihydroxy-N-(mesitylmethyl)-3-methoxy-9a-methyl-9-oxo-9,9a-dihydrodibenzo[b,d] furan-4-carboxamide (exemplary compound No. 1-66)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 0.579 mmol), 2,4,6-trimethylbenzaldehyde (0.342 mL, 2.32 mmol), triethylsilane (0.369 mL, 2.32 mmol), trifluoroacetic acid (0.179 mL, 2.32 mmol) and acetonitrile (10.0 mL) to give the target compound (0.161 g, yield: 58%) as a yellow solid.
$^1$H-NMR ($CDCl_3$, 500MHz):δ ppm: 1.75 (3H, s), 2.28 (3H, s), 2.39 (6H, s), 2.64 (3H, s), 3.79 (3H, s), 4.56-4.64 (2H, m), 6.01 (1H, s), 6.28 (1H, s), 6.69 (1H, brs), 6.89 (2H, s), 10.63 (1H, s), 18.82 (1H, s).
MS (FAB) m/z: 478.1862 $(M+H)^+$

(Example 33) (9aS)-8-Acetyl-N-[(2-ethoxy-1-naphthyl)methyl]-1,7-dihydroxy-3-methoxy-9a-methyl-9-oxo-9,9a-dihydrodibenzo[b,d]furan-4-carboxamide (exemplary compound No. 1-270)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (310 mg, 0.898 mmol), 2-ethoxy-1-naphthaldehyde (560 mg, 2.80 mmol), triethylsilane (420 μL, 2.64 mmol), trifluoroacetic acid (210 μL, 2.73 mmol) and acetonitrile (10 mL) to give the target compound (260 mg, yield: 55%) as a yellow solid.
$^1$H-NMR ($CDCl_3$, 500MHz):δ ppm: 1.49 (3H, t, J=6.8Hz), 1.73 (3H, s), 2.64 (3H, s), 3.68 (3H, s), 4.25 (2H, q, J=6.8Hz), 5.08 (1H, dd, J=5.4, 14.2Hz), 5.16 (1H, dd, J=5.4, 14.2Hz), 6.00 (1H, s), 6.24 (1H,s), 7.24-7.30 (2H, m), 7.37 (1H, t, J=7.8Hz), 7.54 (1H, t, J=7.8Hz), 7.81 (1H, m), 8.24 (1H, d, J=8.3Hz), 10.62 (1H, s), 18.82 (1H, s).
MS (FAB) m/z: 530.1810 $(M+H)^+$

(Example 34) (9aS)-8-Acetyl-N-(2,3-dihydro-1,4-benzodioxin-5-ylmethyl)-1,7-dihydroxy-3-methoxy-9a-methyl-9-oxo-9,9a-dihydrodibenzo[b,d]furan-4-carboxamide (exemplary compound No. 1-467)

(34a) 2,3-Dihydro-1,4-benzodioxine-5-carbaldehyde

2,3-Dihydro-1,4-benzodioxin-5-ylmethanol (1.01 g, 6.07 mmol) was dissolved in methylene chloride (60 mL). Manganese dioxide (3.81 g, 43.8 mmol) was added, and then the mixture was stirred in a nitrogen atmosphere at room temperature for 24 hours. Manganese dioxide was separated by filtration and the solvent was evaporated under reduced pressure. The resulting residue was purified by silica gel column chromatography (elution solvent: ethyl acetate/hexane = 1/4) to give the target compound (835 mg, yield: 84%) as a colorless oil.
$^1$H-NMR ($CDCl_3$, 400MHz):δ ppm: 4.32-4.34 (2H, m), 4.38-4.41 (2H, m), 6.91 (1H, t, J=7.8Hz), 7.10 (1H, dd, J=1.6, 7.8Hz), 7.40 (1H, dd, J=1.6, 7.8Hz).
MS (EI) m/z: 164.0468 $(M^+)$

(34b) (9aS)-8-Acetyl-N-(2,3-dihydro-1,4-benzodioxin-5-ylmethyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (314 mg, 0.909 mmol), 2,3-dihydro-1,4-benzodioxine-5-carbaldehyde produced in Example (34a) (460 mg, 2.80 mmol), triethyl-

silane (420 μL, 2.64 mmol), trifluoroacetic acid (210 μL, 2.73 mmol) and acetonitrile (10 mL) to give the target compound (358 mg, yield: 80%) as a yellow solid.
$^1$H-NMR ($CDCl_3$, 500MHz):δ ppm: 1.74 (3H, s), 2.65 (3H, s), 3.89 (3H, s), 4.27-4.34 (4H, m), 4.59 (1H, dd, J=5.8,14.9Hz), 4.64 (1H, dd, J=5.8, 14.9Hz), 6.01 (1H, s), 6.33 (1H,s), 6.80-6.87 (2H, m), 6.94 (1H, m), 7.50 (1H, brs), 10.69 (1H, s), 18.82 (1H, s).
MS (FAB) m/z: 494.1458 $(M+H)^+$

(Example 35) (9aS)-8-Acetyl-N-(3-fluoro-2-methylbenzyl)-1,7-dihydroxy-3-methoxy-9a-methyl-9-oxo-9,9a-dihydrodibenzo[b,d]furan-4-carboxamide (exemplary compound No. 1-50)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.200 g, 5.79 mmol), 3-fluoro-2-methylbenzaldehyde (0.276 mL, 2.32 mmol), triethylsilane (0.369 mL, 2.32 mmol), trifluoroacetic acid (0.179 mL, 2.32 mmol) and acetonitrile (10.0 mL) to give the target compound (0.259 g, yield: 95%) as a yellow solid.
$^1$H-NMR ($CDCl_3$, 500MHz):δ ppm: 1.75 (3H, s), 2.29 (3H, s), 2.65 (3H, s), 3.87 (3H, s), 4.58-4.69 (2H, m), 6.02 (1H, s), 6.33 (1H, s), 6.97 (1H, m), 7.13-7.15 (2H, m), 7.21 (1H, brs), 10.71 (1H, s), 18.83 (1H, s).
MS (FAB) m/z: 468.1445 $(M+H)^+$.

(Example 36) (9aS)-8-Acetyl-1,7-dihydroxy-3-methoxy-9a-methyl-9-oxo-N-(2,3,5,6-tetramethylbenzyl)-9,9a-dihydrodibenzo[b,d]furan-4-carboxamide (exemplary compound No. 1-116)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,3,5,6-tetramethylbenzaldehyde (0.282 g, 1.74 mmol), triethylsilane (0.554 mL, 3.48 mmol), trifluoroacetic acid (0.268 mL, 3.48 mmol) and acetonitrile (15.0 mL) to give the target compound (0.301 g, yield: 70%) as a yellow solid.
$^1$H-NMR ($CDCl_3$, 500MHz):δ ppm: 1.75 (3H, s), 2.25 (6H, s), 2.29 (6H, s), 2.64 (3H, s), 3.78 (3H, s), 4.61-4.71 (2H, m), 6.00 (1H, s), 6.26 (1H, s), 6.69 (1H, brs), 6.95 (2H, s), 10.60 (1H, s), 18.78 (1H, s).
MS (FAB) m/z: 492.2031 $(M+H)^+$

(Example 37) (9aS)-8-Acetyl-N-(2,3-difluoro-4-methylbenzyl)-1,7-dihydroxy-3-methoxy-9a-methyl-9-oxo-9,9a-dihydrodibenzo[b,d]furan-4-carboxamide (exemplary compound No. 1-70)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 8.69 mmol), 2,3-difluoro-4-methylbenzaldehyde (0.543 mL, 3.48 mmol), triethylsilane (0.554 mL, 3.48 mmol), trifluoroacetic acid (0.268 mL, 3.48 mmol) and acetonitrile (15.0 mL) to give the target compound (0.378 g, yield: 90%) as a yellow solid. $^1$H-NMR ($CDCl_3$, 400MHz):δ ppm: 1.74 (3H, s), 2.28 (3H, s), 2.64 (3H, s), 3.90 (3H, s), 4.57-4.69 (2H, m), 6.01 (1H, s), 6.32 (1H, s), 6.88 (1H, t, J=7.9Hz), 7.08 (1H, t, J=7.9Hz), 7.58 (1H, brs), 10.71 (1H, s), 18.78 (1H, s).
MS (FAB) m/z: 486.1367 $(M+H)^+$

(Example 38) (9aS)-8-Acetyl-1,7-dihydroxy-3-methoxy-9a-methyl-9-oxo-N-(2,3,4,5,6-pentamethylbenzyl)-9,9a-dihydrodibenzo[b,d]furan-4-carboxamide (exemplary compound No. 1-166)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), pentamethylbenzaldehyde (0.613 g, 3.48 mmol), triethylsilane (0.554 mL, 3.48 mmol), trifluoroacetic acid (0.268 mL, 3.48 mmol) and acetonitrile (15.0 mL) to give the target compound (0.353 g, yield: 80%) as a yellow solid.
$^1$H-NMR ($CDCl_3$, 500MHz):δ ppm: 1.75 (3H, s), 2.24 (6H, s), 2.26 (3H, s), 2.34 (6H, s), 2.64 (3H, s), 3.78 (3H, s), 4.61-4.71 (2H, m), 6.00 (1H, s), 6.26 (1H, s), 6.67 (1H, brs), 10.59 (1H, s), 18.78 (1H, s).
MS (FAB) m/z: 506.2180 $(M+H)^+$

(Example 39) (9aS)-8-Acetyl-N-[(4-cyano-1-naphthyl)methyl]-1,7-dihydroxy-3-methoxy-9a-methyl-9-oxo-9,9a-dihydrodibenzo[b,d]furan-4-carboxamide (exemplary compound No. 1-368)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (303 mg, 0.877 mmol), known 1-formyl-4-cyanonaphthalene [Journal of American Chemical Society, 1962, Vol. 84, p.3541-3546] (288 mg, 1.59 mmol), triethylsilane (420 μL, 2.64 mmol), trifluoroacetic acid (210 μL, 2.73 mmol) and acetonitrile (10

mL) to give the target compound (143 mg, yield: 32%) as a yellow solid.
$^1$H-NMR ($CDCl_3$, 400MHz):δ ppm: 1.76 (3H, s), 2.65 (3H, s), 3.80 (3H, s), 5.11 (1H, dd, J=5.6, 15.5H), 5.19 (1H, dd, J=5.6,15.5Hz), 6.01(1H, s), 6.32 (1H,s), 7.54 (1H, brs), 7.61 (1H, d, J=7.1Hz), 7.69-7.76 (2H, m), 7.90 (1H, d, J=7.1Hz), 8.20 (1H, d, J=7.5Hz), 8.31 (1H, d, J=7.5Hz), 10.76 (1H, s), 18.84 (1H, s).
MS (FAB) m/z: 511.1505 $(M+H)^+$

(Example 40) (9aS)-8-Acetyl-N-(2,4-dimethylbenzyl)-1,7-dihydroxy-3-methoxy-9a-methyl-9-oxo-9,9a-dihydrodibenzo [b,d]furan-4-carboxamide (exemplary compound No. 1-40)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,4-dimethylbenzaldehyde (0.485 g, 3.48 mmol), triethylsilane (0.554 mL, 3.48 mmol), trifluoroacetic acid (0.268 mL, 3.48 mmol) and acetonitrile (15.0 mL) to give the target compound (0.385 g, yield: 95%) as a yellow solid.
$^1$H-NMR ($CDCl_3$, 500MHz):δ ppm: 1.75 (3H, s), 2.31 (3H, s), 2.35 (3H, s), 2.64 (3H, s), 3.84 (3H, s), 4.52-4.63 (2H, m), 6.00 (1H, s), 6.30 (1H, s), 6.97-7.00 (2H, m), 7.05 (1H, brs), 7.20 (1H, d, J=7.5Hz), 10.65 (1H, s), 18.78 (1H, s).
MS (FAB) m/z: 464.1724 $(M+H)^+$.

(Example 41) (9aS)-8-Acetyl-N-(3,5-dimethylbenzyl)-1,7-dihydroxy-3-methoxy-9a-methyl-9-oxo-9,9a-dihydrodibenzo [b,d]furan-4-carboxamide (exemplary compound No. 1-44)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 3,5-dimethylbenzaldehyde (0.468 mL, 3.48 mmol), triethylsilane (0.554 mL, 3.48 mmol), trifluoroacetic acid (0.268 mL, 3.48 mmol) and acetonitrile (15.0 mL) to give the target compound (0.364 g, yield: 90%) as a yellow solid.
$^1$H-NMR ($CDCl_3$, 400MHz):δ ppm: 1.76 (3H, s), 2.31 (6H, s), 2.65 (3H, s), 3.87 (3H, s), 4.52-4.63 (2H, m), 6.01 (1H, s), 6.32 (1H, s), 6.91 (1H, brs), 6.97 (2H, s), 7.24 (1H, s), 10.67 (1H, s), 18.78 (1H, s).
MS (ESI) m/z: 464.1696 $(M+H)^+$

(Example 42) (9aS)-8-Acetyl-N-(2,5-dimethylbenzyl)-1,7-dihydroxy-3-methoxy-9a-methyl-9-oxo-9,9a-dihydrodibenzo [b,d]furan-4-carboxamide (exemplary compound No. 1-41)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,5-dimethylbenzaldehyde (0.492 mL, 3.48 mmol), triethylsilane (0.554 mL, 3.48 mmol), trifluoroacetic acid (0.268 mL, 3.48 mmol) and acetonitrile (15.0 mL) to give the target compound (0.378 g, yield: 94%) as a yellow solid.
$^1$H-NMR ($CDCl_3$, 400MHz):δ ppm: 1.75 (3H, s), 2.31 (3H, s), 2.33 (3H, s), 2.64 (3H, s), 3.85 (3H, s), 4.53-4.64 (2H, m), 6.00 (1H, s), 6.32 (1H, s), 6.98-7.08 (3H, m), 7.14 (1H, s), 10.65 (1H, s), 18.78 (1H, s).
MS (ESI) m/z: 464.1711 $(M+H)^+$

(Example 43) (9aS)-8-Acetyl-1,3,7-trihydroxy-9a-methyl-N-(1-naphthylmethyl)-9-oxo-9,9a-dihydrodibenzo[b,d]furan-4-carboxamide (exemplary compound No. 2-2)

(43a) (9aS)-8-Acetyl-3-(benzyloxy)-1,7-dihydroxy-9a-methyl-9-oxo-9,9a-dihydrodibenzo[b,d]furan-4-carboxamide

Cercosporamide obtained in Reference Example 3 (5.30 g, 16.0 mmol) was dissolved in dimethylformamide (60 mL). Benzyl bromide (5.70 mL, 48.0 mmol) and potassium carbonate (6.65 g, 48.1 mmol) were added, and the mixture was stirred in a nitrogen atmosphere at 80˚C for six hours. Potassium carbonate was separated by filtration from the reaction solution and 0.1 N hydrochloric acid (100 mL) was added, followed by extraction with ethyl acetate (300 mL). The organic layer was washed with brine and then dried over anhydrous sodium sulfate. The solvent was evaporated under reduced pressure, and the resulting residue was purified by silica gel column chromatography (elution solvent: methylene chloride/methanol = 50/1 to 20/1) to give the target compound (1.92 g, yield: 28%) as a yellow solid.
$^1$H-NMR ($CDCl_3$, 400MHz):δ ppm: 1.77 (3H, s), 2.65 (3H, s), 5.14 (2H, d, J=1.9Hz), 5.61 (1H, brs), 6.04 (1H, s), 6.45 (1H,s), 7.10 (1H, brs), 7.40-7.46 (5H, m), 10.81 (1H, s), 18.83 (1H, s).
MS (ESI) m/z: 444.1049 $(M+Na)^+$

(43b) (9aS)-8-Acetyl-3-(benzyloxy)-1,7-dihydroxy-9a-methyl-N-(1-naphthylmethyl)-9-oxo-9,9a-dihydrodibenzo[b,d]furan-4-carboxamide

Reaction and post-treatment were carried out in accordance with Example 10 using (9aS)-8-acetyl-3-(benzyloxy)-1,7-dihydroxy-9a-methyl-9-oxo-9,9a-dihydrodibenzo[b,d]furan-4-carboxamide produced in Example (43a) (1.92 g, 4.56 mmol), 1-naphthaldehyde (1.25 mL, 9.20 mmol), triethylsilane (1.45 mL, 9.10 mmol), trifluoroacetic acid (700 μL, 9.09 mmol) and acetonitrile (60 mL) to give the target compound (1.48 g, yield: 58%) as a yellow solid.
$^1$H-NMR ($CDCl_3$, 500MHz):δ ppm: 1.76 (3H, s), 2.65 (3H, s), 4.92-5.06 (4H, m), 6.05 (1H, s), 6.38 (1H,s), 7.08 (2H, d, J=7.5Hz), 7.14 (2H, t, J=7.5Hz), 7.22-7.34 (4H, m), 7.43-7.50 (3H, m), 7.78(1H, dd, J=2.4, 7.3Hz), 7.85 (1H, d, J=7.8Hz), 8.00 (1H, d, J=7.3Hz), 10.72 (1H, s), 18.84 (1H, s).

(43c) (9aS)-8-Acetyl-1,3,7-trihydroxy-9a-methyl-N-(1-naphthylmethyl)-9-oxo-9,9a-dihydrodibenzo[b,d]furan-4-carboxamide

(9aS)-8-Acetyl-3-(benzyloxy)-1,7-dihydroxy-9a-methyl-N-(1-naphthylmethyl)-9-oxo-9,9a-dihydrodibenzo[b,d]fruan-4-carboxamide produced in Example (43b) (998 mg, 1.78 mmol) was dissolved in a mixed solvent of ethanol (30 mL)-ethyl acetate (6 mL). 10% palladium-carbon (502 mg) was added, and the mixture was stirred in a hydrogen (1 atm) atmosphere at room temperature for five hours and 30 minutes. Palladium-carbon was separated by filtration from the reaction solution, and then the solvent was evaporated under reduced pressure. The resulting residue was purified by silica gel column chromatography (elution solvent: methylene chloride/methanol = 100/1 to 80/1) to give the target compound (318 mg, yield: 38%) as a yellow solid.
$^1$H-NMR ($CDCl_3$, 500MHz):δ ppm: 1.69 (3H, s), 2.63 (3H, s), 5.09 (1H, dd, J= 5.4, 14.7Hz), 5.15 (1H, dd, J=5.4, 14.7Hz), 5.68 (1H, s), 6.34 (1H, s), 7.33 (1H, brs), 7.46-7.62 (4H, m), 7.87 (1H, d, J=8.3Hz), 7.93 (1H, d, J=7.8Hz), 8.08 (1H, d, J=8.3Hz), 10.46 (1H, s), 13.21 (1H, s), 18.75 (1H, s).
MS (FAB) m/z: 472.1392$(M+H)^+$

(Example 44) (9aS)-8-Acetyl-1,7-dihydroxy-N-[(2-isopropoxy-1-naphthyl)methyl]-3-methoxy-9a-methyl-9-oxo-9,9a-dihydrodibenzo[b,d]furan-4-carboxamide (exemplary compound No. 1-276)

(44a) 2-Isopropoxy-1-naphthaldehyde

Sodium hydride (408 mg, 9.35 mmol) was suspended in dimethylformamide (30 mL) in a nitrogen atmosphere, and the suspension was cooled to 0°C. 2-Hydroxy-1-naphthaldehyde (1.00 g, 5.81 mmol) was added, and the mixture was stirred for 30 minutes. 2-Iodopropane (1.20 mL, 12.0 mmol) was further added, and the mixture was stirred at room temperature for three hours. An ammonium chloride aqueous solution (100 mL) was added to the reaction solution, followed by extraction with ethyl acetate (100 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 15/1 to 12/1) to give the target compound (893 mg, yield: 72%) as a light yellow oil.
$^1$H-NMR ($CDCl_3$, 400MHz):δ ppm: 1.44 (6H, d, J=5.9Hz), 4.82 (1H, m), 7.28 (1H, d, J=9.0Hz), 7.42 (1H, t, J=7.9Hz), 7.61 (1H, t, J=7.9Hz), 7.76 (1H, d, J=8.6Hz), 8.02 (1H, d, J=9.0Hz), 9.28 (1H, d, J=9.0Hz), 10.90 (1H, s).
MS (EI) m/z: 214 $(M^+)$

(44b) (9aS)-8-Acetyl-1,7-dihydroxy-N-[(2-isopropoxy-1-naphthyl)methyl]-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (468 mg, 1.36 mmol), 2-isopropoxy-1-naphthaldehyde produced in Example (44a) (860 mg, 4.01 mmol), triethylsilane (640 μL, 4.02 mmol), trifluoroacetic acid (310 μL, 4.02 mmol) and acetonitrile (15 mL) to give the target compound (557 mg, yield: 75%) as a yellow solid.
$^1$H-NMR ($CDCl_3$, 400MHz):δ ppm: 1.40 (3H, d, J=2.8Hz), 1.42 (3H, d, J=2.8Hz), 1.73 (3H, s), 2.64 (3H, s), 3.68 (3H, s), 4.73 (1H, m), 5.08 (1H, dd, J=4.8, 13.9Hz), 5.15 (1H, dd, J=4.8, 13.9Hz), 6.00 (1H, s), 6.24 (1H,s), 7.20 (1H, brs), 7.29 (1H, d, J=9.1Hz), 7.38 (1H, m), 7.53 (1H, m), 7.78-7.82 (2H, m), 8.22 (1H, d, J=8.7Hz), 10.62 (1H, s), 18.82 (1H,s).
MS (FAB) m/z: 544.1967 $(M+H)^+$

(Example 45) (9aS)-8-Acetyl-N-(4-fluoro-2,6-dimethylbenzyl)-1,7-dihydroxy-3-methoxy-9a-methyl-9-oxo-9,9a-dihydrodibenzo[b,d]furan-4-carboxamide (exemplary compound No. 1-71)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,6-dimethyl-4-fluorobenzaldehyde (0.330 g, 2.17 mmol), triethylsilane (0.554 mL, 3.48 mmol), trifluoroacetic acid (0.268 mL, 3.48 mmol) and acetonitrile (15.0 mL) to give the target compound (0.282 g, yield: 67%) as a yellow solid. $^1$H-NMR ($CDCl_3$, 500MHz):δ ppm: 1.75 (3H, s), 2.42 (6H, s), 2.65 (3H, s), 3.80 (3H, s), 4.54-4.64 (2H, m), 6.02 (1H, s), 6.29 (1H, s), 6.76-6.78 (3H, m), 10.67 (1H, s), 18.83 (1H, s).
MS (FAB) m/z: 482.1611 $(M+H)^+$.

(Example 46) (9aS)-8-Acetyl-N-(3,4-dimethylbenzyl)-1,7-dihydroxy-3-methoxy-9a-methyl-9-oxo-9,9a-dihydrodibenzo[b,d]furan-4-carboxamide (exemplary compound No. 1-43).

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 3,4-dimethylbenzaldehyde (0.461 mL, 3.48 mmol), triethylsilane (0.554 mL, 3.48 mmol), trifluoroacetic acid (0.268 mL, 3.48 mmol) and acetonitrile (15.0 mL) to give the target compound (0.367 g, yield: 91%) as a yellow solid.
$^1$H-NMR ($CDCl_3$, 400MHz):δ ppm: 1.76 (3H, s), 2.25 (3H, s), 2.27 (3H, s), 2.65 (3H, s), 3.87 (3H, s), 4.53-4.64 (2H, m), 6.02 (1H, s), 6.33 (1H, s), 6.11 (2H, s), 7.15 (1H, s), 10.69 (1H, s), 18.83 (1H, s).
MS (FAB) m/z: 464.1711 $(M+H)^+$

(Example 47) (9aS)-8-Acetyl-1,7-dihydroxy-3-methoxy-9a-methyl-9-oxo-N-(2,4,6-trifluorobenzyl)-9,9a-dihydrodibenzo[b,d]furan-4-carboxamide (exemplary compound No. 1-61)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,4,6-trifluorobenzaldehyde (0.557 mL, 3.48 mmol), triethylsilane (0.554 mL, 3.48 mmol), trifluoroacetic acid (0.268 mL, 3.48 mmol) and acetonitrile (15.0 mL) to give the target compound (0.361 g, yield: 85%) as a yellow solid.
$^1$H-NMR ($CDCl_3$, 500MHz):δ ppm: 1.73 (3H, s), 2.64 (3H, s), 3.89 (3H, s), 4.58-4.73 (2H, m), 6.02 (1H, s), 6.32 (1H, s), 6.68 (2H, t, J=8.3Hz), 7.65 (1H, brs), 10.75 (1H, s), 18.82 (1H, s).
MS (FAB) m/z: 490.1111 $(M+H)^+$

(Example 48) (9aS)-8-Acetyl-N-[(2-butoxy-1-naphthyl)methyl]-1,7-dihydroxy-3-methoxy-9a-methyl-9-oxo-9,9a-dihydrodibenzo[b,d]furan-4-carboxamide (exemplary compound No. 1-279)

(48a) 2-Butoxy-1-naphthaldehyde

Sodium hydride (384 mg, 8.80 mmol) was suspended in dimethylformamide (30 mL) in a nitrogen atmosphere, and the suspension was cooled to 0˚C. 2-Hydroxy-1-naphthaldehyde (1.00 g, 5.81 mmol) was added, and the mixture was stirred for 30 minutes. 1-Iodobutane (1.32 mL, 11.6 mmol) was further added, and the mixture was stirred at room temperature for three hours. An ammonium chloride aqueous solution (100 mL) was added to the reaction solution, followed by extraction with ethyl acetate (100 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1) to give the target compound (1.20 g, yield: 91 %) as a colorless solid.
$^1$H-NMR ($CDCl_3$, 400MHz):δ ppm: 1.01 (3H, t, J=7.4Hz), 1.51-1.60 (2H, m), 1.84-1.91 (2H, m), 4.23 (2H, t, J=6.4Hz), 7.27 (1H, d, J=9.1Hz), 7.39 (1H, t, J=7.6Hz), 7.59 (1H, t, J=7.8Hz), 7.75 (1H, d, J=8.6Hz), 8.02 (1H, d, J=9.0Hz), 9.26 (1H, d, J=8.6Hz), 10.90 (1H, s).
MS (EI) m/z: 228.1 $(M^+)$

(48b) (9aS)-8-Acetyl-N-[(2-butoxy-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (407 mg, 1.18 mmol), 2-butoxy-1-naphthaldehyde produced in Example (48a) (807 mg, 3.53 mmol), triethylsilane (560 μL, 3.52 mmol), trifluoroacetic acid (270 μL, 3.50 mmol) and acetonitrile (15 mL) to give the target compound (499 mg, yield:

76%) as a yellow solid.
$^1$H-NMR ($CDCl_3$, 500MHz):δ ppm: 1.00 (3H, t, J=7.3Hz), 1.52-1.59 (2H, m), 1.73 (3H, s), 1.82-1.88 (2H, m), 2.64 (3H, s), 3.68 (3H, s), 4.17 (2H, t, J=6.6Hz), 5.09 (1H, dd, J=4.9, 13.7Hz), 5.16 (1H, dd, J=4.9, 13.7Hz), 5.99 (1H, s), 6.24 (1H, s), 7.21 (1H, brs), 7.29 (1H, d, J=9.3Hz), 7.36 (1H, m), 7.53 (1H, m), 7.79 (1H, d, J=8.3Hz), 7.82 (1H, d, J=8.8Hz), 8.23 (1H, d, J=8.8Hz), 10.61 (1H, s), 18.82 (1H, s).
MS (FAB) m/z: 558.2123 $(M+H)^+$

(Example 49) (9aS)-8-Acetyl-1,7-dihydroxy-N-[(2-hydroxy-1-naphthyl)methyl]-3-methoxy-9a-methyl-9-oxo-9,9a-dihydrodibenzo[b,d]furan-4-carboxamide (exemplary compound No. 1-291)

(49a) 1-Formyl-2-naphthyl acetate

2-Hydroxy-1-naphthaldehyde (1.02 g, 5.92 mmol) was dissolved in a mixed solvent of acetic anhydride (2 mL)-pyridine (4 mL) in a nitrogen atmosphere, and the solution was stirred at room temperature for three hours. The solvent was evaporated under reduced pressure, and the resulting residue was purified by silica gel column chromatography (elution solvent: hexane/ethyl acetate = 10/1 to 5/1) to give the light brown target compound (1.02 g, yield: 81%).
$^1$H-NMR ($CDCl_3$, 500MHz):δ ppm: 2.44 (3H, s), 7.28 (1H, d, J=9.3Hz), 7.56 (1H, t, J=7.6Hz), 7.68 (1H, t, J=7.8Hz), 7.88 (1H, d, J=8.3Hz), 8.10 (1H, d, J=8.8Hz), 9.14 (1H, d, J=8.8Hz), 10.71 (1H, s).

(49b) 1-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthyl acetate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (409 mg, 1.18 mmol), 1-formyl-2-naphthyl acetate produced in Example (49a) (761 mg, 3.55 mmol), triethylsilane (560 μL, 3.52 mmol), trifluoroacetic acid (270 μL, 3.50 mmol) and acetonitrile (15 mL) to give the target compound (574 mg, yield: 89%) as a yellow solid.
$^1$H-NMR ($CDCl_3$, 500MHz):δ ppm: 1.74 (3H, s), 2.43 (3H, s), 2.64 (3H, s), 3.69 (3H, s), 4.96 (1H, dd, J=4.9, 14.7Hz), 5.04 (1H, dd, J=4.9, 14.7Hz), 6.03 (1H, s), 6.23 (1H, s), 7.20 (1H, d, J=9.3Hz), 7.34 (1H, brs), 7.52 (1H, m), 7.60 (1H, m), 7.86-7.88 (2H, m), 8.20 (1H, d, J=8.8Hz), 10.65 (1H, s), 18.83 (1H, s).

(49c) (9aS)-8-Acetyl-1,7-dihydroxy-N-[(2-hydroxy-1-naphthyl)methyl]-3-methoxy-9a-methyl-9-oxo-9,9a-dihydrodibenzo[b,d]furan-4-carboxamide

1-[({[(9aS)-8-Acetyl-1,7-dihydroxy-3-methoxy-9a-methyl-9-oxo-9,9a-dihydrodibenzo[b,d]fiuan-4-yl]carbonyl}amino)methyl]-2-naphthyl acetate produced in Example (49b) (510 mg, 0.938 mmol) was dissolved in a mixed solvent of methylene chloride (10 mL)-methanol (10 mL). Potassium carbonate (663 mg, 4.80 mmol) was added, and the mixture was stirred at room temperature for three hours. A dilute hydrochloric acid solution (100 mL) was added to the reaction solution, followed by extraction with ethyl acetate (100 mL). The organic layer was washed with brine and then dried over anhydrous sodium sulfate. The solvent was evaporated under reduced pressure, and the resulting residue was purified by silica gel column chromatography (elution solvent: methylene chloride/methanol = 70/1 to 60/1) to give the target compound (306 mg, yield: 65%) as a yellow solid.
$^1$H-NMR ($CDCl_3$, 500MHz):δ ppm: 1.72 (3H, s), 2.64 (3H, s), 3.86 (3H, s), 4.91 (1H, dd, J=6.4, 15.1Hz), 4.97 (1H, dd, J=6.4, 15.1Hz), 6.04 (1H, s), 6.30 (1H, s), 7.26 (1H, d, J=8.8Hz), 7.35 (1H, m), 7.54 (1H, m), 7.74 (1H, d, J=8.8Hz), 7.80 (1H, d, J=8.3Hz), 7.86 (1H, d, J=8.3Hz), 8.34 (1H, brs), 10.06 (1H, s), 10.84 (1H, s), 18.83 (1H, s).
MS (FAB) m/z: 502.1503 $(M+H)^+$

(Example 50) (9aS)-8-Acetyl-1,7-dihydroxy-3-methoxy-N-(4-methoxy-2,3-dimethylbenzyl)-9a-methyl-9-oxo-9,9a-dihydrodibenzo[b,d]furan-4-carboxamide (exemplary compound No. 1-73)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 4-methoxy-2,3-dimethylbenzaldehyde (0.429 g, 2.61 mmol), triethylsilane (0.554 mL, 3.48 mmol), trifluoroacetic acid (0.268 mL, 3.48 mmol) and acetonitrile (15.0 mL) to give the target compound (0.172 g, yield: 40%) as a yellow solid. $^1$H-NMR ($CDCl_3$, 400MHz):δ ppm: 1.75 (3H, s), 2.19 (3H, s), 2.28 (3H, s), 2.65 (3H, s), 3.82 (3H, s), 3.83 (3H, s), 4.54-4.63 (2H, m), 6.01 (1H, s), 6.30 (1H, s), 6.70 (1H, d, J=8.5Hz), 6.98 (1H, brs), 7.16 (1H, d, J=8.5Hz), 10.66 (1H, s), 18.83 (1H, s).
MS (FAB) m/z: 494.1812 $(M+H)^+$.

(Example 51) (9aS)-8-Acetyl-1,7-dihydroxy-3-methoxy-N,9a-dimethyl-N-(1-naphthylmethyl)-9-oxo-9,9a-dihydrodibenzo[b,d]furan-4-carboxamide (exemplary compound No. 2-42)

(51a) (9aS)-8-Acetyl-1,7-dihydroxy-3-methoxy-9a-methyl-9-oxo-9,9a-dihydrodibenzo[b,d]furan-4-carboxylic acid

(9aS)-8-Acetyl-1,7-dihydroxy-3-methoxy-9a-methyl-9-oxo-9,9a-dihydrodibenzo[b,d]furan-4-carboxamide produced in Example (1a) (7.20 g, 20.9 mmol) was dissolved in acetonitrile (150 mL), and 6 N sulfuric acid (100 mL) was added. Sodium nitrite (14.4 g, 209 mmol) was added, and the mixture was stirred at room temperature for 15 hours. Then, the reaction solution was extracted with ethyl acetate (300 mL). The organic layer was washed with brine and then dried over anhydrous magnesium sulfate, and the solvent was evaporated under reduced pressure. The resulting residue was purified by silica gel column chromatography (elution solvent: methylene chloride/methanol = 20/1) to give the target compound (6.38 g, yield: 88%) as a yellow solid.
$^1$H-NMR ($CDCl_3$, 400MHz): δ ppm: 1.76 (3H, s), 2.66 (3H, s), 4.04 (3H,s), 6.06 (1H,s), 6.38 (1H, s), 11.02 (1H, s), 18.80 (1H, s).
MS (FAB) m/z: 369.0582 $(M+Na)^+$.

(51b) (9aS)-8-Acetyl-1,7-dihydroxy-3-methoxy-N,9a-dimethyl-N-(1-naphthylmethyl)-9-oxo-9,9a-dihydrodibenzo[b,d]furan-4-carboxamide

(9aS)-8-Acetyl-1,7-dihydroxy-3-methoxy-9a-methyl-9-oxo-9,9a-dihydrodibenzo[b,d]furan-4-carboxylic acid produced in Example (51a) (384 mg, 1.11 mmol) was dissolved in N,N-dimethylformamide (15 mL), followed by addition of N-methyl-N-(1-naphthylmethyl)amine (475 mg, 2.77 mmol), 1-(3-dimethylaminopropyl)-3-ethylcarbodiimide hydrochloride (426 mg, 2.22 mmol) and 1-hydroxybenzotriazole monohydrate (300 mg, 2.22 mmol). After stirring at room temperature for 20 hours, a saturated ammonium chloride aqueous solution (20 mL) was added to the reaction solution, followed by extraction with ethyl acetate (50 mL). The organic layer was washed with brine and then dried over anhydrous magnesium sulfate, and the solvent was evaporated under reduced pressure. The resulting residue was dissolved in tetrahydrofuran (10 mL). A 1 N sodium hydroxide aqueous solution (30 mL) was added, and the mixture was stirred for 19 hours. Then, 1 N hydrochloric acid (40 mL) was added to the reaction solution, followed by extraction with ethyl acetate (300 mL). The organic layer was washed with brine and then dried over anhydrous magnesium sulfate, and the solvent was evaporated under reduced pressure. The resulting residue was purified by silica gel column chromatography (elution solvent: methylene chloride/methanol = 50/1) to give the target compound (358 mg, yield: 65%) as a yellow solid.
MS (FAB) m/z: 500.1709 $(M+H)^+$.

(Example 52) (9aS)-8-Acetyl-3-ethoxy-1,7-dihydroxy-9a-methyl-N-[(2-methyl-1-naphthyl)methyl]-9-oxo-9,9a-dihydrodibenzo[b,d]furan-4-carboxamide (exemplary compound No. 2-68)

(52a) (9aS)-8-Acetyl-3-ethoxy-1,7-dihydroxy-9a-methyl-9-oxo-9,9a-dihydrodibenzo[b,d]furan-4-carboxamide

Reaction and post-treatment were carried out in accordance with Example (1a) using cercosporamide obtained in Reference Example 3 (1.00 g, 3.02 mmol), ethyl iodide (0.965 mL, 12.1 mmol), potassium carbonate (1.67 g, 12.1 mmol) and N,N-dimethylformamide (8 mL) to give the target compound (0.684 g, yield: 63%) as a yellow solid.
$^1$H-NMR ($CDCl_3$, 500MHz):δ ppm: 1.51 (3H, t, J=6.8Hz), 1.76 (3H, s), 2.65 (3H, s), 4.10-4.20 (2H, m), 5.73 (1H, brs), 6.04 (1H, s), 6.34 (1H, s), 7.27 (1H, brs), 10.78 (1H, s), 18.83 (1H, s).
MS (FAB) m/z: 360 $(M+H)^+$

(52b) (9aS)-8-Acetyl-3-ethoxy-1,7-dihydroxy-9a-methyl-N-[(2-methyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-3-ethoxy-1,7-dihydroxy-9a-methyl-9-oxo-9,9a-dihydrodibenzo[b,d]furan-4-carboxamide produced in Example (52a) (0.300 g, 0.835 mmol), 2-methyl-1-naphthaldehyde (0.284 g, 1.67 mmol), triethylsilane (0.532 mL, 3.34 mmol), trifluoroacetic acid (0.257 mL, 3.34 mmol) and acetonitrile (15.0 mL) to give the target compound (0.261 g, yield: 61%) as a yellow solid.
$^1$H-NMR ($CDCl_3$, 400MHz):δ ppm: 0.73 (3H, t, J=6.8Hz), 1.74 (3H, s), 2.61 (3H, s), 2.64 (3H, s), 3.76-3.84 (2H, m), 5.00-5.15 (2H, m), 6.04 (1H, s), 6.17 (1H, s), 7.29 (1H, brs), 7.31 (1H, d, J=8.3Hz), 7.42 (1H, t, J=7.9Hz), 7.51 (1H, t, J=8.3Hz), 7.72 (1H, d, J=8.3Hz), 7.81 (1H, d, J=8.3Hz), 8.08 (1H, d, J=8.3Hz), 10.64 (1H, s), 18.79 (1H, s).
MS (FAB) m/z: 514.1864 $(M+H)^+$

(Example 53) (9aS)-8-Acetyl-1,7-dihydroxy-3-methoxy-9a-methyl-N-[(3-methyl-1-naphthyl)methyl]-9-oxo-9,9a-dihydrodibenzo[b,d]furan-4-carboxamide (exemplary compound No. 1-211)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (440 mg, 1.27 mmol), known 3-methyl-1-naphthaldehyde [Tetrahedron, 1985, Vol. 41, p.5205-5208] (324 mg, 1.90 mmol), triethylsilane (620 μL, 3.84 mmol), trifluoroacetic acid (295 μL, 3.83 mmol) and acetonitrile (20 mL) to give the target compound (345 mg, yield: 54%) as a yellow solid.
$^1$H-NMR ($CDCl_3$, 500MHz):δ ppm: 1.73 (3H, s), 2.60 (3H, s), 2.64 (3H, s), 3.71 (3H, s), 5.13-4.97 (2H, m), 5.99 (1H, s), 6.27 (1H, s), 7.28 (1H, m), 7.87 (1H, s), 7.48 (1H, m), 7.58 (1H, s), 7.79 (1H, m), 8.04 (1H, m), 10.65 (1H, s), 18.82 (1H, s).
MS (FAB) m/z: 500.1706 $(M+H)^+$.

(Example 54) (9aS)-8-Acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamide (exemplary compound No. 1-435)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (16.4 g, 47.5 mmol), known 2,3-dimethyl-1-naphthaldehyde [Journal of Chemical Society Perkin Transactions 1, 1972, p.892-894] (10.0 g, 54.3 mmol), triethylsilane (23.0 mL, 142 mmol), trifluoroacetic acid (11 mL, 143 mmol) and acetonitrile (500 mL) to give the target compound (23.2 g, yield: 95%) as a yellow solid.
$^1$H-NMR ($CDCl_3$, 400MHz):δ ppm: 1.75 (3H, s), 2.49 (3H, s), 2.53 (3H, s), 2.64 (3H, s), 3.63 (3H, s), 5.19-5.04 (2H, m), 6.02 (1H, s), 6.23 (1H, s), 7.04 (1H, brs), 7.62-7.41 (2H, m), 7.64 (1H, brs), 7.77 (1H, brd, J=7.9Hz), 8.08 (1H, brd, J=8.7Hz), 10.64 (1H,s), 18.84 (1H,s).
MS (FAB) m/z: 514.1906 $(M+H)^+$.

(Example 55) (9aS)-8-Acetyl-1,7-dihydroxy-3-methoxy-9a-methyl-9-oxo-N-[(2-vinyl-1-naphthyl)methyl]-9,9a-dihydrodibenzo[b,d]furan-4-carboxamide (exemplary compound No. 1-261)

(55a) 1-Bromo-2-vinylnaphthalene

Sodium hydride (970 mg, 22.2 mmol) was suspended in tetrahydrofuran (200 mL) in a nitrogen atmosphere, and the suspension was cooled to 0˚C. Methyltriphenylphosphonium bromide (7.66 g, 21.4 mmol) was slowly added, and the mixture was stirred at 0˚C for 30 minutes. 1-Bromo-2-naphthaldehyde (2.01 g, 8.55 mmol) was further added, and the mixture was stirred at room temperature for two hours. An ammonium chloride aqueous solution (200 mL) was added to the reaction solution, followed by extraction with hexane (300 ml). The organic layer was washed with brine and then dried over anhydrous sodium sulfate. The solvent was evaporated under reduced pressure, and the resulting residue was purified by silica gel column chromatography (elution solvent: hexane) to give the target compound (1.18 g, yield: 59%) as a colorless oil.
$^1$H-NMR ($CDCl_3$, 500MHz):δ ppm: 5.49 (1H, dd, J=1.0, 10.7Hz), 5.84 (1H, dd, J=1.0, 17.1Hz), 7.39 (1H, dd, J=10.7, 17.1Hz), 7.50 (1H, m), 7.59 (1H, m), 7.67 (1H, d, J=8.8Hz), 7.77 (1H, d, J=8.3Hz), 7.80 (1H, d, J=8.8Hz), 8.35 (1H, d, J=8.8Hz).

(55b) 2-Vinyl-1-naphthaldehyde

1-Bromo-2-vinylnaphthalene produced in Example (55a) (1.17 g, 5.02 mmol) was dissolved in tetrahydrofuran (25 mL) in a nitrogen atmosphere, and the solution was cooled to -78˚C. n-Butyllithium (2.44 mol/L solution in hexane, 2.50 mL, 6.10 mmol) was slowly added dropwise, and the mixture was stirred at -78˚C for 30 minutes. Dimethylformamide (780 μL, 10.0 mmol) was slowly added, and the mixture was stirred with heating to room temperature for one hour. An ammonium chloride aqueous solution (50 mL) was added to the reaction solution, followed by extraction with ethyl acetate (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 30/1 to 10/1) to give the target compound (475 mg, yield: 52%) as a light yellow oil.
$^1$H-NMR ($CDCl_3$, 500MHz):δ ppm: 5.69 (1H, dd, J=1.0, 11.2Hz), 5.74 (1H, dd, J=1.0, 17.1Hz), 7.47 (1H, dd, J=11.2, 17.1Hz), 7.55 (1H, m), 7.59 (1H, d, J=8.3Hz), 7.64 (1H, m), 7.86 (1H, d, J=8.8Hz), 8.01 (1H, d, J=8.8Hz), 8.97 (1H, d, J=8.8Hz), 10.86 (1H, s).
MS (EI) m/z: 182.0734 $(M^+)$

(55c) (9aS)-8-Acetyl-1,7-dihydroxy-3-methoxy-9a-methyl-9-oxo-N-[(2-vinyl-1-naphthyl)methyl]-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (239 mg, 0.692 mmol), 2-vinyl-1-naphthaldehyde produced in Example (55b) (242 mg, 1.33 mmol), triethylsilane (220 μL, 1.38 mmol), trifluoroacetic acid (105 μL, 1.36 mmol) and acetonitrile (6 mL) to give the target compound (120 mg, yield: 34%) as a yellow solid.
$^1$H-NMR ($CDCl_3$, 500MHz):δ ppm: 1.77 (3H, s), 2.66 (3H, s), 3.65 (3H, s), 5.13-6.69 (6H, m), 6.94-8.20 (8H, m), 10.70 (1H, s), 18.85 (1H, s).
MS (FAB) m/z: 510.1543 $(M-H)^+$.

(Example 56) (9aS)-8-Acetyl-N-[(2-ethyl-1-naphthyl)methyl]-1,7-dihydroxy-3-methoxy-9a-methyl-9-oxo-9,9a-dihydrodibenzo[b,d]furan-4-carboxamide (exemplary compound No. 1-213)

(56a) 2-Ethyl-1-naphthaldehyde

2-Vinyl-1-naphthaldehyde produced in Example (55b) (228 mg, 1.25 mmol) was dissolved in ethyl acetate (12 mL). 10% palladium-carbon (58 mg) was added, and the mixture was stirred in a hydrogen (1 atm) atmosphere for one hour. Palladium-carbon was separated by filtration, and then the solvent was evaporated under reduced pressure. The resulting residue was purified by silica gel column chromatography (elution solvent: hexane/ethyl acetate = 30/1 to 10/1) to give the target compound (222 mg, yield: 96%) as a colorless oil.
$^1$H-NMR ($CDCl_3$, 500MHz):δ ppm: 1.37 (3H, t, J=7.6Hz), 3.17 (2H, q, J=7.6Hz), 7.39 (1H, d, J=8.8Hz), 7.52 (1H, m), 7.63 (1H, m), 7.84 (1H, d, J=8.3Hz), 7.99 (1H, d, J=8.8Hz), 9.01 (1H, d, J=9.3Hz), 10.93 (1H, s).
MS (EI) m/z: 184.0879 $(M^+)$

(56b) (9aS)-8-Acetyl-N-[(2-ethyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (273 mg, 0.791 mmol), 2-ethyl-1-naphthaldehyde produced in Example (56a) (280 mg, 1.52 mmol), triethylsilane (240 μL, 1.51 mmol), trifluoroacetic acid (120 μL, 1.56 mmol) and acetonitrile (8 mL) to give the target compound (323 mg, yield: 80%) as a yellow solid.
$^1$H-NMR ($CDCl_3$, 500MHz):δ ppm: 1.31 (3H, t, J=7.6Hz), 1.74 (3H, s), 2.64 (3H, s), 2.96 (2H, q, J=7.6Hz), 3.61 (3H, s), 5.06 (1H, dd, J=4.9, 14.7Hz), 5.12 (1H, dd, J=4.9, 14.7Hz), 6.01 (1H, s), 6.22 (1H,s), 7.03 (1H, brs), 7.38 (1H, d, J=8.8Hz), 7.47 (1H, m), 7.54 (1H, m), 7.79 (1H, d, J=8.3Hz), 7.85 (1H, d, J=8.3Hz), 8.12 (1H, d, J=8.3Hz), 10.63 (1H, s), 18.83 (1H, s).
MS (FAB) m/z: 514.1848 $(M+H)^+$

(Example 57) (9aS)-8-Acetyl-1,7-dihydroxy-3-methoxy-9a-methyl-9-oxo-N-[(2-propyl-1-naphthyl)methyl]-9,9a-dihydrodibenzo[b,d]furan-4-carboxamide (exemplary compound No. 1-216)

(57a) 1-Bromo-2-prop-1-en-1-ylnaphthalene

Sodium hydride (569 mg, 13.0 mmol) was suspended in tetrahydrofuran (50 mL) in a nitrogen atmosphere, and the suspension was cooled to 0˚C. Ethyltriphenylphosphonium bromide (4.73 g, 12.7 mmol) was slowly added, and the mixture was stirred at 0˚C for 30 minutes. 1-Bromo-2-naphthaldehyde (1.20 g, 5.10 mmol) was further added, and the mixture was stirred at room temperature for one hour and then heated to reflux for 30 minutes. After cooling, an ammonium chloride aqueous solution (100 mL) was added to the reaction solution, followed by extraction with hexane (200 ml). The organic layer was washed with brine and then dried over anhydrous sodium sulfate. The solvent was evaporated under reduced pressure, and the resulting residue was purified by silica gel column chromatography (elution solvent: hexane) to give the target compound (1.19 g, yield: 94%) as a colorless oil.
$^1$H-NMR ($CDCl_3$, 400MHz):δ ppm: 1.81 and 2.00 (3H, ddx2, J=1.6, 6.8Hz), 5.97 and 6.34 (1H, mx2), 6.70 and 7.07 (1H, mx2), 7.41-7.62 (3H, m), 7.71-7.83 (2H, m), 8.34 (1H, m).
MS (EI) m/z: 246.0054 $(M^+)$

(57b) 2-Prop-1-en-1-yl-1-naphthaldehyde

1-Bromo-2-prop-1-en-1-ylnaphthalene produced in Example (57a) (1.18 g, 4.77 mmol) was dissolved in tetrahydrofuran (25 mL) in a nitrogen atmosphere, and the solution was cooled to -78˚C. n-Butyllithium (2.44 mol/L solution in hexane, 2.35 mL, 5.73 mmol) was slowly added dropwise, and the mixture was stirred at - 78˚C for 30 minutes. Dimethylformamide (750 μL, 9.64 mmol) was slowly added, and the mixture was stirred with heating to room temperature for one hour. An ammonium chloride aqueous solution (50 mL) was added to the reaction solution, followed by extraction with ethyl acetate (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1) to give the target compound (825 mg, yield: 88%) as a light yellow oil.
$^1$H-NMR ($CDCl_3$, 400MHz):δ ppm: 1.67 and 2.02 (3H, ddx2, J=1.6, 6.8Hz), 6.15 (1H, m), 6.91 and 7.10 (1H, mx2), 7.33-7.68 (3H, m), 7.82-8.03 (2H, m), 9.00 and 9.24 (1H, dx2, J=8.6Hz), 10.63 and 10.77 (1H, sx2).
MS (EI) m/z: 196.0885 ($M^+$)

(57c) 2-Propyl-1-naphthaldehyde

2-Prop-1-en-1-yl-1-naphthaldehyde produced in Example (57b) (812 mg, 4.14 mmol) was dissolved in ethyl acetate (30 mL). 10% palladium-carbon (144 mg) was added, and the mixture was stirred in a hydrogen (1 atm) atmosphere for two hours. Palladium-carbon was separated by filtration, and then the solvent was evaporated under reduced pressure. The resulting residue was purified by silica gel column chromatography (elution solvent: hexane/ethyl acetate = 20/1) to give the target compound (773 mg, yield: 94%) as a colorless oil.
$^1$H-NMR ($CDCl_3$, 500MHz):δ ppm: 1.03 (3H, t, J=7.3Hz), 1.72-1.79 (2H, m), 3.11 (2H, t, J=7.3Hz), 7.36 (1H, d, J=8.8Hz), 7.51 (1H, m), 7.62 (1H, m), 7.84 (1H, d, J=8.3Hz), 7.97 (1H, d, J=8.3Hz), 9.02 (1H, d, J=8.8Hz), 10.91 (1H, s).
MS (EI) m/z: 198.1046 ($M^+$)

(57d) (9aS)-8-Acetyl-1,7-dihydroxy-3-methoxy-9a-methyl-9-oxo-N-[(2-propyl-1-naphthyl)methyl]-9,9a-dihydrodibenzo [b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (304 mg, 0.880 mmol), 2-propyl-1-naphthaldehyde produced in Example (57c) (523 mg, 2.64 mmol), triethylsilane (420 μL, 2.64 mmol), trifluoroacetic acid (210 μL, 2.73 mmol) and acetonitrile (10 mL) to give the target compound (415 mg, yield: 89%) as a yellow solid.
$^1$H-NMR ($CDCl_3$, 500MHz):δ ppm: 1.03 (3H, t, J=7.3Hz), 1.67-1.74 (2H, m), 1.75 (3H, s), 2.64 (3H, s), 2.89-2.93 (2H, m), 3.60 (3H, s), 5.06 (1H, dd, J=4.4, 14.2Hz), 5.12 (1H, dd, J=4.4, 14.2Hz), 6.01 (1H, s), 6.22 (1H,s), 7.01 (1H, brs), 7.36 (1H, d, J=8.3Hz), 7.46 (1H, m), 7.54 (1H, m), 7.77 (1H, d, J=8.3Hz), 7.84 (1H, d, J=8.3Hz), 8.11 (1H, d, J=8.8Hz), 10.63 (1H, s), 18.83 (1H, s).
MS (FAB) m/z: 528.2048 $(M+H)^+$.

(Example 58) (9aS)-8-Acetyl-N-[(2-butyl-1-naphthyl)methyl]-1,7-dihydroxy-3-methoxy-9a-methyl-9-oxo-9,9a-dihydrodibenzo[b,d]furan-4-carboxamide (exemplary compound No. 1-222)

(58a) 1-Bromo-2-but-1-en-1-ylnaphthalene

Sodium hydride (383 mg, 8.78 mmol) was suspended in tetrahydrofuran (40 mL) in a nitrogen atmosphere, and the suspension was cooled to 0˚C. Propyltriphenylphosphonium bromide (3.30 g, 8.57 mmol) was slowly added, and the mixture was stirred at 0˚C for 30 minutes. 1-Bromo-2-naphthaldehyde (1.07 g, 4.55 mmol) was further added, and the mixture was stirred at room temperature for one hour and then heated to reflux for one hour. After cooling, an ammonium chloride aqueous solution (100 mL) was added to the reaction solution, followed by extraction with hexane (200 ml). The organic layer was washed with brine and then dried over anhydrous sodium sulfate. The solvent was evaporated under reduced pressure, and the resulting residue was purified by silica gel column chromatography (elution solvent: hexane) to give the target compound (1.19 g, yield: 100%) as a colorless oil.
$^1$H-NMR ($CDCl_3$, 400MHz):δ ppm: 1.05 and 1.17 (3H, tx2, J=7.3Hz), 2.19-2.38 (2H, m), 5.83 and 6.36 (1H, mx2), 6.64 and 7.05 (1H, mx2), 7.38-7.63 (3H, m), 7.71-7.82 (2H, m), 8.34 (1H, m).
MS (EI) m/z: 260.0197 ($M^+$)

(58b) 2-But-1-en-1-yl-1-naphthaldehyde

1-Bromo-2-but-1-en-1-ylnaphthalene produced in Example (58a) (1.19 g, 4.55 mmol) was dissolved in tetrahydrofuran (25 mL) in a nitrogen atmosphere, and the solution was cooled to -78˚C. n-Butyllithium (2.59 mol/L solution in hexane, 2.10 mL, 5.44 mmol) was slowly added dropwise, and the mixture was stirred at - 78˚C for 30 minutes. Dimethylformamide (710 μL, 9.13 mmol) was slowly added, and the mixture was stirred with heating to room temperature for one hour. An ammonium chloride aqueous solution (50 mL) was added to the reaction solution, followed by extraction with ethyl acetate (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 10/1) to give the target compound (774 mg, yield: 81 %) as a light yellow oil.
$^1$H-NMR ($CDCl_3$, 400MHz):δ ppm: 0.98 and 1.17 (3H, tx2, J=7.3Hz), 2.01-2.38 (2H, m), 6.03 and 6.21 (1H, mx2), 6.85 and 7.08 (1H, mx2), 7.32-7.67 (3H, m), 7.82-8.02 (2H, m), 9.01 and 9.24 (1H, dx2, J=8.3Hz), 10.66 and 10.78 (1H, sx2).
MS (EI) m/z: 210.1040 ($M^+$)

(58c) 2-Butyl-1-naphthaldehyde

2-But-1-en-1-yl-1-naphthaldehyde produced in Example (58b) (764 mg, 3.63 mmol) was dissolved in ethyl acetate (30 mL). 10% palladium-carbon (163 mg) was added, and the mixture was stirred in a hydrogen (1 atm) atmosphere for two hours. Palladium-carbon was separated by filtration, and then the solvent was evaporated under reduced pressure. The resulting residue was purified by silica gel column chromatography (elution solvent: hexane/ethyl acetate = 30/1 to 20/1) to give the target compound (521 mg, yield: 68%) as a colorless oil.
$^1$H-NMR ($CDCl_3$, 500MHz):δ ppm: 0.96 (3H, t, J=7.3Hz), 1.41-1.48 (2H, m), 1.67-1.73 (2H, m), 3.12 (2H, t, J=7.8Hz), 7.36 (1H, d, J=8.3Hz), 7.51 (1H, m), 7.62 (1H, m), 7.83 (1H, d, J=7.8Hz), 7.96 (1H, d, J=8.3Hz), 9.02 (1H, d, J=8.8Hz), 10.91 (1H, s).
MS (EI) m/z: 212.1194 ($M^+$)

(58d) (9aS)-8-Acetyl-N-[(2-butyl-1-naphthyl)methyl]-1,7-dihydroxy-3-methoxy-9a-methyl-9-oxo-9,9a-dihydrodibenzo[b, d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (303 mg, 0.877 mmol), 2-butyl-1-naphthaldehyde produced in Example (58c) (509 mg, 2.40 mmol), triethylsilane (420 μL, 2.64 mmol), trifluoroacetic acid (210 μL, 2.73 mmol) and acetonitrile (10 mL) to give the target compound (391 mg, yield: 82%) as a yellow solid.
$^1$H-NMR ($CDCl_3$, 500MHz):δ ppm: 0.95 (3H, t, J=7.3Hz), 1.41-1.48 (2H, m), 1.62-1.68 (2H, m), 1.75 (3H, s), 2.64 (3H, s), 2.90-2.94 (2H, m), 3.60 (3H, s), 5.06 (1H, dd, J=4.4, 14.2Hz), 5.12 (1H, dd, J=4.4, 14.2Hz), 6.01 (1H, s), 6.22 (1H, s), 7.00 (1H, brs), 7.36 (1H, d, J=8.8Hz), 7.46 (1H, m), 7.54 (1H, m), 7.77 (1H, d, J=8.3Hz), 7.84 (1H, d, J=7.8Hz), 8.11 (1H, d, J=8.8Hz), 10.63 (1H, s), 18.83 (1H, s).
MS (FAB) m/z: 542.2185 $(M+H)^+$

(Example 59) (9aS)-8-Acetyl-1,3,7-trihydroxy-9a-methyl-N-[(2-methyl-1-naphthyl)methyl]-9-oxo-9,9a-dihydrodibenzo [b,d]furan-4-carboxamide (exemplary compound No. 2-3)

(59a) (9aS)-8-Acetyl-3-(benzyloxy)-1,7-dihydroxy-9a-methyl-N-[(2-methyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-3-(benzyloxy)-1,7-dihydroxy-9a-methyl-9-oxo-9,9a-dihydrodibenzo[b,d]furan-4-carboxamide produced in Example (43a) (1.01 g, 2.39 mmol), 2-methyl-1-naphthaldehyde (1.22 g, 7.17 mmol), triethylsilane (1.15 mL, 7.22 mmol), trifluoroacetic acid (550 μL, 7.14 mmol) and acetonitrile (20 mL) to give the target compound (719 mg, yield: 52%) as a yellow solid.
$^1$H-NMR ($CDCl_3$, 500MHz):δ ppm: 1.76 (3H, s), 2.34 (3H, s), 2.65 (3H, s), 4.85-5.05 (4H, m), 6.05 (1H, s), 6.35 (1H, s), 6.98 (2H, d, J=6.8Hz), 7.05 (2H, t, J=7.8Hz), 7.16-7.24 (3H, m), 7.39-7.44 (2H, m), 7.70(1H, d, J=8.3Hz), 7.80 (1H, m), 8.00 (1H, m), 10.70 (1H, s), 18.84 (1H, s).
MS (FAB) m/z: 576.2021 $(M+H)^+$

(59b) (9aS)-8-Acetyl-1,3,7-trihydroxy-9a-methyl-N-[(2-methyl-1-naphthyl)methyl]-9-oxo-9,9a-dihydrodibenzo[b,d]furan-4-carboxamide

(9aS)-8-Acetyl-3-(benzyloxy)-1,7-dihydroxy-9a-methyl-N-[(2-methyl-1-naphthyl)methyl]-9-oxo-9,9a-dihydrodibenzo[b,d]furan-4-carboxamide produced in Example (59a) (710 mg, 1.23 mmol) was dissolved in a mixed solvent of ethanol (10 mL)-ethyl acetate (10 mL). 10% palladium-carbon (246 mg) was added, and the mixture was stirred in a hydrogen (1 atm) atmosphere at room temperature for five hours and 30 minutes. Palladium-carbon was separated by filtration from the reaction solution, and then the solvent was evaporated under reduced pressure. The resulting residue was purified by reverse phase column chromatography (elution solvent: acetonitrile/water = 70/1 to 95/5) to give the target compound (357 mg, yield: 60%) as a yellow solid.
$^1$H-NMR ($CDCl_3$, 400MHz):$\delta$ ppm: 1.65 (3H, s), 2.62 (3H, s), 2.65 (3H, s), 5.07 (1H, dd, J=4.9, 14.7Hz), 5.19 (1H, dd, J=5.4, 14.7Hz), 5.52 (1H, s), 6.32 (1H, s), 7.15 (1H, brs), 7.39 (1H, d, J=8.3Hz), 7.50 (1H, m), 7.59 (1H, m), 7.79 (1H, d, J=8.8Hz), 7.88 (1H, d, J=8.3Hz), 8.09 (1H, d, J=8.3Hz), 10.41 (1H, s), 13.23 (1H, s), 18.73 (1H, s).
MS (FAB) m/z: 486.1559 $(M+H)^+$

(Example 60) (9aS)-8-Acetyl-1,7-dihydroxy-3-methoxy-9a-methyl-N-[(3-methoxy-1-naphthyl)methyl]-9-oxo-9,9a-dihydrodibenzo[b,d]furan-4-carboxamide (exemplary compound No. 1-268)

(60a) 3-Methoxy-1-naphthaldehyde

Sodium hydride (115 mg, 2.64 mmol) was suspended in N,N-dimethylformamide (20 mL), and known 3-hydroxy-1-naphthaldehyde [Tetrahedron, 1999, Vol. 55, p.5821-5830] (250 mg, 1.45 mmol) was added at 0°C. After stirring for 30 minutes, iodomethane (230 $\mu$L, 3.69 mmoL) was added, and the mixture was stirred at room temperature for one hour. A saturated ammonium chloride aqueous solution (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give the target compound (122 mg, yield: 45%) as a light yellow solid.
$^1$H-NMR ($CDCl_3$, 500MHz):$\delta$ ppm: 4.86 (3H, s), 7.38 (1H, m), 7.55-7.51 (2H, m), 7.64 (1H, m), 7.79 (1H, m), 9.90 (1H, m), 10.35 (1H, s).

(60b) (9aS)-8-Acetyl-1,7-dihydroxy-3-methoxy-9a-methyl-N-[(3-methoxy-1-naphthyl)methyl]-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (200 mg, 0.579 mmol), 3-methoxy-1-naphthaldehyde produced in Example (60a) (122 mg, 0.655 mmol), triethylsilane (280 $\mu$L, 1.73 mmol), trifluoroacetic acid (135 $\mu$L, 1.75 mmol) and acetonitrile (9 mL) to give the target compound (114 mg, yield: 38%) as a yellow solid.
$^1$H-NMR ($CDCl_3$, 400MHz):$\delta$ ppm: 1.75 (3H, s), 2.64 (3H, s), 3.75 (3H, s), 5.12-4.97 (2H, m), 6.00 (1H, s), 6.27 (1H, s), 7.07 (1H, brs), 7.22 (1H, brs), 7.27 (1H, m), 7.49-7.35 (2H, m), 7.76 (1H, brd, J=7.1Hz), 7.98 (1H, brd, J=7.9Hz), 10.64 (1H, s), 18.78 (1H, s).
MS (FAB) m/z: 516.1653 $(M+H)^+$.

(Example 61) (9aS)-8-Acetyl-1,7-dihydroxy-3-methoxy-9a-methyl-N-[(3-hydroxy-1-naphthyl)methyl]-9-oxo-9,9a-dihydrodibenzo[b,d]furan-4-carboxamide (exemplary compound No. 1-292)

(61a) 4-Formyl-2-naphthyl acetate

Known 3-hydroxy-1-naphthaldehyde [Tetrahedron, 1999, Vol. 55, p.5821-5830] (233 mg, 1.35 mmol) was dissolved in pyridine (10 mL) and acetic anhydride (5 mL), and the mixture was stirred for four hours. The solvent was evaporated under reduced pressure, and the resulting residue was directly used for the next reaction.

(61b) 4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthyl acetate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (390 mg,

1.13 mmol), 4-formyl-2-naphthyl acetate produced in Example (61a) (290 mg, 1.35 mmol), triethylsilane (730 μL, 4.52 mmol), trifluoroacetic acid (350 μL, 4.52 mmol) and acetonitrile (17 mL) to give the target compound (488 mg, yield: 79%) as a yellow solid.
$^1$H-NMR ($CDCl_3$, 400MHz):δ ppm: 1.74 (3H, s), 2.35 (3H, s), 2.64 (3H, s), 3.75 (3H, s), 5.17-5.00 (2H, m), 5.99 (1H, s), 6.28 (1H, s), 7.36-7.26 (2H, m), 7.59-7.43 (3H, m), 7.82 (1H, brs), 8.06 (1H, brs), 10.65 (1H, s), 18.78 (1H, s).
MS (FAB) m/z: 544.1614 $(M+H)^+$

(61c) (9aS)-8-Acetyl-1,7-dihydroxy-3-methoxy-9a-methyl-N-[(3-hydroxy-1-naphthyl)methyl]-9-oxo-9,9a-dihydrodibenzo[b,d]furan-4-carboxamide

4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthyl acetate produced in Example (61b) (255 mg, 0.469 mmol) was dissolved in a mixed solvent of methanol (3 mL)-methylene chloride (3 mL). Potassium carbonate (325 mg, 2.35 mmol) was added, and the mixture was stirred in a nitrogen atmosphere at room temperature for one hour. 1 N hydrochloric acid (10 mL) was added, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: methylene chloride/methanol = 50/1 to 20/1) to give the target compound (169 mg, yield: 72%) as a yellow solid.
$^1$H-NMR ($CDCl_3$, 400MHz):δ ppm: 1.71 (3H, s), 2.63 (3H, s), 3.75 (3H, s), 5.06-5.02 (2H, brd, J=5.2Hz), 5.93 (1H, s), 6.26 (1H, s), 7.09 (1H, brs), 7.26-7.19 (2H, m), 7.46-7.33 (2H, m), 7.67 (1H, brd, J=7.9Hz), 7.95 (1H, brd, J=8.3Hz), 10.65 (1H, s), 18.75 (1H, s).
MS (FAB) m/z: 502.1525 $(M+H)^+$

(Example 62) (9aS)-8-Acetyl-1,7-dihydroxy-3-methoxy-9a-methyl-N-[(3-ethyl-1-naphthyl)methyl]-9-oxo-9,9a-dihydrodibenzo[b,d]furan-4-carboxamide (exemplary compound No. 1-214)

(62a) 4-Formyl-1-naphthyl trifluoromethanesulfonate

Sodium hydride (1.38 g, 31.6 mmol) was suspended in tetrahydrofuran (100 mL), and known 3-hydroxy-1-naphthaldehyde [Tetrahedron, 1999, Vol. 55, p.5821-5830] (3.50 g, 20.3 mmol) was added at 0˚C. After stirring for 30 minutes, N-phenyl-bis(trifluoromethanesulfonimide) (7.30 g, 20.4 mmoL) was added, and the mixture was stirred at 0˚C for 1.5 hours. A saturated ammonium chloride aqueous solution (100 mL) was added to the reaction solution, followed by extraction with diethyl ether (200 mL). The organic layer was washed with brine and then dried over anhydrous magnesium sulfate. The solvent was evaporated under reduced pressure, and the resulting residue was purified by silica gel column chromatography (elution solvent: hexane/diethyl ether = 5/1) to give the target compound (6.50 g, yield: ~100%) as a light yellow solid.
$^1$H-NMR ($CDCl_3$, 400MHz):δ ppm: 7.69 (1H, m), 7.77 (1H, m), 7.87 (1H, brd, J=2.7Hz), 7.95 (1H, brd, J=8.2Hz), 8.00 (1H, brd, J=2.7Hz), 9.17 (1H, d, J=8.6Hz), 10.42 (1H, s).

(62b) 3-Ethyl-1-naphthaldehyde

4-Formyl-1-naphthyl trifluoromethanesulfonate produced in Example (62a) (400 mg, 1.31 mmol) was dissolved in N,N-dimethylformamide (13 mL). Tris(dibenzylideneacetone)dipalladium (36 mg, 0.039 mmol), triphenylarsine (48 mg, 0.16 mmol) and lithium chloride (170 mg, 4.01 mmol) were added, and the mixture was stirred at room temperature for 10 minutes. Tetraethyltin (910 μL, 4.60 mmol) was added to the reaction solution, and the mixture was stirred at 60˚C for 23 hours. A saturated ammonium chloride aqueous solution (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/diethyl ether = 10/1) to give the target compound (130 mg, yield: 54%) as a brown oil.
$^1$H-NMR ($CDCl_3$, 400MHz):δ ppm: 1.37 (3H, t, J=7.4Hz), 2.88 (2H, q, J=7.4Hz), 7.55 (1H, m), 7.62 (1H, m), 7.88-7.83 (3H, m), 9.17 (1H, d, J=8.6Hz), 10.39 (1H, s).

(62c) (9aS)-8-Acetyl-1,7-dihydroxy-3-methoxy-9a-methyl-N-[(3-ethyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (280 mg,

0.811 mmol), 3-ethyl-1-naphthaldehyde produced in Example (62b) (190 mg, 1.03 mmol), triethylsilane (400 μL, 2.48 mmol), trifluoroacetic acid (190 μL, 2.47 mmol) and acetonitrile (12 mL) to give the target compound (105 mg, yield: 25%) as a yellow solid.
$^1$H-NMR ($CDCl_3$, 400MHz):δ ppm: 1.30 (3H, m), 1.73 (3H, s), 2.64 (3H, s), 2.79 (3H, m), 3.70 (3H, s), 5.16-4.92 (2H, m), 5.98 (1H, s), 6.24 (1H, s), 8.13-7.07 (7H, m), 10.62 (1H, s), 18.74 (1H, s).
MS (FAB) m/z: 514.1846 $(M+H)^+$

(Example 63) (9aS)-8-Acetyl-1,7-dihydroxy-3-methoxy-9a-methyl-N-[(3-butyl-1-naphthyl)methyl]-9-oxo-9,9a-dihydrodibenzo[b,d]furan-4-carboxamide (exemplary compound No. 1-223)

(63a) 3-Ethyl-1-naphthaldehyde

Reaction and post-treatment were carried out in accordance with Example (62b) using 4-formyl-1-naphthyl trifluoromethanesulfonate produced in Example (62a) (450 mg, 1.48 mmol), tris(dibenzylideneacetone)dipalladium (40 mg, 0.044 mmol), triphenylarsine (55 mg, 0.18 mmol), lithium chloride (190 mg, 4.48 mmol), tetrabutyltin (2.0 mL, 6.09 mmol) and N,N-dimethylformamide (15 mL) to give the target compound (204 mg, yield: 65%) as a brown oil.
$^1$H-NMR ($CDCl_3$, 400MHz):δ ppm: 1.01-0.93 (3H, m), 1.46-1.29 (2H, m), 1.77-1.64 (2H, m), 2.84 (2H, t, J=7.8Hz), 7.54 (1H, m), 7.61 (1H, m), 7.85-7.80 (3H, m), 9.17 (1H, d, J=8.2Hz), 10.36 (1H, s).

(63b) (9aS)-8-Acetyl-1,7-dihydroxy-3-methoxy-9a-methyl-N-[(3-butyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (250 mg, 0.724 mmol), 3-butyl-1-naphthaldehyde produced in Example (63a) (200 mg, 0.942 mmol), triethylsilane (350 μL, 2.17 mmol), trifluoroacetic acid (170 μL, 2.21 mmol) and acetonitrile (10 mL) to give the target compound (165 mg, yield: 42%) as a yellow solid.
$^1$H-NMR ($CDCl_3$, 400MHz):δ ppm: 0.95 (3H, t, J=7.5Hz), 1.39 (2H, m), 1.69 (2H, m), 1.74 (3H, s), 2.76 (2H, t, J=7.5Hz), 3.70 (3H, s), 5.11-4.98 (2H, m), 5.99 (1H, s), 6.25 (1H, s), 7.22 (1H, m), 7.37 (1H, s), 7.51-7.43 (2H, m), 7.57 (1H, m), 7.80 (1H, m), 8.04 (1H, m), 10.63 (1H, s), 18.78 (1H, s).
MS (ESI) m/z: 542.21536 $(M+H)^+$.

(Example 64) (9aS)-8-Acetyl-1,7-dihydroxy-3-methoxy-9a-methyl-9-oxo-N-(2,4,6-triethylbenzyl)-9,9a-dihydrodibenzo [b,d]furan-4-carboxamide (exemplary compound No. 1-68)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), known 2,4,6-triethylbenzaldehyde [Journal of Organic Chemistry, 2004, Vol. 69, p.5568-5577] (0.276 g, 1.45 mmol), triethylsilane (0.554 mL, 3.48 mmol), trifluoroacetic acid (0.268 mL, 3.48 mmol) and acetonitrile (15.0 mL) to give the target compound (0.262 g, yield: 59%) as a yellow solid.
$^1$H-NMR ($CDCl_3$, 400MHz):δ ppm: 1.24 (9H, t, J=7.5Hz), 1.75 (3H, s), 2.62 (2H, q, J=7.5Hz), 2.64 (3H, s), 2.73 (4H, q, J=7.5Hz), 3.87 (3H, s), 4.62-4.63 (2H, m), 6.02 (1H, s), 6.27 (1H, s), 6.68 (1H, brs), 6.95 (2H, s), 10.63 (1H, s), 18.83 (1H, s).
MS (FAB) m/z: 490.1881 $(M+H)^+$

(Example 65) (9aS)-8-Acetyl-1,7-dihydroxy-3-methoxy-9a-methyl-9-oxo-N-(5,6,7,8-tetrahydronaphthalen-1-ylmethyl)-9,9a-dihydrodibenzo[b,d]furan-4-carboxamide (exemplary compound No. 1-465)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), known 5,6,7,8-tetrahydronaphthalene-1-carbaldehyde [Tetrahedron, 1997, Vol. 53, p.15969-15982] (0.278 g, 1.74 mmol), triethylsilane (0.554 mL, 3.48 mmol), trifluoroacetic acid (0.268 mL, 3.48 mmol) and acetonitrile (15.0 mL) to give the target compound (0.400 g, yield: 94%) as a yellow solid.
$^1$H-NMR ($CDCl_3$, 500MHz):δ ppm: 1.76 (3H, s), 1.76-1.82 (2H, m), 1.82-1.88 (2H, m), 2.65 (3H, s), 3.86 (3H, s), 4.55-4.68 (2H, m), 6.01 (1H, s), 6.32 (1H, s), 6.91 (1H, brs), 6.97 (2H, s), 7.24 (1H, s), 10.68 (1H, s), 18.83 (1H, s).
MS (FAB) m/z: 490.1881 $(M+H)^+$.

(Example 66) (9aS)-8-Acetyl-N-{[2-(difluoromethyl)-naphthyl]methyl}-1,7-dihydroxy-3-methoxy-9a-methyl-9-oxo-9,9a-dihydrodibenzo[b,d]furan-4-carboxamide (exemplary compound No. 1-228)

(66a) 1-Bromo-2-(difluoromethyl)naphthalene

1-Bromo-2-naphthaldehyde (1.55 g, 6.58 mmol) was dissolved in methylene chloride (35 mL) in a nitrogen atmosphere. Diethylaminosulfur trifluoride (1.00 mL, 7.57 mmol) was slowly added, and the mixture was heated to reflux for 24 hours. After cooling, a sodium bicarbonate aqueous solution (50 mL) was added to the reaction solution, followed by extraction with methylene chloride (100 ml). The organic layer was washed with brine and then dried over anhydrous sodium sulfate. The solvent was evaporated under reduced pressure, and the resulting residue was purified by silica gel column chromatography (elution solvent: hexane) to give the target compound (1.21 g, yield: 72%) as a colorless oil.
$^1$H-NMR ($CDCl_3$, 400MHz):δ ppm: 7.24 (1H, t, J=55.2Hz), 7.61-7.68 (2H, m), 7.72 (1H, d, J=8.3Hz), 7.89 (1H, d, J=8.8Hz), 7.92 (1H, d, J=8.8Hz), 8.38 (1H, d, J=8.3Hz).
MS (EI) m/z: 256 ($M^+$)

(66b) 2-Difluoromethyl-1-naphthaldehyde

1-Bromo-2-(difluoromethyl)naphthalene produced in Example (66a) (1.21 g, 4.55 mmol) was dissolved in tetrahydrofuran (30 mL) in a nitrogen atmosphere, and the solution was cooled to -78˚C. n-Butyllithium (2.59 mol/L solution in hexane, 2.20 mL, 5.70 mmol) was slowly added dropwise, and the mixture was stirred at - 78˚C for 30 minutes. Dimethylformamide (730 μL, 9.39 mmol) was slowly added, and the mixture was stirred with heating to room temperature for one hour. An ammonium chloride aqueous solution (50 mL) was added to the reaction solution, followed by extraction with ethyl acetate (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 10/1) to give the target compound (714 mg, yield: 74%) as a light yellow oil.
$^1$H-NMR ($CDCl_3$, 400MHz):δ ppm: 7.43 (1H, t, J=55.0Hz), 7.68-7.75 (2H, m), 7.88 (1H, d, J=8.6Hz), 7.98 (1H, d, J=7.8Hz), 8.19 (1H, d, J=9.0Hz), 8.72 (1H, d, J=8.6Hz), 11.08 (1H, s).
MS (EI) m/z: 206.0525 ($M^+$)

(66c) (9aS)-8-Acetyl-N-{[2-(difluoromethyl)-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (392 mg, 1.14 mmol), 2-difluoromethyl-1-naphthaldehyde produced in Example (66b) (705 mg, 3.42 mmol), triethylsilane (545 μL, 3.42 mmol), trifluoroacetic acid (265 μL, 3.44 mmol) and acetonitrile (15 mL) to give the target compound (499 mg, yield: 82%) as a yellow solid.
$^1$H-NMR ($CDCl_3$, 500MHz):δ ppm: 1.74 (3H, s), 2.64 (3H, s), 3.66 (3H, s), 5.14 (1H, dd, J=5.2, 14.7Hz), 5.24 (1H, dd, J=5.2, 14.7Hz), 6.03 (1H, s), 6.23 (1H,s), 7.30 (1H, t, J=55.3Hz), 7.52 (1H, brs), 7.59-7.71 (3H, m), 7.91-7.95 (2H, m), 8.37 (1H, d, J=8.3Hz), 10.70 (1H, s), 18.84 (1H, s).
MS (FAB) m/z: 536.1526 $(M+H)^+$

(Example 67) Methyl 4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthoate (exemplary compound No. 1-319)

(67a) Methyl 4-formyl-2-naphthoate

4-Formyl-1-naphthyl trifluoromethanesulfonate produced in Example (62a) (460 mg, 1.51 mmol) was dissolved in N,N-dimethylformamide (15 mL).
Palladium acetate (10 mg, 0.045 mmol), 1,1-bis(diphenylphosphino)ferrocene (51 mg, 0.092 mmol), triethylamine (430 μL, 3.09 mmol) and methanol (1.3 mL, 32.1 mmol) were added, and the mixture was stirred in a carbon monoxide atmosphere at 60˚C for 2.5 hours. A saturated ammonium chloride aqueous solution (10 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 5/1) to give the target compound (308 mg, yield: 95%) as a light yellow solid.
$^1$H-NMR ($CDCl_3$, 400MHz):δ ppm: 4.04 (3H, s), 7.68 (1H, m), 7.82 (1H, m), 8.05 (1H, brd, J=7.8Hz), 8.59 (1H, brs), 8.84

(1H, brs), 9.31 (1H, d, J=8.6Hz), 10.43 (1H, s).

(67b) Methyl 4-[({[(9aS)-8-acetyl-1,7-dihydroxy-3-methoxy-9a-methyl-9-oxo-9,9a-dihydrodibenzo[b,d]furan-4-yl]carbonyl} amino)methyl]-2-naphthoate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (400 mg, 1.16 mmol), methyl 4-formyl-2-naphthoate produced in Example (67a) (308 mg, 1.44 mmol), triethylsilane (565 μL, 3.50 mmol), trifluoroacetic acid (270 μL, 3.50 mmol) and acetonitrile (20 mL) to give the target compound (452 mg, yield: 72%) as a yellow solid.
$^1$H-NMR ($CDCl_3$, 400MHz):δ ppm: 1.75 (3H, s), 2.64 (3H, s), 3.79 (3H, s), 3.97 (3H, s), 5.20-5.06 (2H, m), 6.01 (1H, s), 6.29 (1H, s), 7.33 (1H, m), 7.57 (1H, m), 7.66 (1H, m), 7.98 (1H, d, J=8.3Hz), 8.12 (1H, s), 8.13 (1H, m), 8.55 (1H, s), 10.65 (1H, s), 18.78 (1H, s).
MS (ESI) m/z: 566.14429 $(M+Na)^+$.

(Example 68) 4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthoic acid (exemplary compound No. 1-295)

A 1 N lithium hydroxide aqueous solution (24 mL) was added to methyl 4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthoate produced in Example (67b) (333 mg, 0.613 mmol), and the mixture was stirred for 1.5 hours. 1 N hydrochloric acid (50 mL) was added, followed by extraction with ethyl acetate (300 mL). The organic layer was washed with brine and then dried over anhydrous sodium sulfate. The solvent was evaporated under reduced pressure, and the resulting residue was purified by reverse phase column chromatography (elution solvent: water/methanol = 1/1 to 1/3) to give the target compound (188 mg, yield: 58%) as a yellow solid.
$^1$H-NMR ($CDCl_3$, 400MHz):δ ppm: 1.74 (3H, s), 2.65 (3H, s), 3.82 (3H, s), 5.22-5.10 (2H, m), 6.00 (1H, s), 6.30 (1H, s), 7.17 (1H, m), 7.72-7.41 (2H, m), 7.99 (1H, m), 8.22-8.08 (2H, m), 8.60 (1H, m), 10.64 (1H, s), 18.78 (1H, s).
MS (ESI) m/z: 530.14626 $(M+H)^+$.

(Example 69) (9aS)-8-Acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide (exemplary compound No. 1-450)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (250 mg, 0.724 mmol), known 2,3,4-trimethyl-1-naphthaldehyde [Journal of Chemical Society Perkin Transactions 1, 1972, p. 892-894] (180 mg, 0.908 mmol), triethylsilane (350 μL, 2.17 mmol), trifluoroacetic acid (170 μL, 2.21 mmol) and acetonitrile (11 mL) to give the target compound (254 mg, yield: 66%) as a yellow solid.
$^1$H-NMR ($CDCl_3$, 400MHz):δ ppm: 1.74 (3H, s), 2.46 (3H, s), 2.55 (3H, s), 2.64 (3H, s), 2.67 (3H, s), 3.62 (3H, s), 5.18-5.02 (2H, m), 6.01 (1H, s), 6.22 (1H, s), 7.01 (1H, brs), 7.49 (2H, m), 8.10 (2H, brs), 10.62 (1H, s), 18.82 (1H, s).
MS (ESI) m/z: 528.20281 $(M+H)^+$.

(Example 70) (9aS)-8-Acetyl-N-(2,3-dihydro-1H-indan-4-ylmethyl)-1,7-dihydroxy-3-methoxy-9a-methyl-9-oxo-9,9a-dihydrodibenzo[b,d]furan-4-carboxamide (exemplary compound No. 1-466)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), known indan-4-carbaldehyde [Tetrahedron, 1997, Vol. 53, p.15969-15982] (0.192 mL, 1.31 mmol), triethylsilane (0.554 mL, 3.48 mmol), trifluoroacetic acid (0.268 mL, 3.48 mmol) and acetonitrile (15.0 mL) to give the target compound (0.278 g, yield: 67%) as a yellow solid.
$^1$H-NMR ($CDCl_3$, 500MHz):δ ppm: 1.76 (3H, s), 2.07-2.13 (2H, m), 2.65 (3H, s), 2.92-2.97 (4H, m), 3.86 (3H, s), 4.57-4.66 (2H, m), 6.02 (1H, s), 6.33 (1H, s), 7.13-7.20 (4H, m), 10.69 (1H, s), 18.83 (1H, s).
MS (FAB) m/z: 476.1713 $(M+H)^+$.

(Example 71)(9aS)-8-Acetyl-1,7-dihydroxy-3-methoxy-9a-methyl-9-oxo-N-(2,3,5,6-tetraethylbenzyl)-9,9a-dihydrodibenzo[b,d]furan-4-carboxamide (exemplary compound No. 1-118)

(71 a) 2,3,5,6-Tetraethylbenzaldehyde

Known 1,2,4,5-tetraethylbenzene [Journal of the American Chemical Society, 1992, Vol. 114, p.255-261] (0.510 g, 2.68 mmol) was dissolved in methylene chloride (25 mL). Aluminum chloride (0.393 g, 2.95 mmol) and dichloromethyl methyl ether (0.267 mL, 2.95 mmol) were added in a nitrogen atmosphere at -78˚C. After stirring at -78˚C for five minutes, water (40 mL) was added, followed by extraction with methylene chloride (30 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 98/2 to 96/4) to give the target compound (0.230 g, yield: 39%) as a white solid.
$^1$H-NMR ($CDCl_3$, 500MHz):δ ppm: 1.19 (6H, t, J=7.5Hz), 1.23 (6H, t, J=7.5Hz), 2.66 (4H, q, J=7.5Hz), 2.86 (4H, q, J=7.5Hz), 7.17 (1H, s), 10.62 (1H, s).
MS (EI) m/z: 218 $(M+)^+$

(71b) (9aS)-8-Acetyl-1,7-dihydroxy-3-methoxy-9a-methyl-9-oxo-N-(2,3,5,6-tetraethylbenzyl)-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,3,5,6-tetraethylbenzaldehyde produced in Example (71a) (0.230 mL, 1.05 mmol), triethylsilane (0.554 mL, 3.48 mmol), trifluoroacetic acid (0.268 mL, 3.48 mmol) and acetonitrile (15.0 mL) to give the target compound (0.138 g, yield: 29%) as a yellow solid.
$^1$H-NMR ($CDCl_3$, 500MHz):δ ppm: 1.18 (6H, t, J=7.5Hz), 1.24 (6H, t, J=7.5Hz), 1.75 (3H, s), 2.65 (4H, q, J=7.5Hz), 2.65 (3H, s), 2.72 (4H, q, J=7.5Hz), 3.76 (3H, s), 4.63-4.65 (2H, m), 6.02 (1H, s), 6.27 (1H, s), 6.66 (1H, brs), 7.02 (1H, s), 10.62 (1H, s), 18.83 (1H, s).
MS (FAB) m/z: 548.2647 $(M+H)^+$

(Example 72) Methyl 3-{4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthyl}propanoate (exemplary compound No. 1-380)

(72a) Methyl (2E)-3-(4-formyl-2-naphthyl)acrylate

4-Formyl-1-naphthyl trifluoromethanesulfonate produced in Example (62a) (746 mg, 2.45 mmol) was dissolved in N,N-dimethylformamide (25 mL). Methyl acrylate (442 μL, 4.91 mmol), palladium acetate (17 mg, 0.076 mmol), 1,1-bis(diphenylphosphino)ferrocene (82 mg, 0.148 mmol) and triethylamine (1.1 mL, 7.89 mmol) were added, and the mixture was stirred at 60˚C for 22 hours. A saturated ammonium chloride aqueous solution (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 5/1) to give the target compound (305 mg, yield: 52%).
$^1$H-NMR ($CDCl_3$, 400MHz):δ ppm: 3.85 (3H, s), 6.62 (1H, d, J=15.9Hz), 7.60 (1H, m), 7.69 (1H, m), 7.85 (1H, d, J=15.9Hz), 7.91 (1H, d, J=8.2Hz), 8.13-8.09 (2H, m), 9.15 (1H, d, J=8.6Hz), 10.38 (1H, s).
MS (EI) m/z: 240 $(M^+)$.

(72b) Methyl (2E)-3-{4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthyl}acrylate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (400 mg, 1.16 mmol), methyl (2E)-3-(4-formyl-2-naphthyl)acrylate produced in Example (72a) (307 mg, 1.28 mmol), triethylsilane (560 μL, 3.47 mmol), trifluoroacetic acid (270 μL, 3.50 mmol) and acetonitrile (20 mL) to give a crude purified product of the target compound as a yellow solid. The product was directly used for the next reaction without further purification.
$^1$H-NMR ($CDCl_3$, 400MHz):δ ppm: 1.74 (3H, s), 2.64 (3H, s), 3.76 (3H, s), 3.83 (3H, s), 5.18-5.04 (2H, m), 5.96 (1H, s), 6.29 (1H, s), 6.56 (1H, d, J=15.9Hz), 7.64-7.51 (3H, m), 7.71 (1H, s), 7.83 (1H, d, J=15.9Hz), 7.94-7.88 (2H, m), 8.04 (1H, d, J=8.3Hz), 10.75 (1H, s), 18.81 (1H, s).

(72c) Methyl 3-{4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthyl}propanoate

The crude purified product of methyl (2E)-3-{4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthyl}acrylate produced in Example (72b) (~1.16 mmol) was dissolved in ethyl acetate (40 mL) and ethanol (10 mL). Palladium-carbon (50 mg) was added, and the mixture was stirred in a hydrogen atmosphere at room temperature for 3.5 hours. The insoluble matter was removed by filtration, and then the solvent was evaporated under reduced pressure. The resulting residue was purified by reverse phase column chromatography (elution solvent: acetonitrile/water = 3/1) to give the target compound (474 mg, yield: 72% in two steps) as a yellow solid.
$^1$H-NMR ($CDCl_3$, 400MHz):δ ppm: 1.75 (3H, s), 2.64 (3H, s), 2.73 (2H, t, J=7.8Hz), 3.10 (2H, t, J=7.8Hz), 3.67 (3H, s), 3.72 (3H, s), 5.11-4.99 (2H, m), 6.00 (1H, s), 6.27 (1H, s), 7.37 (1H, m), 7.52-7.47 (2H, m), 7.60 (1H, brs), 7.81 (1H, m), 8.04 (1H, m), 10.64 (1H, s), 18.79 (1H, s).
MS (ESI) m/z: 572.19466 $(M+H)^+$.

(Example 73) 3-{4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthyl}propanoic acid (exemplary compound No. 1-376)

Reaction and post-treatment were carried out in accordance with Example 68 using methyl 3-{4-[({[(9aS)-8-acetyl-1,7-dihydroxy-3-methoxy-9a-methyl-9-oxo-9,9a-dihydrodibenzo[b,d]furan-4-yl]carbonyl}amino)methyl]-2-naphthyl}propanoate produced in Example 72 (230 mg, 0.402 mmol) and a 1 N lithium hydroxide aqueous solution (24 mL) to give the target compound (232 mg, yield: ~100%) as a yellow solid.
$^1$H-NMR ($CDCl_3$, 500MHz):δ ppm: 1.72 (3H, s), 2.63 (3H, s), 2.76 (2H, m), 3.09 (2H, m), 3.72 (3H, s), 5.10-4.98 (2H, m), 5.96 (1H, s), 6.26 (1H, s), 7.35 (1H, m), 7.39 (1H, s), 7.53-7.45 (2H, m), 7.61 (1H, s), 7.81 (1H, brd, J=6.8Hz), 8.03 (1H, brd, J=6.8Hz), 10.65 (1H, s), 18.80 (1H, s).
MS (ESI) m/z: 558.17790 $(M+H)^+$.

(Example 74) (9aS)-8-Acetyl-N-(2-ethyl-3,5,6-trimethylbenzyl)-1,7-dihydroxy-3-methoxy-9a-methyl-9-oxo-9,9a-dihydrodibenzo[b,d]furan-4-carboxamide (exemplary compound No. 1-119)

(74a) 2-Bromo-3,5,6-trimethylbenzaldehyde

1-Bromo-2,4,5-trimethylbenzene (25.0 g, 0.125 mol) was dissolved in methylene chloride (500 mL). Aluminum chloride (20.1 g, 0.151 mol) and dichloromethyl methyl ether (13.7 mL, 0.151 mol) were added in a nitrogen atmosphere at -78˚C. After stirring at -78˚C for five minutes, water (500 mL) was added, followed by extraction with methylene chloride (400 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 25/1) to give the target compound (10.3 g, yield: 36%) as a white solid.
$^1$H-NMR ($CDCl_3$, 400MHz):δ ppm: 2.24 (3H, s), 2.38 (6H, s), 7.18 (1H, s), 10.47 (1H, s).
MS (EI) m/z: 226 $(M^+)$

(74b) 2,3,5-Trimethyl-6-vinylbenzaldehyde

2-Bromo-3,5,6-trimethylbenzaldehyde produced in Example (74a) (6.20 g, 27.3 mmol) was dissolved in toluene (180 mL). Tetrakistriphenylphosphine palladium (3.15 g, 2.73 mmol) and vinyltributyltin (8.37 mL, 28.7 mmol) were added, and the mixture was stirred with heating to reflux in a nitrogen atmosphere for four hours. The reaction solution was cooled to room temperature and then the solvent was evaporated under reduced pressure. The resulting residue was purified by silica gel column chromatography (elution solvent: hexane/ethyl acetate = 30/1 to 20/1) to give the target compound (3.47 g, yield: 73%) as a yellow solid.
$^1$H-NMR ($CDCl_3$, 400MHz):δ ppm: 2.27 (3H, s), 2.30 (3H, s), 2.42 (3H, s), 5.13 (1H, d, J=17.6Hz), 5.69 (1H, d, J=11.2Hz), 6.92 (1H, dd, J=11.2, 17.6Hz), 7.16 (1H, s). MS (EI) m/z: 174 $(M^+)$

(74c) 2-Ethyl-3,5,6-trimethylbenzaldehyde

2,3,5-Trimethyl-6-vinylbenzaldehyde produced in Example (74b) (3.47 g, 19.9 mmol) was dissolved in acetic acid (70 mL). 10% palladium-carbon (4.24 g) was added, and the mixture was stirred in a hydrogen (1 atm) atmosphere for 10 hours. Palladium-carbon was separated by filtration, and then the solvent was evaporated under reduced pressure. The resulting residue was purified by silica gel column chromatography (elution solvent: hexane/ethyl acetate = 30/1 to

20/1) to give the target compound (2.19 g, yield: 62%) as a white solid.
$^1$H-NMR ($CDCl_3$, 500MHz):δ ppm: 1.17 (3H, t, J=7.5Hz), 2.26 (3H, s), 2.30 (3H, s), 2.39 (3H, s), 2.85 (2H, q, J=7.5Hz), 7.13 (1H, s), 10.62 (1H, s).
MS (EI) m/z: 174 ($M^+$)

(74d) (9aS)-8-Acetyl-N-(2-ethyl-3,5,6-trimethylbenzyl)-1,7-dihydroxy-3-methoxy-9a-methyl-9-oxo-9,9a-dihydrodibenzo [b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2-ethyl-3,5,6-trimethylbenzaldehyde produced in Example (74c) (0.180 g, 1.02 mmol), triethylsilane (0.554 mL, 3.48 mmol), trifluoroacetic acid (0.268 mL, 3.48 mmol) and acetonitrile (15.0 mL) to give the target compound (0.176 g, yield: 40%) as a yellow solid.
$^1$H-NMR ($CDCl_3$, 400MHz):δ ppm: 1.15 (3H, t, J=7.5Hz), 1.75 (3H, s), 2.25 (3H, s), 2.27 (3H, s), 2.29 (3H, s), 2.64 (3H, s), 2.73 (2H, q, J=7.5Hz), 3.76 (3H, s), 4.60-4.69 (2H, m), 6.00 (1H, s), 6.26 (1H, s), 6.67 (1H, brs), 6.95 (1H, s), 10.60 (1H, s), 18.78 (1H, s).
MS (ESI) m/z: 506.2150 $(M+H)^+$

(Example 75) Methyl 3-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,4,5-trimethylbenzoate (exemplary compound No. 1-129)

(75a) 3-Bromo-2,5,6-trimethylbenzaldehyde

1-Bromo-2,4,5-trimethylbenzene (25.0 g, 0.125 mol) was dissolved in methylene chloride (500 mL). Aluminum chloride (20.1 g, 0.151 mol) and dichloromethyl methyl ether (13.7 mL, 0.151 mol) were added in a nitrogen atmosphere at -78˚C. After stirring at -78˚C for five minutes, water (500 mL) was added, followed by extraction with methylene chloride (400 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 25/1) to give the target compound (14.4 g, yield: 51%) as a white solid.
$^1$H-NMR ($CDCl_3$, 400MHz):δ ppm: 2.27 (3H, s), 2.36 (3H, s), 2.55 (3H, s), 7.51 (1H, s), 10.53 (1H, s).
MS (EI) m/z: 226 ($M^+$)

(75b) Methyl 3-formyl-2,4,5-trimethylbenzoate

3-Bromo-2,5,6-trimethylbenzaldehyde produced in Example (75a) (0.400 g, 1.76 mmol) was dissolved in N,N-dimethylformamide (10 mL). Palladium acetate (0.012 g, 0.0528 mmol), 1,1'-bisdiphenylphosphinoferrocene (0.059 g, 0.106 mmol), triethylamine (0.491 mL, 3.52 mmol) and methanol (1.43 mL, 35.2 mmol) were added, and the mixture was stirred in a carbon monoxide (1 atm) atmosphere at 50˚C for six hours. After cooling the reaction solution, an ammonium chloride aqueous solution (20 mL) was added, followed by extraction with ethyl acetate (30 mL). The organic layer was washed with brine and then dried over anhydrous sodium sulfate. The solvent was evaporated under reduced pressure, and the resulting residue was purified by silica gel column chromatography (elution solvent: hexane = 9/1) to give the target compound (0.255 g, yield: 70%) as a white solid.
$^1$H-NMR ($CDCl_3$, 400MHz):δ ppm: 2.32 (3H, s), 2.42 (3H, s), 2.66 (3H, s), 3.90 (3H, s), 7.73 (1H, s), 10.65 (1H, s).
MS (EI) m/z: 206 ($M^+$)

(75c) Methyl 3-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,4,5-trimethylbenzoate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), methyl 3-formyl-2,4,5-trimethylbenzoate produced in Example (75b) (0.250 g, 1.21 mmol), triethylsilane (0.738 mL, 4.63 mmol), trifluoroacetic acid (0.357 mL, 4.63 mmol) and acetonitrile (20.0 mL) to give the target compound (0.314 g, yield: 51%) as a yellow solid.
$^1$H-NMR ($CDCl_3$, 400MHz):δ ppm: 1.75 (3H, s), 2.30 (3H, s), 2.36 (3H, s), 2.57 (3H, s), 2.64 (3H, s), 3.78 (3H, s), 3.88 (3H, s), 4.64-4.74 (2H, m), 6.01 (1H, s), 6.27 (1H, s), 6.78 (1H, brs), 7.55 (1H, s), 10.64 (1H, s), 18.78 (1H, s).
MS (ESI) m/z: 536.1930 $(M+H)^+$.

(Example 76) 3-({[({9aS}-8-Acetyl-1,7-dihydroxy-3-methoxy-9a-methyl-9-oxo-9,9a-dihydrodibenzo[b,d]furan-4-yl)carbonyl]amino}methyl)-2,4,5-trimethylbenzoic acid (exemplary compound No. 1-130)

Methyl 3-({[({9aS}-8-acetyl-1,7-dihydroxy-3-methoxy-9a-methyl-9-oxo-9,9a-dihydrodibenzo[b,d]furan-4-yl) carbonyl]amino}methyl)-2,4,5-trimethylbenzoate produced in Example (75c) (0.183 g, 0.342 mmol) was dissolved in a 1 N sodium hydroxide aqueous solution (15 mL), and the mixture was stirred at room temperature for one hour. After completion of the reaction, the reaction solution was cooled to 0˚C, and a 1 N hydrochloric acid solution (20 mL) was slowly added dropwise. The precipitated product was filtered and dried to give the target compound (0.173 g, yield: 97%) as a yellow solid.
$^1$H-NMR (DMSO-$d_6$, 400MHz):δ ppm: 1.69 (3H, s), 2.25 (3H, s), 2.29 (3H, s), 2.48 (3H, s), 2.56 (3H, s), 3.72 (3H, s), 4.46 (2H, d, J=4.8Hz), 6.10 (1H, s), 6.33 (1H, s), 7.45 (1H, s), 8.27 (1H, brs), 10.23 (1H, s), 12.66 (1H, s).
MS (ESI) m/z: 522.1735 $(M+H)^+$

(Example 77) 4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthyl dimethylcarbamate (exemplary compound No. 1-349)

(77a) 4-Formyl-2-naphthyl dimethylcarbamate

Known 3-hydroxy-1-naphthaldehyde [Tetrahedron, 1999, Vol. 55, p.5821-5830] (300 mg, 1.74 mmol) was dissolved in pyridine (3 mL). Dimethylcarbamoyl chloride (750 μL, 8.15 mmol) was added, and the mixture was stirred at room temperature for 17 hours. A saturated ammonium chloride aqueous solution (10 mL) was added to the reaction solution, followed by extraction with diethyl ether (40 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 4/1) to give the target compound (378 mg, yield: 89%) as a light yellow oil.
$^1$H-NMR ($CDCl_3$, 400MHz):δ ppm: 3.06 (3H, s), 3.17 (3H, s), 7.55 (1H, m), 7.62(1H, m), 7.85-7.80 (3H, m), 9.15 (1H, d, J=8.6Hz), 10.34 (1H, s).
MS (EI) m/z: 243 $(M^+)$.

(77b) 4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthyl dimethylcarbamate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (350 mg, 1.01 mmol), 4-formyl-2-naphthyl dimethylcarbamate produced in Example (77a) (378 mg, 1.55 mmol), triethylsilane (490 μL, 3.03 mmol), trifluoroacetic acid (235 μL, 3.05 mmol) and acetonitrile (15 mL) to give the target compound (402 mg, yield: 69%) as a yellow solid.
$^1$H-NMR ($CDCl_3$, 400MHz):δ ppm: 1.74 (3H, s), 2.64 (3H, s), 3.03 (3H, s), 3.15 (3H, s), 3.75 (3H, s), 5.15-4.98 (2H, m), 6.00 (1H, s), 6.28 (1H, s), 7.41-7.29 (3H, m), 7.51 (2H, brs), 7.82 (1H, brs), 8.05 (1H, brs), 10.65 (1H, s), 18.82 (1H, s).
MS (ESI) m/z: 573.18452 $(M+H)^+$.

(Example 78) (9aS)-8-Acetyl-1,7-dihydroxy-3-methoxy-9a-methyl-N-{[3-(methoxymethyl)-1-naphthyl]methyl}-9-oxo-9,9a-dihydrodibenzo[b,d]furan-4-carboxamide (exemplary compound No. 1-289)

(78a) Methyl 4-(1,3-dioxan-2-yl)-2-naphthoate

Methyl 4-formyl-2-naphthoate produced in Example (67a) (450 mg, 2.10 mmol) was dissolved in toluene (20 mL), and 1,3-propanediol (760 μL, 10.5 mmol) and p-toluenesulfonic acid monohydrate (40 mg, 0.21 mmol) were added. A Dean-Stark water separator was attached, and the mixture was heated to reflux for four hours. Sodium bicarbonate water (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was directly used for the next reaction.

(78b) [4-(1,3-Dioxan-2-yl)-2-naphthyl]methanol

The crude purified product of methyl 4-(1,3-dioxan-2-yl)-2-naphthoate produced in Example (78a) (~2.10 mmol) was dissolved in diethyl ether (30 mL). Lithium aluminum hydride (200 mg, 5.27 mmol) was added at 0˚C, and the mixture was stirred with heating to room temperature for one hour. Water (200 μL) and a 1 M sodium hydroxide aqueous solution

(800 μL) were sequentially added to the reaction solution at 0°C, and the produced salt was removed by Celite. The solvent was evaporated under reduced pressure, and the resulting residue was directly used for the next reaction.

(78c) 2-[3-(Methoxymethyl)-1-naphthyl]-1,3-dioxane

Sodium hydride (125 mg, 2.86 mmol) was suspended in N,N-dimethylformamide (20 mL), and the crude purified product of [4-(1,3-dioxan-2-yl)-2-naphthyl]methanol produced in Example (78b) (~2.10 mmol) was added at 0°C. After stirring for 30 minutes, iodomethane (400 μL, 6.43 mmoL) was added, and the mixture was stirred at room temperature for one hour. A saturated ammonium chloride aqueous solution (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was directly used for the next reaction.

(78d) 3-(Methoxymethyl)-1-naphthaldehyde

The crude purified product of 2-[3-(methoxymethyl)-1-naphthyl]-1,3-dioxane produced in Example (78c) (~2.10 mmol) was dissolved in acetic acid (10 mL) and water (2 mL), and the mixture was stirred at 50°C for two hours. Water (10 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 5/1) to give the target compound (372 mg, yield: 89% in four steps).
$^1$H-NMR ($CDCl_3$, 400MHz):δ ppm: 3.48 (3H, s), 4.68 (2H, s), 7.57 (1H, m), 7.66 (1H, m), 7.89 (1H, brd, J=7.8Hz), 7.97 (1H, brd, J=1.6Hz), 8.02 (1H, brs), 9.19 (1H, d, J=9.0Hz), 10.38 (1H, s).
MS (EI) m/z: 200 ($M^+$).

(78e) (9aS)-8-Acetyl-1,7-dihydroxy-3-methoxy-9a-methyl-N- {[3-(methoxymethyl)-1-naphthyl]methyl}-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (350 mg, 1.01 mmol), 3-(methoxymethyl)-1-naphthaldehyde produced in Example (78d) (370 mg, 1.85 mmol), triethylsilane (490 μL, 3.03 mmol), trifluoroacetic acid (235 μL, 3.05 mmol) and acetonitrile (15 mL) to give the target compound (390 mg, yield: 73%) as a yellow solid.
$^1$H-NMR ($CDCl_3$, 400MHz):δ ppm: 1.73 (3H, s), 2.64 (3H, s), 3.44 (3H, s), 3.72 (3H, s), 4.60 (2H, s), 5.24-4.97 (2H, m), 5.99 (1H, s), 6.27 (1H, s), 7.64-7.44 (3H, m), 7.96-7.68 (2H, m), 8.08 (1H, brs), 10.71 (1H, s), 18.81 (1H, s).
MS (ESI) m/z: 530.17861 $(M+H)^+$.

(Example 79) (9aS)-8-Acetyl-1,7-dihydroxy-3-methoxy-9a-methyl-9-oxo-N-[(4-propoxy-1-naphthyl)methyl]-9,9a-dihydrodibenzo[b,d]furan-4-carboxamide (exemplary compound No. 1-275)

(79a) 4-Propoxy-1-naphthaldehyde

Sodium hydride (193 mg, 4.42 mmol) was suspended in a mixed solvent of tetrahydrofuran (20 mL)-N,N-dimethylformamide (5 mL) in a nitrogen atmosphere, and the suspension was cooled to 0°C. 4-Hydroxy-1-naphthaldehyde (502 mg, 2.92 mmol) was slowly added, and the mixture was stirred at 0°C for 30 minutes. 1-Iodopropane (560 μL, 5.73 mmol) was further added, and the mixture was stirred at room temperature for two hours. An ammonium chloride aqueous solution (30 mL) was added to the reaction solution, followed by extraction with ethyl acetate (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 10/1) to give the target compound (441 mg, yield: 70%) as a light yellow solid.
$^1$H-NMR ($CDCl_3$, 400MHz):δ ppm: 1.17 (3H, t, J=7.4Hz), 1.96-2.05 (2H, m), 4.22 (2H, t, J=6.5Hz), 6.91 (1H, d, J=8.2Hz), 7.58 (1H, m), 7.70 (1H, m), 7.92 (1H, d, J=7.8Hz), 8.37 (1H, d, J=8.6Hz), 9.31 (1H, d, J=8.6Hz), 10.20 (1H, s).
MS (EI) m/z: 214 ($M^+$)

(79b) (9aS)-8-Acetyl-1,7-dihydroxy-3-methoxy-9a-methyl-9-oxo-N-[(4-propoxy-1-naphthyl)methyl]-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (301 mg, 0.872 mmol), 4-propoxy-1-naphthaldehyde produced in Example (79a) (435 mg, 2.03 mmol), triethylsilane (420 μL, 2.64 mmol), trifluoroacetic acid (210 μL, 2.73 mmol) and acetonitrile (10 mL) to give the target compound (280 mg, yield: 59%) as a yellow solid.
$^1$H-NMR ($CDCl_3$, 500MHz):δ ppm: 1.14 (3H, t, J=7.5Hz), 1.74 (3H, s), 1.93-1.99 (2H, m), 2.64 (3H, s), 3.67 (3H, s), 4.10 (2H, t, J=6.4Hz), 4.95 (1H, dd, J=4.9, 14.2Hz), 5.01 (1H, dd, J=4.9, 14.2Hz), 5.99 (1H, s), 6.25 (1H,s), 6.75 (1H, d, J=7.8Hz), 7.17 (1H, brs), 7.42 (1H, d, J=7.8Hz), 7.51 (1H, m), 7.57 (1H, m), 8.03 (1H, d, J=8.3Hz), 8.37 (1H, d, J=8.3Hz), 10.63 (1H, s), 18.80 (1H, brs).
MS (ESI+) m/z: 544.19961 $(M+H)^+$

(Example 80) (9aS)-8-Acetyl-N-[3-(cyclopropylcarbonyl)-1-naphthyl]methyl-1,7-dihydroxy-3-methoxy-9a-methyl-9-oxo-9,9a-dihydrodibenzo[b,d]furan-4-carboxamide (exemplary compound No. 1-307)

(80a) 4-Formyl-2-naphthoic acid

A 1 N lithium hydroxide aqueous solution (24 mL) was added to methyl 4-formyl-2-naphthoate produced in Example (67a) (746 mg, 2.45 mmol), and the mixture was stirred for two hours. 1 N hydrochloric acid (50 mL) was added, followed by extraction with ethyl acetate (100 mL). The organic layer was washed with brine and then dried over anhydrous sodium sulfate. The solvent was evaporated under reduced pressure, and the resulting residue was directly used for the next reaction.

(80b) 4-Formyl-N-methoxy-N-methyl-2-naphthamide

The crude purified product of 4-formyl-2-naphthoic acid produced in Example (80a) (~4.05 mmol) was dissolved in N,N-dimethylformamide (40 mL), followed by addition of N,O-dimethylhydroxylamine hydrochloride (790 mg, 8.10 mmol), 1-(3-dimethylaminopropyl)-3-ethylcarbodiimide hydrochloride (1.60 g, 8.35 mmol), 1-hydroxybenzotriazole monohydrate (1.1 g, 8.14 mmol) and triethylamine (1.2 mL, 8.61 mmol). After stirring at room temperature for 18 hours, a saturated ammonium chloride aqueous solution (50 mL) was added to the reaction solution, followed by extraction with diethyl ether (100 mL). The organic layer was washed with brine and then dried over anhydrous magnesium sulfate, and the solvent was evaporated under reduced pressure. The resulting crude purified product was directly used for the next reaction.

(80c) 4-(1,3-Dioxan-2-yl)-N-methoxy-N-methyl-2-naphthamide

Reaction and post-treatment were carried out in accordance with Example (78a) using the crude purified product of 4-formyl-N-methoxy-N-methyl-2-naphthamide produced in Example (80b) (~4.05 mmol), 1,3-propanediol (1.5 mL, 20.8 mmol), p-toluenesulfonic acid monohydrate (80 mg, 0.42 mmol) and toluene (50 mL). The resulting residue was purified by silica gel column chromatography (elution solvent: hexane/ethyl acetate = 1/1) to give the target compound (1.13 g, yield: 92% in three steps) as a brown oil.
$^1$H-NMR ($CDCl_3$, 400MHz):δ ppm: 1.54 (1H, m), 2.36 (1H, m), 3.39 (3H, s), 3.56 (3H, s), 4.16-4.08 (2H, m), 4.38-4.33 (2H, m), 6.02 (1H, s), 7.49 (1H, m), 7.57 (1H, m), 7.88 (1H, d, J=8.0Hz), 8.00 (1H, m), 8.20 (1H, brs), 8.28 (1H, d, J=8.6Hz).
MS (EI) m/z: 301 $(M^+)$.

(80d) Cyclopropyl [4-(1,3-dioxan-2-yl)-2-naphthyl]methanone

4-(1,3-Dioxan-2-yl)-N-methoxy-N-methyl-2-naphthamide produced in Example (80c) (460 mg, 1.53 mmol) was dissolved in tetrahydrofuran (15 mL), and cyclopropylmagnesium bromide (0.5 M solution in tetrahydrofuran, 6.4 mL, 3.20 mmol) was added at 0˚C. After stirring for two hours, a saturated ammonium chloride aqueous solution (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 3/1) to give the target compound (414 mg, yield: 96%).
$^1$H-NMR ($CDCl_3$, 400MHz):δ ppm: 1.12-1.06 (2H, m), 1.32-1.27 (2H, m), 1.55 (1H, m), 2.38 (1H, m), 2.85 (1H, m), 4.17-4.08 (2H, m), 4.40-4.34 (2H, m), 6.06 (1H, s), 7.53 (1H, m), 7.62 (1H, m), 7.97 (1H, d, J=7.4Hz), 8.33-8.28 (2H, m), 8.52 (1H, s).

(80e) 3-(Cyclopropylcarbonyl)-1-naphthaldehyde

Reaction and post-treatment were carried out in accordance with Example (78d) using cyclopropyl [4-(1,3-dioxan-2-yl)-2-naphthyl]methanone produced in Example (80d) (414 mg, 1.47 mmol), acetic acid (10 mL) and water (2 mL) to give the target compound (268 mg, yield: 81%).
$^1$H-NMR ($CDCl_3$, 400MHz):δ ppm: 1.19-1.14 (2H, m), 1.38-1.33 (2H, m), 2.84 (1H, m), 7.62 (1H, m), 7.75 (1H, m), 8.00 (1H, brd, J=8.2Hz), 8.49 (1H, m), 8.70 (1H, s), 9.22 (1H, d, J=8.6Hz), 10.36 (1H, s).
MS (EI) m/z: 224 ($M^+$).

(80f) (9aS)-8-Acetyl-N-[3-(cyclopropylcarbonyl)-1-naphthyl]methyl-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (350 mg, 1.01 mmol), 3-(cyclopropylcarbonyl)-1-naphthaldehyde produced in Example (80e) (268 mg, 1.20 mmol), triethylsilane (490 μL, 3.03 mmol), trifluoroacetic acid (235 μL, 3.05 mmol) and acetonitrile (15 mL) to give the target compound (454 mg, yield: 81 %) as a yellow solid.
$^1$H-NMR ($CDCl_3$, 500MHz):δ ppm: 1.14-1.08 (2H, m), 1.33-1.27 (2H, m), 1.75 (3H, s), 2.65 (3H, s), 2.85 (1H, m), 3.80 (3H, s), 5.22-5.08 (2H, m), 6.02 (1H, s), 6.30 (1H, s), 7.37 (1H, brs), 7.60 (1H, m), 7.68 (1H, m), 8.03 (1H, brd, J=7.9Hz), 8.12 (1H, s), 8.16 (1H, brd, J=8.3Hz), 8.54 (1H, s), 10.67 (1H, s), 18.82 (1H, s).
MS (ESI) m/z: 554.17997 $(M+H)^+$.

(Example 81) (9aS)-8-Acetyl-N-[(4-ethoxy-1-naphthyl)methyl]-1,7-dihydroxy-3-methoxy-9a-methyl-9-oxo-9,9a-dihydrodibenzo[b,d]furan-4-carboxamide (exemplary compound No. 1-272)

(8 1 a) 4-Ethoxy-1-naphthaldehyde

Sodium hydride (194 mg, 4.45 mmol) was suspended in N,N-dimethylformamide (20 mL) in a nitrogen atmosphere, and the suspension was cooled to 0˚C. 4-Hydroxy-1-naphthaldehyde (517 mg, 3.00 mmol) was slowly added, and the mixture was stirred at 0˚C for 30 minutes. 1-Iodoethane (480 μL, 6.00 mmol) was further added, and the mixture was stirred at room temperature for two hours. A dilute hydrochloric acid solution (30 mL) was added to the reaction solution, followed by extraction with diethyl ether (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 10/1 to 7/1) to give the target compound (521 mg, yield: 87%) as a light green solid.
$^1$H-NMR ($CDCl_3$, 500MHz):δ ppm: 1.60 (3H, t, J=7.0Hz), 4.33 (2H, q, J=7.0Hz), 6.91 (1H, d, J=7.8Hz), 7.57 (1H, m), 7.70 (1H, m), 7.92 (1H, d, J=8.3Hz), 8.37 (1H, d, J=8.3Hz); 9.31 (1H, d, J=8.8Hz), 10.20 (1H, s).
MS (EI) m/z: 200 ($M^+$)

(81b) (9aS)-8-Acetyl-N-[(4-ethoxy-1-naphthyl)methyl]-1,7-dihydroxy-3-methoxy-9a-methyl-9-oxo-9,9a-dihydrodibenzo [b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (302 mg, 0.875 mmol), 4-ethoxy-1-naphthaldehyde produced in Example (81a) (518 mg, 2.59 mmol), triethylsilane (420 μL, 2.64 mmol), trifluoroacetic acid (210 μL, 2.73 mmol) and acetonitrile (10 mL) to give the target compound (283 mg, yield: 61%) as a yellow solid.
$^1$H-NMR ($CDCl_3$, 500MHz):δ ppm: 1.56 (3H, t, J=6.8Hz), 1.74 (3H, s), 2.64 (3H, s), 3.67 (3H, s), 4.22 (2H, q, J=6.8Hz), 4.95 (1H, dd, J=4.9, 14.2Hz), 5.01 (1H, dd, J=4.9, 14.2Hz), 6.00 (1H, s), 6.25 (1H,s), 6.75 (1H, d, J=7.8Hz), 7.16 (1H, brs), 7.42 (1H, d, J=7.8Hz), 7.51 (1H, m), 7.57 (1H, m), 8.04 (1H, d, J=8.3Hz), 8.37 (1H, d, J=8.3Hz), 10.64 (1H, s), 18.82 (1H, s).
MS (ESI+) m/z: 530.17999 $(M+H)^+$

(Example 82) (9aS)-8-Acetyl-N-[(4-butoxy-1-naphthyl)methyl]-1,7-dihydroxy-3-methoxy-9a-methyl-9-oxo-9,9a-dihydrodibenzo[b,d]furan-4-carboxamide (exemplary compound No. 1-281)

(82a) 4-Butoxy-1-naphthaldehyde

Sodium hydride (194 mg, 4.45 mmol) was suspended in N,N-dimethylformamide (20 mL) in a nitrogen atmosphere, and the suspension was cooled to 0˚C. 4-Hydroxy-1-naphthaldehyde (509 mg, 2.96 mmol) was slowly added, and the mixture was stirred at 0˚C for 30 minutes. 1-Iodobutane (1.01 mL, 8.89 mmol) was further added, and the mixture was stirred at room temperature for three hours. A dilute hydrochloric acid solution (30 mL) was added to the reaction solution, followed by extraction with diethyl ether (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 12/1 to 10/1) to give the target compound (588 mg, yield: 87%) as a colorless solid.
$^1$H-NMR ($CDCl_3$, 500MHz):δ ppm: 1.05 (3H, t, J=7.3Hz), 1.59-1.66 (2H, m), 1.93-1.99 (2H, m), 4.26 (2H, t, J=6.4Hz), 6.92 (1H, d, J=7.8Hz), 7.57 (1H, m), 7.70 (1H, m), 7.92 (1H, d, J=7.8Hz), 8.36 (1H, d, J=8.3Hz), 9.31 (1H, d, J=8.3Hz), 10.20 (1H, s).
MS (EI) m/z: 228 ($M^+$)

(82b) (9aS)-8-Acetyl-N-[(4-butoxy-1-naphthyl)methyl]-1,7-dihydroxy-3-methoxy-9a-methyl-9-oxo-9,9a-dihydrodibenzo [b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (304 mg, 0.880 mmol), 4-butoxy-1-naphthaldehyde produced in Example (82a) (585 mg, 2.56 mmol), triethylsilane (420 μL, 2.64 mmol), trifluoroacetic acid (210 μL, 2.73 mmol) and acetonitrile (10 mL) to give the target compound (264 mg, yield: 54%) as a yellow solid.
$^1$H-NMR ($CDCl_3$, 500MHz):δ ppm: 1.03 (3H, t, J=7.6Hz), 1.59-1.65 (2H, m), 1.74 (3H, s), 1.89-1.95 (2H, m), 2.64 (3H, s), 3.67 (3H, s), 4.15 (2H, t, J=6.4Hz), 4.95 (1H, dd, J=4.9, 14.7Hz), 5.01 (1H, dd, J=4.9, 14.7Hz), 6.00 (1H, s), 6.25 (1H,s), 6.75 (1H, d, J=7.8Hz), 7.16 (1H, brs), 7.42 (1H, d, J=7.8Hz), 7.51 (1H, m), 7.57 (1H, m), 8.04 (1H, d, J=8.3Hz), 8.36 (1H, d, J=8.3Hz), 10.63 (1H, s), 18.82 (1H, s).
MS (ESI+) m/z: 558.21510 $(M+H)^+$

(Example 83) (9aS)-8-[(1E)-N-Benzylethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (exemplary compound No. 3-108)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (310 mg, 0.62 mmol) was dissolved in methylene chloride (5 mL). Benzylamine (0.10 mL, 0.93 mmol) was added at room temperature, and the mixture was stirred for 12 hours. The reaction solution was separated with methylene chloride (15 mL) and 0.5 N hydrochloric acid (15 mL). The organic layer was washed with brine (15 mL) and dried over anhydrous sodium sulfate. The solvent was evaporated under reduced pressure, and the residue was purified by silica gel column chromatography (methylene chloride:methanol = 80:1, V/V) and reverse phase silica gel column chromatography (acetonitrile:water = 2:1, V/V) to give the title target compound (343 mg, 94%) as a colorless solid.
$^1$H-NMR ($CDCl_3$, 400MHz): δ ppm: 1.69 (3H, s), 2.62 (3H, s), 2.64 (3H, s), 3.60 (3H, s), 4.66 (2H, d, J=5.6Hz), 5.01-5.13 (2H, m), 5.81 (1H, s), 6.14 (1H, s), 6.95 (1H, m), 7.27-7.54 (9H, m), 7.72 (1H, d, J=8.7Hz), 7.81 (1H, d, J=7.9Hz), 8.11 (1H, d, J=7.9Hz), 11.44 (1H, s), 13.84 (1H, s)
MS (ESI) m/z: 589 $(M+H)^+$

(Example 84) (9aS)-1,7-Dihydroxy-3-methoxy-9a-methyl-8-[(1E)-N-methylethaneimidoyl]-N-[(2-methyl-1-naphthyl)methyl]-9-oxo-9,9a-dihydrodibenzo[b,d]furan-4-carboxamide (exemplary compound No. 3-104)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (233 mg, 0.47 mmol) was dissolved in methylene chloride (4 mL). Methylamine (2 M solution in tetrahydrofuran) (0.47 mL, 0.94 mmol) was added at room temperature, and the mixture was stirred for 12 hours. The reaction solution was separated with methylene chloride (15 mL) and 0.5 N hydrochloric acid (15 mL). The organic layer was washed with brine (15 mL) and dried over anhydrous sodium sulfate. The solvent was evaporated under reduced pressure, and the residue was purified by silica gel column chromatography (methylene chloride:methanol = 80:1, V/V) and reverse phase silica gel column chromatography (acetonitrile:water =

2: 1, V/V) to give the title target compound (183 mg, 77%) as a colorless solid.
$^1$H-NMR ($CDCl_3$, 400MHz):δ ppm: 1.68 (3H, s), 2.62 (3H, s), 2.63 (3H, s), 3.15 (3H, d, J=4.8Hz), 3.61 (3H, s), 5.02-5.14 (2H, m), 5.80 (1H, s), 6.16 (1H, s), 6.96 (1H, m), 7.35 (1H, d, J=8.4Hz), 7.45 (1H, m), 7.55 (1H, m), 7.74 (1H, d, J=8.7Hz), 7.83 (1H, d, J=7.9Hz), 8.13 (1H, d, J=8.7Hz), 11.50 (1H, brs), 13.41 (1H, brs)
MS (ESI) m/z: 513 $(M+H)^+$

(Example 85) (9aS)-8-Acetyl-N-({3-[(dimethylamino)carbonyl]-1-naphthyl}methyl)-1,7-dihydroxy-3-methoxy-9a-methyl-9-oxo-9,9a-dihydrodibenzo[b,d]furan-4-carboxamide (exemplary compound No. 1-331)

4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-2-naphthoic acid produced in Example 68 (255 mg, 0.482 mmol) was dissolved in N,N-dimethylformamide (10 mL), followed by addition of dimethylamine hydrochloride (100 mg, 1.48 mmol), 1-(3-dimethylaminopropyl)-3-ethylcarbodiimide hydrochloride (185 mg, 0.965 mmol), 1-hydroxybenzotriazole monohydrate (130 mg, 0.962 mmol) and triethylamine (135 μL, 0.969 mmol). After stirring at room temperature for 16 hours, a saturated ammonium chloride aqueous solution (20 mL) was added to the reaction solution, followed by extraction with ethyl acetate (50 mL). The organic layer was washed with brine and then dried over anhydrous magnesium sulfate, and the solvent was evaporated under reduced pressure. The resulting residue was dissolved in tetrahydrofuran (5 mL). A 1N sodium hydroxide aqueous solution (15 mL) was added, and the mixture was stirred for 24 hours. Then, 1N hydrochloric acid (10 mL) was added to the reaction solution, followed by extraction with ethyl acetate (50 mL). The organic layer was washed with brine and then dried over anhydrous magnesium sulfate, and the solvent was evaporated under reduced pressure. The resulting residue was purified by silica gel column chromatography (elution solvent: methylene chloride/methanol = 30/1) to give the target compound (179 mg, yield: 69%) as a yellow solid.
$^1$H-NMR ($CDCl_3$, 400MHz):δ ppm: 1.74 (3H, s), 2.65 (3H, s), 3.05 (3H, s), 3.06 (3H, s), 3.79 (3H, s), 5.17-5.05 (2H, m), 5.99 (1H, s), 6.29 (1H,s), 7.42 (1H, m), 7.56 (1H, m), 7.63 (1H, m), 7.95-7.91 (2H, m), 8.11 (1H, brd, J=8.3Hz), 8.19 (1H, s), 10.67 (1H, s), 18.82 (1H, s).
MS (ESI) m/z: 557.19015 $(M+H)^+$.

(Example 86) (9aS)-8-Acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide (exemplary compound No. 2-73)

(86a) (9aS)-8-Acetyl-3-(difluoromethoxy)-1,7-dihydroxy-9a-methyl-9-oxo-9,9a-dihydrodibenzo[b,d]furan-4-carboxamide

Cercosporamide obtained in Reference Example 3 (2.55 g, 7.70 mmol) was suspended in a mixed solvent of a 15M potassium hydroxide aqueous solution (2.1 mL)-methylene chloride (8.2 mL). Tetrabutylammonium bromide (911 mg, 2.83 mmol) was added, and the mixture was stirred in a nitrogen atmosphere at 0°C for 30 minutes. After quietly bubbling with chlorodifluoromethane for 45 minutes, the mixture was stirred at room temperature for seven hours. 2 N hydrochloric acid (40 mL) was added, followed by extraction with ethyl acetate (200 mL). The organic layer was washed with brine and then dried over anhydrous sodium sulfate. The solvent was evaporated under reduced pressure, and the resulting residue was purified by reverse phase column chromatography (elution solvent: acetonitrile/water = 4/6 to 7/3) to give the target compound (592 mg, yield: 20%) as a yellow solid.
$^1$H-NMR ($CDCl_3$, 400MHz):δ ppm: 1.80 (3H, s), 2.67 (3H, s), 5.86 (1H, brs), 6.04 (1H, s), 6.38 (1H, brs), 6.58 (1H, s), 6.60 (1H, t, J=73.0Hz), 10.89 (1H, s), 18.86 (1H, s).
MS (ESI) m/z: 382.07263 $(M+H)^+$

(86b) (9aS)-8-Acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-3-(difluoromethoxy)-1,7-dihydroxy-9a-methyl-9-oxo-9,9a-dihydrodibenzo[b,d]furan-4-carboxamide produced in Example (86a) (326 mg, 0.855 mmol), 2-methyl-1-naphthaldehyde (296 mg, 1.74 mmol), triethylsilane (410 μL, 2.57 mmol), trifluoroacetic acid (200 μL, 2.60 mmol) and acetonitrile (10 mL) to give the target compound (193 mg, yield: 42%) as a yellow solid.
$^1$H-NMR ($CDCl_3$, 400MHz):δ ppm: 1.75 (3H, s), 2.63 (3H, s), 2.64 (3H, s), 3.62 (3H, s), 5.11 (2H, d, J=4.4Hz), 5.87 (1H, s), 6.19 (1H, brs), 6.41 (1H, t, J=73.0Hz), 6.51 (1H, s), 7.36 (1H, d, J=8.7Hz), 7.47 (1H, m), 7.56 (1H, m), 7.76 (1H, d, J=8.3Hz), 7.85 (1H, d, J=7.9Hz), 8.09 (1H, d, J=8.7Hz), 10.69 (1H, s), 18.83 (1H, s).
MS (ESI+) m/z: 536.14961 $(M+H)^+$

(Example 87) (9aS)-1,7-Dihydroxy-3-methoxy-8-[(1E)-N-methoxyethaneimidoyl]-9a-methyl-N-[(2-methyl-1-naphthyl) methyl]-9-oxo-9,9a-dihydrodibenzo[b,d]furan-4-carboxamide (exemplary compound No. 3-18)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (221 mg, 0.44 mmol) was dissolved in tetrahydrofuran:methanol (2:1, 5 mL). O-Methylhydroxylamine hydrochloride (55 mg, 0.66 mmol) and sodium bicarbonate (56 mg, 0.66 mmol) were added at room temperature, and the mixture was stirred for four hours. The reaction solution was separated with ethyl acetate (15 mL) and 0.5 N hydrochloric acid (15 mL). The organic layer was washed with brine (15 mL) and dried over anhydrous sodium sulfate. The solvent was evaporated under reduced pressure, and the residue was purified by silica gel column chromatography (methylene chloride:methanol= 80:1, V/V) and reverse phase silica gel column chromatography (acetonitrile:water = 2:1, V/V) to give the title target compound (210 mg, 90%) as a yellow solid.
$^1$H-NMR ($CDCl_3$, 400MHz):δ ppm: 1.71 (3H, s), 2.54 (3H, s), 2.63 (3H, s), 3.61 (3H, s), 3.93 (3H, s), 5.02-5.14 (2H, m), 5.88 (1H, s), 6.17 (1H, s), 7.02 (1H, m), 7.35 (1H, d, J=8.4Hz), 7.45 (1H, m), 7.55 (1H, m), 7.74 (1H, d, J=8.7Hz), 7.84 (1H, d, J=7.5Hz), 8.13 (1H, d, J=8.4Hz), 11.21 (1H, s), 15.86 (1H, brs)
MS (ESI) m/z: 529 $(M+H)^+$.

(Example 88) (9aS)-8-Acetyl-7-hydroxy-1,3-dimethoxy-9a-methyl-N-[(2-methyl-1-naphthyl)methyl]-9-oxo-9,9a-dihydrodibenzo[b,d]furan-4-carboxamide (exemplary compound No. 2-83)

(88a) (9aS)-8-Acetyl-1,7-dihydroxy-3-methoxy-9a-methyl-9-oxo-9,9a-dihydrodibenzo[b,d]furan-4-carboxylic acid

(9aS)-8-Acetyl-1,7-dihydroxy-3-methoxy-9a-methyl-9-oxo-9,9a-dihydrodibenzo[b,d]furan-4-carboxamide produced in Example (1a) (10.2 g, 29.5 mmol) was suspended in acetonitrile (120 ml) and 6 N sulfuric acid (100 mL). Sodium nitrite (10.2 g, 147 mmol) was added, and the mixture was stirred for four hours. The reaction solution was separated with ethyl acetate (300 mL) and water (100 mL). The organic layer was washed with brine (200 mL) and dried over anhydrous sodium sulfate. The solvent was evaporated under reduced pressure, and the resulting brown solid was washed with diethyl ether to give the title target compound (9.41 g, 92%) as a light brown solid.
$^1$H-NMR ($CDCl_3$, 400MHz):δ ppm: 1.77 (3H, s), 2.66 (3H, s), 4.07 (3H, s), 6.10 (1H, s), 6.41 (1H, s), 11.14 (1H, s), 18.86 (1H, s)
MS (FAB) m/z: 347 $(M+H)^+$

(88b) Benzyl (9aS)-8-acetyl-1,7-dihydroxy-3-methoxy-9a-methyl-9-oxo-9,9a-dihydrodibenzo[b,d]furan-4-carboxylate

(9aS)-8-Acetyl-1,7-dihydroxy-3-methoxy-9a-methyl-9-oxo-9,9a-dihydrodibenzo[b,d]furan-4-carboxylic acid produced in Example (88a) (10.0 g, 28.9 mmol) was dissolved in methylene chloride (150 mL). Benzyl alcohol (4.5 mL, 43.3 mmol), 1-ethyl-3-(3-dimethylaminopropyl)carbodiimide hydrochloride (6.09 g, 31.8 mmol) and 4-dimethylaminopyridine (3.88 g, 31.8 mmol) were added at 0˚C, and then the mixture was stirred in a nitrogen atmosphere at room temperature for four hours. The reaction solution was separated with 0.5 N hydrochloric acid (150 mL) under ice-cooling. The organic layer was washed with 0.5 N hydrochloric acid (150 mL) and water (150 mL) and dried over anhydrous sodium sulfate. The solvent was evaporated under reduced pressure, and the residue was purified by silica gel column chromatography (methylene chloride:methanol = 80:1, V/V) to give the title target compound (9.18 g, 73%) as a light yellow solid.
$^1$H-NMR ($CDCl_3$, 400MHz):δ ppm: 1.74 (3H, s), 2.64 (3H, s), 3.85 (3H, s), 5.36 (2H, s), 5.90 (1H, s), 6.30 (1H, s), 7.24-7.46 (5H, m), 10.57 (1H, s), 18.78 (1H, s)
MS (FAB) m/z: 437 $(M+H)^+$

(88c) Benzyl (9aS)-8-acetyl-7-hydroxy-1,3-dimethoxy-9a-methyl-9-oxo-9,9a-dihydrodibenzo[b,d] furan-4-carboxylate

Benzyl (9aS)-8-acetyl-1,7-dihydroxy-3-methoxy-9a-methyl-9-oxo-9,9a-dihydrodibenzo[b,d]furan-4-carboxylate produced in Example (88b) (1.47 g, 3.37 mmol) was dissolved in N,N-dimethylfomlamide (15 mL). Sodium hydride (202 mg, 8.42 mmol) was added at 0˚C, and the mixture was stirred in a nitrogen atmosphere for 30 minutes. Then, methyl iodide (0.52 mL, 8.42 mmol) was added, and the mixture was stirred for three hours. 0.2 N hydrochloric acid (40 mL) was added to the reaction solution under ice-cooling, followed by extraction with ethyl acetate. The organic layer was washed with brine (40 mL) and dried over anhydrous sodium sulfate. The solvent was evaporated under reduced pressure, and the residue was purified by silica gel column chromatography (methylene chloride:ethyl acetate = 10:1 to 5:1, V/V) to give the title target compound (1.49 g, 98%) as a light yellow solid.
$^1$H-NMR ($CDCl_3$, 400MHz):δ ppm: 1.79 (3H, s), 2.53 (3H, s), 3.90 (3H, s), 3.98 (3H, s), 5.36 (2H, s), 5.79 (1H, s), 6.26 (1H, s), 7.30-7.44 (5H, m), 18.08 (1H, s)

MS (FAB) m/z: 451 $(M+H)^+$

(88d) (9aS)-8-Acetyl-7-hydroxy-1,3-dimethoxy-9a-methyl-9-oxo-9,9a-dihydrodibenzo[b,d]furan-4-carboxylic acid

Benzyl (9aS)-8-acetyl-7-hydroxy-1,3-dimethoxy-9a-methyl-9-oxo-9,9a-dihydrodibenzo[b,d]furan-4-carboxylate produced in Example (88c) (895 mg, 1.99 mmol) was dissolved in ethyl acetate (20 mL). 10% Pd/C (180 mg) was added, and the mixture was stirred in a hydrogen atmosphere for 30 minutes. The reaction solution was filtered through Celite, and the solvent was evaporated under reduced pressure. The resulting crude crystals were washed with diethyl ether to give the title target compound (509 mg, 71 %) as a light yellow solid.
$^1$H-NMR ($CDCl_3$, 400MHz):δ ppm: 1.81 (3H, s), 2.53 (3H, s), 4.03 (3H, s), 4.07 (3H, s), 5.94 (1H, s), 6.34 (1H, s), 18.06 (1H, s)
MS (FAB) m/z: 361 $(M+H)^+$

(88e) (9aS)-8-Acetyl-7-hydroxy-1,3-dimethoxy-9a-methyl-9-oxo-9,9a-dihydrodibenzo[b,d]furan-4-carboxamide

(9aS)-8-Acetyl-7-hydroxy-1,3-dimethoxy-9a-methyl-9-oxo-9,9a-dihydrodibenzo[b,d]furan-4-carboxylic acid produced in Example (88d) (194 mg, 0.54 mmol) was dissolved in N,N-dimethylformamide (4 mL). 1-Hydroxybenzotriazole (87 mg, 0.65 mmol) and 1-ethyl-3-(3-dimethylaminopropyl)carbodiimide hydrochloride (124 mg, 0.65 mmol) were added at room temperature, and the mixture was stirred in a nitrogen atmosphere for 30 minutes. Then, a 2 M solution of ammonia in isopropanol (0.8 mL) was added, and the mixture was stirred for one hour. The reaction solution was separated with ethyl acetate (15 mL) and 0.5 N hydrochloric acid (10 mL). The organic layer was washed with brine (10 mL) and dried over anhydrous sodium sulfate. The solvent was evaporated under reduced pressure, and the residue was purified by silica gel column chromatography (methylene chloride:methanol = 80:1 to 50:1, V/V) to give the title target compound (175 mg, 90%) as a light yellow solid.
$^1$H-NMR ($CDCl_3$, 400MHz):δ ppm: 1.81 (3H, s), 2.53 (3H, s), 3.95 (3H, s), 4.00 (3H, s), 5.79 (1H, brs), 5.91 (1H, s), 6.30 (1H, s), 6.89 (1H, brs), 18.08 (1H, s)
MS (FAB) m/z: 360 $(M+H)^+$

(88f) (9aS)-8-Acetyl-7-hydroxy-1,3-dimethoxy-9a-methyl-N-[(2-methyl-1-naphthyl)methyl]-9-oxo-9,9a-dihydrodibenzo [b,d]furan-4-carboxamid

Reaction and post-treatment were carried out in accordance with Example 10 using (9aS)-8-acetyl-7-hydroxy-1,3-dimethoxy-9a-methyl-9-oxo-9,9a-dihydrodibenzo[b,d]furan-4-carboxamide produced in Example (88e) (175 mg, 0.49 mmol), 2-methyl-1-naphthaldehyde (249 mg, 1.46 mmol), triethylsilane (0.24 mL, 1.46 mmol), trifluoroacetic acid (0.11 mL, 1.46 mmol) and acetonitrile (7 mL) to give the target compound (130 mg, yield: 52%) as a yellow solid.
$^1$H-NMR ($CDCl_3$, 400MHz):δ ppm: 1.78 (3H, s), 2.51 (3H, s), 2.62 (3H, s), 3.68 (3H, s), 3.93 (3H, s), 5.02-5.12 (2H, m), 5.83 (1H, s), 6.17 (1H,s), 6.70 (1H, brs), 7.35-7.55 (3H, m), 7.73 (1H, d, J=8.3Hz), 7.82 (1H, d, J=8.3Hz), 8.10 (1H, d, J=8.3Hz), 18.08 (1H, s).
MS (ESI) m/z: 514 $(M+H)^+$.

(Example 89) (9aS)-8-Acetyl-1-ethoxy-7-hydroxy-3-methoxy-9a-methyl-N-[(2-methyl-1-naphthyl)methyl]-9-oxo-9,9a-dihydrodibenzo[b,d]furan-4-carboxamide (exemplary compound No. 2-88)

(89a) Benzyl (9aS)-8-acetyl-1-ethoxy-7-hydroxy-3-methoxy-9a-methyl-9-oxo-9,9a-dihydrodibenzo[b,d]furan-4-carboxylate

Benzyl (9aS)-8-acetyl-1,7-dihydroxy-3-methoxy-9a-methyl-9-oxo-9,9a-dihydrodibenzo[b,d]furan-4-carboxylate produced in Example (88b) (502 mg, 1.45 mmol) was dissolved in N,N-dimethylformamide (5 mL). Sodium hydride (52 mg, 2.18 mmol) was added at 0˚C, and the mixture was stirred in a nitrogen atmosphere for 30 minutes. Then, ethyl iodide (0.17 mL, 2.18 mmol) was added, and the mixture was stirred for three hours. 0.2 N hydrochloric acid (15 mL) was added to the reaction solution under ice-cooling, followed by extraction with ethyl acetate (15 mL). The organic layer was washed with brine (15 mL) and dried over anhydrous sodium sulfate. The solvent was evaporated under reduced pressure, and the residue was purified by silica gel column chromatography (methylene chloride:methanol = 80:1, V/V) to give the title target compound (564 mg, 84%) as a light yellow oil.
$^1$H-NMR ($CDCl_3$, 400MHz):δ ppm: 1.50 (3H, t, J=7.2Hz), 1.81 (3H, s), 2.51 (3H, s), 3.89 (3H, s), 4.12-4.32 (2H, m), 5.37 (2H, s), 5.80 (1H, s), 6.26 (1H, s), 7.33-7.48 (5H, m), 18.03 (1H, s)
MS (FAB) m/z: 465 $(M+H)^+$

(89b) (9aS)-8-Acetyl-1-ethoxy-7-hydroxy-3-methoxy-9a-methyl-9-oxo-9,9a-dihydrodibenzo[b,d]furan-4-carboxylic acid

Benzyl (9aS)-8-acetyl-1-ethoxy-7-hydroxy-3-methoxy-9a-methyl-9-oxo-9,9a-dihydrodibenzo[b,d]furan-4-carboxylate produced in Example (89a) (564 mg, 1.21 mmol) was dissolved in ethyl acetate (10 mL). 10% Pd/C (110 mg) was added, and the mixture was stirred in a hydrogen atmosphere for 30 minutes. The reaction solution was filtered through Celite, and the solvent was evaporated under reduced pressure. The filtrate was purified by silica gel column chromatography (methylene chloride:methanol = 40:1 to 10:1, V/V) to give the title target compound (449 mg, 99%) as a brown solid.
$^1$H-NMR ($CDCl_3$, 400MHz):δ ppm: 1.53 (3H, t, J=7.1Hz), 1.82 (3H, s), 2.50 (3H, s), 4.07 (3H, s), 4.18-4.33 (2H, m), 5.96 (1H, s), 6.32 (1H, s), 17.97 (1H, s)
MS (FAB) m/z: 375 $(M+H)^+$

(89c) (9aS)-8-Acetyl-1-ethoxy-7-hydroxy-3-methoxy-9a-methyl-9-oxo-9,9a-dihydrodibenzo[b,d]furan-4-carboxamide

(9aS)-8-Acetyl-1-ethoxy-7-hydroxy-3-methoxy-9a-methyl-9-oxo-9,9a-dihydrodibenzo[b,d]furan-4-carboxylic acid produced in Example (89b) (162 mg, 0.43 mmol) was dissolved in N,N-dimethylformamide (4 mL). 1-Hydroxybenzotriazole (70 mg, 0.52 mmol) and 1-ethyl-3-(3-dimethylaminopropyl)carbodiimide hydrochloride (100 mg, 0.52 mmol) were added at room temperature, and the mixture was stirred in a nitrogen atmosphere for 30 minutes. Then, a 2 M solution of ammonia in isopropanol (0.65 mL) was added, and the mixture was stirred for one hour. The reaction solution was separated with ethyl acetate (15 mL) and 0.5 N hydrochloric acid (10 mL). The organic layer was washed with brine (15 mL) and dried over anhydrous sodium sulfate. The solvent was evaporated under reduced pressure, and the residue was purified by silica gel column chromatography (methylene chloride:methanol = 80:1 to 50:1, V/V) to give the title target compound (160 mg, 99%) as a yellow solid.
$^1$H-NMR ($CDCl_3$, 400MHz):δ ppm: 1.50 (3H, t, J=7.2Hz), 1.81 (3H, s), 2.50 (3H, s), 3.94 (3H, s), 4.13-4.32 (2H, m), 5.88 (1H, s), 6.28 (1H, s), 6.33 (1H, brs), 6.96 (1H, brs), 17.98 (1H, s)
MS (FAB) m/z: 374 $(M+H)^+$

(89d) (9aS)-8-Acetyl-1-ethoxy-7-hydroxy-3-methoxy-9a-methyl-N-[(2-methyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-1-ethoxy-7-hydroxy-3-methoxy-9a-methyl-9-oxo-9,9a-dihydrodibenzo[b,d]furan-4-carboxamide produced in Example (89c) (160 mg, 0.43 mmol), 2-methyl-1-naphthaldehyde (219 mg, 1.29 mmol), triethylsilane (0.21 mL, 1.29 mmol), trifluoroacetic acid (0.10 mL, 1.29 mmol) and acetonitrile (8 mL) to give the target compound (121 mg, yield: 54%) as a yellow solid.
$^1$H-NMR ($CDCl_3$, 400MHz):δ ppm: 1.46 (3H, t, J=7.1Hz), 1.79 (3H, s), 2.49 (3H, s), 2.62 (3H, s), 3.66 (3H, s), 4.08-4.25 (2H, m), 5.02-5.14 (2H, m), 5.83 (1H, s), 6.17 (1H,s), 6.76 (1H, brs), 7.34-7.56 (3H, m), 7.74 (1H, d, J=8.3Hz), 7.84 (1H, d, J=8.3Hz), 8.12 (1H, d, J=8.3Hz), 18.03 (1H, s).
MS (ESI) m/z: 528 $(M+H)^+$

(Example 90) (9aS)-8-Acetyl-1-propyloxy-7-hydroxy-3-methoxy-9a-methyl-N-[(2-methyl-1-naphthyl)methyl]-9-oxo-9,9a-dihydrodibenzo[b,d]furan-4-carboxamide (exemplary compound No. 2-98)

(90a) Benzyl (9aS)-8-acetyl-1-propyloxy-7-hydroxy-3-methoxy-9a-methyl-9-oxo-9,9a-dihydrodibenzo[b,d]furan-4-carboxylate

Benzyl (9aS)-8-acetyl-1,7-dihydroxy-3-methoxy-9a-methyl-9-oxo-9,9a-dihydrodibenzo[b,d]furan-4-carboxylate produced in Example (88b) (509 mg, 1.47 mmol) was dissolved in N,N-dimethylformamide (5 mL). Sodium hydride (71 mg, 2.94 mmol) was added at 0˚C, and the mixture was stirred in a nitrogen atmosphere for 30 minutes. Then, propyl iodide (0.29 mL, 2.94 mmol) was added, and the mixture was stirred for three hours. 0.2 N hydrochloric acid (15 mL) was added to the reaction solution under ice-cooling, followed by extraction with ethyl acetate (15 mL). The organic layer was washed with brine (15 mL) and dried over anhydrous sodium sulfate. The solvent was evaporated under reduced pressure, and the residue was purified by silica gel column chromatography (methylene chloride:methanol = 80:1, V/V) to give the title target compound (575 mg, 82%) as a brown oil.
$^1$H-NMR ($CDCl_3$, 400MHz):δ ppm: 1.12 (3H, t, J=7.1Hz), 1.81 (3H, s), 1.87-1.93 (2H, m), 2.50 (3H, s), 3.89 (3H, s), 4.02 (1H, m), 4.16(1H, m), 5.37 (2H, s), 5.79 (1H, s), 6.25 (1H, s), 7.33-7.48 (5H, m), 18.00 (1H, s)
MS (FAB) m/z: 479 $(M+H)^+$

(90b) (9aS)-8-Acetyl-1-propyloxy-7-hydroxy-3-methoxy-9a-methyl-9-oxo-9,9a-dihydrodibenzo[b,d]furan-4-carboxylic acid

Benzyl (9aS)-8-acetyl-1-propyloxy-7-hydroxy-3-methoxy-9a-methyl-9-oxo-9,9a-dihydrodibenzo[b,d]furan-4-carboxylate produced in Example (90a) (493 mg, 1.03 mmol) was dissolved in ethyl acetate (10 mL). 10% Pd/C (100 mg) was added, and the mixture was stirred in a hydrogen atmosphere for 30 minutes. The reaction solution was filtered through Celite, and the solvent was evaporated under reduced pressure. The filtrate was purified by silica gel column chromatography (methylene chloride:methanol = 40:1 to 10:1, V/V) to give the title target compound (392 mg, 98%) as a brown solid.
$^1$H-NMR ($CDCl_3$, 400MHz):$\delta$ ppm: 1.14 (3H, t, J=7.1Hz), 1.82 (3H, s), 1.88-1.96 (2H, m), 2.49 (3H, s), 4.08 (3H, s), 4.05-4.22 (2H, m), 5.96 (1H, s), 6.31 (1H, s), 17.94 (1H, s)
MS (FAB) m/z: 389 $(M+H)^+$

(90c) (9aS)-8-Acetyl-1-propyloxy-7-hydroxy-3-methoxy-9a-methyl-9-oxo-9,9a-dihydrodibenzo[b,d]furan-4-carboxamide

(9aS)-8-Acetyl-1-propyloxy-7-hydroxy-3-methoxy-9a-methyl-9-oxo-9,9a-dihydrodibenzo[b,d]furan-4-carboxylic acid produced in Example (90b) (392 mg, 1.01 mmol) was dissolved in N,N-dimethylformamide (8 mL). 1-Hydroxybenzotriazole (173 mg, 1.21 mmol) and 1-ethyl-3-(3-dimethylaminopropyl)carbodiimide hydrochloride (246 mg, 1.21 mmol) were added at room temperature, and the mixture was stirred in a nitrogen atmosphere for 30 minutes. Then, a 2 M solution of ammonia in isopropanol (1.6 mL) was added, and the mixture was stirred for one hour. The reaction solution was separated with ethyl acetate (15 mL) and 0.5 N hydrochloric acid (15 mL). The organic layer was washed with brine (15 mL) and dried over anhydrous sodium sulfate. The solvent was evaporated under reduced pressure, and the residue was purified by silica gel column chromatography (methylene chloride:methanol = 80:1 to 50:1, V/V) to give the title target compound (267 mg, 68%) as a yellow solid.
$^1$H-NMR ($CDCl_3$, 400MHz):$\delta$ ppm: 1.12 (3H, t, J=7.1Hz), 1.82 (3H, s), 1.86-1.96 (2H, m), 2.49 (3H, s), 3.95 (3H, s), 4.03 (1H, m), 4.16 (1H, m), 5.79 (1H, brs), 5.90 (1H, s), 6.27 (1H, s), 6.90 (1H, brs), 17.95 (1H, s)
MS (FAB) m/z: 388 $(M+H)^+$

(90d) (9aS)-8-Acetyl-1-propyloxy-7-hydroxy-3-methoxy-9a-methyl-N-[(2-methyl-1-naphthyl)methyl]-9-oxo-9,9a-dihydrodibenzo[b,d]furan-4-carboxamide

Reaction and post-treatment were carried out in accordance with Example 10 using (9aS)-8-acetyl-1-propyloxy-7-hydroxy-3-methoxy-9a-methyl-9-oxo-9,9a-dihydrodibenzo[b,d]furan-4-carboxamide produced in Example (90c) (267 mg, 0.69 mmol), 2-methyl-1-naphthaldehyde (352 mg, 2.07 mmol), triethylsilane (0.33 mL, 2.07 mmol), trifluoroacetic acid (0.16 mL, 2.07 mmol) and acetonitrile (10 mL) to give the target compound (179 mg, yield: 48%) as a yellow solid.
$^1$H-NMR ($CDCl_3$, 400MHz):$\delta$ ppm: 1.09 (3H, t, J=7.2Hz), 1.79 (3H, s), 1.82-1.90 (2H, m), 2.48 (3H, s), 2.62 (3H, s), 3.66 (3H, s), 3.96 (1H, m), 4.11 (1H, m), 5.08 (2H, m), 5.83 (1H, s), 6.16 (1H, s), 6.78 (1H, m), 7.35 (1H, d, J=8.4Hz), 7.46 (1H, m), 7.55 (1H, m), 7.74 (1H, d, J=8.4Hz), 7.84 (1H, d, J=8.4Hz), 8.12 (1H, d, J=8.4Hz), 18.00 (1H, s)
MS (ESI) m/z: 542 $(M+H)^+$.

(Example 91) (9aS)-8-Acetyl-N-{[4-(but-2-yn-1-yloxy)-1-naphthyl]methyl}-1,7-dihydroxy-3-methoxy-9a-methyl-9-oxo-9,9a-dihydrodibenzo[b,d]furan-4-carboxamide (exemplary compound No. 1-284)

(9 1 a) 4-(But-2-yn-1-yloxy)-1-naphthaldehyde

Sodium hydride (201 mg, 4.61 mmol) was suspended in N,N-dimethylformamide (20 mL) in a nitrogen atmosphere, and the suspension was cooled to 0˚C. 4-Hydroxy-1-naphthaldehyde (520 mg, 3.02 mmol) was slowly added, and the mixture was stirred at 0˚C for 30 minutes. 1-Bromo-2-butyne (680 $\mu$L, 7.53 mmol) was further added, and the mixture was stirred at room temperature for four hours. A dilute hydrochloric acid solution (30 mL) was added to the reaction solution, followed by extraction with diethyl ether (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 25/1 to 15/1) to give the target compound (570 mg, yield: 84%) as a light yellow solid.
$^1$H-NMR ($CDCl_3$,400MHz):$\delta$ ppm: 1.89 (3H, t, J=2.4Hz), 4.96 (2H, q, J=2.4Hz), 7.06 (1H, d, J=7.8Hz), 7.58 (1H, m), 7.70 (1H, m), 7.94 (1H, d, J=8.2Hz), 8.36 (1H, d, J=8.2Hz), 9.30 (1H, d, J=8.6Hz), 10.23 (1H, s).
MS (EI) m/z: 224 $(M^+)$

(9 1 b) (9aS)-8-Acetyl-N- {[4-(but-2-yn-1-yloxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (305 mg, 0.883 mmol), 4-(but-2-yn-1-yloxy)-1-naphthaldehyde produced in Example (91a) (565 mg, 2.52 mmol), triethylsilane (420 μL, 2.64 mmol), trifluoroacetic acid (210 μL, 2.73 mmol) and acetonitrile (10 mL) to give the target compound (331 mg, yield: 68%) as a yellow solid.
$^1$H-NMR ($CDCl_3$, 500MHz):δ ppm: 1.75 (3H, s), 1.88 (3H, s), 2.64 (3H, s), 3.69 (3H, s), 4.86 (2H, s), 4.97 (1H, dd, J=5.4, 14.7Hz), 5.03 (1H, dd, J=5.4, 14.7Hz), 6.00 (1H, s), 6.26 (1H,s), 6.89 (1H, d, J=7.8Hz), 7.19 (1H, brs), 7.45 (1H, d, J=7.8Hz), 7.52 (1H, m), 7.58 (1H, m), 8.05 (1H, d, J=8.3Hz), 8.36 (1H, d, J=8.3Hz), 10.63 (1H, s), 18.83 (1H, s).
MS (ESI+) m/z: 554.17913 $(M+H)^+$

(Example 92) (9aS)-8-Acetyl-1,7-dihydroxy-3-methoxy-9a-methyl-9-oxo-N-{[4-(pent-2-yn-1-yloxy)-1-naphthyl]methyl}-9,9a-dihydrodibenzo[b,d]furan-4-carboxamide (exemplary compound No. 1-287)

(92a) 4-(Pent-2-yn-1--yloxy)-1-naphthaldehyde

Sodium hydride (200 mg, 4.58 mmol) was suspended in N,N-dimethylformamide (20 mL) in a nitrogen atmosphere, and the suspension was cooled to 0˚C. 4-Hydroxy-1-naphthaldehyde (524 mg, 3.04 mmol) was slowly added, and the mixture was stirred at 0˚C for 30 minutes. 1-Bromo-2-pentyne (770 μL, 7.53 mmol) was further added, and the mixture was stirred at room temperature for four hours. A dilute hydrochloric acid solution (30 mL) was added to the reaction solution, followed by extraction with diethyl ether (100 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 15/1) to give the target compound (630 mg, yield: 87%) as a light yellow oil.
$^1$H-NMR ($CDCl_3$, 400MHz):δ ppm: 1.15 (3H, t, J=7.4Hz), 2.22-2.29 (2H, m), 4.98 (2H, t, J=2.2Hz), 7.07 (1H, d, J=7.8Hz), 7.58 (1H, m), 7.70 (1H, m), 7.94 (1H, d, J=8.2Hz), 8.36 (1H, d, J=7.8Hz), 9.30 (1H, d, J=8.6Hz), 10.23 (1H, s).
MS (EI) m/z: 238 $(M^+)$

(92b) (9aS)-8-Acetyl-1,7-dihydroxy-3-methoxy-9a-methyl-9-oxo-N-{[4-(pent-2-yn-1-yloxy)-1-naphthyl]methyl}-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (310 mg, 0.898 mmol), 4-(pent-2-yn-1-yloxy)-1-naphthaldehyde produced in Example (92a) (621 mg, 2.61 mmol), triethylsilane (420 μL, 2.64 mmol), trifluoroacetic acid (210 μL, 2.73 mmol) and acetonitrile (10 mL) to give the target compound (328 mg, yield: 64%) as a yellow solid.
$^1$H-NMR ($CDCl_3$, 500MHz):δ ppm: 1.15 (3H, t, J=7.3), 1.75 (3H, s), 2.25 (2H, q, J=7.3Hz), 2.64 (3H, s), 3.68 (3H, s), 4.88 (2H, s), 4.97 (1H, dd, J=4.9, 14.2Hz), 5.03 (1H, dd, J=4.9, 14.2Hz), 6.00 (1H, s), 6.26 (1H,s), 6.90 (1H, d, J=7.8Hz), 7.19 (1H, brs), 7.44 (1H, d, J=7.8Hz), 7.52 (1H, m), 7.58 (1H, m), 8.05 (1H, d, J=8.3Hz), 8.36 (1H, d, J=8.3Hz), 10.65 (1H, s), 18.83 (1H, s).
MS (ESI+) m/z: 568.19472 $(M+H)^+$

(Example 93) (9aS)-8-[(lE)-N-Ethoxyethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (exemplary compound No. 3-24)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (239 mg, 0.48 mmol) was dissolved in tetrahydrofuran:methanol (2:1, 5 mL). O-Ethylhydroxylamine hydrochloride (70 mg, 0.72 mmol) and sodium bicarbonate (60 mg, 0.72 mmol) were added at room temperature, and the mixture was stirred for four hours. The reaction solution was treated in the same manner as in Example 87 to give the title target compound (227 mg, 87%) as a yellow solid.
$^1$H-NMR ($CDCl_3$, 400MHz):δ ppm: 1.37 (3H, t, J=7.1), 1.71 (3H, s), 2.58 (3H, s), 2.63 (3H, s), 3.60 (3H, s), 4.14 (2H, dd, J=7.1, 13.9Hz), 5.02-5.14 (2H, m), 5.87 (1H, s), 6.17 (1H, s), 7.01 (1H, m), 7.35 (1H, d, J=8.7Hz), 7.45 (1H, m), 7.54 (1H, m), 7.74 (1H, d, J=8.3Hz), 7.83 (1H, d, J=7.6Hz), 8.13 (1H, d, J=8.7Hz), 11.2 8 (1H, s), 15.95 (1H, brs)
MS (ESI) m/z: 543 $(M+H)^+$

(Example 94) (9aS)-8-Acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide (exemplary compound No. 1-121)

(94a) 2,3,5-Trimethyl-6-[(1E)-1-propenyl]benzaldehyde

2-Bromo-3,5,6-trimethylbenzaldehyde produced in Example (74a) (8.00 g, 35.2 mmol) was dissolved in ethylene glycol dimethyl ether (192 mL) and water (48 mL). Tetrakistriphenylphosphine palladium (2.03 g, 1.76 mmol), trans-propenylboronic acid (3.33 g, 38.7 mmol) and potassium carbonate (29.2 g, 2.11 mol) were added, and the mixture was stirred with heating to reflux in a nitrogen atmosphere for eight hours. The reaction solution was cooled to room temperature and then water (200 mL) was added, followed by extraction with ethyl acetate (250 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 30/1) to give the target compound (5.35 g, yield: 81%) as a white solid.
$^1$H-NMR ($CDCl_3$, 500MHz):δ ppm: 1.95 (3H, d, J=6.8Hz), 2.25 (3H, s), 2.28 (3H, s), 2.41 (3H, s), 5.54 (1H, s), 6.58 (1H, d, J=15.6Hz), 7.13 (1H, s), 10.15 (1H, s).
MS (EI) m/z: 188 ($M^+$)

(94b) 2,3,5-Trimethyl-6-propylbenzaldehyde

2,3,5-Trimethyl-6-[(1E)-1-propenyl]benzaldehyde produced in Example (94a) (5.35 g, 28.4 mmol) was dissolved in ethyl acetate (80 mL). 10% palladium-carbon (3.02 g) was added, and the mixture was stirred in a hydrogen (1 atm) atmosphere for five hours. Palladium-carbon was separated by filtration, and then the solvent was evaporated under reduced pressure. The resulting residue was purified by silica gel column chromatography (elution solvent: hexane/ethyl acetate = 30/1 to 25/1) to give the target compound (3.01 g, yield: 56%) as a white solid.
$^1$H-NMR ($CDCl_3$, 500MHz):δ ppm: 1.01 (3H, t, J=7.3Hz), 1.50-1.57 (2H, m), 2.25 (3H, s), 2.29 (3H, s), 2.38 (3H, s), 2.78 (2H, t, J=7.8Hz), 7.13 (1H, s), 10.61 (1H, s). MS (EI) m/z: 190 ($M^+$)

(94c) (9aS)-8-Acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,3,5-trimethyl-6-propylbenzaldehyde produced in Example (94b) (0.200 g, 1.05 mmol), triethylsilane (0.554 mL, 3.48 mmol), trifluoroacetic acid (0.268 mL, 3.48 mmol) and acetonitrile (15.0 mL) to give the target compound (0.160 g, yield: 35%) as a yellow solid.
$^1$H-NMR ($CDCl_3$, 500MHz):δ ppm: 1.03 (3H, t, J=7.3Hz), 1.48-1.57 (2H, m), 1.75 (3H, s), 2.25 (3H, s), 2.26 (3H, s), 2.29 (3H, s), 2.64-2.67 (2H, m), 2.65 (3H, s), 2.73 (2H, q, J=7.8Hz), 3.77 (3H, s), 4.61-4.69 (2H, m), 6.01 (1H, s), 6.28 (1H, s), 6.64 (1H, brs), 6.96 (1H, s), 10.62 (1H, s), 18.83 (1H, s).
MS (ESI) m/z: 520.2150 $(M+H)^+$

(Example 95) (9aS)-8-Acetyl-1,7-dihydroxy-3-methoxy-9a-methyl-9-oxo-N-(2,3,6-trimethylbenzyl)-9,9a-dihydrodibenzo[b,d]furan-4-carboxamide (exemplary compound No. 1-64)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), known 2,3,6-trimethylbenzaldehyde [Journal of Organic Chemistry, 1941, Vol. 6, p.489-500] (0.180 g, 1.21 mmol), triethylsilane (0.554 mL, 3.48 mmol), trifluoroacetic acid (0.268 mL, 3.48 mmol) and acetonitrile (15.0 mL) to give the target compound (0.188 g, yield: 45%) as a yellow solid.
$^1$H-NMR ($CDCl_3$, 400MHz):δ ppm: 1.75 (3H, s), 2.27 (3H, s), 2.31 (3H, s), 2.39 (3H, s), 2.64 (3H, s), 3.78 (3H, s), 4.60-4.70 (2H, m), 6.00 (1H, s), 6.27 (1H, s), 6.71 (1H, brs), 6.95 (1H, d, J=7.5Hz), 7.02 (1H, d, J=7.5Hz), 10.61 (1H, s), 18.78 (1H, s).
MS (ESI) m/z: 478.1859 $(M+H)^+$.

(Example 96) (9aS)-8-Acetyl-N-(3-butyl-2,5,6-trimethylbenzyl)-1,7-dihydroxy-3-methoxy-9a-methyl-9-oxo-9,9a-dihydrodibenzo[b,d]furan-4-carboxamide (exemplary compound No. 1-123)

(96a) (3-Bromo-2,5,6-trimethylphenyl)methanol

2-Bromo-3,5,6-trimethylbenzaldehyde produced in Example (74a) (1.00 g, 4.40 mmol) was dissolved in tetrahydrofuran (30 mL). A solution of diisobutylaluminum hydride in toluene (6.54 mL, 6.60 mmol) was added at 0˚C, and the mixture was stirred in a nitrogen atmosphere for one hour. After completion of the reaction, a saturated (+)-potassium sodium tartrate aqueous solution (60 mL) was added, and the mixture was heated to room temperature with stirring. After stirring at room temperature for one hour, the aqueous layer was extracted with ethyl acetate (60 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10 to 65/35) to give the target compound (0.930 g, yield: 92%) as a yellow solid.
$^1$H-NMR ($CDCl_3$, 500MHz):δ ppm: 2.24 (3H, s), 2.29 (3H, s), 2.50 (3H, s), 4.79 (2H, s), 7.35 (1H, s).
MS (EI) m/z: 228 ($M^+$)

(96b) (3-Butyl-2,5,6-trimethylphenyl)methanol

(3-Bromo-2,5,6-trimethylphenyl)methanol produced in Example (96a) (0.930 g, 4.06 mmol) was dissolved in tetrahydrofuran (30 mL), and then the solution was cooled to -78˚C. A solution of n-butyllithium in hexane (4.70 mL, 12.2 mmol) was added, and the mixture was heated to 0˚C with stirring in a nitrogen atmosphere. After stirring at 0˚C for one hour, a saturated ammonium chloride aqueous solution (50 mL) was added, followed by extraction with ethyl acetate (50 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10 to 65/35) to give the target compound (0.277 g, yield: 33%) as a colorless solid.
$^1$H-NMR ($CDCl_3$, 500MHz):δ ppm: 0.94 (3H, t, J=7.3Hz), 1.36-1.43 (2H, m), 1.48-1.54 (2H, m), 2.25 (3H, s), 2.30 (3H, s), 2.34 (3H, s), 2.57 (2H, t, J=7.8Hz), 4.77 (2H, s), 6.95 (1H, s).
MS (EI) m/z: 206 ($M^+$)

(96c) 3-Butyl-2,5,6-trimethylbenzaldehyde

(3-Butyl-2,5,6-trimethylphenyl)methanol produced in Example (96b) (0.277 g, 1.34 mmol) was dissolved in chloroform (30 mL). Then, manganese dioxide (1.17 g, 13.4 mmol) was added, and the mixture was stirred with heating to reflux in a nitrogen atmosphere for 24 hours. The reaction solution was cooled to room temperature, and then manganese dioxide was separated by filtration. Thereafter, the solvent was evaporated under reduced pressure, and the resulting residue was purified by silica gel column chromatography (elution solvent: hexane/ethyl acetate = 20/1 to 10/1) to give the target compound (0.148 g, yield: 54%) as a white solid.
$^1$H-NMR ($CDCl_3$, 500MHz):δ ppm: 0.94 (3H, t, J=7.3Hz), 1.35-1.43 (2H, m), 1.48-1.54 (2H, m), 2.27 (3H, s), 2.39 (3H, s), 2.42 (3H, s), 2.59 (2H, t, J=7.8Hz), 7.11 (1H, s), 10.65 (1H, s).
MS (EI) m/z: 204 ($M^+$)

(96d) (9aS)-8-Acetyl-N-(3-butyl-2,5,6-trimethylbenzyl)-1,7-dihydroxy-3-methoxy-9a-methyl-9-oxo-9,9a-dihydrodibenzo [b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.250 g, 0.869 mmol), 3-butyl-2,5,6-trimethylbenzaldehyde produced in Example (96c) (0.145 g, 0.72 mmol), triethylsilane (0.461 mL, 2.90 mmol), trifluoroacetic acid (0.223 mL, 2.90 mmol) and acetonitrile (15.0 mL) to give the target compound (0.138 g, yield: 36%) as a yellow solid.
$^1$H-NMR ($CDCl_3$, 400MHz):δ ppm: 0.94 (3H, t, J=7.3Hz), 1.35-1.44 (2H, m), 1.48-1.56 (2H, m), 1.75 (3H, s), 2.26 (3H, s), 2.29 (3H, s), 2.32 (3H, s), 2.58 (2H, q, J=7.8Hz), 2.64 (3H, s), 3.77 (3H, s), 4.61-4.71 (2H, m), 6.00 (1H, s), 6.26 (1H, s), 6.66 (1H, brs), 6.93 (1H, s), 10.60 (1H, s), 18.78 (1H, s).
MS (ESI) m/z: 534.2477 $(M+H)^+$

(Example 97) (9aS)-8-Acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide (exemplary compound No. 1-76)

(97a) 4-Formyl-3,5-dimethylphenyl acetate

4-Hydroxy-2,6-dimethylbenzaldehyde (0.387 g, 2.58 mmol) was dissolved in methylene chloride (25 mL) and pyridine (5 mL). Then, acetic anhydride (0.292 mL, 3.09 mmol) was added, and the mixture was stirred in a nitrogen atmosphere for one hour. After completion of the reaction, the solvent was evaporated under reduced pressure. The resulting residue was purified by silica gel column chromatography (elution solvent: hexane/ethyl acetate = 95/5 to 80/20)

to give the target compound (0.495 g, yield: 100%) as a white solid.
$^1$H-NMR ($CDCl_3$, 400MHz):δ ppm: 2.31 (3H, s), 2.62 (6H, s), 6.85 (1H, s), 10.56 (1H, s).
MS (FAB) m/z: 193 $(M+H)^+$

(97b) (9aS)-4-({[(8-Acetyl-1,7-dihydroxy-3-methoxy-9a-methyl-9-oxo-9,9a-dihydrodibenzo[b,d]furan-4-yl)carbonyl]amino}methyl)-3,5-dimethylphenyl acetate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.800 g, 2.32 mmol), 4-formyl-3,5-dimethylphenyl acetate produced in Example (97a) (0.490 g, 2.55 mmol), triethylsilane (1.48 mL, 9.27 mmol), trifluoroacetic acid (0.714 mL, 9.27 mmol) and acetonitrile (30.0 mL) to give the target compound (0.994 g, yield: 83%) as a yellow solid.
$^1$H-NMR ($CDCl_3$, 400MHz):δ ppm: 1.75 (3H, s), 2.29 (3H, s), 2.42 (6H, s), 2.65 (3H, s), 3.81 (3H, s), 4.55-4.66 (2H, m), 6.02 (1H, s), 6.29 (1H, s), 6.73 (1H, brs), 6.80 (2H, s), 10.66 (1H, s), 18.83 (1H, s).
MS (FAB) m/z: 522 $(M+H)^+$

(97c) (9aS)-8-Acetyl-1,7-dihydroxy-N-(4-hydroxy-2,6-dimethylbenzyl)-3-methoxy-9a-methyl-9-oxo-9,9a-dihydrodibenzo[b,d]furan-4-carboxamide

(9aS)-4-({[(8-Acetyl-1,7-dihydroxy-3-methoxy-9a-methyl-9-oxo-9,9a-dihydrodibenzo[b,d]furan-4-yl)carbonyl] amino}methyl)-3,5-dimethylphenyl acetate produced in Example (97b) (0.990 g, 1.90 mmol) was dissolved in a 1 N sodium hydroxide aqueous solution (25 mL) and tetrahydrofuran (5 mL), and the mixture was stirred at room temperature for 12 hours. The reaction solution was cooled to 0˚C, and a dilute hydrochloric acid solution (50 mL) was slowly added dropwise. The precipitated product was filtered and dried to give the target compound (0.910 g, yield: 100%) as a yellow solid.
$^1$H-NMR ($CDCl_3$, 500MHz):δ ppm: 1.74 (3H, s), 2.37 (6H, s), 2.64 (3H, s), 3.79 (3H, s), 4.51-4.60 (2H, m), 4.96 (1H, brs), 5.98 (1H, s), 6.28 (1H, s), 6.56 (2H, s), 6.69 (1H, brs), 10.63 (1H, s), 18.81 (1H, s).
MS (FAB) m/z: 480 $(M+H)^+$

(97d) (9aS)-8-Acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide

(9aS)-8-Acetyl-1,7-dihydroxy-N-(4-hydroxy-2,6-dimethylbenzyl)-3-methoxy-9a-methyl-9-oxo-9,9a-dihydrodibenzo[b,d]furan-4-carboxamide produced in Example (97c) (0.150 g, 0.313 mmol) was dissolved in N,N-dimethylformamide (4 mL). Potassium carbonate (0.173 g, 1.25 mmol) and 1-bromo-2-butyne (0.056 mL, 0.626 mmol) were added, and the mixture was stirred in a nitrogen atmosphere at 60˚C for 12 hours. After completion of the reaction, the reaction solution was cooled to 0˚C. 0.1 N hydrochloric acid (10 mL) was added, followed by extraction with ethyl acetate (10 mL). The organic layer was washed with brine and then dried over anhydrous sodium sulfate. The solvent was evaporated under reduced pressure, and the resulting residue was purified by silica gel column chromatography (elution solvent: methylene chloride/methanol = 80/1 to 40/1) to give the target compound (0.060 g, yield: 36%) as a yellow solid.
$^1$H-NMR ($CDCl_3$, 500MHz):δ ppm: 1.75 (3H, s), 1.87 (3H, s), 2.40 (6H, s), 2.65 (3H, s), 3.79 (3H, s), 4.52-4.62 (2H, m), 4.62-4.63 (2H, m), 6.01 (1H, s), 6.28 (1H, s), 6.67 (3H, brs), 10.64 (1H, s), 18.82 (1H, s).
MS (ESI) m/z: 532.1966 $(M+H)^+$.

(Example 98) (9aS)-8-[(1E)-N-(Allyloxy)ethaneimidoyl]-1,7-dihydroxy-3-methoxy-9a-methyl-N-[(2-methyl-1-naphthyl) methyl]-9-oxo-9,9a-dihydrodibenzo[b,d]furan-4-carboxamide (exemplary compound No. 3-48)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (202 mg, 0.40 mmol) was dissolved in tetrahydrofuran:methanol (2:1, 5 mL). O-Allylhydroxylamine hydrochloride (53 mg, 0.49 mmol) and sodium bicarbonate (41 mg, 0.49 mmol) were added at room temperature, and the mixture was stirred for four hours. The reaction solution was treated in the same manner as in Example 87 to give the title target compound (200 mg, 89%) as a yellow solid. $^1$H-NMR ($CDCl_3$; 400MHz):δ ppm: 1.70 (3H, s), 2.55 (3H, s), 2.62 (3H, s), 3.59 (3H, s), 4.54 (2H, d, J=6.4Hz), 5.01-5.13 (2H, m), 5.41-5.47 (2H, m), 5.86 (1H, s), 5.99
(1H, m), 6.16 (1H, s), 7.00 (1H, m), 7.33 (1H, d, J=8.3Hz), 7.43 (1H, m), 7.53 (1H, m), 7.72 (1H, d, J=8.3Hz), 7.81 (1H, d, J=8.3Hz), 8.11 (1H, d, J=8.3Hz), 11.21 (1H, s), 15.84 (1H, brs)
MS (ESI) m/z: 555 $(M+H)^+$

(Example 99) (9aS)-8-[(1E)-N-(Cyclopropylmethoxy)ethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (exemplary compound No. 3-80)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (200 mg, 0.40 mmol) was dissolved in tetrahydrofuran:methanol (2:1, 5 mL). O-(Cyclopropylmethyl)hydroxylamine hydrochloride (59 mg, 0.48 mmol) and sodium bicarbonate (40 mg, 0.48 mmol) were added at room temperature, and the mixture was stirred for four hours. The reaction solution was treated in the same manner as in Example 87 to give the title target compound (207 mg, 91 %) as a yellow solid.
$^1$H-NMR ($CDCl_3$, 400MHz):δ ppm: 0.36 (2H, m), 0.69 (2H, m), 1.19 (1H, m), 1.71 (3H, s), 2.61 (3H, s), 2.62 (3H, s), 3.60 (3H, s), 3.88 (2H, d, J=7.1Hz), 5.01-5.13 (2H, m), 5.85 (1H, s), 6.15 (1H, s), 6.98 (1H, m), 7.33 (1H, d, J=8.3Hz), 7.44 (1H, m), 7.53 (1H, m), 7.72 (1H, d, J=8.3Hz), 7.82 (1H, d, J=8.7Hz), 8.11 (1H, d, J=8.7Hz), 11.30 (1H, s), 15.98 (1H, brs)
MS (ESI) m/z: 569 $(M+H)^+$

(Example 100) (9aS)-1,7-Dihydroxy-3-methoxy-9a-methyl-N-[(2-methyl-1-naphthyl)methyl]-9-oxo-8-[(1E)-N-propoxyethaneimidoyl]-9,9a-dihydrodibenzo[b,d]furan-4-carboxamide (exemplary compound No. 3-28)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (200 mg, 0.40 mmol) was dissolved in tetrahydrofuran:methanol (2:1, 5 mL). O-Propylhydroxylamine hydrochloride (54 mg, 0.48 mmol) and sodium bicarbonate (40 mg, 0.48 mmol) were added at room temperature, and the mixture was stirred for four hours. The reaction solution was treated in the same manner as in Example 87 to give the title target compound (201 mg, 90%) as a yellow solid. $^1$H-NMR ($CDCl_3$, 400MHz):δ ppm: 1.02 (3H, t, J=7.3Hz), 1.71 (3H, s), 1.77 (2H, m), 2.58 (3H, s), 2.63 (3H, s), 3.60 (3H, s), 4.04 (2H, t, J=6.4Hz), 5.03-5.14 (2H, m), 5.87 (1H, s), 6.17 (1H, s), 7.00 (1H, m), 7.35 (1H, d, J=8.3Hz), 7.45 (1H, m), 7.54 (1H, m), 7.74 (1H, d, J=8.3Hz), 7.83 (1H, d, J=8.3Hz), 8.13 (1H, d, J=8.3Hz), 11.28 (1H, s), 16.01 (1H, brs)
MS (ESI) m/z: 557 $(M+H)^+$.

(Example 101) (9aS)-8-Acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide (exemplary compound No. 1-77)

(101a) 2-Bromo-1,3-dimethyl-5-(2-pentynyloxy)benzene

4-Bromo-3,5-dimethylphenol (2.00 g, 9.95 mmol) was dissolved in N,N-dimethylformamide (30 mL). Sodium hydride (0.358 g, 14.9 mmol) was added at 0˚C, and the mixture was stirred in a nitrogen atmosphere for 30 minutes. 1-Bromo-2-pentyne (1.52 mL, 14.9 mmol) was further added, and the mixture was stirred at room temperature for three hours. A dilute hydrochloric acid solution (50 mL) was added to the reaction solution, followed by extraction with ethyl acetate (60 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 97/3 to 90/10) to give the target compound (2.65 g, yield: 99%) as a colorless liquid.
$^1$H-NMR ($CDCl_3$, 400MHz):δ ppm: 1.14 (3H, t, J=7.3Hz), 2.20-2.26 (2H, m), 2.38 (6H, s), 4.60(2H, s), 6.69 (2H, s).
MS (EI) m/z: 266 $(M^+)$

(101b) 2,6-Dimethyl-4-(2-pentynyloxy)benzaldehyde

2-Bromo-1,3-dimethyl-5-(2-pentynyloxy)benzene produced in Example (101a) (2.65 g, 9.92 mmol) was dissolved in tetrahydrofuran (50 mL), and then the solution was cooled to -78˚C. A solution of sec-butyllithium in hexane (10.2 mL, 9.92 mmol) was added, and the mixture was stirred in a nitrogen atmosphere for 10 minutes. Thereafter, N, N-dimethylformamide (1.54 mL, 19.8 mmol) was added dropwise, and the mixture was stirred at -78˚C for 30 minutes. Then, a saturated ammonium chloride aqueous solution (80 mL) was added, followed by extraction with ethyl acetate (100 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 95/5 to 85/15) to give the target compound (0.240 g, yield: 11%) as white crystals.
$^1$H-NMR ($CDCl_3$, 500MHz):δ ppm: 1.14 (3H, t, J=7.3Hz), 2.22-2.27 (2H, m), 2.61 (6H, s), 4.70(2H, s), 6.66 (2H, s), 10.49 (1H, s).
MS (EI) m/z: 216 $(M^+)$

(101c) (9aS)-8-Acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), 2,6-dimethyl-4-(2-pentynyloxy)benzaldehyde produced in Example (101b) (0.240 g, 1.11 mmol), triethylsilane (0.738 mL, 4.63 mmol), trifluoroacetic acid (0.357 mL, 4.63 mmol) and acetonitrile (15.0 mL) to give the target compound (0.184 g, yield: 17%) as a yellow solid.
$^1$H-NMR ($CDCl_3$, 400MHz):δ ppm: 1.14 (3H, t, J=7.3Hz), 1.74 (3H, s), 2.17-2.27 (2H, m), 2.39 (6H, s), 2.64 (3H, s), 3.78 (3H, s), 4.51-4.61 (2H, m), 6.00 (1H, s), 6.27 (1H, s), 6.66 (3H, brs), 6.95 (1H, s), 10.61 (1H, s), 18.78 (1H, s).
MS (ESI) m/z: 546.2155 $(M+H)^+$

(Example 102) (9aS)-8-Acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide (exemplary compound No. 1-134)

(102a) 4-(2-Butynyloxy)-2,3,6-trimethylbenzaldehyde

Known 4-hydroxy-2,3,6-trimethylbenzaldehyde [Journal of Organic Chemistry, 1962, Vol. 27, p.18839-1842] (0.600 g, 3.65 mmol) was dissolved in N,N-dimethylformamide (8 mL). Sodium hydride (0.132 g, 5.48 mmol) was added at 0˚C, and the mixture was stirred in a nitrogen atmosphere for 30 minutes. 1-Bromo-2-butyne (0.495 mL, 5.48 mmol) was further added, and the mixture was stirred at room temperature for three hours. After completion of the reaction, a dilute hydrochloric acid solution (30 mL) was added to the reaction solution, followed by extraction with ethyl acetate (40 mL). The organic layer was washed with brine and then dried over anhydrous sodium sulfate. The solvent was evaporated under reduced pressure; and the resulting residue was purified by silica gel column chromatography (elution solvent: hexane/ethyl acetate = 95/5 to 80/20) to give the target compound (0.696 g, yield: 88%) as white crystals.
$^1$H-NMR ($CDCl_3$, 400MHz):δ ppm: 1.87 (3H, s), 2.16 (3H, s), 2.52 (3H, s), 2.59 (3H, s), 4.70 (2H, s), 6.64 (1H, s), 10.50 (1H, s).
MS (EI) m/z: 216 $(M^+)$

(102b) (9aS)-8-Acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), 4-(2-butynyloxy)-2,3,6-trimethylbenzaldehyde produced in Example (102a) (0.348 g, 1.61 mmol), triethylsilane (0.738 mL, 4.63 mmol), trifluoroacetic acid (0.357 mL, 4.63 mmol) and acetonitrile (15.0 mL) to give the target compound (0.059 g, yield: 7%) as a yellow solid.
$^1$H-NMR ($CDCl_3$, 400MHz):δ ppm: 1.75 (3H, s), 1.86 (3H, s), 2.17 (3H, s), 2.30 (3H, s), 2.41 (3H, s), 2.64 (3H, s), 3.78 (3H, s), 4.54-4.65 (4H, m), 6.00 (1H, s), 6.27 (1H, s), 6.65 (1H, brs), 6.68 (1H, s), 10.60 (1H, s), 18.78 (1H, s).
MS (ESI) m/z: 546.2139 $(M+H)^+$

(Example 103) (9aS)-8-Acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide (exemplary compound No. 1-135)

(103a) 4-(2-Pentynyloxy)-2,3,6-trimethylbenzaldehyde

Known 4-hydroxy-2,3,6-trimethylbenzaldehyde [Journal of Organic Chemistry, 1962, Vol. 27, p.1839-1842] (0.500 g, 3.05 mmol) was dissolved in N,N-dimethylformamide (8 mL). Sodium hydride (0.110 g, 4.57 mmol) was added at 0˚C, and the mixture was stirred in a nitrogen atmosphere for 30 minutes. 1-Bromo-2-pentyne (0.467 mL, 4.57 mmol) was further added, and the mixture was stirred at room temperature for three hours. After completion of the reaction, a dilute hydrochloric acid solution (30 mL) was added to the reaction solution, followed by extraction with ethyl acetate (40 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 95/5 to 80/20) to give the target compound (0.678 g, yield: 97%) as white crystals.
$^1$H-NMR ($CDCl_3$, 400MHz):δ ppm: 1.14 (3H, t, J=7.4Hz), 2.17 (3H, s), 2.21-2.27 (2H, m), 2.53 (3H, s), 2.60 (3H, s), 4.73 (2H, s), 6.68 (1H, s), 10.54 (1H, s).
MS (EI) m/z: 230 $(M^+)$

(103b) (9aS)-8-Acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.300 g, 0.869 mmol), 2,3,6-trimethyl-4-(2-pentynyloxy)benzaldehyde produced in Example (103a) (0.339 g, 1.47 mmol), triethylsilane (0.554 mL, 3.48 mmol), trifluoroacetic acid (0.268 mL, 3.48 mmol) and acetonitrile (15.0 mL) to give the target compound (0.068 g, yield: 14%) as a yellow solid.
$^1$H-NMR ($CDCl_3$, 500MHz):δ ppm: 1.15 (3H, t, J=7.4Hz), 1.75 (3H, s), 2.18 (3H, s), 2.22-2.26 (2H, m), 2.31 (3H, s), 2.41 (3H, s); 2.65 (3H, s), 3.79 (3H, s), 4.56-4.65 (2H, m), 4.66 (2H, s), 6.01 (1H, s), 6.28 (1H, s), 6.66 (1H, brs), 6.72 (1H, s), 10.62 (1H, s), 18.82 (1H, s).
MS (ESI) m/z: 582.2149 $(M+H)^+$.

(Example 104) (9aS)-8-Acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide (exemplary compound No. 1-419)

(104a) 4-(Benzyloxy)-1-naphthaldehyde

Sodium hydride (99.7 mg, 2.28 mmol) was suspended in N,N-dimethylformamide (10 mL) in a nitrogen atmosphere, and the suspension was cooled to 0˚C. 4-Hydroxy-1-naphthaldehyde (257 mg, 1.49 mmol) was slowly added, and the mixture was stirred at 0˚C for 30 minutes. (Bromomethyl)benzene (350 μL, 2.94 mmol) was further added, and the mixture was stirred at room temperature for two hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 10/1 to 5/1) to give the target compound (378 mg, yield: 97%) as a light yellow oil.
$^1$H-NMR ($CDCl_3$, 400MHz):δ ppm: 5.34 (2H, s), 6.98 (1H, d, J=8.2Hz), 7.35-7.44 (3H, m), 7.49-7.58 (3H, m), 7.69 (1H, m), 7.89 (1H, d, J=7.8Hz), 8.39 (1H, d, J=8.6Hz), 9.29 (1H, d, J=8.6Hz), 10.18 (1H, s).
MS (FAB) m/z: 263 $(M+H)^+$

(104b) (9aS)-8-Acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (229 mg, 0.663 mmol), 4-(benzyloxy)-1-naphthaldehyde produced in Example (104a) (368 mg, 1.40 mmol), triethylsilane (320 μL, 2.01 mmol), trifluoroacetic acid (150 μL, 1.95 mmol) and acetonitrile (8 mL) to give the target compound (201 mg, yield: 51 %) as a yellow solid.
$^1$H-NMR ($CDCl_3$, 500MHz):δ ppm: 1.75 (3H, s), 2.64 (3H, s), 3.68 (3H, s), 4.96 (1H, dd, J=4. 8, 14.7Hz), 5.03 (1H, dd, J=4.8, 14.7Hz), 5.26 (2H, s), 6.00 (1H, s), 6.26 (1H,s), 6.84 (1H, d, J=7.9Hz), 7.19 (1H, brs), 7.34-7.61 (8H, m), 8.06 (1H, d, J=8.3Hz), 8.43 (1H, d, J=8.3Hz), 10.65 (1H, s), 18.83 (1H, s).
MS (ESI+) m/z: 592.20063 $(M+H)^+$

(Example 105) (9aS)-8-[(1E)-N-(Benzyloxy)ethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (exemplary compound No. 3-84)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (230 mg, 0.46 mmol) was dissolved in tetrahydrofuran:methanol (2:1, 5 mL). O-Benzylhydroxylamine hydrochloride (110 mg, 0.69 mmol) and sodium bicarbonate (58 mg, 0.69 mmol) were added at room temperature, and the mixture was stirred for four hours. The reaction solution was treated in the same manner as in Example 87 to give the title target compound (237 mg, 85%) as a yellow solid.
$^1$H-NMR ($CDCl_3$, 400MHz):δ ppm: 1.68 (3H, s), 2.50 (3H, s), 2.63 (3H, s), 3.60 (3H, s), 5.07 (2H, s), 5.01-5.14 (2H, m), 5.85 (1H, s), 6.17 (1H, s), 7.02 (1H, m), 7.35 (1H, d, J=8.8Hz), 7.40-7.47 (6H, m), 7.55 (1H, m), 7.74 (1H, d, J=8.3Hz), 7.84 (1H, d, J=7.9Hz), 8.12 (1H, d, J=8.7Hz), 11.23 (1H, s), 15.72 (1H, brs)
MS (ESI) m/z: 605 $(M+H)^+$.

(Example 106) (9aS)-8-Acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-420)

(106a) 4-[(4-Fluorobenzyl)oxy]-1-naphthaldehyde

Sodium hydride (103 mg, 2.37 mmol) was suspended in N,N-dimethylformamide (10 mL) in a nitrogen atmosphere, and the suspension was cooled to 0˚C. 4-Hydroxy-1-naphthaldehyde (255 mg, 1.48 mmol) was slowly added, and the mixture was stirred at 0˚C for 30 minutes. 1-(Bromomethyl)-4-fluorobenzene (280 μL, 2.25 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 5/1) to give the target compound (372 mg, yield: 90%) as a colorless solid.
$^1$H-NMR ($CDCl_3$, 500MHz):δ ppm: 5.32 (2H, s), 7.00 (1H, d, J=7.8Hz), 7.12-7.15 (2H, m), 7.49-7.52 (2H, m), 7.58 (1H, m), 7.72 (1H, m), 7.93 (1H, d, J=7.8Hz), 8.38 (1H, d, J=8.3Hz), 9.32 (1H, d, J=8.3Hz), 10.22 (1H, s).
MS (EI) m/z: 280 ($M^+$)

(106b) (9aS)-8-Acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (231 mg, 0.668 mmol), 4-[(4-fluorobenzyl)oxy]-1-naphthaldehyde produced in Example (106a) (368 mg, 1.31 mmol), triethylsilane (320 μL, 2.01 mmol), trifluoroacetic acid (155 μL, 2.01 mmol) and acetonitrile (8 mL) to give the target compound (278 mg, yield: 68%) as a yellow solid.
$^1$H-NMR ($CDCl_3$, 500MHz):δ ppm: 1.74 (3H, s), 2.64 (3H, s), 3.69 (3H, s), 4.97 (1H, dd, J=5.4, 14.7Hz), 5.02 (1H, dd, J=5.4, 14.7Hz), 5.22 (2H, s), 6.00 (1H, s), 6.26 (1H, s), 6.83 (1H, d, J=7.8Hz), 7.09-7.13 (2H, m), 7.20 (1H, brs), 7.43 (1H, d, J=7.8Hz), 7.49-7.54 (3H, m), 7.59 (1H, m), 8.06 (1H, d, J=8.3Hz), 8.39 (1H, d, J=8.3Hz), 10.65 (1H, s), 18.83 (1H, s).
MS (ESI+) m/z: 610.18793 $(M+H)^+$

(Example 107) (9aS)-8-Acetyl-1,7-dihydroxy-3-methoxy-9a-methyl-9-oxo-N-[(4-{[4-(trifluoromethyl)benzyl]oxy}-1-naphthyl)methyl]-9,9a-dihydrodibenzo[b,d]furan-4-carboxamide (exemplary compound No. 1-426)

(107a) 4-{[4-(Trifluoromethyl)benzyl]oxy}-1-naphthaldehyde

Sodium hydride (104 mg, 2.38 mmol) was suspended in N,N-dimethylformamide (10 mL) in a nitrogen atmosphere, and the suspension was cooled to 0˚C. 4-Hydroxy-1-naphthaldehyde (257 mg, 1.49 mmol) was slowly added, and the mixture was stirred at 0˚C for 30 minutes. 1-(Bromomethyl)-4-(trifluoromethyl)benzene (533 mg, 2.23 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 5/1) to give the target compound (451 mg, yield: 92%) as a colorless solid.
$^1$H-NMR ($CDCl_3$, 500MHz):δ ppm: 5.42 (2H, s), 6.98 (1H, d, J=7.8Hz), 7.59-7.75 (6H, m), 7.93 (1H, d, J=7.8Hz), 8.41 (1H, d, J=8.8Hz), 9.33 (1H, d, J=8.8Hz), 10.23 (1H, s).
MS (EI) m/z: 330 ($M^+$)

(107b) (9aS)-8-Acetyl-1,7-dihydroxy-3-methoxy-9a-methyl-9-oxo-N-[(4-{[4-(trifluoromethyl)benzyl]oxy}-1-naphthyl)methyl]-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (234 mg, 0.677 mmol), 4-{[4-(trifluoromethyl)benzyl]oxy}-1-naphthaldehyde produced in Example (107a) (445 mg, 1.35 mmol), triethylsilane (320 μL, 2.01 mmol), trifluoroacetic acid (155 μL, 2.01 mmol) and acetonitrile (8 mL) to give the target compound (292 mg, yield: 65%) as a yellow solid.
$^1$H-NMR ($CDCl_3$, 500MHz):δ ppm: 1.74 (3H, s), 2.64 (3H, s), 3.69 (3H, s), 4.97 (1H, dd, J=5.4, 14.7Hz), 5.03 (1H, dd, J=5.4, 14.7Hz), 5.32 (2H, s), 6.00 (1H, s), 6.26 (1H, s), 6.81 (1H, d, J=7.8Hz), 7.21 (1H, brs), 7.43 (1H, d, J=7.8Hz), 7.54-7.69 (6H, m), 8.08 (1H, d, J=8.3Hz), 8.41 (1H, d, J=8.3Hz), 10.65 (1H, s), 18.83 (1H, s).

MS (ESI+) m/z: 660.18228 $(M+H)^+$

(Example 108) (9aS)-8-Acetyl-N-({4-[(4-chlorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-423)

(108a) 4-[(4-Chlorobenzyl)oxy]-1-naphthaldehyde

Sodium hydride (102 mg, 2.34 mmol) was suspended in N,N-dimethylformamide (10 mL) in a nitrogen atmosphere, and the suspension was cooled to 0˚C. 4-Hydroxy-1-naphthaldehyde (267 mg, 1.55 mmol) was slowly added, and the mixture was stirred at 0˚C for 30 minutes. 1-(Bromomethyl)-4-chlorobenzene (485 mg, 2.36 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 10/1 to 4/1) to give the target compound (428 mg, yield: 93%) as a colorless solid.
$^1$H-NMR ($CDCl_3$, 400MHz):δ ppm: 5.32 (2H, s), 6.98 (1H, d, J=8.2Hz), 7.41-7.48 (4H, m), 7.59 (1H, m), 7.72 (1H, m), 7.92 (1H, d, J=7.8Hz), 8.38 (1H, d, J=8.6Hz), 9.32 (1H, d, J=8.6Hz), 10.22 (1H, s).
MS (EI) m/z: 296 $(M^+)$

(108b) (9aS)-8-Acetyl-N-({4-[(4-chlorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (231 mg, 0.669 mmol), 4-[(4-chlorobenzyl)oxy]-1-naphthaldehyde produced in Example (108a) (423 mg, 1.43 mmol), triethylsilane (320 μL, 2.01 mmol), trifluoroacetic acid (155 μL, 2.01 mmol) and acetonitrile (8 mL) to give the target compound (275 mg, yield: 66%) as a yellow solid.
$^1$H-NMR ($CDCl_3$, 500MHz):δ ppm: 1.74 (3H, s), 2.64 (3H, s), 3.68 (3H, s), 4.96 (1H, dd, J=4.9, 14.7Hz), 5.02 (1H, dd, J=4.9, 14.7Hz), 5.22 (2H, s), 6.00 (1H, s), 6.26 (1H, s), 6.81 (1H, d, J=7.8Hz), 7.20 (1H, brs), 7.38-7.47 (5H, m), 7.53 (1H, m), 7.60 (1H, m), 8.06 (1H, d, J=8.3Hz), 8.39 (1H, d, J=8.3Hz), 10.65 (1H, s), 18.83 (1H, s).
MS (ESI+) m/z: 626.15677 $(M+H)^+$.

(Example 109) Methyl 4-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoate (exemplary compound No. 1-141)

(109a) 2,3,6-Trimethyl-4-[(trifluoromethyl)sulfonyl]benzaldehyde

Known 4-hydroxy-2,3,6-trimethylbenzaldehyde [Journal of Organic Chemistry, 1962, Vol. 27, p.1839-1842] (2.45 g, 14.9 mmol) was dissolved in tetrahydrofuran (100 mL). Sodium hydride (0.645 g, 26.9 mmol) was added at 0˚C, and the mixture was stirred in a nitrogen atmosphere for 30 minutes. N-Phenylbis(trifluoromethanesulfonimide) (5.32 g, 14.9 mmol) was further added, and the mixture was stirred at room temperature for three hours. After completion of the reaction, a dilute hydrochloric acid solution (150 mL) was added to the reaction solution, followed by extraction with ethyl acetate (200 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/0 to 95/5) to give the target compound (4.25 g, yield: 96%) as white crystals.
$^1$H-NMR ($CDCl_3$, 500MHz):δ ppm: 2.29 (3H, s), 2.53 (3H, s), 2.56 (3H, s), 7.01 (1H, s), 10.59 (1H, s).
MS (EI) m/z: 296 $(M^+)$

(109b) Methyl 4-formyl-2,3,5-trimethylbenzoate

2,3,6-Trimethyl-4-[(trifluoromethyl)sulfonyl]benzaldehyde produced in Example (109a) (4.20 g, 1.42 mmol) was dissolved in N,N-dimethylformamide (80 mL). Palladium acetate (0.095 g, 0.425 mmol), 1,1'-bisdiphenylphosphinoferrocene (0.472 g, 0.852 mmol), triethylamine (3.96 mL, 28.4 mmol) and methanol (11.5 mL, 0.284 mmol) were added, and the mixture was stirred in a carbon monoxide (1 atm) atmosphere at 50˚C for 1.5 hours. After cooling the reaction solution, an ammonium chloride aqueous solution (120 mL) was added, followed by extraction with ethyl acetate (120 mL). The organic layer was washed with brine and then dried over anhydrous sodium sulfate. The solvent was evaporated under reduced pressure, and the resulting residue was purified by silica gel column chromatography (elution solvent:

hexane = 100/0 to 94/6) to give the target compound (2.46 g, yield: 84%) as a yellow solid.
$^1H$-NMR ($CDCl_3$, 400MHz):δ ppm: 2.41 (3H, s), 2.49 (3H, s), 2.51 (3H, s), 3.91 (3H, s), 7.38 (1H, s), 10.60 (1H, s).
MS (EI) m/z: 206 ($M^+$)

(109c) Methyl 4-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.230 g, 0.666 mmol), methyl 4-formyl-2,3,5-trimethylbenzoate produced in Example (109b) (0.137 g, 0.666 mmol), triethylsilane (0.424 mL, 2.66 mmol), trifluoroacetic acid (0.205 mL, 2.66 mmol) and acetonitrile (15.0 mL) to give the target compound (0.212 g, yield: 59%) as a yellow solid.
$^1H$-NMR ($CDCl_3$, 400MHz):δ ppm: 1.75 (3H, s), 2.35 (3H, s), 2.42 (3H, s), 2.43 (3H, s), 2.64 (3H, s), 3.78 (3H, s), 3.89 (3H, s), 4.60-4.72 (2H, m), 6.01 (1H, s), 6.27 (1H, s), 6.81 (1H, brs), 7.43 (1H, s), 10.66 (1H, s), 18.79 (1H, s).
MS (ESI) m/z: 536.1928 $(M+H)^+$

(Example 110) (9aS)-8-Acetyl-N-{2,6-dimethyl-4-[(phenylsulfonyl)amino]benzyl}-1,7-dihydroxy-3-methoxy-9a-methyl-9-oxo-9,9a-dihydrodibenzo[b,d]furan-4-carboxamide (exemplary compound No. 1-87)

(110a) 2,6-Dimethyl-4-[(phenylsulfonyl)amino]benzaldehyde

Known 4-amino-2,6-dimethylbenzaldehyde [Australian Journal of Chemistry, 1977, Vol. 30, p.927-930] (0.180 g, 1.21 mmol) was dissolved in methylene chloride (6 mL) and pyridine (0.5 mL), and benzenesulfonyl chloride (0.170 g, 1.33 mmol) was added at 0˚C. The mixture was heated to room temperature in a nitrogen atmosphere and then stirred for 10 hours. After completion of the reaction, an ammonium chloride aqueous solution (10 mL) was added, followed by extraction with ethyl acetate (12 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 70/30 to 60/40) to give the target compound (0.184 g, yield: 53%) as a yellow powder.
$^1H$-NMR ($CDCl_3$, 400MHz):δ ppm: 2.53 (6H, s), 6.77 (2H, s), 7.47 (2H, dd, J=7.4, 8.6Hz), 7.57 (1H, t, J=7.4Hz), 7.84 (2H, d, J=8.6Hz), 10.43 (1H, s).
MS (EI) m/z: 289 ($M^+$)

(110b) (9aS)-8-Acetyl-N- {2,6-dimethyl-4-[(phenylsulfonyl)amino]benzyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.220 g, 0.637 mmol), 2,6-dimethyl-4-[(phenylsulfonyl)amino]benzaldehyde produced in Example (110a) (0.184 g, 0.637 mmol), triethylsilane (0.406 mL, 2.55 mmol), trifluoroacetic acid (0.196 mL, 2.55 mmol) and acetonitrile (15.0 mL) to give the target compound (0.112 g, yield: 28%) as a yellow solid.
$^1H$-NMR ($CDCl_3$, 400MHz):δ ppm: 1.73 (3H, s), 2.32 (6H, s), 2.63 (3H, s), 3.76 (3H, s), 4.47-4.58 (2H, m), 5.93 (1H, s), 6.27 (1H, s), 6.67-6.78 (4H, m), 7.44 (2H, dd, J=7.4, 8.6Hz), 7.53 (1H, t, J=7.4Hz), 7.80 (2H, d, J=8.6Hz), 10.63 (1H, s), 18.76 (1H, s).
MS (ESI) m/z: 619.1760 $(M+H)^+$.

(Example 111) (9aS)-8-Acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (exemplary compound No. 1-92)

(111a) 4-{[(2,4-Dichlorophenyl)sulfonyl]amino}-2,6-dimethylbenzaldehyde

Known 4-amino-2,6-dimethylbenzaldehyde [Australian Journal of Chemistry, 1977, Vol. 30, p.927-930] (0.300 g, 2.01 mmol) was dissolved in methylene chloride (6 mL) and pyridine (0.5 mL), and 2,4-dichlorobenzenesulfonyl chloride (0.543 g, 2.21 mmol) was added at 0˚C. The mixture was heated to room temperature in a nitrogen atmosphere and then stirred for one hour. After completion of the reaction, an ammonium chloride aqueous solution (10 mL) was added, followed by extraction with ethyl acetate (12 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10 to 80/20) to give the target compound

(0.247 g, yield: 34%) as a yellow powder.
$^{1}$H-NMR ($CDCl_3$, 400MHz):δ ppm: 2.52 (6H, s), 6.78 (2H, s), 7.15 (1H, brs), 7.37 (1H, dd, J=2.0, 8.6Hz), 7.49 (1H, d, J=2.0Hz), 8.04 (1H, d, J=8.6Hz), 10.41 (1H, s). MS (FAB) m/z: 357 ($M^+$)

(111b) (9aS)-8-Acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.238 g, 0.689 mmol), 4-{[(2,4-dichlorophenyl)sulfonyl]amino}-2,6-dimethylbenzaldehyde produced in Example (111a) (0.247 g, 0.689 mmol), triethylsilane (0.439 mL, 2.76 mmol), trifluoroacetic acid (0.213 mL, 2.76 mmol) and acetonitrile (15.0 mL) to give the target compound (0.152 g, yield: 32%) as a yellow solid.
$^{1}$H-NMR ($CDCl_3$, 400MHz):δ ppm: 1.73 (3H, s), 2.33 (6H, s), 2.64 (3H, s), 3.75 (3H, s), 4.44-4.56 (2H, m), 5.96 (1H, s), 6.26 (1H, s), 6.72 (1H, brs), 6.78 (2H, s), 6.94 (1H, brs), 7.33 (1H, dd, J=2.0, 8.6Hz), 7.50 (1H, d, J=2.0Hz), 7.96 (1H, d, J=8.6Hz), 10.64 (1H, s), 18.77 (1H, s).
MS (ESI) m/z: 687.1011 $(M+H)^+$

(Example 112) (9aS)-8-Acetyl-1,7-dihydroxy-3-methoxy-9a-methyl-9-oxo-N-[(4-phenoxy-1-naphthyl)methyl]-9,9a-dihydrodibenzo[b,d]furan-4-carboxamide (exemplary compound No. 1-403)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (306 mg, 0.886 mmol), known 4-phenoxy-1-naphthaldehyde [Journal of Organic Chemistry, 1995, Vol. 60, p.6592-6594] (514 mg, 2.07 mmol), triethylsilane (420 μL, 2.64 mmol), trifluoroacetic acid (210 μL, 2.73 mmol) and acetonitrile (10 mL) to give the target compound (387 mg, yield: 67%) as a yellow solid.
$^{1}$H-NMR ($CDCl_3$, 400MHz):δ ppm: 1.75 (3H, s), 2.65 (3H, s), 3.73 (3H, s), 5.02 (1H, dd, J=5.2, 14.7Hz), 5.08 (1H, dd, J=5.2, 14.7Hz), 6.01 (1H, s), 6.28 (1H, s), 6.88 (1H, d, J=7.5Hz), 7.03-7.08 (2H, m), 7.13 (1H, m), 7.20 (1H, brs), 7.33-7.38 (2H, m), 7.43 (1H, d, J=7.5Hz), 7.55 (1H, m), 7.62 (1H, m), 8.13 (1H, d, J=8.3Hz), 8.31 (1H, d, J=8.3Hz), 10.67 (1H, s), 18.83 (1H, s).
MS (ESI+) m/z: 578.17931 $(M+H)^+$

(Example 113) (9aS)-1,7-Dihydroxy-3-methoxy-9a-methyl-N-[(2-methyl-1-naphthyl)methyl]-9-oxo-8-[(1E)-N-(prop-2-ynyloxy)ethaneimidoyl]-9,9a-dihydrodibenzo[b,d]furan-4-carboxamide (exemplary compound No. 3-70)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (242 mg, 0.48 mmol) was dissolved in tetrahydrofuran:methanol (2:1, 5 mL). O-Propargylhydroxylamine hydrochloride (78 mg, 0.73 mmol) and sodium bicarbonate (61 mg, 0.73 mmol) were added at room temperature, and the mixture was stirred for four hours. The reaction solution was treated in the same manner as in Example 87 to give the title target compound (236 mg, 88%) as a yellow solid.
$^{1}$H-NMR ($CDCl_3$, 400MHz):δ ppm: 1.73 (3H, s), 2.49 (3H, s), 2.61 (1H, s), 2.63 (3H, s), 3.61 (3H, s), 4.68 (2H, s), 5.03-5.14 (2H, m), 5.93 (1H, s), 6.18 (1H, s), 7.04 (1H, m), 7.35 (1H, d, J=8.3Hz), 7.46 (1H, m), 7.55 (1H, m), 7.74 (1H, d, J=8.3Hz), 7.83 (1H, d, J=7.9Hz), 8.13 (1H, d, J=8.8Hz), 11.10 (1H, s), 15.41 (1H, s)
MS (ESI) m/z: 553 $(M+H)^+$

(Example 114) (9aS)-8-Acetyl-1,7-dihydroxy-3-methoxy-9a-methyl-9-oxo-N-({4-[(phenylsulfonyl)amino]-1-naphthyl} methyl)-9,9a-dihydrodibenzo[b,d]furan-4-carboxamide (exemplary compound No. 1-433)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (252 mg, 0.730 mmol), known N-(4-formyl-1-naphthyl)benzenesulfonamide [Journal of Organic Chemistry USSR, 1967, p. 1461-1462] (241 mg, 0.774 mmol), triethylsilane (350 μL, 2.20 mmol), trifluoroacetic acid (170 μL, 2.21 mmol) and acetonitrile (10 mL) to give the target compound (315 mg, yield: 67%) as a yellow solid.
$^{1}$H-NMR ($CDCl_3$, 500MHz):δ ppm: 1.73 (3H, s), 2.64 (3H, s), 3.74 (3H, s), 5.01 (1H, dd, J=5.2, 15.2Hz), 5.07 (1H, dd, J=5.2, 15.2Hz), 5.90 (1H, s), 6.29 (1H, s), 6.95 (1H, s), 7.30-7.54 (8H, m), 7.76 (2H, d, J=7.3z), 7.86 (1H, d, J=8.3Hz), 8.06 (1H, d, J=8.3Hz), 10.68 (1H, s), 18.80 (1H, s).
MS (ESI+) m/z: 641.16307 $(M+H)^+$

(Example 115) (9aS)-8-Acetyl-1,7-dihydroxy-3-methoxy-9a-methyl-9-oxo-N-{[4-(phenylthio)-1-naphthyl]methyl}-9,9a-dihydrodibenzo[b,d]furan-4-carboxamide (exemplary compound No. 1-417)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (299 mg, 0.886 mmol), known 4-(phenylthio)-1-naphthaldehyde [Journal of Organic Chemistry, 1995, Vol. 60, p.6592-6594] (459 mg, 1.74 mmol), triethylsilane (420 μL, 2.64 mmol), trifluoroacetic acid (210 μL, 2.73 mmol) and acetonitrile (10 mL) to give the target compound (374 mg, yield: 73%) as a yellow solid.
$^1$H-NMR ($CDCl_3$, 500MHz):δ ppm: 1.75 (3H, s), 2.65 (3H, s), 3.75 (3H, s), 5.07 (1H, dd, J=5 .4, 15 .2Hz), 5.13 (1H, dd, J=5.4, 15.2Hz), 6.01 (1H, s), 6.29 (1H, s), 7.16-7.25 (5H, m), 7.36 (1H, brs), 7.49 (1H, d, J=7.3Hz), 7.55-7.62 (3H, m), 8.15 (1H, d, J=8.3Hz), 8.46 (1H, d, J=8.3Hz), 10.70 (1H, s), 18.83 (1H, s).
MS (ESI+) m/z: 594.16004 $(M+H)^+$.

(Example 116) (9aS)-8-Acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-437)

(116a) 2-Ethyl-3-methyl-1-naphthaldehyde

N,N,N'-Trimethylethylenediamine (3.3 mL, 25.4 mmol) was dissolved in tetrahydrofuran (200 mL), and butyllithium (2.71 M solution in hexane, 9.3 mL, 25.2 mmol) was added dropwise at -20˚C. After stirring for 15 minutes, 2,3-dimethyl-1-naphthaldehyde (4.22 g, 22.9 mmol) was dissolved in tetrahydrofuran (50 mL), and the mixture was stirred for 15 minutes. After cooling to -78˚C, butyllithium (2.71 M solution in hexane, 13.0 mL, 35.2 mmol) was added dropwise, and the mixture was stirred with heating to -40˚C for 80 minutes. Iodomethane (10.0 mL, 161 mmol) was further added, and the mixture was heated to room temperature and stirred for three hours. 1 N hydrochloric acid (200 mL) was added to the reaction solution, followed by extraction with diethyl ether (400 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give the target compound (2.70 g, yield: 59%) as a light yellow solid.
$^1$H-NMR ($CDCl_3$, 400MHz):δ ppm: 1.30 (3H, t, J=7.4Hz), 2.53 (3H, s), 3.13 (2H, q, J=7.4Hz), 7.45 (1H, m), 7.52 (1H, m), 7.73 (1H, brd, J=8.6Hz), 7.82 (1H, s), 8.80 (1H, d, J=7.8Hz), 10.92 (1H, s).
MS (EI) m/z: 198 $(M^+)$.

(116b) (9aS)-8-Acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (150 mg, 0.434 mmol), 2-ethyl-3-methyl-1-naphthaldehyde produced in Example (116a) (100 mg, 0.505 mmol), triethylsilane (210 μL, 1.30 mmol), trifluoroacetic acid (100 μL, 1.30 mmol) and acetonitrile (7 mL) to give the target compound (155 mg, yield: 68%) as a yellow solid.
$^1$H-NMR ($CDCl_3$, 400MHz):δ ppm: 1.26 (3H, t, J=7.1Hz), 1.74 (3H, s), 2.52 (3H, s), 2.63 (3H, s), 2.97 (2H, m), 3.59 (3H, s), 5.05 (1H, dd, J=14.3, 4.7Hz), 5.12 (1H, dd, J=14.3, 4.7Hz), 5.99 (1H, s), 6.20 (1H, s), 7.02 (1H, m), 7.49-7.38 (2H, m), 7.62 (1H, brs), 7.74 (1H, brd, J=7.9Hz), 8.03 (1H, brd, J=8.7Hz), 10.60 (1H, s), 18.78 (1H, s). MS (ESI) m/z: 528.20151 $(M+H)^+$.

(Example 117) [({(1E)-1-[(9bS)-3,9-Dihydroxy-7-methoxy-9b-methyl-6-({[(2-methyl-1-naphthyl)methyl]amino}carbonyl)-1-oxo-1,9b-dihydrodibenzo[b,d]furan-2-yl]ethylidene}amino)oxy]acetic acid (exemplary compound No. 3-92)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (235 mg, 0.47 mmol) was dissolved in tetrahydrofuran:methanol (2:1, 5 mL). Carboxymethoxylamine 1/2 hydrochloride (77 mg, 0.71 mmol) and sodium bicarbonate (30 mg, 0.35 mmol) were added at room temperature, and the mixture was stirred for four hours. The reaction solution was treated in the same manner as in Example 87 to give the title target compound (262 mg, 97%) as a yellow solid.
$^1$H-NMR ($CDCl_3$, 400MHz):δ ppm: 1.73 (3H, s), 2.51 (3H, s), 2.62 (3H, s), 3.58 (3H, s), 4.70 (2H, s), 5.03-5.14 (2H, m), 6.03 (1H, s), 6.18 (1H, s), 7.36 (1H, d, J=8.3Hz), 7.41-7.58 (4H, m), 7.76 (1H, d, J=8.4Hz), 7.85 (1H, d, J=8.3Hz), 8.09 (1H, d, J=8.7Hz), 11.23 (1H, s)
MS (ESI) m/z: 573 $(M+H)^+$

(Example 118) (9aS)-1,7-Dihydroxy-3-methoxy-9a-methyl-N-[(2-methyl-1-naphthyl)methyl]-9-oxo-8-[(1E)-N-(2-oxopropoxy)ethaneimidoyl]-9,9a-dihydrodibenzo[b,d]furan-4-carboxamide (exemplary compound No. 3-96)

(118a) N-[(3,5-Dioxahex-1-enyl)-2-methoxy]-phthalimide

N-Hydroxyphthalimide (654 mg, 4.01 mmol) was dissolved in N,N-dimethylformamide (10 mL). Triethylamine (0.67 mL, 4.81 mmol), 2-(chloromethyl)-3,5-dioxahex-1-ene (0.60 mL, 4.81 mmol) and tetrabutylammonium iodide (296 mg, 0.80 mmol) were added at room temperature, and the mixture was stirred at 80˚C for two hours. The reaction solution was separated with ethyl acetate (20 mL) and water (20 mL). The organic layer was washed with brine (20 mL) and dried over anhydrous sodium sulfate. The solvent was evaporated under reduced pressure, and the residue was purified by silica gel column chromatography (hexane:ethyl acetate = 3:1, V/V) to give the title target compound (891 mg, 84%) as a colorless oil.
$^1$H-NMR ($CDCl_3$, 400MHz):δ ppm: 3.47 (3H, s), 4.36 (1H, d, J=2.4Hz), 4.53 (1H, d, J=2.4Hz), 4.62 (2H, s), 5.02 (2H, s), 7.74-7.76 (2H, m), 7.83-7.85 (2H, m).
MS (FAB) m/z: 264 $(M+H)^+$

(118b) (9aS)-1,7-Dihydroxy-3-methoxy-9a-methyl-8-[(1E)-1-methyl-5-methylene-3,6,8-trioxa-2-azanon-1-en-1-yl]-N-[(2-methyl-1-naphthyl)methyl]-9-oxo-9,9a-dihydrodibenzo[b,d]furan-4-carboxamide

N-[(3,5-Dioxahex-1-enyl)-2-methoxy]-phthalimide synthesized in Example (118a) (597 mg, 2.27 mmol) was dissolved in ethanol (8 mL). Butylamine (0.21 mL, 2.15 mmol) was added at room temperature, and the mixture was stirred at 60˚C for three hours. The reaction solution was separated with ethyl acetate (20 mL) and water (20 mL). The organic layer was washed with brine (20 mL) and dried over anhydrous sodium sulfate. The solvent was evaporated under reduced pressure, and the resulting oily compound (300 mg) was dissolved in tetrahydrofuran:methanol (2:1, 6 mL). (9aS)-8-Acetyl-1,7-dihydroxy-3-methoxy-9a-methyl-N-[(2-methyl-1-naphthyl)methyl]-9-oxo-9,9a-dihydrodibenzo [b,d]furan-4-carboxamide produced in Example 27 (297 mg, 0.59 mmol) was added at room temperature, and the mixture was stirred for four hours. The reaction solution was separated with ethyl acetate (15 mL) and 0.5 N hydrochloric acid (15 mL). The organic layer was washed with brine (15 mL) and dried over anhydrous sodium sulfate. The solvent was evaporated under reduced pressure, and the residue was purified by silica gel column chromatography (methylene chloride:methanol = 80:1, V/V) and reverse phase silica gel column chromatography (acetonitrile:water = 2:1, V/V) to give the title target compound (272 mg, 74%) as a yellow solid.
$^1$H-NMR ($CDCl_3$, 400MHz):δ ppm: 1.71 (3H, s), 2.53 (3H, s), 2.63 (3H, s), 3.44 (3H, s), 3.61 (3H, s), 4.43 (1H, s), 4.48 (2H, s), 4.56 (1H, s), 5.04 (2H, s), 5.02-5.15 (2H, m), 5.90 (1H, s), 6.18 (1H, s), 7.03 (1H, m), 7.35 (1H, d, J=8.4Hz), 7.46 (1H, m), 7.55 (1H, m), 7.75 (1H, d, J=8.3Hz), 7.84 (1H, d, J=8.3Hz), 8.13 (1H, d, J=8.3Hz), 11.23 (1H, s), 15.73 (1H, s)
MS (FAB) m/z: 615 $(M+H)^+$

(118c) (9aS)-1,7-Dihydroxy-3-methoxy-9a-methyl-N-[(2-methyl-1-naphthyl)methyl]-9-oxo-8-[(1E)-N-(2-oxopropoxy) ethaneimidoyl]-9,9a-dihydrodibenzo[b,d]furan-4-carboxamide

(9aS)-1,7-Dihydroxy-3-methoxy-9a-methyl-8-[(1E)-1-methyl-5-methylene-3,6,8-trioxa-2-azanon-1-en-1-yl]-N-[(2-methyl-1-naphthyl)methyl]-9-oxo-9,9a-dihydrodibenzo[b,d]furan-4-carboxamide synthesized in Example (118b) (272 mg, 0.44 mmol) was dissolved in tetrahydrofuran (3 mL). 0.2 N sulfuric acid (3 mL) was added at room temperature, and the mixture was stirred for one hour. The reaction solution was separated with ethyl acetate (10 mL) and water (10 mL). The organic layer was washed with brine (10 mL) and dried over anhydrous sodium sulfate. The solvent was evaporated under reduced pressure, and the residue was purified by silica gel column chromatography (methylene chloride:methanol = 80:1, V/V) and reverse phase silica gel column chromatography (acetonitrile:water = 8:5 to 2:1, V/V) to give the title target compound (134 mg, 53%) as a yellow solid.
$^1$H-NMR ($CDCl_3$, 400MHz):δ ppm: 1.72 (3H, s), 2.21 (3H, s), 2.51 (3H, s), 2.62 (3H, s), 3.60 (3H, s), 4.64 (2H, s), 5.01-5.13 (2H, m), 5.90 (1H, s), 6.17 (1H, s), 7.01 (1H, m), 7.33 (1H, d, J=8.3Hz), 7.44 (1H, m), 7.53 (1H, m), 7.73 (1H, d, J=8.3Hz), 7.82 (1H, d, J=7.9Hz), 8.10 (1H, d, J=8.3Hz), 11.00 (1H, s), 14.72 (1H, brs)
MS (ESI) m/z: 571 $(M+H)^+$.

(Example 119) (9aS)-8-Acetyl-N-{4-[(2-butynyloxy)methyl]-2,3,6-trimethylbenzyl}-1,7-dihydroxy-3-methoxy-9a-methyl-9-oxo-9,9a-dihydrodibenzo[b,d]furan-4-carboxamide (exemplary compound No. 1-139)

(119a) Methyl 4-(1,3-dioxan-2-yl)-2,3,5-trimethylbenzoate

Methyl 4-formyl-2,3,5-trimethylbenzoate produced in Example (109b) (1.40 g, 6.79 mmol) was dissolved in

toluene (50 mL). 1,3-Propanediol (0.981 mL, 13.6 mmol) and p-toluenesulfonic acid monohydrate (0.117 g, 0.679 mmol) were added, and the mixture was stirred with heating to reflux using a Dean-Stark water separator in a nitrogen atmosphere for two hours. The reaction solution was cooled to room temperature and then a saturated sodium bicarbonate aqueous solution (100 mL) was added, followed by extraction with ethyl acetate (80 mL). The organic layer was washed with brine and then dried over anhydrous sodium sulfate. The solvent was evaporated under reduced pressure and the residue was dried to give the target crude product (1.79 g) as a colorless oil.

(119b) [4-(1,3-Dioxan-2-yl)-2,3,5-trimethylphenyl]methanol

Methyl 4-(1,3-dioxan-2-yl)-2,3,5-ftimethylbenzoate produced in Example (119a) (1.79 g) was dissolved in tetrahydrofuran (50 mL), and lithium aluminum hydride (0.309 g, 8.15 mmol) was slowly added at 0˚C. After stirring in a nitrogen atmosphere at 0˚C for 30 minutes, water (0.3 mL) and a 15% sodium hydroxide aqueous solution (0.3 mL) were added to the reaction solution, and the mixture was stirred at room temperature for one hour. Water (0.9 mL) was added again, and then the mixture was filtered through Celite. After washing with ethyl acetate (50 mL), the solvent was evaporated from the resulting filtrate under reduced pressure and the residue was dried to give the target crude product (1.60 g) as a colorless oil.

(119c) 4-(Hydroxymethyl)-2,3,6-trimethylbenzaldehyde

[4-(1,3-Dioxan-2-yl)-2,3,5-trimethylphenyl]methanol produced in Example (119b) (1.60 g) was dissolved in a 80% acetic acid aqueous solution (50 mL), and the mixture was stirred at 60˚C for one hour. After completion of the reaction, the reaction solution was cooled to room temperature and then a saturated sodium bicarbonate aqueous solution (100 mL) was added, followed by extraction with ethyl acetate (80 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 80/20 to 50/50) to give the target compound (1.15 g, yield: 95%, three steps) as white crystals.
$^1$H-NMR ($CDCl_3$, 500MHz):δ ppm: 2.24 (3H, s), 2.50 (3H, s), 2.55 (3H, s), 4.72 (2H, s), 7.15 (1H, s), 10.63 (1H, s).
MS(EI)m/z: 178 ($M^+$)

(119d) 4-[(2-Butynyloxy)methyl]-2,3,6-trimethylbenzaldehyde

4-(Hydroxymethyl)-2,3,6-trimethylbenzaldehyde produced in Example (119c) (0.200 g, 1.12 mmol) was dissolved in N,N-dimethylformamide (8 mL). Sodium hydride (0.40 g, 1.68 mmol) was added at 0˚C, and the mixture was stirred in a nitrogen atmosphere for 30 minutes. 1-Bromo-2-butyne (0.152 mL, 5.48 mmol) was further added, and the mixture was stirred at 60˚C for two hours. After completion of the reaction, a dilute hydrochloric acid solution (30 mL) was added to the reaction solution, followed by extraction with ethyl acetate (40 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.189 g, yield: 73%) as yellow crystals.
$^1$H-NMR ($CDCl_3$, 400MHz):δ ppm: 1.89 (3H, t, J=2.4Hz), 2.24 (3H, s), 2.49 (3H, s), 2.53 (3H, s), 4.17 (2H, q, J=2.4Hz), 4.56 (2H, s), 7.09 (1H, s), 10.59 (1H, s).
MS (EI) m/z: 230 ($M^+$)

(119e) (9aS)-8-Acetyl-N-{4-[(2-butynyloxy)methyl]-2,3,6-trimethylbenzyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.280 g, 0.811 mmol), 4-[(2-butynyloxy)methyl]-2,3,6-trimethylbenzaldehyde produced in Example (119d) (0.189 g, 0.821 mmol), triethylsilane (0.516 mL, 3.24 mmol), trifluoroacetic acid (0.250 mL, 3.24 mmol) and acetonitrile (15.0 mL) to give the target compound (0.200 g, yield: 44%) as a yellow solid.
$^1$H-NMR ($CDCl_3$, 500MHz):δ ppm: 1.75 (3H, s), 1.89 (3H, t, J=2.4Hz), 2.26 (3H, s), 2.33 (3H, s), 2.40 (3H, s), 2.65 (3H, s), 3.79 (3H, s), 4.16 (2H, q, J=2.4Hz), 4.55 (2H, s), 4.60-4.69 (2H, m), 6.02 (1H, s), 6.28 (1H, s), 6.71 (1H, brs), 7.06 (1H, s), 10.65 (1H, s), 18.83 (1H, s).
MS (ESI) m/z: 560.2272 $(M+H)^+$

(Example 120) (9aS)-8-Acetyl-N-[4-(benzoylamino)-2,3-dimethylbenzyl]-1,7-dihydroxy-3-methoxy-9a-methyl-9-oxo-9,9a-dihydrodibenzo[b,d]furan-4-carboxamide (exemplary compound No. 1-83)

(120a) 4-(Benzoylamino)-2,3-dimethylbenzaldehyde

Known N-(3,5-dimethylphenyl)benzamide [Journal of Heterocyclic Chemistry, 2002, Vol. 39, p.965-973] (0.929 g, 4.13 mmol) was dissolved in methylene chloride (30 mL), and titanium chloride (0.996 mL, 9.09 mmol) and dichloromethyl methyl ether (0.441 mL, 4.54 mmol) were added in a nitrogen atmosphere at -40˚C. After stirring at room temperature for 30 minutes, water (50 mL) was added, followed by extraction with methylene chloride (40 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.303 g, yield: 30%) as a white solid. $^1$H-NMR ($CDCl_3$, 400MHz):δ ppm: 2.64 (6H, s), 7.42 (2H, s), 7.49 (2H, t, J=7.0Hz), 7.57 (1H, t, J=7.0Hz), 7.84 (1H, brs), 7.85 (2H, d, J=7.0Hz), 10.51 (1H, s).
MS (EI) m/z: 253 ($M^+$)

(120b) (9aS)-8-Acetyl-N-[4-(benzoylamino)-2,3-dimethylbenzyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), 4-(benzoylamino)-2,3-dimethylbenzaldehyde produced in Example (120a) (0.300 g, 1.18 mmol), triethylsilane (0.738 mL, 4.63 mmol), trifluoroacetic acid (0.357 mL, 4.63 mmol) and acetonitrile (20.0 mL) to give the target compound (0.470 g, yield: 70%) as a yellow solid.
$^1$H-NMR ($CDCl_3$, 400MHz):δ ppm: 1.74 (3H, s), 2.43 (6H, s), 2.64 (3H, s), 3.79 (3H, s), 4.55-4.66 (2H, m), 5.97 (1H, s), 6.27 (1H, s), 6.76 (1H, brs), 7.36 (2H, s), 7.45-7.49 (2H, m), 7.52-7.56 (2H, m), 7.82-7.86 (3H, m), 10.62 (1H, s), 18.77 (1H, s). MS (ESI) m/z: 583.2099 $(M+H)^+$.

(Example 121) (9aS)-8-Acetyl-N-{4-[(2,4-dichlorobenzyl)oxy]-2,6-dimethylbenzyl}-1,7-dihydroxy-3-methoxy-9a-methyl-9-oxo-9,9a-dihydrodibenzo[b,d]furan-4-carboxamide (exemplary compound No. 1-81)

(9aS)-8-Acetyl-1,7-dihydroxy-N-(4-hydroxy-2,6-dimethylbenzyl)-3-methoxy-9a-methyl-9-oxo-9,9a-dihydrodibenzo[b,d]furan-4-carboxamide produced in Example (97c) (0.250 g, 0.521 mmol) was dissolved in N,N-dimethylformamide (5 mL). Potassium carbonate (0.288 g, 2.08 mmol) and 2,4-dichlorobenzyl chloride (0.217 mL, 1.56 mmol) were added, and the mixture was stirred in a nitrogen atmosphere at 60˚C for 12 hours. After completion of the reaction, the reaction solution was cooled to 0˚C. 0.1 N hydrochloric acid (10 mL) was added, followed by extraction with ethyl acetate (10 mL). The organic layer was washed with brine and then dried over anhydrous sodium sulfate. The solvent was evaporated under reduced pressure, and the resulting residue was purified by silica gel column chromatography (elution solvent: methylene chloride/methanol = 80/1 to 40/1) to give the target compound (0.205 g, yield: 62%) as a yellow solid.
$^1$H-NMR ($CDCl_3$, 400MHz):δ ppm: 1.74 (3H, s), 2.40 (6H, s), 2.64 (3H, s), 3.79 (3H, s), 4.51-4.62 (2H, m), 5.08 (2H, s), 6.00 (1H, s), 6.27 (1H, s), 6.67 (2H, s), 6.69 (1H, brs), 7.26 (1H, d, J=8.7Hz), 7.40 (1H, s), 7.48 (1H, d, J=8.7Hz), 10.62 (1H, s), 18.78 (1H, s).
MS (ESI) m/z: 638.1334 $(M+H)^+$

(Example 122) 2,4-Dichlorophenyl 4-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoate (exemplary compound No. 1-153)

(122a) 4-({[({9aS}-8-Acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoic acid

Methyl 4-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoate produced in Example (109c) (1.60 g, 2.99 mmol) was dissolved in a 1 N sodium hydroxide aqueous solution (35 mL), and the mixture was stirred at room temperature for 30 minutes. The reaction solution was cooled to 0˚C, and a dilute hydrochloric acid solution (70 mL) was slowly added dropwise. The precipitated product was filtered and dried to give the target compound (1.56 g, yield: 100%) as a yellow solid.
$^1$H-NMR ($CDCl_3$, 500MHz):δ ppm: 1.75 (3H, s), 2.38 (3H, s), 2.45 (3H, s), 2.53 (3H, s), 2.65 (3H, s), 3.80 (3H, s), 4.64-4.75 (2H, m), 6.03 (1H, s), 6.30 (1H, s), 6.87 (1H, brs), 7.64 (1H, s), 10.69 (1H, s), 18.83 (1H, s).
MS (ESI) m/z: 522.1761 $(M+H)^+$

(122b) 2,4-Dichlorophenyl 4-({[({9aS}-8-acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoate

4-({[({9aS}-8-Acetyl-1,7-dihydroxy-3-methoxy-9a-methyl-9-oxo-9,9a-dihydrodibenzo[b,d]furan-4-yl)carbonyl]amino}methyl)-2,3,5-trimethylbenzoic acid produced in Example (122a) (0.400 g, 0.767 mmol) was dissolved in N,N-dimethylformamide (10 mL). 1-Ethyl-3-(3'-dimethylaminopropyl)carbodiimide hydrochloride (0.294 g, 1.53 mmol), 4-dimethylaminopyridine (0.019 g, 0.153 mmol) and 2,4-dichlorophenol (0.313 g, 1.92 mmol) were added, and the mixture was stirred in a nitrogen atmosphere at room temperature for 12 hours. After completion of the reaction, the reaction solution was cooled to 0˚C, and a 0.1 N hydrochloric acid aqueous solution (20 mL) was slowly added dropwise. The precipitated product was filtered and purified by silica gel column chromatography (elution solvent: methylene chloride/methanol = 80/1 to 40/1) to give the target compound (0.150 g, yield: 30%) as a yellow solid.
$^1$H-NMR ($CDCl_3$, 400MHz):δ ppm: 1.76 (3H, s), 2.41 (3H, s), 2.50 (3H, s), 2.55 (3H, s), 2.65 (3H, s), 3.82 (3H, s), 4.67-4.77 (2H, m), 6.03 (1H, s), 6.30 (1H, s), 6.91 (1H, brs), 7.21 (1H, d, J=8.3Hz), 7.32 (1H, d, J=8.3Hz), 7.52 (1H, s), 7.82 (1H, s), 10.70 (1H, s), 18.83 (1H, s).
MS (ESI) m/z: 666.1284 $(M+H)^+$

(Example 123) Methyl [({(1E)-1-[(9bS)-3,9-dihydroxy-7-methoxy-9b-methyl-6-({[(2-methyl-1-naphthyl)methyl]amino}carbonyl)-1-oxo-1,9b-dihydrodibenzo[b,d]furan-2-yl]ethylidene}amino)oxy]acetate (exemplary compound No. 3-100)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (259 mg, 0.52 mmol) was dissolved in tetrahydrofuran:methanol (2:1, 5 mL). Methyl (aminooxy)acetate hydrochloride (110 mg, 0.78 mmol) and sodium bicarbonate (48 mg, 0.57 mmol) were added at room temperature, and the mixture was stirred for four hours. The reaction solution was treated in the same manner as in Example 87 to give the title target compound (234 mg, 91 %) as a yellow solid.
$^1$H-NMR ($CDCl_3$, 400MHz):δ ppm: 1.72 (3H, s), 2.49 (3H, s), 2.63 (3H, s), 3.60 (3H, s), 3.82 (3H, s), 4.64 (2H, s), 5.01-5.13 (2H, m), 5.91 (1H, s), 6.17 (1H, s), 7.02 (1H, m), 7.33 (1H, d, J=8.3Hz), 7.44 (1H, m), 7.53 (1H, m), 7.73 (1H, d, J=8.3Hz), 7.82 (1H, d, J=7.9Hz), 8.10 (1H, d, J=8.7Hz), 11.01 (1H, s), 14.73 (1H, s).
MS (ESI) m/z: 587 $(M+H)^+$

(Example 124) (9aS)-1,7-dihydroxy-8-[(lE)-N-(2-hydroxyethoxy)ethaneimidoyl]-3-methoxy-9a-methyl-N-[(2-methyl-1-naphthyl)methyl]-9-oxo-9,9a-dihydrodibenzo[b,d]furan-4-carboxamide (exemplary compound No. 3-88)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (218 mg, 0.44 mmol) was dissolved in tetrahydrofuran:methanol (2:1, 5 mL). O-(2-Hydroxyethyl)-hydroxylamine (50 mg, 0.65 mmol) was added at room temperature, and the mixture was stirred for four hours. The reaction solution was treated in the same manner as in Example 87 to give the title target compound (200 mg, 82%) as a yellow solid.
$^1$H-NMR ($CDCl_3$, 400MHz):δ ppm: 1.71 (3H, s), 2.55 (3H, s), 2.63 (3H, s), 3.60 (3H, s), 3.96 (2H, m), 4.23 (2H, m), 5.01-5.13 (2H, m), 5.88 (1H, s), 6.16 (1H, s), 7.03 (1H, m), 7.34 (1H, d, J=8.0Hz), 7.44 (1H, m), 7.53 (1H, m), 7.73 (1H, d, J=8.7Hz), 7.82 (1H, d, J=7.9Hz), 8.11 (1H, d, J=8.3Hz), 11.16 (1H, s), 15.63 (1H, s).
MS (ESI) m/z: 559 $(M+H)^+$

(Example 125) (9aS)-N-[4-(But-2-ynyloxy)-2,3,6-trimethylbenzyl]-1,7-dihydroxy-3-methoxy-8-[(1E)-N-methoxyethaneimidoyl]-9a-methyl-9-oxo-9,9a-dihydrodibenzo[b,d]furan-4-carboxamide (exemplary compound No. 3-14)

(9aS)-8-Acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide produced in Example (102b) (208 mg, 0.38 mmol) was dissolved in tetrahydrofuran:methanol (2:1, 5 mL). O-Methylhydroxylamine hydrochloride (48 mg, 0.57 mmol) and sodium bicarbonate (48 mg, 0.57 mmol) were added at room temperature, and the mixture was stirred for four hours. The reaction solution was treated in the same manner as in Example 87 to give the title target compound (167 mg, 76%) as a yellow solid.
$^1$H-NMR ($CDCl_3$, 400MHz):δ ppm: 1.71 (3H, s), 1.86 (3H, s), 2.17 (3H, s), 2.30 (3H, s), 2.41 (3H, s), 2.54 (3H, s), 3.76 (3H, s), 3.92 (3H, s), 4.54-4.63 (4H, m), 5.88 (1H, s), 6.21 (1H, s), 6.62 (1H, m), 6.67 (1H, s), 11.16 (1H, s), 15.81 (1H, brs)
MS (ESI) m/z: 575 $(M+H)^+$.

(Example 126) (9aS)-8-[(1E)-N-(allyloxy)ethaneimidoyl]-N-[4-(but-2-ynyloxy)-2,3,6-trimethylbenzyl]-1,7-dihydroxy-3-methoxy-9a-methyl-9-oxo-9,9a-dihydrodibenzo[b,d]furan-4-carboxamide (exemplary compound No. 3-44)

(9aS)-8-Acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide produced in Example (102b) (210 mg, 0.38 mmol) was dissolved in tetrahydrofuran:methanol (2:1,5 mL). O-Allylhydroxylamine hydrochloride (63 mg, 0.58 mmol) and sodium bicarbonate (49 mg, 0.58 mmol) were added at room temperature, and the mixture was stirred for four hours. The reaction solution was treated in the same manner as in Example 87 to give the title target compound (159 mg, 69%) as a yellow solid. $^1$H-NMR ($CDCl_3$, 400MHz):δ ppm: 1.63 (3H, s), 1.72 (3H, s), 1.87 (3H, s), 2.17 (3H, s), 2.31 (3H, s), 2.41 (3H, s), 2.56 (3H, s), 3.77 (3H, s), 4.54-4.63 (6H, m), 5.41-5.48 (2H, m), 5.87 (1H, s), 6.00 (1H, m), 6.21 (1H, s), 6.63 (1H, m), 6.68 (1H, s), 11.20 (1H, s), 15.84 (1H, brs)
MS (ESI) m/z: 601 $(M+H)^+$

(Example 127) (9aS)-8-Acetyl-N-{4-[(2,4-dichlorobenzoyl)amino]-2,6-dimethylbenzyl}-1,7-dihydroxy-3-methoxy-9a-methyl-9-oxo-9,9a-dihydrodibenzo[b,d]furan-4-carboxamide (exemplary compound No. 1-86)

(127a) 4-[(2,4-Dichlorobenzoyl)amino]-2,6-dimethylbenzaldehyde

2,4-Dichloro-N-(3,5-dimethylphenyl)benzamide (1.66 g, 5.64 mmol) was dissolved in methylene chloride (60 mL). Titanium chloride (1.36 mL, 12.4 mmol) and dichloromethyl methyl ether (0.561 mL, 6.20 mmol) were added in a nitrogen atmosphere at -78˚C. After stirring at room temperature for 12 hours, the reaction solution was cooled and water (100 mL) was added, followed by extraction with methylene chloride (60 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10 to 70/30) to give the target compound (0.380 g, yield: 21%) as a white solid.
$^1$H-NMR ($CDCl_3$, 500MHz): δ ppm: 2.65 (6H, s), 7.39 (1H, t, J=8.3Hz), 7.40 (2H, s), 7.51 (1H, s), 7.75 (1H, d, J=8.3Hz), 7.95 (1H, brs), 10.55 (1H, s).
MS (EI) m/z: 321 $(M^+)$
(127b)
Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), 4-[(2,4-dichlorobenzoyl)amino]-2,6-dimethylbenzaldehyde produced in Example (127a) (0.380 g, 1.18 mmol), triethylsilane (0.738 mL, 4.63 mmol), trifluoroacetic acid (0.357 mL, 4.63 mmol) and acetonitrile (20.0 mL) to give the target compound (0.280 g, yield: 37%) as a yellow solid.
$^1$H-NMR ($CDCl_3$, 400MHz):δ ppm: 1.75 (3H, s), 2.44 (6H, s), 2.64 (3H, s), 3.80 (3H, s), 4.56-4.66 (2H, m), 5.99 (1H, s), 6.28 (1H, s), 6.78 (1H, brs), 7.34 (2H, s), 7.35 (1H, d, J=8.3Hz), 7.46 (1H, s), 7.70 (1H, d, J=8.3Hz), 7.83 (1H, brs), 10.64 (1H, s), 18.77 (1H, s).
MS (ESI) m/z: 651.1312 $(M+H)^+$

(Example 128) (9aS)-8-Acetyl-1,7-dihydroxy-3-methoxy-9a-methyl-9-oxo-N-[2,3,6-trimethyl-4-(phenoxymethyl)benzyl]-9,9a-dihydrodibenzo[b,d]furan-4-carboxamide (exemplary compound No. 1-138)

(128a) 2,3,6-Trimethyl-4-(phenoxymethyl)benzaldehyde

4-(Hydroxymethyl)-2,3,6-trimethylbenzaldehyde produced in Example (119c) (0.250 g, 1.40 mmol), phenol (0.123 g, 1.40 mmol) and triphenylphosphine (0.367 g, 1.40 mmol) were dissolved in tetrahydrofuran (10 mL). Diethyl azodicarboxylate (0.243 mL, 1.54 mmol) was slowly added dropwise at 0˚C, and the mixture was stirred in a nitrogen atmosphere at room temperature for two hours. After completion of the reaction, the solvent was evaporated under reduced pressure. The resulting residue was purified by silica gel column chromatography (elution solvent: hexane/ethyl acetate = 95/5) to give the target compound (0.320 g, yield: 90%) as white crystals.
$^1$H-NMR ($CDCl_3$, 400MHz):δ ppm: 2.27 (3H, s), 2.52 (3H, s), 2.54 (3H, s), 5.02 (2H, s), 6.95-7.00 (3H, m), 7.18 (1H, s), 7.30 (2H, t, J=8.0Hz), 10.61 (1H, s).
MS (EI) m/z: 254 $(M^+)$

(128b) (9aS)-8-Acetyl-1,7-dihydroxy-3-methoxy-9a-methyl-9-oxo-N-[2,3,6-trimethyl-4-(phenoxymethyl)benzyl]-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), 2,3,6-trimethyl-4-(phenoxymethyl)benzaldehyde produced in Example (128a) (0.320 g, 1.26 mmol), triethylsilane (0.738 mL, 4.63 mmol), trifluoroacetic acid (0.357 mL, 4.63 mmol) and acetonitrile (20.0 mL) to give the target compound (0.404 g, yield: 60%) as a yellow solid. $^1$H-NMR ($CDCl_3$, 400MHz):δ ppm: 1.75 (3H, s), 2.27 (3H, s), 2.36

(3H, s), 2.41 (3H, s), 2.64 (3H, s), 3.79 (3H, s), 4.61-4.71 (2H, m), 4.99 (2H, s), 6.01 (1H, s), 6.28 (1H, s), 6.73 (1H, brs), 6.95-7.00 (3H, m), 7.13 (1H, s), 7.28-7.32 (2H, m), 10.63 (1H, s), 18.78 (1H, s).
MS (ESI) m/z: 584.2282 $(M+H)^+$

(Example 129) (9aS)-8-Acetyl-N-{4-[(2,4-dichlorophenoxy)methyl]-2,3,6-trimethylbenzyl}-1,7-dihydroxy-3-methoxy-9a-methyl-9-oxo-9,9a-dihydrodibenzo[b,d]furan-4-carboxamide (exemplary compound No. 1-147)

(129a) 4-[(2,4-Dichlorophenoxy)methyl]-2,3,6-trimethylbenzaldehyde

4-(Hydroxymethyl)-2,3,6-trimethylbenzaldehyde produced in Example (119c) (0.250 g, 1.40 mmol), 2,4-dichlorophenol (0.229 g, 1.40 mmol) and triphenylphosphine (0.367 g, 1.40 mmol) were dissolved in tetrahydrofuran (10 mL). Diethyl azodicarboxylate (0.243 mL, 1.54 mmol) was slowly added dropwise at 0˚C, and the mixture was stirred in a nitrogen atmosphere at room temperature for two hours. After completion of the reaction, the solvent was evaporated under reduced pressure. The resulting residue was purified by silica gel column chromatography (elution solvent: hexane/ethyl acetate = 95/5) to give the target compound (0.448 g, yield: 99%) as white crystals.
$^1$H-NMR ($CDCl_3$, 500MHz):δ ppm: 2.28 (3H, s), 2.53 (3H, s), 2.55 (3H, s), 5.09 (2H, s), 6.91 (1H, d, J=8.8Hz), 7.19 (1H, dd, J=2.4, 8.8Hz), 7.22 (1H, s), 7.41 (1H, d, J=2.4 Hz), 10. 64 (1H, s).
MS (EI) m/z: 322 $(M^+)$

(129b) (9aS)-8-Acetyl-N-{4-[(2,4-dichlorophenoxy)methyl]-2,3,6-trimethylbenzyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), 4-[(2,4-dichlorophenoxy)methyl]-2,3,6-trimethylbenzaldehyde produced in Example (129a) (0.440 g, 1.36 mmol), triethylsilane (0.738 mL, 4.63 mmol), trifluoroacetic acid (0.357 mL, 4.63 mmol) and acetonitrile (20.0 mL) to give the target compound (0.519 g, yield: 69%) as a yellow solid.
$^1$H-NMR ($CDCl_3$, 400MHz):δ ppm: 1.75 (3H, s), 2.28 (3H, s), 2.36 (3H, s), 2.42 (3H, s), 2.64 (3H, s), 3.79 (3H, s), 4.61-4.71 (2H, m), 5.04 (2H, s), 6.01 (1H, s), 6.27 (1H, s), 6.75 (1H, brs), 6.94 (1H, d, J=8.8Hz), 7.13 (1H, s), 7.17 (1H, dd, J=2.4, 8.8Hz), 7.37 (1H, d, J=2.4Hz), 10.63 (1H, s), 18.78 (1H, s).
MS (ESI) m/z: 652.1527 $(M+H)^+$

(Example 130) (9aS)-8-Acetyl-N-{[4-(2-butynyloxy)-2,3-dimethyl-1-naphthyl]methyl}-1,7-dihydroxy-3-methoxy-9a-methyl-9-oxo-9,9a-dihydrodibenzo[b,d]furan-4-carboxamide (exemplary compound No. 1-454)

(130a) 4-Hydroxy-2,3-dimethyl-1-naphthaldehyde

2,3-Dimethyl-1-naphthol (493 mg, 2.86 mmol) was dissolved in dichloromethane (20 mL). Dichloro(methoxy)methane (285 μL, 3.15 mmol) and aluminum trichloride (420 mg, 3.15 mmol) were added at -78˚C, and the mixture was stirred with heating to -40˚C for two hours. Water (20 mL) was added to the reaction solution, followed by extraction with diethyl ether (100 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 4/1) to give the target compound (346 mg, yield: 60%) as a light yellow solid.
$^1$H-NMR ($CDCl_3$, 500MHz):δ ppm: 2.38 (3H, s), 2.73 (3H, s), 5.85 (1H, m), 7.51 (1H, m), 7.59 (1H, m), 8.14 (1H, brd, J=8.2Hz), 9.01 (1H, brd, J=8.8Hz), 10.90 (1H, s).

(130b) 4-(2-Butynyloxy)-2,3-dimethyl-1-naphthaldehyde

Reaction and post-treatment were carried out in accordance with Example (60a) using 4-hydroxy-2,3-dimethyl-1-naphthaldehyde produced in Example (130a) (170 mg, 0.849 mmol), sodium hydride (50 mg, 1.15 mmol), 1-bromo-2-butyne (150 μL, 1.71 mmol) and N,N-dimethylformamide (10 mL) to give the target compound (120 mg, yield: 56%) as a white solid.
$^1$H-NMR ($CDCl_3$, 400MHz):δ ppm: 1.88 (3H, m), 2.46 (3H, s), 2.69 (3H, s), 4.63 (2H, m), 7.60-7.49 (2H, m), 8.18 (1H, brd, J=8.6Hz), 8.84 (1H, brd, J=8.2Hz), 10.95 (1H, s).
MS (EI) m/z: 252 $(M^+)$.

(130c) (9aS)-8-Acetyl-N-{[4-(2-butynyloxy)-2,3-dimethyl-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (200 mg, 0.579 mmol), 4-(2-butynyloxy)-2,3-dimethyl-1-naphthaldehyde produced in Example (130b) (122 mg, 0.476 mmol), triethylsilane (280 μL, 1.73 mmol), trifluoroacetic acid (135 μL, 1.75 mmol) and acetonitrile (8 mL) to give the target compound (167 mg, yield: 60%) as a yellow solid.
$^1$H-NMR ($CDCl_3$, 400MHz):δ ppm: 1.74 (3H, s), 1.90 (3H, s), 2.45 (3H, s), 2.53 (3H, s), 2.64 (3H, s), 3.63 (3H, s), 4.58 (2H, s), 5.04 (1H, dd, J=14.7,4.4Hz), 5.12 (1H, dd, J=14.7, 4.7Hz), 6.01 (1H, s), 6.23 (1H, s), 7.01 (1H, m), 7.55-7.47 (2H, m), 8.09 (1H, brd, J=7.9Hz), 8.18 (1H, brd, J=7.9Hz), 10.64 (1H, s), 18.83 (1H, s).
MS (ESI) m/z: 582.21308 $(M+H)^+$.

(Example 131) (9aS)-8-Acetyl-N-{[2,3-dimethyl-4-(2-pentynyloxy)-1-naphthyl]methyl}-1,7-dihydroxy-3-methoxy-9a-methyl-9-oxo-9,9a-dihydrodibenzo[b,d]furan-4-carboxamide (exemplary compound No. 1-455)

(131a) 2,3-Dimethyl-4-(2-pentynyloxy)-1-naphthaldehyde

Reaction and post-treatment were carried out in accordance with Example (60a) using 4-hydroxy-2,3-dimethyl-1-naphthaldehyde produced in Example (130a) (170 mg, 0.849 mmol), sodium hydride (50 mg, 1.15 mmol), 1-bromo-2-pentyne (175 μL, 1.71 mmol) and N,N-dimethylformamide (10 mL) to give the target compound (120 mg, yield: 53%) as a white solid.
$^1$H-NMR ($CDCl_3$, 400MHz):δ ppm: 1.12 (3H, dt, J=7.4, 1.2Hz), 2.24 (2H, m), 2.46 (3H, s), 2.69 (3H, s), 4.66 (2H, m), 7.60-7.50 (2H, m), 8.19 (1H, d, J=8.2Hz), 8.84 (1H, d, J=8.6Hz), 10.95 (1H, s).
MS (EI) m/z: 266 $(M^+)$.

(131b) (9aS)-8-Acetyl-N-{[2,3-dimethyl-4-(2-pentynyloxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (200 mg, 0.579 mmol), 2,3-dimethyl-4-(2-pentynyloxy)-1-naphthaldehyde produced in Example (131a) (120 mg, 0.451 mmol), triethylsilane (280 μL, 1.73 mmol), trifluoroacetic acid (135 μL, 1.75 mmol) and acetonitrile (8 mL) to give the target compound (173 mg, yield: 65%) as a yellow solid.
$^1$H-NMR ($CDCl_3$, 400MHz):δ ppm: 1.15 (3H, t, J=7.5Hz), 1.74 (3H, s), 2.26 (2H, m), 2.46 (3H, s), 2.53 (3H, s), 2.64 (3H, s), 3.63 (3H, s), 4.61 (2H, s), 5.04 (1H, dd, J=14.7, 4.4Hz), 5.12 (1H, dd, J=14.7, 4.4Hz), 6.01 (1H, s), 6.23 (1H, s), 7.01 (1H, m), 7.55-7.47 (2H, m), 8.08 (1H, brd, J=8.1Hz), 8.19 (1H, brd, J=8.1Hz), 10.64 (1H, s), 18.63 (1H, s).
MS (ESI) m/z: 596.22925 $(M+H)^+$.

(Example 132) (9aS)-8-Acetyl-1,7-dihydroxy-N-(4-hydroxy-2,3,6-trimethylbenzyl)-3-methoxy-9a-methyl-9-oxo-9,9a-dihydrodibenzo[b,d]furan-4-carboxamide (exemplary compound No. 1-131)

(132a) 4-[({[(9aS)-8-Acetyl-1,7-dihydroxy-3-methoxy-9a-methyl-9-oxo-9,9a-dihydrodibenzo[b,d]furan-4-yl]carbonyl} amino)methyl]-2,3,5-trimethylphenyl acetate

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (7.50 g, 21.7 mmol), 4-formyl-2,3,5-trimethylphenyl acetate (6.72 g, 32.6 mmol), triethylsilane (7.02 mL, 43.4 mmol), trifluoroacetic acid (3.35 mL, 43.4 mmol) and acetonitrile (200 mL) to give the target compound (11.3 g, yield: 97%) as a yellow solid.
$^1$H-NMR ($CDCl_3$, 400MHz):δ ppm: 1.76 (3H, s), 2.08 (3H, s), 2.33 (6H, s), 2.39 (3H, s), 2.65 (3H, s), 3.80 (3H, s), 4.58-4.68 (2H, m), 6.02 (1H, s), 6.29 (1H, s), 6.71 (1H, m), 6.75 (1H, s), 10.65 (1H, s), 18.83 (1H, s)
MS (FAB) m/z: 536 $(M+H)^+$

(132b) (9aS)-8-Acetyl-1,7-dihydroxy-N-(4-hydroxy-2,3,6-trimethylbenzyl)-3-methoxy-9a-methyl-9-oxo-9,9a-dihydrodibenzo[b,d]furan-4-carboxamide

4-[({[(9aS)-8-Acetyl-1,7-dihydroxy-3-methoxy-9a-methyl-9-oxo-9,9a-dihydrodibenzo[b,d]furan-4-yl]carbonyl} amino)methyl]-2,3,5-trimethylphenyl acetate produced in Example (132a) (11.3 g, 21.0 mmol) was dissolved in tetrahydrofuran (100 mL). A 1 N sodium hydroxide aqueous solution (100 mL) was added, and the mixture was stirred at room temperature for one hour. The reaction solution was separated with 1 N hydrochloric acid (100 mL) and ethyl acetate (200 mL). The organic layer was washed with brine (200 mL) and dried over anhydrous sodium sulfate. The solvent was evaporated under reduced pressure, and the residue was purified by silica gel column chromatography (methylene chloride:methanol = 80:1, V/V) and reverse phase silica gel column chromatography (acetonitrile:water = 2:1, V/V) to give the title target compound (9.47 g, 90%) as a yellow solid.
$^1$H-NMR ($CDCl_3$, 400MHz):δ ppm: 1.72 (3H, s), 2.16 (3H, s), 2.29 (3H, s), 2.32 (3H, s), 2.63 (3H, s), 3.78 (3H, s), 4.53-4.62 (2H, m), 5.49 (1H, brs), 5,88 (1H, s), 6.26 (1H, s), 6.53 (1H, s), 6.68 (1H, m), 10.58 (1H, s), 18.73 (1H, s)
MS (ESI) m/z: 494 $(M+H)^+$

(Example 133) (9aS)-8-Acetyl-N-(2,6-dimethyl-4-propoxybenzyl)-1,7-dihydroxy-3-methoxy-9a-methyl-9-oxo-9,9a-dihydrodibenzo[b,d]furan-4-carboxamide (exemplary compound No. 1-75)

(9aS)-8-Acetyl-1,7-dihydroxy-N-(4-hydroxy-2,6-dimethylbenzyl)-3-methoxy-9a-methyl-9-oxo-9,9a-dihydrodibenzo[b,d]furan-4-carboxamide produced in Example (97c) (0.300 g, 0.626 mmol) was dissolved in N,N-dimethylformamide (8 mL). Potassium carbonate (0.345 g, 2.50 mmol) and n-propyl iodide (0.244 mL, 2.50 mmol) were added, and the mixture was stirred in a nitrogen atmosphere at 60˚C for 12 hours. After completion of the reaction, the reaction solution was cooled to 0˚C. 0.1 N hydrochloric acid (20 mL) was added, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: methylene chloride/methanol = 80/1 to 40/1) to give the target compound (0.117 g, yield: 36%) as a yellow solid.
$^1$H-NMR ($CDCl_3$, 400MHz):δ ppm: 1.03 (3H, t, J=7.3Hz), 1.75 (3H, s), 1.75-1.82 (2H, m), 2.39 (6H, s), 2.65 (3H, s), 3.79 (3H, s), 3.90 (2H, t, J=6.6Hz), 4.51-4.61 (2H, m), 6.01 (1H, s), 6.28 (1H, s), 6.62 (2H, s), 6.67 (1H, brs), 10.63 (1H, s), 18.82 (1H, s).
MS (ESI) m/z: 522.2089 $(M+H)^+$

(Example 134) (9aS)-8-Acetyl-N-[(2,3-dimethyl-4-propoxy-1-naphthyl)methyl]-1,7-dihydroxy-3-methoxy-9a-methyl-9-oxo-9,9a-dihydrodibenzo[b,d]furan-4-carboxamide (exemplary compound No. 1-453)

(134a) 2,3-Dimethyl-4-propoxy-1-naphthaldehyde

Reaction and post-treatment were carried out in accordance with Example (60a) using 4-hydroxy-2,3-dimethyl-1-naphthaldehyde produced in Example (130a) (203 mg, 1.01 mmol), sodium hydride (60 mg, 1.38 mmol), 1-iodopropane (200 μL, 2.05 mmol) and N,N-dimethylformamide (15 mL) to give the target compound (245 mg, yield: ~100%) as a light yellow oil.
$^1$H-NMR ($CDCl_3$, 400MHz):δ ppm: 1.16 (3H, t, J=7.4Hz), 1.97 (2H, m), 2.41 (3H, s), 2.69 (3H, s), 3.90 (2H, t, J=6.7Hz), 7.57-7.45 (2H, m), 8.10 (1H, d, J=8.2Hz), 8.85 (1H, d, J=8.2Hz), 10.91 (1H, s).
MS (FAB) m/z: 243 $(M+H)^+$.

(134b) (9aS)-8-Acetyl-N-[(2,3-dimethyl-4-propoxy-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (230 mg, 0.666 mmol), 2,3-dimethyl-4-propoxy-1-naphthaldehyde produced in Example (134a) (245 mg, 1.01 mmol), triethylsilane (325 μL, 2.01 mmol), trifluoroacetic acid (155 μL, 2.01 mmol) and acetonitrile (10 mL) to give the target compound (203 mg, yield: 53%) as a yellow solid.
$^1$H-NMR ($CDCl_3$, 400MHz):δ ppm: 1.16 (3H, t, J=7.4Hz), 1.74 (3H, s), 1.97 (2H, m), 2.41 (3H, s), 2.53 (3H, s), 2.64 (3H, s), 3.63 (3H, s), 3.88 (2H, t, J=6.7Hz), 5.04 (1H, dd, J=14.1, 4.3Hz), 5.11 (1H, dd, J=14.1, 4.3Hz), 6.01 (1H, s), 6.23 (1H, s), 7.00 (1H, brs), 7.54-7.44 (2H, m), 8.08 (1H, brd, J=7.8Hz), 8.13 (1H, brd, J=7.8Hz), 10.64 (1H, s), 18.84 (1H, s).
MS (ESI) m/z: 572.22639 $(M+H)^+$.

(Example 135) (9aS)-8-Acetyl-N-({4-[(4-fluorobenzyl)oxy]-2,3-dimethyl-1-naphthyl}methyl)-1,7-dihydroxy-3-methoxy-9a-methyl-9-oxo-9,9a-dihydrodibenzo[b,d]furan-4-carboxamide (exemplary compound No. 1-456)

(135a) 4-[(4-Fluorobenzyl)oxy]-2,3-dimethyl-1-naphthaldehyde

Reaction and post-treatment were carried out in accordance with Example (60a) using 4-hydroxy-2,3-dimethyl-1-naphthaldehyde produced in Example (130a) (203 mg, 1.01 mmol), sodium hydride (60 mg, 1.38 mmol), 4-fluorobenzyl bromide (255 μL, 2.05 mmol) and N,N-dimethylformamide (15 mL) to give the target compound (380 mg, yield: ~100%) as a light yellow oil.
$^1$H-NMR ($CDCl_3$, 400MHz):δ ppm: 2.39 (3H, s), 2.70 (3H, s), 4.95 (2H, brs), 7.14-7.08 (2H, m), 7.59-7.46 (2H, m), 8.09 (1H, d, J=8.6Hz), 8.85 (1H, d, J=8.6Hz), 10.93 (1H, s).
MS (FAB) m/z: 309 $(M+H)^+$.

(135b) (9aS)-8-Acetyl-N-({4-[(4-fluorobenzyl)oxy]-2,3-dimethyl-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (230 mg, 0.666 mmol), 4-[(4-fluorobenzyl)oxy]-2,3-dimethyl-1-naphthaldehyde produced in Example (135a) (310 mg, 1.01 mmol), triethylsilane (325 μL, 2.01 mmol), trifluoroacetic acid (155 μL, 2.01 mmol) and acetonitrile (10 mL) to give the target compound (183 mg, yield: 43%) as a yellow solid.
$^1$H-NMR ($CDCl_3$, 400MHz):δ ppm: 1.74 (3H, s), 2.42 (3H, s), 2.53 (3H, s), 2.64 (3H, s), 3.64 (3H, s), 4.93 (2H, s), 5.06 (1H, dd, J=14.3, 4.4Hz), 5.13 (1H, dd, J=14.3, 4.4Hz), 6.01 (1H, s), 6.24 (1H, s), 7.04 (1H, m), 7.13 (1H, m), 7.53 (1H, m), 8.12 (1H, m), 10.65 (1H, s), 18.84 (1H, s).
MS (ESI) m/z: 638.21626 $(M+H)^+$.

(Example 136) (9aS)-8-Acetyl-N-{[4-(2-butynyloxy)-2-methyl-1-naphthyl]methyl}-1,7-dihydroxy-3-methoxy-9a-methyl-9-oxo-9,9a-dihydrodibenzo[b,d]furan-4-carboxamide (exemplary compound No. 1-442)

(136a) 4-Hydroxy-2-methyl-1-naphthaldehyde

3-Methyl-1-naphthol (1.57 g, 9.92 mmol) was dissolved in dichloromethane (100 mL). Dichloro(methoxy)methane (900 μL, 9.95 mmol) and titanium tetrachloride (1.10 mL, 10.0 mmol) were added at -78˚C, and the mixture was stirred with heating to -40˚C for two hours. Water (100 mL) was added to the reaction solution, followed by extraction with methylene chloride (50 mL) and diethyl ether (200 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 4/1) to give the target compound (885 mg, yield: 48%) as a light yellow solid.
$^1$H-NMR ($CDCl_3$, 400MHz):δ ppm: 2.78 (3H, s), 5.82 (1H, brs), 6.66 (1H, s), 7.49 (1H, m), 7.64 (1H, m), 8.18 (1H, d, J=8.2Hz), 9.13 (1H, d, J=8.6Hz), 10.78 (1H, s).

(136b) 4-(2-Butynyloxy)-2-methyl-1-naphthaldehyde

Reaction and post-treatment were carried out in accordance with Example (60a) using 4-hydroxy-2-methyl-1-naphthaldehyde produced in Example (136a) (177 mg, 0.951 mmol), sodium hydride (60 mg, 1.38 mmol), 1-bromo-2-butyne (170 μL, 1.94 mmol) and N,N-dimethylformamide (10 mL) to give the target compound (177 mg, yield: 78%) as a white solid.
$^1$H-NMR ($CDCl_3$, 400MHz):δ ppm: 1.89 (3H, t, J=2.3Hz), 2.83 (3H, s), 4.90 (2H, q, J=2.3Hz), 6.73 (1H, brs), 7.46 (1H, m), 7.61 (1H, m), 8.26 (1H, brd, J=8.6Hz), 9.10 (1H, d, J=8.6Hz), 10.79 (1H, s).
MS (EI) m/z: 238 $(M^+)$.

(136c) (9aS)-8-Acetyl-N-{[4-(2-butynyloxy)-2-methyl-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (220 mg, 0.637 mmol), 4-(2-butynyloxy)-2-methyl-1-naphthaldehyde produced in Example (136b) (177 mg, 0.473 mmol), triethylsilane (310 μL, 1.92 mmol), trifluoroacetic acid (150 μL, 1.95 mmol) and acetonitrile (10 mL) to give the target compound (190 mg, yield: 52%) as a yellow solid.
$^1$H-NMR ($CDCl_3$, 500MHz):δ ppm: 1.74 (3H, s), 1.89 (3H, m), 2.61 (3H, s), 2.64 (3H, s), 3.61 (3H, s), 4.85 (2H, m), 4.97 (1H, dd, J=14.2, 4.4Hz), 5.04 (1H, dd, J=14.7, 4.2Hz), 6.00 (1H, s), 6.23 (1H,s), 6.79 (1H, s), 6.97 (1H, brs), 7.45 (1H, t, J=7.6Hz), 7.55 (1H, t, J=7.6Hz), 8.03 (1H, d, J=8.3Hz), 8.30 (1H, d, J=8.3Hz), 10.62 (1H, s), 18.82 (1H, s).
MS (ESI) m/z: 568.19767 $(M+H)^+$.

(Example 137) (9aS)-8-Acetyl-N-{[2-methyl-4-(2-pentynyloxy)-1-naphthyl]methyl}-1,7-dihydroxy-3-methoxy-9a-methyl-9-oxo-9,9a-dihydrodibenzo[b,d]furan-4-carboxamide (exemplary compound No. 1-443)

(137a) 2-Methyl-4-(2-pentynyloxy)-1-naphthaldehyde

Reaction and post-treatment were carried out in accordance with Example (60a) using 4-hydroxy-2-methyl-1-naphthaldehyde produced in Example (136a) (177 mg, 0.951 mmol), sodium hydride (60 mg, 1.38 mmol), 1-bromo-2-butyne (200 μL, 1.96 mmol) and N,N-dimethylformamide (10 mL) to give the target compound (186 mg, yield: 78%) as a white solid.
$^1$H-NMR ($CDCl_3$, 400MHz):δ ppm: 1.16 (3H, dt, J=7.4Hz), 2.26 (2H, m), 2.83 (3H, s), 4.92 (2H, m), 6.75 (1H, brs), 7.46 (1H, m), 7.61 (1H, m), 8.26 (1H, d, J=8.6Hz), 9.10 (1H, d, J=8.6Hz), 10.79 (1H, s).
MS (EI) m/z: 252 ($M^+$).

(137b) (9aS)-8-Acetyl-N-{[2-methyl-4-(2-pentynyloxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (220 mg, 0.637 mmol), 2-methyl-4-(2-pentynyloxy)-1-naphthaldehyde produced in Example (137a) (186 mg, 0.737 mmol), triethylsilane (310 μL, 1.92 mmol), trifluoroacetic acid (150 μL, 1.95 mmol) and acetonitrile (10 mL) to give the target compound (230 mg, yield: 62%) as a yellow solid.
$^1$H-NMR ($CDCl_3$, 500MHz):δ ppm: 1.16 (3H, t, J=7.5Hz), 1.74 (3H, s), 2.26 (2H, m), 2.61 (3H, s), 2.64 (3H, s), 3.61 (3H, s), 4.87 (2H, m), 4.97 (1H, dd, J=14.2, 4.4Hz), 5.04 (1H, dd, J=14.7, 4.2Hz), 6.00 (1H, s), 6.22 (1H,s), 6.81 (1H, s), 6.97 (1H, brs), 7.45 (1H, t, J=7.8Hz), 7.55 (1H, t, J=7.8Hz), 8.03 (1H, d, J=8.3Hz), 8.30 (1H, d, J=8.3Hz), 10.62 (1H, s), 18.82 (1H, s).
MS (ESI) m/z: 582.21182 $(M+H)^+$.

(Example 138) (9aS)-8-Acetyl-N-[(2-methyl-4-propoxy-1-naphthyl)methyl]-1,7-dihydroxy-3-methoxy-9a-methyl-9-oxo-9,9a-dihydrodibenzo[b,d]furan-4-carboxamide (exemplary compound No. 1-441)

(138a) 2-Methyl-4-propoxy-1-naphthaldehyde

Reaction and post-treatment were carried out in accordance with Example (60a) using 4-hydroxy-2-methyl-1-naphthaldehyde produced in Example (136a) (177 mg, 0.951 mmol), sodium hydride (60 mg, 1.38 mmol), 1-iodopropane (190 μL, 1.95 mmol) and N,N-dimethylformamide (10 mL) to give the target compound (232 mg, yield: ~100%) as a light yellow oil.
$^1$H-NMR ($CDCl_3$, 400MHz):δ ppm: 1.15 (3H, t, J=7.4Hz), 2.02-1.94 (2H, m), 2.81 (3H, s), 4.16 (2H, t, J=6.3Hz), 6.60 (1H, s), 7.45 (1H, m), 7.61 (1H, m), 8.27 (1H, d, J=8.4Hz), 9.12 (1H, d, J=9.0Hz), 10.77 (1H, s).
MS (EI) m/z: $228(M)^+$.

(138b) (9aS)-8-Acetyl-N-[(2-methyl-4-propoxy-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (220 mg, 0.637 mmol), 2-methyl-4-propoxy-1-naphthaldehyde produced in Example (138a) (232 mg, 1.02 mmol), triethylsilane (310 μL, 1.92 mmol), trifluoroacetic acid (150 μL, 1.95 mmol) and acetonitrile (10 mL) to give the target compound (227 mg, yield: 64%) as a yellow solid.
$^1$H-NMR ($CDCl_3$, 400MHz):δ ppm: 1.14 (3H, t, J=7.4Hz), 1.74 (3H, s), 1.94 (2H, m), 2.59 (3H, s), 2.63 (3H, s), 3.60 (3H, s), 4.10 (1H, m), 4.95 (1H, dd, J=14.3, 4.3Hz), 5.02 (1H, dd, J=14.3, 4.8Hz), 5.99 (1H, s), 6.21 (1H,s), 6.93 (1H, brs), 7.43 (1H, m), 7.53 (1H, m), 8.01 (1H, brd, J=8.7Hz), 8.30 (1H, brd, J=8.7Hz), 10.59 (1H, s), 18.78 (1H, s).
MS (ESI) m/z: 558.20983 $(M+H)^+$.

(Example 139) (9aS)-8-Acetyl-N-({4-[(4-fluorobenzyl)oxy]-2-methyl-1-naphthyl} methyl)-1,7-dihydroxy-3-methoxy-9a-methyl-9-oxo-9,9a-dihydrodibenzo[b,d]furan-4-carboxamide (exemplary compound No. 1-444)

(139a) 4-[(4-Fluorobenzyl)oxy]-2-methyl-1-naphthaldehyde

Reaction and post-treatment were carried out in accordance with Example (60a) using 4-hydroxy-2-methyl-1-naphthaldehyde produced in Example (136a) (177 mg, 0.951 mmol), sodium hydride (60 mg, 1.38 mmol), 4-fluorobenzyl bromide (240 μL, 1.93 mmol) and N,N-dimethylformamide (10 mL) to give the target compound (183 mg, yield: 65%) as a light yellow oil.
$^1$H-NMR ($CDCl_3$, 400MHz):δ ppm: 2.82 (3H, s), 5.26 (2H, brs),6.71 (1H, s), 7.14-7.09 (2H, m), 7.50-7.45 (3H, m), 7.63 (1H, m), 8.28 (1H, d, J=8.3Hz), 9.12 (1H, d, J=8.6Hz), 10.80 (1H, s).
MS (EI) m/z: 294 ($M^+$).

(139b) (9aS)-8-Acetyl-N-({4-[(4-fluorobenzyl)oxy]-2-methyl-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (220 mg, 0.637 mmol), 4-[(4-fluorobenzyl)oxy]-2-methyl-1-naphthaldehyde produced in Example (139a) (183 mg, 0.622 mmol), triethylsilane (310 μL, 1.92 mmol), trifluoroacetic acid (150 μL, 1.95 mmol) and acetonitrile (10 mL) to give the target compound (223 mg, yield: 56.5%) as a yellow solid.
$^1$H-NMR ($CDCl_3$, 400MHz):δ ppm: 1.73 (3H, s), 2.59 (3H, s), 2.63 (3H, s), 3.61 (3H, s), 4.92-5.06 (2H, m), 5.19 (2H, s), 5.97 (1H, s), 6.21 (1H, s), 6.73 (1H, s), 6.97 (1H, brs), 7.10 (1H, t, J=8.3Hz), 7.43 (1H, t, J=7.5Hz), 7.48 (1H, dd, J=8.3, 5.2Hz), 7.55 (1H, t, J=7.5Hz), 8.03 (1H, d, J=8.3Hz), 8.30 (1H, d, J=8.3Hz), 10.60 (1H, s), 18.77 (1H, s).
MS (ESI) m/z: 624.20038 $(M+H)^+$.

(Example 140) (9aS)-8-Acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (exemplary compound No. 1-93)

(140a) 2,4-Dichloro-N-(3,5-dimethylphenyl)-5-methylbenzenesulfonamide

3,5-Dimethylaniline (0.37 mL, 3.00 mmol) was dissolved in methylene chloride (20 mL). Pyridine (0.27 mL, 3.30 mmol) and 2,4-dichloro-5-methylbenzenesulfonyl chloride (818 mg, 3.15 mmol) were added at room temperature, and the mixture was stirred for one hour. The reaction solution was separated with 0.5 N hydrochloric acid (20 mL). The organic layer was washed with brine (20 mL) and dried over anhydrous sodium sulfate. The solvent was evaporated under reduced pressure, and the residue was purified by silica gel column chromatography (hexane:ethyl acetate = 10: 1, V/V) to give the title target compound (431 mg, 42%) as a colorless solid.
$^1$H-NMR ($CDCl_3$, 400MHz):δ ppm: 2.22 (6H, s), 2.33 (3H, s), 6.71 (2H, s), 6.73 (1H, s), 6.84 (1H, brs), 7.47 (1H, s), 7.84 (1H, s)

(140b) 2,4-Dichloro-N-(4-formyl-3,5-dimethylphenyl)-5-methylbenzenesulfonamide

2,4-Dichloro-N-(3,5-dimethylphenyl)-5-methylbenzenesulfonamide produced in Example (140a) (431 mg, 1.25 mmol) was dissolved in methylene chloride (8 mL). Titanium (IV) chloride (0.27 mL, 2.50 mmol) and dichloromethyl methyl ether (0.14 mL, 1.50 mmol) were added at -78˚C, and the mixture was stirred for 30 minutes.
The reaction solution was heated to 0˚C and separated with methylene chloride (15 mL) and water (20 mL). The organic layer was washed with brine (15 mL) and dried over anhydrous sodium sulfate. The solvent was evaporated under reduced pressure, and the residue was purified by silica gel column chromatography (hexane:ethyl acetate = 10: 1 1 to 1:1; V/V) to give the title target compound (197 mg, 42%) as a colorless solid.
$^1$H-NMR ($CDCl_3$, 400MHz):δ ppm: 2.38 (3H, s), 2.52 (6H, s), 6.80 (2H, s), 7.30 (1H, brs), 7.46 (1H, s), 7.96 (1H, s), 10.41 (1H, s)

(140c) (9aS)-8-Acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (122 mg, 0.35 mmol), 2,4-dichloro-N-(4-formyl-3,5-dimethylphenyl)-5-methylbenzenesulfonamide produced in Example (140b)

(197 mg, 0.53 mmol), triethylsilane (0.11 mL, 0.71 mmol), trifluoroacetic acid (54 μL, 0.71 mmol) and acetonitrile (5 mL) to give the target compound (165 mg, yield: 67%) as a yellow solid.
$^1$H-NMR ($CDCl_3$, 400MHz):δ ppm: 1.73 (3H, s), 2.34 (6H, s), 2.35 (3H, s), 2.64 (3H, s), 3.76 (3H, s), 4.46-4.58 (2H, m), 5.93 (1H, s), 6.28 (1H, s), 6.78 (1H, m), 6.83 (2H, s), 7.20 (1H, s), 7.49 (1H, s), 7.90 (1H, s), 10.66 (1H, s), 18.79 (1H, s)
MS (ESI) m/z: 701 $(M+H)^+$.

(Example 141) (9aS)-8-Acetyl-N-{2,6-dimethyl-4-[(methylsulfonyl)amino]benzyl}-1,7-dihydroxy-3-methoxy-9a-methyl-9-oxo-9,9a-dihydrodibenzo[b,d]furan-4-carboxamide (exemplary compound No. 1-78)

(141a) N-(3,5-Dimethylphenyl)methanesulfonamide

Reaction and post-treatment were carried out in accordance with Example (140a) using 3,5-dimethylaniline (0.81 mL, 6.60 mmol), methylene chloride (16 mL), triethylamine (1.10 mL, 7.92 mmol) and methanesulfonyl chloride (0.61 mL, 7.92 mmol) to give the target compound (1.30 g, yield: 99%) as a colorless solid.

(141b) N-(4-Formyl-3,5-dimethylphenyl)methanesulfonamide

Reaction and post-treatment were carried out in accordance with Example (140b) using N-(3,5-dimethylphenyl) methanesulfonamide produced in Example (141a) (1.30 g, 6.52 mmol), methylene chloride (20 mL), titanium (IV) chloride (1.43 mL, 13.0 mmol) and dichloromethyl methyl ether (0.71 mL, 7.82 mmol) to give the target compound (587 mg, yield: 40%) as a colorless solid.
$^1$H-NMR ($CDCl_3$, 500MHz):δ ppm: 2.62 (6H, s), 3.11 (3H, s), 6.66 (1H, brs), 6.89 (2H, s), 10.52 (1H, s)

(141c) (9aS)-8-Acetyl-N-{2,6-dimethyl-4-[(methylsulfonyl)amino]benzyl}-1,7-dihydroxy-3-methoxy-9a-methyl-9-oxo-9,9a-dihydrodibenzo[b,d]furan-4-carboxamide

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (300 mg, 0.87 mmol), N-(4-formyl-3,5-dimethylphenyl)methanesulfonamide produced in Example (141b) (395 mg, 1.74 mmol), triethylsilane (0.28 mL, 1.74 mmol), trifluoroacetic acid (0.13 mL, 1.74 mmol) and acetonitrile (10 mL) to give the target compound (371 mg, yield: 77%) as a yellow solid.
$^1$H-NMR ($CDCl_3$, 400MHz):δ ppm: 1.74 (3H, s), 2.43 (6H, s), 2.64 (3H, s), 3.03 (3H, s); 3.81 (3H, s), 4.55-4.65 (2H, m), 5.95 (1H, s), 6.30 (1H, s), 6.69 (1H, s), 6.85 (1H, m), 6.93 (2H, s), 10.66 (1H, s), 18.80 (1H, s)
MS (ESI) m/z: 557 $(M+H)^+$

(Example 142) (9aS)-8-Acetyl-N-{2,6-dimethyl-4-[(propylsulfonyl)amino]benzyl}-1,7-dihydroxy-3-methoxy-9a-methyl-9-oxo-9,9a-dihydrodibenzo[b,d]furan-4-carboxamide (exemplary compound No. 1-80)

(142a) N-(3,5-Dimethylphenyl)propane-1-sulfonamide

Reaction and post-treatment were carried out in accordance with Example (140a) using 3,5-dimethylaniline (0.81 mL, 6.60 mmol), methylene chloride (16 mL), triethylamine (1.10 mL, 7.92 mmol) and propylsulfonyl chloride (0.89 mL, 7.92 mmol) to give the target compound (1.48 g, yield: 99%) as a colorless solid.

(142b) N-(4-Formyl-3,5-dimethylphenyl)propane-1-sulfonamide

Reaction and post-treatment were carried out in accordance with Example (140b) using N-(3,5-dimethylphenyl) propane-1-sulfonamide produced in Example (142a) (1.48 g, 6.51 mmol), methylene chloride (20 mL), titanium (IV) chloride (1.43 mL, 13.0 mmol) and dichloromethyl methyl ether (0.71 mL, 7.81 mmol) to give the target compound (952 mg, yield: 57%) as a colorless solid.
$^1$H-NMR ($CDCl_3$, 400MHz):δ ppm: 1.05 (3H, t, J=7.4Hz), 1.88 (2H, m), 2.61 (6H, s), 3.17 (2H, m), 6.74 (1H, brs), 6.88 (2H, s), 10.51 (1H, s)

(142c) (9aS)-8-Acetyl-N-{2,6-dimethyl-4-[(propylsulfonyl)amino]benzyl}-1,7-dihydroxy-3-methoxy-9a-methyl-9-oxo-9,9a-dihydrodibenzo[b,d]furan-4-carboxamide

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (300 mg,

0.87 mmol), N-(4-formyl-3,5-dimethylphenyl)propane-1-sulfonamide produced in Example (142b) (444 mg, 1.74 mmol), triethylsilane (0.28 mL, 1.74 mmol), trifluoroacetic acid (0.13 mL, 1.74 mmol) and acetonitrile (10 mL) to give the target compound (410 mg, yield: 81 %) as a yellow solid.
$^1$H-NMR ($CDCl_3$, 400MHz):δ ppm: 1.04 (3H, t, J=7.5Hz), 1.74 (3H, s), 1.87 (2H, m), 2.42 (6H, s), 2.64 (3H, s), 3.09 (2H, m), 3.81 (3H, s), 4.54-4.65 (2H, m), 5.97 (1H, s), 6.30 (1H, s), 6.54 (1H, s), 6.83 (1H, m), 6.91 (2H, s), 10.67 (1H, s), 18.81 (1H, s) MS (ESI) m/z: 585 $(M+H)^+$

(Example 143) (9aS)-8-Acetyl-N-{4-[(2,4-dichlorobenzyl)oxy]-2,3,6-trimethylbenzyl}-1,7-dihydroxy-3-methoxy-9a-methyl-9-oxo-9,9a-dihydrodibenzo[b,d]furan-4-carboxamide (exemplary compound No. 1-149)

(9aS)-8-Acetyl-1,7-dihydroxy-N-(4-hydroxy-2,3,6-trimethylbenzyl)-3-methoxy-9a-methyl-9-oxo-9,9a-dihydrodibenzo[b,d]furan-4-carboxamide produced in Example (132b) (0.500 g, 1.01 mmol) was dissolved in N,N-dimethylformamide (7 mL). Potassium carbonate (0.560 g, 4.05 mmol) and 2,4-dichlorobenzyl chloride (0.563 mL, 4.05 mmol) were added, and the mixture was stirred in a nitrogen atmosphere at 60˚C for 12 hours. After completion of the reaction, the reaction solution was cooled to 0˚C. 0.1 N hydrochloric acid (10 mL) was added, followed by extraction with ethyl acetate (10 mL). The organic layer was washed with brine and then dried over anhydrous sodium sulfate. The solvent was evaporated under reduced pressure, and the resulting residue was purified by silica gel column chromatography (elution solvent: methylene chloride/methanol = 80/1 to 40/1) to give the target compound (0.466 g, yield: 71 %) as a yellow solid.
$^1$H-NMR ($CDCl_3$, 400MHz):δ ppm: 1.75 (3H, s), 2.24 (3H, s), 2.34 (3H, s), 2.41 (3H, s), 2.65 (3H, s), 3.79 (3H, s), 4.57-4.67 (2H, m), 5.10 (2H, s), 6.02 (1H, s), 6.28 (1H, s), 6.64 (1H, s), 6.69 (1H, br), 7.30 (1H, dd, J=1.6, 8.3Hz), 7.43 (1H, d, J=1.6Hz), 7.54 (1H, d, J=8.3Hz), 10.64 (1H, s), 18.83 (1H, s).
MS (ESI) m/z: 652.1512 $(M+H)^+$.

(Example 144) (9aS)-8-Acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-421)

(144a) 4-[(3-Fluorobenzyl)oxy]-1-naphthaldehyde

Sodium hydride (112 mg, 2.57 mmol) was suspended in N,N-dimethylformamide (10 mL) in a nitrogen atmosphere, and the suspension was cooled to 0˚C. 4-Hydroxy-1-naphthaldehyde (254 mg, 1.48 mmol) was slowly added, and the mixture was stirred at 0˚C for 30 minutes. 1-(Bromomethyl)-3-fluorobenzene (270 μL, 2.21 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 5/1) to give the target compound (359 mg, yield: 87%) as a yellow oil.
$^1$H-NMR ($CDCl_3$, 400MHz):δ ppm: 5.35 (2H, s), 6.97 (1H, d, J=8.2Hz), 7.08 (1H, m), 7.24-7.30 (2H, m), 7.41 (1H, m), 7.61 (1H, m), 7.73 (1H, m), 7.92 (1H, d, J=8.2Hz), 8.41 (1H, d, J=8.6Hz), 9.32 (1H, d, J=8.6Hz), 10.22 (1H, s).
MS (EI) m/z: 280.0891 $(M^+)$

(144b)(9aS)-8-Acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (309 mg, 0.895 mmol), 4-[(3-fluorobenzyl)oxy]-1-naphthaldehyde produced in Example (144a) (351 mg, 1.25 mmol), triethylsilane (430 μL, 2.70 mmol), trifluoroacetic acid (210 μL, 2.73 mmol) and acetonitrile (10 mL) to give the target compound (394 mg, yield: 72%) as a yellow solid.
$^1$H-NMR ($CDCl_3$, 400MHz):δ ppm: 1.75 (3H, s), 2.64 (3H, s), 3.69 (3H, s), 4.96 (1H, dd, J=4.8, 14.3Hz), 5.02 (1H, dd, J=4.8, 14.3Hz), 5.26 (2H, s), 6.00 (1H, s), 6.26 (1H, s), 6.81 (1H, d, J=7.5Hz), 7.04 (1H, m), 7.20 (1H, brs), 7.24-7.43 (4H, m), 7.53-7.62 (2H, m), 8.07 (1H, d, J=7.9Hz), 8.42 (1H, d, J=7.9Hz), 10.65 (1H, s), 18.83 (1H, s). MS (ESI+) m/z: 610.18836 $(M+H)^+$

(Example 145) (9aS)-8-Acetyl-N-({4-[(2-fluorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-446)

(145a) 4-[(2-Fluorobenzyl)oxy]-1-naphthaldehyde

Sodium hydride (109 mg, 2.50 mmol) was suspended in N,N-dimethylformamide (10 mL) in a nitrogen atmosphere, and the suspension was cooled to 0˚C. 4-Hydroxy-l-naphthaldehyde (260 mg, 1.51 mmol) was slowly added, and the mixture was stirred at 0˚C for 30 minutes. 1-(Bromomethyl)-2-fluorobenzene (270 μL, 2.24 mmol) was further added, and the mixture was stirred at room temperature for two hours. A dilute hydrochloric acid solution (30 mL) was added to the reaction solution, followed by extraction with ethyl acetate (50 ml).

The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 5/1) to give the target compound (413 mg, yield: 98%) as a yellow solid.
$^1$H-NMR ($CDCl_3$, 400MHz):δ ppm: 5.43 (2H, s), 7.05 (1H, d, J=8.2Hz), 7.14-7.24 (2H, m), 7.39 (1H, m), 7.57-7.62 (2H, m), 7.72 (1H, m), 7.94 (1H, d, J=8.2Hz), 8.39 (1H, d, J=8.6Hz), 9.32 (1H, d, J=8.6Hz), 10.22 (1H, s).
MS (EI) m/z: 280.0898 ($M^+$)

(145b) (9aS)-8-Acetyl-N-({4-[(2-fluorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (308 mg, 0.892 mmol), 4-[(2-fluorobenzyl)oxy]-1-naphthaldehyde produced in Example (145a) (408 mg, 1.45 mmol), triethylsilane (430 μL, 2.70 mmol), trifluoroacetic acid (210 μL, 2.73 mmol) and acetonitrile (10 mL) to give the target compound (404 mg, yield: 74%) as a yellow solid.
$^1$H-NMR ($CDCl_3$, 400MHz):δ ppm: 1.75 (3H, s), 2.64 (3H, s), 3.69 (3H, s), 4.96 (1H, dd, J=4.8, 14.3Hz), 5.03 (1H, dd, J=4.8, 14.3Hz), 5.33 (2H, s), 6.00 (1H, s), 6.26 (1H, s), 6.88 (1H, d, J=7.5Hz), 7.11-7.22 (3H, m), 7.32-7.65 (5H, m), 8.06 (1H, d, J=8.3Hz), 8.40 (1H, d, J=8.3Hz), 10.65 (1H, s), 18.83 (1H, s).
MS (ESI+) m/z: 610.18902 $(M+H)^+$.

(Example 146) (9aS)-8-Acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-429)

(146a) 4-[(2,4-Difluorobenzyl)oxy]-1-naphthaldehyde

Sodium hydride (103 mg, 2.36 mmol) was suspended in N,N-dimethylformamide (10 mL) in a nitrogen atmosphere, and the suspension was cooled to 0˚C. 4-Hydroxy-1-naphthaldehyde (255 mg, 1.48 mmol) was slowly added, and the mixture was stirred at 0˚C for 30 minutes. 1-(Bromomethyl)-2,4-difluorobenzene (285 μL, 2.22 mmol) was further added, and the mixture was stirred at room temperature for five hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 5/1) to give the target compound (398 mg, yield: 90%) as a light yellow solid.
$^1$H-NMR ($CDCl_3$, 400MHz):δ ppm: 5.35 (2H, s), 6.88-6.97 (2H, m), 7.01 (1H, d, J=8.2Hz), 7.52-7.58 (2H, m), 7.69 (1H, m), 7.92 (1H, d, J=8.2Hz), 8.33 (1H, d, J=7.8Hz), 9.29 (1H, d, J=8.6Hz), 10.20 (1H, s).
MS (ESI+) m/z: 299.08939 $(M+H)^+$

(146b) (9aS)-8-Acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (311 mg, 0.901 mmol), 4-[(2,4-difluorobenzyl)oxy]-1-naphthaldehyde produced in Example (146a) (394 mg, 1.32 mmol), triethylsilane (430 μL, 2.70 mmol), trifluoroacetic acid (210 μL, 2.73 mmol) and acetonitrile (10 mL) to give the target compound (402 mg, yield: 71%) as a yellow solid.
$^1$H-NMR ($CDCl_3$, 400MHz):δ ppm: 1.75 (3H, s), 2.64 (3H, s), 3.69 (3H, s), 4.97 (1H, dd, J=5.2, 14.7Hz), 5.03 (1H, dd, J=5.2, 14.7Hz), 5.27 (2H, s), 6.00 (1H, s), 6.26 (1H, s), 6.85-6.96 (3H, m), 7.20 (1H, brs), 7.44 (1H, d, J=7.9Hz), 7.51-7.61 (3H, m), 8.06 (1H, d, J=8.3Hz), 8.36 (1H, d, J=8.3Hz), 10.65 (1H, s), 18.83 (1H, s).

MS (ESI+) m/z: 628.18073 $(M+H)^+$

(Example 147) (9aS)-8-Acetyl-N-({4-[(3-chlorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-424)

(147a) 4-[(3-Chlorobenzyl)oxy]-1-naphthaldehyde

Sodium hydride (102 mg, 2.34 mmol) was suspended in N,N-dimethylformamide (10 mL) in a nitrogen atmosphere, and the suspension was cooled to 0˚C. 4-Hydroxy-1-naphthaldehyde (259 mg, 1.50 mmol) was slowly added, and the mixture was stirred at 0˚C for 30 minutes. 1-(Bromomethyl)-3-chlorobenzene (295 μL, 2.25 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 5/1) to give the target compound (380 mg, yield: 85%) as a yellow solid.
$^1$H-NMR ($CDCl_3$, 400MHz):δ ppm: 5.31 (2H, s), 6.95 (1H, d, J=8.2Hz), 7.33-7.40 (3H, m), 7.50 (1H, s), 7.58 (1H, m), 7.70 (1H, m), 7.90 (1H, d, J=8.2Hz), 8.38 (1H, d, J=8.2Hz), 9.29 (1H, d, J=8.2Hz), 10.19 (1H, s).
MS (ESI+) m/z: 297.06949 $(M+H)^+$

(147b) (9aS)-8-Acetyl-N-({4-[(3-chlorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (310 mg, 0.898 mmol), 4-[(3-chlorobenzyl)oxy]-1-naphthaldehyde produced in Example (147a) (375 mg, 1.26 mmol), triethylsilane (430 μL, 2.70 mmol), trifluoroacetic acid (210 μL, 2.73 mmol) and acetonitrile (10 mL) to give the target compound (394 mg, yield: 70%) as a yellow solid.
$^1$H-NMR ($CDCl_3$, 400MHz):δ ppm: 1.75 (3H, s), 2.64 (3H, s), 3.69 (3H, s), 4.96 (1H, dd, J=5.2, 14.7Hz), 5.03 (1H, dd, J=5.2, 14.7Hz), 5.24 (2H, s), 6.00 (1H, s), 6.26 (1H,s), 6.80 (1H, d, J=7.9Hz), 7.20 (1H, brs), 7.32-7.43 (4H, m), 7.53-7.63 (3H, m), 8.07 (1H, d, J=7.5Hz), 8.41 (1H, d, J=7.5Hz), 10.65 (1H, s), 18.83 (1H, s).
MS (ESI+) m/z: 626.15685 $(M+H)^+$

(Example 148) (9aS)-8-Acetyl-N-(4-{[(2,4-difluorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (exemplary compound No. 1-91)

(148a) N-(3,5-Dimethylphenyl)-2,4-difluorobenzenesulfonamide

Reaction and post-treatment were carried out in accordance with Example (140a) using 3,5-dimethylaniline (0.81 mL, 6.60 mmol), methylene chloride (16 mL), triethylamine (1.10 mL, 7.92 mmol) and 2,4-difluorobenzenesulfonyl chloride (1.06 mL, 7.92 mmol) to give the target compound (1.94 g, yield: 99%) as a colorless solid.
$^1$H-NMR ($CDCl_3$, 400MHz):δ ppm: 2.22 (6H, s), 6.66 (1H, s), 6.71 (2H, s), 6.75 (1H, s), 6.91-6.95 (2H, m), 7.85 (1H, m)

(148b) 2,4-Difluoro-N-(4-formyl-3,5-dimethylphenyl)benzenesulfonamide

Reaction and post-treatment were carried out in accordance with Example (140b) using N-(3,5-dimethylphenyl)-2,4-difluorobenzenesulfonamide produced in Example (148a) (1.94 g, 6.52 mmol), methylene chloride (30 mL), titanium (IV) chloride (1.43 mL, 13.0 mmol) and dichloromethyl methyl ether (0.71 mL, 7.83 mmol) to give the target compound (945 mg, yield: 45%) as a colorless solid.
$^1$H-NMR ($CDCl_3$, 400MHz):δ ppm: 2.53 (6H, s), 6.80 (2H, s); 6.91-7.03 (3H, m), 7.97 (1H, m), 10.45 (1H, s)

(148c) (9aS)-8-Acetyl-N-(4-{[(2,4-difluorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (250 mg, 0.72 mmol), 2,4-difluoro-N-(4-formyl-3,5-dimethylphenyl)benzenesulfonamide produced in Example (148b) (471 mg, 1.45 mmol), triethylsilane (0.23 mL, 1.45 mmol), trifluoroacetic acid (0.11 mL, 1.45 mmol) and acetonitrile (10 mL) to give the target compound (435 mg, yield: 92%) as a yellow solid.

$^1H$-NMR ($CDCl_3$, 400MHz):δ ppm: 1.72 (3H, s), 2.33 (6H, s), 2.63 (3H, s), 3.76 (3H, s), 4.47-4.58 (2H, m), 5.88 (1H, s), 6.28 (1H, s), 6.77 (1H, m), 6.82 (2H, s), 6.91-6.96 (2H, m), 7.22 (1H, brs), 7.88 (1H, m), 10.64 (1H, s), 18.77 (1H, s)
MS (ESI) m/z: 655 $(M+H)^+$.

(Example 149) (9aS)-8-Acetyl-N-(4-{[(2-chlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (exemplary compound No. 1-90)

(149a) 2-Chloro-N-(3,5-dimethylphenyl)benzenesulfonamide

Reaction and post-treatment were carried out in accordance with Example (140a) using 3,5-dimethylaniline (0.81 mL, 6.60 mmol), methylene chloride (16 mL), triethylamine (1.10 mL, 7.92 mmol), 4-dimethylaminopyridine (81 mg, 0.66 mmol) and 2-chlorobenzenesulfonyl chloride (0.99 mL, 7.26 mmol) to give the target compound (1.93 g, yield: 99%) as a colorless solid.

(149b) 2-Chloro-N-(4-formyl-3,5-dimethylphenyl)benzenesulfonamide

Reaction and post-treatment were carried out in accordance with Example (140b) using 2-chloro-N-(3,5-dimethylphenyl)benzenesulfonamide produced in Example (149a) (1.93 g, 6.52 mmol), methylene chloride (30 mL), titanium (IV) chloride (1.43 mL, 13.0 mmol) and dichloromethyl methyl ether (0.71 mL, 7.83 mmol) to give the target compound (1.53 g, yield: 72%) as a colorless solid.
$^1H$-NMR ($CDCl_3$, 400MHz):δ ppm: 2.51 (6H, s), 6.79 (2H, s), 7.14 (1H, m), 7.39 (1H, m), 7.47-7.50 (2H, m), 8.12 (1H, m), 10.40 (1H, s)

(149c) (9aS)-8-Acetyl-N-(4-{[(2-chlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (250 mg, 0.72 mmol), 2-chloro-N-(4-formyl-3,5-dimethylphenyl)benzenesulfonamide produced in Example (149b) (469 mg, 1.45 mmol), triethylsilane (0.23 mL, 1.45 mmol), trifluoroacetic acid (0.11 mL, 1.45 mmol) and acetonitrile (10 mL) to give the target compound (339 mg, yield: 72%) as a yellow solid.
$^1H$-NMR ($CDCl_3$, 400MHz):δ ppm: 1.73 (3H, s), 2.31 (6H, s), 2.64 (3H, s), 3.75 (3H, s), 4.45-4.56 (2H, m), 5.94 (1H, s), 6.27 (1H, s), 6.72 (1H, m), 6.82 (2H, s), 7.13 (1H, s), 7.37 (1H, m), 7.16-7.52 (2H, m), 8.06 (1H, m), 10.65 (1H, s), 18.80 (1H, s)
MS (ESI) m/z: 653 $(M+H)^+$

(Example 150) (9aS)-8-Acetyl-N-(4-{[(4-chlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (exemplary compound No. 1-88)

(150a) 4-Chloro-N-(3,5-dimethylphenyl)benzenesulfonamide

Reaction and post-treatment were carried out in accordance with Example (140a) using 3,5-dimethylaniline (0.81 mL, 6.60 mmol), methylene chloride (16 mL), triethylamine (1.10 mL, 7.92 mmol), 4-dimethylaminopyridine (81 mg, 0.66 mmol) and 4-chlorobenzenesulfonyl chloride (1.67 mL, 7.92 mmol) to give the target compound (1.43 g, yield: 73%) as a colorless solid.

(150b) 4-Chloro-N-(4-formyl-3,5-dimethylphenyl)benzenesulfonamide

Reaction and post-treatment were carried out in accordance with Example (140b) using 4-chloro-N-(3,5-dimethylphenyl)benzenesulfonamide produced in Example (150a) (1.43 g, 4.83 mmol), methylene chloride (20 mL), titanium (IV) chloride (1.06 mL, 9.67 mmol) and dichloromethyl methyl ether (0.52 mL, 5.80 mmol) to give the target compound (617 mg, yield: 39%) as a colorless solid.
$^1H$-NMR ($CDCl_3$, 400MHz):δ ppm: 2.55 (6H, s), 6.79 (2H, s), 6.84 (1H, brs), 7.45-7.48 (2H, m), 7.78-7.82 (2H, m), 10.47 (1H, s)

(150c) (9aS)-8-Acetyl-N-(4-{[(4-chlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide

Reaction and post-treatment were carried out in accordance with Example (1b) using (9aS)-8-acetyl-1,7-dihydroxy-3-methoxy-9a-methyl-9-oxo-9,9a-dihydrodibenzo[b,d]furan-4-carboxamide produced in Example (1a) (250 mg, 0.72 mmol), 4-chloro-N-(4-formyl-3,5-dimethylphenyl)benzenesulfonamide produced in Example (150b) (469 mg, 1.45 mmol), triethylsilane (0.23 mL, 1.45 mmol), trifluoroacetic acid (0.11 mL, 1.45 mmol) and acetonitrile (10 mL) to give the target compound (332 mg, yield: 70%) as a yellow solid.
$^1$H-NMR (DMSO, 400MHz):δ ppm: 1.67 (3H, s), 2.25 (6H, s), 2.56 (3H, s), 3.69 (3H, s), 4.22-4.32 (2H, m), 6.08 (1H, brs), 6.31 (1H, s), 6.75 (1H, s), 7.61 (2H, d, J=8.7Hz), 7.76 (2H, d, J=8.7Hz), 8.15 (1H, m), 10.25 (1H, s)
MS (ESI) m/z: 653 $(M+H)^+$.

(Example 151) 4-[({[(9aS)-8-Acetyl-1,7-dihydroxy-3-methoxy-9a-methyl-9-oxo-9,9a-dihydrodibenzo[b,d]furan-4-yl]carbonyl}amino)methyl]-1-naphthyl diethylcarbamate (exemplary compound No. 1-353)

(151a) 4-Formyl-1-naphthyl diethylcarbamate

4-Hydroxy-1-naphthaldehyde (200 mg, 1.16 mmol) was dissolved in pyridine (5 mL). Diethylcarbamoyl chloride (660 μL, 5.21 mmol) was added, and the mixture was stirred at 50˚C for one hour. A saturated ammonium chloride aqueous solution (10 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 3/1) to give the target compound (291 mg, yield: 92%) as a light yellow solid.
$^1$H-NMR ($CDCl_3$, 400MHz):δ ppm: 1.27 (3H, t, J=7.0Hz), 1.39 (3H, t, J=7.0 Hz), 3.46 (2H, q, J=7.0 Hz), 3.62 (2H, q, J=7.0 Hz), 7.49 (1H, d, J=7.8Hz), 7.60 (1H, m), 7.69 (1H, m), 7.98 (1H, d, J=7.8Hz), 8.05 (1H, brd, J=8.6Hz), 9.28 (1H, d, J=8.6Hz), 10.30 (1H, s).
MS (EI) m/z: 271 $(M^+)$.

(151b) 4-[({[(9aS)-8-Acetyl-1,7-dihydroxy-3-methoxy-9a-methyl-9-oxo-9,9a-dihydrodibenzo[b,d]furan-4-yl]carbonyl} amino)methyl]-1-naphthyl diethylcarbamate

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (370 mg, 1.07 mmol), 4-formyl-1-naphthyl diethylcarbamate produced in Example (151a) (291 mg, 1.07 mmol), triethylsilane (520 μL, 3.22 mmol), trifluoroacetic acid (250 μL, 3.25 mmol) and acetonitrile (10 mL) to give the target compound (386 mg, yield: 60%) as a yellow solid.
$^1$H-NMR ($CDCl_3$, 400MHz):δ ppm: 1.26 (3H, m), 1.39 (3H, t, J=6.7Hz), 1.75 (3H, s), 2.64 (3H, s), 3.45 (2H, q, J=6.7Hz), 3.63 (2H, q, J=6.7Hz), 3.71 (3H, s), 5.12-4.98 (2H, m), 6.00 (1H, s), 6.27 (1H, s), 7.28-7.23 (2H, m), 7.60-7.49 (3H, m), 7.98 (1H, brd, J=7.9Hz), 8.11 (1H, brd, J=7.5Hz), 10.66 (1H, s), 18.83 (1H, s).
MS (ESI) m/z: 601.21498 $(M+H)^+$.

(Example 152) (9aS)-8-Acetyl-N-{4-[(2,4-dichlorobenzyl)oxy]-2,3,5,6-tetramethylbenzyl}-1,7-dihydroxy-3-methoxy-9a-methyl-9-oxo-9,9a-dihydrodibenzo[b,d]furan-4-carboxamide (exemplary compound No. 1-183)

(152a) 4-[(2,4-Dichlorobenzyl)oxy]-2,3,5,6-tetramethylbenzaldehyde

Known 4-hydroxy-2,3,5,6-tetramethylbenzaldehyde [Journal of Organic Chemistry, 1962, Vol. 27, p.1839-1842] (0.250 g, 1.40 mmol) was dissolved in N,N-dimethylformamide (5 mL). Sodium hydride (0.067 g, 2.81 mmol) was added at 0˚C, and the mixture was stirred in a nitrogen atmosphere for 30 minutes. 2,4-Dichlorobenzyl chloride (0.390 mL, 2.81 mmol) was further added, and the mixture was stirred at room temperature for 30 minutes. After completion of the reaction, a dilute hydrochloric acid solution (25 mL) was added to the reaction solution, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.473 g, yield: 98%) as white crystals.
$^1$H-NMR ($CDCl_3$, 400MHz):δ ppm: 2.22 (6H, s), 2.44 (6H, s), 4.48 (2H, s), 7.35 (1H, dd, J=2.0, 8.3Hz), 7.44 (1H, d, J=2.0Hz), 7.66 (1H, d, J=8.3Hz), 10.62 (1H, s).
MS (FAB) m/z: 337 $(M+H)^+$

(152b) (9aS)-8-Acetyl-N-{4-[(2,4-dichlorobenzyl)oxy]-2,3,5,6-tetramethylbenzyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.450 g, 1.30 mmol), 4-[(2,4-dichlorobenzyl)oxy]-2,3,5,6-tetramethylbenzaldehyde produced in Example (152a) (0.465 g, 1.38 mmol), triethylsilane (0.830 mL, 5.21 mmol), trifluoroacetic acid (0.401 mL, 5.21 mmol) and acetonitrile (15 mL) to give the target compound (0.558 g, yield: 64%) as a yellow solid.
$^1$H-NMR ($CDCl_3$, 500MHz):δ ppm: 1.75 (3H, s), 2.23 (6H, s), 2.33 (6H, s), 2.65 (3H, s), 3.80 (3H, s), 4.63-4.70 (2H, m), 4.78 (2H, s), 6.02 (1H, s), 6.29 (1H, s), 6.74 (1H, brs), 7.35 (1H, dd, J=2.0, 8.3Hz), 7.43 (1H, d, J=2.0Hz), 7.71 (1H, d, J=8.3Hz), 10.64 (1H, s), 18.83 (1H, s).
MS (ESI) m/z: 666.1693 $(M+H)^+$

(Example 153) (9aS)-8-Acetyl-N-[4-(benzyloxy)-2,3,5,6-tetramethylbenzyl]-1,7-dihydroxy-3-methoxy-9a-methyl-9-oxo-9,9a-dihydrodibenzo[b,d]furan-4-carboxamide (exemplary compound No. 1-171)

(153a) 4-(Benzyloxy)-2,3,5,6-tetramethylbenzaldehyde

Known 4-hydroxy-2,3,5,6-tetramethylbenzaldehyde [Journal of Organic Chemistry, 1962, Vol. 27, p.1839-1842] (0.300 g, 1.68 mmol) was dissolved in N,N-dimethylformamide (5 mL). Sodium hydride (0.081 g, 3.37 mmol) was added at 0˚C, and the mixture was stirred in a nitrogen atmosphere for 30 minutes. Benzyl bromide (0.400 mL, 3.37 mmol) was further added, and the mixture was stirred at room temperature for 30 minutes. After completion of the reaction, a dilute hydrochloric acid solution (25 mL) was added to the reaction solution, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.428 g, yield: 95%) as white crystals.
$^1$H-NMR ($CDCl_3$, 500MHz):δ ppm: 2.25 (6H, s), 2.44 (6H, s), 4.74 (2H, s), 7.38 (1H, d, J=7.3Hz), 7.42 (2H, t, J=7.3Hz), 7.49 (2H, d, J=7.3Hz), 10.62 (1H, s).
MS (FAB) m/z: 269 $(M+H)^+$

(153b) (9aS)-8-Acetyl-N-[4-(benzyloxy)-2,3,5,6-tetramethylbenzyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.500 g, 1.45 mmol), 4-(benzyloxy)-2,3,5,6-tetramethylbenzaldehyde produced in Example (153a) (0.425 g, 1.58 mmol), triethylsilane (0.923 mL, 5.79 mmol), trifluoroacetic acid (0.446 mL, 5.79 mmol) and acetonitrile (15 mL) to give the target compound (0.695 g, yield: 80%) as a yellow solid.
$^1$H-NMR ($CDCl_3$, 400MHz):δ ppm: 1.75 (3H, s), 2.27 (6H, s), 2.32 (6H, s), 2.64 (3H, s), 3.79 (3H, s), 4.60-4.71 (2H, m), 4.71 (2H, s), 6.01 (1H, s), 6.27 (1H, s), 6.70 (1H, brs), 7.34-7.42 (3H, m), 7.49 (2H, d, J=7.1Hz), 10.61 (1H, s), 18.78 (1H, s).
MS (ESI) m/z: 598.2483 $(M+H)^+$.

(Example 154) (9aS)-8-Acetyl-1,7-dihydroxy-3-methoxy-9a-methyl-9-oxo-N-[2,3,5,6-tetramethyl-4-(2-pentynyloxy)benzyl]-9,9a-dihydrodibenzo[b,d]furan-4-carboxamide (exemplary compound No. 1-173)

(154a) 2,3,5,6-Tetramethyl-4-(2-pentynyloxy)benzaldehyde

Known 4-hydroxy-2,3,5,6-tetramethylbenzaldehyde [Journal of Organic Chemistry, 1962, Vol. 27, p.1839-1842] (0.300 g, 1.68 mmol) was dissolved in N,N-dimethylformamide (5 mL). Sodium hydride (0.081 g, 3.37 mmol) was added at 0˚C, and the mixture was stirred in a nitrogen atmosphere for 30 minutes. 1-Bromo-2-pentyne (0.345 mL, 3.37 mmol) was further added, and the mixture was stirred at 0˚C for 30 minutes. After completion of the reaction, a dilute hydrochloric acid solution (25 mL) was added to the reaction solution, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.385 g, yield: 94%) as white crystals.
$^1$H-NMR ($CDCl_3$, 500MHz):δ ppm: 1.15 (3H, t, J=7.5Hz), 2.22-2.27 (2H, m), 2.26 (6H, s), 2.42 (6H, s), 4.41 (2H, t, J=2.2Hz), 10.60 (1H, s).
MS (FAB) m/z: 245 $(M+H)^+$

(154b)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.500 g, 1.45 mmol), 2,3,5,6-tetramethyl-4-(2-pentynyloxy)benzaldehyde produced in Example (154a) (0.380 g, 1.56 mmol), triethylsilane (0.923 mL, 5.79 mmol), trifluoroacetic acid (0.446 mL, 5.79 mmol) and acetonitrile (15 mL) to give the target compound (0.549 g, yield: 66%) as a yellow solid.
$^1$H-NMR ($CDCl_3$, 400MHz):δ ppm: 1.16 (3H, t, J=7.5Hz), 1.75 (3H, s), 2.23-2.30 (2H, m), 2.25 (6H, s), 2.30 (6H, s), 2.64 (3H, s), 3.78 (3H, s), 4.35 (2H, t, J=2.2Hz), 4.58-4.68 (2H, m), 6.00 (1H, s), 6.26 (1H, s), 6.68 (1H, brs), 10.60 (1H, s), 18.78 (1H, s).
MS (ESI) m/z: 574.2441 $(M+H)^+$

(Example 155) (9aS)-8-Acetyl-N-{4-[(4-fluorobenzyl)oxy]-2,3,5,6-tetramethylbenzyl}-1,7-dihydroxy-3-methoxy-9a-methyl-9-oxo-9,9a-dihydrodibenzo[b,d]furan-4-carboxamide (exemplary compound No. 1-174)

(155a) 4-[(4-Fluorobenzyl)oxy]-2,3,5,6-tetramethylbenzaldehyde

Known 4-hydroxy-2,3,5,6-tetramethylbenzaldehyde [Journal of Organic Chemistry, 1962, Vol. 27, p.1839-1842] (0.300 g, 1.68 mmol) was dissolved in N,N-dimethylformamide (6 mL). Sodium hydride (0.081 g, 3.37 mmol) was added at 0˚C, and the mixture was stirred in a nitrogen atmosphere for 30 minutes. 4-Fluorobenzyl bromide (0.420 mL, 3.37 mmol) was further added, and the mixture was stirred at 0˚C for 30 minutes. After completion of the reaction, a dilute hydrochloric acid solution (25 mL) was added to the reaction solution, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.450 g, yield: 93%) as white crystals.
$^1$H-NMR ($CDCl_3$, 500MHz):δ ppm: 2.23 (6H, s), 2.44 (6H, s), 4.70 (2H, s), 7.10 (2H, t, J=8.6Hz), 7.45 (2H, dd, J=5.4Hz, J=8.6Hz), 10.62 (1H, s).
MS (EI) m/z: 286 $(M^+)$

(155b) (9aS)-8-Acetyl-N-{4-[(4-fluorobenzyl)oxy]-2,3,5,6-tetramethylbenzyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.500 g, 1.45 mmol), 4-[(4-fluorobenzyl)oxy]-2,3,5,6-tetramethylbenzaldehyde produced in Example (155a) (0.450 g, 1.57 mmol), triethylsilane (0.923 mL, 5.79 mmol), trifluoroacetic acid (0.446 mL, 5.79 mmol) and acetonitrile (15 mL) to give the target compound (0.616 g, yield: 69%) as a yellow solid.
$^1$H-NMR ($CDCl_3$, 400MHz):δ ppm: 1.75 (3H, s), 2.25 (6H, s), 2.32 (6H, s), 2.64 (3H, s), 3.79 (3H, s), 4.60-4.70 (2H, m), 4.67 (2H, s), 6.01 (1H, s), 6.27 (1H, s), 6.71 (1H, brs), 7.08 (2H, t, J=8.6Hz), 7.45 (2H, dd, J=5.4Hz, J=8.6Hz), 10.61 (1H, s), 18.78 (1H, s).
MS (ESI) m/z: 616.2377 $(M+H)^+$.

(Example 156) (9aS)-8-Acetyl-1,7-dihydroxy-3-methoxy-9a-methyl-9-oxo-N-(2,3,5,6-tetramethyl-4-propoxybenzyl)-9-oxo-9,9a-dihydrodibenzo[b,d]furan-4-carboxamide (exemplary compound No. 1-169)

(156a) 2,3,5,6-Tetramethyl-4-propoxybenzaldehyde

Known 4-hydroxy-2,3,5,6-tetramethylbenzaldehyde [Journal of Organic Chemistry, 1962, Vol. 27, p.1839-1842] (0.300 g, 1.68 mmol) was dissolved in N,N-dimethylformamide (6 mL). Sodium hydride (0.081 g, 3.37 mmol) was added at 0˚C, and the mixture was stirred in a nitrogen atmosphere for 30 minutes. n-Propyl iodide (0.329 mL, 3.37 mmol) was further added, and the mixture was stirred at 0˚C for 30 minutes. After completion of the reaction, a dilute hydrochloric acid solution (25 mL) was added to the reaction solution, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.370 g, yield: 100%) as a colorless oil.
$^1$H-NMR ($CDCl_3$, 400MHz):δ ppm: 1.09 (3H, t, J=7.2Hz), 1.81-1.90 (2H, m), 2.22 (6H, s), 2.43 (6H, s), 3.65 (2H, t, J=6.7Hz), 10.60 (1H, s).
MS (EI) m/z: 220 $(M^+)$

(156b) (9aS)-8-Acetyl-1,7-dihydroxy-3-methoxy-9a-methyl-9-oxo-N-(2,3,5,6-tetramethyl-4-propoxybenzyl)-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.500 g, 1.45 mmol), 2,3,5,6-tetramethyl-4-propoxybenzaldehyde produced in Example (156a) (0.370 g, 1.68 mmol), triethylsilane (0.923 mL, 5.79 mmol), trifluoroacetic acid (0.446 mL, 5.79 mmol) and acetonitrile (20.0 mL) to give the target compound (0.606 g, yield: 76%) as a yellow solid.
$^1$H-NMR ($CDCl_3$, 400MHz):δ ppm: 1.08 (3H, t, J=7.2Hz), 1.75 (3H, s), 1.79-1.88 (2H, m), 2.21 (6H, s), 2.30 (6H, s), 2.64 (3H, s), 3.62 (2H, t, J=6.7Hz), 3.77 (3H, s), 4.58-4.68 (2H, m), 6.00 (1H, s), 6.26 (1H, s), 6.66 (1H, brs), 10.60 (1H, s), 18.78 (1H, s).
MS (ESI) m/z: 550.2427 $(M+H)^+$

(Example 157) (9aS)-8-Acetyl-1,7-dihydroxy-N-(4-hydroxy-2,3,5,6-tetramethylbenzyl)-3-methoxy-9a-methyl-9-oxo-9,9a-dihydrodibenzo[b,d]furan-4-carboxamide (exemplary compound No. 1-168)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.400 g, 1.16 mmol), known 4-hydroxy-2,3,5,6-tetramethylbenzaldehyde [Journal of Organic Chemistry, 1962, Vol. 27, p. 1839-1842] (0.206 g, 1.16 mmol), triethylsilane (0.738 mL, 4.63 mmol), trifluoroacetic acid (0.357 mL, 4.63 mmol) and acetonitrile (15 mL) to give the target compound (0.100 g, yield: 17%) as a yellow solid.
$^1$H-NMR ($CDCl_3$, 500MHz):δ ppm: 1.74 (3H, s), 2.20 (6H, s), 2.32 (6H, s), 2.64 (3H, s), 3.78 (3H, s), 4.59-4.67 (2H, m), 5.98 (1H, s), 6.28 (1H, s), 6.67 (1H, brs), 10.62 (1H, s), 18.81 (1H, s).
MS (ESI) m/z: 508.2003 $(M+H)^+$

(Example 158) (9aS)-1,7-Dihydroxy-3-methoxy-9a-methyl-N-(4-hydroxy-2,3,6-trimethylbenzyl)-9-oxo-8-[(1E)-N-(prop-2-ynyloxy)ethaneimidoyl]-9,9a-dihydrodibenzo[b,d]furan-4-carboxamide (exemplary compound No. 3-65)

(9aS)-8-Acetyl-1,7-dihydroxy-N-(4-hydroxy-2,3,6-trimethylbenzyl)-3-methoxy-9a-methyl-9-oxo-9,9a-dihydrodibenzo[b,d]furan-4-carboxamide produced in Example (132b) (252 mg, 0.51 mmol) was dissolved in tetrahydrofuran: methanol (2:1, 5 mL). O-Propargylhydroxylamine hydrochloride (82 mg, 0.77 mmol) and sodium bicarbonate (64 mg, 0.77 mmol) were added at room temperature, and the mixture was stirred for four hours. The reaction solution was treated in the same manner as in Example 87 to give the title target compound (136 mg, 49%) as a yellow solid.
$^1$H-NMR ($CDCl_3$, 400MHz):δ ppm: 1.70 (3H, s), 2.16 (3H, s), 2.29 (3H, s), 2.32 (3H, s), 2.49 (3H, s), 2.61 (1H, m), 3.77 (3H, s), 4.53-4.63 (2H, m), 4.67 (2H, d, J=2.4Hz), 5.78 (1H, s), 5,80 (1H, s), 6.23 (1H, s), 6.56 (1H, s), 6.70 (1H, m), 11.06 (1H, s), 15.36 (1H, brs)
MS (ESI) m/z: 547 $(M+H)^+$

(Example 159) (9aS)-8-[(1E)-N-(Buta-2-ynyloxy)ethaneimidoyl]-1,7-dihydroxy-3-methoxy-9a-methyl-N-[(2-methyl-1-naphthyl)methyl]-9-oxo-9,9a-dihydrodibenzo[b,d]furan-4-carboxamide (exemplary compound No. 3-76)

(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamide produced in Example 27 (250 mg, 0.50 mmol) was dissolved in tetrahydrofuran:methanol (2:1, 5 mL). O-(2-Butynyl)-hydroxylamine hydrochloride (91 mg, 0.75 mmol) and sodium bicarbonate (63 mg, 0.75 mmol) were added at room temperature, and the mixture was stirred for four hours. The reaction solution was treated in the same manner as in Example 87 to give the title target compound (235 mg, 83%) as a yellow solid.
$^1$H-NMR ($CDCl_3$, 400MHz):δ ppm: 1.71 (3H, s), 1.91 (3H, s), 2.52 (3H, s), 2.62 (3H, s), 3.59 (3H, s), 4.62 (2H, m), 5.01-5.13 (2H, m), 5.89 (1H, s), 6.16 (1H, s), 7.01 (1H, m), 7.33 (1H, d, J=8.3 Hz), 7.43 (1H, m), 7.53 (1H, m), 7.72 (1H, d, J=8.8Hz), 7.81 (1H, d, J=8.3Hz), 8.11 (1H, d, J=8.7Hz), 11.16 (1H, s), 15.59 (1H, s).
MS (ESI) m/z: 567 $(M+H)^+$

(Example 160) (9aS)-8-Acetyl-1,7-dihydroxy-3-methoxy-9a-methyl-9-oxo-N-(2,3,6-trimethyl-4-propoxybenzyl)-9-oxo-9,9a-dihydrodibenzo[b,d]furan-4-carboxamide (exemplary compound No. 1-132)

(9aS)-8-Acetyl-1,7-dihydroxy-N-(4-hydroxy-2,3,6-trimethylbenzyl)-3-methoxy-9a-methyl-9-oxo-9,9a-dihydrodibenzo[b,d]furan-4-carboxamide produced in Example (132b) (0.400 g, 0.811 mmol) was dissolved in N,N-dimethylformamide (5 mL). Potassium carbonate (0.448 g, 3.24 mmol) and n-propyl iodide (0.316 mL, 3.24 mmol) were added, and the mixture was stirred in a nitrogen atmosphere at 50°C for 12 hours. After completion of the reaction, the reaction

solution was cooled to 0˚C. 0.1 N hydrochloric acid (10 mL) was added, followed by extraction with ethyl acetate (10 mL). The organic layer was washed with brine and then dried over anhydrous sodium sulfate. The solvent was evaporated under reduced pressure, and the resulting residue was purified by silica gel column chromatography (elution solvent: methylene chloride/methanol = 80/1 to 40/1) to give the target compound (0.228 g, yield: 53%) as a yellow solid.
$^1$H-NMR ($CDCl_3$, 400MHz):δ ppm: 1.05 (3H, t, J=7.3Hz), 1.75 (3H, s), 1.79-1.85 (2H, m), 2.17 (3H, s), 2.30 (3H, s), 2.39 (3H, s), 2.64 (3H, s), 3.78 (3H, s), 3.90 (2H, t, J=6.3Hz), 4.54-4.64 (2H, m), 6.00 (1H, s), 6.26 (1H, s), 6.57 (1H, s), 6.63 (1H, brs), 10.60 (1H, s), 18.78 (1H, s).
MS (ESI) m/z: 536.2287 $(M+H)^+$.

(Example 161) (9aS)-8-Acetyl-N-[4-(2-butynyloxy)-2,3,5,6-tetramethylbenzyl]-1,7-dihydroxy-3-methoxy-9a-methyl-9-oxo-9,9a-dihydrodibenzo[b,d]furan-4-carboxamide (exemplary compound No. 1-172)

(161 a) 4-(2-Butynyloxy)-2,3,5,6,-tetramethylbenzaldehyde

Known 4-hydroxy-2,3,5,6-tetramethylbenzaldehyde [Journal of Organic Chemistry, 1962, Vol. 27, p.1839-1842] (0.300 g, 1.68 mmol) was dissolved in N,N-dimethylformamide (6 mL). Sodium hydride (0.081 g, 3.37 mmol) was added at 0˚C, and the mixture was stirred in a nitrogen atmosphere for 30 minutes. 1-Bromo-2-butyne (0.304 mL, 3.37 mmol) was further added, and the mixture was stirred at 0˚C for 30 minutes. After completion of the reaction, a dilute hydrochloric acid solution (25 mL) was added to the reaction solution, followed by extraction with ethyl acetate (20 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 90/10) to give the target compound (0.345 g, yield: 89%) as white crystals.
$^1$H-NMR ($CDCl_3$, 400MHz):δ ppm: 1.89 (3H, s), 2.25 (6H, s), 2.42 (6H, s), 4.37 (2H, s), 10.60 (1H, s).
MS (EI) m/z: 230 $(M^+)$

(161b) (9aS)-8-Acetyl-N-[4-(2-butynyloxy)-2,3,5,6,-tetramethylbenzyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.500 g, 1.45 mmol), 4-(2-butynyloxy)-2,3,5,6,-tetramethylbenzaldehyde produced in Example (161a) (0.345 g, 1.50 mmol), triethylsilane (0.923 mL, 5.79 mmol), trifluoroacetic acid (0.446 mL, 5.79 mmol) and acetonitrile (20.0 mL) to give the target compound (0.541 g, yield: 67%) as a yellow solid.
$^1$H-NMR ($CDCl_3$, 400MHz):δ ppm: 1.75 (3H, s), 1.89 (3H, s), 2.25 (6H, s), 2.30 (6H, s), 2.64 (3H, s), 3.78 (3H, s), 4.33 (2H, s), 4.57-4.68 (2H, m), 6.00 (1H, s), 6.26 (1H, s), 6.68 (1H, brs), 10.60 (1H, s), 18.78 (1H, s).
MS (ESI) m/z: 560.2322 $(M+H)^+$ .

(Example 162) (9aS)-8-[(1E)-N-(Allyloxy)ethaneimidoyl]-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide (exemplary compound No. 3-39)

(9aS)-8-Acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide produced in Example (111b) (0.300 g, 0.436 mmol) was dissolved in tetrahydrofuran (6 mL) and methanol (3 mL). Sodium bicarbonate (0.044 g, 0.524 mmol) and O-allylhydroxylamine hydrochloride (0.057 g, 0.524 mmol) were added, and the mixture was stirred in a nitrogen atmosphere at room temperature for four hours. After completion of the reaction, dilute hydrochloric acid (10 mL) was added, followed by extraction with ethyl acetate (12 mL). The organic layer was washed with brine and then dried over anhydrous sodium sulfate. The solvent was evaporated under reduced pressure, and the resulting residue was purified by silica gel column chromatography (elution solvent: methylene chloride/methanol = 50/1) to give the target compound (0.308 g, yield: 95%) as a yellow powder.
$^1$H-NMR ($CDCl_3$, 500MHz):δ ppm: 1.70 (3H, s), 2.33 (6H, s), 2.55 (3H, brs), 3.74 (3H, s), 4.45-4.56 (2H, m), 4.56 (2H, d, J=5.9Hz), 5.44 (1H, d, J=10.7Hz), 5.86 (1H, d, J=17.1Hz), 5.86 (1H, s), 6.00 (1H, m), 6.22 (1H, s), 6.71 (1H, brs), 6.79 (2H, s), 6.91 (1H, brs), 7.33 (1H, dd, J=2.0, 8.6Hz), 7.52 (1H, d, J=2.0Hz), 7.98 (1H, d, J=8.6Hz), 11.29 (1H, s), 15.90 (1H, s).
MS (ESI) m/z: 742.1381 $(M+H)^+$

(Example 163) (9aS)-8-Acetyl-N-({4-[(3-chlorobenzyl)oxy]-2-methyl-1-naphthyl}methyl)-1,7-dihydroxy-3-methoxy-9a-methyl-9-oxo-9,9a-dihydrodibenzo[b,d]furan-4-carboxamide (exemplary compound No. 1-448)

(163a) 4-[(3-Chlorobenzyl)oxy]-2-methyl-1-naphthaldehyde

Reaction and post-treatment were carried out in accordance with Example (60a) using 4-hydroxy-2-methyl-1-naphthaldehyde produced in Example (136a) (200 mg, 1.07 mmol), sodium hydride (65 mg, 1.63 mmol), 3-chlorobenzyl bromide (285 μL, 2.17 mmol) and N,N-dimethylformamide (15 mL) to give the target compound (283 mg, yield: 85%) as a light yellow oil.
$^1$H-NMR ($CDCl_3$, 400MHz):δ ppm: 2.81 (3H, s), 5.27 (2H, s),6.67 (1H, s), 7.39-7.33 (3H, m), 7.51-7.46 (2H, m), 7.63 (1H, m), 8.30 (1H, d, J=8.6Hz), 9.11 (1H, d, J=8.6Hz), 10.79 (1H, s).
MS (EI) m/z: 310 ($M^+$).

(163b) (9aS)-8-Acetyl-N-({4-[(3-chlorobenzyl)oxy]-2-methyl-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (310 mg, 0.898 mmol), 4-[(3-chlorobenzyl)oxy]-2-methyl-1-naphthaldehyde produced in Example (163a) (283 mg, 0.911 mmol), triethylsilane (435 μL, 2.69 mmol), trifluoroacetic acid (210 μL, 2.73 mmol) and acetonitrile (13 mL) to give the target compound (360 mg, yield: 63%) as a yellow solid.
$^1$H-NMR ($CDCl_3$, 500MHz):δ ppm: 1.74 (3H, s), 2.60 (3H, s), 2.64 (3H, s), 3.62 (3H, s), 4.98 (1H, dd, J=14.2, 4.4Hz), 5.04 (1H, dd, J=14.2, 4.4Hz), 5.22 (2H, s), 5.99 (1H, s), 6.23 (1H, s), 6.73 (1H, s), 6.99 (1H, m), 7.38-7.33 (2H, m), 7.42 (1H, brd, J=7.3Hz), 7.48 (1H, m), 7.54 (1H, s), 7.58 (1H, m), 8.06 (1H, d, J=8.3Hz), 8.35 (1H, d, J=8.3Hz), 10.62 (1H, s), 18.82 (1H, s).
MS (ESI) m/z: 640.17522 $(M+H)^+$.

(Example 164) (9aS)-8-Acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide (exemplary compound No. 1-414)

(164a) 4-(2,4-Dichlorophenoxy)-1-naphthaldehyde

Sodium hydride (78.3 mg, 1.79 mmol) was suspended in dimethyl sulfoxide (10 mL) in a nitrogen atmosphere. Then, 2,4-dichlorophenol (286 mg, 1.75 mmol) was added, and the mixture was stirred at room temperature for 30 minutes. 4-Fluoro-1-naphthaldehyde (207 mg, 1.19 mmol) was further added, and the mixture was stirred at room temperature for four hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 10/1) to give the target compound (165 mg, yield: 42%) as a yellow solid.
$^1$H-NMR ($CDCl_3$, 500MHz):δ ppm: 6.66 (1H, d, J=7.8Hz), 7.16 (1H, d, J=8.8Hz), 7.34 (1H, dd, J=2.4, 8.8Hz), 7.58 (1H, d, J=2.4Hz), 7.68 (1H, t, J=7.3), 7.78 (1H, m), 7.85 (1H, d, J=8.3Hz), 8.48 (1H, d, J=8.3Hz), 9.35 (1H, d, J=8.8Hz), 10.26 (1H, s). MS (EI) m/z: 316.0064 ($M^+$)

(164b) (9aS)-8-Acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (164 mg, 0.475 mmol), 4-(2,4-dichlorophenoxy)-1-naphthaldehyde produced in Example (164a) (159 mg, 0.502 mmol), triethylsilane (230 μL, 1.44 mmol), trifluoroacetic acid (110μ L, 1.43 mmol) and acetonitrile (5 mL) to give the target compound (192 mg, yield: 62%) as a yellow solid.
$^1$H-NMR ($CDCl_3$, 500MHz):δ ppm: 1.75 (3H, s), 2.65 (3H, s), 3.73 (3H, s), 5.02 (1H, dd, J=4.9, 14.7Hz), 5.08 (1H, dd, J=5.4, 14.7Hz), 6.00 (1H, s), 6.28 (1H, s), 6.74 (1H, d, J=7.8Hz), 6.90 (1H, d, J=8.3Hz), 7.19 (1H, d, J=8.8Hz), 7.30 (1H, brs), 7.43 (1H, d, J=7.8Hz), 7.52 (1H, s), 7.57-7.65 (2H, m), 8.13 (1H, d, J=8.3Hz), 8.29 (1H, d, J=8.3Hz), 10.68 (1H, s), 18.83 (1H, s).
MS (ESI+) m/z: 646.10728 $(M+H)^+$

(Example 165) (9aS)-8-Acetyl-N-{[4-(2-chlorophenoxy)-1-naphthyl]methyl}-1,7-dihydroxy-3-methoxy-9a-methyl-9-oxo-9,9a-dihydrodibenzo[b,d]furan-4-carboxamide (exemplary compound No. 1-409)

(165a) 4-(2-Chlorophenoxy)-1-naphthaldehyde

Sodium hydride (165 mg, 3.78 mmol) was suspended in dimethyl sulfoxide (12 mL) in a nitrogen atmosphere. Then, 2-chlorophenol (478 mg, 3.72 mmol) was added, and the mixture was stirred at room temperature for 30 minutes. 4-Fluoro-1-naphthaldehyde (400 mg, 2.30 mmol) was further added, and the mixture was stirred at room temperature for four hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 25/1 to 10/1) to give the target compound (179 mg, yield: 27%) as a yellow oil.
$^1$H-NMR ($CDCl_3$, 400MHz):δ ppm: 6.62 (1H, d, J=7.8Hz), 7.20-7.27 (2H, m), 7.35 (1H, dt, J=1.6, 7.8Hz), 7.54 (1H, dd, J=1.6, 7.8Hz), 7.65 (1H, m), 7.75 (1H, m), 7.81 (1H, d, J=7.8Hz), 8.50 (1H, d, J=8.6Hz), 9.32 (1H, d, J=8.6Hz), 10.21 (1H, s).
MS (EI) m/z: 282.0451 ($M^+$)

(165b) (9aS)-8-Acetyl-N-{[4-(2-chlorophenoxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (206 mg, 0.597 mmol), 4-(2-chlorophenoxy)-1-naphthaldehyde produced in Example (165a) (169 mg, 0.598 mmol), triethylsilane (435 μL, 2.73 mmol), trifluoroacetic acid (210 μL, 2.73 mmol) and acetonitrile (10 mL) to give the target compound (228 mg, yield: 62%) as a yellow solid.
$^1$H-NMR ($CDCl_3$, 400MHz):δ ppm: 1.75 (3H, s), 2.64 (3H, s), 3.72 (3H, s), 5.00 (1H, dd, J=5.2, 14.7Hz), 5.06 (1H, dd, J=5.2, 14.7Hz), 6.00 (1H, s), 6.26 (1H, s), 6.70 (1H, d, J=7.5Hz), 6.98 (1H, dd, J=1.6, 7.9Hz), 7.11 (1H, m), 7.19-7.27 (2H, m), 7.39 (1H, d, J=7.5Hz), 7.50 (1H, dd, J=2.0, 7.9Hz), 7.54-7.64 (2H, m), 8.11 (1H, d, J=7.9Hz), 8.35 (1H, d, J=8.3Hz), 10.65 (1H, s), 18.79 (1H, s).
MS (ESI+) m/z: 612.14348 $(M+H)^+$.

(Example 166) (9aS)-8-Acetyl-N- {[4-(3-chlorophenoxy)-1-naphthyl]methyl}-1,7-dihydroxy-3-methoxy-9a-methyl-9-oxo-9,9a-dihydrodibenzo[b,d]furan-4-carboxamide (exemplary compound No. 1-408)

(166a) 4-(3-Chlorophenoxy)-1-naphthaldehyde

Sodium hydride (156 mg, 3.58 mmol) was suspended in dimethyl sulfoxide (12 mL) in a nitrogen atmosphere. Then, 3-chlorophenol (484 mg, 3.76 mmol) was added, and the mixture was stirred at room temperature for 30 minutes. 4-Fluoro-1-naphthaldehyde (403 mg, 2.31 mmol) was further added, and the mixture was stirred at room temperature for four hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 50/1 to 12/1) to give the target compound (466 mg, yield: 71%) as a yellow solid.
$^1$H-NMR ($CDCl_3$, 400MHz):δ ppm: 6.86 (1H, d, J=7.8Hz), 7.04 (1H, m), 7.16 (1H, t, J=2.1Hz), 7.23 (1H, m), 7.36 (1H, t, J=8.0Hz), 7.63 (1H, m), 7.75 (1H, m), 7.86 (1H, d, J=7.8Hz), 8.37 (1H, d, J=8.2Hz), 9.32 (1H, d, J=8.6Hz), 10.24 (1H, s).
MS (EI) m/z: 282.0449 ($M^+$)

(166b) (9aS)-8-Acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (311 mg, 0.901 mmol), 4-(3-chlorophenoxy)-1-naphthaldehyde produced in Example (1 66a) (458 mg, 1.62 mmol), triethylsilane (435 μL, 2.73 mmol), trifluoroacetic acid (210 μL, 2.73 mmol) and acetonitrile (10 mL) to give the target compound (442 mg, yield: 80%) as a yellow solid.
$^1$H-NMR ($CDCl_3$, 400MHz):δ ppm: 1.75 (3H, s), 2.64 (3H, s), 3.74 (3H, s), 5.02 (1H, dd, J=5.6, 14.7Hz), 5.09 (1H, dd, J=5.6, 14.7Hz), 6.00 (1H, s), 6.27 (1H, s), 6.90-6.95 (2H, m), 6.99 (1H, m), 7.07 (1H, m), 7.22-7.31 (2H, m), 7.46 (1H, d, J=7.5Hz), 7.53 (1H, m), 7.61 (1H, m), 8.12 (1H, d, J=8.3Hz), 8.18 (1H, d, J=8.3Hz), 10.66 (1H, s), 18.79 (1H,s).

MS (ESI+) m/z: 612.14378 $(M+H)^+$

(Example 167) (9aS)-8-Acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide (exemplary compound No. 1-407)

(167a) 4-(4-Chlorophenoxy)-1-naphthaldehyde

Sodium hydride (166 mg, 3.80 mmol) was suspended in dimethyl sulfoxide (12 mL) in a nitrogen atmosphere. Then, 4-chlorophenol (485 mg, 3.77 mmol) was added, and the mixture was stirred at room temperature for 30 minutes. 4-Fluoro-1-naphthaldehyde (402 mg, 2.31 mmol) was further added, and the mixture was stirred at room temperature for four hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 50/1 to 15/1) to give the target compound (381 mg, yield: 58%) as a yellow solid.
$^1$H-NMR ($CDCl_3$, 400MHz):δ ppm: 6.80 (1H, d, J=8.2Hz), 7.08-7.12 (2H, m), 7.38-7.42 (2H, m), 7.63 (1H, m), 7.75 (1H, m), 7.84 (1H, d, J=7.8Hz), 8.40 (1H, d, J=8.6Hz), 9.32 (1H, d, J=8.6Hz), 10.22 (1H, s).
MS (EI) m/z: 282.0449 $(M^+)$

(167b) (9aS)-8-Acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (312 mg, 0.904 mmol), 4-(4-chlorophenoxy)-1-naphthaldehyde produced in Example (167a) (371 mg, 1.31 mmol), triethylsilane (435 μL, 2.73 mmol), trifluoroacetic acid (210 μL, 2.73 mmol) and acetonitrile (10 mL) to give the target compound (438 mg, yield: 79%) as a yellow solid.
$^1$H-NMR ($CDCl_3$, 400MHz):δ ppm: 1.75 (3H, s), 2.64 (3H, s), 3.73 (3H, s), 5.01 (1H, dd, J=5.2, 14.7Hz), 5.08 (1H, dd, J=5.2, 14.7Hz), 6.00 (1H, s), 6.27 (1H, s), 6.87 (1H, d, J=7.9Hz), 6.94-6.98 (2H, m), 7.26-7.30 (3H, m), 7.43 (1H, d, J=7.5Hz), 7.53 (1H, m), 7.61 (1H, m), 8.11 (1H, d, J=8.3Hz), 8.22 (1H, d, J=8.3Hz), 10.66 (1H, s), 18.79 (1H, s).
MS (ESI+) m/z: 612.14636 $(M+H)^+$

(Example 168) (9aS)-8-Acetyl-N-({4-[(2-chlorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-425)

(168a) 4-[(2-Chlorobenzyl)oxy]-1-naphthaldehyde

Sodium hydride (97.0 mg, 2.22 mmol) was suspended in N,N-dimethylformamide (10 mL) in a nitrogen atmosphere, and the suspension was cooled to 0˚C. 4-Hydroxy-1-naphthaldehyde (251 mg, 1.46 mmol) was slowly added, and the mixture was stirred at 0˚C for 30 minutes. 1-(Bromomethyl)-2-chlorobenzene (280 μL, 2.15 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 7/1) to give the target compound (403 mg, yield: 93%) as a light yellow solid.
$^1$H-NMR ($CDCl_3$, 500MHz):δ ppm: 5.47 (2H, s), 7.01 (1H, d, J=7.8Hz), 7.32-7.36 (2H, m), 7.47 (1H, m), 7.59-7.65 (2H, m), 7.72 (1H, m), 7.93 (1H, d, J=7.8Hz), 8.44 (1H, d, J=8.3Hz), 9.33 (1H, d, J=8.8Hz), 10.22 (1H, s).
MS (EI) m/z: 296.0604 $(M^+)$

(168b) (9aS)-8-Acetyl-N-({4-[(2-chlorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (314 mg, 0.909 mmol), 4-[(2-chlorobenzyl)oxy]-1-naphthaldehyde produced in Example (168a) (396 mg, 1.33 mmol), triethylsilane (435 μL, 2.73 mmol), trifluoroacetic acid (210 μL, 2.73 mmol) and acetonitrile (10 mL) to give the target compound (380 mg, yield: 67%) as a yellow solid.
$^1$H-NMR ($CDCl_3$, 400MHz):δ ppm: 1.74 (3H, s), 2.64 (3H, s), 3.68 (3H, s), 4.96 (1H, dd, J=5.2, 14.3Hz), 5.02 (1H, dd,

J=5.2, 14.3Hz), 5.35 (2H, s), 5.99 (1H, s), 6.24 (1H,s), 6.83 (1H, d, J=7.9Hz), 7.18 (1H, brs), 7.26-7.33 (2H, m), 7.40-7.44 (2H, m), 7.51-7.67 (3H, m), 8.05 (1H, d, J=7.9Hz), 8.43 (1H, d, J=7.9Hz), 10.63 (1H, s), 18.78 (1H, s).
MS (ESI+) m/z: 626.16218 $(M+H)^+$.

(Example 169) (9aS)-8-Acetyl-N-({4-[(3-chloro-4-fluorobenzyl)oxy]-1-naphthyl}methyl)-1,7-dihydroxy-3-methoxy-9a-methyl-9-oxo-9,9a-dihydrodibenzo[b,d]furan-4-carboxamide (exemplary compound No. 1-431)

(169a) 4-[(3-Chloro-4-fluorobenzyl)oxy]-1-naphthaldehyde

Sodium hydride (97.0 mg, 2.22 mmol) was suspended in N,N-dimethylformamide (10 mL) in a nitrogen atmosphere, and the suspension was cooled to 0˚C. 4-Hydroxy-1-naphthaldehyde (252 mg, 1.46 mmol) was slowly added, and the mixture was stirred at 0˚C for 30 minutes. 4-(Bromomethyl)-2-chloro-1-fluorobenzene (480 mg, 2.15 mmol) was further added, and the mixture was stirred at room temperature for three hours. A dilute hydrochloric acid solution (30 mL) was added to the reaction solution, followed by extraction with ethyl acetate (50 ml). The organic layer was washed with brine and then dried over anhydrous sodium sulfate. The solvent was evaporated under reduced pressure, and the resulting residue was purified by silica gel column chromatography (elution solvent: hexane/ethyl acetate = 20/1 to 3/1) to give the target compound (371 mg, yield: 81%) as a light yellow solid.
$^1$H-NMR ($CDCl_3$, 500MHz):$\delta$ ppm: 5.29 (2H, s), 6.97 (1H, d, J=7.8Hz), 7.22 (1H, t, J=8.5Hz), 7.40 (1H, m), 7.58-7.62 (2H, m), 7.73 (1H, m), 7.92 (1H, d, J=8.3Hz), 8.37 (1H, d, J=8.8Hz), 9.32 (1H, d, J=8.8Hz), 10.23 (1H, s).
MS (EI) m/z: 314.0511 $(M^+)$

(169b) (9aS)-8-Acetyl-N-({4-[(3-chloro-4-fluorobenzyl)oxy]-1-naphthyl}methyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (312 mg, 0.904 mmol), 4-[(3-chloro-4-fluorobenzyl)oxy]-1-naphthaldehyde produced in Example (169a) (366 mg, 1.16 mmol), triethylsilane (435 $\mu$L, 2.73 mmol), trifluoroacetic acid (210 $\mu$L, 2.73 mmol) and acetonitrile (10 mL) to give the target compound (307 mg, yield: 53%) as a yellow solid.
$^1$H-NMR ($CDCl_3$, 400MHz):$\delta$ ppm: 1.74 (3H, s), 2.64 (3H, s), 3.68 (3H, s), 4.96 (1H, dd, J=5.2, 14.7Hz), 5.02 (1H, dd, J=5.2, 14.7Hz), 5.18 (2H, s), 5.99 (1H, s), 6.24 (1H, s), 6.78 (1H, d, J=7.9Hz), 7.15-7.22 (2H, m), 7.35-7.42 (2H, m), 7.50-7.60 (3H, m), 8.05 (1H, d, J=7.9Hz), 8.35 (1H, d, J=8.3Hz), 10.63 (1H, s), 18.79 (1H, s).
MS (ESI+) m/z: 644.15188 $(M+H)^+$

(Example 170) (9aS)-8-Acetyl-N-(4-{[(2,4-dichlorophenyl)sulfonyl]amino}benzyl)-1,7-dihydroxy-3-methoxy-9a-methyl-9-oxo-9,9a-dihydrodibenzo[b,d]furan-4-carboxamide (exemplary compound No. 1-26)

(170a) Methyl 4-{[(2,4-dichlorophenyl)sulfonyl]amino}benzoate

Methyl 4-aminobenzoate (1.00 g, 6.62 mmol) was dissolved in methylene chloride (30 mL) and pyridine (1 mL), and 2,4-dichlorobenzenesulfonyl chloride (1.00 g, 6.62 mmol) was added at 0˚C. The mixture was heated to room temperature in a nitrogen atmosphere and then stirred for one hour. After completion of the reaction, an ammonium chloride aqueous solution (40 mL) was added, followed by extraction with ethyl acetate (50 mL). The organic layer was washed with brine and then dried over anhydrous sodium sulfate. The solvent was evaporated under reduced pressure, and the resulting residue was purified by silica gel column chromatography (elution solvent: hexane = 50/50) to give the target compound (2.14 g, yield: 90%) as white crystals.
$^1$H-NMR ($CDCl_3$, 400MHz):$\delta$ ppm: 3.85 (3H, s), 7.13 (2H, d, J=9.0Hz), 7.14 (1H, brs), 7.32 (1H, dd, J=2.0, 8.6Hz), 7.48 (1H, d, J=2.0Hz), 7.89 (2H, d, J=9.0Hz), 7.98 (1H, d, J=8.6Hz).
MS (FAB) m/z: 360 $(M+H)^+$

(170b) 2,4-Dichloro-N-[4-(hydroxymethyl)phenyl]benzenesulfonamide

Methyl 4-{[(2,4-dichlorophenyl)sulfonyl]amino}benzoate produced in Example (170a) (2.14 g, 5.94 mmol) was dissolved in tetrahydrofuran (60 mL), and lithium aluminum hydride (0.676 g, 17.8 mmol) was slowly added at 0˚C. After stirring in a nitrogen atmosphere at 0˚C for 30 minutes, a dilute hydrochloric acid solution (40 mL) and ethyl acetate (30 mL) were added to the reaction solution, and the mixture was stirred at room temperature for one hour. The organic layer was separated, washed with brine and then dried over anhydrous sodium sulfate. The solvent was evaporated under reduced pressure and the residue was dried to give the target crude product (1.97 g) as a yellow oil.

(170c) 4-{[(2,4-Dichlorophenyl)sulfonyl]amino}benzaldehyde

2,4-Dichloro-N-[4-(hydroxymethyl)phenyl]benzenesulfonamide produced in Example (170b) (1.97 g) was dissolved in chloroform (60 mL). Then, manganese dioxide (7.75 g, 89.1 mmol) was added, and the mixture was stirred in a nitrogen atmosphere at room temperature for 1.5 hours. Manganese dioxide was separated by filtration and then the solvent was evaporated under reduced pressure. The resulting residue was purified by silica gel column chromatography (elution solvent: hexane/ethyl acetate = 80/20 to 60/40) to give the target compound (1.31 g, yield: 67%, two steps) as a yellowish brown solid.
$^1$H-NMR ($CDCl_3$, 400MHz):$\delta$ ppm: 7.25 (2H, d, J=8.6Hz), 7.30 (1H, brs), 7.38 (1H, dd, J=2.0, 8.6Hz), 7.51 (1H, d, J=2.0Hz), 7.77 (2H, d, J=8.6Hz), 8.05 (1H, d, J=8.6Hz), 9.88 (1H, s).
MS (FAB) m/z: 330 $(M+H)^+$

(170d) (9aS)-8-Acetyl-N-(4-{[(2,4-dichlorophenyl)sulfonyl]amino}benzyl)-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.136 g, 0.397 mmol), 4-{[(2,4-dichlorophenyl)sulfonyl]amino}benzaldehyde produced in Example (170c) (0.137 g, 0.397 mmol), triethylsilane (0.253 mL, 1.59 mmol), trifluoroacetic acid (0.122 mL, 1.59 mmol) and acetonitrile (15.0 mL) to give the target compound (0.187 g, yield: 72%) as a yellow solid.
$^1$H-NMR ($CDCl_3$, 400MHz):$\delta$ ppm: 1.74 (3H, s), 2.64 (3H, s), 3.85 (3H, s), 4.48-4.60 (2H, m), 5.98 (1H, s), 6.32 (1H, s), 7.00 (1H, brs), 7.07 (2H, d, J=8.3Hz), 7.21 (2H, d, J=8.3Hz), 7.29 (1H, dd, J=2.0, 8.6Hz), 7.35 (1H, brs), 7.50 (1H, d, J=2.0Hz), 7.90 (1H, d, J=8.6Hz), 10.71 (1H, s), 18.78 (1H, s).
MS (ESI) m/z: 659.0679 $(M+H)^+$.

(Example 171) (9aS)-8-Acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide (exemplary compound No. 1-492)

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (290 mg, 0.840 mmol), known 2,4-dimethyl-1-naphthaldehyde [Journal of Chemical Society Perkin Transactions 1, 1972, p. 892-894] (155 mg, 0.841 mmol), triethylsilane (410 $\mu$L, 2.54 mmol), trifluoroacetic acid (195 $\mu$L, 2.53 mmol) and acetonitrile (10 mL) to give the target compound (312 mg, yield: 73%) as a yellow solid.
$^1$H-NMR ($CDCl_3$, 400MHz):$\delta$ ppm: 1.74 (3H, s), 2.49 (3H, s), 2.59 (3H, s), 2.64 (3H, s), 2.68 (3H, s), 3.62 (3H, s), 5.02 (1H, dd, J=14.2, 4.4Hz), 5.09 (1H, dd, J=14.2, 4.4Hz), 6.01 (1H, s), 6.22 (1H, s), 6.99 (1H, m), 7.21 (1H, s), 7.50 (1H, m), 7.56 (1H, m), 8.01 (1H, brd, J=7.3Hz), 8.12 (1H, brd, J=7.8Hz), 10.62 (1H, s), 18.82 (1H, s). MS (ESI) m/z: 514.18862 $(M+H)^+$.

(Example 172) (9aS)-8-Acetyl-N-[(6-fluoro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-547)

(172a) 2-Bromo-6-fluoro-3,4-dihydronaphthalene-1-carbaldehyde

Phosphorus bromide (1.45 mL, 15.3 mmol) was slowly added dropwise to a solution of N,N-dimethylformamide (1.45 mL, 18.6 mmol) in chloroform (30 mL) at 0˚C, and the mixture was stirred in a nitrogen atmosphere at 0˚C for two hours. Thereafter, a solution of known 6-fluoro-3,4-dihydronaphthalen-2(1H)-one [Journal of Medicinal Chemistry, 1995, Vol. 38, p.2202-2216] (637 mg, 3.88 mmol) in chloroform (10 mL) was added at 0˚C. The mixture was heated to room temperature and then stirred with heating to reflux for two hours. After completion of the reaction, the reaction solution was cooled to 0˚C and then a saturated sodium bicarbonate aqueous solution (50 mL) was added dropwise, followed by extraction with methylene chloride (50 mL). The organic layer was washed with brine and then dried over anhydrous magnesium sulfate. The solvent was evaporated under reduced pressure, and the resulting crude purified product as a light yellow oil was directly used for the next reaction.

(172b) 2-Bromo-6-fluoro-1-naphthaldehyde

2-Bromo-6-fluoro-3,4-dihydronaphthalene-1-carbaldehyde produced in Example (172a) (990 mg, 3.88 mmol) was dissolved in toluene (30 mL). 2,3-Dichloro-5,6-dicyano-1,4-benzoquinone (2.20 g, 9.69 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 10/1) to give the target compound (333 mg, yield: 34%, two steps) as a white solid.
$^1$H-NMR ($CDCl_3$, 400MHz):δ ppm: 7.38-7.46 (2H, m), 7.70 (1H, d, J=8.6Hz), 7.80 (1H, d, J=9.0Hz), 9.13 (1H, dd, J=5.5, 9.4Hz), 10.71 (1H, s).

(172c) 2-Methyl-6-fluoro-1-naphthaldehyde

2-Bromo-6-fluoro-1-naphthaldehyde produced in Example (172b) (330 mg, 1.30 mmol) was dissolved in toluene (13 mL).
Tetrakis(triphenylphosphine)palladium (45 mg, 0.039 mmol) and tetramethyltin (540 μL, 3.40 mmol) were added, and the mixture was stirred with heating to reflux for six hours. After completion of the reaction, the reaction solution was filtered through Celite, and the solvent was evaporated under reduced pressure. The resulting residue was purified by silica gel column chromatography (elution solvent: hexane/ethyl acetate = 10/1) to give the target compound (232 mg, yield: 95%) as a light yellow solid.
$^1$H-NMR ($CDCl_3$, 400MHz):δ ppm: 2.80 (3H, s), 7.34-7.44 (3H, m), 7.86 (1H, d, J-8.3 Hz), 9.05(1H, dd, J=5.9, 9.3Hz), 10.90 (1H, s).
MS (EI) m/z: 188 ($M^+$).

(172d) (9aS)-8-Acetyl-N-[(6-fluoro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (420 mg, 1.22 mmol), 2-methyl-6-fluoro-1-naphthaldehyde produced in Example (172c) (230 mg, 1.22 mmol), triethylsilane (590 μL, 3.65 mmol), trifluoroacetic acid (280 μL, 3.63 mmol) and acetonitrile (18 mL) to give the target compound (495 mg, yield: 79%) as a yellow solid.
$^1$H-NMR ($CDCl_3$, 400MHz):δ ppm: 1.74 (3H, s), 2.61 (3H, s), 2.64 (3H, s), 3.64 (3H, s), 4.98-5.13 (2H, m), 6.01 (1H, s), 6.23 (1H, s), 7.04 (1H, m), 7.31 (1H, m), 7.37 (1H, d, J=8.3Hz), 7.44 (1H, brd, J=9.2Hz), 7.68 (1H, d, J=8.3Hz), 8.13 (1H, m), 10.65 (1H, s), 18.82 (1H, s).
MS (ESI) m/z: 540.14208 $(M+Na)^+$.

(Example 173) (9aS)-8-Acetyl-N-[(6-chloro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-553)

(173a) 2-Bromo-6-chloro-3,4-dihydronaphthalene-1-carbaldehyde

Reaction and post-treatment were carried out in accordance with Example (172a) using known 6-chloro-3,4-dihydronaphthalen-2(1H)-one [Journal of Organic Chemistry, 1968, Vol. 33, p.4288-4290] (1.0 g, 5.54 mmol), N,N-dimethylformamide (1.30 mL, 16.7 mmol), phosphorus bromide (1.35 mL, 14.2 mmol) and chloroform (30 mL) to give a crude purified product as a light yellow oil.
$^1$H-NMR ($CDCl_3$, 400MHz):δ ppm: 2.86 (1H, t, J=7.8Hz), 3.01 (1H, t, J=7.8Hz), 7.20 (1H, dd, J=2.0, 8.2Hz), 7.24 (1H, s), 7.93 (1H, d, J=8.2Hz), 10.26 (1H, s).

(173b) 2-Bromo-6-chloro-1-naphthaldehyde

Reaction and post-treatment were carried out in accordance with Example (172b) using 2-bromo-6-chloro-3,4-dihydronaphthalene-1-carbaldehyde produced in Example (173a) (1.11 g, 4.09 mmol), 2,3-dichloro-5,6-dicyano-1,4-benzoquinone (2.20 g, 9.69 mmol) and toluene (40 mL) to give the target compound (410 mg, yield: 37%, two steps) as a white solid.
$^1$H-NMR ($CDCl_3$, 400MHz):δ ppm: 7.57 (1H, brd, J=9.0Hz), 7.68-7.81 (3H, m), 9.05 (1H, d, J=9.0Hz), 10.69 (1H, s).

(173c) 2-Methyl-6-chloro-1-naphthaldehyde

Reaction and post-treatment were carried out in accordance with Example (172c) using 2-bromo-6-chloro-1-naphthaldehyde produced in Example (173b) (405 mg, 1.50 mmol), tetrakis(triphenylphosphine)palladium (52 mg, 0.045 mmol), tetramethyltin (625 μL, 4.51 mmol) and toluene (15 mL) to give the target compound (285 mg, yield: 93%) as a light yellow solid.
$^1$H-NMR ($CDCl_3$, 400MHz):δ ppm: 2.80 (3H, s), 7.36 (1H, d, J=8.3Hz), 7.53 (1H, dd, J=2.0, 9.3Hz), 7.78 (1H, s), 7.83

(1H, d, J=8.3 Hz), 8.96(1H, d, J=9.3Hz), 10.89 (1H, s).
MS (EI) m/z: 204 ($M^+$).

(173d) (9aS)-8-Acetyl-N-[(6-chloro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (470 mg, 1.36 mmol), 2-methyl-6-chloro-1-naphthaldehyde produced in Example (173c) (283 mg, 1.38 mmol), triethylsilane (660 μL, 4.09 mmol), trifluoroacetic acid (315 μL, 4.09 mmol) and acetonitrile (20 mL) to give the target compound (445 mg, yield: 61 %) as a yellow solid.
$^1$H-NMR ($CDCl_3$, 400MHz):δ ppm: 1.74 (3H, s), 2.61 (3H, s), 2.63 (3H, s), 3.63 (3H, s), 5.00 (1H, dd, J=4.7, 14.3 Hz), 5.08 (1H, dd, J=4.7, 14.3 Hz), 6.00 (1H, s), 6.22 (1H, s), 7.02 (1H, m), 7.36 (1H, d, J=8.3Hz), 7.46 (1H, dd, J=2.1, 9.2Hz), 7.64 (1H, d, J=8.3Hz), 7.79 (1H, d, J=2.1Hz), 8.05 (1H, d, J=9.2Hz), 10.63 (1H, s), 18.78 (1H, s).
MS (ESI) m/z: 534.13247 $(M+H)^+$.

(Example 174) (9aS)-8-Acetyl-N-[(2,6-dimethyl-1-naphthyl)methyl]-1,7-dihydroxy-3-methoxy-9a-methyl-9-oxo-9,9a-dihydrodibenzo[b,d]furan-4-carboxamide (exemplary compound No. 1-596)

(174a) 1-Bromo-2,6-dimethylnaphthalene

2,6-Dimethylnaphthalene (1.0 g, 6.40 mmol) was dissolved in N,N-dimethylformamide (70 mL). N-Bromosuccinimide (1.14 g, 6.41 mmol) was added, and the mixture was stirred at room temperature for 11 hours. After completion of the reaction, water (50 mL) was added to the reaction solution, followed by extraction with hexane (100 mL). The organic layer was dried over anhydrous magnesium sulfate, and then the solvent was evaporated under reduced pressure. The resulting residue was purified by silica gel column chromatography (elution solvent: hexane) to give the target compound (1.29 g, yield: 86%) as a white solid.

(174b) 2,6-Dimethyl-1-naphthaldehyde

1-Bromo-2,6-dimethylnaphthalene produced in Example (174a) (1.29 g, 5.49 mmol) was dissolved in tetrahydrofuran (50 mL). Butyllithium (2.71 M solution in hexane, 3.50 mL, 9.49 mmol) was added dropwise at -78˚C, and the mixture was directly stirred for 30 minutes. N,N-Dimethylformamide (2.00 mL, 25.8 mmol) was further added dropwise, and the mixture was stirred with heating to room temperature for two hours. A saturated ammonium chloride aqueous solution (50 mL) was added, followed by extraction with diethyl ether (100 mL). The organic layer was dried over anhydrous magnesium sulfate. The solvent was evaporated under reduced pressure, and the resulting residue was purified by silica gel column chromatography (elution solvent: hexane/ethyl acetate = 5/1) to give the target compound (352 mg, yield: 35%) as a white solid.
$^1$H-NMR ($CDCl_3$, 400MHz):δ ppm: 2.50 (3H, s), 2.80 (3H, s), 7.29 (1H, d, J=8.6Hz), 7.44 (1H, dd, J=2.0, 9.0H), 7.58 (1H, s), 7.84 (1H, d, J=8.6Hz), 8.85 (1H, d, J=9.0Hz), 10.92 (1H, s).
MS (EI) m/z: 184 ($M^+$).

(174c) (9aS)-8-Acetyl-N-[(2,6-dimethyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (370 mg, 1.07 mmol), 2,6-dimethyl-1-naphthaldehyde produced in Example (174b) (200 mg, 1.09 mmol), triethylsilane (520 μL, 3.22 mmol), trifluoroacetic acid (250 μL, 3.25 mmol) and acetonitrile (15 mL) to give the target compound (421 mg, yield: 77%) as a yellow solid.
$^1$H-NMR ($CDCl_3$, 400MHz):δ ppm: 1.73 (3H, s), 2.50 (1H, s), 2.59 (3H, s), 2.63 (3H, s), 3.62 (3H, s), 5.01 (1H, dd, J=4.7, 14.3Hz), 5.08 (1H, dd, J=4.7, 14.3Hz), 5.99 (1H, s), 6.21 (1H, s), 6.98 (1H, m), 7.29 (1H, d, J=8.3Hz), 7.36 (1H, brd, J=8.7Hz), 7.59 (1H, brs), 7.64 (1H, d, J=8.3Hz), 7.99 (1H, d, J=8.7Hz), 10.60 (1H, s), 18.77 (1H, s). MS (ESI) m/z: 514.18470 $(M+H)^+$.

(Example 175) (9aS)-8-Acetyl-N-[(2,7-dimethyl-1-naphthyl)methyl]-1,7-dihydroxy-3-methoxy-9a-methyl-9-oxo-9,9a-dihydrodibenzo[b,d]furan-4-carboxamide (exemplary compound No. 1-597)

(175a) 1-Bromo-2,7-dimethylnaphthalene

Reaction and post-treatment were carried out in accordance with Example (174a) using 2,7-dimethylnaphthalene (1.0 g, 6.40 mmol), N-bromosuccinimide (1.14 g, 6.41 mmol) and N,N-dimethylformamide (70 mL) to give the target compound (1.39 g, yield: 91%) as a white solid.

(175b) 2,7-Dimethyl-1-naphthaldehyde

Reaction and post-treatment were carried out in accordance with Example (174b) using 1-bromo-2,7-dimethylnaphthalene produced in Example (175a) (1.29 g, 5.49 mmol), butyllithium (2.71 M solution in hexane, 3.50 mL, 9.49 mmol), N,N-dimethylformamide (2.00 mL, 25.8 mmol) and tetrahydrofuran (50 mL) to give the target compound (810 mg, yield: 76%) as a yellow oil.
$^1$H-NMR ($CDCl_3$, 400MHz):δ ppm: 2.56 (3H, s), 2.81 (3H, s), 7.27 (1H, d, J=8.6Hz), 7.34 (1H, d, J=8.2Hz), 7.73 (1H, d, J=8.2Hz), 7.90 (1H, d, J=8.6Hz), 8.78 (1H, s), 10.95 (1H, s).
MS (EI) m/z: 184 ($M^+$).

(175c) (9aS)-8-Acetyl-N-[(2,7-dimethyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (370 mg, 1.07 mmol), 2,7-dimethyl-1-naphthaldehyde produced in Example (175b) (200 mg, 1.09 mmol), triethylsilane (520 μL, 3.22 mmol), trifluoroacetic acid (250 μL, 3.25 mmol) and acetonitrile (15 mL) to give the target compound (411 mg, yield: 75%) as a yellow solid.
$^1$H-NMR ($CDCl_3$, 400MHz):δ ppm: 1.74 (3H, s), 2.54 (3H, s), 2.60 (1H, s), 2.63 (3H, s), 3.62 (3H, s), 4.98-5.09 (2H, m), 5.98 (1H, s), 6.22 (1H, s), 6.94 (1H, m), 7.25-7.30 (2H, m), 7.68 (1H, d, J=8.3Hz), 7.72 (1H, d, J=8.3Hz), 7.84 (1H, brs), 10.60 (1H, s), 18.78 (1H, s).
MS (ESI) m/z: 514.18730 $(M+H)^+$.

(Example 176) (9aS)-8-Acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-613)

(176a) 5,7-Difluoro-2-methyl-1-naphthaldehyde

Reaction and post-treatment were carried out in accordance with Example (172c) using 2-bromo-5,7-difluoro-1-naphthaldehyde produced in Example (185c) (270 mg, 0.996 mmol), tetrakis(triphenylphosphine)palladium (35 mg, 0.030 mmol), tetramethyltin (420 μL, 3.03 mmol) and toluene (10 mL) to give the target compound (184 mg, yield: 90%) as a light yellow solid.
$^1$H-NMR ($CDCl_3$, 400MHz):δ ppm: 2.85 (3H, s), 7.00 (1H, m), 7.35 (1H, d, J=8.2Hz), 8.19 (1H, d, J=8.6Hz), 8.61 (1H, d, J=12.2Hz), 10.85 (1H, s).
MS (EI) m/z: 206 ($M^+$).

(176b) (9aS)-8-Acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (300 mg, 0.869 mmol), 5,7-difluoro-2-methyl-1-naphthaldehyde produced in Example (176a) (184 mg, 0.892 mmol), triethylsilane (420 μL, 2.60 mmol), trifluoroacetic acid (200 μL, 2.60 mmol) and acetonitrile (15 mL) to give the target compound (297 mg, yield: 64%) as a yellow solid.
$^1$H-NMR ($CDCl_3$, 400MHz):δ ppm: 1.74 (3H, s), 2.64 (3H, s), 2.64 (3H, s), 3.69 (3H, s), 4.96 (1H, dd, J=4.2, 14.2Hz), 5.02 (1H, dd, J=4.2, 14.2Hz), 6.02 (1H, s), 6.25 (1H, s), 6.97 (1H, m), 7.03 (1H, m), 7.36 (1H, d, J=8.3Hz), 7.55 (1H, d, J=10.7Hz), 7.97 (1H, d, J=8.3Hz), 10.65 (1H, s), 18.82 (1H, s).
MS (ESI) m/z: 536.15379 $(M+H)^+$.

(Example 177) (9aS)-8-Acetyl-N-[(4-bromo-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-557)

(177a) 1-Bromo-3-methylnaphthalene

Known 4-bromo-2-methyl-1-naphthylamine [Journal of Medicinal Chemistry, 1972, Vol. 15, p.569-570] (2.20 g, 9.32 mmol) was dissolved in tetrahydrofuran (80 mL). 1 N hydrochloric acid (29 mL, 29.0 mmol) and sodium nitrite (1.30 g, 18.8 mmol) were added at 0˚C, and the mixture was directly stirred for one hour. Then, sodium cyanoborohydride (6.00 g, 95.5 mmol) was dissolved in water (10 mL), and the aqueous solution was added to the reaction solution, and the mixture was stirred at room temperature for four hours. Water (50 mL) was added to the reaction solution, followed by extraction with hexane (100 mL). The organic layer was washed with brine and then dried over anhydrous magnesium sulfate. The solvent was evaporated under reduced pressure, and the resulting residue was purified by silica gel column chromatography (elution solvent: hexane) to give the target compound (740 mg, yield: 36%) as a colorless oil.
$^1$H-NMR ($CDCl_3$, 500MHz):δ ppm: 2.44 (3H, s), 7.35-7.43 (3H, m), 7.62 (1H, d, J=7.8Hz), 7.69-7.75 (2H, m).

(177b) 4-Bromo-2-methyl-1-naphthaldehyde

1-Bromo-3-methylnaphthalene produced in Example (177a) (740 mg, 3.35 mmol) was dissolved in methylene chloride (20 mL). Dichloro(methoxy)methane (335 μL, 3.70 mmol) and titanium tetrachloride (410 μL, 3.74 mmol) were added at - 78˚C, and the mixture was stirred with heating to -50˚C for three hours. After completion of the reaction, water (20 mL) was added to the reaction solution, followed by extraction with methylene chloride (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give the target compound (426 mg, yield: 51 %) as a white solid.
$^1$H-NMR ($CDCl_3$, 500MHz):δ ppm: 2.78 (3H, s), 7.57-7.67 (2H, m), 7.71 (1H, s), 8.26 (1H, d, J=8.6Hz), 8.94 (1H, d, J=9.0Hz), 10.90 (1H, s).
MS (EI) m/z: 248 ($M^+$).

(177c) (9aS)-8-Acetyl-N-[(4-bromo-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (205 mg, 0.593 mmol), 4-bromo-2-methyl-1-naphthaldehyde produced in Example (177b) (150 mg, 0.602 mmol), triethylsilane (290 μL, 1.80 mmol), trifluoroacetic acid (140 μL, 1.82 mmol) and acetonitrile (10 mL) to give the target compound (299 mg, yield: 87%) as a yellow solid.
$^1$H-NMR ($CDCl_3$, 400MHz):δ ppm: 1.74 (3H, s), 2.60 (3H, s), 2.64 (3H, s), 3.61 (3H, s), 5.00 (1H, dd, J=4.4, 14.7Hz), 5.08 (1H, dd, J=4.4, 14.7Hz), 6.00 (1H, s), 6.22 (1H, s), 7.06 (1H, m), 7.53-7.61 (2H, m), 7.68 (1H, s), 8.10 (1H, d, J=8.3Hz), 8.25 (1H, brd, J=8.3Hz), 10.64 (1H, s), 18.78 (1H, s).
MS (ESI) m/z: 578.08066 $(M+H)^+$.

(Example 178) (9aS)-8-Acetyl-N-[(4-chloro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-551)

(178a) 4-Chloro-2-methyl-1-naphthaldehyde

Reaction and post-treatment were carried out in accordance with Example (177a) using known 1-chloro-3-methylnaphthalene [Tetrahedron Letters, 1990, Vol. 31, p.6883-6886] (460 mg, 2.60 mmol), dichloro(methoxy)methane (290 μL, 3.21 mmol), titanium tetrachloride (350 μL, 3.19 mmol) and methylene chloride (30 mL) to give the target compound (385 mg, yield: 72%) as a brown solid.
$^1$H-NMR ($CDCl_3$, 500MHz):δ ppm: 2.79 (3H, s), 7.47 (1H, s), 7.59 (1H, m), 7.66 (1H, m), 8.29 (1H, d, J=8.2Hz), 8.97 (1H, d, J=8.6Hz), 10.88 (1H, s).
MS (EI) m/z: 204 ($M^+$).

(178b) (9aS)-8-Acetyl-N-[(4-chloro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (250 mg, 0.724 mmol), 4-chloro-2-methyl-1-naphthaldehyde produced in Example (178a) (150 mg, 0.733 mmol), triethylsilane (350 μL, 2.17 mmol), trifluoroacetic acid (170 μL, 2.21 mmol) and acetonitrile (10 mL) to give the target compound (298 mg, yield: 77%) as a yellow solid.
$^1$H-NMR ($CDCl_3$, 400MHz):δ ppm: 1.74 (3H, s), 2.61 (3H, s), 2.64 (3H, s), 3.62 (3H, s), 5.01 (1H, dd, J=4.2, 14.2Hz), 5.08 (1H, dd, J=4.2, 14.2Hz), 6.00 (1H, s), 6.23 (1H, s), 7.07 (1H, m), 7.48 (1H, brs), 7.55-7.63 (2H, m), 8.13 (1H, d, J=8.3Hz), 8.29 (1H, d, J=8.3Hz), 10.65 (1H, s), 18.82 (1H, s).
MS (ESI) m/z: 534.12937 $(M+H)^+$.

(Example 179) (9aS)-8-Acetyl-N-[(5-chloro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-552)

(179a) 4-(2-Chlorophenyl)butanal

1-Chloro-2-iodobenzene (3.14 mL, 25.7 mmol) was dissolved in N,N-dimethylformamide (30 mL). 3-Buten-1-ol (3.30 mL, 38.3 mmol), tetrabutylammonium chloride (7.15 g, 25.7 mmol), sodium bicarbonate (5.40 g, 64.3 mmol) and palladium acetate (175 mg, 0.779 mmol) were added, and the mixture was stirred at 55˚C for 18 hours. After completion of the reaction, water (50 mL) was added to the reaction solution, followed by extraction with diethyl ether (100 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give the target compound (3.82 g, yield: 81%) as a brown oil.
$^1$H-NMR ($CDCl_3$, 400MHz):δ ppm: 1.99 (2H, m), 2.50 (2H, t, J=7.4Hz), 2.79 (2H, t, J=7.4Hz), 7.13-7.23 (3H, m), 7.36 (1H, d, J=7.4Hz), 9.79 (1H, s).

(179b) 4-(2-Chlorophenyl)butanoic acid

4-(2-Chlorophenyl)butanal produced in Example (179a) was dissolved in tert-butanol (40 mL) and water (10 mL). 2-Methyl-2-butene (10.0 mL, 94.4 mmol), sodium dihydrogenphosphate dihydrate (3.66 g, 23.5 mmol) and sodium chlorite (8.40 g, 74.3 mmol) were added at 0˚C, and the mixture was stirred at room temperature for four hours. 2 N hydrochloric acid (30 mL) was added, followed by extraction with ethyl acetate (100 mL). The organic layer was dried over anhydrous magnesium sulfate. The solvent was evaporated under reduced pressure, and the resulting residue was directly used for the next reaction.

(179c) 5-Chloro-3,4-dihydronaphthalen-1(2H)-one

The crude purified product of 4-(2-chlorophenyl)butanoic acid produced in Example (179b) was dissolved in thionyl chloride (50 mL), and the mixture was stirred with heating to reflux for two hours. After completion of the reaction, the reaction solution was evaporated under reduced pressure and dried. The resulting crude product was dissolved in methylene chloride (100 mL). Aluminum chloride (2.60 g, 19.5 mmol) was added at 0˚C, and the mixture was stirred at room temperature for 19 hours. After completion of the reaction, water (100 mL) was added to the reaction solution, followed by extraction with methylene chloride (100 mL). The organic layer was washed with brine and then dried over anhydrous magnesium sulfate. The solvent was evaporated under reduced pressure, and the resulting residue was purified by silica gel column chromatography (elution solvent: hexane) to give the target compound (2.39 g, yield: 90%, two steps) as a brown solid.

(179d) 1-Bromo-S-chloro-3,4-dihydronaphthalene-2-carbaldehyde

Phosphorus bromide (3.80 mL, 40.0 mmol) was slowly added dropwise to a solution of N,N-dimethylformamide (3.60 mL, 46.3 mmol) in chloroform (120 mL) at 0˚C, and the mixture was stirred in a nitrogen atmosphere at 0˚C for two hours. Thereafter, a solution of 5-chloro-3,4-dihydronaphthalen-1(2H)-one produced in Example (179c) (2.39 g, 13.2 mmol) in chloroform (10 mL) was added at 0˚C. The mixture was heated to room temperature and then stirred with heating to reflux for five hours. After completion of the reaction, the reaction solution was cooled to 0˚C and then a saturated sodium bicarbonate aqueous solution (200 mL) was added dropwise, followed by extraction with methylene chloride (20 mL). The organic layer was washed with brine and then dried over anhydrous magnesium sulfate. The solvent was evaporated under reduced pressure, and the resulting crude purified product as a light yellow oil was directly used for the next reaction.

(179e) 1-Bromo-5-chloro-2-naphthaldehyde

The crude purified product of 1-bromo-5-chloro-3,4-dihydronaphthalene-2-carbaldehyde produced in Example (179d) was dissolved in toluene (250 mL). 2,3-Dichloro-5,6-dicyano-1,4-benzoquinone (7.50 g, 33.0 mmol) was added, and the mixture was stirred with heating to reflux for four days. The reaction solution was evaporated under reduced pressure. The resulting residue was purified by silica gel column chromatography (elution solvent: hexane/toluene = 90/10 to 70/30) to give the target compound (546 mg, yield: 15%, two steps) as a white solid.
$^1$H-NMR ($CDCl_3$, 400MHz):δ ppm: 7.58 (1H, dd, J=7.8, 8.2Hz), 7.76 (1H, d, J=7.8Hz), 8.01 (1H, d, J=8.6Hz), 8.33 (1H, d, J=8.2Hz), 8.46 (1H, d, J=8.6Hz), 10.64 (1H, s).

(179f) 1-Bromo-5-chloro-2-methylnaphthalene

1-Bromo-5-chloro-2-naphthaldehyde produced in Example (179e) (546 mg, 2.01 mmol) was suspended in diethylene glycol (20 mL). Potassium hydroxide (340 mg, 6.06 mmol) and hydrazine monohydrate (280 μL, 5.14 mmol) were added, and the mixture was stirred at 180˚C for 1.5 hours. After completion of the reaction, the reaction solution was cooled to room temperature. Water (20 mL) was added, followed by extraction with diethyl ether (50 mL). The organic layer was dried over anhydrous magnesium sulfate, and then the solvent was evaporated under reduced pressure. The resulting residue was purified by silica gel column chromatography (elution solvent: hexane) to give the target compound (340 mg, yield: 66%) as a white solid.
$^1$H-NMR ($CDCl_3$, 400MHz):δ ppm: 2.63 (3H, s), 7.42-7.46 (2H, m), 7.54 (1H, d, J=7.4Hz), 8.15 (1H, d, J=8.6 Hz), 8.23 (1H, d, J=8.6Hz).

(179g) 5-Chloro-2-methyl-1-naphthaldehyde

Reaction and post-treatment were carried out in accordance with Example (174b) using 1-bromo-5-chloro-2-methylnaphthalene produced in Example (179f) (340 mg, 1.33 mmol), butyllithium (2.71 M solution in hexane, 800 μL, 2.17 mmol), N,N-dimethylformamide (420 μL, 5.40 mmol) and tetrahydrofuran (20 mL) to give the target compound (126 mg, yield: 46%) as a yellow oil.
$^1$H-NMR ($CDCl_3$, 400MHz):δ ppm: 2.83 (3H, s), 7.45 (1H, d, J=8.6Hz), 7.51 (1H, dd, J=7.4, 8.6Hz), 7.58 (1H, d, J=7.4Hz), 8.42 (1H, d, J=8.6Hz), 8.98 (1H, d, J=8.6Hz), 10.92 (1H, s).
MS (EI) m/z: 204 ($M^+$).

(179h) (9aS)-8-Acetyl-N-[(5-chloro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (210 mg, 0.608 mmol), 5-chloro-2-methyl-1-naphthaldehyde produced in Example (179g) (125 mg, 0.610 mmol), triethylsilane (295 μL, 1.83 mmol), trifluoroacetic acid (140 μL, 1.82 mmol) and acetonitrile (10 mL) to give the target compound (259 mg, yield: 80%) as a yellow solid.
$^1$H-NMR ($CDCl_3$, 400MHz):δ ppm: 1.74 (3H, s), 2.64 (3H, s), 2.64 (3H, s), 3.62 (3H, s), 5.04 (1H, dd, J=4.2, 14.2Hz), 5.11 (1H, dd, J=4.2, 14.2Hz), 6.01 (1H, s), 6.23 (1H, s), 7.06 (1H, m), 7.42-7.48 (2H, m), 7.56 (1H, d, J=7.3Hz), 8.06 (1H, d, J=8.8Hz), 8.21 (1H, d, J=8.8Hz), 10.65 (1H, s), 18.82 (1H, s).
MS (ESI) m/z: 534.12980 $(M+H)^+$.

(Example 180) (9aS)-8-Acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-545)

(180a) 4-Fluoro-2-methyl-1-naphthaldehyde

N,N,N'-Trimethylethylenediamine (21 mL, 162 mmol) was dissolved in tetrahydrofuran (800 mL), and butyllithium (2.67 M solution in hexane, 60.0 mL, 160 mmol) was added dropwise at -30˚C. After stirring for 30 minutes, 4-fluor-1-naphthaldehyde (23.5 g, 135 mmol) was added at -30˚C, and the mixture was stirred for 30 minutes. After cooling to -78˚C, butyllithium (2.67 M solution in hexane, 151 mL, 403 mmol) was added dropwise, and the mixture was stirred with heating to 0˚C for six hours. Iodomethane (67.0 mL, 1.08 mmol) was further added at 0˚C, and the mixture was heated to room temperature and stirred for 24 hours. 1 N hydrochloric acid (500 mL) was added to the reaction solution, followed by extraction with diethyl ether (500 mL). The organic layer was washed with brine and then dried over anhydrous magnesium sulfate. The solvent was evaporated under reduced pressure, and the resulting residue was purified by

silica gel column chromatography (elution solvent: hexane/ethyl acetate = 10/1). Then, the resulting crude purified product was recrystallized from hexane to give the target compound (5.70 g, yield: 23%) as a white solid.
$^1$H-NMR ($CDCl_3$, 400MHz):δ ppm: 2.82 (3H, s), 7.03 (1H, d, J=10.6Hz), 7.55 (1H, m), 7.67 (1H, m), 8.10 (1H, d, J=7.4Hz), 9.04 (1H, brd, J=8.6Hz), 10.85 (1H, s). MS (EI) m/z: 188 ($M^+$).

(180b) (9aS)-8-Acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (12.6 g, 36.5 mmol), 4-fluoro-2-methyl-1-naphthaldehyde produced in Example (180a) (5.70 g, 30.3 mmol), triethylsilane (15.0 mL, 92.9 mmol), trifluoroacetic acid (7.0 mL, 90.8 mmol) and acetonitrile (300 mL) to give the target compound (14.4 g, yield: 92%) as a yellow solid.
$^1$H-NMR ($CDCl_3$, 400MHz):δ ppm: 1.74 (3H, s), 2.61 (3H, s), 2.64 (3H, s), 3.63 (3H, s), 5.00 (1H, dd, J=4.8, 14.3Hz), 5.08 (1H, dd, J=4.8, 14.3Hz), 6.01 (1H, s), 6.24 (1H, s), 7.03 (1H, m), 7.05 (1H, d, J=11.1Hz), 7.52 (1H, m), 7.61 (1H, m), 8.08-8.13 (2H, m), 10.66 (1H, s), 18.84 (1H, s).
MS (ESI) m/z: 518.16034 $(M+H)^+$.

(Example 181) (9aS)-8-Acetyl-N-[(7-chloro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-554)

(181a) 4-(4-Chlorophenyl)butanal

Reaction and post-treatment were carried out in accordance with Example (179a) using 1-chloro-4-iodobenzene (6.13 g, 25.7 mmol), 3-buten-1-ol (3.30 mL, 38.3 mmol), tetrabutylammonium chloride (7.15 g, 25.7 mmol), sodium bicarbonate (5.40 g, 64.3 mmol), palladium acetate (175 mg, 0.779 mmol) and N,N-dimethylformamide (30 mL) to give the target compound (3.40 g, yield: 72%) as a brown oil.
$^1$H-NMR ($CDCl_3$, 400MHz):δ ppm: 1.94 (2H, m), 2.46 (2H, t, J=7.4Hz), 2.63 (2H, t, J=7.4Hz), 7.08-7.13 (2H, m), 7.23-7.29 (2H, m), 9.77 (1H, s).

(181b) 4-(4-Chlorophenyl)butanoic acid

Reaction and post-treatment were carried out in accordance with Example (179b) using 4-(4-chlorophenyl) butanal produced in Example (181a), 2-methyl-2-butene (10.0 mL, 94.4 mmol), sodium dihydrogenphosphate dihydrate (3.66 g, 23.5 mmol), sodium chlorite (8.40 g, 74.3 mmol), tert-butanol (40 mL) and water (10 mL) to give a crude purified product of the title compound. The product was directly used for the next reaction.

(181c) 7-Chloro-3,4-dihydronaphthalen-1(2H)-one

Reaction and post-treatment were carried out in accordance with Example (179c) using the crude purified product of 4-(4-chlorophenyl)butanoic acid produced in Example (181b), thionyl chloride (60 mL), aluminum chloride (4.00 g, 30.0 mmol) and methylene chloride (100 mL) to give the target compound (3.21 g, yield: 78%, two steps) as a brown solid.
$^1$H-NMR ($CDCl_3$, 400MHz):δ ppm: 2.13 (2H, m), 2.65 (2H, t, J=6.3Hz), 2.93 (2H, t, J=6.3Hz), 7.19 (1H, d, J=8.2Hz), 7.41 (1H, dd, J=2.4, 8.2Hz), 7.98 (1H, d, J=2.4Hz).

(181d) 1-Bromo-7-chloro-3,4-dihydronaphthalene-2-carbaldehyde

Reaction and post-treatment were carried out in accordance with Example (179d) using 7-chloro-3,4-dihydronaphthalen-1(2H)-one produced in Example (181c) (2.39 g, 13.2 mmol), N,N-dimethylformamide (3.60 mL, 46.3 mmol), phosphorus bromide (3.80 mL, 40.0 mmol) and chloroform (120 mL) to give a crude purified product as a brown solid.

(181e) 1-Bromo-7-chloro-2-naphthaldehyde

Reaction and post-treatment were carried out in accordance with Example (179e) using the crude purified product 1-bromo-7-chloro-3,4-dihydronaphthalene-2-carbaldehyde produced in Example (181d), 2,3-dichloro-5,6-dicyano-1,4-benzoquinone (6.10 g, 26.9 mmol) and 1,2-dichloroethane (250 mL) to give the target compound (1.46 g, yield: 30%, two steps) as a white solid.

$^1$H-NMR ($CDCl_3$, 400MHz):δ ppm: 7.63 (1H, dd, J=2.0, 8.3Hz), 7.83 (1H, d, J=8.8Hz), 7.85 (1H, d, J=8.3Hz), 7.94 (1H, d, J=8.8Hz), 8.51 (1H, d, J=2.0Hz), 10.65 (1H, s).

(181f) 1-Bromo-7-chloro-2-methylnaphthalene

Reaction and post-treatment were carried out in accordance with Example (179f) using 1-bromo-7-chloro-2-naphthaldehyde produced in Example (181e) (1.33 g, 4.90 mmol), potassium hydroxide (830 mg, 14.8 mmol), hydrazine monohydrate (670 μL, 12.3 mmol) and diethylene glycol (60 mL) to give the target compound (950 mg, yield: 76%) as a white solid.

(181g) 7-Chloro-2-methyl-1-naphthaldehyde

Reaction and post-treatment were carried out in accordance with Example (174b) using 1-bromo-7-chloro-2-methylnaphthalene produced in Example (181f) (950 mg, 3.71 mmol), butyllithium (2.71 M solution in hexane, 2.00 mL, 5.42 mmol), N,N-dimethylformamide (1.40 mL, 18.6 mmol) and tetrahydrofuran (50 mL) to give the target compound (330 mg, yield: 43%) as a yellow solid.
$^1$H-NMR ($CDCl_3$, 400MHz):δ ppm: 2.83 (3H, s), 7.34 (1H, d, J=8.6Hz), 7.45 (1H, dd, J=2.0, 8.6Hz), 7.75 (1H, d, J=8.6Hz), 7.91 (1H, d, J=8.6Hz), 9.10 (1H, d, J=2.0Hz), 10.88 (1H, s).
MS (EI) m/z: 204 ($M^+$).

(181h) (9aS)-8-Acetyl-N-[(7-chloro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (450 mg, 1.30 mmol), 7-chloro-2-methyl-1-naphthaldehyde produced in Example (181g) (330 mg, 1.61 mmol), triethylsilane (630 μL, 3.90 mmol), trifluoroacetic acid (300 μL, 3.89 mmol) and acetonitrile (15 mL) to give the target compound (540 mg, yield: 78%) as a yellow solid.
$^1$H-NMR ($CDCl_3$, 400MHz):δ ppm: 1.74 (3H, s), 2.61 (3H, s), 2.64 (3H, s), 3.64 (3H, s), 4.98-5.13 (2H, m), 6.01 (1H, s), 6.23 (1H, s), 7.04 (1H, m), 7.31 (1H, m), 7.37 (1H, d, J=8.3Hz), 7.44 (1H, brd, J=9.2Hz), 7.68 (1H, d, J=8.3Hz), 8.13 (1H, m), 10.65 (1H, s), 18.82 (1H, s).
MS (ESI) m/z: 534.12981 $(M+H)^+$.

(Example 182) (9aS)-8-Acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-548)

(182a) 4-(4-Fluorophenyl)butanal

Reaction and post-treatment were carried out in accordance with Example (179a) using 1-fluoro-4-iodobenzene (2.97 g, 25.7 mmol), 3-buten-1-ol (3.30 mL, 38.3 mmol), tetrabutylammonium chloride (7.15 g, 25.7 mmol), sodium bicarbonate (5.40 g, 64.3 mmol), palladium acetate (175 mg, 0.779 mmol) and N,N-dimethylformamide (30 mL) to give the target compound (3.19 g, yield: 75%) as a brown oil.
$^1$H-NMR ($CDCl_3$, 400MHz):δ ppm: 1.94 (2H, m), 2.45 (2H, t, J=7.4Hz), 2.63 (2H, t, J=7.4Hz), 6.95-7.02 (2H, m), 7.10-7.15 (2H, m), 9.77 (1H, s).

(182b) 4-(4-Fluorophenyl)butanoic acid

Reaction and post-treatment were carried out in accordance with Example (179b) using 4-(4-fluorophenyl) butanal produced in Example (182a), 2-methyl-2-butene (10.0 mL, 94.4 mmol), sodium dihydrogenphosphate dihydrate (3.66 g, 23.5 mmol), sodium chlorite (8.40 g, 74.3 mmol), tert-butanol (40 mL) and water (10 mL) to give a crude purified product of the title compound. The product was directly used for the next reaction.

(182c) 7-Fluoro-3,4-dihydronaphthalen-1(2H)-one

Reaction and post-treatment were carried out in accordance with Example (179c) using the crude purified product of 4-(4-fluorophenyl)butanoic acid produced in Example (182b), thionyl chloride (80 mL), aluminum chloride (5.50 g, 41.2 mmol) and methylene chloride (150 mL) to give the target compound (3.83 g, yield: 74%, two steps) as a brown solid.

(182d) 1-Bromo-7-fluoro-3,4-dihydronaphthalene-2-carbaldehyde

Reaction and post-treatment were carried out in accordance with Example (179d) using 7-fluoro-3,4-dihydronaphthalen-1(2H)-one produced in Example (182c) (3.83 g, 23.3 mmol), N,N-dimethylformamide (5.60 mL, 72.0 mmol), phosphorus bromide (5.60 mL, 59.0 mmol) and chloroform (200 mL) to give a crude purified product as a brown solid.

(182e) 1-Bromo-7-fluoro-2-naphthaldehyde

Reaction and post-treatment were carried out in accordance with Example (179e) using the crude purified product 1-bromo-7-fluoro-3,4-dihydronaphthalene-2-carbaldehyde produced in Example (182d), 2,3-dichloro-5,6-dicyano-1,4-benzoquinone (11.1 g, 48.9 mmol) and 1,2-dichloroethane (250 mL) to give the target compound (1.51 g, yield: 26%, two steps) as a white solid.
$^1$H-NMR ($CDCl_3$, 400MHz):$\delta$ ppm: 7.47 (1H, m), 7.85-7.93 (3H, m), 8.16 (1H, dd, J=2.4, 10.6Hz), 10.66 (1H, s).

(182f) 1-Bromo-7-fluoro-2-methylnaphthalene

1-Bromo-7-fluoro-2-naphthaldehyde produced in Example (182e) (665 mg, 2.63 mmol) was dissolved in methylene chloride (30 mL).
Tris(pentafluorophenyl)borane (1.50 g, 2.93 mmol) and poly(methylhydrosiloxane) (15 mL, 7.90 mmol) were added, and the mixture was stirred at room temperature for 15 hours. The reaction solution was evaporated under reduced pressure, and the resulting residue was purified by silica gel column chromatography (elution solvent: hexane) to give the target compound (205 mg, yield: 33%) as a colorless oil.

(182g) 7-Fluoro-2-methyl-1-naphthaldehyde

Reaction and post-treatment were carried out in accordance with Example (174b) using 1-bromo-7-fluoro-2-methylnaphthalene produced in Example (182f) (316 mg, 1.32 mmol), butyllithium (2.71 M solution in hexane, 700 $\mu$L, 1.90 mmol), N,N-dimethylformamide (410 $\mu$L, 5.29 mmol) and tetrahydrofuran (20 mL) to give the target compound (147 mg, yield: 59%) as a yellow solid.
$^1$H-NMR ($CDCl_3$, 400MHz):$\delta$ ppm: 2.83 (3H, s), 7.26 (1H, m), 7.28 (1H, d, J=8.2Hz), 7.79 (1H, dd, J=5.9, 8.6Hz), 7.91 (1H, d, J=8.2Hz), 8.79 (1H, dd, J=2.4, 12.2Hz), 10.85 (1H, s).
MS (EI) m/z: 188 ($M^+$).

(182h) (9aS)-8-Acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (265 mg, 0.767 mmol), 7-fluoro-2-methyl-1-naphthaldehyde produced in Example (182g) (147 mg, 0.781 mmol), triethylsilane (370 $\mu$L, 2.29 mmol), trifluoroacetic acid (180 $\mu$L, 2.34 mmol) and acetonitrile (10 mL) to give the target compound (242 mg, yield: 61%) as a yellow solid.
$^1$H-NMR ($CDCl_3$, 400MHz):$\delta$ ppm: 1.74 (3H, s), 2.63 (3H, s), 2.63 (3H, s), 3.67 (3H, s), 4.97 (1H, dd, J=4.8, 14.3Hz), 5.03 (1H, dd, J=4.8, 14.3Hz), 6.01 (1H, s), 6.23 (1H, s), 6.97 (1H, m), 7.22 (1H, m), 7.30 (1H, d, J=8.3Hz), 7.68-7.73 (2H, m), 7.81 (1H, dd, J=5.9, 9.1 Hz), 10.62 (1H, s), 18.78 (1H, s).
MS (ESI) m/z: 518.15792 $(M+H)^+$.

(Example 183) (9aS)-8-Acetyl-N-[(5-fluoro-2-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-546)

(183a) 4-(2-Fluorophenyl)butanal

Reaction and post-treatment were carried out in accordance with Example (179a) using 1-fluoro-2-iodobenzene (3.00 g, 25.7 mmol), 3-buten-1-ol (3.30 mL, 38.3 mmol), tetrabutylammonium chloride (7.15 g, 25.7 mmol), sodium bicarbonate (5.40 g, 64.3 mmol), palladium acetate (175 mg, 0.779 mmol) and N,N-dimethylformamide (30 mL) to give the target compound (3.54 g, yield: 83%) as a brown oil.
$^1$H-NMR ($CDCl_3$, 400MHz):$\delta$ ppm: 1.96 (2H, m), 2.48 (2H, t, J=7.4Hz), 2.70 (2H, t, J=7.4Hz), 6.99-7.10 (2H, m), 7.15-7.22 (2H, m), 9.77 (1H, s).

(183b) 4-(2-Fluorophenyl)butanoic acid

Reaction and post-treatment were carried out in accordance with Example (179b) using 4-(2-fluorophenyl) butanal produced in Example (183a), 2-methyl-2-butene (10.0 mL, 94.4 mmol), sodium dihydrogenphosphate dihydrate (3.66 g, 23.5 mmol), sodium chlorite (8.40 g, 74.3 mmol), tert-butanol (40 mL) and water (10 mL) to give a crude purified product of the title compound. The product was directly used for the next reaction.

(183c) 5-Fluoro-3,4-dihydronaphthalen-1(2H)-one

Reaction and post-treatment were carried out in accordance with Example (179c) using the crude purified product of 4-(2-fluorophenyl)butanoic acid produced in Example (183b), thionyl chloride (80 mL), aluminum chloride (4.76 g, 35.7 mmol) and methylene chloride (150 mL) to give the target compound (3.12 g, yield: 69%, two steps) as a light yellow solid.
$^1$H-NMR ($CDCl_3$, 400MHz):δ ppm: 2.16 (2H, m), 2.67 (2H, t, J=6.3Hz), 2.96 (2H, t, J=6.3Hz), 7.20-7.32 (2H, m), 7.84 (1H, d, J=7.4Hz).

(183d) 1-Bromo-5-fluoro-3,4-dihydronaphthalene-2-carbaldehyde

Reaction and post-treatment were carried out in accordance with Example (179d) using 5-fluoro-3,4-dihydronaphthalen-1(2H)-one produced in Example (183c) (3.12 g, 19.0 mmol), N,N-dimethylformamide (4.50 mL, 57.9 mmol), phosphorus bromide (4.50 mL, 47.4 mmol) and chloroform (200 mL) to give a crude purified product as a brown solid.

(183e) 1-Bromo-5-fluoro-2-naphthaldehyde

Reaction and post-treatment were carried out in accordance with Example (179e) using the crude purified product 1-bromo-5-fluoro-3,4-dihydronaphthalene-2-carbaldehyde produced in Example (183d), 2,3-dichloro-5,6-dicyano-1,4-benzoquinone (9.06 g, 39.9 mmol) and 1,2-dichloroethane (250 mL) to give the target compound (925 mg, yield: 19%, two steps) as a white solid.
$^1$H-NMR ($CDCl_3$, 400MHz):δ ppm: 7.36 (1H, dd, J=8.6, 10.6Hz), 7.61 (1H, m), 7.96 (1H, d, J=8.6Hz), 8.14 (1H, d, J=8.6Hz), 8.29 (1H, d, J=8.6Hz), 10.64 (1H, s).

(183f) 1-Bromo-5-fluoro-2-methylnaphthalene

1-Bromo-5-fluoro-2-naphthaldehyde produced in Example (183e) (925 mg, 3.66 mmol) was dissolved in methylene chloride (40 mL).
Tris(pentafluorophenyl)borane (2.06 g, 4.02 mmol) and triethylsilane (5.90 mL, 36.5 mmol) were added, and the mixture was stirred at room temperature for 17 hours. The reaction solution was evaporated under reduced pressure, and the resulting residue was purified by silica gel column chromatography (elution solvent: hexane) to give the target compound (428 mg, yield: 49%) as a colorless oil.
$^1$H-NMR ($CDCl_3$, 400MHz):δ ppm: 2.63 (3H, s), 7.13 (1H, dd, J=8.6, 10.6Hz), 7.38 (1H, d, J=8.6Hz), 7.42-7.49 (1H, m), 7.96 (1H, d, J=8.2Hz), 8.05 (1H, d, J=8.6Hz).

(183g) 5-Fluoro-2-methyl-1-naphthaldehyde

Reaction and post-treatment were carried out in accordance with Example (174b) using 1-bromo-5-fluoro-2-methylnaphthalene produced in Example (183f) (428 mg, 1.79 mmol), butyllithium (2.71 M solution in hexane, 1.00 mL, 2.71 mmol), N,N-dimethylformamide (700 μL, 9.04 mmol) and tetrahydrofuran (20 mL) to give the target compound (181 mg, yield: 54%) as a white solid.
$^1$H-NMR ($CDCl_3$, 400MHz):δ ppm: 2.79 (3H, s), 7.15 (1H, dd, J=8.8, 10.3Hz), 7.36 (1H, d, J=8.6Hz), 7.51 (1H, m), 8.19 (1H, d, J=8.8Hz), 8.70 (1H, d, J=8.8Hz), 10.90 (1H, s).
MS (EI) m/z: 188 ($M^+$).

(183h) (9aS)-8-Acetyl-N-[(5-fluoro-2-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (330 mg, 0.956 mmol), 5-fluoro-2-methyl-1-naphthaldehyde produced in Example (183g) (181 mg, 0.962 mmol), triethylsilane

(465 μL, 2.88 mmol), trifluoroacetic acid (220 μL, 2.86 mmol) and acetonitrile (15 mL) to give the target compound (400 mg, yield: 81%) as a yellow solid.
$^1$H-NMR ($CDCl_3$, 400MHz):δ ppm: 1.74 (3H, s), 2.64 (3H, s), 2.64 (3H, s), 3.63 (3H, s), 5.03 (1H, dd, J=4.8, 14.3Hz), 5.11 (1H, dd, J=4.8, 14.3Hz), 6.02 (1H, s), 6.24 (1H, s), 7.06-7.15 (2H, m), 7.39-7.50 (2H, m), 7.90 (1H, d, J=8.7Hz), 8.03 (1H, d, J=8.7Hz), 10.67 (1H, s), 18.84 (1H, s).
MS (ESI) m/z: 518.15972 $(M+H)^+$.

(Example 184) (9aS)-8-Acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide (exemplary compound No. 1-566)

(184a) 7-Fluoro-3,4-dihydronaphthalen-2(1H)-one

(3-Fluorophenyl)acetic acid (15.0 g, 97.3 mmol) was dissolved in methylene chloride (200 mL). N,N-Dimethylformamide (200 μL) and oxalyl chloride (9.34 mL, 107 mmol) were slowly added dropwise at room temperature, and the mixture was stirred for two hours. After completion of the reaction, the reaction solution was evaporated under reduced pressure and dried. The resulting crude product was dissolved in methylene chloride (200 mL) again, and aluminum chloride (19.5 g, 146 mmol) was added at 0˚C. After stirring at room temperature for 15 minutes, the mixture was slowly bubbled with methylene gas and directly stirred at room temperature for 1.5 hours. After completion of the reaction, a dilute hydrochloric acid solution (150 mL) was added to the reaction solution, followed by extraction with methylene chloride (200 mL). The organic layer was washed with brine and then dried over anhydrous sodium sulfate. The solvent was evaporated under reduced pressure, and the resulting residue was purified by silica gel column chromatography (elution solvent: hexane = 70/30) to give the target compound (16.0 g, yield: 100%, two steps) as yellow crystals.
$^1$H-NMR ($CDCl_3$, 400MHz):δ ppm: 2.54 (2H, t, J=6.7Hz), 3.05 (2H, t, J=6.7Hz), 3.55 (2H, s), 6.89-6.97 (2H, m), 7.08 (1H, dd, J=5.5, 8.2Hz).

(184b) 2-Bromo-7-fluoro-3,4-dihydronaphthalene-1-carbaldehyde

Phosphorus bromide (23.1 mL, 243 mmol) was slowly added dropwise to a solution of N,N-dimethylformamide (22.7 mL, 292 mmol) in chloroform (200 mL) at 0˚C, and the mixture was stirred in a nitrogen atmosphere at 0˚C for two hours. Thereafter, a solution of 7-fluoro-3,4-dihydronaphthalen-2(1H)-one produced in Example (184a) (16.0 g, 97.3 mmol) in chloroform (100 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (300 mL) was slowly added, followed by extraction with methylene chloride (150 mL). The organic layer was washed with brine and then dried over anhydrous sodium sulfate. The solvent was evaporated under reduced pressure, and the resulting residue was purified by silica gel column chromatography (elution solvent: hexane = 90/10) to give the target compound (19.5 g, yield: 78%) as yellow crystals.
$^1$H-NMR ($CDCl_3$, 400MHz):δ ppm: 2.86 (2H, t, J=7.6Hz), 3.03 (2H, t, J=7.6Hz), 6.93 (1H, ddd, J=2.4, 8.2, 10.6Hz), 7.10 (1H, dd, J=5.9, 8.2Hz), 7.81 (1H, dd, J=2.4, 10.6Hz), 10.29 (1H, s).
MS (EI) m/z: 254 $(M^+)$.

(184c) 2-Bromo-7-fluoro-1-naphthaldehyde

2-Bromo-7-fluoro-3,4-dihydronaphthalene-1-carbaldehyde produced in Example (184b) (19.5 g, 76.4 mmol) was dissolved in toluene (300 mL). 2,3-Dichloro-5,6-dicyano-1,4-benzoquinone (26.0 g, 115 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 70/30) to give the target compound (12.0 g, yield: 62%) as yellow crystals.
$^1$H-NMR ($CDCl_3$, 500MHz):δ ppm: 7.37 (1H, ddd, J=2.4, 8.8, 10.3Hz), 7.68 (1H, d, J=8.3Hz), 7.85 (1H, dd, J=5.9, 8.8Hz), 7.87 (1H, d, J=8.3Hz), 8.91 (1H, dd, J=2.4, 11.7Hz), 10.75 (1H, s).
MS (EI) m/z: 252 $(M^+)$.

(184d) 2-Ethyl-7-fluoro-1-naphthaldehyde

2-Bromo-7-fluoro-1-naphthaldehyde produced in Example (184c) (12.0 g, 76.4 mmol) was dissolved in N,N-dimethylformamide (150 mL). Tetrakis(triphenylphosphine)palladium (2.74 g, 2.37 mmol) and tetraethyltin (11.3 mL, 56.9 mmol) were added, and the mixture was stirred at 120˚C for two hours. After completion of the reaction, the reaction solution was cooled to 0˚C and then water (100 mL) was slowly added, followed by extraction with ethyl acetate (120

mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 50/1) to give the target compound (6.0 g, yield: 63%) as a yellow syrup.
$^1$H-NMR ($CDCl_3$, 500MHz):δ ppm: 1.38 (3H, t, J=7.8Hz), 3.18 (2H, q, J=7.8Hz), 7.29 (1H, ddd, J=2.5, 8.8, 10.8Hz), 7.34 (1H, d, J=8.8Hz), 7.82 (1H, dd, J=5.9, 8.8Hz), 7.98 (1H, d, J=8.8Hz), 8.84 (1H, dd, J=2.5, 11.7Hz), 10.85 (1H, s).
MS (EI) m/z: 202 ($M^+$).

(184e) (9aS)-8-Acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (10.0 g, 29.0 mmol), 2-ethyl-7-fluoro-1-naphthaldehyde produced in Example (184d) (6.0 g, 29.7 mmol), triethylsilane (13.8 mL, 86.9 mmol), trifluoroacetic acid (6.69 mL, 86.9 mmol) and acetonitrile (200 mL) to give the target compound (11.2 g, yield: 73%) as a yellow solid.
$^1$H-NMR ($CDCl_3$, 400MHz):δ ppm: 1.30 (3H, t, J=7.5Hz), 1.74 (3H, s), 2.64 (3H, s), 2.95 (2H, q, J=7.5Hz), 3.66 (3H, s), 4.96-5.05 (2H, m), 6.01 (1H, s), 6.23 (1H, s), 6.95 (1H, brs), 7.21-7.26 (1H, m), 7.32 (1H, d, J=8.3Hz), 7.71 (1H, dd, J=2.4, 11.5Hz), 7.75 (1H, d, J=8.3Hz), 7.82 (1H, dd, J=5.9, 9.1Hz), 10.60 (1H, s), 18.78 (1H,s).
MS (ESI) m/z: 532.1783 $(M+H)^+$.

(Example 185) (9aS)-8-Acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide (exemplary compound No. 1-628)

(185a) 5,7-Difluoro-3,4-dihydronaphthalen-2(1H)-one

(3,5-Difluorophenyl)acetic acid (11.8 g, 68.6 mmol) was dissolved in methylene chloride (150 mL). N,N-Dimethylformamide (150 μL) and oxalyl chloride (6.58 mL, 75.4 mmol) were slowly added dropwise at room temperature, and the mixture was stirred for two hours. After completion of the reaction, the reaction solution was evaporated under reduced pressure and dried. The resulting crude product was dissolved in methylene chloride (150 mL) again, and aluminum chloride (13.7 g, 103 mmol) was added at 0˚C. After stirring at room temperature for 15 minutes, the mixture was slowly bubbled with methylene gas and directly stirred at room temperature for 1.5 hours. After completion of the reaction, a dilute hydrochloric acid solution (100 mL) was added to the reaction solution, followed by extraction with methylene chloride (150 mL). The organic layer was washed with brine and then dried over anhydrous sodium sulfate. The solvent was evaporated under reduced pressure, and the resulting residue was purified by silica gel column chromatography (elution solvent: hexane = 70/30) to give the target compound (11.9 g, yield: 95%, two steps) as yellow crystals.
$^1$H-NMR ($CDCl_3$, 500MHz):δ ppm: 2.55 (2H, t, J=6.8Hz), 3.06 (2H, t, J=6.8Hz), 3.58 (2H, s), 6.69 (1H, dd, J=2.4, 8.8Hz), 6.73 (1H, ddd, J=2.4, 9.3, 9.3Hz).
MS (EI) m/z: 182 ($M^+$).

(185b) 2-Bromo-5,7-difluoro-3,4-dihydronaphthalene-1-carbaldehyde

Phosphorus bromide (2.45 mL, 25.8 mmol) was slowly added dropwise to a solution of N,N-dimethylformamide (2.41 mL, 31.0 mmol) in chloroform (40 mL) at 0˚C, and the mixture was stirred in a nitrogen atmosphere at 0˚C for two hours. Thereafter, a solution of 5,7-difluoro-3,4-dihydronaphthalen-2(1H)-one produced in Example (185a) (1.88 g, 10.3 mmol) in chloroform (15 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (60 mL) was slowly added, followed by extraction with methylene chloride (60 mL). The organic layer was washed with brine and then dried over anhydrous sodium sulfate. The solvent was evaporated under reduced pressure, and the resulting residue was purified by silica gel column chromatography (elution solvent: hexane = 90/10) to give the target compound (1.79 g, yield: 63%) as yellow crystals.
$^1$H-NMR ($CDCl_3$, 400MHz):δ ppm: 2.89 (2H, t, J=7.8Hz), 3.04 (2H, t, J=7.8Hz), 6.77 (1H, ddd, J=2.4, 8.8, 9.3 Hz), 7.68 (1H, dd, J=2.4, 10.3Hz), 10.29 (1H, s).
MS (EI) m/z: 272 ($M^+$).

(185c) 2-Bromo-5,7-difluoro-1-naphthaldehyde

2-Bromo-5,7-difluoro-3,4-dihydronaphthalene-1-carbaldehyde produced in Example (185b) (6.0 g, 22.0 mmol)

was dissolved in toluene (120 mL). 2,3-Dichloro-5,6-dicyano-1,4-benzoquinone (9.98 g, 43.9 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 70/30) to give the target compound (2.77 g, yield: 47%) as white crystals.
$^1$H-NMR ($CDCl_3$, 400MHz):δ ppm: 7.08 (1H, ddd, J=2.4, 8.2, 9.8Hz), 7.71 (1H, d, J=8.6Hz), 8.12 (1H, d, 8.6Hz), 7.81 (1H, dd, J=2.4, 11.7Hz), 10.71 (1H, s).
MS (EI) m/z: 270 ($M^+$).

(185d) 2-Ethyl-5,7-difluoro-1-naphthaldehyde

2-Bromo-5,7-difluoro-1-naphthaldehyde produced in Example (185c) (2.10 g, 7.75 mmol) was dissolved in toluene (30 mL).
Tetrakis(triphenylphosphine)palladium (0.895 g, 0.775 mmol) and tetraethyltin (3.83 mL, 19.4 mmol) were added, and the mixture was stirred at 150˚C for six hours. After completion of the reaction, the reaction solution was cooled to 0˚C and then water (30 mL) was added, followed by extraction with ethyl acetate (30 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 50/1) to give the target compound (1.33 g, yield: 78%) as yellow crystals.
$^1$H-NMR ($CDCl_3$, 400MHz):δ ppm: 1.38 (3H, t, J=7.4Hz), 3.17(2H, q, J=7.4Hz), 6.99 (1H, ddd, J=2.0, 10.2, 10.2Hz), 7.37 (1H, d, J=8.6Hz), 8.11 (1H, d, J=8.6Hz), 8.62 (1H, dd, J=2.0, 12.1Hz), 10.79 (1H, s).
MS (FAB) m/z: 221 $(M+H)^+$.

(185e) (9aS)-8-Acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (2.10 g, 6.08 mmol), 2-ethyl-5,7-difluoro-1-naphthaldehyde produced in Example (185d) (1.33 g, 6.04 mmol), triethylsilane (3.87 mL, 24.3 mmol), trifluoroacetic acid (1.87 mL, 24.3 mmol) and acetonitrile (100 mL) to give the target compound (2.00 g, yield: 60%) as a yellow solid.
$^1$H-NMR ($CDCl_3$, 400MHz):δ ppm: 1.31 (3H, t, J=7.5Hz), 1.74 (3H, s), 2.64 (3H, s), 2.97 (2H, q, J=7.5Hz), 3.68 (3H, s), 4.95-5.05 (2H, m), 6.03 (1H, s), 6.25 (1H, s), 6.96-7.01 (1H, m), 7.39 (1H, d, J=8.7Hz), 7.56 (1H, dd, J=2.4, 10.3Hz), 8.02 (1H, d, J=8.7Hz), 10.66 (1H, s), 18.84 (1H, s).
MS (ESI) m/z: 550.17158 $(M+H)^+$.

(Example 186) (9aS)-8-Acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide (exemplary compound No. 1-572)

(186a) 2-Bromo-7-chloro-3,4-dihydronaphthalene-1-carbaldehyde

Phosphorus bromide (15.2 mL, 161 mmol) was slowly added dropwise to a solution of N,N-dimethylformamide (15.0 mL, 193 mmol) in chloroform (150 mL) at 0˚C, and the mixture was stirred in a nitrogen atmosphere at 0˚C for two hours. Thereafter, a solution of known 7-chloro-3,4-dihydronaphthalen-2(1H)-one [Journal of Organic Chemistry, 1968, Vol. 33, p.4288-4290] (11.6 g, 64.2 mmol) in chloroform (60 mL) was added at 0˚C. The mixture was heated to room temperature and then stirred with heating to reflux for two hours. After completion of the reaction, the reaction solution was cooled to 0˚C and then a saturated sodium bicarbonate aqueous solution (200 mL) was slowly added, followed by extraction with methylene chloride (150 mL). The organic layer was washed with brine and then dried over anhydrous sodium sulfate. The solvent was evaporated under reduced pressure, and the resulting residue was purified by silica gel column chromatography (elution solvent: hexane = 90/10) to give the target compound (13.0 g, yield: 75%) as yellow crystals.
$^1$H-NMR ($CDCl_3$, 500MHz):δ ppm: 2.87 (2H, t, J=7.3Hz), 3.03 (2H, t, J=7.3Hz), 7.08 (1H, d, J=7.8Hz), 7.21 (1H, dd, J=2.0, 7.8Hz), 8.05 (1H, d, J=2.0Hz), 10.30 (1H, s).
MS (EI) m/z: 270 ($M^+$).

(186b) 2-Bromo-7-chloro-1-naphthaldehyde

2-Bromo-7-chloro-3,4-dihydronaphthalene-1-carbaldehyde produced in Example (186a) (13.0 g, 47.9 mmol) was dissolved in toluene (250 mL). 2,3-Dichloro-5,6-dicyano-1,4-benzoquinone (16.3 g, 71.8 mmol) was added, and

the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 70/30) to give the target compound (9.0 g, yield: 70%) as yellow crystals.
$^1$H-NMR ($CDCl_3$, 400MHz):δ ppm: 7.53 (1H, dd, J=2.0, 8.6Hz), 7.71 (1H, d, J=8.6Hz), 7.79 (1H, d, 8.6Hz), 7.86 (1H, d, J=8.6Hz), 9.21 (1H, d, J=2.0Hz), 10.73 (1H, s).
MS (EI) m/z: 268 ($M^+$).

(186c) 7-Chloro-2-ethyl-1-naphthaldehyde

2-Bromo-7-chloro-1-naphthaldehyde produced in Example (186b) (9.0 g, 33.4 mmol) was dissolved in N,N-dimethylformamide (100 mL). Tetrakis(triphenylphosphine)palladium (3.86 g, 3.34 mmol) and tetraethyltin (6.61 mL, 33.4 mmol) were added, and the mixture was stirred at 120˚C for three hours. After completion of the reaction, the reaction solution was cooled to 0˚C and then water (120 mL) was slowly added, followed by extraction with ethyl acetate (120 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 30/1) to give the target compound (3.83 g, yield: 52%) as a yellow syrup.
$^1$H-NMR ($CDCl_3$, 400MHz):δ ppm: 1.37 (3H, t, J=7.4Hz), 3.16 (2H, q, J=7.4Hz), 7.37 (1H, d, J=8.6Hz), 7.46 (1H, dd, J=2.0, 8.6Hz), 7.75 (1H, d, J=8.6Hz), 7.95 (1H, d, J=8.6Hz), 9.13 (1H, d, J=2.0Hz), 10.84 (1H, s).
MS (EI) m/z: 218 ($M^+$).

(186d) (9aS)-8-Acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (5.84 g, 16.9 mmol), 7-chloro-2-ethyl-1-naphthaldehyde produced in Example (186c) (3.70 g, 16.9 mmol), triethylsilane (10.8 mL, 67.7 mmol), trifluoroacetic acid (5.22 mL, 67.7 mmol) and acetonitrile (100 mL) to give the target compound (6.0 g, yield: 65%) as a yellow solid.
$^1$H-NMR ($CDCl_3$, 400MHz):δ ppm: 1.29 (3H, t, J=7.5Hz), 1.74 (3H, s), 2.64 (3H, s), 2.93-2.99 (2H, m), 3.67 (3H, s), 4.97-5.06 (2H, m), 6.01 (1H, s), 6.23 (1H, s), 6.99 (1H, brs), 7.36 (1H, d, J=8.3Hz), 7.40 (1H, dd, J=1.6, 8.3Hz), 7.74 (1H, d, J=8.3Hz), 7.77 (1H, d, J=8.3Hz), 10.62 (1H, s), 18.78 (1H, s).
MS (ESI) m/z: 548.14637 $(M+H)^+$.

(Example 187) (9aS)-8-Acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide (exemplary compound No. 1-563)

(187a) 2-Bromo-4-fluoro-1-naphthaldehyde

N,N,N'-Trimethylethylenediamine (2.9 mL, 22.3 mmol) was dissolved in tetrahydrofuran (150 mL), and butyllithium (2.67 M solution in hexane, 8.40 mL, 22.4 mmol) was added dropwise at -30˚C. After stirring for 30 minutes, 4-fluor-1-naphthaldehyde (3.00 g, 17.2 mmol) was added at -30˚C, and the mixture was stirred for 30 minutes. After cooling to -78˚C, butyllithium (2.67 M solution in hexane, 16.0 mL, 42.7 mmol) was added dropwise, and the mixture was stirred with heating to 0˚C for three hours. 1,2-Dibromo-1,1,2,2-tetrafluoroethane (8.30 mL, 69.0 mol) was further added at 0˚C, and the mixture was heated to room temperature and stirred for 18 hours. 1 N hydrochloric acid (200 mL) was added to the reaction solution, followed by extraction with diethyl ether (300 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1). Then, the resulting crude purified product was recrystallized from hexane to give the target compound (2.35 g, yield: 57%) as a white solid.
$^1$H-NMR ($CDCl_3$, 400MHz):δ ppm: 7.44 (1H, d, J=9.3Hz), 7.65 (1H, m), 7.75 (1H, m), 8.13 (1H, d, J=8.8Hz), 9.23 (1H, d, J=8.8Hz), 10.71 (1H, s).
MS (EI) m/z: 198 ($M^+$).

(187b) 2-Ethyl-4-fluoro-1-naphthaldehyde

2-Bromo-4-fluoro-1-naphthaldehyde produced in Example (187a) (38.0 g, 150 mmol) was dissolved in N,N-dimethylformamide (500 mL). A (bis(diphenylphosphino)ferrocene)palladium dichloride-methylene chloride complex (3.70 g, 4.53 mmol), diethylmethoxyborane (30.0 mL, 228 mmol) and potassium carbonate (52.0 g, 376 mmol) were

added, and the mixture was stirred at 70˚C for 2.5 hours. After completion of the reaction, 2 N hydrochloric acid (300 mL) was added, followed by extraction with diethyl ether (500 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give the target compound (27.4 g, yield: 90%) as a light yellow solid.
$^1$H-NNM ($CDCl_3$, 400MHz):δ ppm: 1.38 (3H, t, J=7.8Hz), 3.16 (2H, q, J=7.8Hz), 7.07 (1H, d, J=11.0Hz), 7.57 (1H, m), 7.68 (1H, m), 8.12 (1H, d, J=8.2Hz), 9.09 (1H, d, J=8.6Hz), 10.84 (1H, s).
MS (EI) m/z: 202 ($M^+$).

(187c) (9aS)-8-Acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (7.00 g, 20.3 mmol), 2-ethyl-4-fluoro-1-naphthaldehyde produced in Example (187b) (3.74 g, 18.5 mmol), triethylsilane (9.00 mL, 55.7 mmol), trifluoroacetic acid (4.30 mL, 55.8 mmol) and acetonitrile (200 mL) to give the target compound (7.72 g, yield: 79%) as a yellow solid.
$^1$H-NMR ($CDCl_3$, 400MHz):δ ppm: 1.31 (3H, t, J=7.5Hz), 1.74 (3H, s), 2.64 (3H, s), 2.93 (2H, q, J=7.5Hz), 3.61 (3H, s), 5.00 (1H, dd, J=4.8, 14.7Hz), 5.06 (1H, dd, J=4.8, 14.7Hz), 6.00 (1H, s), 6.22 (1H, s), 7.00 (1H, m), 7.05 (1H, d, J=11.5Hz), 7.53 (1H, m), 8.59 (1H, m), 8.07-8.12 (2H, m), 10.63 (1H, s), 18.78 (1H, s).
MS (ESI) m/z: 532.17768 $(M+H)^+$.

(Example 188) (9aS)-8-Acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide (exemplary compound No. 1-630)

(188a) 6,7-Difluoro-3,4-dihydronaphthalen-2(1H)-one

(3,4-Difluorophenyl)acetic acid (15.5 g, 90.0 mmol) was dissolved in methylene chloride (200 mL). N,N-Dimethylformamide (200 μL) and oxalyl chloride (8.64 mL, 99.1 mmol) were slowly added dropwise at room temperature, and the mixture was stirred for two hours. After completion of the reaction, the reaction solution was evaporated under reduced pressure and dried. The resulting crude product was dissolved in methylene chloride (200 mL) again, and aluminum chloride (18.0 g, 135 mmol) was added at 0˚C. After stirring at room temperature for 15 minutes, the mixture was slowly bubbled with methylene gas and directly stirred at room temperature for 2.0 hours. After completion of the reaction, a dilute hydrochloric acid solution (150 mL) was added to the reaction solution, followed by extraction with methylene chloride (200 mL). The organic layer was washed with brine and then dried over anhydrous sodium sulfate. The solvent was evaporated under reduced pressure, and the resulting residue was purified by silica gel column chromatography (elution solvent: hexane = 70/30) to give the target compound (16.1 g, yield: 97%, two steps) as yellow crystals.
$^1$H-NMR ($CDCl_3$, 400MHz):δ ppm: 2.55 (2H, t, J=6.8Hz), 3.02 (2H, t, J=6.8Hz), 3.52 (2H, s), 6.94 (1H, dd, J=8.3, 9.8Hz), 7.05 (1H, dd, J=8.3, 9.8Hz).
MS (EI) m/z: 182 ($M^+$).

(188b) 2-Bromo-6,7-difluoro-3,4-dihydronaphthalene-1-carbaldehyde

Phosphorus bromide (20.8 mL, 219 mmol) was slowly added dropwise to a solution of N,N-dimethylformamide (20.4 mL, 262 mmol) in chloroform (200 mL) at 0˚C, and the mixture was stirred in a nitrogen atmosphere at 0˚C for two hours. Thereafter, a solution of 6,7-difluoro-3,4-dihydronaphthalen-2(1H)-one produced in Example (188a) (16.1 g, 87.4 mmol) in chloroform (100 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (300 mL) was slowly added, followed by extraction with methylene chloride (150 mL). The organic layer was washed with brine and then dried over anhydrous sodium sulfate. The solvent was evaporated under reduced pressure, and the resulting residue was purified by silica gel column chromatography (elution solvent: hexane = 90/10) to give the target compound (18.0 g, yield: 75%) as yellow crystals.
$^1$H-NMR ($CDCl_3$, 400MHz):δ ppm: 2.85 (2H, t, J=7.4Hz), 3.03 (2H, t, J=7.4Hz), 6.95 (1H, dd, J=8.0, 10.4Hz), 7.99 (1H, dd, J=8.6, 12.5Hz), 10.27 (1H, s).
MS (EI) m/z: 272 ($M^+$).

(188c) 2-Bromo-6,7-difluoro-1-naphthaldehyde

2-Bromo-6,7-difluoro-3,4-dihydronaphthalene-1-carbaldehyde produced in Example (188b) (18.0 g, 65.9 mmol) was dissolved in toluene (300 mL). 2,3-Dichloro-5,6-dicyano-1,4-benzoquinone (22.4 g, 98.9 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 70/30) to give the target compound (7.88 g, yield: 44%) as yellow crystals.
$^1$H-NMR ($CDCl_3$, 400MHz):δ ppm: 7.58 (1H, dd, J=8.2, 10.2Hz), 7.72 (1H, d, J=8.6Hz), 7.81 (1H, d, 8.6Hz), 7.58 (1H, dd, J=8.2, 10.2Hz), 10.72 (1H, s).
MS (EI) m/z: 270 ($M^+$).

(188d) 2-Ethyl-6,7-difluoro-1-naphthaldehyde

2-Bromo-6,7-difluoro-1-naphthaldehyde produced in Example (188c) (7.88 g, 29.1 mmol) was dissolved in toluene (150 mL).
Tetrakis(triphenylphosphine)palladium (3.36 g, 2.91 mmol) and tetraethyltin (11.5 mL, 58.1 mmol) were added, and the mixture was stirred at 135˚C for 12 hours. After completion of the reaction, the reaction solution was cooled to 0˚C and then water (150 mL) was slowly added, followed by extraction with ethyl acetate (120 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 30/1) to give the target compound (3.53 g, yield: 55%) as a yellowish white solid.
$^1$H-NMR ($CDCl_3$, 400MHz):δ ppm: 1.31 (3H, t, J=7.4Hz), 3.11 (2H, q, J=7.4Hz), 7.33 (1H, d, J=8.6Hz), 7.49 (1H, dd, J=8.2, 10.6Hz), 7.85 (1H, d, J=8.6Hz), 8.97 (1H, dd, J=8.2, 13.7Hz), 10.75 (1H, s).
MS (EI) m/z: 220 ($M^+$).

(188e) (9aS)-8-Acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (4.00 g, 11.6 mmol), 2-ethyl-6,7-difluoro-1-naphthaldehyde produced in Example (188d) (2.53 g, 11.5 mmol), triethylsilane (5.54 mL, 34.8 mmol), trifluoroacetic acid (2.68 mL, 34.8 mmol) and acetonitrile (80 mL) to give the target compound (4.89 g, yield: 77%) as a yellow solid.
$^1$H-NMR ($CDCl_3$, 400MHz):δ ppm: 1.29 (3H, t, J=7.5Hz), 1.75 (3H, s), 2.64 (3H, s), 2.93 (2H, q, J=7.5Hz), 3.67 (3H, s), 4.94-5.04 (2H, m), 6.02 (1H, s), 6.24 (1H, s), 6.93 (1H, brs), 7.35 (1H, d, J=8.7Hz), 7.52 (1H, dd, J=7.9, 10.7Hz), 7.68 (1H, d, J=8.7Hz), 7.86 (1H, dd, J=8.1, 12.5Hz), 10.64 (1H, s), 18.79 (1H, s).
MS (ESI) m/z: 550.16675 $(M+H)^+$.

(Example 189) (9aS)-8-Acetyl-N-[(2-ethyl-4-fluoro-7-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-637)

(189a) 2-(3-(4-Bromo-2-fluorophenyl)2-propenyl)-1,3-dioxolane

(2-(1,3-Dioxolan-2-yl)ethyl)triphenylphosphonium bromide (19.0 g, 42.9 mmol) was suspended in tetrahydrofuran (200 mL). tert-Butoxypotassium (4.80 g, 42.8 mmol) was added at 0˚C, and the mixture was stirred for 20 minutes. 4-Bromo-2-fluorobenzaldehyde (5.23 g, 33.0 mmol) was added, and then the mixture was heated to room temperature and stirred for one hour. After completion of the reaction, water (200 mL) was added to the reaction solution, followed by extraction with diethyl ether (300 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give an E/Z mixture of the target compound (7.70 g, yield: 85%).

(189b) 2-(3-(4-Bromo-2-fluorophenyl)propyl)-1,3-dioxolane

The E/Z mixture of 2-(3-(4-bromo-2-fluorophenyl)2-propenyl)-1,3-dioxolane produced in Example (189a) (7.70 g, 28.2 mmol) was dissolved in ethyl acetate (150 mL). A 10% palladium-carbon catalyst (800 mg) was added, and the mixture was stirred in a hydrogen atmosphere at room temperature for five hours. After completion of the reaction, the reaction solution was filtered through Celite and the solvent was evaporated under reduced pressure to give the target

compound (7.76 g, yield: 100%) as a yellow oil.
$^1$H-NMR ($CDCl_3$, 400MHz):δ ppm: 1.67-1.76 (4H, m), 2.64 (2H, m), 3.83 (2H, m), 3.95 (2H, m), 4.87 (1H, t, J=4.3Hz), 7.04 (1H, dd, J=7.8, 8.2Hz), 7.14-7.19 (2H, m).

(189c) 4-(4-Bromo-2-fluorophenyl)butanoic acid

2-(3-(4-Bromo-2-fluorophenyl)propyl)-1,3-dioxolane produced in Example (189b) (7.76 g, 28.2 mmol) was suspended in tetrahydrofuran (80 mL) and water (80 mL). Oxone$^{(R)}$ (52.0 g, 84.6 mmol) was added, and the mixture was stirred at room temperature for 23 hours. After completion of the reaction, the reaction solution was filtered, followed by extraction with diethyl ether (300 mL). The organic layer was washed with brine and then dried over anhydrous magnesium sulfate. The solvent was evaporated under reduced pressure, and the resulting residue was directly used for the next reaction.

(189d) 7-Bromo-5-fluoro-3,4-dihydronaphthalen-1(2H)-one

Reaction and post-treatment were carried out in accordance with Example (179c) using the crude purified product of 4-(4-bromo-2-fluorophenyl)butanoic acid produced in Example (189c), thionyl chloride (150 mL), aluminum chloride (4.90 g, 36.7 mmol) and methylene chloride (150 mL) to give the target compound (3.82 g, yield: 56%, three steps) as a light yellow solid.
$^1$H-NMR ($CDCl_3$, 400MHz):δ ppm: 2.15 (2H, m), 2.67 (2H, t, J=6.3Hz), 2.89 (2H, t, J=6.3Hz), 7.39 (1H, dd, J=1.6, 8.2Hz), 7.98 (1H, brs).

(189e) 7-Ethyl-5-fluoro-3,4-dihydronaphthalen-1(2H)-one

7-Bromo-5-fluoro-3,4-dihydronaphthalen-1(2H)-one produced in Example (189d) (3.82 g, 15.7 mmol) was dissolved in toluene (100 mL). Tetrakis(triphenylphosphine)palladium (550 mg, 0.476 mmol) and tetraethyltin (6.20 mL, 31.3 mmol) were added, and the mixture was stirred with heating to reflux for four hours. After completion of the reaction, the reaction solution was filtered through Celite, and the solvent was evaporated under reduced pressure. The resulting residue was purified by silica gel column chromatography (elution solvent: hexane/toluene = 2/1) to give the target compound (2.65 g, yield: 88%) as a light yellow solid.
$^1$H-NMR ($CDCl_3$, 400MHz):δ ppm: 1.23 (3H, t, J=7.4Hz), 2.13 (2H, m), 2.61-2.69 (4H, m), 2.91 (2H, q, J=7.4Hz), 7.06 (1H, dd, J=1.6, 9.8Hz), 7.66 (1H, brs).

(189f) 1-Bromo-7-ethyl-5-fluoro-3,4-dihydronaphthalene-2-carbaldehyde

Reaction and post-treatment were carried out in accordance with Example (179d) using 7-ethyl-5-fluoro-3,4-dihydronaphthalen-1(2H)-one produced in Example (189e) (2.65 g, 13.8 mmol), N,N-dimethylformamide (4.00 mL, 51.4 mmol), phosphorus bromide (4.00 mL, 42.1 mmol) and chloroform (200 mL), followed by further purification by silica gel column chromatography (elution solvent: hexane/ethyl acetate = 20/1) to give the target compound (2.50 g, yield: 64%) as a brown solid.
$^1$H-NMR ($CDCl_3$, 400MHz):δ ppm: 1.27 (3H, t, J=7.4Hz), 2.60 (2H, t, J=8.6Hz), 2.68 (2H, q, J=7.4Hz), 2.81 (2H, t, J=8.6Hz), 6.98 (1H, dd, J=1.6, 9.8Hz), 7.52 (1H, brs), 10.22 (1H, s).

(189g) 1-Bromo-7-ethyl-5-fluoro-2-naphthaldehyde

Reaction and post-treatment were carried out in accordance with Example (179e) using 1-bromo-7-ethyl-5-fluoro-3,4-dihydronaphthalene-2-carbaldehyde produced in Example (189f) (2.50 g, 8.83 mmol), 2,3-dichloro-5,6-dicyano-1,4-benzoquinone (4.00 g, 17.6 mmol) and 1,2-dichloroethane (100 mL) to give a mixture of the target compound and a 7-vinyl product.

(189h) 1-Bromo-7-ethyl-5-fluoro-2-methylnaphthalene

The mixture of 1-bromo-7-ethyl-5-fluoro-2-naphthaldehyde produced in Example (189g) was dissolved in ethanol (80 mL). Concentrated hydrochloric acid (15 mL) and a 10% palladium-carbon catalyst (1.0 g) were added, and the mixture was stirred in a hydrogen atmosphere at room temperature for five hours. The reaction solution was filtered through Celite, followed by extraction with diethyl ether (100 mL). The solvent was evaporated under reduced pressure, and the resulting residue was purified by silica gel column chromatography (elution solvent: hexane) to give the target compound (201 mg, yield: 12%, two steps) as a white solid. $^1$H-NMR ($CDCl_3$, 400MHz):δ ppm: 1.30 (3H, t, J=7.4Hz),

2.49 (3H, s), 2.76 (2H, q, J=7.4Hz), 6.92 (1H, dd, J=1.6, 11.4Hz), 7.27 (1H, dd, J=1.6, 8.6Hz), 7.29 (1H, s), 7.52 (1H, s), 7.91 (1H, d, J=8.6Hz).

(189i) 2-Ethyl-4-fluoro-7-methyl-1-naphthaldehyde

Dichloro(methoxy)methane (130 μL, 1.44 mmol) was dissolved in methylene chloride (20 mL). Tin tetrachloride (410 μL, 1.45 mmol) was added at 0˚C, and the mixture was stirred for one hour. 1-Bromo-7-ethyl-5-fluoro-2-methylnaphthalene produced in Example (189h) (200 mg, 1.06 mmol) was added, and then the mixture was stirred at room temperature for two hours. After completion of the reaction, water (20 mL) was added to the reaction solution, followed by extraction with diethyl ether (50 mL). The organic layer was washed with brine and then dried over anhydrous magnesium sulfate. The solvent was evaporated under reduced pressure, and the resulting residue was purified by silica gel column chromatography (elution solvent: hexane/ethyl acetate = 10/1) to give the target compound (185 mg, yield: 80%) as a light yellow oil.
$^1$H-NMR ($CDCl_3$, 400MHz):δ ppm: 1.36 (3H, t, J=7.4Hz), 2.56 (3H, s), 3.13 (2H, q, J=7.4Hz), 6.97 (1H, d, J=11.4Hz), 7.38 (1H, dd, J=1.6, 8.2Hz), 7.98 (1H, d, J=8.2Hz), 8.88 (1H, brs), 10.78 (1H, s).
MS (EI) m/z: 216 ($M^+$).

(189j) (9aS)-8-Acetyl-N-[(2-ethyl-4-fluoro-7-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (300 mg, 0.869 mmol), 2-ethyl-4-fluoro-7-methyl-1-naphthaldehyde produced in Example (189i) (185 mg, 0.855 mmol), triethylsilane (420 μL, 2.60 mmol), trifluoroacetic acid (200 μL, 2.60 mmol) and acetonitrile (15 mL) to give the target compound (377 mg, yield: 80%) as a yellow solid.
$^1$H-NMR ($CDCl_3$, 400MHz):δ ppm: 1.29 (3H, t, J=7.5Hz), 1.75 (3H, s), 2.55 (3H, s), 2.64 (3H, s), 2.91 (2H, q, J=7.5Hz), 3.62 (3H, s), 4.98-5.03 (2H, m), 6.01 (1H, s), 6.24 (1H, s), 6.95 (1H, m), 6.99 (1H, d, J=11.5Hz), 7.36 (1H, brd, J=8.3Hz), 7.85 (1H, s), 8.01 (1H, d, J=8.3Hz), 10.65 (1H, s), 18.83 (1H, s).
MS (ESI) m/z: 546.19298 $(M+H)^+$.

(Example 190) (9aS)-8-Acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide (exemplary compound No. 1-641)

(190a) 5,6,7-Trifluoro-3,4-dihydronaphthalen-2(1H)-one

(3,4,5-Trifluorophenyl)acetic acid (15.4 g, 81.0 mmol) was dissolved in methylene chloride (200 mL). N,N-Dimethylformamide (200 μL) and oxalyl chloride (7.77 mL, 89.1 mmol) were slowly added dropwise at room temperature, and the mixture was stirred for two hours. After completion of the reaction, the reaction solution was evaporated under reduced pressure and dried. The resulting crude product was dissolved in methylene chloride (200 mL) again, and aluminum chloride (16.0 g, 122 mmol) was added at 0˚C. After stirring at room temperature for 15 minutes, the mixture was slowly bubbled with methylene gas and directly stirred at room temperature for 1.5 hours. After completion of the reaction, a dilute hydrochloric acid solution (150 mL) was added to the reaction solution, followed by extraction with methylene chloride (200 mL). The organic layer was washed with brine and then dried over anhydrous sodium sulfate. The solvent was evaporated under reduced pressure, and the resulting residue was purified by silica gel column chromatography (elution solvent: hexane = 70/30) to give the target compound (14.7 g, yield: 91%, two steps) as yellow crystals.
$^1$H-NMR ($CDCl_3$, 400MHz):δ ppm: 2.55 (2H, t, J=6.6Hz), 3.08 (2H, t, J=6.6Hz), 3.52 (2H, s), 6.74-6.83 (1H, m).
MS (EI) m/z: 200 ($M^+$).

(190b) 2-Bromo-5,6,7-trifluoro-3,4-dihydronaphthalene-1-carbaldehyde

Phosphorus bromide (4.51 mL, 47.5 mmol) was slowly added dropwise to a solution of N,N-dimethylformamide (4.43 mL, 57.0 mmol) in chloroform (50 mL) at 0˚C, and the mixture was stirred in a nitrogen atmosphere at 0˚C for two hours. Thereafter, a solution of 5,6,7-trifluoro-3,4-dihydronaphthalen-2(1H)-one produced in Example (190a) (3.80 g, 19.0 mmol) in chloroform (25 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (80 mL) was slowly added, followed by extraction with methylene chloride (60 mL). The organic layer was washed with brine and then dried over anhydrous sodium sulfate. The solvent

was evaporated under reduced pressure, and the resulting residue was purified by silica gel column chromatography (elution solvent: hexane = 80/20) to give the target compound (3.05 g, yield: 55%) as yellow crystals.
$^1$H-NMR ($CDCl_3$, 400MHz):δ ppm: 2.92 (2H, t, J=7.6Hz), 3.05 (2H, t, J=7.6Hz), 7.78-7.88 (1H, m), 10.27 (1H, s).
MS (EI) m/z: 290 ($M^+$).

(190c) 2-Bromo-5,6,7-trifluoro-1-naphthaldehyde

2-Bromo-5,6,7-trifluoro-3,4-dihydronaphthalene-1-carbaldehyde produced in Example (190b) (13.2 g, 45.4 mmol) was dissolved in toluene (200 mL). 2,3-Dichloro-5,6-dicyano-1,4-benzoquinone (15.5 g, 68.3 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 60/40) to give the target compound (6.95 g, yield: 53%) as yellow crystals.
$^1$H-NMR ($CDCl_3$, 400MHz):δ ppm: 7.77 (1H, d, J=9.0Hz), 8.01 (1H, d, J=9.0Hz), 8.88 (1H, ddd, J=2.4, 7.2, 13.1Hz), 10.69 (1H, s).
MS (EI) m/z: 288 ($M^+$).

(190d) 5,6,7-Trifluoro-2-methyl-1-naphthaldehyde

2-Bromo-5,6,7-trifluoro-1-naphthaldehyde produced in Example (190c) (3.50 g, 12.1 mmol) was dissolved in N,N-dimethylformamide (50 mL). Tetrakis(triphenylphosphine)palladium (1.40 g, 1.21 mmol) and tetramethyltin (5.03 mL, 36.3 mmol) were added, and the mixture was stirred at 80˚C for two hours. After completion of the reaction, the reaction solution was cooled to 0˚C and then water (60 mL) was slowly added, followed by extraction with ethyl acetate (40 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 25/1) to give the target compound (2.48 g, yield: 92%) as yellowish white crystals.
$^1$H-NMR ($CDCl_3$, 400MHz):δ ppm: 2.86 (3H, s), 7.44 (1H, d, J=8.6Hz), 8.19 (1H, d, J=8.6Hz), 8.85 (1H, ddd, J=2.4, 7.2, 13.3Hz), 10.85 (1H, s).
MS (EI) m/z: 224 ($M^+$).

(190e) (9aS)-8-Acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (4.00 g, 11.6 mmol), 5,6,7-trifluoro-2-methyl-1-naphthaldehyde produced in Example (190d) (2.48 g, 11.1 mmol), triethylsilane (5.54 mL, 34.8 mmol), trifluoroacetic acid (2.68 mL, 34.8 mmol) and acetonitrile (120 mL) to give the target compound (4.29 g, yield: 67%) as a yellow solid.
$^1$H-NMR ($CDCl_3$, 500MHz):δ ppm: 1.75 (3H, s), 2.63 (3H, s), 2.64 (3H, s), 3.71 (3H, s), 4.95-5.04 (2H, m), 6.02 (1H, s), 6.26 (1H, s), 7.12 (1H, brs), 7.42 (1H, d, J=8.8Hz), 7.67-7.72 (1H, m), 7.96 (1H, d, J=8.8Hz), 10.73 (1H, s), 18.83 (1H, s). MS (ESI) m/z: 554.14176 $(M+H)^+$.

(Example 191) (9aS)-8-Acetyl-N-[(2-ethyl-5,6,7-trifluoro-1-naphthyl)methyl]-1,7-dihydroxy-3-methoxy-9a-methyl-9-oxo-9,9a-dihydrodibenzo[b,d]furan-4-carboxamide (exemplary compound No. 1-643)

(191a) 2-Ethyl-5,6,7-trifluoro-1-naphthaldehyde

2-Bromo-5,6,7-trifluoro-1-naphthaldehyde produced in Example (190c) (3.50 g, 12.1 mmol) was dissolved in N,N-dimethylformamide (50 mL). Tetrakis(triphenylphosphine)palladiuin (1.40 g, 1.21 mmol) and tetraethyltin (7.18 mL, 36.3 mmol) were added, and the mixture was stirred at 120˚C for two hours. After completion of the reaction, the reaction solution was cooled to 0˚C and then water (60 mL) was slowly added, followed by extraction with ethyl acetate (40 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 30/1) to give the target compound (2.88 g, yield: 100%) as yellowish white crystals.
$^1$H-NMR ($CDCl_3$, 400MHz):δ ppm: 1.38 (3H, t, J=7.6Hz), 3.18 (2H, q, J=7.6Hz), 7.43 (1H, d, J=8.6Hz), 8.19 (1H, d, J=8.6Hz), 8.83 (1H, ddd, J=2.4, 7.4, 13.3Hz), 10.78 (1H, s).
MS (EI) m/z: 238 ($M^+$).

(191b) (9aS)-8-Acetyl-N-[(2-ethyl-5,6,7-trifluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (4.00 g, 11.6 mmol), 2-ethyl-5,6,7-trifluoro-1-naphthaldehyde produced in Example (191a) (2.94 g, 12.3 mmol), triethylsilane (5.54 mL, 34.8 mmol), trifluoroacetic acid (2.68 mL, 34.8 mmol) and acetonitrile (120 mL) to give the target compound (4.51 g, yield: 69%) as a yellow solid.
$^1$H-NMR ($CDCl_3$, 500MHz):δ ppm: 1.31 (3H, t, J=7.6Hz), 1.75 (3H, s), 2.64 (3H, s), 2.96 (2H, q, J=7.6Hz), 3.68 (3H, s), 4.95-5.04 (2H, m), 6.04 (1H, s), 6.26 (1H, s), 6.99 (1H, brs), 7.44 (1H, d, J=8.8Hz), 7.68-7.72 (1H, m), 7.99 (1H, d, J=8.8Hz), 10.68 (1H, s), 18.83 (1H, s).
MS (ESI) m/z: 568.16202 $(M+H)^+$.

(Example 192) (9aS)-8-Acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide (exemplary compound No. 1-567)

(192a) 8-Fluoro-3,4-dihydronaphthalen-2(1H)-one

(2-Fluorophenyl)acetic acid (21.0 g, 136 mmol) was dissolved in methylene chloride (250 mL). N,N-Dimethylformamide (250 μL) and oxalyl chloride (13.1 mL, 150 mmol) were slowly added dropwise at room temperature, and the mixture was stirred for two hours. After completion of the reaction, the reaction solution was evaporated under reduced pressure and dried. The resulting crude product was dissolved in methylene chloride (250 mL) again, and aluminum chloride (27.2 g, 204 mmol) was added at 0˚C. After stirring at room temperature for 15 minutes, the mixture was slowly bubbled with methylene gas and directly stirred at room temperature for 1.5 hours. After completion of the reaction, a dilute hydrochloric acid solution (200 mL) was added to the reaction solution, followed by extraction with methylene chloride (200 mL). The organic layer was washed with brine and then dried over anhydrous sodium sulfate. The solvent was evaporated under reduced pressure, and the resulting residue was purified by silica gel column chromatography (elution solvent: hexane = 65/35) to give the target compound (15.0 g, yield: 67%, two steps) as a yellow solid.
$^1$H-NMR ($CDCl_3$, 500MHz):δ ppm: 2.60 (2H, t, J=6.8Hz), 3.10 (2H, t, J=6.8Hz), 3.58 (2H, s), 6.95 (1H, t, J=8.8Hz), 7.02 (1H, d, J=7.3Hz), 7.19 (1H, dd, J=7.8, 13.7Hz).

(192b) 2-Bromo-8-fluoro-3,4-dihydronaphthalene-1-carbaldehyde

Phosphorus bromide (25.4 mL, 268 mmol) was slowly added dropwise to a solution of N,N-dimethylformamide (24.9 mL, 320 mmol) in chloroform (200 mL) at 0˚C, and the mixture was stirred in a nitrogen atmosphere at 0˚C for two hours. Thereafter, a solution of 8-fluoro-3,4-dihydronaphthalen-2(1H)-one produced in Example (192a) (17.5 g, 107 mmol) in chloroform (100 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (300 mL) was slowly added, followed by extraction with methylene chloride (150 mL). The organic layer was washed with brine and then dried over anhydrous sodium sulfate. The solvent was evaporated under reduced pressure, and the resulting residue was purified by silica gel column chromatography (elution solvent: hexane = 50/1 to 20/1) to give the target compound (15.6 g, yield: 57%) as a yellow syrup.
$^1$H-NMR ($CDCl_3$, 500MHz):δ ppm: 2.86-2.94 (4H, m), 6.95-6.99 (2H, m), 7.22 (1H, ddd, J=5.2, 7.6, 7.6Hz), 10.17 (1H, d, J=4.4Hz).
MS (EI) m/z: 254 $(M^+)$.

(192c) 2-Bromo-8-fluoro-1-naphthaldehyde

2-Bromo-8-fluoro-3,4-dihydronaphthalene-1-carbaldehyde produced in Example (192b) (15.6 g, 61.2 mmol) was dissolved in toluene (300 mL). 2,3-Dichloro-5,6-dicyano-1,4-benzoquinone (20.8 g, 91.7 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 50/50) to give the target compound (4.84 g, yield: 31%) as yellow crystals.
$^1$H-NMR ($CDCl_3$, 400MHz):δ ppm: 7.49 (1H, ddd, J=5.1, 7.8, 7.8Hz), 7.66 (1H, d, J=9.0Hz), 7.67 (1H, d, J=9.0Hz), 7.79 (1H, dd, J=2.0, 8.6Hz), 10.62 (1H, d, J=9.0Hz). MS (EI) m/z: 252 $(M^+)$.

(192d) 2-Ethyl-8-fluoro-1-naphthaldehyde

2-Bromo-8-fluoro-1-naphthaldehyde produced in Example (192c) (0.340 g, 1.34 mmol) was dissolved in N,N-dimethylformamide (5 mL).
Tetrakis(triphenylphosphine)palladium (0.155 g, 0.134 mmol) and tetraethyltin (0.398 mL, 2.01 mmol) were added, and the mixture was stirred at 120˚C for two hours. After completion of the reaction, the reaction solution was cooled to 0˚C and then water (8 mL) was slowly added, followed by extraction with ethyl acetate (5 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 50/1) to give the target compound (0.206 g, yield: 76%) as a yellow syrup.
$^1$H-NMR ($CDCl_3$, 400MHz):δ ppm: 1.30 (3H, t, J=7.4Hz), 2.90 (2H, q, J=7.4Hz), 7.26 (1H, ddd, J=1.2, 8.2, 12.9Hz), 7.43 (1H, ddd, J=5.1, 8.2, 8.2Hz), 7.44 (1H, d, J=8.6Hz), 7.67 (1H, dd, J=1.2, 8.2Hz), 7.90 (1H, dd, J=2.0, 8.6Hz), 10.89 (1H, d, J=11.7Hz).
MS (EI) m/z: 202 ($M^+$).

(192e) (9aS)-8-Acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (4.00 g, 11.6 mmol), 2-ethyl-8-fluoro-1-naphthaldehyde produced in Example (192d) (2.38 g, 11.8 mmol), triethylsilane (7.38 mL, 46.3 mmol), trifluoroacetic acid (3.57 mL, 46.3 mmol) and acetonitrile (70 mL) to give the target compound (2.62 g, yield: 42%) as a yellow solid.
$^1$H-NMR ($CDCl_3$, 400MHz):δ ppm: 1.31 (3H, t, J=7.5Hz), 1.71 (3H, s), 2.63 (3H, s), 3.11-3.29 (2H, m), 3.70 (3H, s), 5.04-5.19 (2H, m), 5.97 (1H, s), 6.23 (1H, s), 7.20 (1H, dd, J=7.5, 15.1Hz), 7.35 (1H, ddd, J=1.6, 7.5, 8.5Hz), 7.38 (1H, d, J=8.3Hz), 7.61 (1H, d, J=8.3Hz), 7.73 (1H, dd, J=1.2, 8.5Hz), 7.88 (1H, brs), 10.62 (1H, s), 18.71 (1H, s).
MS (ESI) m/z: 532.17693 $(M+H)^+$.

(Example 193) (9aS)-8-Acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide (exemplary compound No. 1-632)

(193a) 7,8-Difluoro-3,4-dihydronaphthalen-2(1H)-one

(2,3-Difluorophenyl)acetic acid (15.1 g, 87.7 mmol) was dissolved in methylene chloride (200 mL). N,N-Dimethylformamide (200 μL) and oxalyl chloride (8.42 mL, 96.5 mmol) were slowly added dropwise at room temperature, and the mixture was stirred for two hours. After completion of the reaction, the reaction solution was evaporated under reduced pressure and dried. The resulting crude product was dissolved in methylene chloride (200 mL) again, and aluminum chloride (17.5 g, 132 mmol) was added at 0˚C. After stirring at room temperature for 15 minutes, the mixture was slowly bubbled with methylene gas and directly stirred at room temperature for 1.5 hours. After completion of the reaction, a dilute hydrochloric acid solution (150 mL) was added to the reaction solution, followed by extraction with methylene chloride (200 mL). The organic layer was washed with brine and then dried over anhydrous sodium sulfate. The solvent was evaporated under reduced pressure, and the resulting residue was purified by silica gel column chromatography (elution solvent: hexane = 70/30) to give the target compound (11.5 g, yield: 71 %, two steps) as yellow crystals.
$^1$H-NMR ($CDCl_3$, 400MHz):δ ppm: 2.58 (2H, t, J=6.7Hz), 3.06 (2H, t, J=6.7Hz), 3.58 (2H, s), 6.94 (1H, dd, J=4.7, 8.6Hz), 7.00 (1H, ddd, J=7.4, 8.6, 9.8Hz).
MS (EI) m/z: 182 ($M^+$).

(193b) 2-Bromo-7,8-difluoro-3,4-dihydronaphthalene-1-carbaldehyde

Phosphorus bromide (14.8 mL, 156 mmol) was slowly added dropwise to a solution of N,N-dimethylformamide (14.6 mL, 187 mmol) in chloroform (200 mL) at 0˚C, and the mixture was stirred in a nitrogen atmosphere at 0˚C for two hours. Thereafter, a solution of 7,8-difluoro-3,4-dihydronaphthalen-2(1H)-one produced in Example (193a) (11.5 g, 62.4 mmol) in chloroform (100 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (300 mL) was slowly added, followed by extraction with methylene chloride (150 mL). The organic layer was washed with brine and then dried over anhydrous sodium sulfate. The solvent was evaporated under reduced pressure, and the resulting residue was purified by silica gel column chromatography

(elution solvent: hexane = 90/10) to give the target compound (9.5 g, yield: 56%) as yellow crystals.
$^1$H-NMR ($CDCl_3$, 400MHz):δ ppm: 2.83 (2H, t, J=7.0Hz), 2.93 (2H, dt, J=1.6, 7.0Hz), 6.91 (1H, ddd, J=1.6, 4.7, 8.6Hz), 7.05 (1H, ddd, J=7.8, 8.6, 9.8Hz), 10.20 (1H, d, J=3.9Hz).
MS (EI) m/z: 272 ($M^+$).

(193c) 2-Bromo-7,8-difluoro-1-naphthaldehyde

2-Bromo-7,8-difluoro-3,4-dihydronaphthalene-1-carbaldehyde produced in Example (193b) (9.5 g, 34.8 mmol) was dissolved in toluene (200 mL). 2,3-Dichloro-5,6-dicyano-1,4-benzoquinone (11.8 g, 52.2 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 50/50) to give the target compound (3.37g, yield: 36%) as yellow crystals.
$^1$H-NMR ($CDCl_3$, 500MHz):δ ppm: 7.45 (1H, ddd, J=7.3, 8.6, 9.3Hz), 7.65-7.68 (2H, m), 7.79 (1H, dd, J=2.0, 8.8Hz), 10.65 (1H, d, J=7.8Hz).
MS (EI) m/z: 270 ($M^+$).

(193d) 2-Ethyl-7,8-difluoro-1-naphthaldehyde

2-Bromo-7,8-difluoro-1-naphthaldehyde produced in Example (193c) (0.383 g, 1.41 mmol) was dissolved in toluene (5 mL).
Tetrakis(triphenylphosphine)palladium (0.163 g, 0.141 mmol) and tetraethyltin (0.698 mL, 3.53 mmol) were added, and the mixture was stirred at 150˚C for 12 hours. After completion of the reaction, the reaction solution was cooled to 0˚C and then water (5 mL) was slowly added, followed by extraction with ethyl acetate (5 mL). The organic layer was washed with brine and then dried over anhydrous sodium sulfate. The solvent was evaporated under reduced pressure, and the resulting residue was purified by silica gel column chromatography (elution solvent: hexane/ethyl acetate = 100/1 to 50/1) to give the target compound (0.232 g, yield: 75%) as white crystals.
$^1$H-NMR ($CDCl_3$, 400MHz):δ ppm: 1.30 (3H, t, J=7.6Hz), 2.91 (2H, q, J=7.6Hz), 7.35 (1H, ddd, J=7.4, 9.0, 9.0Hz), 7.39 (1H, d, J=8.6Hz), 7.63 (1H, ddd, J=1.6, 5.1, 9.0Hz), 7.85 (1H, dd, J=2.0, 8.6Hz), 10.87 (1H, d, J=11.0Hz).
MS (EI) m/z: 220 ($M^+$).

(193e) (9aS)-8-Acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (2.26 g, 6.54 mmol), 2-ethyl-7,8-difluoro-1-naphthaldehyde produced in Example (193d) (1.44 g, 6.54 mmol), triethylsilane (4.17 mL, 26.2 mmol), trifluoroacetic acid (2.01 mL, 26.2 mmol) and acetonitrile (30 mL) to give the target compound (2.20 g, yield: 61%) as a yellow solid.
$^1$H-NMR ($CDCl_3$, 400MHz):δ ppm: 1.31 (3H, t, J=7.5Hz), 1.75 (3H, s), 2.63 (3H, s), 3.14-3.34 (2H, m), 3.77 (3H, s), 5.02-5.18 (2H, m), 5.99 (1H, s), 6.25 (1H, s), 7.30 (1H, ddd, J=7.5, 8.7, 9.3Hz), 7.34 (1H, d, J=8.7Hz), 7.58 (1H, ddd, J=1.6, 5.2, 8.7Hz), 7.68 (1H, dd, J=2.0, 8.7Hz), 7.98 (1H, brs), 10.65 (1H, s), 18.75 (1H, s). MS (ESI) m/z: 550.16736 $(M+H)^+$.

(193f) 7,8-Difluoro-2-vinyl-1-naphthaldehyde

2-Bromo-7,8-difluoro-1-naphthaldehyde produced in Example (193c) (3.37 g, 12.4 mmol) was dissolved in toluene (50 mL).
Tetrakis(triphenylphosphine)palladium (1.43 g, 1.24 mmol) and tetravinyltin (3.99 mL, 13.7 mmol) were added, and the mixture was stirred at 80˚C for three hours. After completion of the reaction, the reaction solution was cooled to 0˚C and then water (50 mL) was slowly added, followed by extraction with ethyl acetate (40 mL). The organic layer was washed with brine and then dried over anhydrous sodium sulfate. The solvent was evaporated under reduced pressure, and the resulting residue was simply purified by silica gel column chromatography (elution solvent: hexane/ethyl acetate = 100/1 to 50/1) to give the target crude product (2.57 g, yield: 95%) as a yellow solid.

(193d) 2-Ethyl-7,8-difluoro-1-naphthaldehyde

The crude product of 7,8-difluoro-2-vinyl-1-naphthaldehyde produced in Example (193f) (2.57 g, 11.8 mmol) was dissolved in ethanol (35 mL). 10% palladium-carbon (3.76 g) was added, and the mixture was stirred in a hydrogen

(1 atm) atmosphere for four hours. Palladium-carbon was separated by filtration, and then the solvent was evaporated under reduced pressure. The resulting residue was purified by silica gel column chromatography (elution solvent: hexane/ethyl acetate = 100/0 to 90/10) to give the target compound (1.44 g, yield: 56%) as a yellow solid.

(Example 194) (9aS)-8-Acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide (exemplary compound No. 1-631)

(194a) 6,8-Difluoro-3,4-dihydronaphthalen-2(1H)-one

(2,4-Difluorophenyl)acetic acid (2.00 g, 11.6 mmol) was dissolved in methylene chloride (25 mL). N,N-Dimethylformamide (30 μL) and oxalyl chloride (1.11 mL, 12.8 mmol) were slowly added dropwise at room temperature, and the mixture was stirred for two hours. After completion of the reaction, the reaction solution was evaporated under reduced pressure and dried. The resulting crude product was dissolved in methylene chloride (30 mL) again, and aluminum chloride (2.32 g, 17.4 mmol) was added at 0˚C. After stirring at room temperature for 15 minutes, the mixture was slowly bubbled with methylene gas and directly stirred at room temperature for 1.5 hours. After completion of the reaction, a dilute hydrochloric acid solution (30 mL) was added to the reaction solution, followed by extraction with methylene chloride (20 mL). The organic layer was washed with brine and then dried over anhydrous sodium sulfate. The solvent was evaporated under reduced pressure, and the resulting residue was purified by silica gel column chromatography (elution solvent: hexane = 70/30) to give the target compound (1.37 g, yield: 65%, two steps) as yellow crystals.
$^1$H-NMR ($CDCl_3$, 500MHz):δ ppm: 2.59 (2H, t, J=6.8Hz), 3.08 (2H, t, J=6.8Hz), 3.52 (2H, s), 6.72 (1H, dt, J=2.4, 8.8Hz), 6.79 (1H, dd, J=2.4, 8.8Hz).
MS (EI) m/z: 182 ($M^+$).

(194b) 2-Bromo-6,8-difluoro-3,4-dihydronaphthalene-1-carbaldehyde

Phosphorus bromide (1.79 mL, 18.8 mmol) was slowly added dropwise to a solution of N,N-dimethylformamide (1.75 mL, 22.6 mmol) in chloroform (20 mL) at 0˚C, and the mixture was stirred in a nitrogen atmosphere at 0˚C for two hours. Thereafter, a solution of 6,8-difluoro-3,4-dihydronaphthalen-2(1H)-one produced in Example (194a) (1.37 g, 7.52 mmol) in chloroform (10 mL) was slowly added at 0˚C. The mixture was heated to room temperature and then stirred with heating to reflux for two hours. After completion of the reaction, the reaction solution was cooled to 0˚C and then a saturated sodium bicarbonate aqueous solution (30 mL) was slowly added, followed by extraction with methylene chloride (15 mL). The organic layer was washed with brine and then dried over anhydrous sodium sulfate. The solvent was evaporated under reduced pressure, and the resulting residue was purified by silica gel column chromatography (elution solvent: hexane = 80/20) to give the target compound (1.19 g, yield: 58%) as yellow crystals.
$^1$H-NMR ($CDCl_3$, 500MHz):δ ppm: 2.84-2.94 (4H, m), 6.71 (1H, dd, J=2.4, 8.8Hz), 6.74 (1H, dt, J=2.4, 8.8Hz), 10.16 (1H, d, J=3.4Hz).
MS (EI) m/z: 272 ($M^+$).

(194c) 2-Bromo-6,8-difluoro-1-naphthaldehyde

2-Bromo-6,8-difluoro-3,4-dihydronaphthalene-1-carbaldehyde produced in Example (194b) (0.900 mg, 3.30 mmol) was dissolved in toluene (50 mL). 2,3-Dichloro-5,6-dicyano-1,4-benzoquinone (1.50 g, 6.59 mmol) was added, and the mixture was stirred with heating to reflux for 72 hours. After completion of the reaction, the reaction solution was evaporated under reduced pressure. The resulting residue was purified by silica gel column chromatography (elution solvent: hexane/toluene = 90/10 to 50/50) to give the target compound (0.142 g, yield: 12%) as white crystals.
$^1$H-NMR ($CDCl_3$, 400MHz):δ ppm: 7.13 (1H, ddd, J=2.4, 8.6, 12.1Hz), 7.35 (1H, ddd, J=1.2, 2.4, 8.6Hz), 7.70 (1H, d, J=9.0Hz), 7.75 (1H, dd, J=1.2, 9.0Hz), 10.61 (1H, d, J=9.0Hz).
MS (EI) m/z: 270 ($M^+$).

(194d) 2-Ethyl-6,8-difluoro-1-naphthaldehyde

2-Bromo-6,8-difluoro-1-naphthaldehyde produced in Example (194c) (0.142 g, 0.524 mmol) was dissolved in toluene (2.5 mL).
Tetrakis(triphenylphosphine)palladium (0.060 g, 0.0524 mmol) and tetraethyltin (0.259 mL, 1.31 mmol) were added, and the mixture was stirred at 150˚C for three hours. After completion of the reaction, the reaction solution was cooled to 0˚C and then water (3 mL) was slowly added, followed by extraction with ethyl acetate (2 mL). The organic layer was washed with brine and then dried over anhydrous sodium sulfate. The solvent was evaporated under reduced pressure,

and the resulting residue was purified by silica gel column chromatography (elution solvent: hexane/ethyl acetate = 100/1 to 50/1) to give the target compound (0.050 g, yield: 43%) as a yellow syrup.
$^1$H-NMR ($CDCl_3$, 400MHz):δ ppm: 1.29 (3H, t, J=7.6Hz), 2.87 (2H, q, J=7.6Hz), 7.09 (1H, ddd, J=2.4, 8.6, 12.5Hz), 7.31 (1H, ddd, J=0.8, 2.4, 8.6Hz), 7.45 (1H, d, J=8.6Hz), 7.82 (1H, dd, J=2.0, 8.6Hz), 10.84 (1H, d, J=12.1Hz).
MS (EI) m/z: 220 ($M^+$).

(194e) (9aS)-8-Acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (0.100 g, 0.290 mmol), 2-ethyl-6,8-difluoro-1-naphthaldehyde produced in Example (194d) (0.050 g, 0.227 mmol), triethylsilane (0.185 mL, 1.16 mmol), trifluoroacetic acid (0.089 mL, 1.16 mmol) and acetonitrile (2 mL) to give the target compound (0.028 g, yield: 23%) as a yellow solid.
$^1$H-NMR ($CDCl_3$, 400MHz):δ ppm: 1.30 (3H, t, J=7.5Hz), 1.71 (3H, s), 2.63 (3H, s), 3.09-3.28 (2H, m), 3.73 (3H, s), 5.00-5.16 (2H, m), 5.99 (1H, s), 6.25 (1H, s), 7.05 (1H, ddd, J=2.4, 8.3, 14.3Hz), 7.28 (1H, dd, J=2.4, 8.7Hz), 7.40 (1H, d, J=8.3Hz), 7.66 (1H, dd, J=2.0, 8.3Hz), 7.82 (1H, brs), 10.65 (1H, s), 18.76 (1H, s).
MS (ESI) m/z: 550.16871 $(M+H)^+$.

(Example 195) (9aS)-8-Acetyl-N-[(2-ethyl-5-fluoro-7-methyl-1-naphthyl)methyl]-1,7-dihydroxy-3-methoxy-9a-methyl-9-oxo-9,9a-dihydrodibenzo[b,d]furan-4-carboxamide (exemplary compound No. 1-638)

(195a) 5-Fluoro-7-methyl-3,4-dihydronaphthalen-1(2H)-one

7-Bromo-5-fluoro-3,4-dihydronaphthalen-1(2H)-one produced in Example (189d) (5.20 g, 21.4 mmol) was dissolved in toluene (100 mL). Tetrakis(triphenylphosphine)palladium (750 mg, 0.649 mmol) and tetramethyltin (6.00 mL, 43.3 mmol) were added, and the mixture was stirred with heating to reflux for six hours. After completion of the reaction, the reaction solution was filtered through Celite, and the solvent was evaporated under reduced pressure. The resulting residue was purified by silica gel column chromatography (elution solvent: hexane/toluene = 2/1) to give the target compound (3.81 g, yield: 100%) as a light yellow solid.
$^1$H-NMR ($CDCl_3$, 400MHz):δ ppm: 2.13 (2H, m), 2.36 (3H, s), 2.64 (2H, t, J=6.3Hz), 2.91 (2H, t, J=6.3Hz), 7.06 (1H, brd, J=9.8Hz), 7.66 (1H, s).

(195b) 1-Bromo-5-fluoro-7-methyl-3,4-dihydronaphthalene-2-carbaldehyde

Reaction and post-treatment were carried out in accordance with Example (179d) using 5-fluoro-7-methyl-3,4-dihydronaphthalen-1(2H)-one produced in Example (195a) (4.15 g, 21.4 mmol), N,N-dimethylformamide (6.10 mL, 78.4 mmol), phosphorus bromide (6.10 mL, 64.2 mmol) and chloroform (200 mL), followed by further purification by silica gel column chromatography (elution solvent: hexane/ethyl acetate = 20/1) to give the target compound (3.93 g, yield: 68%) as a yellow solid.
$^1$H-NMR ($CDCl_3$, 400MHz):δ ppm: 2.40 (3H, s), 2.60 (2H, t, J=7.4Hz), 2.82 (2H, t, J=7.4Hz), 6.97 (1H, brd, J=9.4Hz), 7.53 (1H, brs), 10.25 (1H, s).

(195c) 4-Bromo-8-fluoro-6-methyl-3-vinyl-1,2-dihydronaphthalene

Sodium hydride (55%, 880 mg, 20.2 mmol) was suspended in tetrahydrofuran (100 mL). Methyltriphenylphosphonium bromide (7.90 g, 22.1 mmol) was added at 0˚C, and the mixture was directly stirred for 30 minutes. 1-Bromo-5-fluoro-7-methyl-3,4-dihydronaphthalene-2-carbaldehyde produced in Example (195b) (3.93 g, 14.6 mmol) in tetrahydrofuran (20 mL) was added to the reaction solution at 0˚C, and the mixture was stirred at room temperature for two hours. After completion of the reaction, water (100 mL) was added to the reaction solution, followed by extraction with diethyl ether (100 mL). The organic layer was washed with brine and then dried over anhydrous magnesium sulfate. The solvent was evaporated under reduced pressure, and the resulting residue was purified by silica gel column chromatography (elution solvent: hexane) to give the target compound (3.60 g, yield: 92%) as a white solid.
$^1$H-NMR ($CDCl_3$, 400MHz):δ ppm: 2.34 (3H, s), 2.55 (2H, t, J=7.4Hz), 2.82 (2H, t, J=7.4Hz), 5.38 (1H, d, J=11.0Hz), 5.52 (1H, d, J=17.2Hz), 6.77 (1H, d, J=9.8Hz), 7.11 (1H, dd, J=11.0, 17.2Hz), 7.32 (1H, s).

(195d) 1-Bromo-5-fluoro-7-methyl-2-vinylnaphthalene

Reaction and post-treatment were carried out in accordance with Example (179e) using 4-bromo-8-fluoro-6-methyl-3-vinyl-1,2-dihydronaphthalene produced in Example (195c) (3.60 g, 13.6 mmol), 2,3-dichloro-5,6-dicyano-1,4-benzoquinone (6.20 g, 27.3 mmol) and 1,2-dichloroethane (150 mL) to give the target compound (1.70 g, yield: 48%) as a white solid.
$^1$H-NMR ($CDCl_3$, 400MHz):δ ppm: 2.54 (3H, s), 5.49 (1H, dd, J=0.8, 11.0Hz), 5.83 (1H, dd, J=0.8, 17.2Hz), 7.01 (1H, dd, J=1.6, 10.6Hz), 7.33 (1H, dd, J=11.0, 17.2Hz), 7.61 (1H, d, J=9.0Hz), 7.89 (1H, s), 7.95 (1H, d, J=9.0Hz).

(195e) 1-Bromo-2-ethyl-5-fluoro-7-methylnaphthalene

1-Bromo-5-fluoro-7-methyl-2-vinylnaphthalene produced in Example (195d) (1.70 g, 6.41 mmol) was dissolved in ethyl acetate (80 mL). A 10% palladium-carbon catalyst (200 mg) was added, and the mixture was stirred in a hydrogen atmosphere at room temperature for two hours. The reaction solution was filtered through Celite and the solvent was evaporated under reduced pressure to give a crude purified product of the target compound.
$^1$H-NMR ($CDCl_3$, 400MHz):δ ppm: 1.29 (3H, d, J=7.4Hz), 2.53 (3H, s), 2.98 (2H, q, J=7.4Hz), 6.98 (1H, brd, J=9.8Hz), 7.32 (1H, d, J=8.2Hz), 7.85 (1H, s), 7.93 (1H, d, J=8.2Hz).

(195f) 2-Ethyl-5-fluoro-7-methyl-1-naphthaldehyde

Reaction and post-treatment were carried out in accordance with Example (174b) using the crude purified product of 1-bromo-2-ethyl-5-fluoro-7-methylnaphthalene produced in Example (195e), butyllithium (2.67 M solution in hexane, 3.40 mL, 9.08 mmol), N,N-dimethylformamide (2.50 mL, 32.1 mmol) and tetrahydrofuran (70 mL) to give the target compound (450 mg, yield: 32%, two steps) as a yellow solid.
$^1$H-NMR ($CDCl_3$, 400MHz):δ ppm: 1.36 (2H, t, J=7.4Hz), 2.53 (3H, s), 3.14 (2H, q, J=7.4Hz), 7.01 (1H, d, J=10.6Hz), 7.34 (1H, d, J=8.6Hz), 8.19 (1H, d, J=8.6Hz), 8.54 (1H, s), 10.85 (1H, s).
MS (EI) m/z: 216 ($M^+$).

(195g) (9aS)-8-Acetyl-N-[(2-ethyl-5-fluoro-7-methyl-1-naphthyl)methyl]-1,7-dihydroxy-3-methoxy-9a-methyl-9-oxo-9,9a-dihydrodibenzo[b,d]furan-4-carboxamide

Reaction and post-treatment were carried out in accordance with Example 10 using (9aS)-8-acetyl-1,7-dihydroxy-3-methoxy-9a-methyl-9-oxo-9,9a-dihydrodibenzo[b,d]furan-4-carboxamide produced in Example (1a) (790 mg, 2.29 mmol), 2-ethyl-5-fluoro-7-methyl-1-naphthaldehyde produced in Example (195f) (450 mg, 2.08 mmol), triethylsilane (1.00 mL, 6.19 mmol), trifluoroacetic acid (480 μL, 6.23 mmol) and acetonitrile (25 mL) to give the target compound (803 mg, yield: 71 %) as a yellow solid.
$^1$H-NMR ($CDCl_3$, 400MHz):δ ppm: 1.30 (3H, t, J=7.5Hz), 1.75 (3H, s), 2.53 (3H, s), 2.64 (3H, s), 2.95 (2H, q, J=7.5Hz), 3.62 (3H, s), 5.02-5.06 (2H, m), 6.02 (1H, s), 6.24 (1H, s), 6.98 (1H, d, J=10.7Hz), 7.00 (1H, m), 7.37 (1H, brd, J=8.7Hz), 7.64 (1H, s), 8.00 (1H, d, J=8.7Hz), 10.65 (1H, s), 18.83 (1H, s).
MS (ESI) m/z: 546.19650 $(M+H)^+$.

The known substance cercosporamide was produced according to the following Reference Examples.

(Reference Example 1) Fermentation of Lachnum fuscescens SANK 19096 with flask

A section of mycelia measuring about 5 mm on each side was cut out from a slant growing of strain SANK 19096 and was suspended in about 2 ml of physiological saline and homogenized with a glass potter. The entire amount was then aseptically inoculated into a 100 ml volumetric Erlenmeyer flask containing 30 ml of the medium shown in Table 1 (referred to as "Medium A"). Pre-culture was then carried out for 5 days at 23˚C and 210 revolutions per minute (abbreviated as "rpm") on a rotary shaker.

After the resulting pre-culture was inoculated at 5% (volume/volume) (abbreviated as "v/v") into a 500 ml volumetric Erlenmeyer flask containing 80 ml of Medium A, fermentation was carried out for 7 days at 26˚C and 210 rpm on a rotary shaker. HPLC analysis was then carried out under the conditions to be described later. At that time, a peak which possessed an equal retention time of cercosporamide (FIG. 1) was observed in the culture. The peak at 9.315 minutes in FIG. 1 indicates cercosporamide.

(Table 4)

| Medium A: Medium Composition | |
|---|---|
| Sucrose | 20 g |
| Raw potato | 100 g |
| Polypepton | 10 g |
| Potassium dihydrogenphosphate | 5 g |
| Magnesium sulfate heptahydrate | 2.5 g |
| Antifoaming agent CB-442 (NOF Corp.) | 100 mg |
| Tap water | 1000 ml |

The medium was sterilized at 121 ˚C for 20 minutes without adjusting pH.
HPLC analysis was carried out under the conditions indicated below.

Separation column: YMC J'sphere ODS-H80 S-4, 4.6ϕ x 150 mm (YMC Co., Ltd.)
Mobile phase: acetonitrile: 0.4% triethylamine-phosphate buffer (pH 3.2) (45:55)
Flow rate: 1.0 ml/minute
Detection: Ultraviolet absorption at 225 nm
Retention time: 9.3 minutes

(Reference Example 2) Large-Scale Fermentation of Lachnum fuscescens SANK 19096

A section of mycelia from a slant growing of strain SANK 19096 was suspended in about 2 ml of physiological saline and homogenized with a glass potter. The entire amount was inoculated into a 100 ml volumetric Erlenmeyer flask containing 30 ml of the medium shown in Table 5 (referred to as "Medium B") followed by cultivation for 7 days at 23˚C and 210 rpm on a rotary shaker. Following completion of the culture, an equal volume of 20% glycerol was added and mixed into the culture broth. This mixture was then stored at -80˚C and used as a seed culture broth for further use.
2 ml of the seed culture broth was inoculated into a 100 ml volumetric Erlenmeyer flask containing 30 ml of Medium B. The initial pre-cultivation (first pre-cultivation) was carried out for 5 days at 23˚C and 210 rpm on a rotary shaker.
Culture broth obtained in the first pre-culture (first pre-culture broth) was inoculated at 5% (v/v) into a 2 L volumetric Erlenmeyer flask containing 500 ml of Medium B followed by carrying out a second process of pre-culture (second pre-cultivation) for 4 days at 23˚C and 210 rpm on a rotary shaker.
Culture broth obtained in the second pre-culture (second pre-cultivation broth) was inoculated at 5% (v/v) into a 60 L volumetric fermenter containing 30 L of Medium B. A third process of pre-culturing (third pre-cultivation) was carried out for 2 days at an aeration rate of 30 L per minute and a culturing temperature of 23˚C while adjusting the agitation speed so as to maintain the dissolved oxygen concentration within the range of 3 to 5 ppm.
Culture broth obtained in the third pre-culture (third pre-cultivation broth) was inoculated at 5% (v/v) into a 600 L volumetric fermenter containing 300 L of Medium B. A fourth process of pre-culturing (fourth pre-cultivation) was carried out for 2 days at an aeration rate of 300 L per minute and a culturing temperature of 23˚C while adjusting the agitation speed so as to maintain the dissolved oxygen concentration within the range of 3 to 5 ppm.
Culture broth obtained in the fourth pre-culture (fourth pre-cultivation broth) was inoculated at 5% (v/v) into a 6,000 L volumetric fermenter containing 4,000 L of Medium B. Fermentation was carried out for 8 days at a culturing temperature of 26˚C and an aeration rate of 2,000 L per minute while adjusting the agitation speed so as to maintain the dissolved oxygen concentration at 5 ppm. 200 L each of 20% sucrose solution was added on days 4 and 5 of the fermentation. 300 L each of 20% sucrose solution was further added on days 6 and 7 of the fermentation. Cercosporamide production was confirmed by the HPLC under the conditions indicated below.

(Table 5)

| Medium B: Medium Composition | |
|---|---|
| Glucose | 40 g |
| Potato granules | 20 g |
| Polypepton | 10 g |
| Potassium dihydrogenphosphate | 5 g |
| Magnesium sulfate heptahydrate | 2.5 g |
| Antifoaming agent CB-442 (NOF Corp.) | 100 mg |

(continued)

| Medium B: Medium Composition |
| --- |
| Tap water 1000 ml |

The medium was sterilized at 121 ˚C for 20 minutes without adjusting pH.

HPLC analysis was carried out under the conditions indicated below.

Separation column: Cadenza CD-C18, 4.6ϕ x 75 mm (Imtakt Corp.)
Mobile phase: acetonitrile: 0.02% trifluoroacetic acid (40:60); eluted while changing solvent composition for 8 minutes (90:10)
Flow rate: 1.0 ml/minute
Detection: Ultraviolet absorption at 225 nm
Retention time: 5.4 minutes

(Reference Example 3) Purification of Cercosporamide from Fermented Broth of Lachnum fuscescens SANK 19096

The desired substance in the form of cercosporamide was confirmed by the HPLC under the conditions described in Reference Example 2.

4,800 L of a fermented broth of Lachnum fuscescens SANK 19096 obtained in Reference Example 2 was filtered using Celite 545 (Celite Corp.) as a filtration aid. 2,500 L of tap water was added to 781 kg of the resulting mycelial cake including the Celite and the cake as uniformly suspended therein. The material was then extracted by addition of 2,500 L of acetone to the suspension. 4,973 L of the resulting filtered extract was applied to a 80 L Diaion HP-20 column (Mitsubishi Chemical Corp.) equilibrated with about 500 L of a 50% aqueous acetone solution. The Diaion HP-20 column was washed with a 50% aqueous acetone solution. The liquid that passed solution through the column and washing solution were combined to obtain 5,500 L of solution.

75% (v/v) sulfuric acid was added to the resulting solution to adjust the pH to 3.0 followed by extraction by addition of 3,000 L of ethyl acetate. 4,218 L of solution extracted with ethyl acetate was washed with 1,000 L of 25% brine. The ethyl acetate was removed from 3,841 L of washed extract by concentration in vacuo. After concentrating to 81 L in vacuo, the ethyl acetate was further evaporated off using a 20 L volumetric rotary evaporator to obtain a concentrate.

About 10 L of the resulting concentrate was allowed to stand at 4˚C to crystallize cercosporamide. 4,700 g of the resulting wet crystal was dried in vacuo to obtain 4,140 g of dry cercosporamide crystals.

Test Example 1: Blood glucose lowering effect

Six-week-old male KK mice were purchased from CLEA Japan, Inc. and then were fed until 15 to 20 weeks old and affected with diabetes. The mice were individually fed during the adaptation period and the test period, and water and feed (FR2, Funabashi Farm) were freely ingested.

At the start of the experiment, after body weight measurement, blood was collected from the tail vein of the mice into a heparin-coated glass tube and centrifuged, and then plasma was separated. The glucose level in the plasma was measured by Glucoloader GXT (A&T Corp.), and individuals having a blood glucose level of about 350 mg/dl or more were selected. Groups each having 3 to 4 mice were used for the experiment, and the mice were grouped to make the average body weight and the average blood glucose level similar. Each compound was administered to a compound administration group by feeding a mixture with feed containing 0.03% of the compound. A separate group in which the mice were fed only with feed was a control group.

The experiment period (drug administration period) was three days. The grouping day was the 0th day. On the 3rd day, body weight was measured and blood was collected from the tail vein to measure the blood glucose level.

The blood glucose lowering efficacy was determined by the following formula.

$$\text{Blood glucose lowering efficacy} = [(\text{Control group blood glucose level} - \text{Compound administration group blood glucose level})/\text{Control group blood glucose level}] \times 100$$

The results obtained are shown in Table 6.

(Table 6)

| Examples | Blood glucose lowering efficacy (%) |
|---|---|
| 27 | 42 |
| 36 | 36 |
| 54 | 34 |
| 56 | 24 |
| 69 | 47 |
| 86 | 31 |
| 87 | 45 |
| 94 | 44 |
| 97 | 53 |
| 101 | 47 |
| 102 | 63 |
| 103 | 62 |
| 104 | 53 |
| 106 | 51 |
| 111 | 31 |
| 116 | 32 |
| 140 | 18 |
| 144 | 41 |
| 146 | 49 |
| 147 | 62 |
| 162 | 43 |
| 164 | 43 |
| 166 | 47 |
| 167 | 30 |
| 177 | 47 |
| 176 | 40 |
| 177 | 43 |
| 178 | 37 |
| 180 | 60 |
| 181 | 45 |
| 182 | 50 |
| 183 | 38 |
| 184 | 37 |
| 185 | 27 |
| 186 | 53 |
| 187 | 44 |
| 188 | 57 |
| 189 | 47 |
| 190 | 58 |
| 192 | 42 |
| 193 | 50 |
| 195 | 36 |

As is clear from Table 6, the compound of the present invention has an excellent blood glucose lowering effect. Accordingly, the compound of the present invention is assumed to be useful as a therapeutic agent for diabetes (in particular, a therapeutic agent for type II diabetes).

Preparation Example 1: Capsules

| | |
|---|---|
| Compound of Example 27 or 185 | 50 mg |
| Lactose | 128 mg |
| Corn starch | 70 mg |
| Magnesium stearate | 2 mg 250 mg |

The above-formulated powder is mixed and allowed to pass through a 60-mesh sieve. Then, the powder is put in 250 mg gelatin capsules No. 3 to prepare capsules.

Preparation Example 2: Tablets

| | |
|---|---|
| Compound of Example 27 or 185 | 50 mg |
| Lactose | 126 mg |
| Corn starch | 23 mg |
| Magnesium stearate | 1 mg |
| | 200 mg |

The above-formulated powder is mixed, wet granulated using corn starch paste, dried, and then tableted using a tableting machine to prepare tablets each having a weight of 200 mg. The tablets may be sugar-coated as necessary.

Industrial Applicability

The cercosporamide derivative having the general formula (I) according to the present invention, a pharmacologically acceptable salt thereof or an ester thereof has an excellent hypoglycemic effect and is useful as a therapeutic and/or prophylactic agent for diabetes (in particular, type II diabetes).

BRIEF DESCRIPTION OF THE DRAWING

Figure 1 shows a chromatogram obtained during HPLC analysis of cercosporamide obtained from a fermented broth of Lachnum fuscescens SANK 19096.

## Claims

**1.** A cercosporamide derivative having the general formula (I):

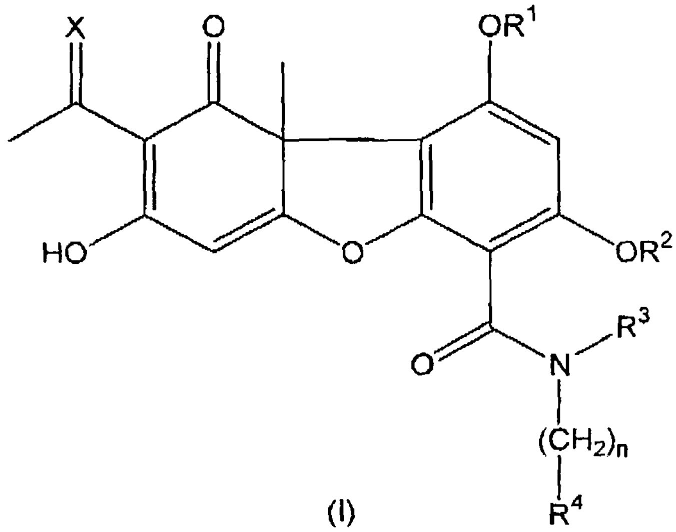

(I)

[wherein

X represents an oxygen atom, a group represented by the formula =N-O-$R^5$ or a group represented by the formula =N-$R^5$,

$R^1$ represents a hydrogen atom or a $C_1$-$C_6$ alkyl group,

$R^2$ represents a hydrogen atoms, a $C_1$-$C_6$ alkyl group or a $C_1$-$C_6$ halogenated alkyl group,

$R^3$ represents a hydrogen atom or a $C_1$-$C_6$ alkyl group,

$R^4$ represents a $C_6$-$C_{10}$ aryl group which may be substituted with one to five group(s) independently selected from Substituent Group a, a heterocyclic group which may be substituted with one to five group(s) independently selected from Substituent Group b or a phenyl group fused with $C_3$-$C_6$ cycloalkane which may be substituted with one to three group(s) independently selected from Substituent Group b,

$R^5$ represents a $C_1$-$C_6$ alkyl group, a $C_1$-$C_6$ halogenated alkyl group, a $C_2$-$C_6$ alkenyl group, a $C_2$-$C_6$ alkynyl group or a $C_1$-$C_6$ alkyl group substituted with one group selected from Substituent Group c,

n represents 1, 2 or 3,

Substituent Group a represents the group consisting of a halogen atom, a $C_1$-$C_6$ alkyl group, a $C_1$-$C_6$ halogenated alkyl group, a $C_2$-$C_6$ alkenyl group, a $C_2$-$C_6$ alkynyl group, a $C_1$-$C_6$ alkoxy group, a $C_1$-$C_6$ halogenated alkoxy group, a $C_2$-$C_6$ alkenyloxy group, a $C_2$-$C_6$ alkynyloxy group, a ($C_1$-$C_6$ alkoxy)-($C_1$-$C_6$ alkyl) group, a ($C_2$-$C_6$ alkenyloxy)-($C_1$-$C_6$ alkyl) group, a ($C_2$-$C_6$ alkynyloxy)-($C_1$-$C_6$ alkyl) goup, a $C_1$-$C_6$ alkylthio group, a hydroxy group, a carboxyl group, a $C_2$-$C_7$ alkylcarbonyl group, a $C_4$-$C_7$ cycloalkylcarbonyl group, a $C_2$-$C_7$ alkylcarbonyloxy group, a $C_2$-$C_7$ alkoxycarbonyl group, an amino group, a mono-$C_1$-$C_6$ alkylamino group, a di-($C_1$-$C_6$ alkyl)amino group, a mono-($C_1$-$C_6$ alkyl)aminocarbonyl group, a di-($C_1$-$C_6$ alkyl)aminocarbonyl group, a mono-($C_1$-$C_6$ alkyl)aminocarbonyloxy group, a di-($C_1$-$C_6$ alkyl)aminocarbonyloxy group, a mono-$C_2$-$C_7$ alkylcarbonylamino group, a mono-$C_1$-$C_6$ alkylsulfonylamino group, a cyano group, a nitro group, a carbamoyl group, a phenyloxy group which may be substituted with one to five group(s) independently selected from Substituent Group d, a phenylthio group which may be substituted with one to five group(s) independently selected from Substituent Group d, a phenyloxymethyl group which may be substituted with one to five group(s) independently selected from Substituent Group d, a phenylmethyloxy group which may be substituted with one to five group(s) independently selected from Substituent Group d, a phenylthiomethyl group which may be substituted with one to five group(s) independently selected from Substituent Group d, a phenyloxycarbonyl

group which may be substituted with one to five group(s) independently selected from Substituent Group d, a phenylcarbonylamino group which may be substituted with one to five group(s) independently selected from Substituent Group d, a phenylsulfonylamino group which may be substituted with one to five group(s) independently selected from Substituent Group d, a phenylaminocarbonyloxy group which may be substituted with one to five group(s) independently selected from Substituent Group d, a phenyloxycarbonylamino group which may be substituted with one to five group(s) independently selected from Substituent Group d, a phenylaminocarbonylamino group which may be substituted with one to five group(s) independently selected from Substituent Group d, a $C_1$-$C_6$ alkyl group substituted with one carboxyl group, a $C_1$-$C_6$ alkyl group substituted with one $C_2$-$C_7$ alkoxycarbonyl group, a $C_1$-$C_6$ alkoxy group substituted with one $C_2$-$C_7$ alkoxycarbonyl group and a $C_1$-$C_6$ alkoxy group substituted with one di-($C_1$-$C_6$ alkyl)aminocarbonyl group,

Substituent Group b represents the group consisting of a halogen atom, a $C_1$-$C_6$ alkyl group, a $C_1$-$C_6$ halogenated

alkyl group, a $C_1$-$C_6$ alkoxy group, a $C_2$-$C_6$ alkynyloxy group, a hydroxy group and a carboxyl group,
Substituent Group c represents the group consisting of a $C_3$-$C_6$ cycloalkyl group, a phenyl group, a hydroxy group, a carboxyl group, a $C_2$-$C_7$ alkylcarbonyl group and a $C_2$-$C_7$ alkoxycarbonyl group, and
Substituent Group d represents the group consisting of a halogen atom, a $C_1$-$C_6$ alkyl group, a $C_1$-$C_6$ halogenated alkyl group and a $C_1$-$C_6$ alkoxy group],
a pharmacologically acceptable salt thereof or an ester thereof.

2. The cercosporamide derivative, a pharmacologically acceptable salt thereof or an ester thereof according to claim 1, wherein the general formula (I) is a general formula (Ia):

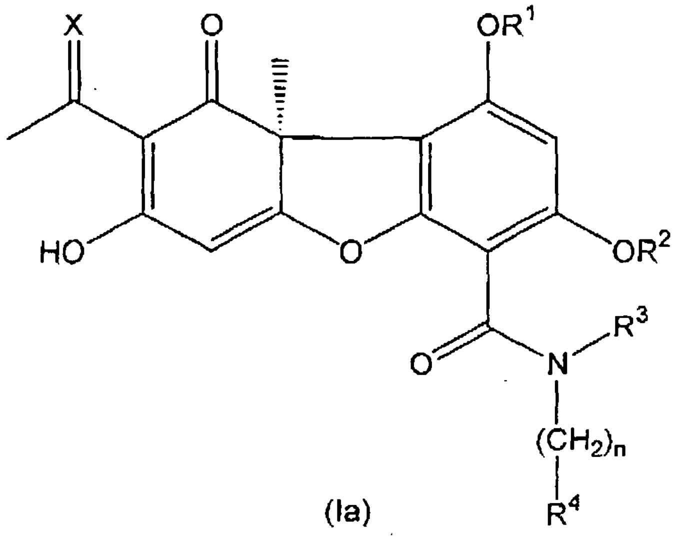

(Ia)

3. The cercosporamide derivative, a pharmacologically acceptable salt thereof or an ester thereof according to claim 1 or 2, wherein X is an oxygen atom or a group represented by the formula =N-O-$R^5$.

4. The cercosporamide derivative, a pharmacologically acceptable salt thereof or an ester thereof according to any one of claims 1 to 3, wherein $R^5$ is a methyl group, an ethyl group, a propyl group, a 2-propenyl group or a cyclopropylmethyl group.

5. The cercosporamide derivative, a pharmacologically acceptable salt thereof or an ester thereof according to claim 1 or 2, wherein X is an oxygen atom.

6. The cercosporamide derivative, a pharmacologically acceptable salt thereof or an ester thereof according to any one of claims 1 to 5, wherein $R^1$ is a hydrogen atom.

7. The cercosporamide derivative, a pharmacologically acceptable salt thereof or an ester thereof according to any one of claims 1 to 6, wherein $R^2$ is a methyl group.

8. The cercosporamide derivative, a pharmacologically acceptable salt thereof or an ester thereof according to any one of claims 1 to 7, wherein $R^3$ is a hydrogen atom.

9. The cercosporamide derivative, a pharmacologically acceptable salt thereof or an ester thereof according to any one of claims 1 to 8, wherein $R^4$ is a phenyl group substituted with one to five group(s) independently selected from Substituent Group a or a 1-naphthyl group which may be substituted with one to three group(s) independently selected from Substituent Group a.

10. The cercosporamide derivative, a pharmacologically acceptable salt thereof or an ester thereof according to any one of claims 1 to 8, wherein $R^4$ is a phenyl group substituted with one to five group(s) independently selected from Substituent Group e or a 1-naphtbyl group which may be substituted with one to three group(s) independently selected from Substituent Group e, and

Substituent Group e represents the group consisting of a halogen atom; a $C_1$-$C_6$ alkyl group; a $C_1$-$C_6$ halogenated alkyl group; a $C_2$-$C_6$ alkenyl group; a $C_1$-$C_6$ alkoxy group; a $C_2$-$C_6$ alkynyloxy group; a ($C_1$-$C_6$ alkoxy)-($C_1$-$C_6$ alkyl) group; ($C_2$-$C_6$ alkynyloxy)-($C_1$-$C_6$ alkyl) group; a hydroxy group; a carboxyl group; a $C_4$-$C_7$ cycloalkylcarbonyl group; a $C_2$-$C_7$ alkoxycarbonyl group; a di-($C_1$-$C_6$ alkyl)aminocarbonyl group; a di-($C_1$-$C_6$ alkyl)aminocarbonyloxy group; a mono-$C_1$-$C_6$ alkylsulfonylamino group; a cyano group; a nitro group; a phenyloxy group, a phenylthio group, a phenyloxymethyl group, a phenylmethyloxy group, a phenyloxycarbonyl group, a phenylcarbonylamino group or a phenylsulfonylamino group which may be substituted with one to three group(s) independently selected from a fluorine atom, a chlorine atom, a methyl group and a trifluoromethyl group; a $C_1$-$C_6$ alkyl group substituted with one carboxyl group; and a $C_1$-$C_6$ alkyl group substituted with one $C_2$-$C_7$ alkoxycarbonyl group.

**11.** The cercosporamide derivative, a pharmacologically acceptable salt thereof or an ester thereof according to any one of claims 1 to 8, wherein $R^4$ is a 1-naphthyl group substituted with one to five group(s) independently selected from a fluorine atom, a chlorine atom, a bromine atom, a methyl group and an ethyl group.

**12.** The cercosporamide derivative, a pharmacologically acceptable salt thereof or an ester thereof according to any one of claims 1 to 8, wherein $R^4$ is a 1-naphthyl group substituted at the 2- and/or 3-position(s) with one or two group(s) independently selected from a methyl group and an ethyl group and substituted at the 4-, 5-, 6-, 7- and/or 8-position(s) with one to three group(s) independently selected from a fluorine atom and a chlorine atom.

**13.** The cercosporamide derivative, a pharmacologically acceptable salt thereof or an ester thereof according to any one of claims 1 to 8, wherein $R^4$ is a 1-naphthyl group substituted at the 2-position with a methyl group or an ethyl group and substituted at the 4-, 5-, 6-, 7- and/or 8-position(s) with one to three group(s) independently selected from a fluorine atom and a chlorine atom.

**14.** The cercosporamide derivative, a pharmacologically acceptable salt thereof or an ester thereof according to any one of claims 1 to 13, wherein n is 1.

**15.** The cercosporamide derivative, a pharmacologically acceptable salt thereof or an ester thereof according to claim 1, wherein the compound having the general formula (I) is
(9aS)-8-acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide,
(9aS)-8-acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichloro-5-methylphenyl)sulfonyl]amino}-2,6-dimethylbenzyl)-1,7-dihydroxy-3-methoxy-9a-methyl-9-oxo-9,9a-dihydrodibenzo[b,d]furan4-carboxamide,
(9aS)-8-acetyl-1,7-dihydroxy-3-methoxy-9a-methyl-9-oxo-N-(2,3,5,6-tetramethylbenzyl)-9,9a-dihydrodibenzo[b,d]furan-4-carboxamide,
(9aS)-8-acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide,
(9aS)-8-acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide,
(9aS)-8-acetyl-1,7-dihydroxy-3-methoxy-9a-methyl-N-(1-naphthylmethyl)-9-oxo-9,9a-dihydrodibenzo[b,d]furan-4-carboxamide,
(9aS)-8-acetyl-N-[(4-fluoro-1-naphthyl)methyl]-1,7-dihydroxy-3-methoxy-9a-methyl-9-oxo-9,9a-dihydrodibenzo[b,d]furan-carboxamide,
(9aS)-8-acetyl-1,7-dihydroxy-3-methoxy-9a-methyl-N-[(2-methyl-1-naphthyl)methyl]-9-oxo-9,9a-dihydrodibenzo[b,d]furan-4-carboxamide,
(9aS)-8-acetyl-N-[(2-ethyl-1-naphthyl)methyl]-1,7-dihydroxy-3-methoxy-9a-methyl-9-oxo-9,9a-dibydrodibenzo[b,d]furan-4-carboxamide,
(9aS)-8-acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-chorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3-diznethyl-1-naphthyl)methyl]-9-oxo-9,9a-dihydrodibenzo[b,d]furan-4-carboxamide,
(9aS)-8-acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide,
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide,
(9aS)-8-acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide,
(9aS)-1,7-dihydroxy-3-methoxy-8-[(1E)-N-methoxyethaneimidoyl]-9a-methyl-N-[(2-methyl-1-naphthyl)methyl]-9-oxo-9,9a-dihydrodibenzo[b,d]furan-4-carboxamide or
(9aS)-8-[(1E)-N-(allyloxy)ethaneimidoyl]-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide.

**16.** The cercosporamide derivative, a pharmacologically acceptable salt thereof or an ester thereof according to claim 1, wherein the compound having the general formula (I) is
(9aS)-8-acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide or
(9aS)-8-acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide.

**17.** The cercosporamide derivative or a pharmacologically acceptable salt thereof according to claim 1, wherein the compound having the general formula (I) is
(9aS)-8-acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamide,
(9aS)-8-acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide,
(9aS)-8-acetyl-N-(4-{[(2,4-dichloro-5-methylphenyl)sulfonyl]amino}-2,6-dimethylbenzyl)-1,7-dihydroxy-37-methoxy-9a-methyl-9-oxo-9,9a-dihydrodibenzo[b,d]furan-4-carboxamide,
(9aS)-8-acetyl-1,7-dihydroxy-3-methoxy-9a-methyl-9-oxo-N-(2,3,5,6-tetramethylbenzyl)-9,9a-dihydrodibenzo[b,d]furan-4-carboxamide,
(9aS)-8-acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamide,
(9aS)-8-acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-9-oxo-N-[2,3,6-trimethyl-4-(2-pentynyloxy)benzyl]-9,9a-dihydrodibenzo[b,d]furan-4-carboxamide,
(9aS)-8-acetyl-1,7-dihydroxy-3-methoxy-9a-methyl-N-(1-naphthylmethyl)-9-oxo-9,9a-dihydrodibenzo[b,d]furan-4-carboxamide,
(9aS)-8-acetyl-N-[(4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2-methyl-1-naphthyl)methyl]-9-oxo-9,9a-dihydrodibenzo[b,d]furan-4-carboxamide,
(9aS)-8-acetyl-N-[(2-ethyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-{[4-(4-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(3-chlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-1-N-{[4-(2,4-dichlorophenoxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-{[4-(benzyloxy)-1-naphthyl]methyl}-1,7-dihydroxy-3-methoxy-9a-methyl-9-oxo-9,9a-dihydrodibenzo[b,d]furan-4-carboxamide,
(9aS)-8-acetyl-N-({4-[(4-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-fluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(3-chlorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-N-({4-[(2,4-difluorobenzyl)oxy]-1-naphthyl}methyl)-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamide,
(9aS)-8-acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamide,
(9aS)-8-acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamide,
(9aS)-8-acetyl-3-(difluoromethoxy)-1,7-dihydroxy-9a-methyl-N-[(2-methyl-1-naphthyl)methyl]-9-oxo-9,9a-dihydrodibenzo[b,d]furan-4-carboxamide,
(9aS)-1,7-dihydroxy-3-methoxy-8-[(1E)-N-methoxyethaneimidoyl]-9a-methyl-N-[(2-methyl-1-naphthyl)methyl]-9-oxo-9,9a-dihydrodibenzo[b,d]furan-4-carboxamide or
(9aS)-8-[(1E)-N-(allyloxy)ethaneimidoyl]-N-(4-{[(2,4-dichlorophenyl)sulfonyl]amino}-2,6-dimethylbenzyl)-1,7-dihydroxy-3-methoxy-9a-methyl-9-oxo-9,9a-dihydrodibenzo[b,d]furan-4-carboxamide.

**18.** The cercosporamide derivative or a pharmacologically acceptable salt thereof according to claim 1, wherein the compound having the general formula (I) is
(9aS)-8-acetyl-N-[(4-fluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-fluoro-2-methyl-1-naphthyl)methyl]-1,7-dihydroxy-3-methoxy-9a-methyl-9-oxo-9,9a-dihydrodibenzo[b,d]furan-4-carboxamide,

(9aS)-8-acetyl-N-[(2-ethyl-4-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-8-fluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(7-chloro-2-ethyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(5,7-difluoro-2-methyl-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-5,7-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-6,7-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-6,8-difluoro-1-naphthyl)methyl]-1,7-dihydroxy-3-methoxy-9a-methyl-9-oxo-9,9a-dihydrodibenzo[b,d]furan-4-carboxamide,
(9aS)-8-acetyl-N-[(2-ethyl-7,8-difluoro-1-naphthyl)methyl]-1,7-dihydroxy-3-methoxy-9a-methyl-9-oxo-9,9a-dihydrodibenzo[b,d]furan-4-carboxamide or
(9aS)-8-acetyl-1,7-dihydroxy-3-methoxy-9a-methyl-9-oxo-N-[(5,6,7-trifluoro-2-methyl-1-naphthyl)methyl]-9,9a-dihydrodibenzo[b,d]furan-4-carboxamide.

**19.** A pharmaceutical composition comprising the cercosporamide derivative a pharmacologically acceptable salt thereof or an ester thereof according to any one of claims 1 to 18 as an active ingredient.

**20.** The pharmaceutical composition according to claim 19 for lowering blood glucose, comprising the cercosporamide derivative, a pharmacologically acceptable salt thereof or an ester thereof according to any one of claims 1 to 18 as an active ingredient.

**21.** The pharmaceutical composition according to claim 19 for treatment and/or prevention of diabetes, comprising the cercosporamide derivative, a pharmacologically acceptable salt thereof or an ester thereof according to any one of claims 1 to 18 as an active ingredient.

**22.** Use of the cercosporamide derivative, a pharmacologically acceptable salt thereof or an ester thereof according to any one of claims 1 to 18 for the manufacture of a pharmaceutical composition.

**23.** The use according to claim 22, wherein the pharmaceutical composition is a composition for lowering blood glucose.

**24.** The use according to claim 22, wherein the pharmaceutical composition is a composition for treatment and/or prevention of diabetes.

## Patentansprüche

**1.** Cercosporamidderivat, das die allgemeine Formel (I):

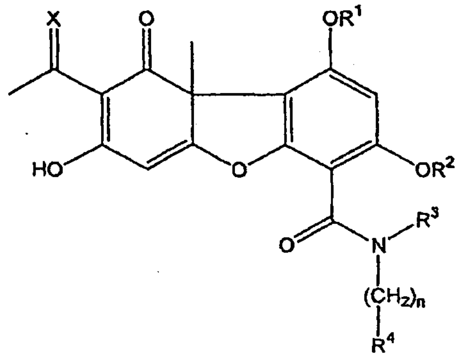

I

aufweist, [wobei

X ein Sauerstoffatom, eine Gruppe, die durch die Formel =N-O-$R^5$ dargestellt ist oder eine Gruppe, die durch die Formel =N-$R^5$ dargestellt ist, darstellt,

$R^1$ ein Wasserstoffatom oder eine $C_1$-$C_6$-Alkylgruppe darstellt;

$R^2$ ein Wasserstoffatom, eine $C_1$-$C_6$-Alkylgruppe oder eine halogenierte$C_1$-$C_6$-Alkylgruppe darstellt;

$R^3$ ein Wasserstoffatom oder eine $C_1$-$C_6$-Alkylgruppe darstellt;

$R^4$ eine $C_6$-$C_{10}$-Arylgruppe, die mit einer bis fünf Gruppe(n) substituiert sein kann, die unabhängig ausgewählt sind aus der Substituentengruppe a, eine heterocyclische Gruppe, die mit einer bis fünf Gruppe(n) substituiert sein kann, die unabhängig ausgewählt sind aus der Substituentengruppe b oder eine Phenylgruppe darstellt, die mit $C_3$-$C_6$-Cycloalkan anelliert ist, das mit einer bis drei Gruppe(n) substituiert sein kann, die unabhängig ausgewählt sind aus der Substituentengruppe b,

$R^5$ eine $C_1$-$C_6$-Alkylgruppe, eine halogenierte $C_1$-$C_6$-Alkylgruppe, eine $C_2$-$C_6$-Alkenylgruppe, eine $C_2$-$C_6$-Alkynylgruppe oder eine $C_1$-$C_6$-Alkylgruppe darstellt, die mit einer Gruppe substituiert ist, die aus der Substituentengruppe c ausgewählt ist,

n 1, 2 oder 3 darstellt,

die Substituentengruppe a die Gruppe darstellt bestehend aus einem Halogenatom, einer $C_1$-$C_6$-Alkylgruppe, einer halogenierten $C_1$-$C_6$-Alkylgruppe, einer $C_2$-$C_6$-Alkenylgruppe, einer $C_2$-$C_6$-Alkynylgruppe, einer $C_1$-$C_6$-Alkoxygruppe, einer halogenierten $C_1$-$C_6$-Alkoxygruppe, einer $C_2$-$C_6$-Alkenyloxygruppe, einer $C_2$-$C_6$-Alkynyloxygruppe, einer ($C_1$-$C_6$-Alkoxy)-($C_1$-$C_6$-Alkyl)-gruppe, einer ($C_2$-$C_6$-Alkenyloxy)-($C_1$-$C_6$-Alkyl)-gruppe, einer ($C_2$-$C_6$-Alkynyloxy)-($C_1$-$C_6$-Alkyl)-gruppe, einer $C_1$-$C_6$-Alkylthiogruppe, einer Hydroxygruppe, einer Carboxylgruppe, einer $C_2$-$C_7$-Alkylcarbonylgruppe, einer $C_4$-$C_7$-Cycloalkylcarbonylgruppe, einer $C_2$-$C_7$-Alkylcarbonyloxygruppe, einer $C_2$-$C_7$-Alkoxycarbonylgruppe, einer Aminogruppe, einer Mono-$C_1$-$C_6$-Alkylaminogruppe, einer Di-($C_1$-$C_6$-Alkyl)aminogruppe, Mono(-$C_1$-$C_6$-Alkyl)aminocarbonylgruppe, einer Di-($C_1$-$C_6$-Alkyl)aminocarbonylgruppe, einer Mono-($C_1$-$C_6$-Alkyl)aminocarbonyloxygruppe, einer Di-($C_1$-$C_6$-Alkyl)aminocarbonyloxygruppe, einer Mono-$C_2$-$C_7$-Alkylcarbonylaminogruppe, einer Mono-$C_1$-$C_6$-Alkylsulfonylaminogruppe, einer Cyanogruppe, einer Nitrogruppe, einer Carbamoylgruppe, einer Phenyloxygruppe, die mit einer bis fünf Gruppe(n) substituiert sein kann, die unabhängig ausgewählt sind von der Substituentengruppe d, einer Phenylthiogruppe, die mit einer bis fünf Gruppe(n) substituiert sein kann, die unabhängig ausgewählt sind von der Substituentengruppe d, einer Phenyloxymethylgruppe, die mit einer bis fünf Gruppe(n) substituiert sein kann, die unabhängig ausgewählt sind von der Substituentengruppe d, einer Phenylmethyloxygruppe, die mit einer bis fünf Gruppe(n) substituiert sein kann, die unabhängig ausgewählt sind von der Substituentengruppe d, einer Phenylthiomethylgruppe, die mit einer bis fünf Gruppe(n) substituiert sein kann, die unabhängig ausgewählt sind von der Substituentengruppe d, einer Phenyloxycarbonylgruppe, die mit einer bis fünf Gruppe(n) substituiert sein kann, die unabhängig ausgewählt sind von der Substituentengruppe d, einer Phenylcarbonylaminogruppe, die mit einer bis fünf Gruppe(n) substituiert sein kann, die unabhängig ausgewählt sind von der Substituentengruppe d, einer Phenylsulfonylaminogruppe, die mit einer bis fünf Gruppe(n) substituiert sein kann, die unabhängig ausgewählt sind von der Substituentengruppe d, einer Phenylaminocarbonyloxygruppe, die mit einer bis fünf Gruppe(n) substituiert sein kann, die unabhängig ausgewählt sind von der Substituentengruppe d, einer Phenyloxycarbonylaminogruppe, die mit einer bis fünf Gruppe(n) substituiert sein kann, die unabhängig ausgewählt sind von der Substituentengruppe d, einer Phenylaminocarbonylaminogruppe, die mit einer bis fünf Gruppe (n) substituiert sein kann, die unabhängig ausgewählt sind von der Substituentengruppe d, einer $C_1$-$C_6$-Alkylgruppe, die mit einer Carboxylgruppe substituiert ist, einer $C_1$-$C_6$-Alkylgruppe, die mit einer $C_2$-$C_7$-Alkoxycarbonylgruppe substituiert ist, einer $C_1$-$C_6$-Alkoxygruppe, die mit einer $C_2$-$C_7$-Alkoxycarbonylgruppe substituiert ist und einer $C_1$-$C_6$-Alkoxygruppe, die mit einer Di-($C_1$-$C_6$-Alkyl)aminogruppe substituiert ist,

die Substituentengruppe b die Gruppe darstellt, die aus einem Halogenatom, einer $C_1$-$C_6$-Alkylgruppe, einer halogenierten $C_1$-$C_6$-Alkylgruppe, einer $C_1$-$C_6$-Alkoxygruppe, einer $C_2$-$C_6$-Alkynyloxygruppe, einer Hydroxygruppe und einer Carboxylgruppe besteht,
die Substituentengruppe c eine Gruppe darstellt, die aus einer $C_3$-$C_6$-Cycloalkylgruppe, einer Phenylgruppe, einer Hydroxygruppe, einer Carboxylgruppe, einer $C_2$-$C_7$-Alkylcarbonylgruppe und einer $C_2$-$C_7$-Alkoxycarbonylgruppe besteht und
die Substituentengruppe d eine Gruppe darstellt, die aus einem Halogenatom, einer $C_1$-$C_6$-Alkylgruppe, einer halogenierten $C_1$-$C_6$-Alkylgruppe, einer $C_1$-$C_6$-Alkoxygruppe besteht],
pharmakologisch akzeptables Salz davon oder Ester davon.

**2.** Cercosporamidderivat, pharmakologisch akzeptables Salz davon oder Ester davon nach Anspruch 1, wobei die allgemeine Formel (I) eine allgemeine Formel (Ia):

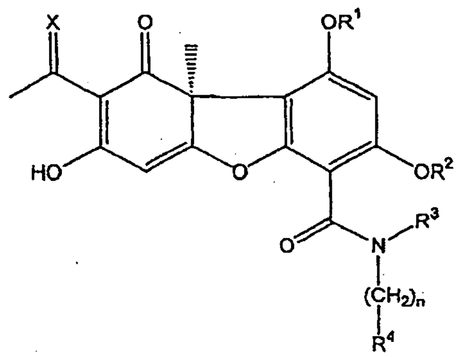

(Ia)

ist.

**3.** Cercosporamidderivat, pharmakologisch akzeptables Salz davon oder Ester davon nach Anspruch 1 oder 2, wobei X ein Sauerstoffatom oder eine Gruppe ist, die durch die Formel =N-O-$R^5$ dargestellt ist.

**4.** Cercosporamidderivat, pharmakologisch akzeptables Salz davon oder Ester davon nach einem der Ansprüche 1 bis 3, wobei $R^5$ eine Methylgruppe, eine Ethylgruppe, eine Propylgruppe, eine 2-Propenylgruppe oder eine Cyclopropylmethylgruppe ist.

**5.** Cercosporamidderivat, pharmakologisch akzeptables Salz davon oder Ester davon nach Anspruch 1 oder 2, wobei X ein Sauerstoffatom ist.

**6.** Cercosporamidderivat, pharmakologisch akzeptables Salz davon oder Ester davon nach einem der Ansprüche 1 bis 5, wobei $R^1$ ein Wasserstoffatom ist.

**7.** Cercosporamidderivat, pharmakologisch akzeptables Salz davon oder Ester davon nach einem der Ansprüche 1 bis 6, wobei $R^2$ eine Methylgruppe ist.

**8.** Cercosporamidderivat, pharmakologisch akzeptables Salz davon oder Ester davon nach einem der Ansprüche 1 bis 7, wobei $R^3$ ein Wasserstoffatom ist.

**9.** Cercosporamidderivat, pharmakologisch akzeptables Salz davon oder Ester davon nach einem der Ansprüche 1 bis 8, wobei $R^4$ eine Phenylgruppe, die mit einer bis fünf Gruppe(n) substituiert ist, die unabhängig aus der Substituentengruppe a ausgewählt sind, oder eine 1-Naphthylgruppe ist, die mit einer bis drei Gruppe(n) substituiert sein kann, die unabhängig ausgewählt sind aus der Substituentengruppe a.

**10.** Cercosporamidderivat, pharmakologisch akzeptables Salz davon oder Ester davon nach einem der Ansprüche 1 bis 8, wobei $R^4$ eine Phenylgruppe, die mit einer bis fünf Gruppe(n) substituiert ist, die unabhängig aus der Substituentengruppe e ausgewählt sind, oder eine 1-Naphthylgruppe ist, die mit einer bis drei Gruppe(n) substituiert sein kann, die unabhängig ausgewählt sind aus der Substituentengruppe e und

die Substituentengruppe e die Gruppe darstellt bestehend aus einem Halogenatom; einer $C_1$-$C_6$-Alkylgruppe; einer halogenierten $C_1$-$C_6$-Alkylgruppe; einer $C_2$-$C_6$-Alkenylgruppe; einer $C_1$-$C_6$-Alkoxygruppe; einer $C_2$-$C_6$-Alkynyloxygruppe; einer ($C_1$-$C_6$-Alkoxy)-($C_1$-$C_6$-Alkyl)-gruppe; einer ($C_2$-$C_6$-Alkynyloxy)-($C_1$-$C_6$-Alkyl)-gruppe; einer Hydroxygruppe; einer Carboxylgruppe; einer $C_4$-$C_7$-Cycloalkylcarbonylgruppe; einer $C_2$-$C_7$-Alkoxycarbonylgruppe; einer Di-($C_1$-$C_6$-Alkyl)aminocarbonylgruppe; einer Di-($C_1$-$C_6$-Alkyl)aminocarbonyloxygruppe; einer Mono-$C_1$-$C_6$-Alkylsulfonylaminogruppe; einer Cyanogruppe; einer Nitrogruppe; einer Phenyloxygruppe, einer Phenylthiogruppe, einer Phenyloxymethylgruppe, einer Phenylmethyloxygruppe, einer Phenyloxycarbonylgruppe, einer Phenylcarbonylaminogruppe oder einer Phenylsulfonylaminogruppe, die mit einer bis drei Gruppe(n) substituiert sein kann, die unabhängig ausgewählt sind von einem Fluoratom, einem Chloratom, einer Methylgruppe und einer Trifluormethylgruppe; einer $C_1$-$C_6$-Alkylgruppe, die mit einer Carboxylgruppe substituiert ist; und einer $C_1$-$C_6$-Alkylgruppe, die mit einer $C_2$-$C_7$-Alkoxycarbonylgruppe substituiert ist.

**11.** Cercosporamidderivat, pharmakologisch akzeptables Salz davon oder Ester davon nach einem der Ansprüche 1 bis 8, wobei $R^4$ eine 1-Naphthylgruppe ist, die mit einer bis fünf Gruppe(n) substituiert ist, die unter einem Fluoratom, einem Chloratom, einem Bromatom, einer Methylgruppe und einer Ethylgruppe ausgewählt sind.

**12.** Cercosporamidderivat, pharmakologisch akzeptables Salz davon oder Ester davon nach einem der Ansprüche 1 bis 8, wobei $R^4$ eine 1-Naphthylgruppe ist, die an den Positionen 2 und/oder 3 mit einer oder zwei Gruppe(n) substituiert ist, die unabhängig ausgewählt sind unter einer Methylgruppe und einer Ethylgruppe und die an den Positionen 4, 5, 6, 7 und/oder 8 mit einer bis drei Gruppe(n) substituiert ist, die unabhängig ausgewählt sind unter einem Fluoratom und einem Chloratom.

**13.** Cercosporamidderivat, pharmakologisch akzeptables Salz davon oder Ester davon nach einem der Ansprüche 1 bis 8, wobei $R^4$ eine 1-Naphthylgruppe ist, die an Position 2 mit einer Methylgruppe oder einer Ethylgruppe substituiert und an den Positionen 4, 5, 6, 7 und/oder 8 mit einer bis drei Gruppe(n) substituiert ist, die unabhängig ausgewählt sind unter einem Fluoratom und einem Chloratom.

**14.** Cercosporamidderivat, pharmakologisch akzeptables Salz davon oder Ester davon nach einem der Ansprüche 1 bis 13, wobei n 1 beträgt.

**15.** Cercosporamidderivat, pharmakologisch akzeptables Salz davon oder Ester davon nach Anspruch 1, wobei die Verbindung, die die allgemeine Formel (I) aufweist, Folgendes ist
(9aS)-8-Acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamid,
(9aS)-8-Acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamid,
(9aS)-8-Acetyl-N-(4-{[(2,4-dichlorphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamid,
(9aS)-8-Acetyl-N-(4-{[(2,4-dichlor-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-9-oxo-N-(2,3,5,6-tetramethylbenzyl)-9,9a-dihydrodibenzo[b,d]furan-4-carboxamid,
(9aS)-8-Acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamid,
(9aS)-8-Acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-9-oxo-N-(2,3,6-trimethyl-4-(2-pentynyloxy)benzyl)-9,9a-dihydrodibenzo[b,d]furan-4-carboxamid,
(9aS)-8-Acetyl-1,7-dihydroxy-3-methoxy-9a-methyl-N-(1-naphthylmethyl)-9-oxo-9,9a-dihydrodibenzo[b,d]furan-4-carboxamid,
(9aS)-8-Acetyl-N-[(4-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamid,
(9aS)-8-Acetyl-N-[(2-ethyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-{[4-(4-chlorphenoxy)-1-naphthyl)methyl}-1,7-dihydroxy-3-methoxy-9a-methyl-9-oxo-9,9a-dihydrodibenzo[b,d]furan-4-carboxamid,

(9aS)-8-Acetyl-N-{[4-(3-chlorphenoxy)-1-naphthyl]methyl}-1,7-dihydroxy-3-methoxy-9a-methyl-9-oxo-9,9a-dihydrodibenzo[b,d]furan-4-carboxamid,
(9aS)-8-Acetyl-N-{[4-(2,4-dichlorphenoxy)-1-naphthyl)methyl}-1,7-dihydroxy-3-methoxy-9a-methyl-9-oxo-9,9a-dihydrodibenzo[b,d]furan-4-carboxamid,
(9aS)-8-Acetyl-N-{[4-(benzyloxy)-1-naphthyl)methyl}-1,7-dihydroxy-3-methoxy-9a-methyl-9-oxo-9,9a-dihydrodibenzo[b,d]furan-4-carboxamid,
(9aS)-8-Acetyl-N-({4-[(4-fluorbenzyl)oxy]-1-naphthyl})methyl}-1,7-dihydroxy-3-methoxy-9a-methyl-9-oxo-9,9a-dihydrodibenzo[b,d]furan-4-carboxamid,
(9aS)-8-Acetyl-N-({4-[(3-fluorbenzyl)oxy]-1-naphthyl})methyl}-1,7-dihydroxy-3-methoxy-9a-methyl-9-oxo-9,9a-dihydrodibenzo[b,d]furan-4-carboxamid,
(9aS)-8-Acetyl-N-({4-[(3-chlorbenzyl)oxy]-1-naphthyl})methyl)-1,7-dihydroxy-3-methoxy-9a-methyl-9-oxo-9,9a-dihydrodibenzo[b,d]furan-4-carboxamid,
(9aS)-8-Acetyl-N-({4-[(2,4-difluorbenzyl)oxy]-1-naphthyl})methyl)-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamid,
(9aS)-8-Acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamid,
(9aS)-8-Acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamid,
(9aS)-8-Acetyl-3-(difluormethoxy)-1,7-dihydroxy-9a-methyl-N-[(2-methyl-1-naphthyl)methyl]-9-oxo-9,9a-dihydrodibenzo[b,d]furan-4-carboxamid,
(9aS)-1,7-Dihydroxy-3-methoxy-8[(1E)-N-methoxyethanimidoyl]-9a-methyl-N-[(2-methyl-1-naphthyl)methyl]-9-oxo-9,9a-dihydrodibenzo[b,d]furan-4-carboxamid
oder
(9aS)-8-(1E)-N-(allyloxy(ethanimidoyl]-N-(4-{[(2,4-dichlorphenyl)sulfonyl]amino}-2,6-dimethybenzyl)-1,7-dihydroxy-3-methoxy-9a-methyl-9-oxo-9,9a-dihydrodibenzo[b,d]furan-4-carboxamid.

**16.** Cercosporamidderivat, pharmakologisch akzeptables Salz davon oder Ester davon nach Anspruch 1, wobei die Verbindung, die die allgemeine Formel (I) aufweist, Folgendes ist
(9aS)-8-Acetyl-N-[(4-fluor-2-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(7-fluor-2-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-4-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-7-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-8-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(7-chlor-2-ethyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(5,7-difluor-2-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-5,7-difluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-6,7-difluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-6,8-difluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-7,8-difluor-1-naphthyl)methyl]-1,7-dihydroxy-3-methoxy-9a-methyl-9-oxo-9,9a-dihydrodibenzo[b,d]furan-4-carboxamid oder
(9aS)-8-Acetyl-1,7-dihydroxy-3-methoxy-9a-methyl-9-oxo-N-[(5,6,7-trifluor-2-methyl-1-naphthyl)methyl]-9,9a-dihydrodibenzo[b,d]furan-4-carboxamid,

**17.** Cercosporamidderivat, pharmakologisch akzeptables Salz davon oder Ester davon nach Anspruch 1, wobei die

Verbindung, die die allgemeine Formel (I) aufweist, Folgendes ist
(9aS)-8-Acetyl-N-[4-(2-butynyloxy)-2,6-dimethylbenzyl]-1,7-dihydroxy-3-methoxy-9a-methyl-9-oxo-9,9a-dihydrodibenzo[b,d]furan-4-carboxamid,
(9aS)-8-Acetyl-N-[2,6-dimethyl-4-(2-pentynyloxy)benzyl]-1,7-dihydroxy-3-methoxy-9a-methyl-9-oxo-9,9a-dihydrodibenzo[b,d]furan-4-carboxamid,
(9aS)-8-Acetyl-N-(4-{[(2,4-dichlorphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamid,
(9aS)-8-Acetyl-N-(4-{[(2,4-dichlor-5-methylphenyl)sulfonyl]amino}-2,6-dimethylbenzyl)-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-9-oxo-N-(2,3,5,6-tetramethylbenzyl)-9,9a-dihydrodibenzo[b,d]furan-4-carboxamid,
(9aS)-8-Acetyl-1,7-dihydroxy-3-methoxy-9a-methyl-9-oxo-N-(2,3,5-trimethyl-6-propylbenzyl)-9,9a-dihydrodibenzo[b,d]furan-4-carboxamid,
(9aS)-8-Acetyl-N-[4-(2-butynyloxy)-2,3,6-trimethylbenzyl]-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-9-oxo-N-(2,3,6-trimethyl-4-(2-pentynyloxy)benzyl)-9,9a-dihydrodibenzo[b,d]furan-4-carboxamid,
(9aS)-8-Acetyl-1,7-dihydroxy-3-methoxy-9a-methyl-N-(1-naphthylmethyl)-9-oxo-9,9a-dihydrodibenzo[b,d]furan-4-carboxamid,
(9aS)-8-Acetyl-N-[(4-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-N-[(2-methyl-1-naphthyl)methyl]-9-oxo-9,9a-dihydrodibenzo[b,d]furan-4-carboxamid,
(9aS)-8-Acetyl-N-[(2-ethyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-{[4-(4-chlorphenoxy)-1-naphthyl)methyl}-1,7-dihydroxy-3-methoxy-9a-methyl-9-oxo-9,9a-dihydrodibenzo[b,d]furan-4-carboxamid,
(9aS)-8-Acetyl-N-{[4-(3-chlorphenoxy)-1-naphthyl]methyl}-1,7-dihydroxy-3-methoxy-9a-methyl-9-oxo-9,9a-dihydrodibenzo[b,d]furan-4-carboxamid,
(9aS)-8-Acetyl-N-{[4-(2,4-dichlorphenoxy)-1-naphthyl)methyl}-1,7-dihydroxy-3-methoxy-9a-methyl-9-oxo-9,9a-dihydrodibenzo[b,d]furan-4-carboxamid,
(9aS)-8-Acetyl-N-{[4-(benzyloxy)-1-naphthyl)methyl}-1,7-dihydroxy-3-methoxy-9a-methyl-9-oxo-9,9a-dihydrodibenzo[b,d]furan-4-carboxamid,
(9aS)-8-Acetyl-N-({4-[(4-fluorbenzyl)oxy]-1-naphthyl})methyl}-1,7-dihydroxy-3-methoxy-9a-methyl-9-oxo-9,9a-dihydrodibenzo[b,d]furan-4-carboxamid,
(9aS)-8-Acetyl-N-({4-[(3-fluorbenzyl)oxy]-1-naphthyl})methyl}-1,7-dihydroxy-3-methoxy-9a-methyl-9-oxo-9,9a-dihydrodibenzo[b,d]furan-4-carboxamid,
(9aS)-8-Acetyl-N-({4-[(3-chlorbenzyl)oxy]-1-naphthyl})methyl)-1,7-dihydroxy-3-methoxy-9a-methyl-9-oxo-9,9a-dihydrodibenzo[b,d]furan-4-carboxamid,
(9aS)-8-Acetyl-N-({4-[(2,4-difluorbenzyl)oxy]-1-naphthyl})methyl)-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-N-[(2,3-dimethyl-1-naphthyl)methyl]-9-oxo-9,9a-dihydrodibenzo[b,d]furan-4-carboxamid,
(9aS)-8-Acetyl-N-[(2-ethyl-3-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-1,7-dihydroxy-3-methoxy-9a-methyl-N-[(2,3,4-trimethyl-1-naphthyl)methyl]-9-oxo-9,9a-dihydrodibenzo[b,d]furan-4-carboxamid,
(9aS)-8-Acetyl-1,7-dihydroxy-3-methoxy-9a-methyl-N-[(2,4-dimethyl-1-naphthyl)methyl]-9-oxo-9,9a-dihydrodibenzo[b,d]furan-4-carboxamid,
(9aS)-8-Acetyl-3-(difluormethoxy)-1,7-dihydroxy-9a-methyl-N-[(2-methyl-1-naphthyl)methyl]-9-oxo-9,9a-dihydrodibenzo[b,d]furan-4-carboxamid,
(9aS)-1,7-Dihydroxy-3-methoxy-8[(1E)-N-methoxyethanimidoyl]-9a-methyl-N-[(2-methyl-1-naphthyl)methyl]-9-oxo-9,9a-dihydrodibenzo[b,d]furan-4-carboxamid
oder
(9aS)-8-(1E)-N-(allyloxy(ethanimidoyl]-N-(4-{[(2,4-dichlorphenyl)sulfonyl]amino}-2,6-dimethybenzyl)-1,7-dihydroxy-3-methoxy-9a-methyl-9-oxo-9,9a-dihydrodibenzo[b,d]furan-4-carboxamid.

**18.** Cercosporamidderivat oder pharmakologisch akzeptables Salz davon nach Anspruch 1, wobei die Verbindung, die

die allgemeine Formel (I) aufweist, Folgendes ist
(9aS)-8-Acetyl-N-[(4-fluor-2-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(7-fluor-2-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-4-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-7-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-8-fluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(7-chlor-2-ethyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(5,7-difluor-2-methyl-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-5,7-difluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-6,7-difluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-6,8-difluor-1-naphthyl)methyl]-1,7-dihydroxy-3-methoxy-9a-methyl-9-oxo-9,9a-dihydrodibenzo[b,d]furan-4-carboxamid,
(9aS)-8-Acetyl-N-[(2-ethyl-7,8-difluor-1-naphthyl)methyl]-1,7-dihydroxy-3-methoxy-9a-methyl-9-oxo-9,9a-dihydrodibenzo[b,d]furan-4-carboxamid oder
(9aS)-8-Acetyl-1,7-dihydroxy-3-methoxy-9a-methyl-9-oxo-N-[(5,6,7-trifluor-2-methyl-1-naphthyl)methyl]-9,9a-dihydrodibenzo[b,d]furan-4-carboxamid.

**19.** Pharmazeutische Zusammensetzung umfassend das Cercosporamidderivat, ein pharmakologisch akzeptables Salz davon oder einen Ester davon nach einem der Ansprüche 1 bis 18 als aktiven Bestandteil.

**20.** Pharmazeutische Zusammensetzung nach Anspruch 19 zum Reduzieren der Blutglukose, umfassend das Cercosporamidderivat, ein pharmakologisch akzeptables Salz davon oder einen Ester davon nach einem der Ansprüche 1 bis 18 als aktiven Bestandteil.

**21.** Pharmazeutische Zusammensetzung nach Anspruch 19 für die Behandlung und/oder Prävention von Diabetes, umfassend das Cercosporamidderivat, ein pharmakologisch akzeptables Salz davon oder einen Ester davon nach einem der Ansprüche 1 bis 18 als aktiven Bestandteil.

**22.** Verwendung des Cercosporamidderivats, eines pharmakologisch akzeptablen Salzes davon oder eines Esters davon nach einem der Ansprüche 1 bis 18 für die Herstellung einer pharmazeutischen Zusammensetzung.

**23.** Verwendung nach Anspruch 22, wobei die pharmazeutische Zusammensetzung eine Zusammensetzung zum Reduzieren der Blutglukose ist.

**24.** Verwendung nach Anspruch 22, wobei die pharmazeutische Zusammensetzung eine Zusammensetzung für die Behandlung und/oder Prävention von Diabetes ist.

## Revendications

**1.** Dérivé de cercosporamide de formule générale (I):

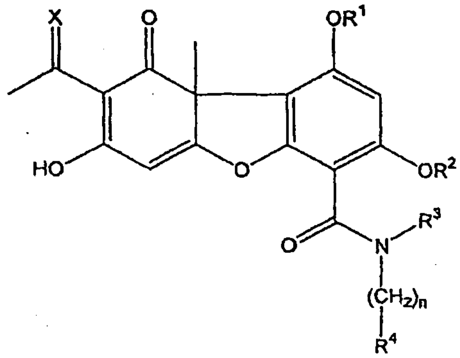

I

[dans laquelle

X représente un atome d'oxygène, un groupe représenté par la formule $=N-O-R^5$ ou un groupe représenté par la formule $=N-R^5$,

$R^1$ représente un atome d'hydrogène ou un groupe alkyle en $C_1$-$C_6$,

$R^2$ représente un atome d'hydrogène, un groupe alkyle en $C_1$-$C_6$ ou un groupe alkyle halogéné en $C_1$-$C_6$,

$R^3$ représente un atome d'hydrogène ou un groupe alkyle en $C_1$-$C_6$,

$R^4$ représente un groupe aryle en $C_6$-$C_{10}$ qui peut être substitué par 1 à 5 groupes indépendamment choisis dans le groupe de substituants a, un groupe hétérocyclique qui peut être substitué par 1 à 5 groupes indépendamment choisis dans le groupe de substituants b ou un groupe phényle condensé avec un cycloalcane en $C_3$-$C_6$ qui peut être substitué par 1 à 3 groupes indépendamment choisis dans le groupe de substituants b,

$R^5$ représente un groupe alkyle en $C_1$-$C_6$, un groupe alkyle halogéné en $C_1$-$C_6$, un groupe alcényle en $C_2$-$C_6$, un groupe alcynyle en $C_2$-$C_6$ ou un groupe alkyle en $C_1$-$C_6$ substitué par 1 groupe choisi dans le groupe de substituants c,

n représente 1, 2 ou 3,

le groupe de substituants a représente le groupe constitué d'un atome d'halogène, d'un groupe alkyle en $C_1$-$C_6$, d'un groupe alkyle halogéné en $C_1$-$C_6$, d'un groupe alcényle en $C_2$-$C_6$, d'un groupe alcynyle en $C_2$-$C_6$, d'un groupe alcoxy en $C_1$-$C_6$, d'un groupe alcoxy halogéné en $C_1$-$C_6$, d'un groupe alcényloxy en $C_2$-$C_6$, d'un groupe alcynyloxy en $C_2$-$C_6$, d'un groupe (alcoxy en $C_1$-$C_6$)-(alkyle en $C_1$-$C_6$), d'un groupe (alcényloxy en $C_2$-$C_6$)-(alkyle en $C_1$-$C_6$), d'un groupe (alcynyloxy en $C_2$-$C_6$)-(alkyle en $C_1$-$C_6$), d'un groupe alkylthio en $C_1$-$C_6$, d'un groupe hydroxy, d'un groupe carboxy, d'un groupe alkylcarbonyle en $C_2$-$C_7$, d'un groupe cycloalkylcarbonyle en $C_4$-$C_7$, d'un groupe alkylcarbonyloxy en $C_2$-$C_7$, d'un groupe alcoxycarbonyle en $C_2$-$C_7$, d'un groupe amino, d'un groupe mono(alkyl en $C_1$-$C_6$)amino, d'un groupe di(alkyl en $C_1$-$C_6$)amino, d'un groupe mono(alkyl en $C_1$-$C_6$)aminocarbonyle, d'un groupe di(alkyl en $C_1$-$C_6$)aminocarbonyle, d'un groupe mono(alkyl en $C_1$-$C_6$)aminocarbonyloxy, d'un groupe di (alkyl en $C_1$-$C_6$)aminocarbonyloxy, d'un groupe mono(alkyl en $C_2$-$C_7$)carbonylamino, d'un groupe mono(alkyl en $C_1$-$C_6$)sulfonylamino, d'un groupe cyano, d'un groupe nitro, d'un groupe carbamoyle, d'un groupe phényloxy qui peut être substitué par 1 à 5 groupes indépendamment choisis dans le groupe de substituants d, d'un groupe phénylthio qui peut être substitué par 1 à 5 groupes indépendamment choisis dans le groupe de substituants d, d'un groupe phényloxyméthyle qui peut être substitué par 1 à 5 groupes indépendamment choisis dans le groupe de substituants d, d'un groupe phénylméthyloxy qui peut être substitué par 1 à 5 groupes indépendamment choisis dans le groupe de substituants d, d'un groupe phénylthiométhyle qui peut être substitué par 1 à 5 groupes indépendamment choisis dans le groupe de substituants d, d'un groupe phényloxycarbonyle qui peut être substitué par 1 à 5 groupes indépendamment choisis dans le groupe de substituants d, d'un groupe phénylcarbonylamino qui peut être substitué par 1 à 5 groupes indépendamment choisis dans le groupe de substituants d, d'un groupe phénylsulfonylamino qui peut être substitué par 1 à 5 groupes indépendamment choisis dans le groupe de substituants d, d'un groupe phénylaminocarbonyloxy qui peut être substitué par 1 à 5 groupes indépendamment choisis dans le groupe de substituants d, d'un groupe phényloxycarbonylamino qui peut être substitué par 1 à 5 groupes indépendamment choisis dans le groupe de substituants d, d'un groupe phénylaminocarbonylamino qui peut être substitué par 1 à 5 groupes indépendamment choisis dans le groupe de substituants d, d'un groupe alkyle en $C_1$-$C_6$ substitué par 1 groupe carboxy, d'un groupe alkyle en $C_1$-$C_6$ substitué par 1 groupe alcoxycarbonyle en $C_2$-$C_7$, d'un groupe alcoxy en $C_1$-$C_6$ substitué par 1 groupe alcoxycarbonyle en $C_2$-$C_7$ et d'un groupe alcoxy en $C_1$-$C_6$ substitué par 1 groupe di(alkyl en $C_1$-$C_6$)-aminocarbonyle,

le groupe de substituants b représente le groupe constitué d'un atome d'halogène, d'un groupe alkyle en $C_1$-$C_6$, d'un groupe alkyle halogéné en $C_1$-$C_6$, d'un groupe alcoxy en $C_1$-$C_6$, d'un groupe alcynyloxy en $C_2$-$C_6$, d'un groupe

hydroxy et d'un groupe carboxy,
le groupe de substituants c représente le groupe constitué d'un groupe cycloalkyle en $C_3$-$C_6$, d'un groupe phényle, d'un groupe hydroxy, d'un groupe carboxy, d'un groupe alkylcarbonyle en $C_2$-$C_7$ et d'un groupe alcoxycarbonyle en $C_2$-$C_7$, et
le groupe de substituants d représente le groupe constitué d'un atome d'halogène, d'un groupe alkyle en $C_1$-$C_6$, d'un groupe alkyle halogéné en $C_1$-$C_6$ et d'un groupe alcoxy en $C_1$-$C_6$],
un de ses sels pharmacologiquement acceptables ou un de ses esters.

**2.** Dérivé de cercosporamide, un de ses sels pharmacologiquement acceptables ou un de ses esters selon la revendication 1, dans lequel la formule générale (I) est une formule générale (Ia):

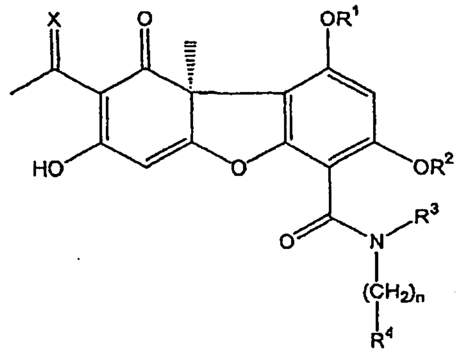

(Ia)

**3.** Dérivé de cercosporamide, un de ses sels pharmacologiquement acceptables ou un de ses esters selon la revendication 1 ou 2, dans lequel X est un atome d'oxygène ou un groupe représenté par la formule =N-O-$R^5$.

**4.** Dérivé de cercosporamide, un de ses sels pharmacologiquement acceptables ou un de ses esters selon l'une quelconque des revendications 1 à 3, dans lequel $R^5$ est un groupe méthyle, un groupe éthyle, un groupe propyle, un groupe 2-propényle ou un groupe cyclopropylméthyle.

**5.** Dérivé de cercosporamide, un de ses sels pharmacologiquement acceptables ou un de ses esters selon la revendication 1 ou 2, dans lequel X est un atome d'oxygène.

**6.** Dérivé de cercosporamide, un de ses sels pharmacologiquement acceptables ou un de ses esters selon l'une quelconque des revendications 1 à 5, dans lequel $R^1$ est un atome d'hydrogène.

**7.** Dérivé de cercosporamide, un de ses sels pharmacologiquement acceptables ou un de ses esters selon l'une quelconque des revendications 1 à 6, dans lequel $R^2$ est un groupe méthyle.

**8.** Dérivé de cercosporamide, un de ses sels pharmacologiquement acceptables ou un de ses esters selon l'une quelconque des revendications 1 à 7, dans lequel $R^3$ est un atome d'hydrogène.

**9.** Dérivé de cercosporamide, un de ses sels pharmacologiquement acceptables ou un de ses esters selon l'une quelconque des revendications 1 à 8, dans lequel $R^4$ est un groupe phényle substitué par 1 à 5 groupes indépendamment choisis dans le groupe de substituants a ou un groupe 1-naphtyle qui peut être substitué par 1 à 3 groupes indépendamment choisis dans le groupe de substituants a.

**10.** Dérivé de cercosporamide, un de ses sels pharmacologiquement acceptables ou un de ses esters selon l'une quelconque des revendications 1 à 8, dans lequel $R^4$ est un groupe phényle substitué par 1 à 5 groupes indépendamment choisis dans le groupe de substituants e ou un groupe 1-naphtyle qui peut être substitué par 1 à 3 groupes indépendamment choisis dans le groupe de substituants e, et
le groupe de substituants e représente le groupe constitué d'un atome d'halogène; d'un groupe alkyle en $C_1$-$C_6$; d'un groupe alkyle halogéné en $C_1$-$C_6$; d'un groupe alcényle en $C_2$-$C_6$; d'un groupe alcoxy en $C_1$-$C_6$; d'un groupe alcynyloxy en $C_2$-$C_6$; d'un groupe (alcoxy en $C_1$-$C_6$)-(alkyle en $C_1$-$C_6$); d'un groupe (alcynyloxy en $C_2$-$C_6$)-(alkyle en $C_1$-$C_6$); d'un groupe hydroxy; d'un groupe carboxy; d'un groupe cycloalkylcarbonyle en $C_4$-$C_7$; d'un groupe

alcoxycarbonyle en $C_2$-$C_7$; d'un groupe di(alkyl en $C_1$-$C_6$)aminocarbonyle; d'un groupe di(alkyl en $C_1$-$C_6$)aminocarbonyloxy; d'un groupe mono(alkyl en $C_1$-$C_6$)sulfonylamino; d'un groupe cyano; d'un groupe nitro; d'un groupe phényloxy, d'un groupe phénylthio, d'un groupe phényloxyméthyle, d'un groupe phénylméthyloxy, d'un groupe phényloxycarbonyle, d'un groupe phénylcarbonylamino ou d'un groupe phénylsulfonylamino qui peuvent être substitués par 1 à 3 groupes indépendamment choisis parmi un atome de fluor, un atome de chlore, un groupe méthyle et un groupe trifluorométhyle; d'un groupe alkyle en $C_1$-$C_6$ substitué par 1 groupe carboxy; et d'un groupe alkyle en $C_1$-$C_6$ substitué par 1 groupe alcoxycarbonyle en $C_2$-$C_7$.

**11.** Dérivé de cercosporamide, un de ses sels pharmacologiquement acceptables ou un de ses esters selon l'une quelconque des revendications 1 à 8, dans lequel $R^4$ est un groupe 1-naphtyle substitué par 1 à 5 groupes indépendamment choisis parmi un atome de fluor, un atome de chlore, un atome de brome, un groupe méthyle et un groupe éthyle.

**12.** Dérivé de cercosporamide, un de ses sels pharmacologiquement acceptables ou un de ses esters selon l'une quelconque des revendications 1 à 8, dans lequel $R^4$ est un groupe 1-naphtyle substitué en position 2 et/ou 3 par 1 ou 2 groupes indépendamment choisis parmi un groupe méthyle et un groupe éthyle et substitué en position 4, 5, 6, 7 et/ou 8 par 1 à 3 groupes indépendamment choisis parmi un atome de fluor et un atome de chlore.

**13.** Dérivé de cercosporamide, un de ses sels pharmacologiquement acceptables ou un de ses esters selon l'une quelconque des revendications 1 à 8, dans lequel $R^4$ est un groupe 1-naphtyle substitué en position 2 par un groupe méthyle ou un groupe éthyle et substitué en position 4, 5, 6, 7 et/ou 8 par 1 à 3 groupes indépendamment choisis parmi un atome de fluor et un atome de chlore.

**14.** Dérivé de cercosporamide, un de ses sels pharmacologiquement acceptables ou un de ses esters selon l'une quelconque des revendications 1 à 13, dans lequel n vaut 1.

**15.** Dérivé de cercosporamide, un de ses sels pharmacologiquement acceptables ou un de ses esters selon la revendication 1, dans lequel le composé de formule générale (I) est le suivant:

(9aS)-8-acétyl-N-[4-(2-butynyloxy)-2,6-diméthylbenzyl]-1,7-dihydroxy-3-méthoxy-9a-méthyl-9-oxo-9,9a-dihydrodibenzo[b,d]furan-4-carboxamide,
(9aS)-8-acétyl-N-[2,6-diméthyl-4-(2-pentynyloxy)benzyl]-1,7-dihydroxy-3-méthoxy-9a-méthyl-9-oxo-9,9a-dihydrodibenzo[b,d]furan-4-carboxamide,
(9aS)-8-acétyl-N-(4-{[(2,4-dichlorophényl)sulfonyl]amino}-2,6-diméthylbenzyl)-1,7-dihydroxy-3-méthoxy-9a-méthyl-9-oxo-9,9a-dihydrodibenzo[b,d]furan-4-carboxamide,
(9aS)-8-acétyl-N-(4-{[(2,4-dichloro-5-méthylphényl)sulfonyl]amino}-2,6-diméthylbenzyl)-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-9-oxo-N-(2,3,5,6-tétraméthylbenzyl)-9,9a-dihydrodibenzo[b,d]furan-4-carboxamide,
(9aS)-8-acétyl-1,7-dihydroxy-3-méthoxy-9a-méthyl-9-oxo-N-(2,3,5-triméthyl-6-propylbenzyl)-9,9a-dihydrodibenzo[b,d]furan-4-carboxamide,
(9aS)-8-acétyl-N-[4-(2-butynyloxy)-2,3,6-triméthylbenzyl]-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-9-oxo-N-[2,3,6-triméthyl-4-(2-pentynyloxy)benzyl]-9,9a-dihydrodibenzo[b,d]furan-4-carboxamide,
(9aS)-8-acétyl-1,7-dihydroxy-3-méthoxy-9a-méthyl-N-(1-naphtylméthyl)-9-oxo-9,9a-dihydrodibenzo[b,d]furan-4-carboxamide,
(9aS)-8-acétyl-N-[(4-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-N-[(2-méthyl-1-naphtyl)méthyl]-9-oxo-9,9a-dihydrodibenzo[b,d]furan-4-carboxamide,
(9aS)-8-acétyl-N-[(2-éthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-{[4-(4-chlorophénoxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-{[4-(3-chlorophénoxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-{[4-(2,4-dichlorophénoxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-{[4-(benzyloxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-({4-[(4-fluorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-N-({4-[(3-fluorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-N-({4-[(3-chlorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-N-({4-[(2,4-difluorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-N-[(2,3-diméthyl-1-naphtyl)méthyl]-9-oxo-9,9a-dihydrodibenzo[b,d]furan-4-carboxamide,
(9aS)-8-acétyl-N-[(2-éthyl-3-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-N-[(2,3,4-triméthyl-1-naphtyl)méthyl]-9-oxo-9,9a-dihydrodibenzo[b,d]furan-4-carboxamide,
(9aS)-8-acétyl-1,7-dihydroxy-3-méthoxy-9a-méthyl-N-[(2,4-diméthyl-1-naphtyl)méthyl]-9-oxo-9,9a-dihydrodibenzo[b,d]furan-4-carboxamide,
(9aS)-8-acétyl-3-(difluorométhoxy)-1,7-dihydroxy-9a-méthyl-N-[(2-méthyl-1-naphtyl)méthyl]-9-oxo-9,9a-dihydrodibenzo[b,d]furan-4-carboxamide,
(9aS)-1,7-dihydroxy-3-méthoxy-8-[(1E)-N-méthoxyéthaneimidoyl]-9a-méthyl-N-[(2-méthyl-1-naphtyl)méthyl]-9-oxo-9,9a-dihydrodibenzo[b,d]furan-4-carboxamide ou
(9aS)-8-[(1E)-N-(allyloxy)éthaneimidoyl]-N-(4-{[(2,4-dichlorophényl)sulfonyl]amino}-2,6-diméthylbenzyl)-1,7-dihydroxy-3-méthoxy-9a-méthyl-9-oxo-9,9a-dihydrodibenzo[b,d]furan-4-carboxamide.

**16.** Dérivé de cercosporamide, un de ses sels pharmacologiquement acceptables ou un de ses esters selon la revendication 1, dans lequel le composé de formule générale (I) est le suivant:

(9aS)-8-acétyl-N-[(4-fluoro-2-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(7-fluoro-2-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-4-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-7-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-8-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(7-chloro-2-éthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(5,7-difluoro-2-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-5,7-difluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-6,7-difluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-6,8-difluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-7,8-difluoro-1-naphtyl)méthyl]-1,7-dihydroxy-3-méthoxy-9a-méthyl-9-oxo-9,9a-dihydrodibenzo[b,d]furan-4-carboxamide ou
(9aS)-8-acétyl-1,7-dihydroxy-3-méthoxy-9a-méthyl-9-oxo-N-[(5,6,7-trifluoro-2-méthyl-1-naphtyl)méthyl]-9,9a-dihydrodibenzo[b,d]furan-4-carboxamide.

**17.** Dérivé de cercosporamide ou un de ses sels pharmacologiquement acceptables selon la revendication 1, dans lequel le composé de formule générale (I) est le suivant:

(9aS)-8-acétyl-N-[4-(2-butynyloxy)-2,6-diméthylbenzyl]-1,7-dihydroxy-3-méthoxy-9a-méthyl-9-oxo-9,9a-dihydrodibenzo[b,d]furan-4-carboxamide,
(9aS)-8-acétyl-N-[2,6-diméthyl-4-(2-pentynyloxy)benzyl]-1,7-dihydroxy-3-méthoxy-9a-méthyl-9-oxo-9,9a-dihydrodibenzo[b,d]furan-4-carboxamide,
(9aS)-8-acétyl-N-(4-{[(2,4-dichlorophényl)sulfonyl]amino}-2,6-diméthylbenzyl)-1,7-dihydroxy-3-méthoxy-9a-méthyl-9-oxo-9,9a-dihydrodibenzo[b,d]furan-4-carboxamide,
(9aS)-8-acétyl-N-(4-{[(2,4-dichloro-5-méthylphényl)sulfonyl]amino}-2,6-diméthylbenzyl)-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-9-oxo-N-(2,3,5,6-tétraméthylbenzyl)-9,9a-dihydrodibenzo[b,d]furan-4-carboxamide,
(9aS)-8-acétyl-1,7-dihydroxy-3-méthoxy-9a-méthyl-9-oxo-N-(2,3,5-triméthyl-6-propylbenzyl)-9,9a-dihydrodibenzo[b,d]furan-4-carboxamide,
(9aS)-8-acétyl-N-[4-(2-butynyloxy)-2,3,6-triméthylbenzyl]-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-9-oxo-N-[2,3,6-triméthyl-4-(2-pentynyloxy)benzyl]-9,9a-dihydrodibenzo[b,d]furan-4-carboxamide,
(9aS)-8-acétyl-1,7-dihydroxy-3-méthoxy-9a-méthyl-N-(1-naphtylméthyl)-9-oxo-9,9a-dihydrodibenzo[b,d]furan-4-carboxamide,
(9aS)-8-acétyl-N-[(4-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-N-[(2-méthyl-1-naphtyl)méthyl]-9-oxo-9,9a-dihydrodibenzo[b,d]furan-4-carboxamide,
(9aS)-8-acétyl-N-[(2-éthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-{[4-(4-chlorophénoxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-{[4-(3-chlorophénoxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-{[4-(2,4-dichlorophénoxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-{[4-(benzyloxy)-1-naphtyl]méthyl}-1,7-dihydroxy-3-méthoxy-9a-méthyl-9-oxo-9,9a-dihydrodibenzo[b,d]furan-4-carboxamide,
(9aS)-8-acétyl-N-({4-[(4-fluorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-N-({4-[(3-fluorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-N-({4-[(3-chlorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-N-({4-[(2,4-difluorobenzyl)oxy]-1-naphtyl}méthyl)-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-N-[(2,3-diméthyl-1-naphtyl)méthyl]-9-oxo-9,9a-dihydrodibenzo[b,d]furan-4-carboxamide,
(9aS)-8-acétyl-N-[(2-éthyl-3-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-1,7-dihydroxy-3-méthoxy-9a-méthyl-N-[(2,3,4-triméthyl-1-naphtyl)méthyl]-9-oxo-9,9a-dihydrodibenzo[b,d]furan-4-carboxamide,
(9aS)-8-acétyl-1,7-dihydroxy-3-méthoxy-9a-méthyl-N-[(2,4-diméthyl-1-naphtyl)méthyl]-9-oxo-9,9a-dihydrodibenzo[b,d]furan-4-carboxamide,
(9aS)-8-acétyl-3-(difluorométhoxy)-1,7-dihydroxy-9a-méthyl-N-[(2-méthyl-1-naphtyl)méthyl]-9-oxo-9,9a-dihydrodibenzo[b,d]furan-4-carboxamide,
(9aS)-1,7-dihydroxy-3-méthoxy-8-[(1E)-N-méthoxyéthaneimidoyl]-9a-méthyl-N-[(2-méthyl-1-naphtyl)méthyl]-9-oxo-9,9a-dihydrodibenzo[b,d]furan-4-carboxamide ou
(9aS)-8-[(1E)-N-(allyloxy)éthaneimidoyl]-N-(4-{[(2,4-dichlorophényl)sulfonyl]amino}-2,6-diméthylbenzyl)-1,7-dihydroxy-3-méthoxy-9a-méthyl-9-oxo-9,9a-dihydrodibenzo[b,d]furan-4-carboxamide.

**18.** Dérivé de cercosporamide ou un de ses sels pharmacologiquement acceptables selon la revendication 1, dans lequel le composé de formule générale (I) est le suivant:

(9aS)-8-acétyl-N-[(4-fluoro-2-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(7-fluoro-2-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-4-fluoro-1-naphtyl)méthyl]-1,7-dihydroxy-3-méthoxy-9a-méthyl-9-oxo-9,9a-dihydrodibenzo[b,d]furan-4-carboxamide),
(9aS)-8-acétyl-N-[(2-éthyl-7-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-8-fluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(7-chloro-2-éthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(5,7-difluoro-2-méthyl-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-5,7-difluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-6,7-difluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-6,8-difluoro-1-naphtyl)méthyl]-1,7-dihydroxy-3-méthoxy-9a-méthyl-9-oxo-9,9a-dihydrodibenzo[b,d]furan-4-carboxamide,
(9aS)-8-acétyl-N-[(2-éthyl-7,8-difluoro-1-naphtyl)méthyl]-1,7-dihydroxy-3-méthoxy-9a-méthyl-9-oxo-9,9a-dihydrodibenzo[b,d]furan-4-carboxamide ou
(9aS)-8-acétyl-1,7-dihydroxy-3-méthoxy-9a-méthyl-9-oxo-N-[(5,6,7-trifluoro-2-méthyl-1-naphtyl)méthyl]-9,9a-dihydrodibenzo[b,d]furan-4-carboxamide.

**19.** Composition pharmaceutique comprenant le dérivé de cercosporamide, un de ses sels pharmacologiquement acceptables ou un de ses esters selon l'une quelconque des revendications 1 à 18 en tant que principe actif.

**20.** Composition pharmaceutique selon la revendication 19 destinée à abaisser le taux de glucose dans le sang, comprenant le dérivé de cercosporamide, un de ses sels pharmacologiquement acceptables ou un de ses esters selon l'une quelconque des revendications 1 à 18 en tant que principe actif.

**21.** Composition pharmaceutique selon la revendication 19 destinée au traitement et/ou à la prévention du diabète, comprenant le dérivé de cercosporamide, un de ses sels pharmacologiquement acceptables ou un de ses esters selon l'une quelconque des revendications 1 à 18 en tant que principe actif.

**22.** Utilisation du dérivé de cercosporamide, d'un de ses sels pharmacologiquement acceptables ou d'un de ses esters selon l'une quelconque des revendications 1 à 18 pour la fabrication d'une composition pharmaceutique.

**23.** Utilisation selon la revendication 22, dans laquelle la composition pharmaceutique est une composition destinée à abaisser le taux de glucose dans le sang.

**24.** Utilisation selon la revendication 22, dans laquelle la composition pharmaceutique est une composition destinée au traitement et/ou à la prévention du diabète.

[Fig. 1]

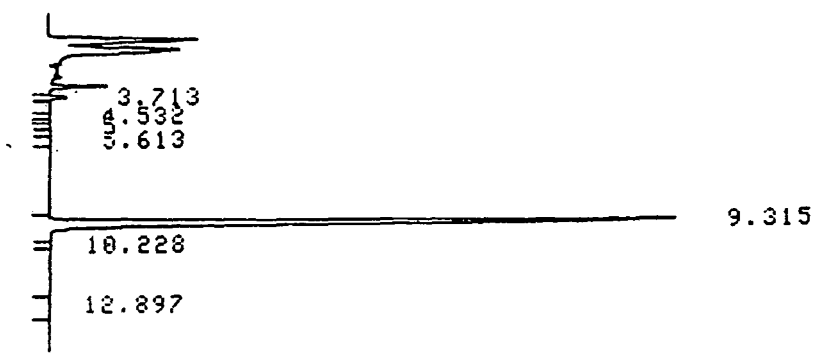

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4983587 A
- WO 03059905 A

### Non-patent literature cited in the description


- *J. Org. Chem.,* 1991, vol. 56, 909-910
- *Tetrahedron,* 1992, vol. 48, 4757-4766
- *Molecular and Cellular Biology,* 1994, vol. 14, 1017-1025
- *Phytochemistry,* 1992, vol. 31, 2999-3001
- *Eukaryotic Cell,* 2004, vol. 3, 932-943
- protecting group that can be cleaved in vivo by a biological method such as hydrolysis. Design of Prodrugs. Elsevier, 1985
- Bunshi Sekkei. Iyakuhin No Kaihatsu. Hirokawa Publishing Company, 1990, vol. 7
- **T. W. Greene et al.** Protective Groups in Organic Synthesis. John Wiley & Sons, Inc, 1999
- *Journal of Organic Chemistry,* 1991, vol. 56, 909-910
- *Journal of Organic Chemistry,* 1995, vol. 60, 7739-7746
- **Theodora W. Greene ; Peter G. M. Wuts.** Protective Groups in Organic Synthesis. A Wiley-Interscience Publication, 1999
- *Tetrahedron Lett.,* 1999, vol. 40, 2295-2298
- *Journal of Organic Chemistry,* 1968, vol. 33, 4288-4290
- *Journal of Chemical Society Perkin Transaction,* 1988, vol. 1, 2595-2601
- **Kornerup, A. ; Wanscher, J.H.** Methuen Handbook of Colour. 1978, 1-252
- **Spooner, BM.** *Bibliotheca Mycologica,* 1987, vol. 116, 1-711
- **Dennis, RWG.** *Mycological Papers,* 1949, vol. 32, 1-97
- **Tanaka, I ; Hosoya, T.** *Mycoscience,* 2001, vol. 42, 597-609
- **Baral, HO ; Krieglsteiner, GJ.** *Beihefte zur Zeitschrift für Mykologie,* 1985, vol. 6, 1-160
- **Krik, PM et al.** Ainsworth & Bisby's Dictionary of Fungi. CABI International, 2001, 1-655
- *Journal of American Chemical Society,* 1962, vol. 84, 3541-3546
- *Tetrahedron,* 1985, vol. 41, 5205-5208
- *Journal of Chemical Society Perkin Transactions,* 1972, vol. 1, 892-894
- *Tetrahedron,* 1999, vol. 55, 5821-5830
- *Journal of Organic Chemistry,* 2004, vol. 69, 5568-5577
- *Tetrahedron,* 1997, vol. 53, 15969-15982
- *Journal of the American Chemical Society,* 1992, vol. 114, 255-261
- *Journal of Organic Chemistry,* 1941, vol. 6, 489-500
- *Journal of Organic Chemistry,* 1962, vol. 27, 18839-1842
- *Journal of Organic Chemistry,* 1962, vol. 27, 1839-1842
- *Australian Journal of Chemistry,* 1977, vol. 30, 927-930
- *Journal of Organic Chemistry,* 1995, vol. 60, 6592-6594
- *Journal of Organic Chemistry USSR,* 1967, 1461-1462
- *Journal of Heterocyclic Chemistry,* 2002, vol. 39, 965-973
- *Journal of Medicinal Chemistry,* 1995, vol. 38, 2202-2216
- *Journal of Medicinal Chemistry,* 1972, vol. 15, 569-570
- *Tetrahedron Letters,* 1990, vol. 31, 6883-6886